# EUROPEAN PATENT APPLICATION

(11) **EP 1 712 227 A1**
(43) Date of publication of application: **18.10.2006**
(21) Application number: 06002885.9
(22) Date of filing: 19.11.2002
(51) Int. Cl.: A61K 31/13, A61P 25/28

(54) **Amino diols useful in the treatment of alzheimer's disease**

(30) Priority: 19.11.2001 US 332863
(62) Divisional of application: 02789765.1
(71) Applicant: ELAN PHARMACEUTICALS, INC., South San Francisco, CA 94080 (US); Pharmacia & Upjohn, Kalamazoo, MI 49007 (US)
(72) Inventor: Schostarez, Heinrich J., Portage MI 49024 (US); Hanson, Gunnar J., Skokie IL 60076 (US)
(74) Representative: Adam, Holger

(57) **Abstract**

The present invention relates to the treatment of Alzheimer's disease and other similar diseases, and more specifically to the use of compounds that inhibit betasecretase, an enzyme that cleaves amyloid precursor protein to produce A beta peptide, a major component of the amyloid plaques found in the brains of Alzheimer's sufferers, in such methods.

## Description

This application claims priority to U.S. Provisional Patent Application No.: 60/332,863, filed on November 19, 2001.

### Field of the Invention

The present invention relates to the treatment of Alzheimer's disease and other similar diseases, and more specifically to the use of compounds that inhibit beta-secretase, an enzyme that cleaves amyloid precursor protein to produce A beta peptide, a major component of the amyloid plaques found in the brains of Alzheimer's sufferers, in such methods.

### Background of the Invention

Alzheimer's disease (AD) is a progressive degenerative disease of the brain primarily associated with aging. Clinical presentation of AD is characterized by loss of memory, cognition, reasoning, judgment, and orientation. As the disease progresses, motor, sensory, and linguistic abilities are also affected until there is global impairment of multiple cognitive functions. These cognitive losses occur gradually, but typically lead to severe impairment and eventual death in the range of four to twelve years.

Alzheimer's disease is characterized by two major pathologic observations in the brain: neurofibrillary tangles and beta amyloid (or neuritic) plaques, comprised predominantly of an aggregate of a peptide fragment know as A beta. Individuals with AD exhibit characteristic beta-amyloid deposits in the brain (beta amyloid plaques) and in cerebral blood vessels (beta amyloid angiopathy) as well as neurofibrillary tangles. Neurofibrillary tangles occur not only in Alzheimer's disease but also in other dementia-inducing disorders. On autopsy, large numbers of these lesions are generally found in areas of the human brain important for memory and cognition.

Smaller numbers of these lesions in a more restricted anatomical distribution are found in the brains of most aged humans who do not have clinical AD. Amyloidogenic plaques and vascular amyloid angiopathy also characterize the brains of individuals with Trisomy 21 (Down's Syndrome), Hereditary Cerebral Hemorrhage with Amyloidosis of the Dutch-Type (HCHWA-D), and other neurodegenerative disorders. Beta-amyloid is a defining feature of AD, now believed to be a causative precursor or factor in the development of disease. Deposition of A beta in areas of the brain responsible for cognitive activities is a major factor in the development of AD. Beta-amyloid plaques are predominantly composed of amyloid beta peptide (A beta, also sometimes designated betaA4). A beta peptide is derived by proteolysis of the amyloid precursor protein (APP) and is comprised of 39-42 amino acids. Several proteases called secretases are involved in the processing of APP.

Cleavage of APP at the N-terminus of the A beta peptide by beta-secretase and at the C-terminus by one or more gamma-secretases constitutes the beta-amyloidogenic pathway, i.e. the pathway by which A beta is formed. Cleavage of APP by alpha-secretase produces alpha-sAPP, a secreted form of APP that does not result in beta-amyloid plaque formation. This alternate pathway precludes the formation of A beta peptide. A description of the proteolytic processing fragments of APP is found, for example, in U.S. Patent Nos. 5,441,870; 5,721,130; and 5,942,400.

An aspartyl protease has been identified as the enzyme responsible for processing of APP at the beta-secretase cleavage site. The beta-secretase enzyme has been disclosed using varied nomenclature, including BACE, Asp, and Memapsin. See, for example, Sindha et al., 1999, Nature 402:537-554 (p501) and published PCT application WOOO/17369.

Several lines of evidence indicate that progressive cerebral deposition of beta-amyloid peptide (A beta) plays a seminal role in the pathogenesis of AD and can precede cognitive symptoms by years or decades. See, for example, Selkoe, 1991, *Neuron* 6:487. Release of A beta from neuronal cells grown in culture and the presence of A beta in cerebrospinal fluid (CSF) of both normal individuals and AD subjects has been demonstrated. See, for example, Seubert et al., 1992, Nature 359:325-327.

It has been proposed that A beta peptide accumulates as a result of APP processing by beta-secretase, thus inhibition of this enzyme's activity is desirable for the treatment of AD. *In vivo* processing of APP at the beta-secretase cleavage site is thought to be a rate-limiting step in A beta production, and is thus a therapeutic target for the treatment of AD. See for example, Sabbagh, M., et al., 1997, *Alz*. *Dis. Rev.* 3, 1-19.

BACE1 knockout mice fail to produce A beta, and present a normal phenotype. When crossed with transgenic mice that over express APP, the progeny show reduced amounts of A beta in brain extracts as compared with control animals (Luo et al., 2001 *Nature Neuroscience* 4:231-232). This evidence further supports the proposal that inhibition of beta-secretase activity and reduction of A beta in the brain provides a therapeutic method for the treatment of AD and other beta amyloid disorders. At present there are no effective treatments for halting, preventing, or reversing the progression of Alzheimer's disease. Therefore, there is an urgent need for pharmaceutical agents capable of slowing the progression of Alzheimer's disease and/or preventing it in the first place.

Compounds that are effective inhibitors of beta-secretase, that inhibit beta-secretase-mediated cleavage of APP, that are effective inhibitors of A beta production, and/or are effective to reduce amyloid beta deposits or plaques, are needed for the treatment and prevention of disease characterized by amyloid beta deposits or plaques, such as AD.

At present there are no effective treatments for halting, preventing, or reversing the progression of Alzheimer's disease. Therefore, there is an urgent need for pharmaceutical agents capable of slowing the progression of Alzheimer's disease and/or preventing it in the first place.

Compounds that are effective inhibitors of beta-secretase, that inhibit beta-secretase-mediated cleavage of APP, that are effective inhibitors of A beta production, and/or are effective to reduce amyloid beta deposits or plaques, are needed for the treatment and prevention of disease characterized by amyloid beta deposits or plaques, such as AD.

### SUMMARY OF INVENTION

The present invention relates to methods of treating a subject who has, or in preventing a subject from developing, a disease or condition selected from the group consisting of Alzheimer's disease, for helping prevent or delay the onset of Alzheimer's disease, for helping to slow the progression of Alzheimer's disease, for treating subjects with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease and who is in need of such treatment which comprises administration of a therapeutically effective amount of a compound described in U.S. Patent Nos. 5,420,127, 5,428,064, 5,416,111, 5,424,307, 5,424,319, 5,418,249, 5,416,104, 5,428,054, 5,416,092, 5,411,957, 5,416,085, 5,411,962, 5,411,958, 5,414,012, 5,416,095, 5,416,083, 4,977,141, 4,931,429, 5,089,471, 4,877,785, 4,900,746, 5,229,420, 4,902,706, 5,229,369, 4,900,745, 5,246,959, 5,175,170, 5,180,744, 5,175,181, 5,171,751, 5,210,095, 5,283,250, 5,180,725, 5,179,102, 5,212,175, 5,147,888, 5,217,991, 5,223,512, 5,231,111, 5,223,534, 5,223,532, 5,223,514, 5,212,174, 5,215,996, 5,217,989, 5,290,787, 5,217,988, 5,216,013, 5,227,401, 5,484,811, 5,942,548, 6,174,923, 5,223,535, 5,484,812, 5,330,996, 5,508,295, 5,849,773, 5,314,920, 5,317,039, 5,246,969, 5,461,074, 5,268,391, 5,312,838, 5,416,119, 5,488,066, 5,756,549, 5,212,185, 5,304,552, 5,385,898, 5,780,494, 5,422,349, 5,409,922, 5,411,953, 5,411,959, 5,411,975, 5,409,913, 5,409,925, 5,409,921, 5,411,961, 5,413,998, 5,411,954, 5,416,084, 5,409,945, 5,411,950, 5,409,924, 5,373,017, 5,252,591, 5,434,162, 5,563,154, 5,298,505, 5,414,018, 5,488,067, 5,432,201, and 5,968,984, i.e., a compound of formulas (I)-(X): 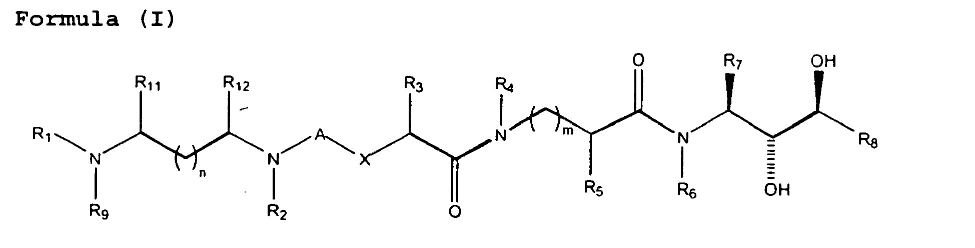 wherein A is selected from methylene, CO, SO, and SO₂;
wherein X is selected from oxygen atom, methylene and 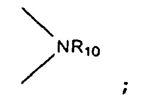 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide,
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl;
R₅ is selected from 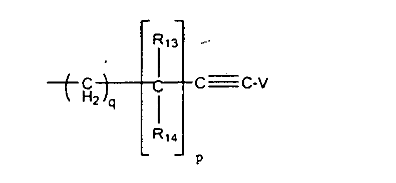 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 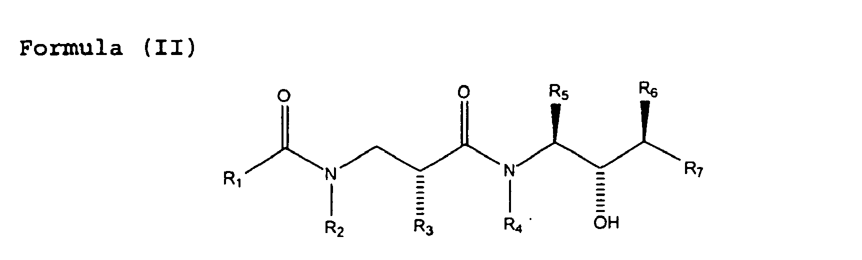 wherein R₁ is selected from aryl, aralkyl, heteroaryl and heteroaralkyl, including the following: 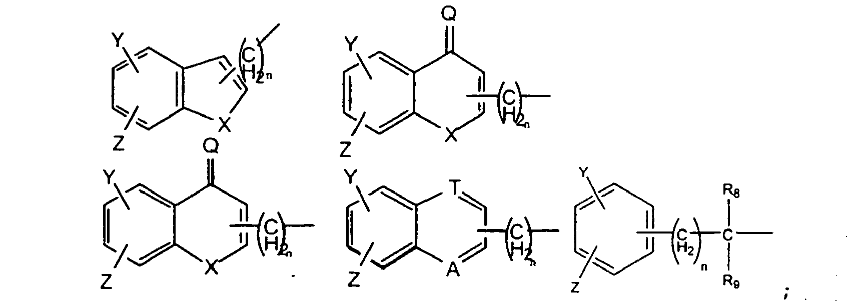 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 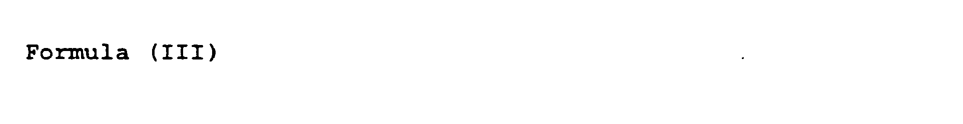 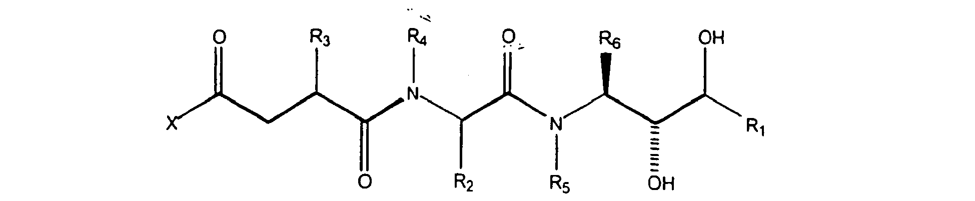 wherein X is selected from 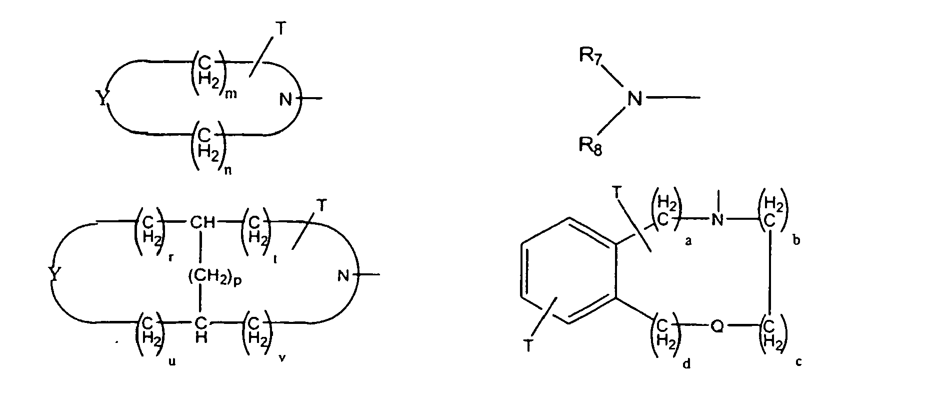 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 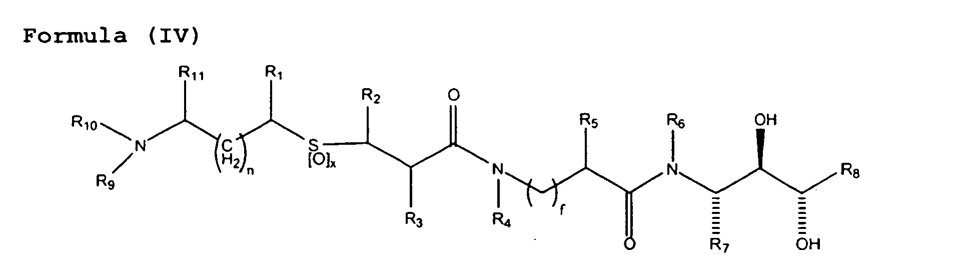 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 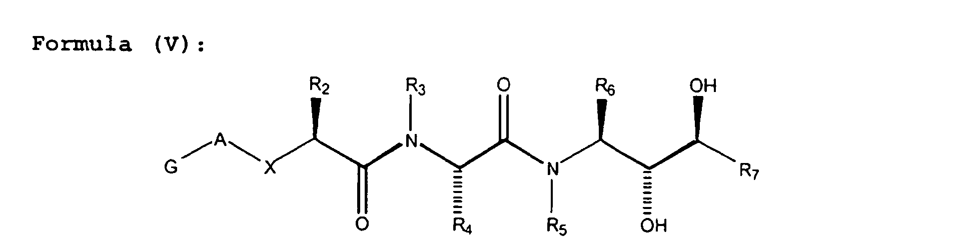 wherein A is selected from CO and SO₂
X is selected form oxygen atom and methylene; .
G is selected from B or 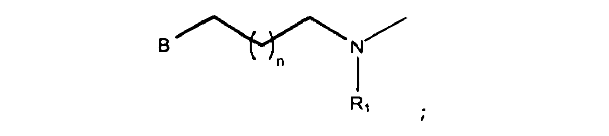 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 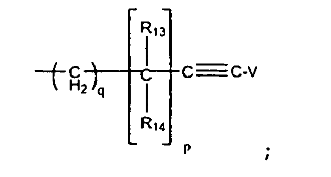 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 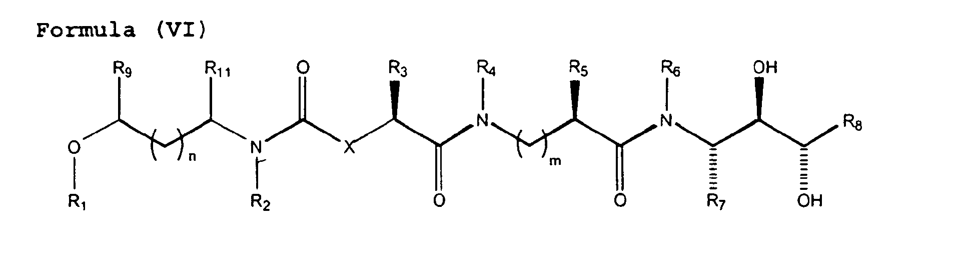 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphtylmethyl, aryl and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 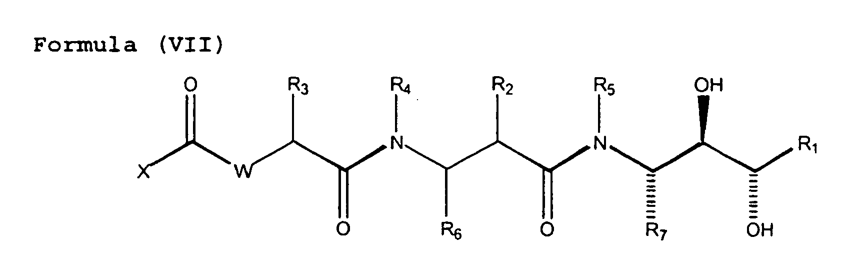 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 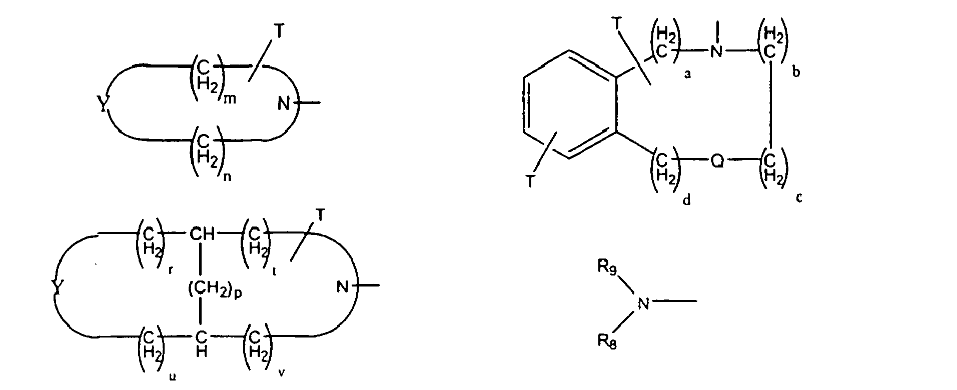 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 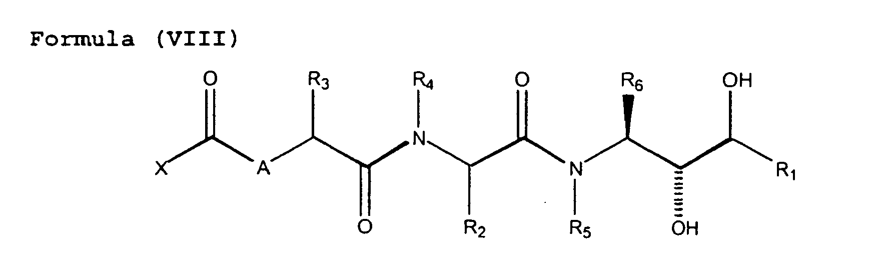 wherein X is selected from 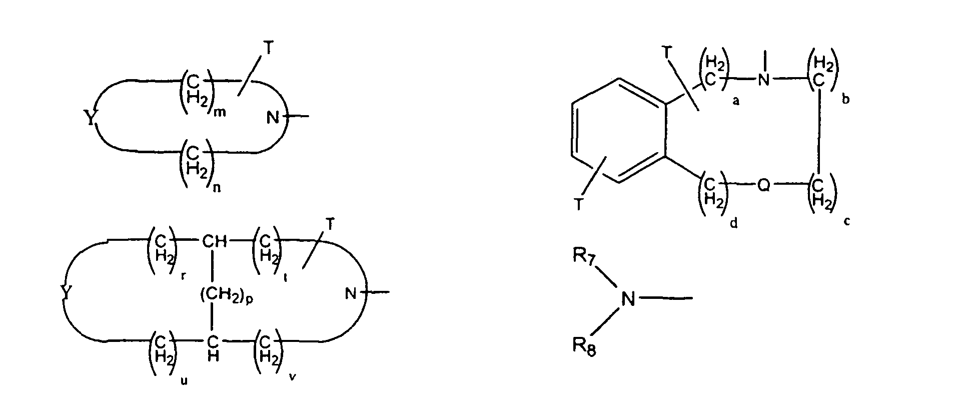 wherein Y and Q are selected from CH₂, CH-O-R₉, O, S, SO, SO₂ and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 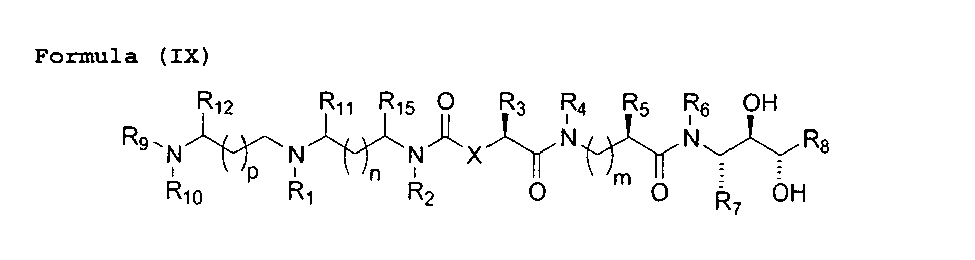 wherein X is selected from oxygen atom, methylene and >NR₁3 ,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalky-1, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH(OH)R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 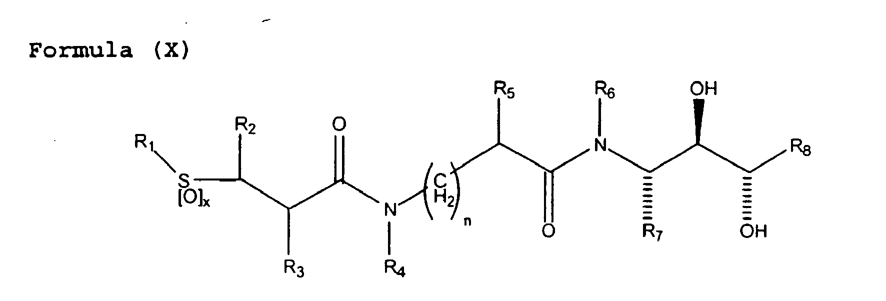 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl; and pharmaceutically-acceptable salts thereof.

The above referenced U.S. Patents disclose compounds of the general formulas (I)-(X) and their use as agents for inhibition of renin. The reader is directed to U.S. Patent Nos. 5,420,127, 5,428,064, 5,416,111, 5,424,307, 5,424,319, 5,418,249, 5,416,104, 5,428,054, 5,416,092, 5,411,957, 5,416,085, 5,411,962, 5,411,958, 5,414,012, 5,416,095, 5,416,083, 4,977,141, 4,931,429, 5,089,471, 4,877,785, 4,900,746, 5,229,420, 4,902,706, 5,229,369, 4,900,745, 5,246,959, 5,175,170, 5,180,744, 5,175,181, 5,171,751, 5,210,095, 5,283,250, 5,180,725, 5,179,102, 5,212,175, 5,147,888, 5,217,991, 5,223,512, 5,231,111, 5,223,534, 5,223,532, 5,223,514, 5,212,174, 5,215,996, 5,217,989, 5,290,787, 5,217,988, 5,216,013, 5,227,401, 5,484,811, 5,942,548, 6,174,923, 5,223,535, 5,484,812, 5,330,996, 5,508,295, 5,849,773, 5,314,920, 5,317,039, 5,246,969, 5,461,074, 5,268,391, 5,312,838, 5,416,119, 5,488,066, 5,756,549, 5,212,185, 5,304,552, 5,385,898, 5,780,494, 5,422,349, 5,409,922, 5,411,953, 5,411,959, 5,411,975, 5,409,913, 5,409,925, 5,409,921, 5,411,961, 5,413,998, 5,411,954, 5,416,084, 5,409,945, 5,411,950, 5,409,924, 5,373,017, 5,252,591, 5,434,162, 5,563,154, 5, 298, 505, 5, 414, 018, 5,433,067, 5,432.201, and 5, 968, 984, for methods of preparing the compounds of the invention. The disclosure of each of these documents is incorporated herein by reference, in its entirety.

The present invention provides methods comprising compounds, compositions, and kits for inhibiting beta-secretase-mediated cleavage of amyloid precursor protein (APP). More particularly, the methods comprising compounds, compositions, and kits are effective to inhibit the production of A beta peptide and to treat or prevent any human or veterinary disease or condition associated with a pathological form of A beta peptide.

### Detailed Description of the Invention

The aforementioned U.S. Patents disclose various compounds of the formulas (I)-(X): 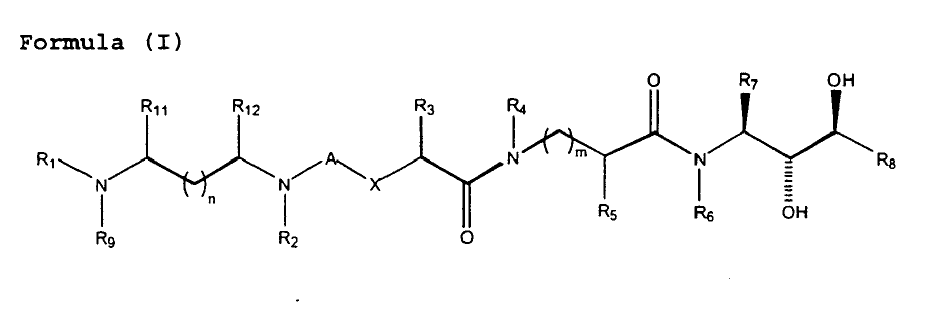 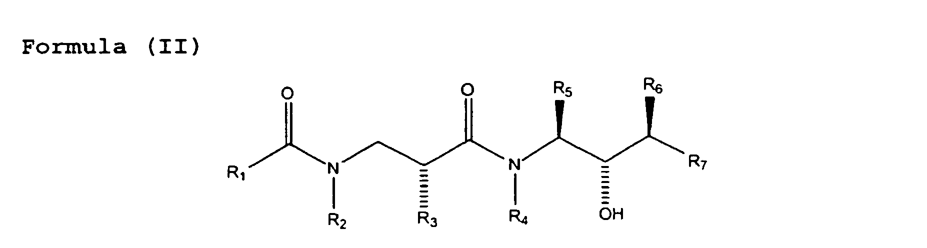 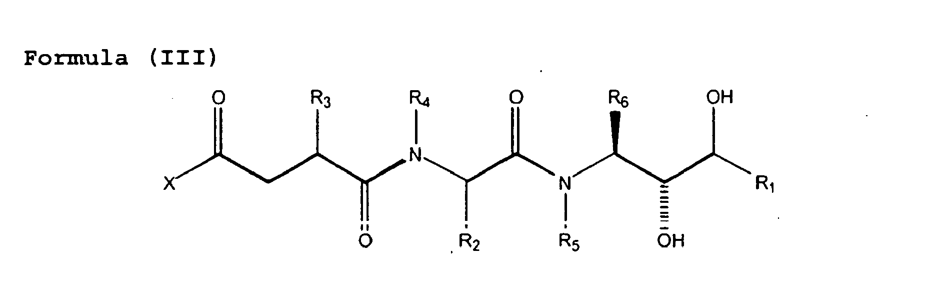 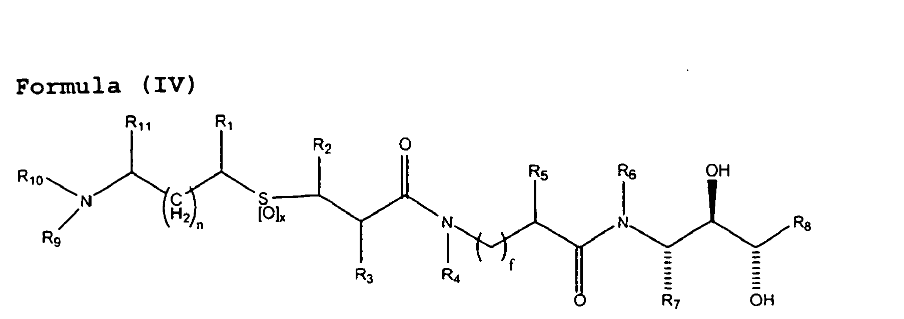 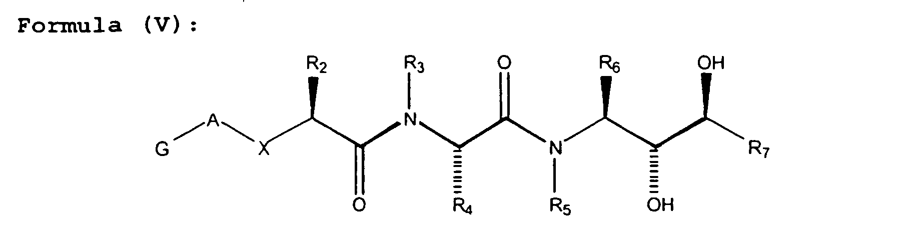 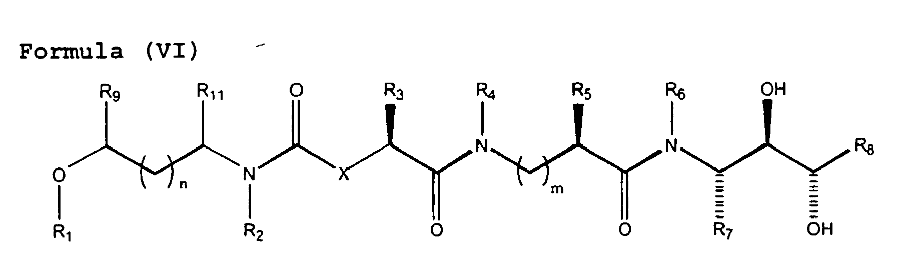 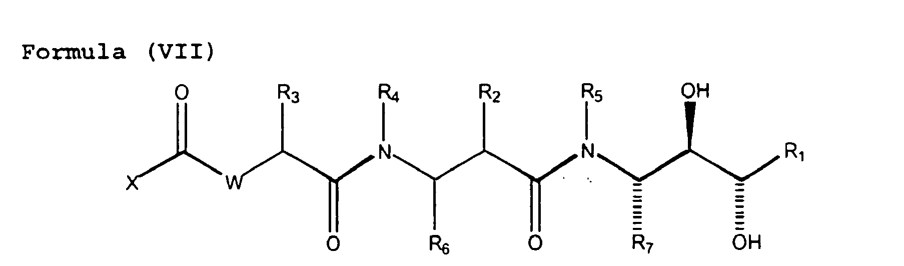 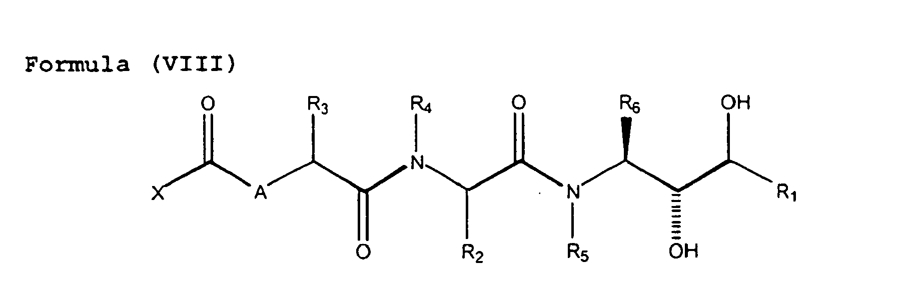 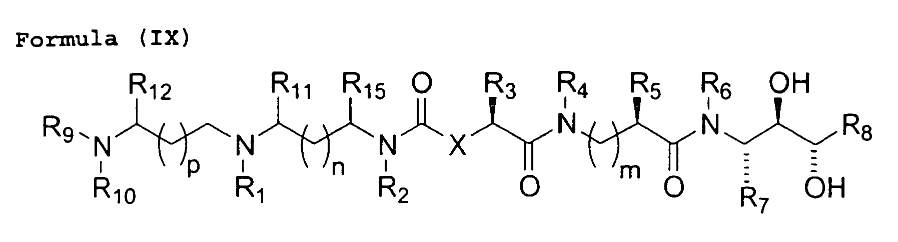 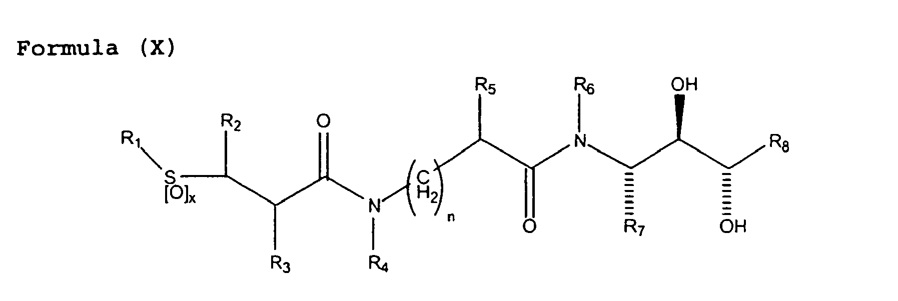 wherein the variable substituents and provisos for each of formulas (I)-(X) are as defined above, for each particular formula, and which are useful for the inhibition of the enzyme renin. These patents do not have any disclosure with regard to Alzheimer's disease.

The following U.S. Patents disclose how to make the above compounds and how to use them for the inhibition of the renin enzyme. U.S. Patent Nos. 5,420,127, 5,428,064, 5,416,111, 5,424,307, 5,424,319, 5,418,249, 5,416,104, 5,428,054, 5,416,092, 5,411,957, 5,416,085, 5,411,962, 5,411,958, 5,414,012, 5,416,095, 5,416,083, 4,977,141, 4,931,429, 5,089,471, 4,877,785, 4,900,746, 5,229,420, 4,902,706, 5,229,369, 4,900,745, 5,246,959, 5,175,170, 5,180,744, 5,175,181, 5,171,751, 5,210,095, 5,283,250, 5,180,725, 5,179,102, 5,212,175, 5,147,888, 5,217,991, 5,223,512, 5,231,111, 5,223,534, 5,223,532, 5,223,514, 5,212,174, 5,215,996, 5,217,989, 5,290,787, 5,217,988, 5,216,013, 5,227,401, 5,484,811, 5,942,548, 6,174,923, 5,223,535, 5,484,812, 5,330,996, 5,508,295, 5,849,773, 5,314,920, 5,317,039, 5,246,969, 5,461,074, 5,268,391, 5,312,838, 5,416,119, 5,488,066, 5,756,549, 5,212,185, 5,304,552, 5,385,898, 5,780,494, 5,422,349, 5,409,922, 5,411,953, 5,411,959, 5,411,975, 5,409,913, 5,409,925, 5,409,921, 5,411,961, 5,413,998, 5,411,954, 5,416,084, 5,409,945, 5,411,950, 5,409,924, 5,373,017, 5,252,591, 5,434,162, 5,563,154, 5,298,505, 5,414,018, 5,488,067, 5,432,201, and 5,968,984 are incorporated herein by reference, in their entirety.

In one aspect, the present invention relates to methods of treating a subject who has, or in preventing a subject from developing, a disease or condition selected from the group consisting of Alzheimer's disease, for helping prevent or delay the onset of Alzheimer's disease, for helping to slow the progression of Alzheimer's disease, for treating subjects with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease and who is in need of such treatment which comprises administration of a therapeutically effective amount of a compound of formula (I), or pharmaceutically acceptable salts thereof: 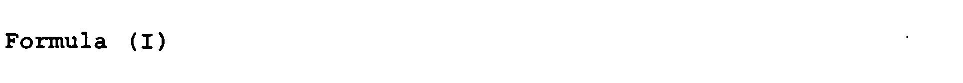 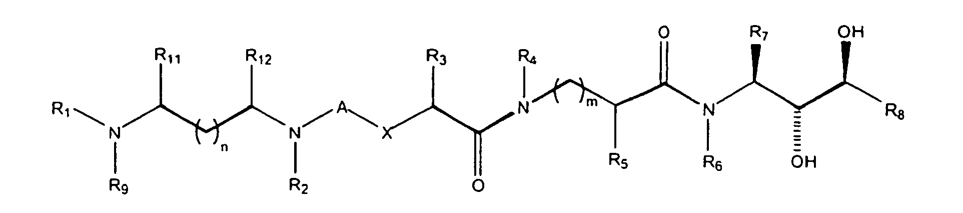 wherein A is selected from methylene, CO, SO, and SO₂;
wherein X is selected from oxygen atom, methylene and 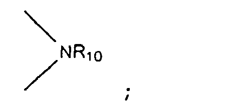 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl ;
R₅ is selected from 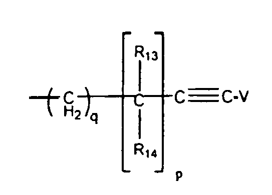 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5.

In an aspect, the methods of the invention comprise administration of a compound, or pharmaceutically acceptable salt thereof, of the formula (II): 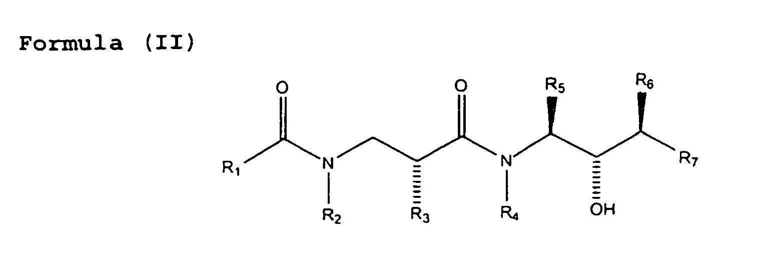 wherein R₁ is selected from aryl, aralkyl, heteroaryl and heteroaralkyl, including the following: 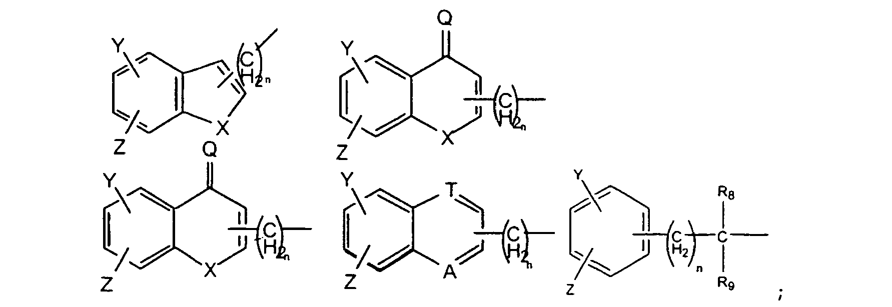 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected, from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano.

In an aspect, the methods of the invention comprise administration of a compound, or pharmaceutically acceptable salt thereof, of the formula (III): 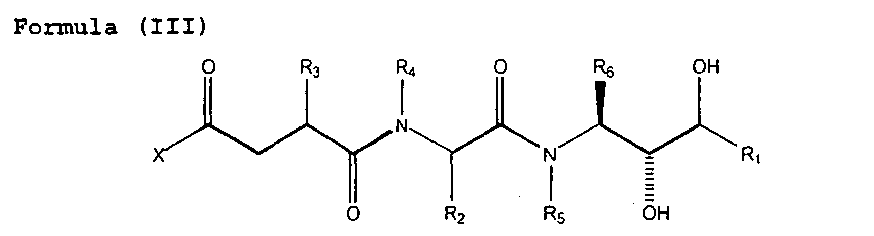 wherein X is selected from 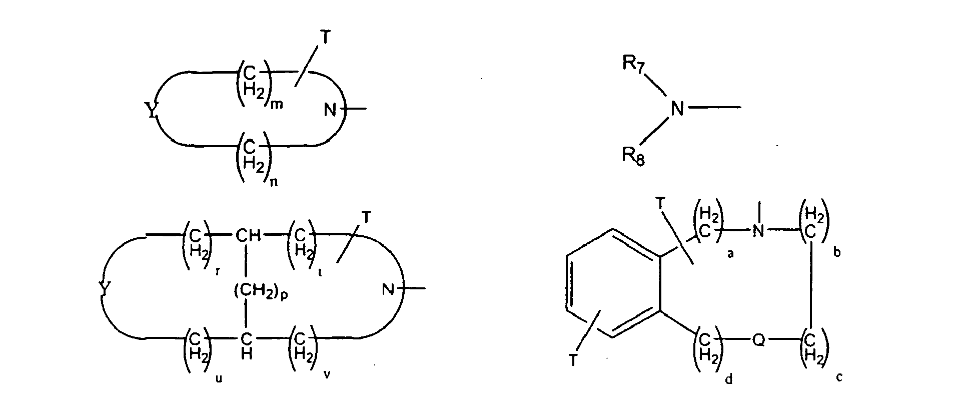 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group.

In an aspect, the methods of the invention comprise administration of a compound, or pharmaceutically acceptable salt thereof, of the formula (IV): 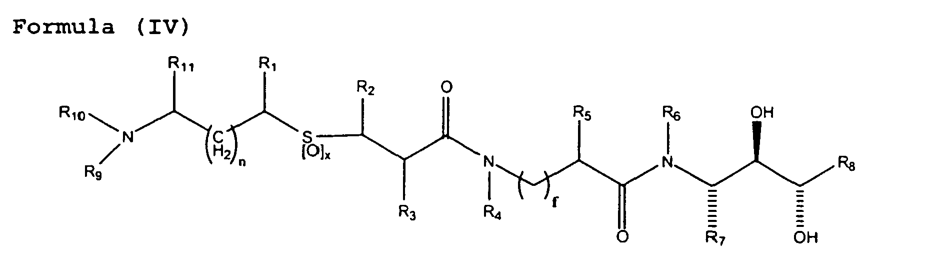 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl.

In an aspect, the methods of the invention comprise administration of a compound, or pharmaceutically acceptable salt thereof, of the formula (V): 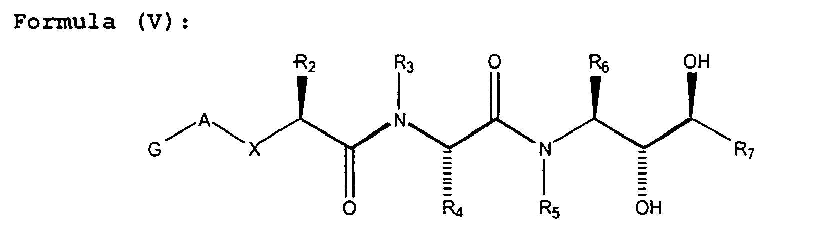 wherein A is selected from CO and SO₂;
X is selected form oxygen atom and methylene;
G is selected from B or 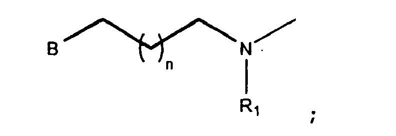 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 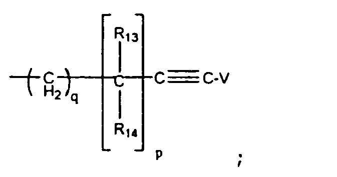 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl.

In an aspect, the methods of the invention comprise administration of a compound, or pharmaceutically acceptable salt thereof, of the formula (VI): 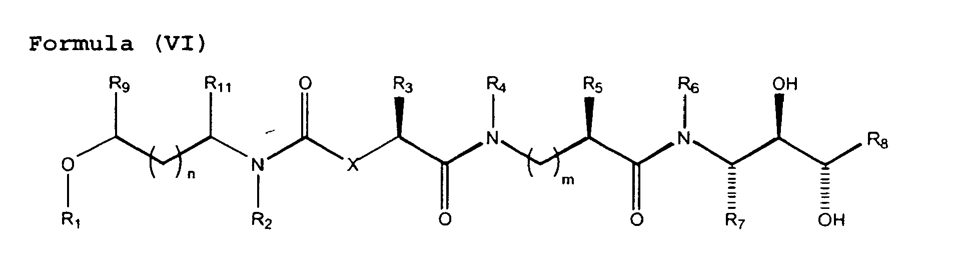 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl.

In an aspect, the methods of the invention comprise administration of a compound, or pharmaceutically acceptable salt thereof, of the formula (VII): 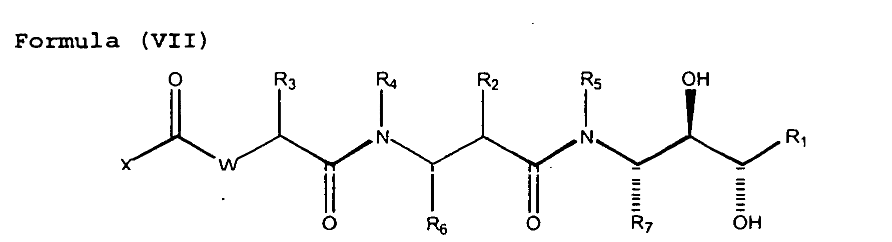 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 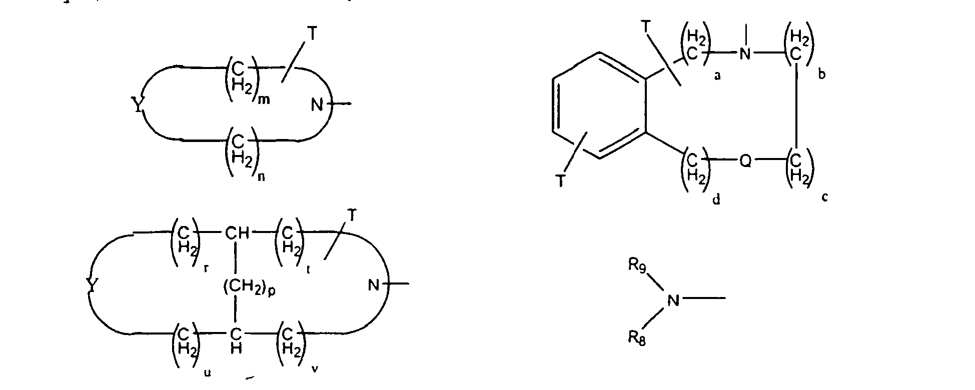 wherein Y and Q are selected from CH₂, CH-O-R₁₀, O, S, SO , SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group.

In an aspect, the methods of the invention comprise administration of a compound, or pharmaceutically acceptable salt thereof, of the formula (VIII): 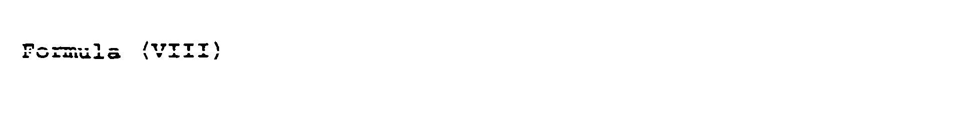 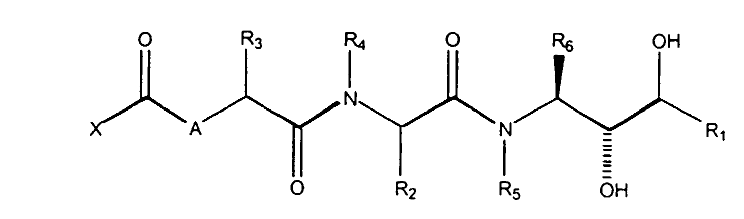 wherein X is selected from 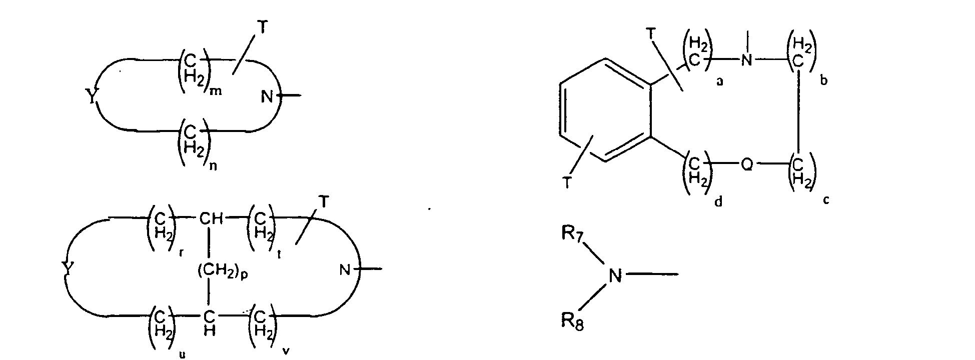 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from _alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group.

In an aspect, the methods of the invention comprise administration of a compound, or pharmaceutically acceptable salt thereof, of the formula (IX): 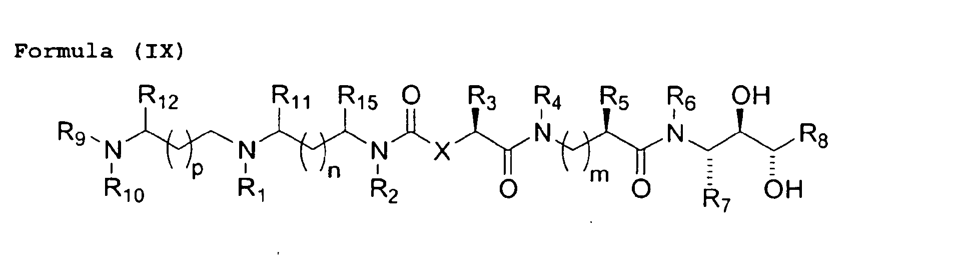 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH (OH) R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl.

In an aspect, the methods of the invention comprise administration of a compound, or pharmaceutically acceptable salt thereof, of the formula (I)-(X).

In certain embodiments of the above aspects, the invention provides methods comprising administration of a compound, or pharmaceutically acceptable salt thereof, or composition comprising a compound, selected from the group consisting of representative compounds of Formulas (I)-(X):
Representative compounds of Formula (I):
   0-{N-[2-(N,N-dimethylamino)ethyl]-N-methylaminocarbonyl}-3-L-phenyllactyl-L-histidine amide of (2S,3R,4S)-2-amino-1-cyclohexyl-3,4-dihydroxy-6-methylheptane;
   O-{N-[2-(N-methylamino)ethyl]-N-methylaminocarbonyl}-3-L-phenyllactyl-L-histidine amide of (2S,3R,4S)-2-amino-1-cyclohexyl-3,4-dihydroxy-6-methylheptane;
   O-{N-[2-(N-methylamino)ethyl]-N-methylaminocarbonyl}-3-L-phenyllactyl-L-leucine amide of (2S,3R,4S)-2-amino-1-cyclohexyl-3,4-dihydroxy-6-methylheptane;
   O-{N-[2-(N,N-dimethylamino)ethyl]-N-methylaminocarbonyl}-3-L-phenyllactyl-L-leucine amide of (2S,3R,4S)-2-amino-1-cyclohexyl-3,4-dihydroxy-6-methylheptane;
   O-{N-[2-(N,N-dimethylamino)ethyl]-N-methylaminocarbonyl}-3-L-phenyllactyl-α-(R)-methyl-β-alanine amide of (2S,3R,4S)-2-amino-1-cyclohexyl-3,4-dihydroxy-6-methylheptane;
   O-{N-[2-(N,N-dimethylamino)ethyl]-N-methylaminocarbonyl}-3-L-homophenyllactyl-α-(R)-methyl-β-alanine amide of (2S,3R,4S)-2-amino-1-cyclohexyl-3,4-dihydroxy-6-methylheptane;
   O-N-[2-(N,N-dimethylamino)ethyl]-N-methylaminocarbonyl}-3-L-homophenyllactyl-α-(R)-ethyl-β-alanine amide of (2S,3R,4S)-2-amino-1-cyclohexyl-3,4-dihydroxy-6-methylheptane;
   3-N-[2-(N,N-dimethylamino)ethyl]-N-methylaminocarbonyl}-2-(R)-(2-phenylethyl)-propionyl-α-(R)-ethyl-β-alanine amide of (2S,3R,4S)-2-amino-1-cyclohexyl-3,4-dihydroxy-6-methylheptane;
   3-{N-[2-(N-piperdino)ethyl]-N-methylaminocarbonyl}-3-L-phenyllactyl-L-histidine amide of (2S,3R,4S)-2-amino-1-cyclohexyl-3,4-dihydroxy-6-methylheptane;
   3-N-[2-(N-piperdino)ethyl]-N-methylaminocarbonyl}-2-(R) - (2-phenylethyl) -propionyl-α- (R) -ethyl-β-alanine amide of (2S,3R,4S)-2-amino-1-cyclohexyl-3,4-dihydroxy-6-methylheptane;
   O-{N-[2-N,N-dimethylamino)ethyl]-N-methylaminocarbonyl}-3-L-phenyllactyl-L-histindine amide of (2S,3R,4S)-2-amino-1-cyclohexyl-3,4-dihydroxy-6-methylheptane;
   O-(N-(N-methyl-N-Boc-aminoethyl)-N-methylaminocarbonyl)-3-L-phenyllactyl-L-(im-tosyl)-histidine amide of (2S,3R,4S)-2-amino-1-cyclohexyl-3,4-dihydroxy-6-methylheptane;
   N-Boc-α-(R)-methyl-β-alanine amide of (2S,3R,4S)-2-amino-1-cyclohexyl-3,4-dihydroxy-6-methylheptane;
   N1-[1R*-[[[1S,1R*-(cyclohexylmethyl)-2S*,3R*-dihydroxyhexynyl]amino]carbonyl]-3-butynyl]-N4-[2-(dimethylamino)ethyl]-N4-methyl-2S*-(phenylmethyl)butanediamide;
   [1R*-[[[1R*-[[[1S,1R*-(cyclohexylmethyl)-2S*,3R*-dihydroxyhexynyl]amino]carbonyl]-3-butynyl]amino]carbonyl]-2-phenylethyl)[2-(dimethylamino)ethyl]methylcarbamate;
   N1-[1R*-[[[1S,1R*-(cyclohexylmethyl)-2S*,3R*-dihydroxy-5-methylhexyl]amino]carbonyl]-3-butynyl]-N4-[2-(dimethylamino)ethyl]-N4-[2-(dimethylamino)ethyl]-N4-methyl-2S*-(phenylmethyl)butanediamide;
   [1R*- [[[1R*- [[[1S,1R*- (cyclohexylmethyl) -2S*, 3R*-dihydroxy-5-methylhexyl]amino]carbonyl]-3-butynyl] amino] carbonyl] -2-phenylethyl) [2-(dimethylamino)ethyl]methylcarbamate;
   [1R*-[[[1-[[[1S,1R*-(cyclohexylmethyl)-2S*,3R*-dihydroxy-5-methylhexyl]amino]carbonyl]-3-butynyl]amino]carbonyl]-2-phenylethyl) [2-(dimethylamino) ethyl] methylcarbamate;
   [1R*-[[[1-[[[1S,1R*-(cyclohexylmethyl)-2S*,3R*-dihydroxy-5-methylhexyl]amino]carbonyl]-3-butynyl]amino]carbonyl]-2-phenylethyl][2-(dimethylamino)ethyl]methylcarbamate;
   {N-[2-(N,N-dimethylamino)ethyl]-N-methylaminocarbonyl}-3-L-phenyllactyl-α-(R)-methyl-β-alanine amide of (2S,3R,4S)-2-amino-1-cyclohexyl-3,4-dihydroxy-6-methylheptane;
   3-{N-[4-(N-methyl-N-boc-amino)butyl-N-methylaminocarbonyl}-2-(R)-phenethylpropionyl-α-(R)-methyl-β-alanine amide of (2S,3R,4S)-2-amino-1-cyclohexyl-3,4-dihydroxy-6-methylheptane;
   O-{N-[2-(N,N-dimethylamino)ethyl]-N-methylaminocarbonyl}-3-L-benzyllactyl-α-(R)-methyl-β-alanine amide of (2S,3R,4S)-2-amino-1-cyclohexyl-3,4-dihydroxy-6-methylheptane;
   O-{N-[2-(N,N-dimethylamino)ethyl]-N-methylaminocarbonyl}-3-L-benzyllactyl-α-(R)-ethyl-β-alanine amide of (2S,3R,4S)-2-amino-1-cyclohexyl-3,4-dihydroxy-6-methylheptane;
   O-{N-[2-(N-piperidino)ethyl]-N-methylaminocarbonyl}-3-L-phenyllactyl-L-histidine amide of (2S,3R,4S)-2-amino-1-cyclohexyl-3,4-dihydroxy-6-methylheptane;
   O-{N-[2-(N-piperidino)ethyl]-N-methylaminocarbonyl}-2-(R)-(2-phenylethyl)-propionyl-α-(R)-ethyl-α-alanine amide of (2S,3R,4S)-2-amino-1-cyclohexyl-3,4-dihydroxy-6-methylheptane;
   3-{N-[2-(N,N-dimethylamino)ethyl-N-methylaminocarbonyl}-2-R-benzyl-propionyl-α-(R)-methyl-β-alanine amide of (2S,3R,4S)-2-amino-1-cyclohexyl-3,4-dihydroxy-6-methylheptane;
   O-{N-[2-(N,N-dimethylamino)ethyl]-N-isopropylaminocarbonyl}-3-L-phenyllactyl-L-leucine amide of (2S,3R,4S)-2-amino-1-cyclohexyl-3,4-dihydroxy-6-methylheptane;
   3-{N-[4-(N-met:hylamino)but:yl]-N-met:hylaminocarbonyl}-2-R-phenethyl-propionyl-α-(R)-methyl-β-alanine amide of (2S,3R,4S)-2-amino-1-cyclohexyl-3,4-dihydroxy-6-methylheptane;
   O-{N-[2-N-methyl-N-boc-amino)ethyl)butyl]-N-methylaminocarbonyl}-3-L-phenyllactyl-L-histidine amide of (2S,3R,4S)-2-amino-1-cyclohexyl-3,4-dihydroxy-6-methylheptane;
   O-{N-[2-(N-methyl-N-boc-amino)ethyl]-N-methylaminocarbonyl}-3-L-phenyllactyl-α-(R)-methyl-β-alanine amide of (2S,3R,4S)-2-amino-1-cyclohexyl-3,4-dihydroxy-6-methylheptane;
   O-{N-[2-(N-methylamino)ethyl] -N-methylaminocarbonyl}-3-L-phenyllactyl-α-(R)-methyl-β-alanine amide of (2S,3R,4S)-2-amino-1-cyclohexyl-3,4-dihydroxy-6-methylheptane;
   3{N-[2-(N-methylamino)ethyl]-N-methylaminocarbonyl}-2-R-phenethylpropionyl-α-(R)-methyl-β-alanine amide of (2S,3R,4S)-2-amino-1-cyclohexyl-3,4-dihydroxy-6-methylheptane trifluoroacetate salt;
   Boc-(im-Tosyl)-L-histidine amide of (2S,3R,4S)-2-amino-1-cyclohexyl-3,4-dihydroxy-6-methylheptane;
   (im-Tosyl)-L-histidine amide of (2S,3R,4S)-2-amino-1-cyclohexyl-3,4-dihydroxy-6-methyheptane;
   O-{N-[2-(N,N-dimethylamino)ethyl]-N-methylaminocarbonyl}-3-L-phenyllactyl-L-histidine amide of (2S,3R,4S)-2-amino-1-cyclohexyl-3,4-dihydroxy-6-methylheptane;
   O-(N-(N-methyl-N-Boc-aminoethyl)-N-methylaminocarbonyl)-3-L-phenyllactic acid;
   O-(N-(dimethylaminoethyl)-N-methyl-aminocarbonyl)-3-L-phenyllactic acid;
   0-{N-2-(N,N-dimethylamino)ethyl]-N-methylaminocarbonyl}-3-L-phenyllactyl-L-leucine amide of (2S,3R,4S)-2-amino-1-cyclohexyl-3,4-dihydroxy-6-methylheptane;
   O-{N-[2-(N,N-dimethylamino)ethyl]-N-methylaminocarbonyl-3-L-phenyllactyl-α-(R)-methyl-β-alanine amide of (2S,3R,4S)-2-amino-1-cyclohexyl-3,4-dihydroxy-6-methylheptane;
   O-{N-[2-(N-methyl-N-Boc-amino)ethyl]-N-methylaminocarbonyl-3-L-phenyllactyl-L-histidine amide of (2S, 3R, 4S)-2-amino-1-cyclohexyl-3,4-dihydroxy-6-methylheptane;
   O-{N-[N- [2-(N-methyl-N-Boc-amino)ethyl]-N-methylaminocarbonyl}-3-L-phenyllactyl-α-(R)-methyl-β-alanine amide of (2S, 3R, 4S)-2-amino-1-cyclohexyl-3,4-dihydroxy-6-methylheptane;
   O-{N-2-(N,N-dimethylamino)ethyl]-N-isopropylaminocarbonyl-3-L-phenyl lactic acid;
   3-{N-2-(N,N-dimethylamino)ethyl]-N-methylaminocarbonyl}-2-R-benzylpropionic acid hydrochloride salt;
   3-{N-[2-(N,N-dimethylamino)ethyl]-N-methylaminocarbonyl}-2-R-benzylpropionyl-histidine amide of (2S,3R,4S)-2-amino-1-cyclohexyl-3,4-dihydroxy-6-methylheptane;
   O-{N-[2-(N,N-dimethylamino)-1-(R,S)-methylethyl]-N-methylaminocarbonyl}-3-L-homophenyllactyl-α-(R)-methyl-β-alanine amide of (2S,3R,4S)-2-amino-1-cyclohexyl-3,4-dihydroxy-6-methylheptane;
   O-{N-[2-(N,N-dimethylamino)ethyl]-N-methylaminocarbonyl}-3-L-homophenyllactyl-α-(R)-ethyl-β-alanine amide of (2S,3R,4S)-2-amino-1-cyclohexyl-3,4-dihydroxy-6-methylheptane;
   3-{N-[2-(N-methyl-N-2-(4-imidazole)ethylamino)ethyl]-N-methylaminocarbonyl}-2-R-benzylpropionyl-histidine amide of (2S, 3R, 4S)-2-amino-1-cyclohexyl-3,4-dihydroxy-6-methylheptane;
   N1-[2-[[1S, 1R*-(cyclohexylmethyl-2S*, 3R*-dihydroxy-5-hexynyl]amino]-2-oxo-1R*-(4-thiazolylmethyl)ethyl]-N4-[2-(dimethylamino)ethyl]-N4-methyl-2S*-(phenylmethyl)butanediamide;
   [[[2-[[1S,1R*-(cyclohexylmethyl)-2S*,3R*-dihydroxy-5-hexynyl]amino]-2-oxo-1R*-(4-thiazolylmethyl)ethyl]amino]-2-oxo-1R*-(phenylmethyl)ethyl][2-(dimethylamino)ethyl]methylcarbamate;
   N1-[1R*-[[[1S,1R*-(cyclohexylmethyl)-2S*,3R*-dihydroxy-5-methylhexyl]amino]carbonyl]-3-butynyl]-N4-[2-(dimethyl-amino)ethyl]-N4-methyl-2S*-(phenylmethyl)butanediamide;
   [1R*-[[[1R*-[[[1S,1R*-(cyclohexylmethyl)-2S*,3R*-dihydroxy-5-methylhexyl]amino]carbonyl]-3-butynyl]amino]carbonyl]-2-phenylethyl)[2-dimethylamino)ethyl]methylcarbamate; 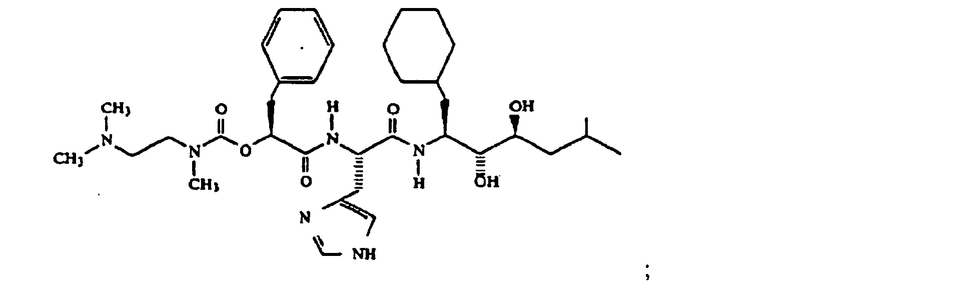 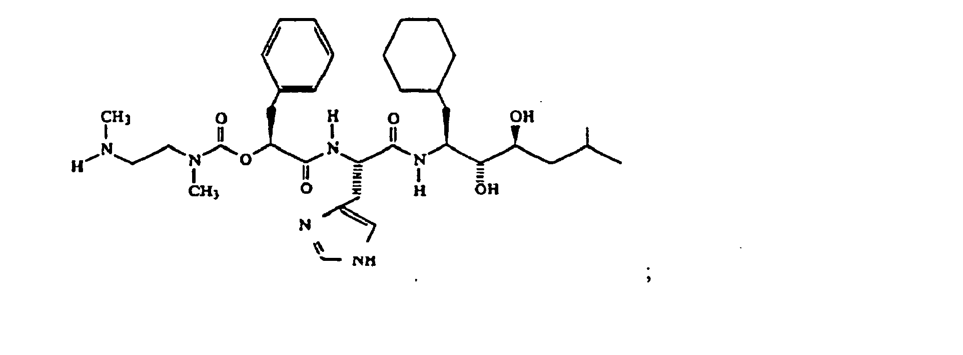 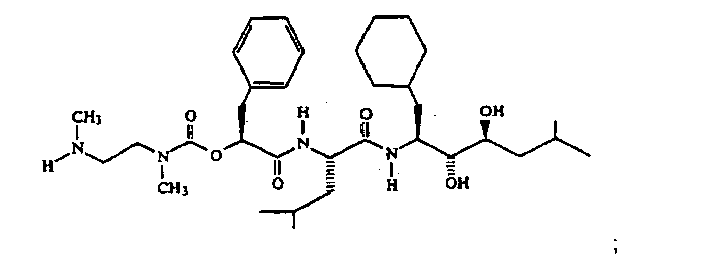 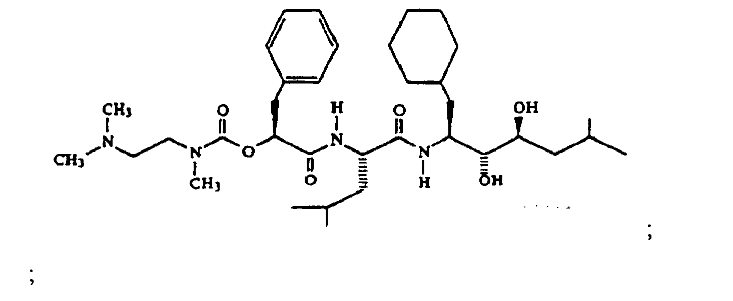 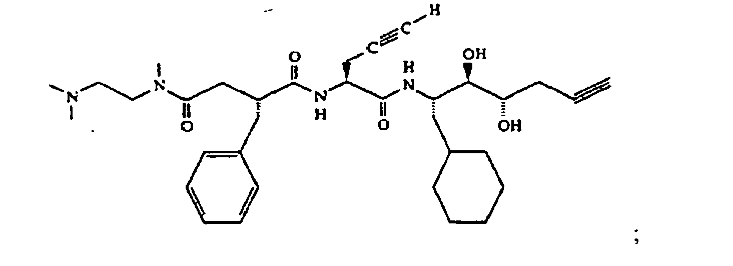 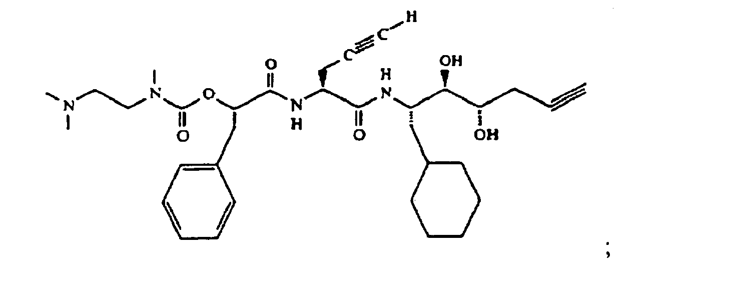 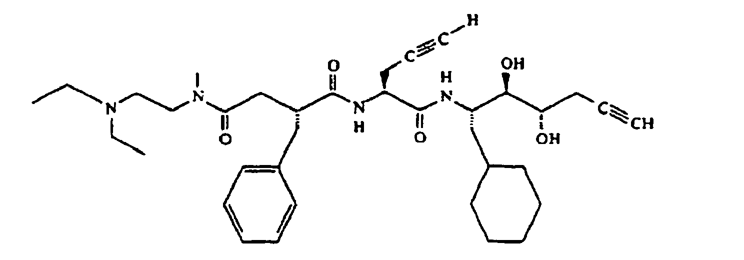 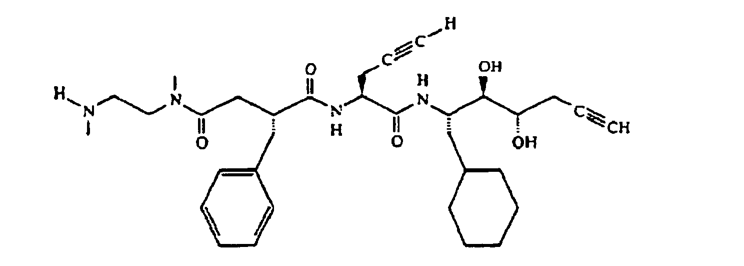 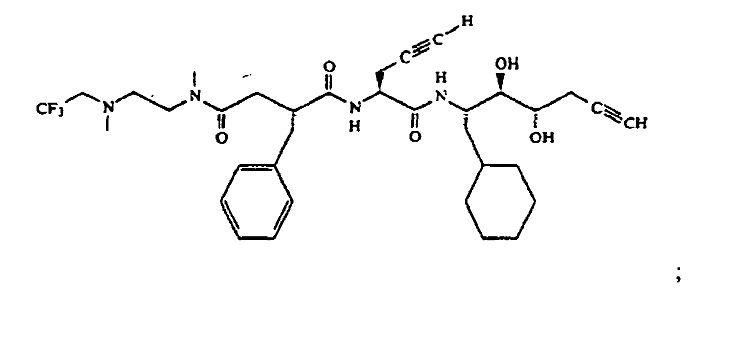 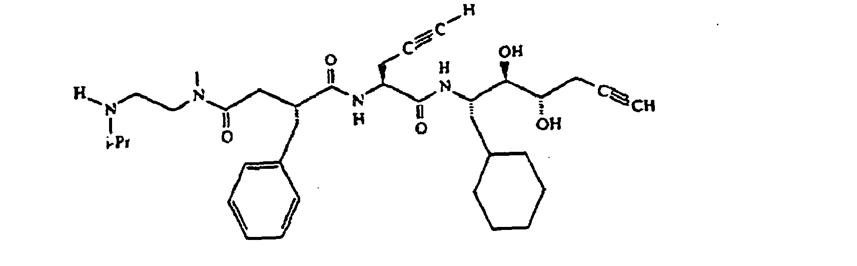 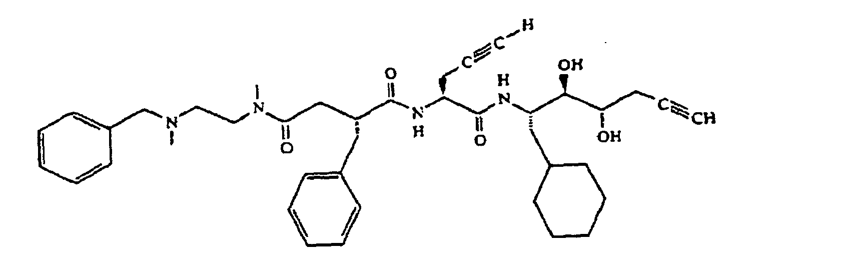 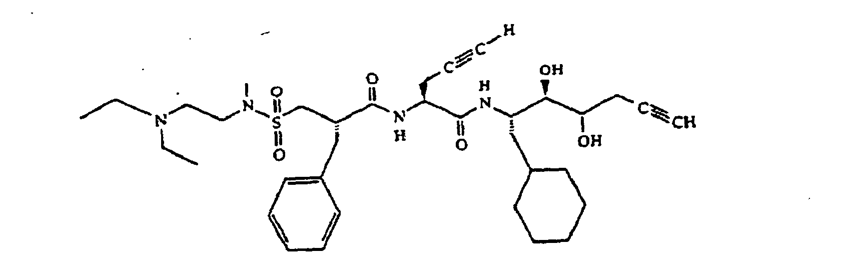 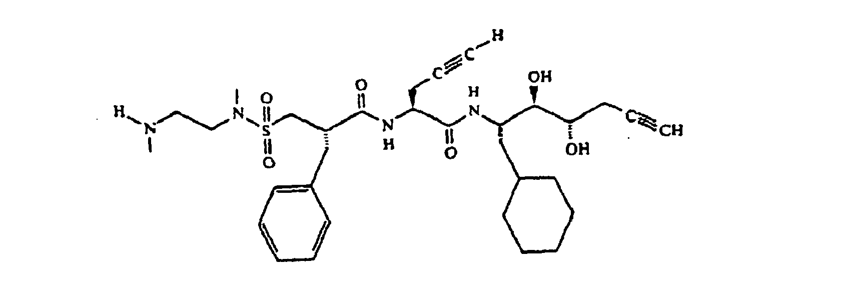 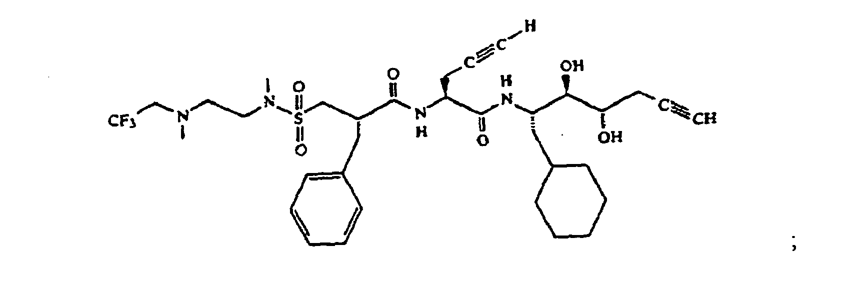 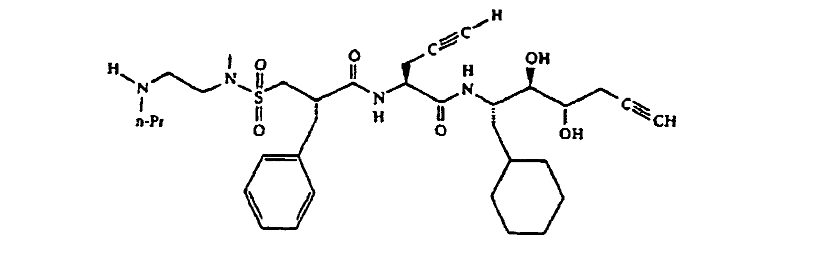 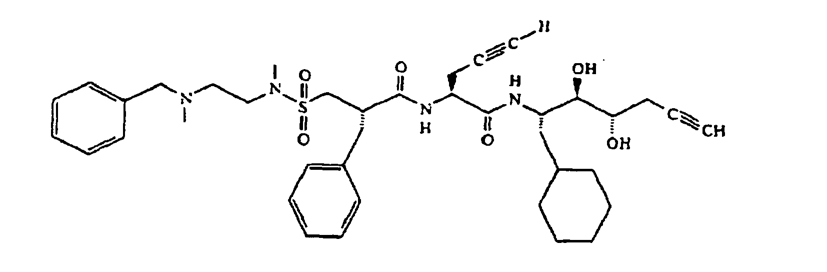 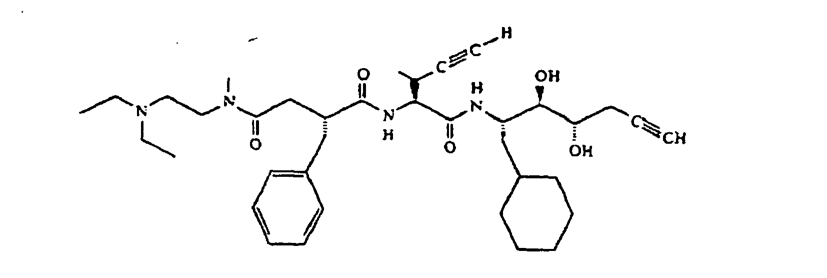 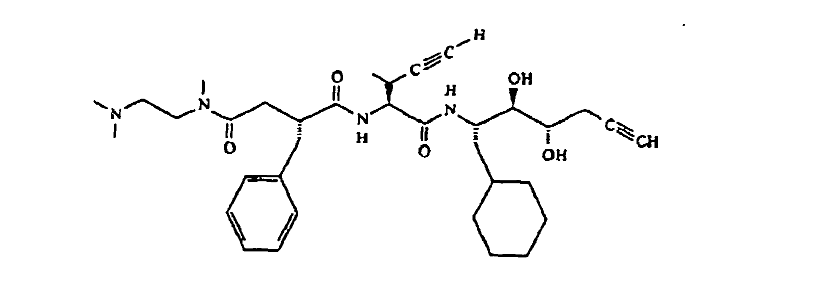 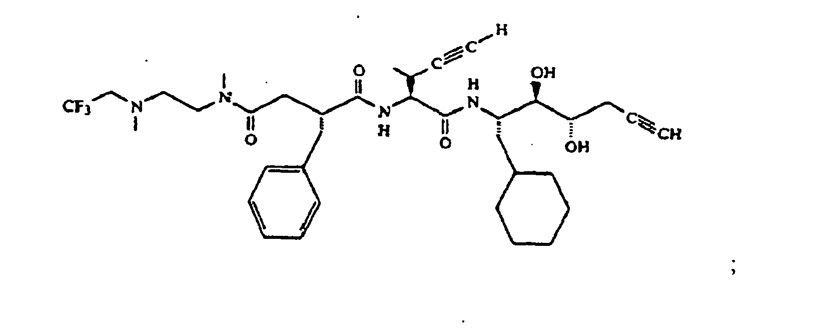 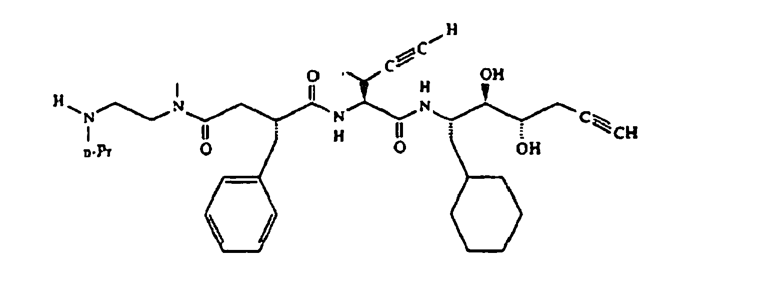 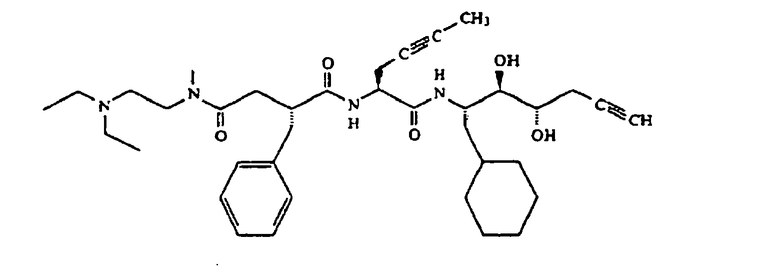 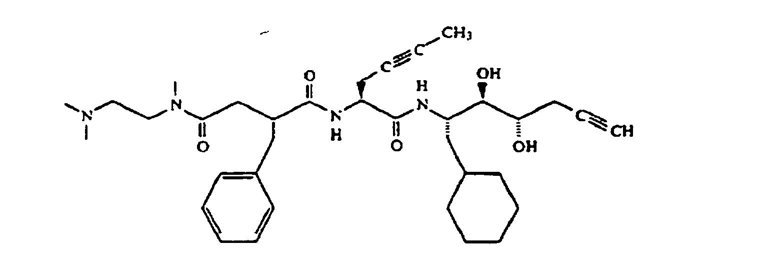 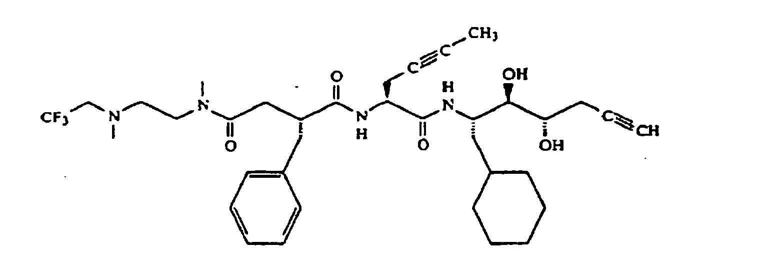 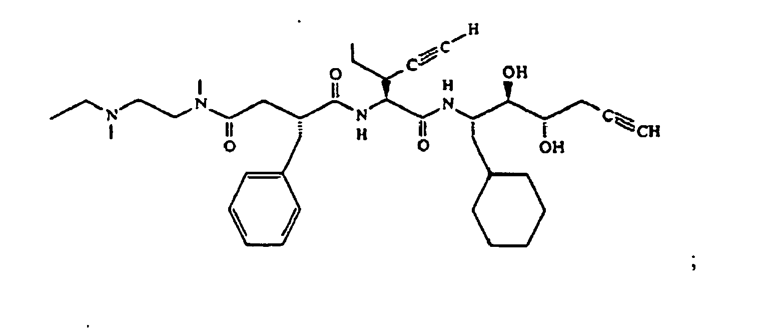 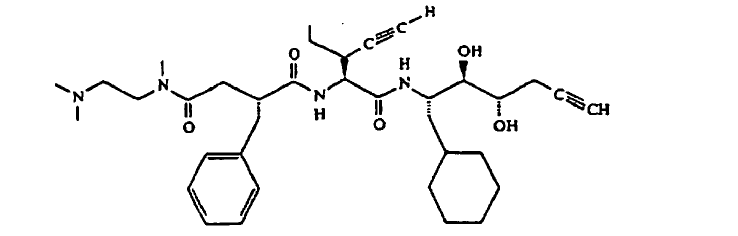 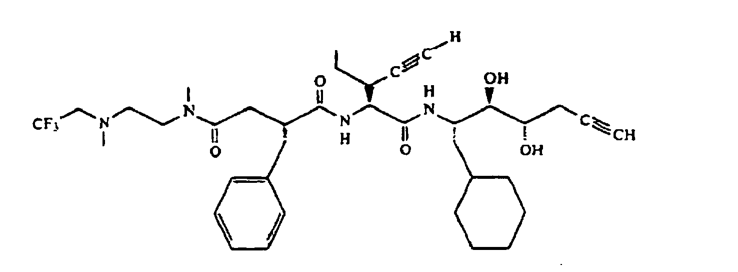 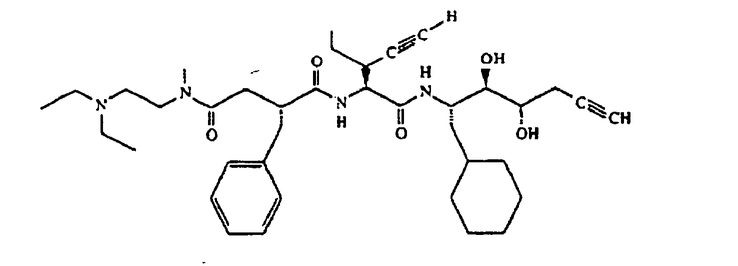 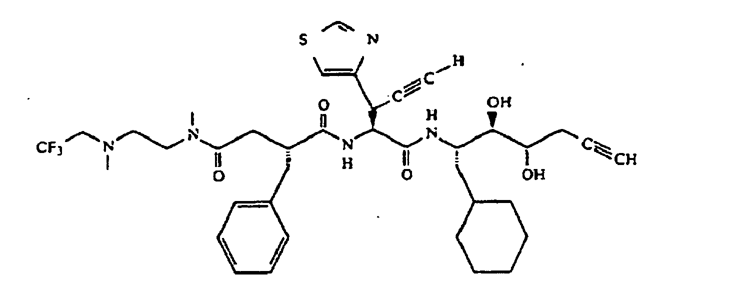 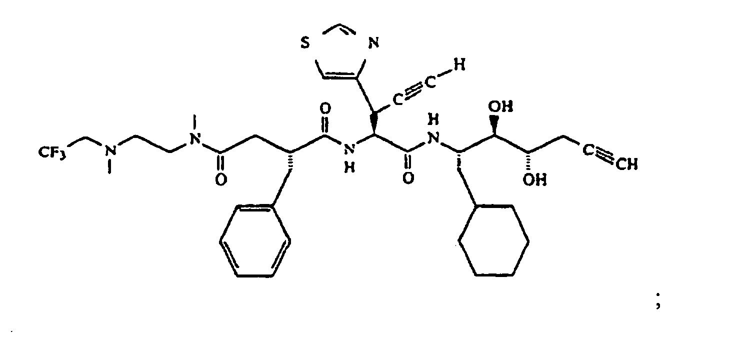 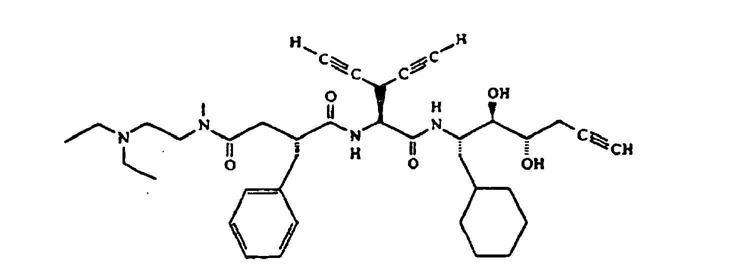 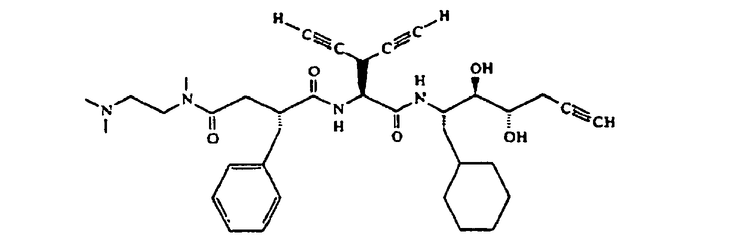 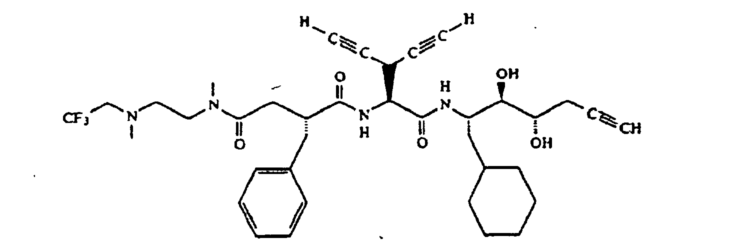 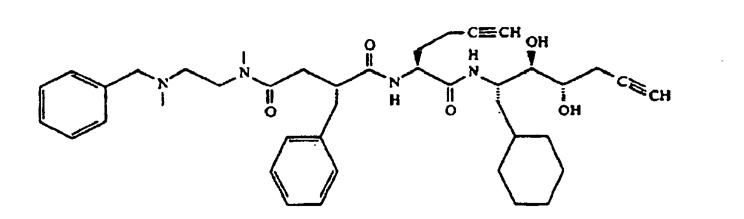 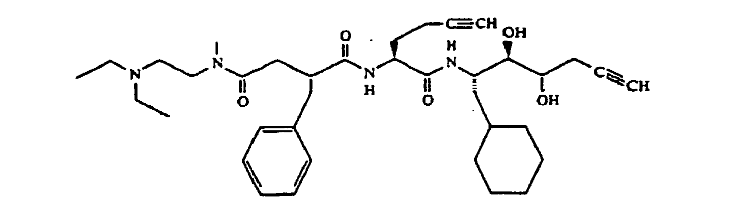 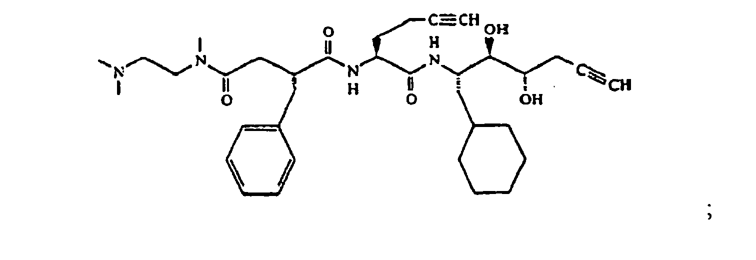 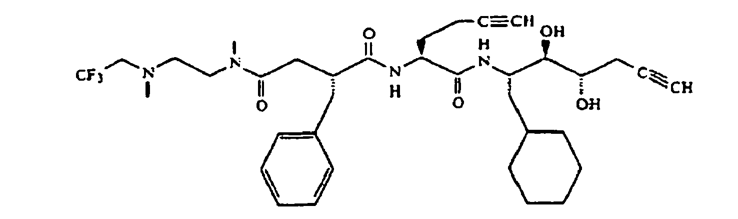 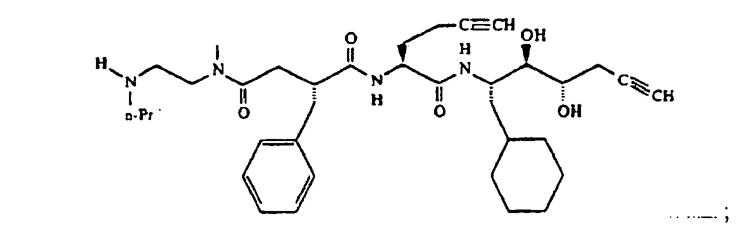 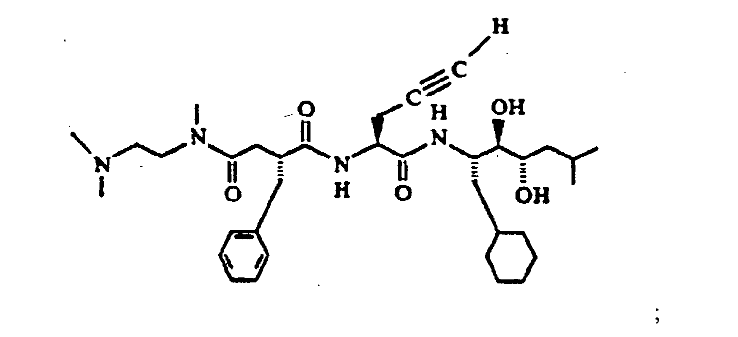 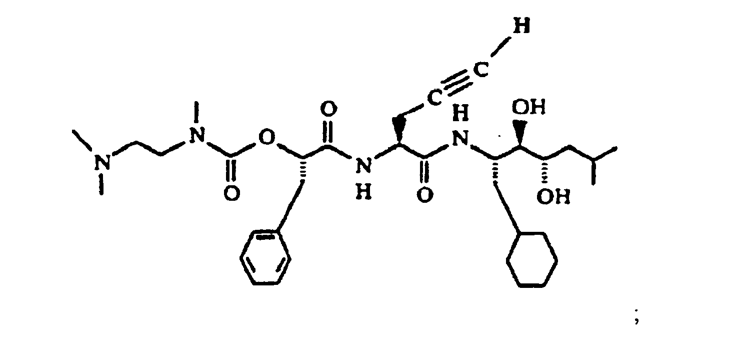 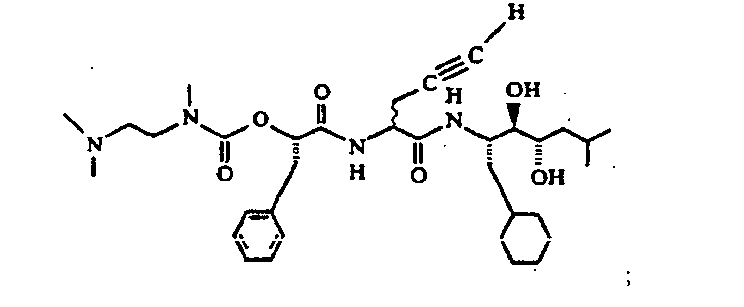 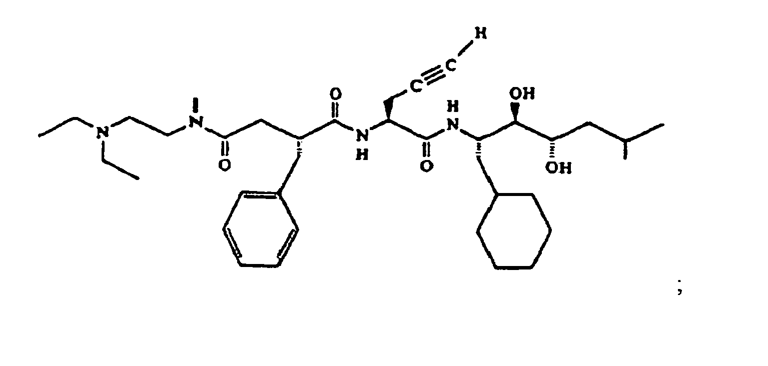 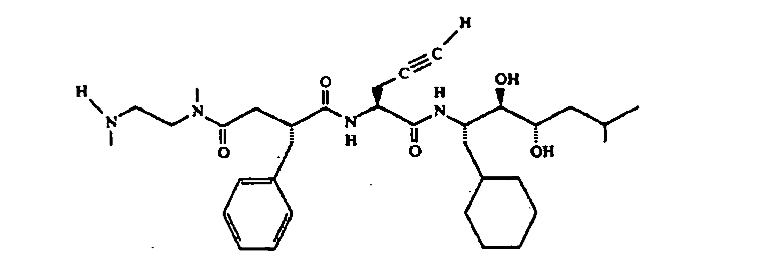 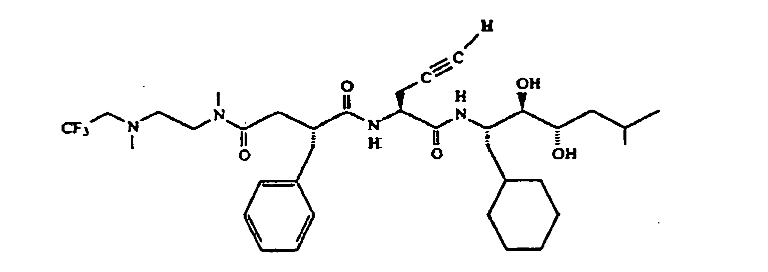 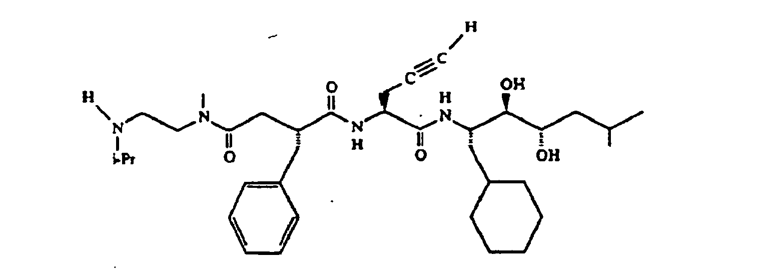 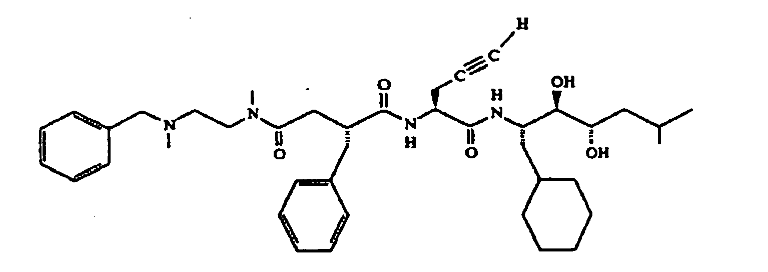 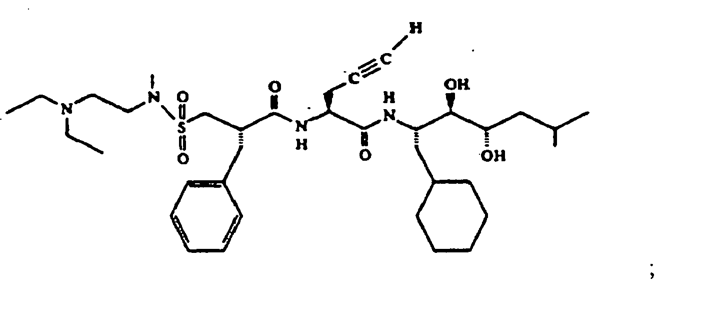 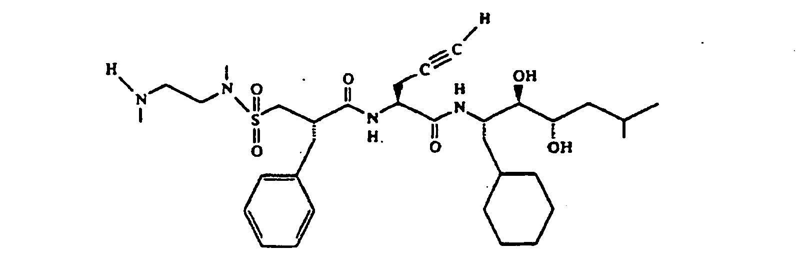 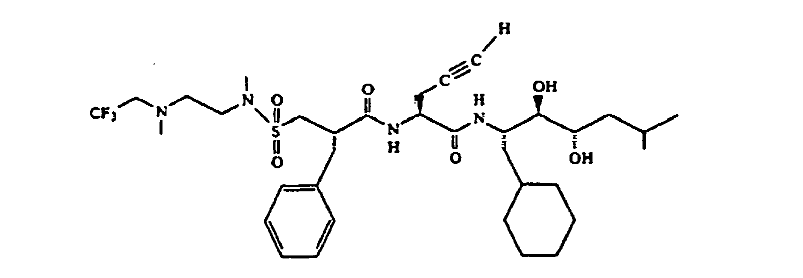 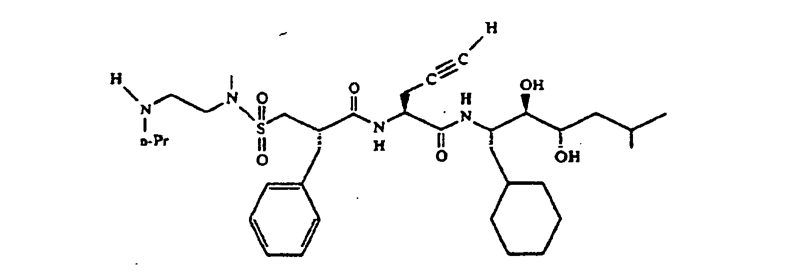 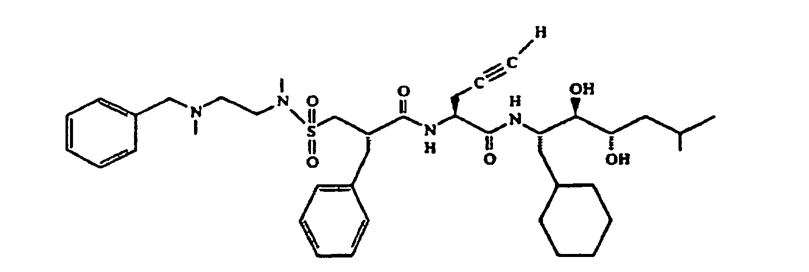 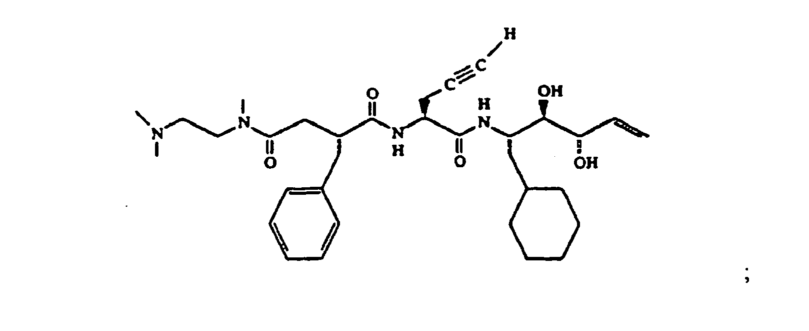 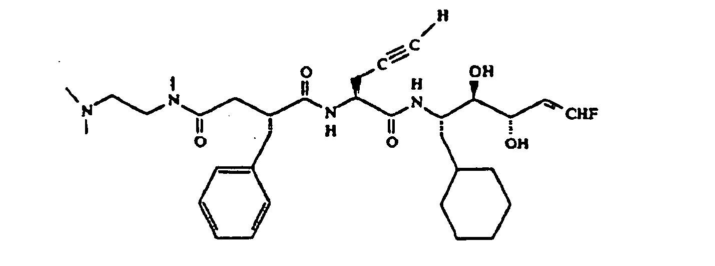 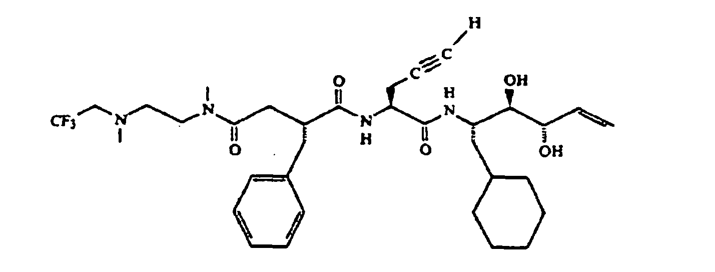 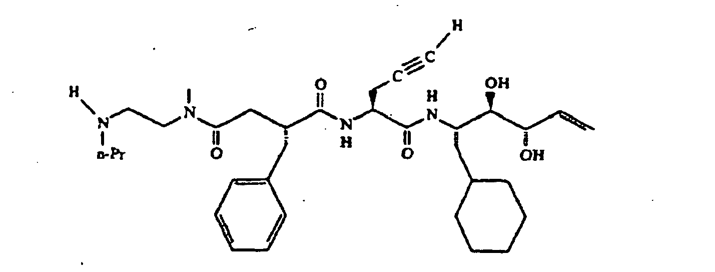 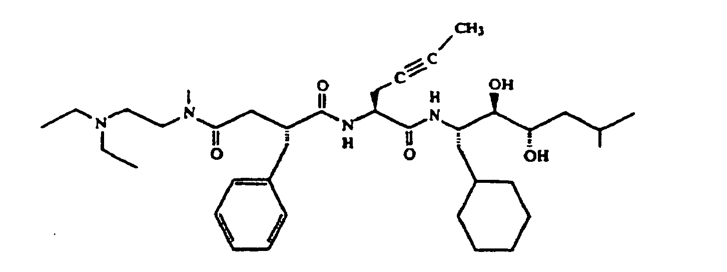 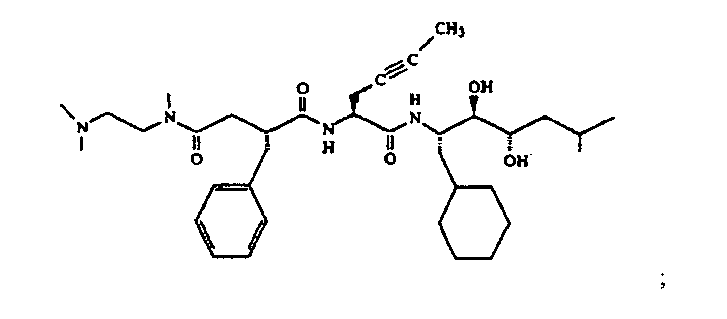 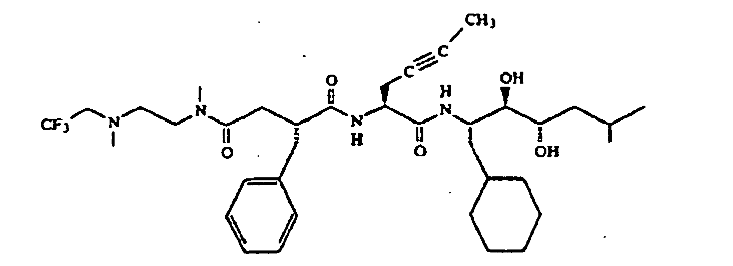 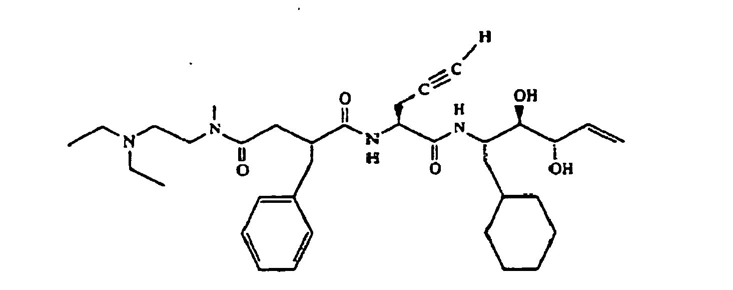 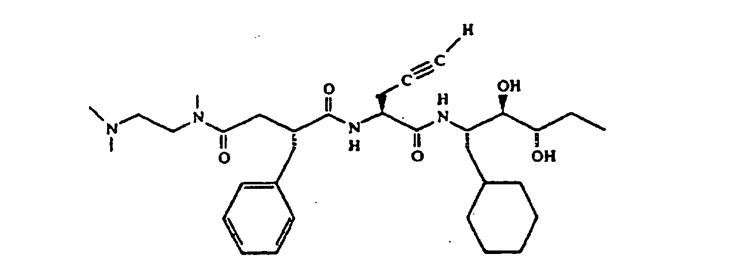 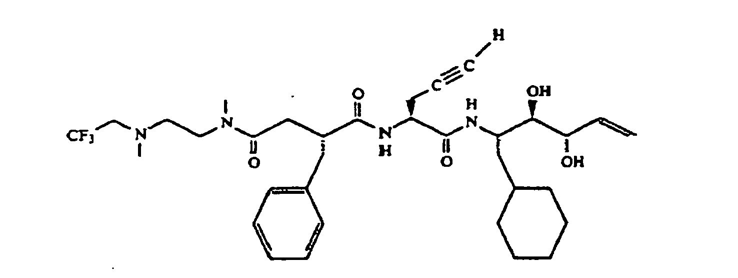 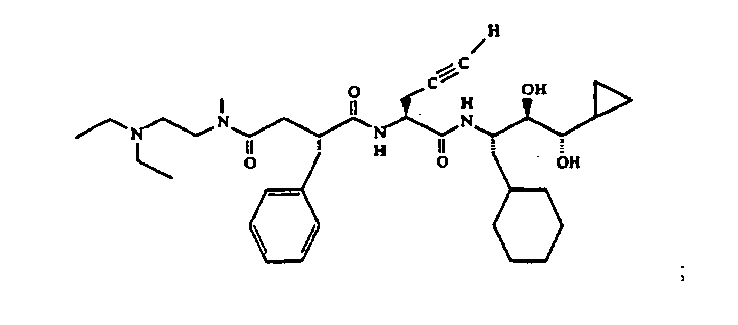 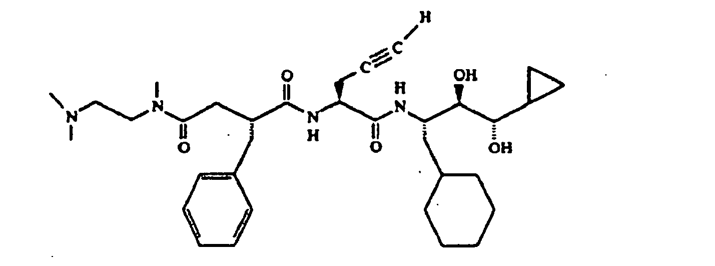 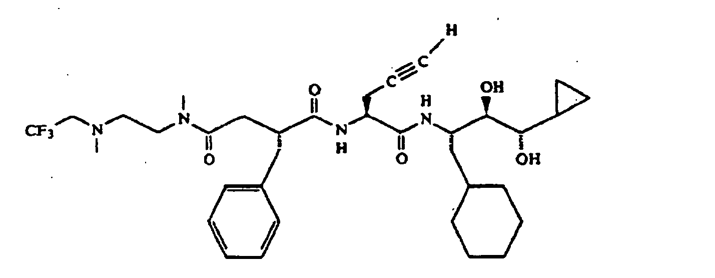 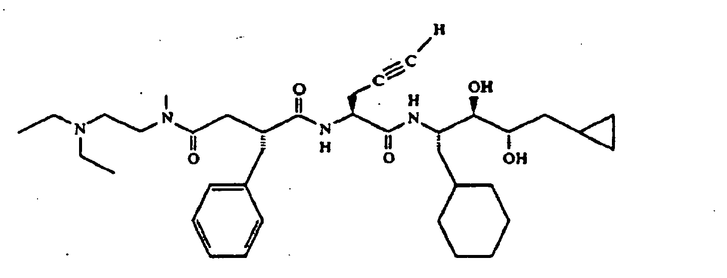 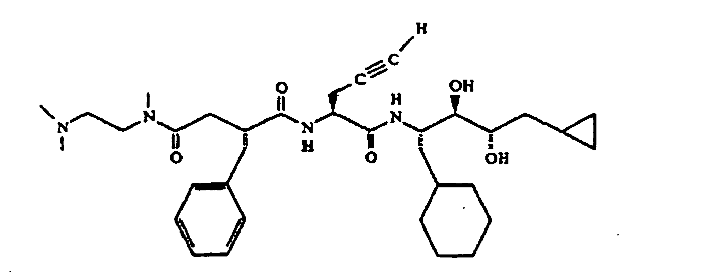 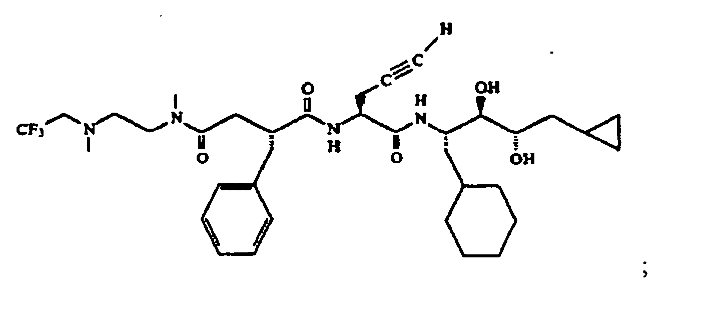 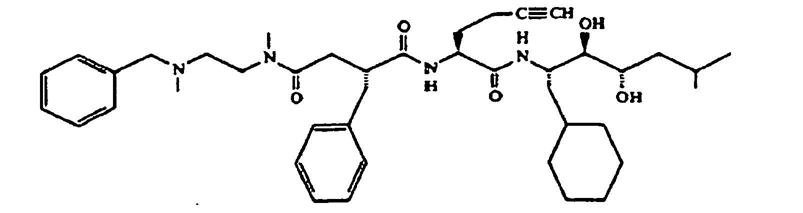 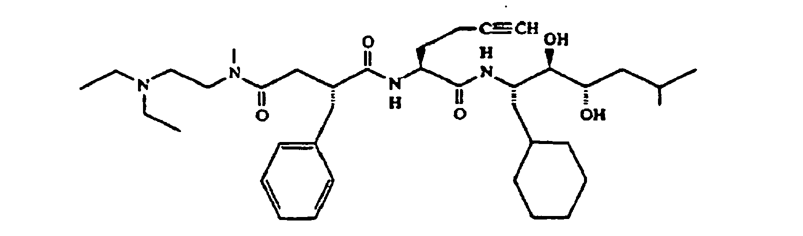 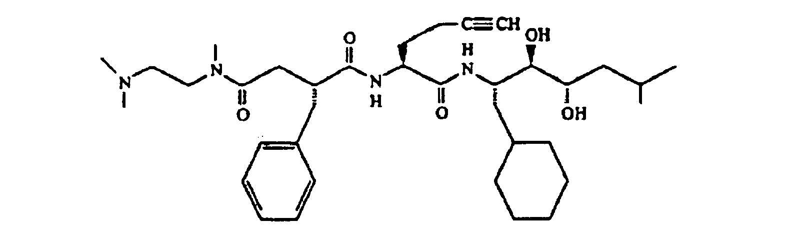 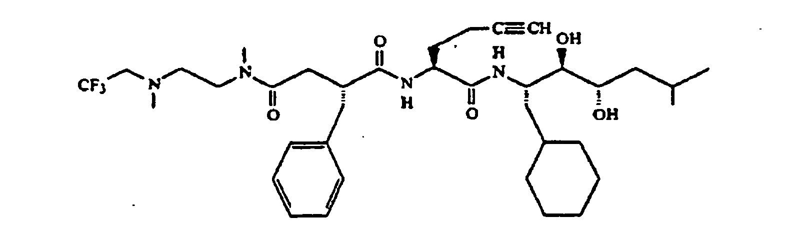 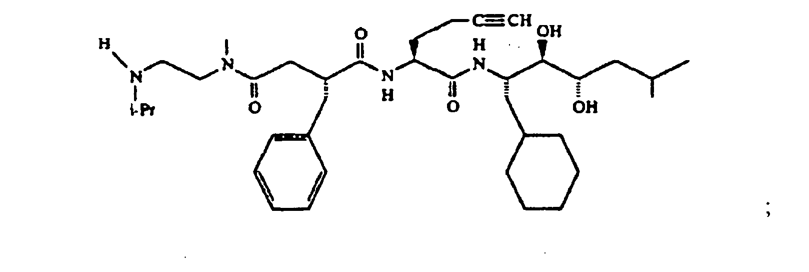 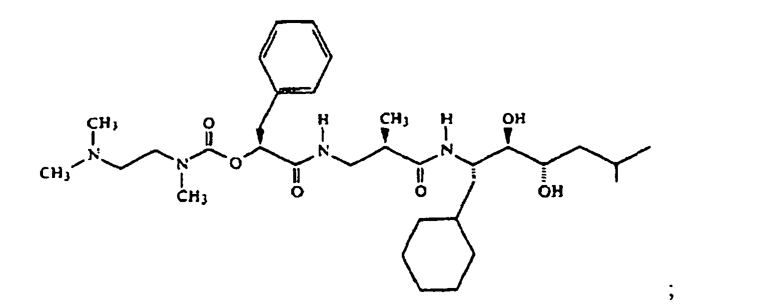 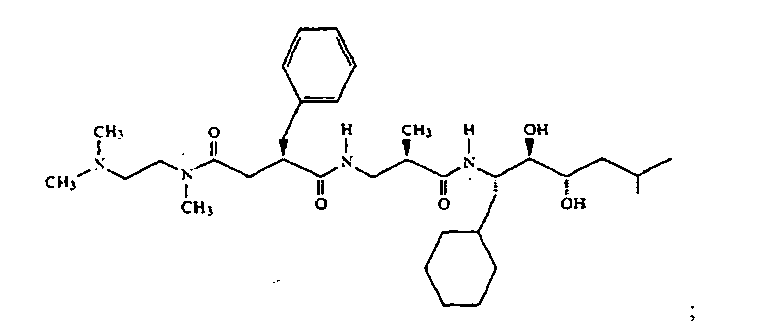 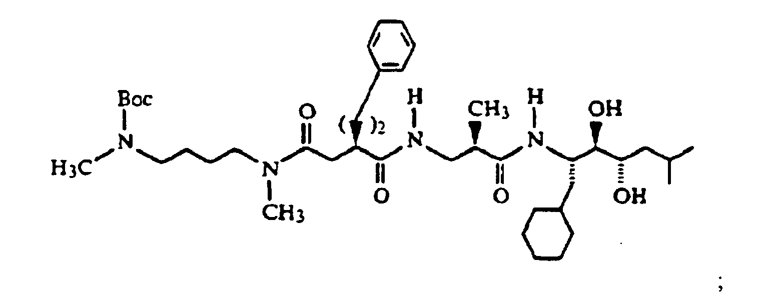 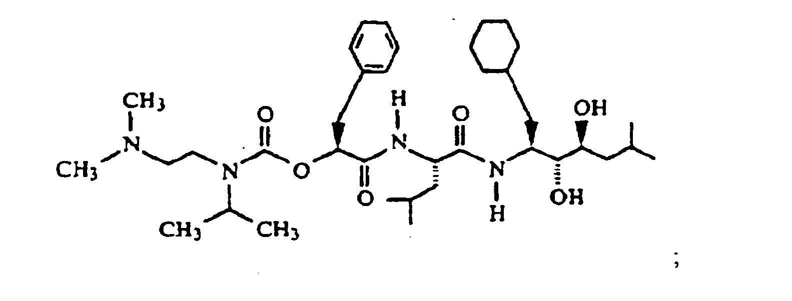 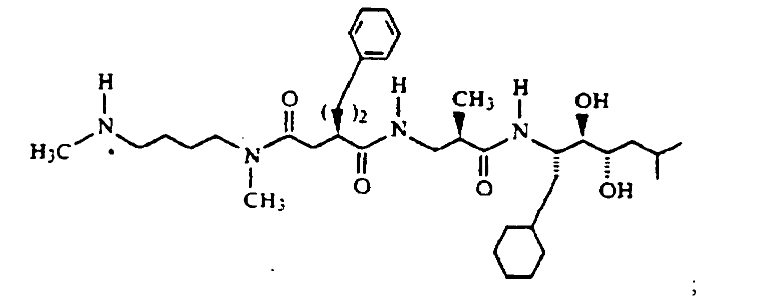 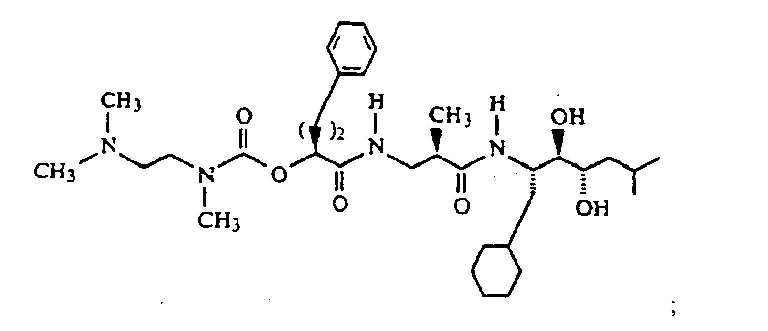 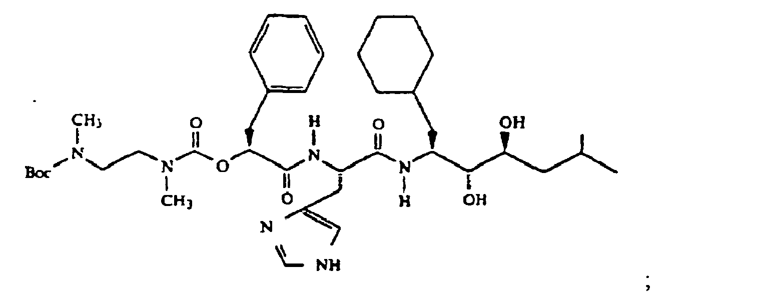 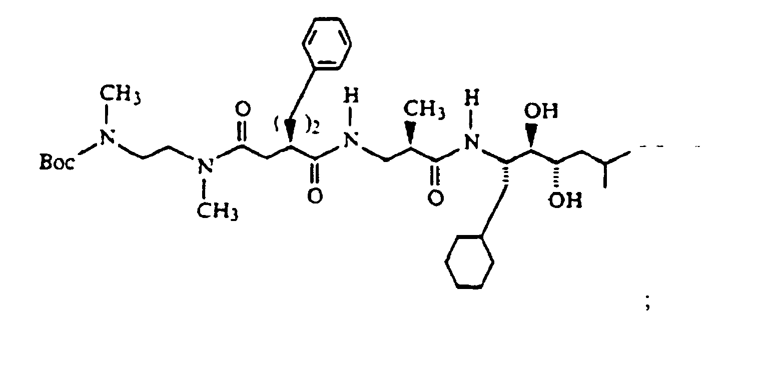 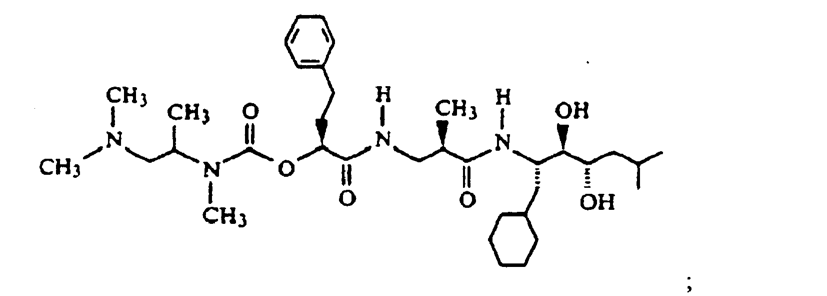 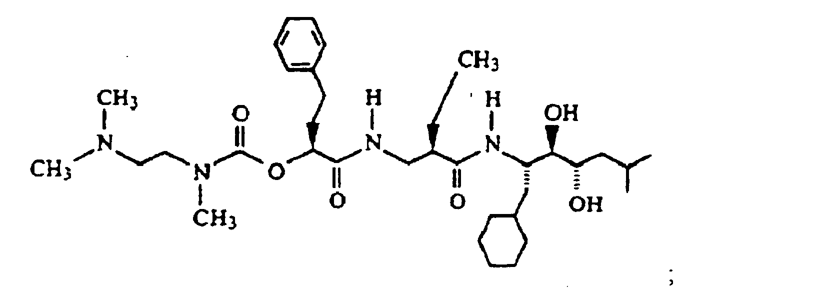 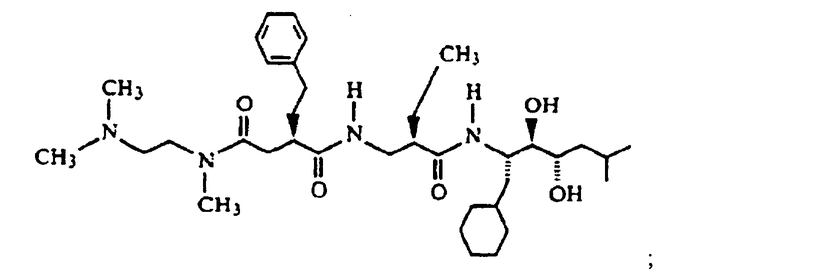 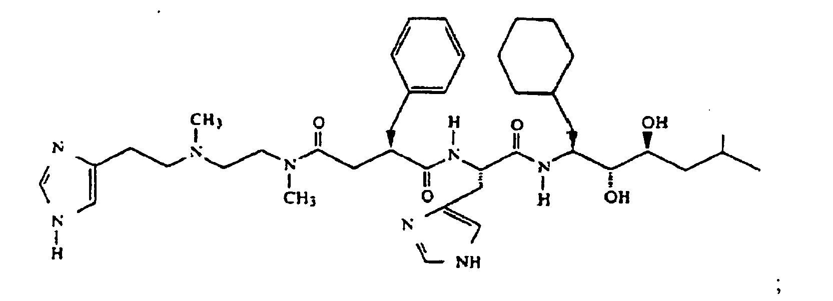 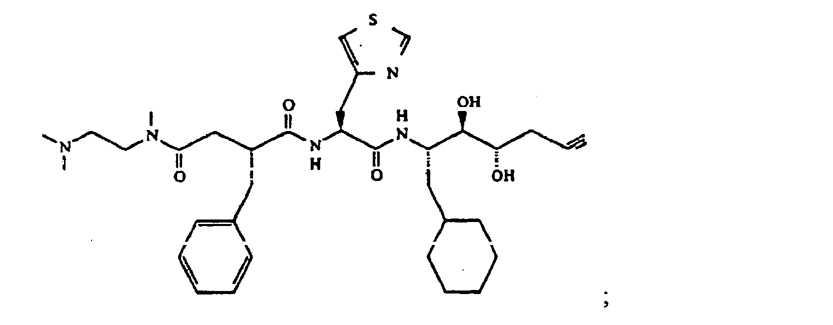 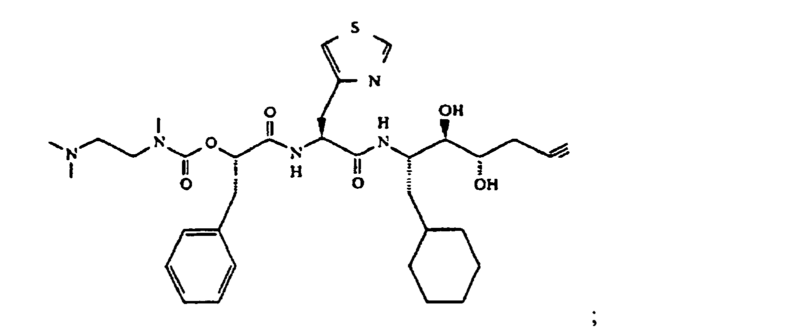 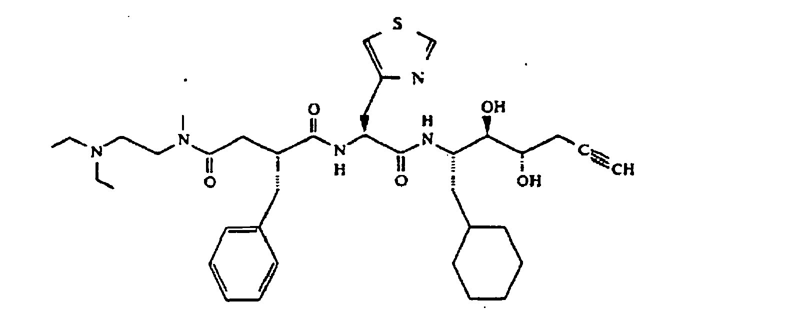 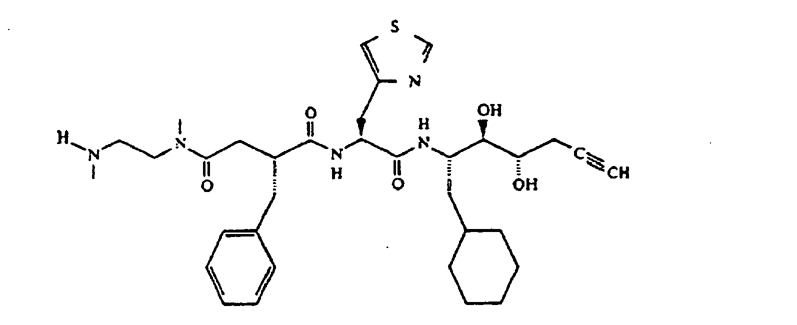 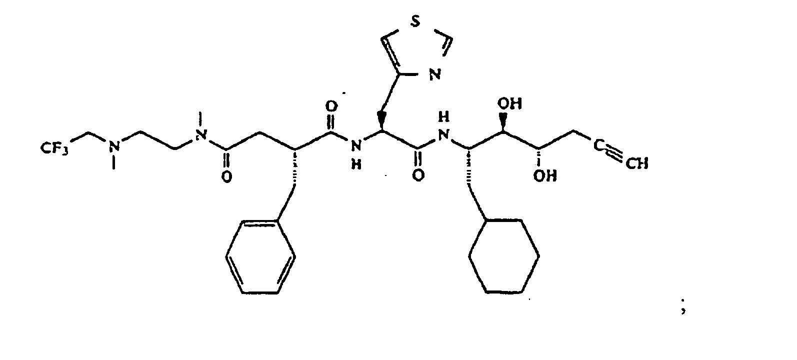 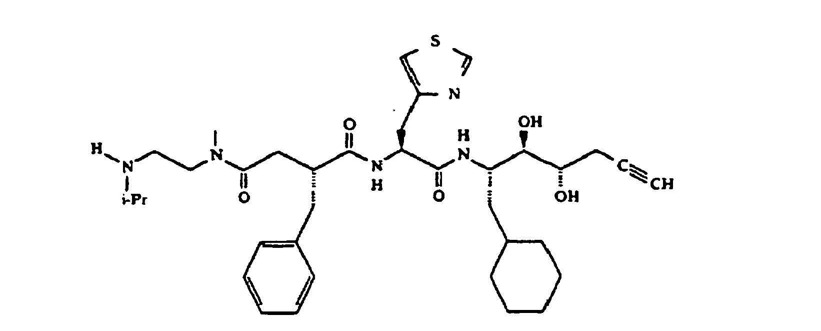 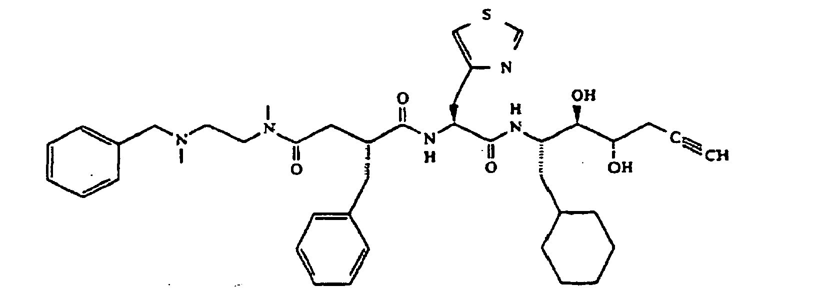 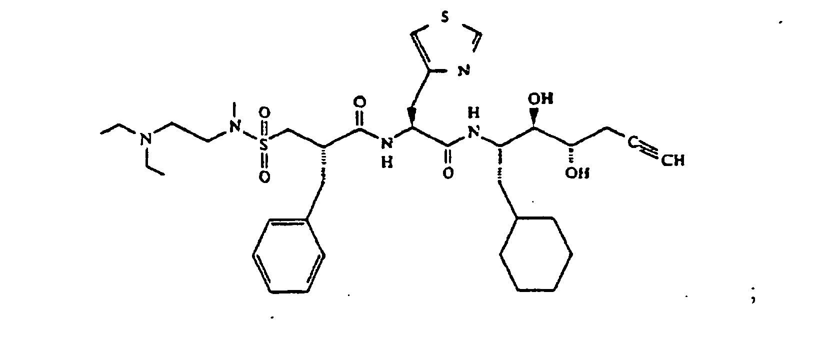 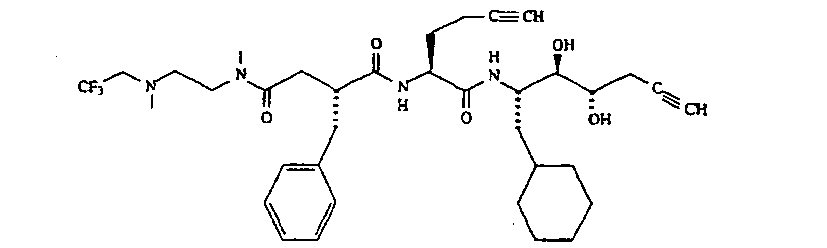 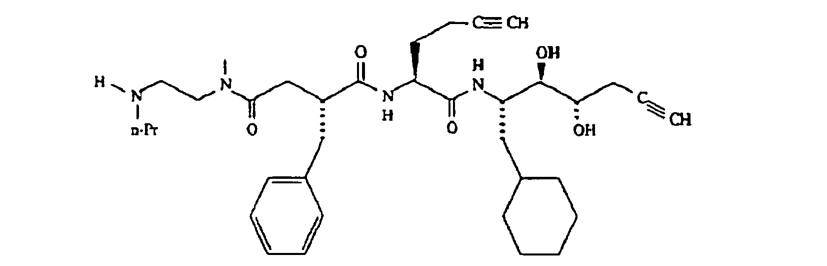 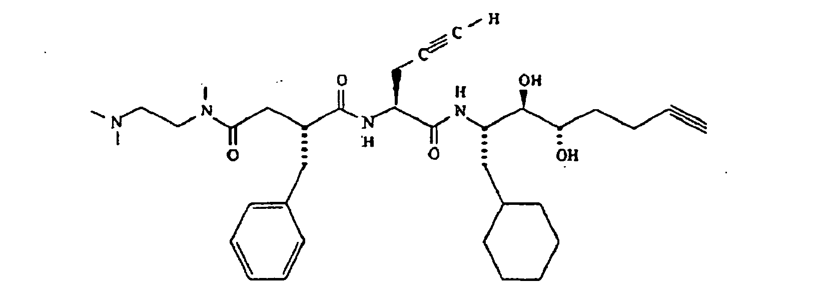 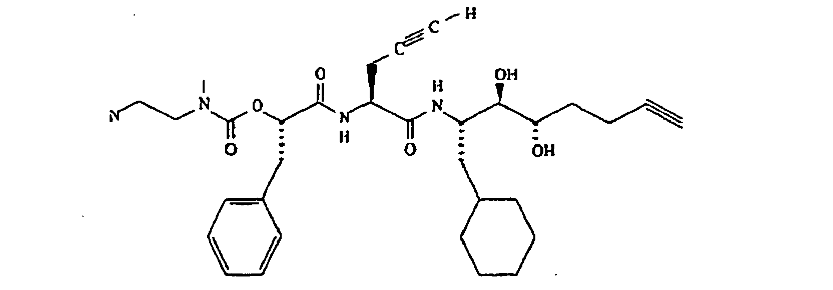 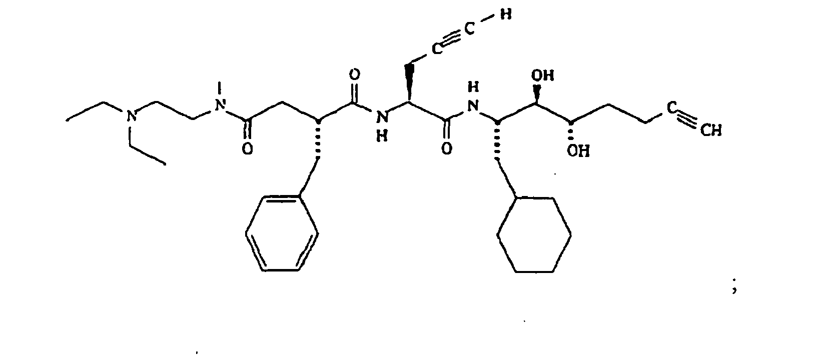 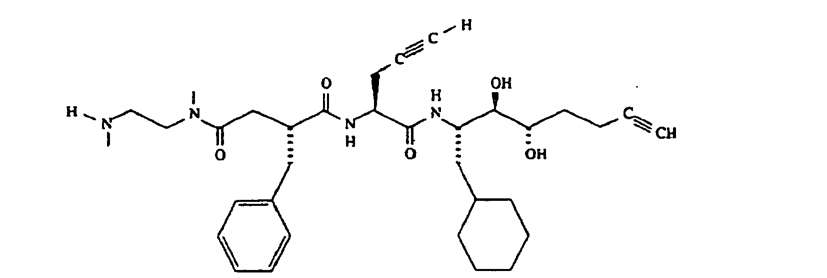 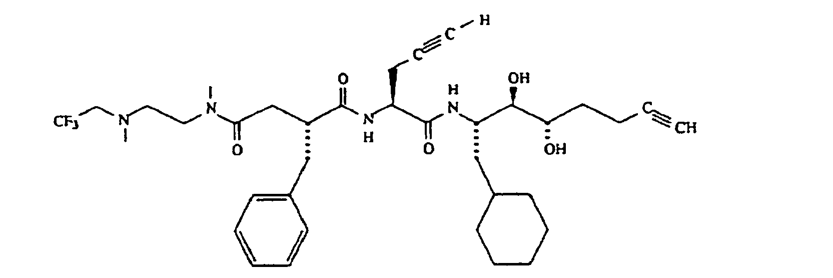 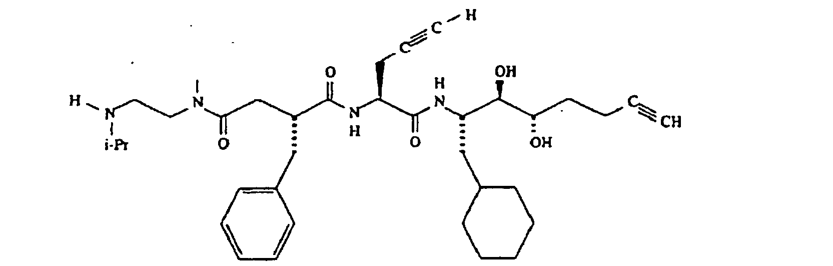 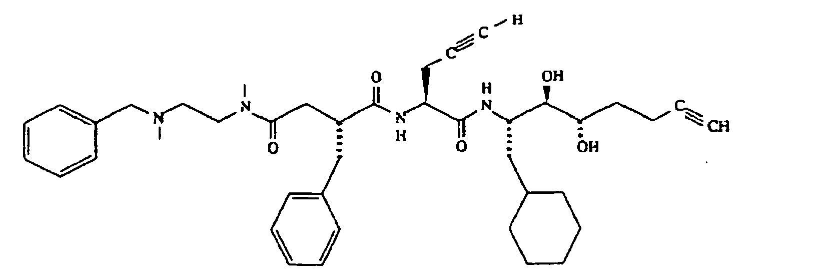 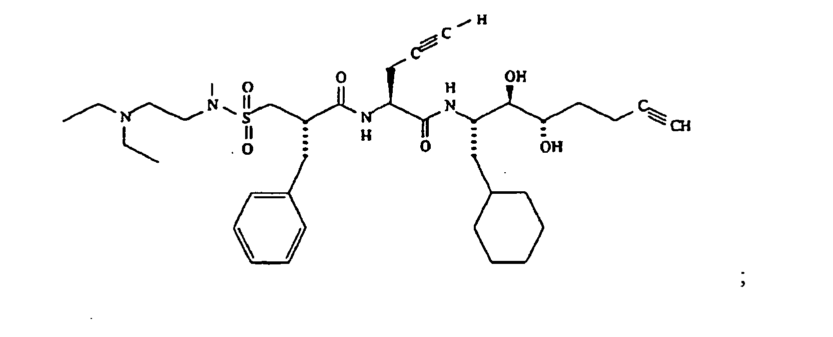 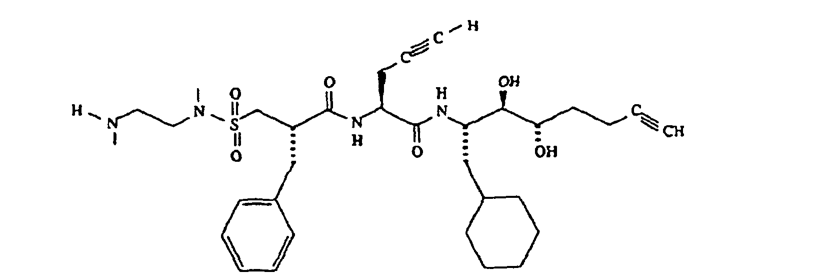 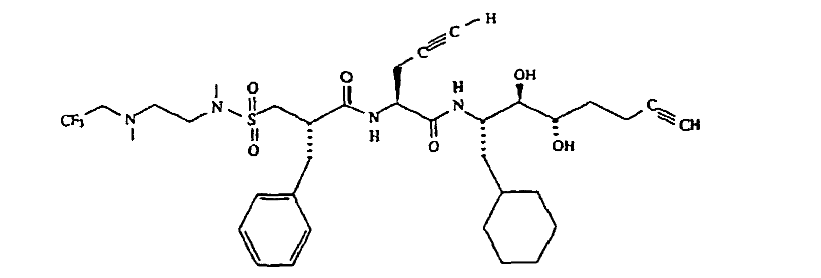 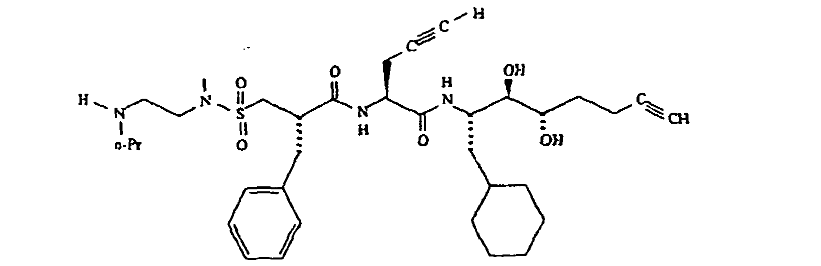 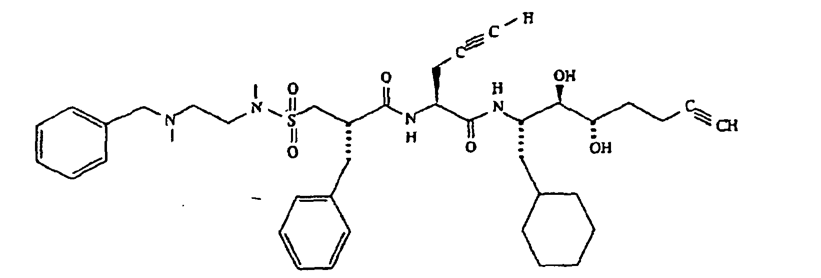 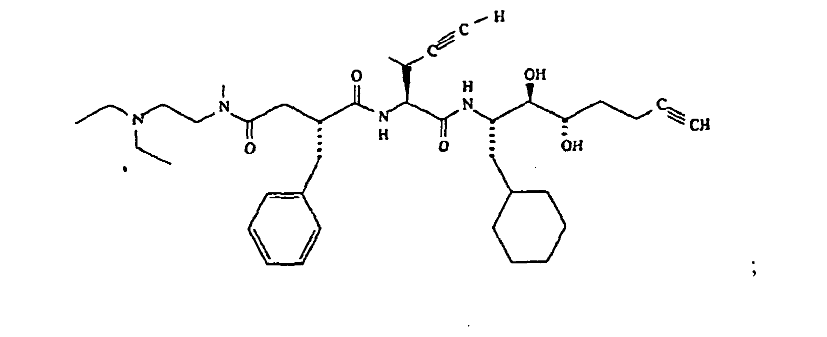 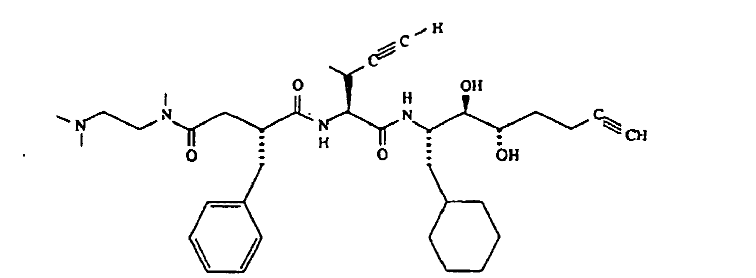 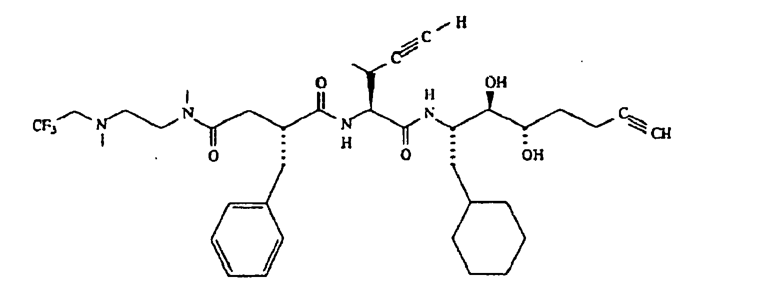 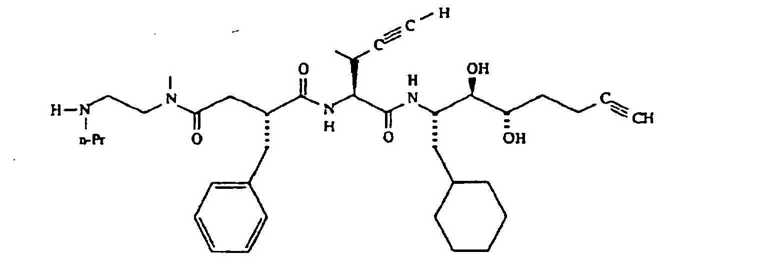 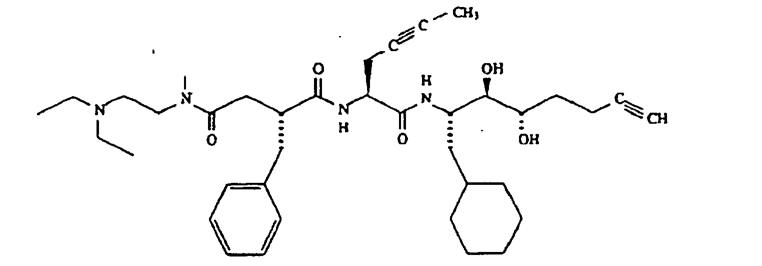 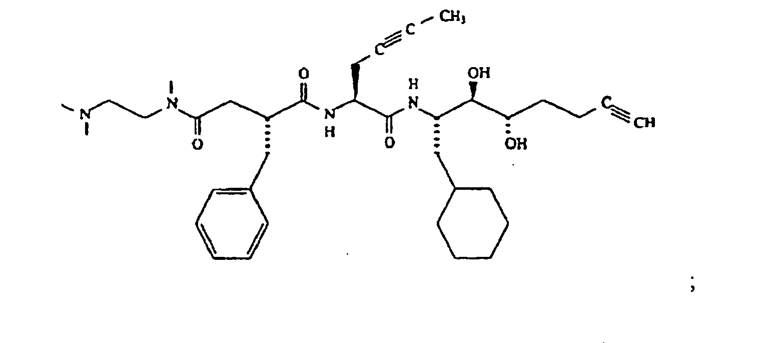 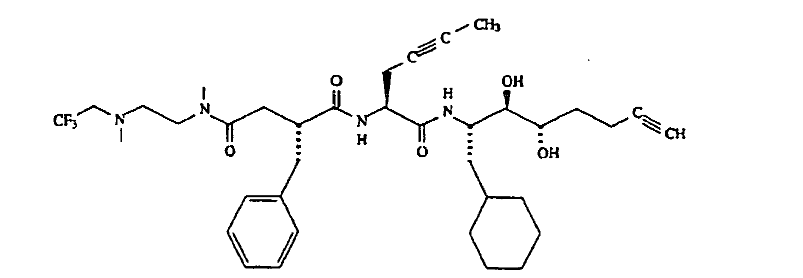 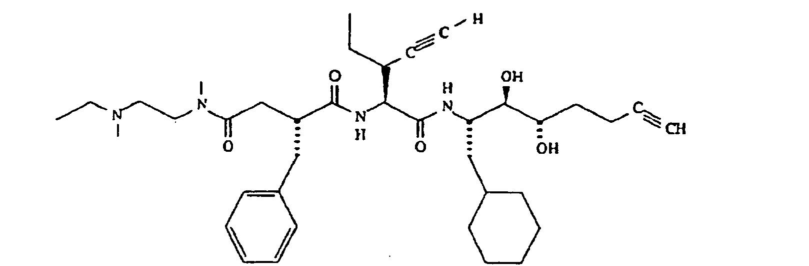 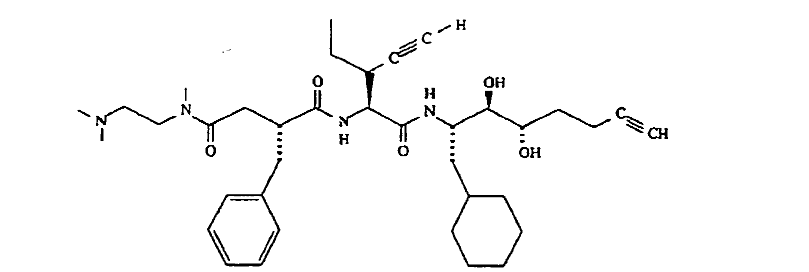 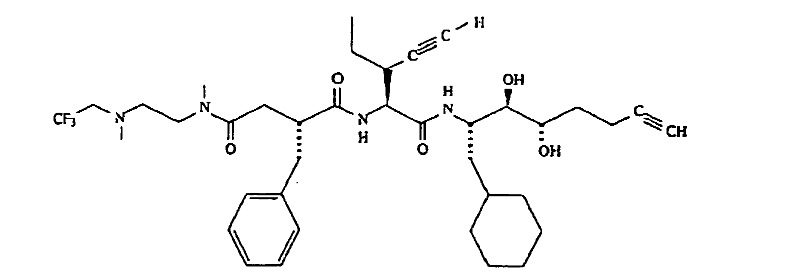 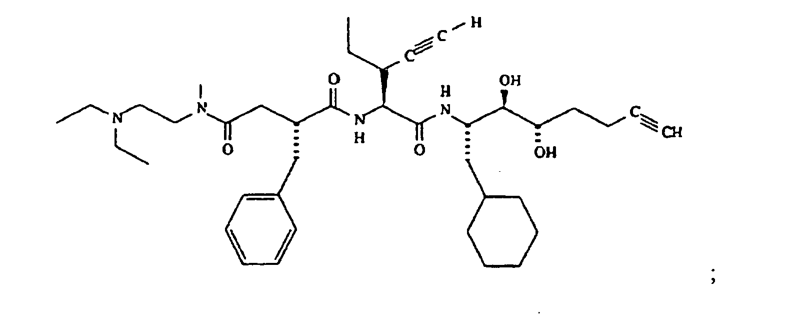 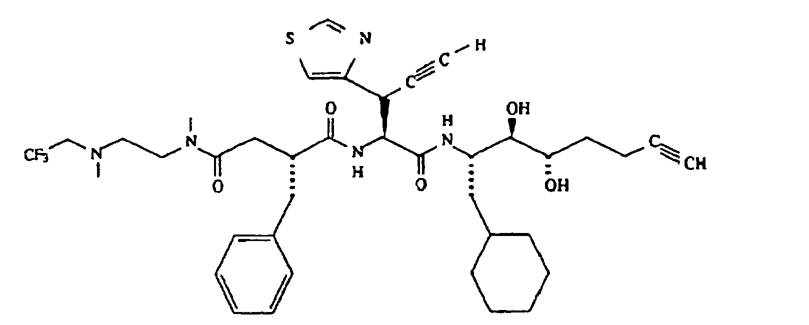 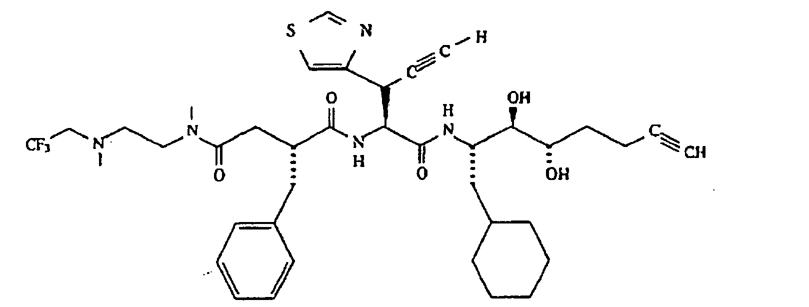 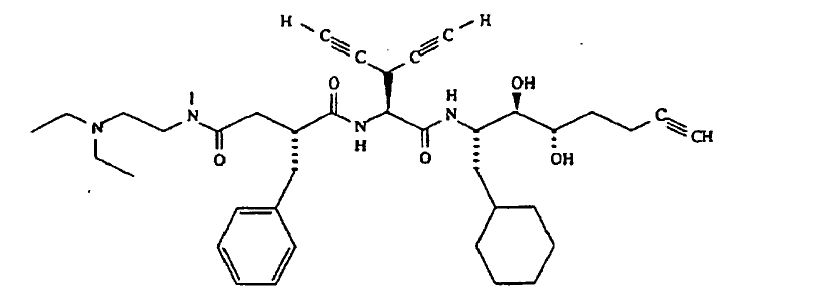 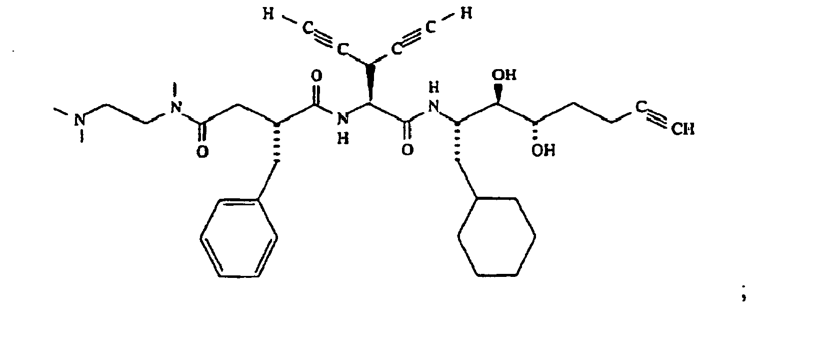 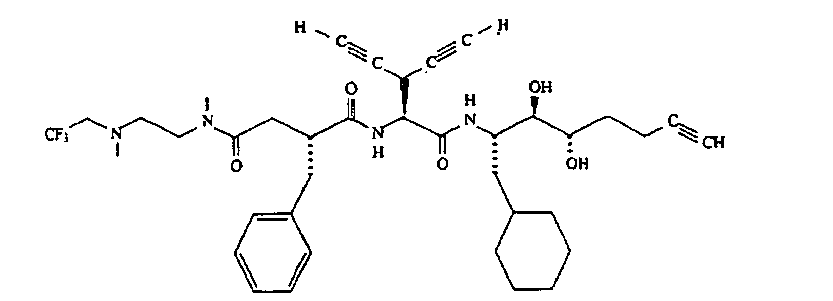 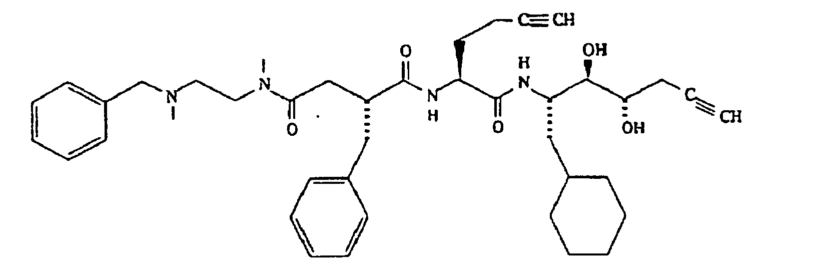 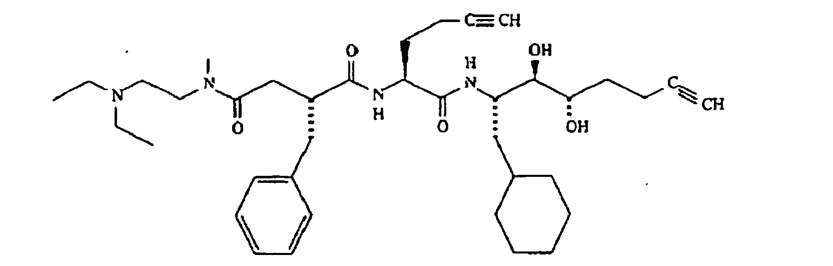 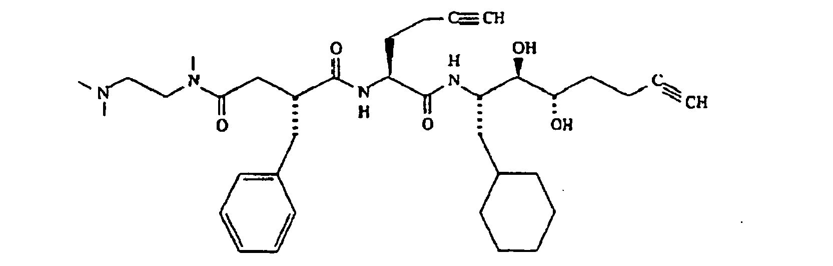 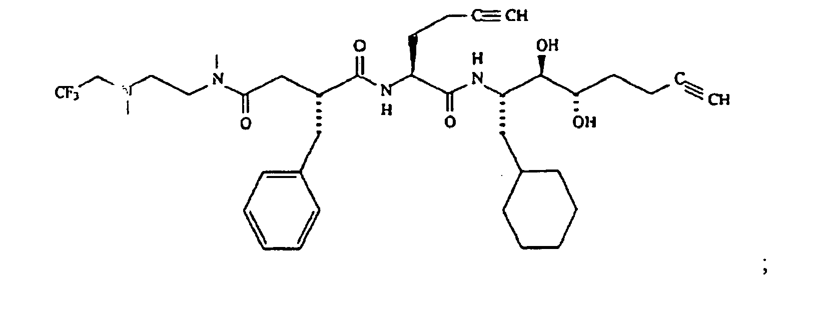 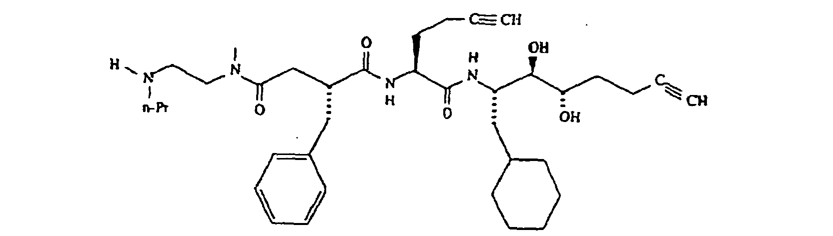 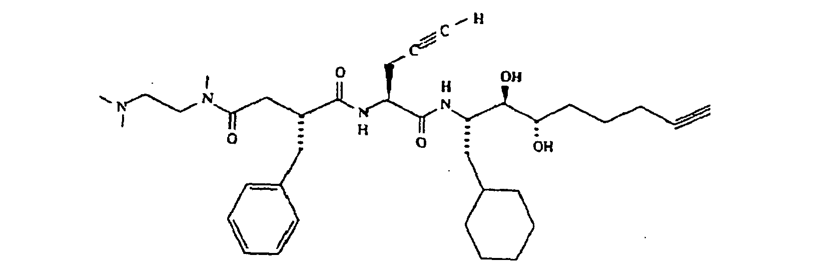 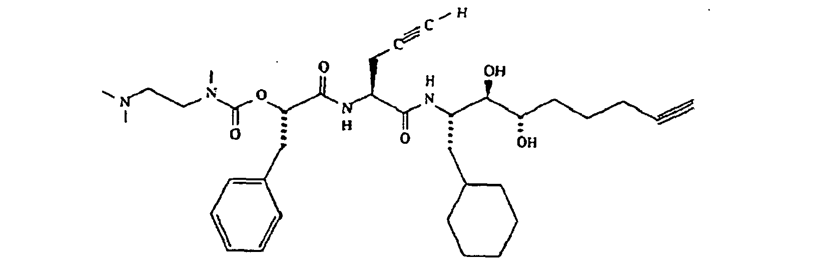 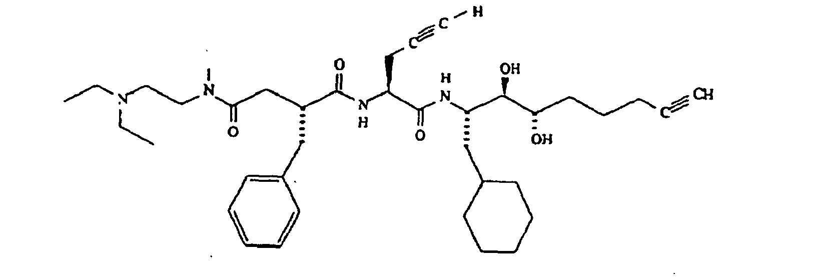 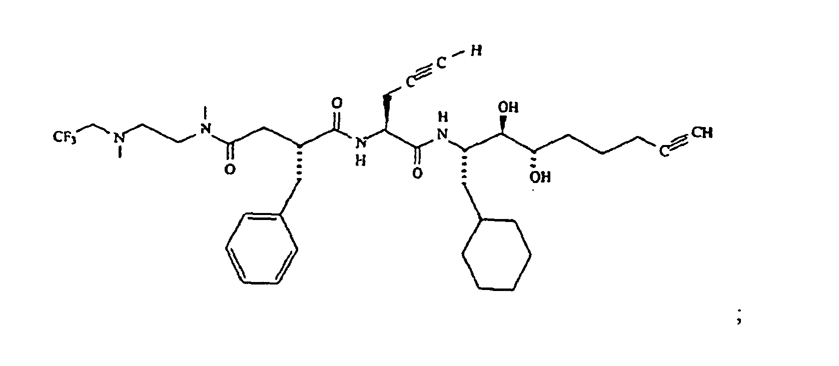 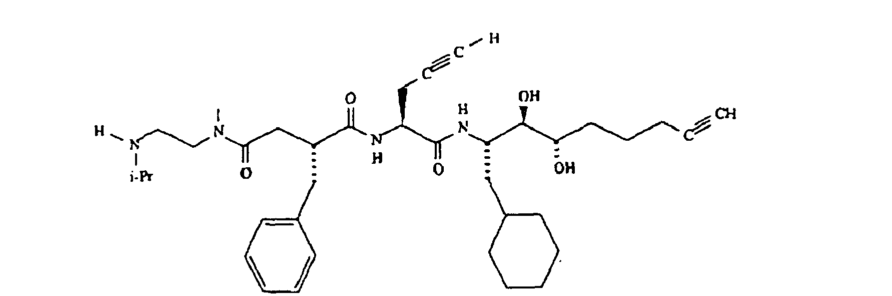 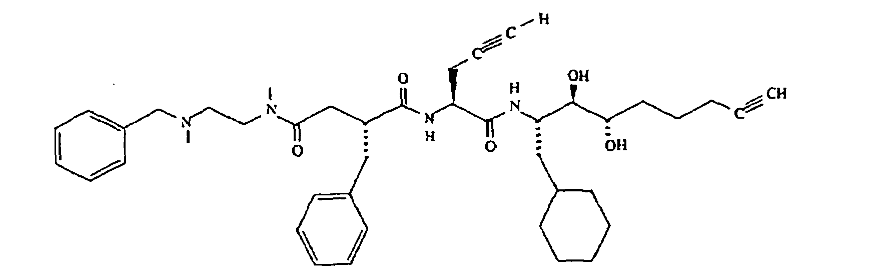 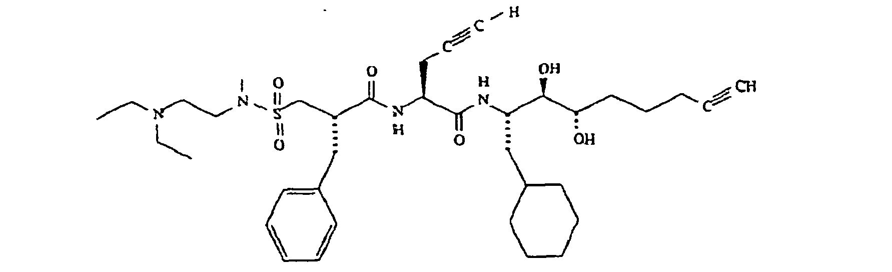 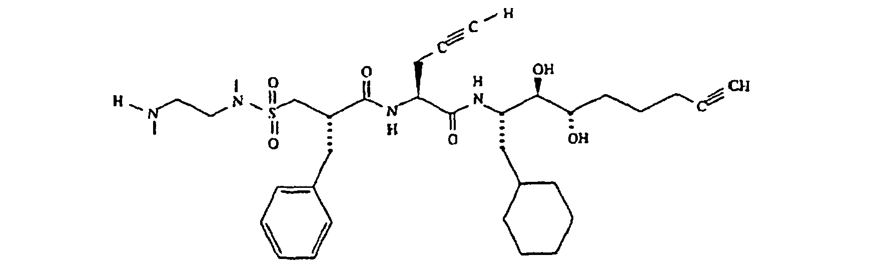 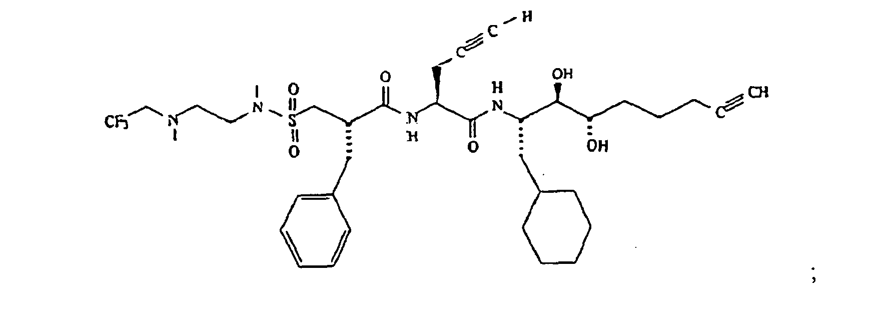 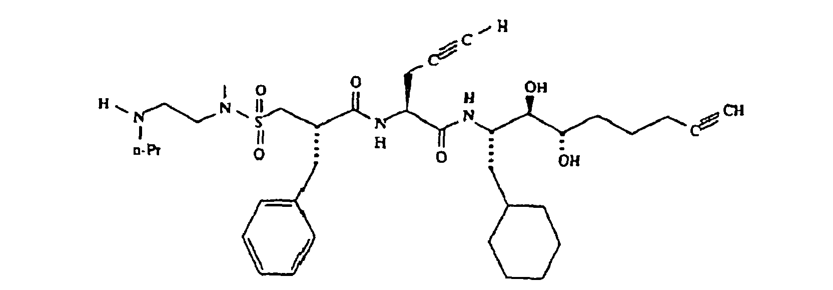 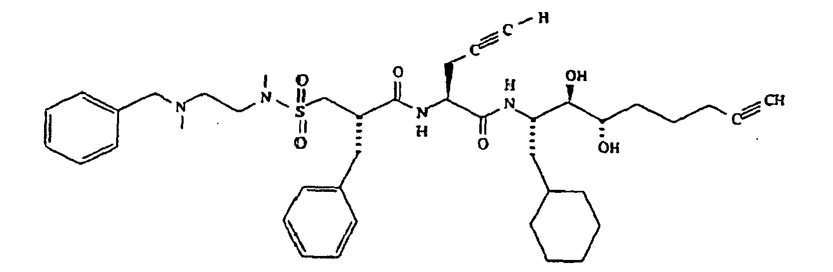 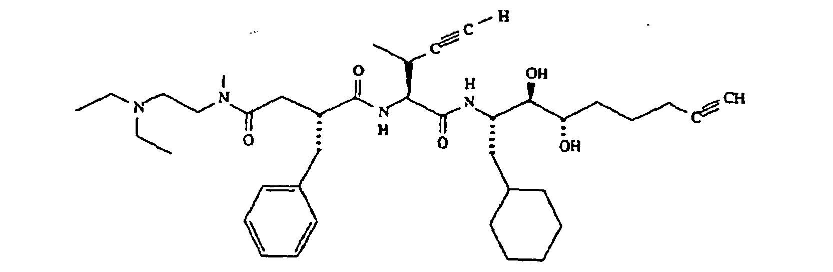 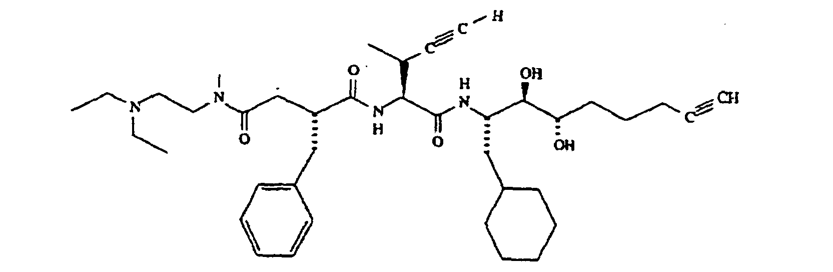 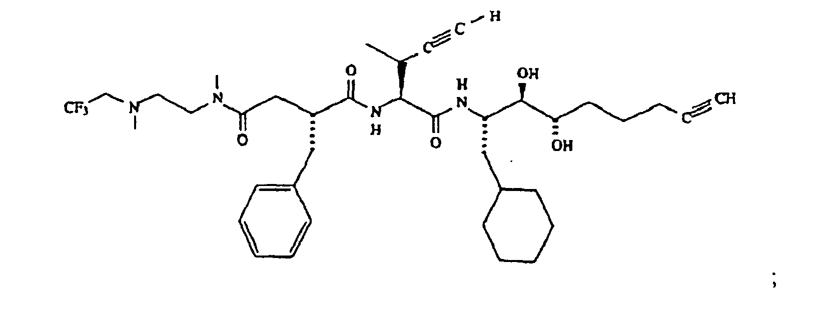 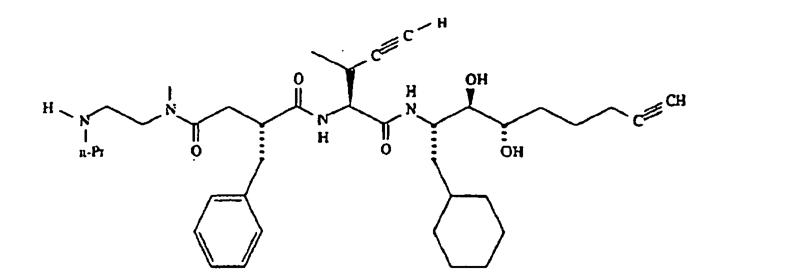 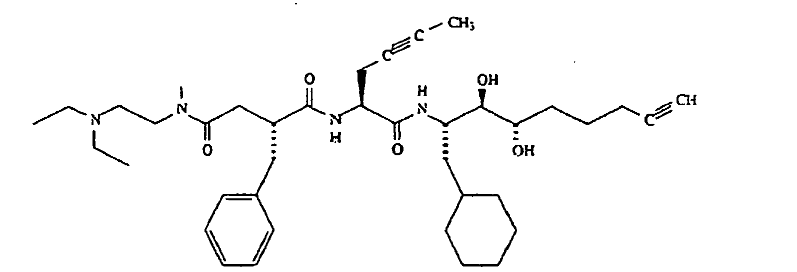 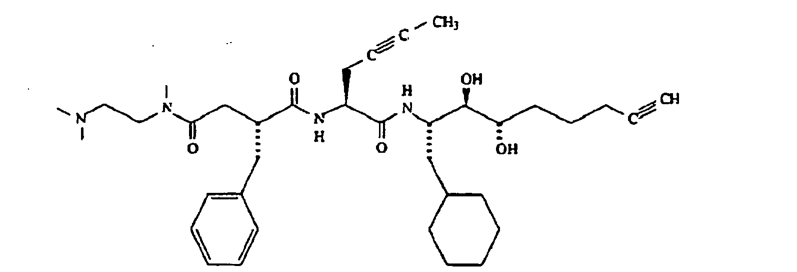 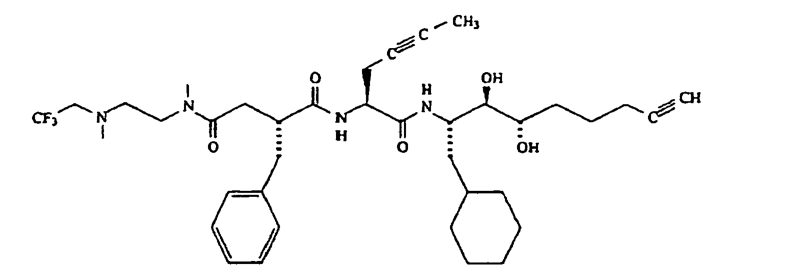 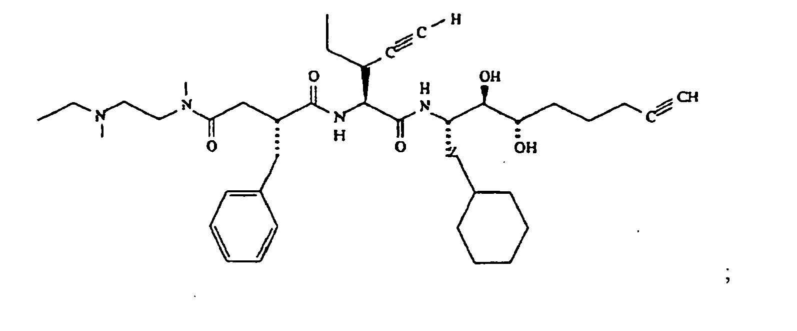 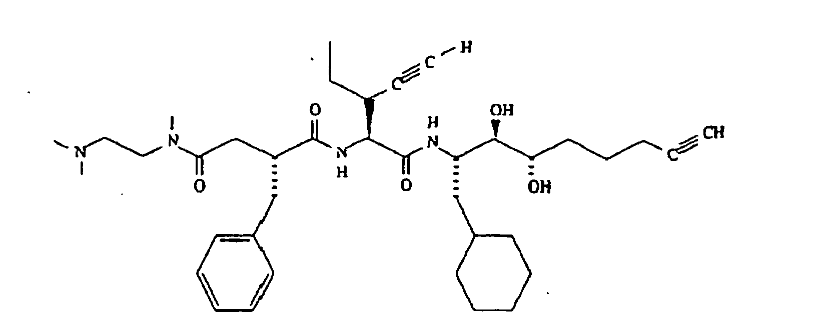 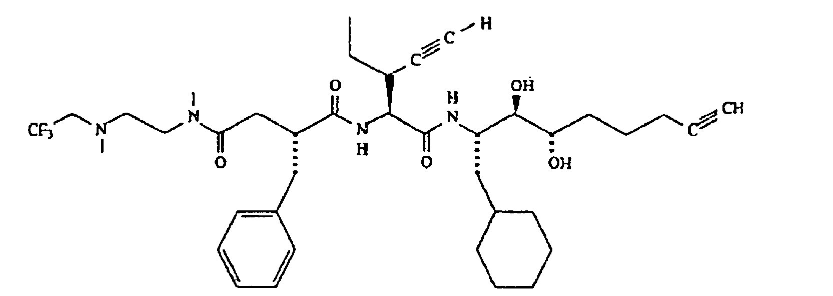 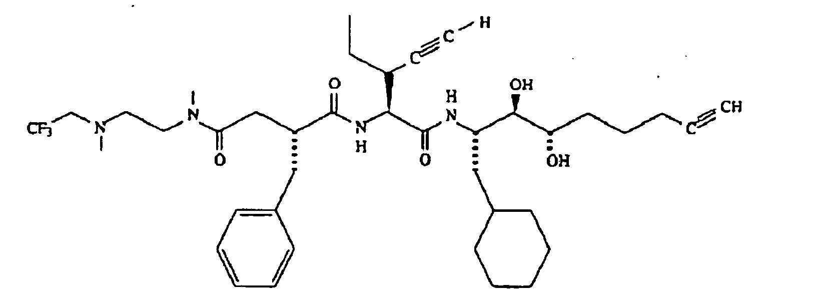 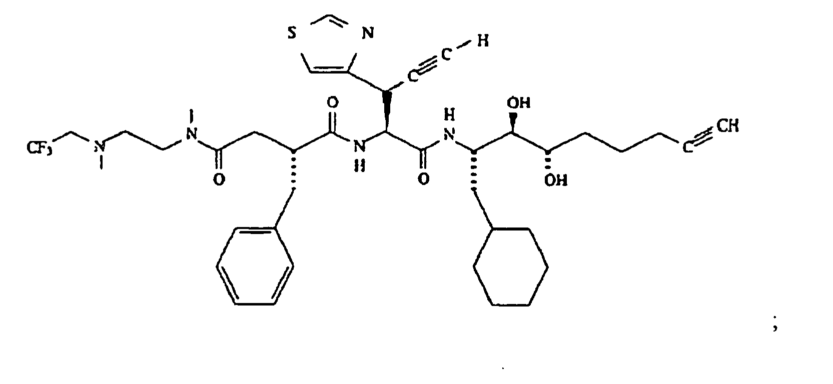 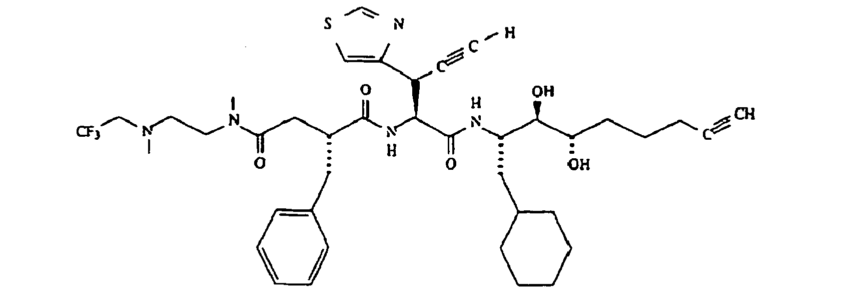 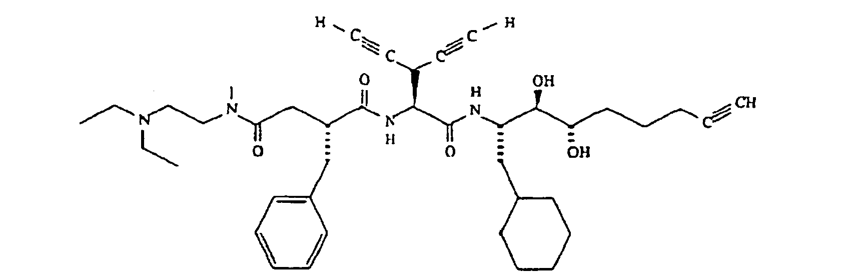 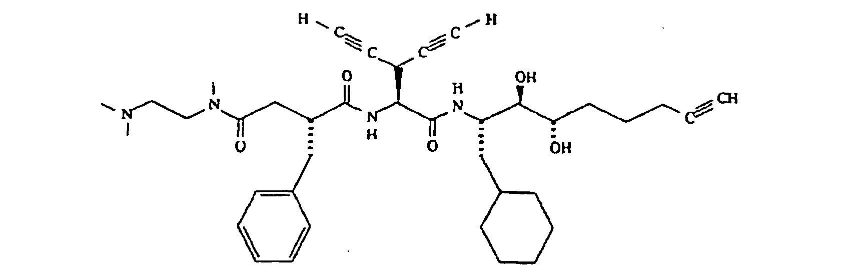 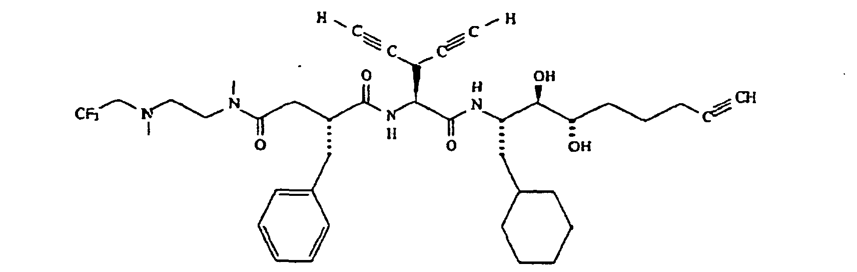 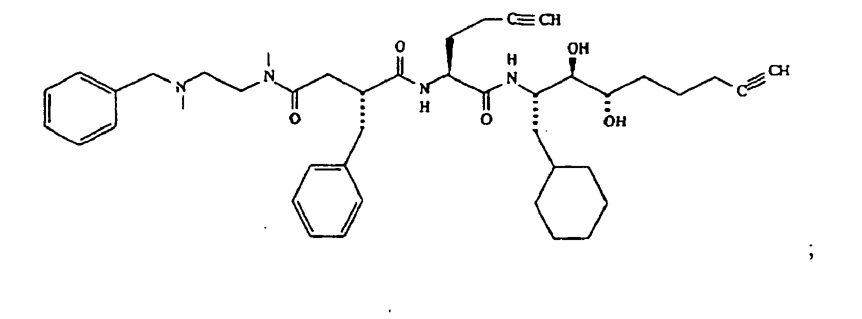 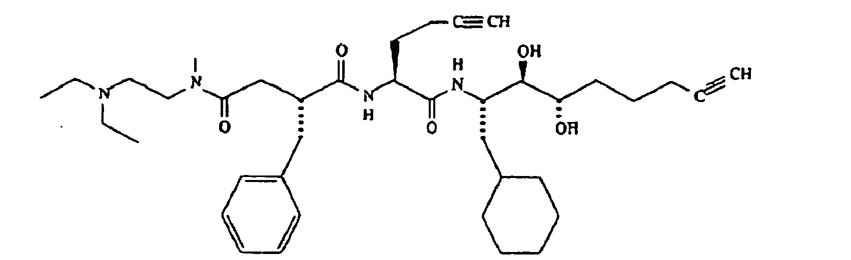 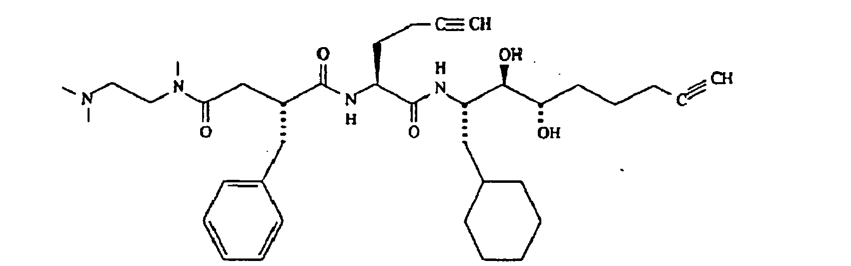 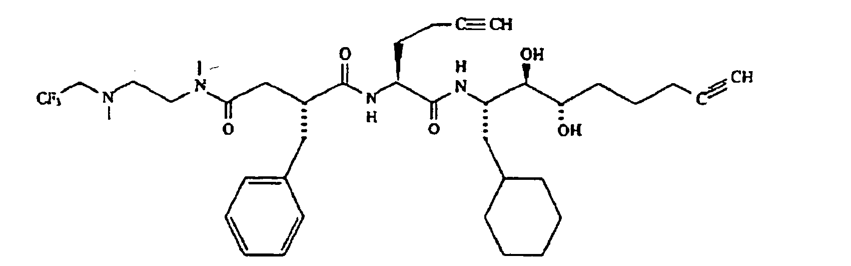 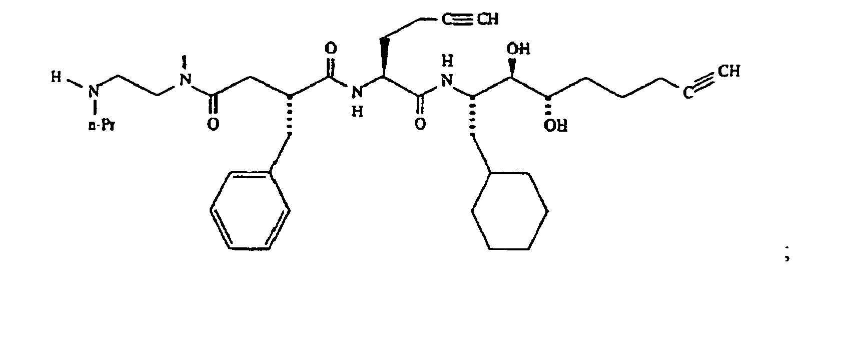 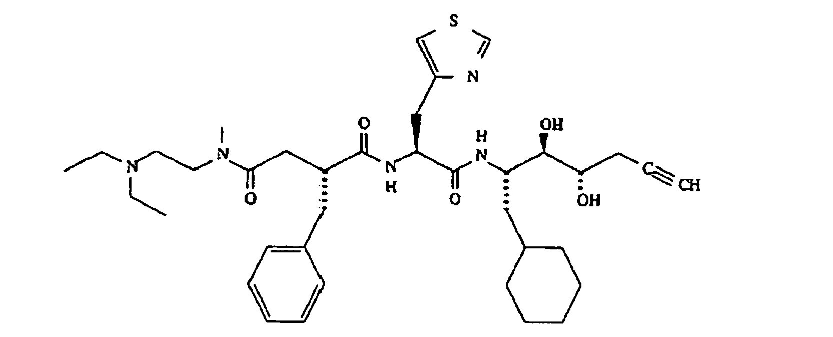 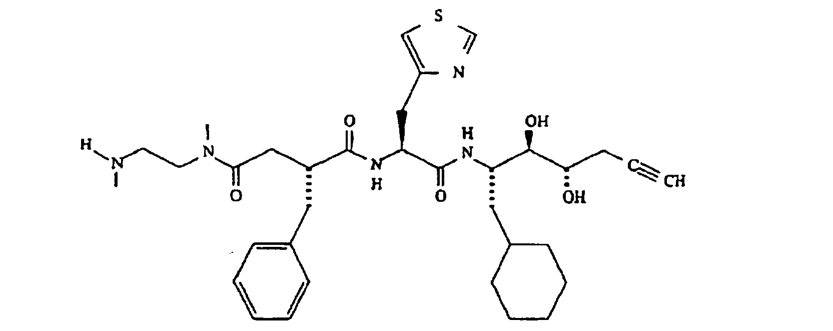 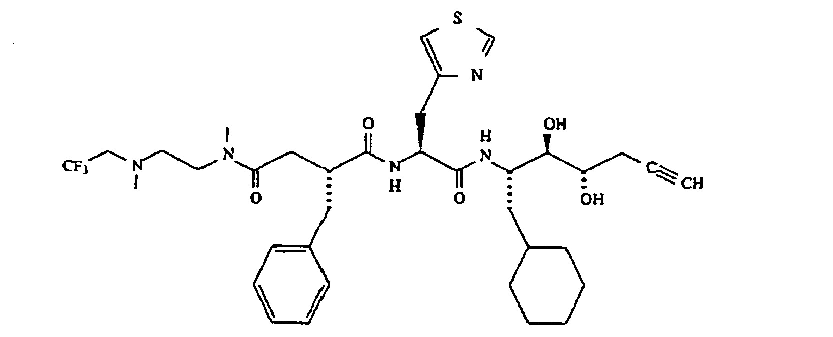 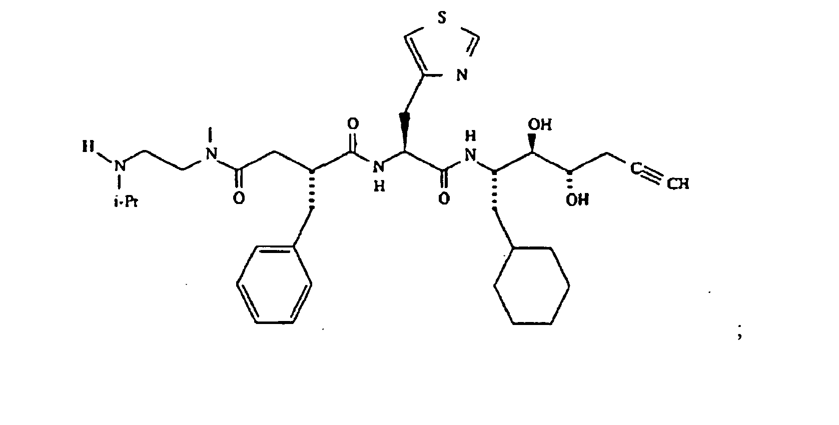 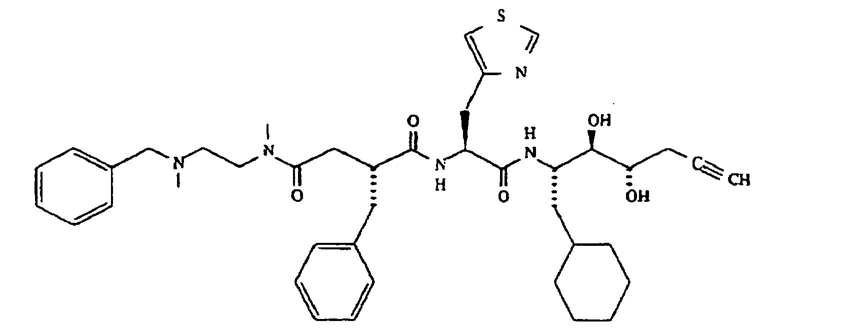 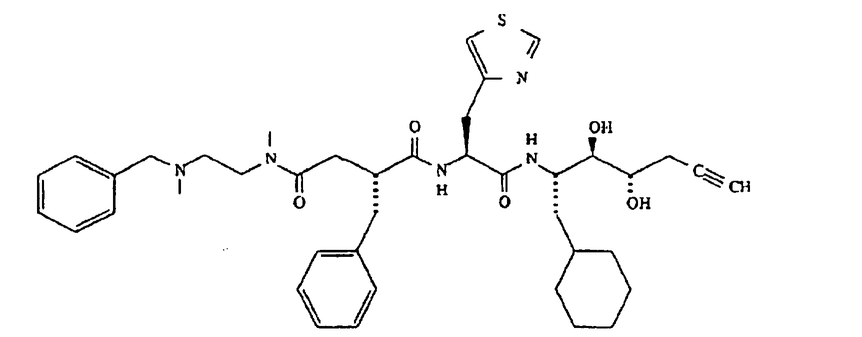 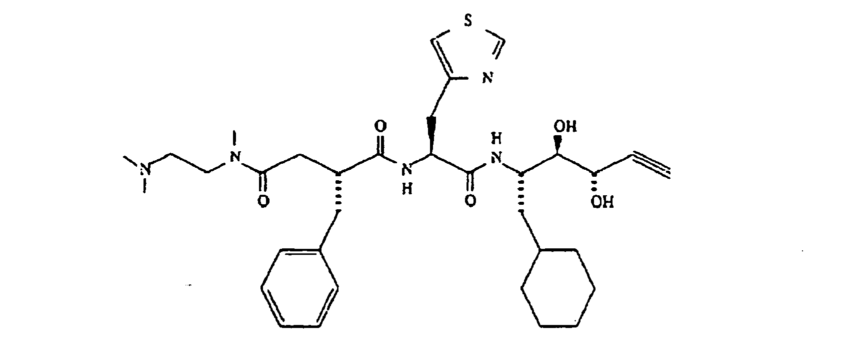 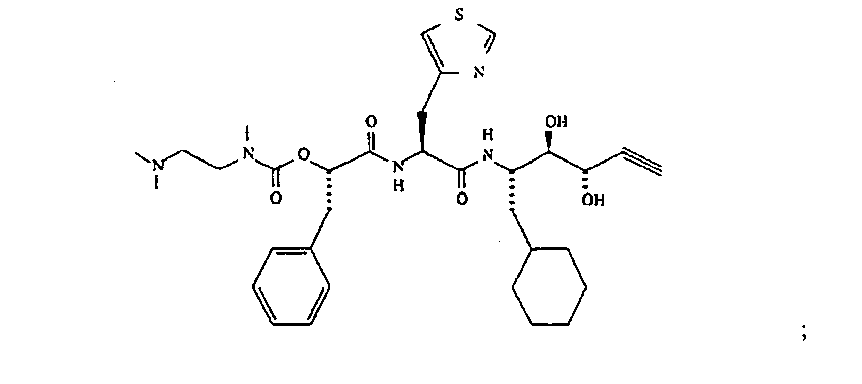 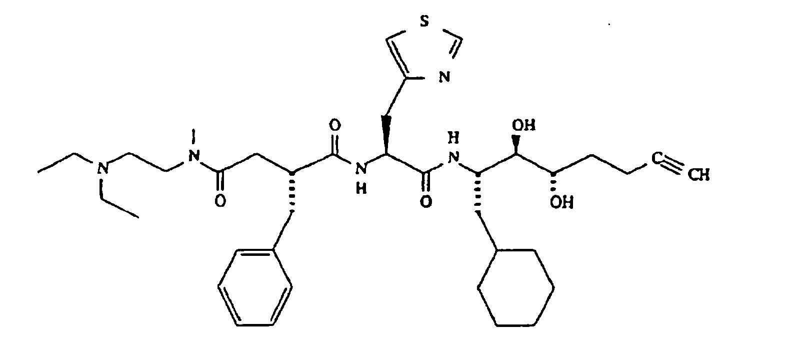 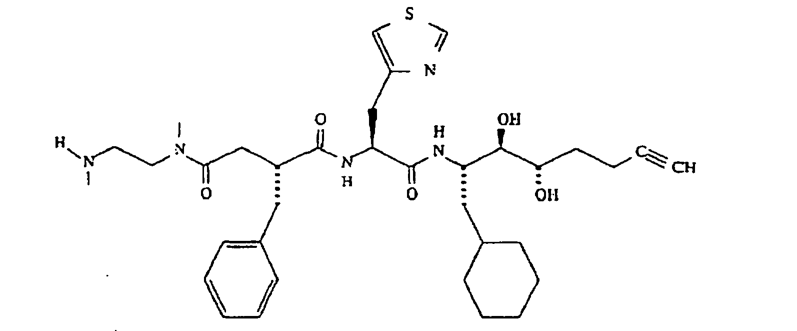 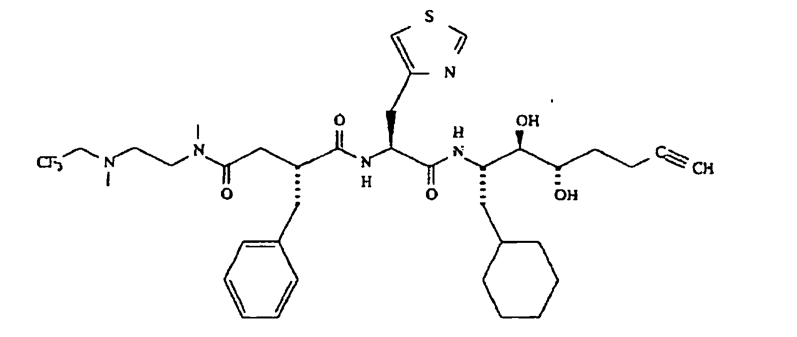 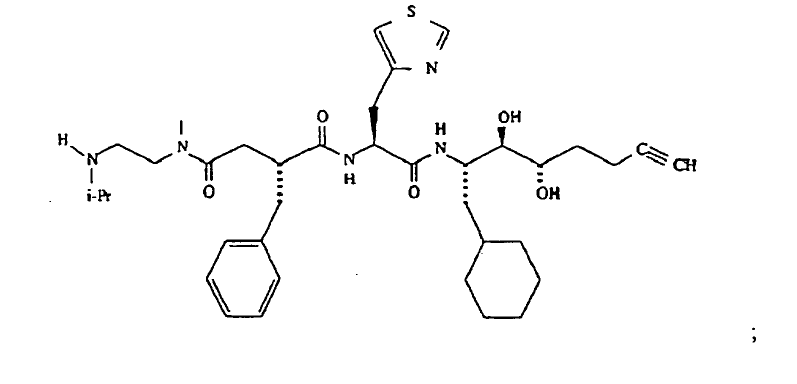 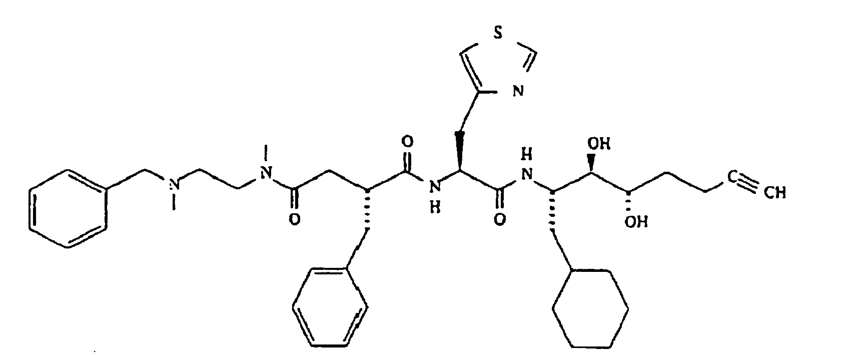 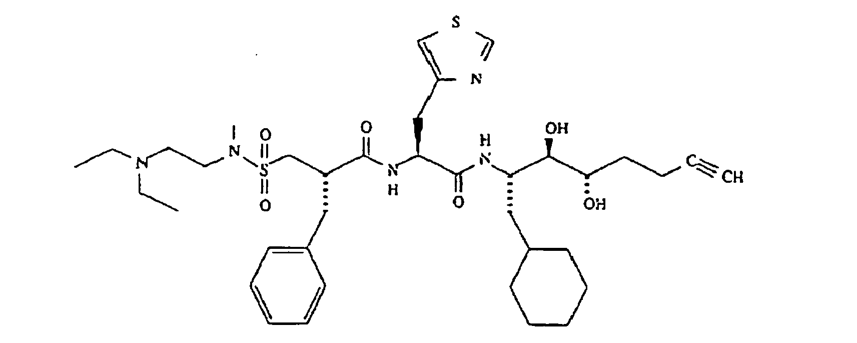 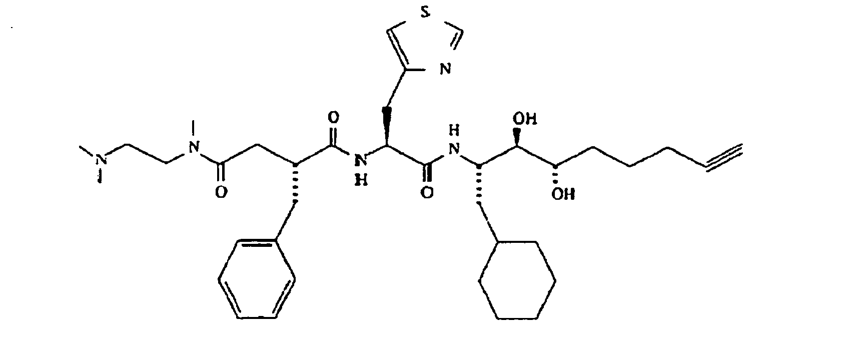 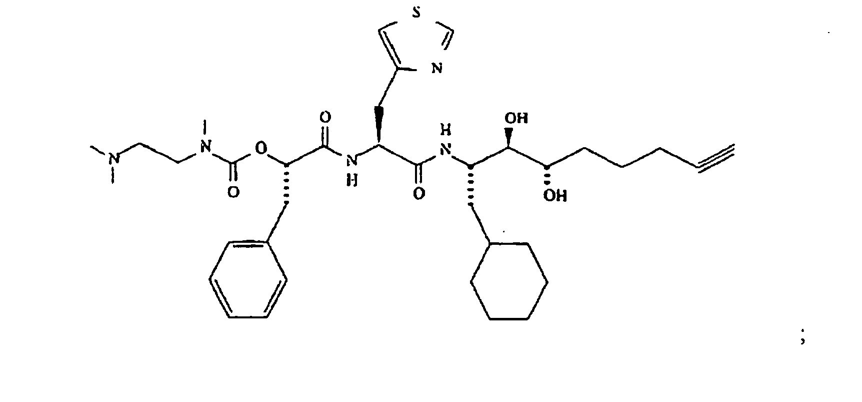 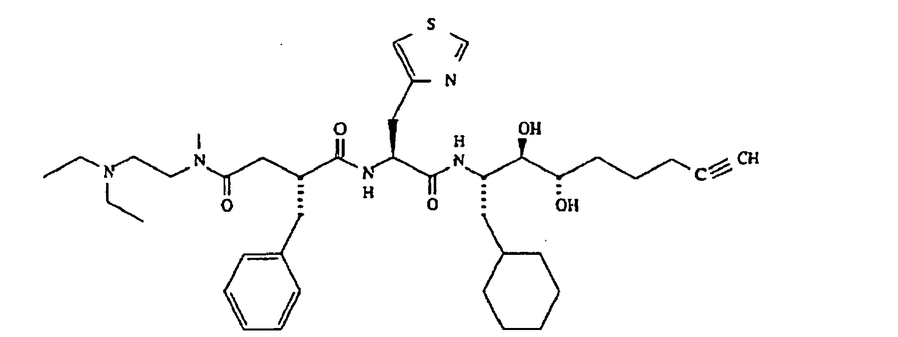 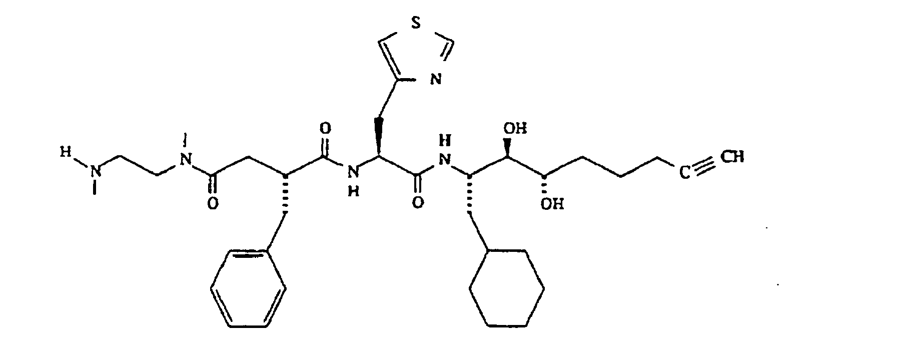 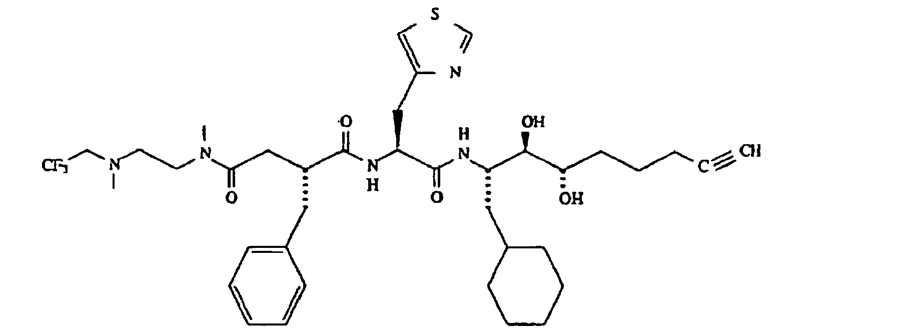 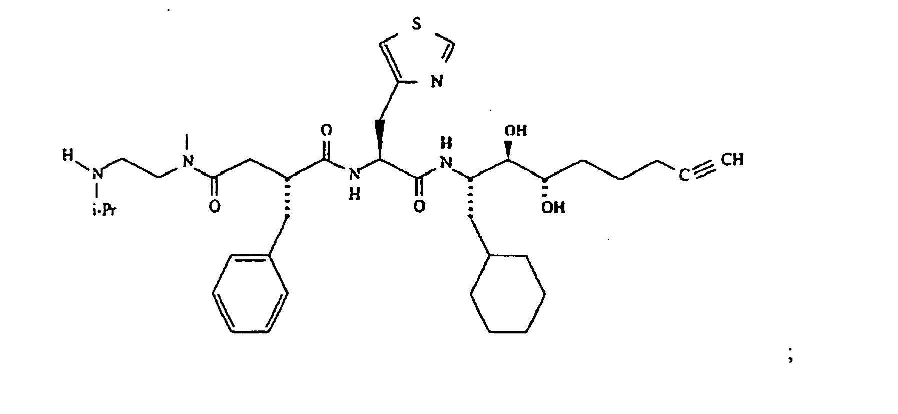 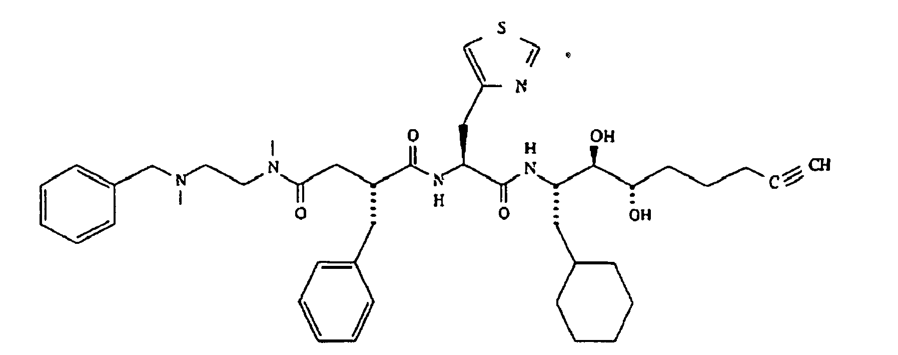 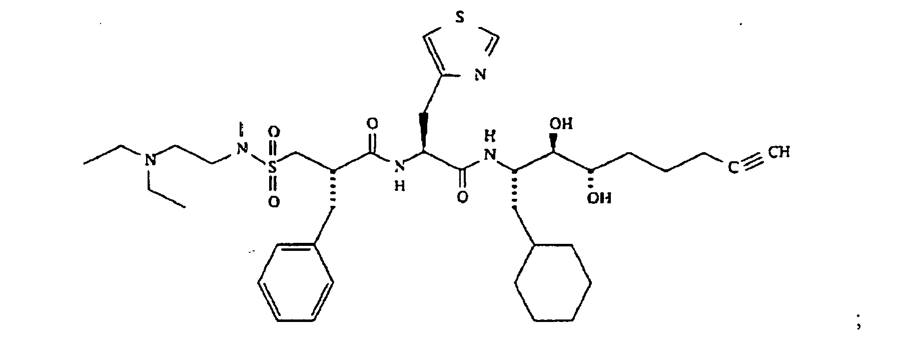 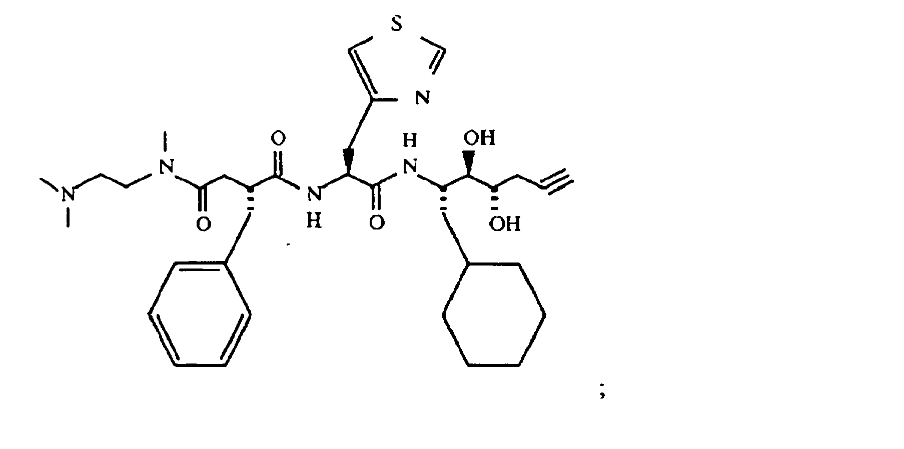 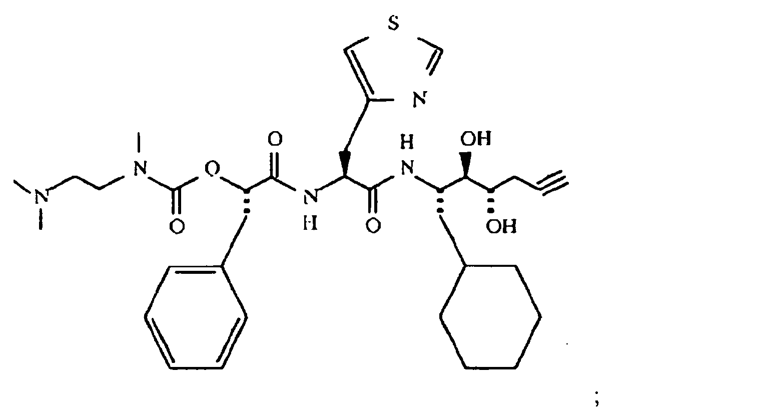 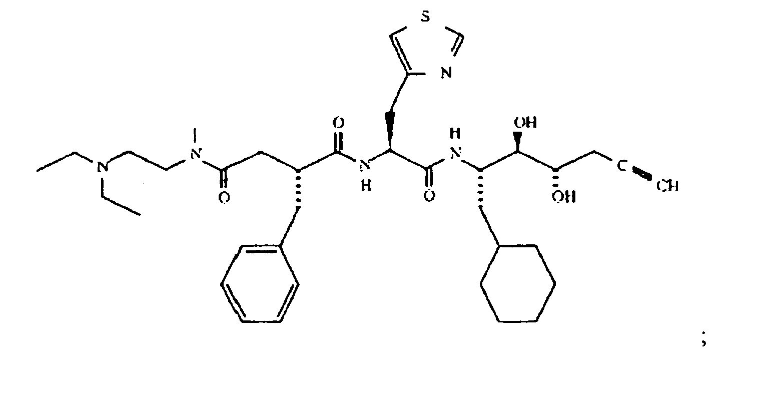 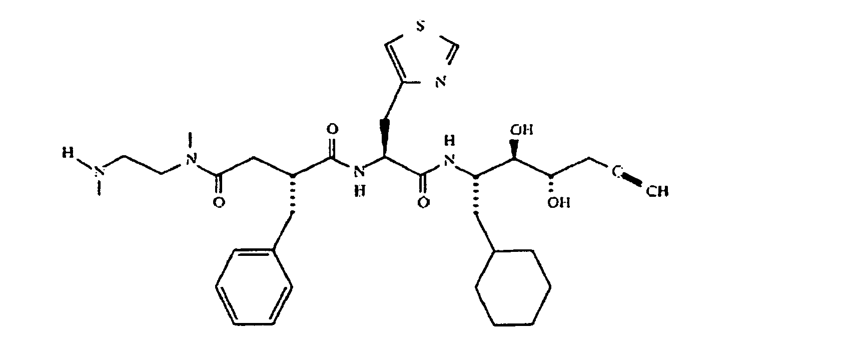 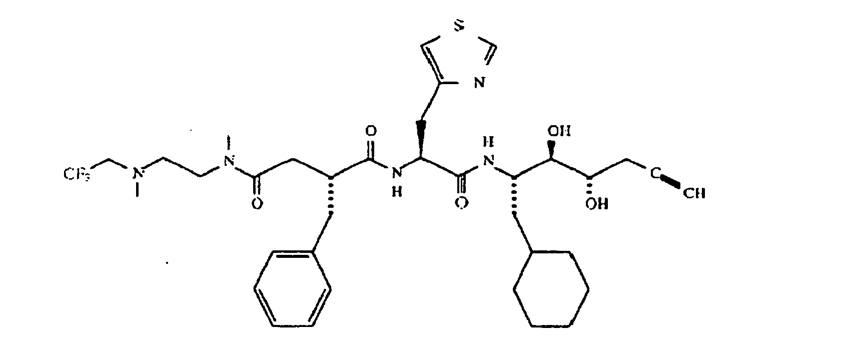 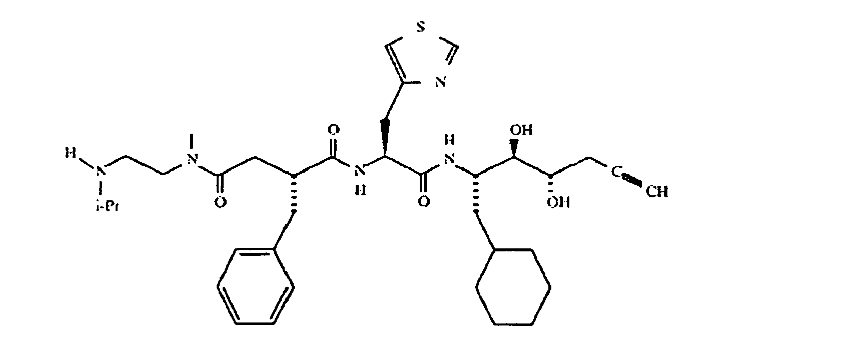 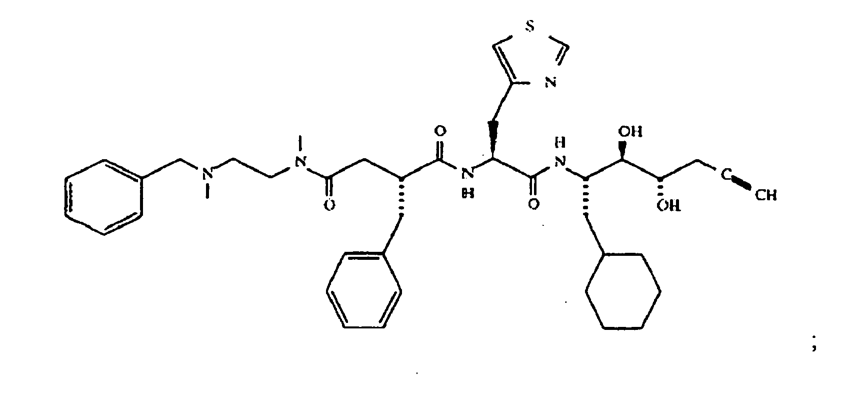 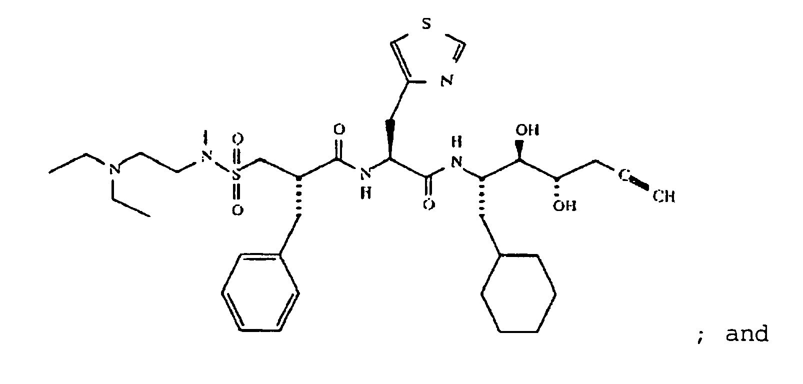 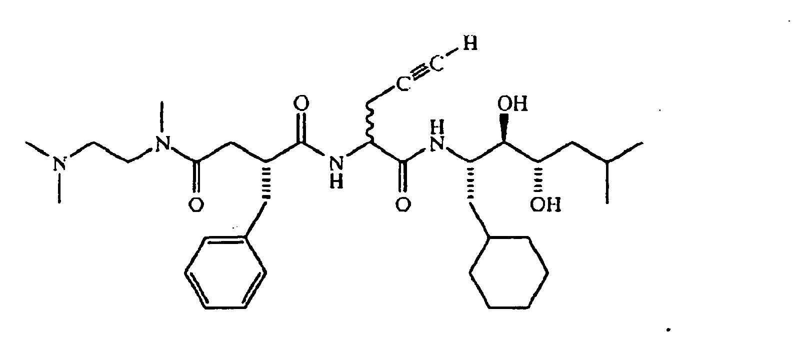
Representative compounds of Formula (II):
   N-[3-[[1S,1R*-(cyclohexylmethyl)-2S*,3R*-dihydroxy-5-methylhexyl]amino]-2S*-methyl-3-oxopropyl]benzenebutanamide;
   N-[3-[[1S,1R*-(cyclohexylmethyl)-2S*,3R*-dihydroxy-5-methylhexyl]amino]-2S*-methyl-3-oxopropyl]-1H-indole-2-carboxamide;
   N-[3-[[1S,1R*-(cyclohexylmethyl)-2S*,3R*-dihydroxy-5-methylhexyl]amino]-2S*-methyl-3-oxopropyl]benzofuran-2-carboxamide;
   N-3-[[1S,1R*-(cyclohexylmethyl)-2S*,3R*-dihydroxy-5-methylhexyl]amino]-2S*-methyl-3-oxopropyl]1H-indole-2-acetamide;
   N-[3-[[1S,1R*-(cyclohexylmethyl)-2S*,3R*-dihydroxy-5-methylhexyl]amino-]-2S*-methyl-3-oxopropyl]-N-methyl-benzenebutanamide;
   N-[3-[[1S,1R*-(cyclohexylmethyl)-2S*,3R*-dihydroxy-5-methylhexyl]amino]-2S*-methyl-3-oxopropyl]cyclohexanebutanamide;
   N-[3-[[1S,1R*-(cyclohexylmethyl)-2S*,3R*-dihydroxy-5-methylhexyl]amino]-2S*-methyl-3-oxopropyl]-N-methyl-cyclohexanebutanamide;
   N-[3-[[1S,1R*-(cyclohexylmethyl)-2S*,3R*-dihydroxy-5-methylhexyl]amino]-2S*-methyl-3-oxopropyl]-4-methoxybenzenepropanamide;
   N-3-[[1S,1R*-(cyclohexylmethyl)-2S*,3R*-dihydroxy-5-methylhexyl]amino]-2S*-methyl-3-oxopropyl]-2-quinaldylamide;
   N-[3-[[1S,1R*-(cyclohexylmethyl)-2S*,3R*-dihydroxy-5-methylhexyl]amino]-2S*-methyl-3-oxopropyl]-2-quinoxalinecarboxamide;
   N-[3-[[1S,1R*-(cyclohexylmethyl)-2S*,3R*-dihydroxy-5-methylhexyl] amino] -2S*-methyl-3-oxopropyl]-nalidixylamide;
   N-[3-[[1S,1R*-(cyclohexylmethyl)-2S*,3R*-dihydroxy-5-methylhexyl] amino] -2S*-methyl-3-oxopropyl]-N-nalidixylamide;
   N-[3-[[1S,1R*-(cyclohexylmethyl)-2S*,3R*-dihydroxy-5-methylhexyl]amino]-2S*-methyl-3-oxopropyl]-(R,S)-α-methyl-hydrocinnamide;
   N-[3-[[1S,1R*-(cyclohexylmethyl)-2S*,3R*-dihydroxy-5-methylhexyl]amino]-2S*-methyl-3-oxopropyl]N-methyl-1H-indole-2-carboxamide;
   N-[3-[[1S,1R*-(cyclohexylmethyl)-2S*,3R*-dihydroxy-5-methylhexyl]amino]-2S*-methyl-3-oxopropyl]-5-fluoro-1H-indole-3-carboxamide;
   N-[3-([1S,1R*-(cyclohexylmethyl)-2S*,3R*-dihydroxy-5-methylhexyl]amino]-2S*-methyl-3-oxopropyl]-5-fluoro-1H-indole-2-carboxamide
   N-[3-[[1S,1R*-(cyclohexylmethyl)-2S*,3R*-dihydroxy-5-methylhexyl]amino]-2S*-methyl-3-oxopropyl]-1H-indole-3-carboxamide;
   N-[3-[[1S,1R*-(cyclohexylmethyl)-2S*,3R*-dihydroxy-5-methylhexyl]amino]-2S*-methyl-3-oxopropyl]-1H-indole-3-acetamide;
   N-[3-[[1S,1R*-(cyclohexylmethyl)-2S*,3R*-dihydroxy-5-methylhexyl]amino]-2S*-methyl-3-oxopropyl]-1H-indole-3-propanamide;
   N-[3-[[1S,1R*-(cyclohexylmethyl)-2S*,3R*-dihydroxy-5-methylhexyl]amino]-2S*-methyl-3-oxopropyl]-7-methoxy-benzofuran-2-carboxamide;
   N-[3-[[1S,1R*-(cyclohexylmethyl)-2S*,3R*-dihydroxy-5-methyhexyl]amino]-2S*-methyl-3-oxopropyl]-4-oxo-4H-1-benzopyran-3-carboxamide;
   N-[3-[[1S,1R*-(cyclohexylmethyl)-2S*,3R*-dihydroxy-5-methylhexyl]amino]-2S*-methyl-3-oxopropyl]-4-oxo-4H-1-benzopyran-2-carboxamide; and
   N-[3-[[1S,1R*-(cyclohexylmethyl)-2S*,3R*-dihydroxy-5-methylhexyl]amino]-2S*-methyl-3-oxopropyl]-7-chloro-benzofuran-2-carboxamide.
Representative compounds of Formula (III):
   (3S,4S)-N-[(tert-Butyloxy)carbonyl]-4-amino-3-acetoxy-5-phenylpentene;
   (2R,3S)-N-[(tert-Butyloxy)carbonyl]-3-amino-2-acetoxy-4-phenylbutanal;
   (2S,3R,4S)-N-[(tert-Butyloxy)carbonyl]-2-amino-1-phenyl-3,4-dihydroxy-6-methylheptane;
   (2S,3R,4S)-N-[(tert-Butyloxy)carbonyl]-2-amino-1-cyclohexyl-3,4-dihydroxy-6-methylheptane;
   (2S,3R)-Boc-L-Leucinamide of 2-amino-1-cyclohexyl-3,4-dihydroxybutane;
   D,L-Monomethyl-2-(1-naphthylmethyl)succinate;
   D,L-Methyl-3-(N-morpholinocarbonyl)-2-(1-naphthylmethyl) propionate;
   D,L-3-(N-Morpholinocarbonyl)-2-(1-naphthylmethyl)propionic acid; and
   3-(N-morpholinocarbonyl)-2-(R,S)-(1-naphthylmethyl)propionyl-L-leucinamide of (2S,3R)-2-amino-1-cyclohexyl-3,4-dihydroxybutane.
Representative compounds of Formula (IV)
   N-[1R*-[[[1S,1R*-(cyclohexylmethyl)-2S*,3R*-dihydroxy-5-methylhexyl]amino]carbonyl]-3-butynyl]-αR*-[[[2-(dimethylamino)ethyl]sulfonyl]methyl]benzenepropanamide;
   N-[2-[[1S,-1R*-(cyclohexylmethyl)-2S*,3R* -dihydroxy-5-methylhexyl]amino]-1R*-(cyclopropylmethyl)-2-oxoethyl]-α-[[[2-(dimethylamino)ethyl]sulfonyl]methyl] benzenepropanamide;
   N-[2-[[1S,1R*-(cyclohexylmethyl)-2S*,3R*-dihydroxy-5-methylhexyl]amino]-1R * -(cycloproylmethyl)-2-oxoethyl]-α-[[[2-(dimethylamino)ethyl]thio]methyl]benzenepropanamide;
   N-[2-[[1S,1R*-(cyclohexylmethyl)-2S*,3R*-dihydroxy-5-methylhexyl]amino]-1R * -(cyclopropylmethyl)-2-oxoethyl]-α-[[[2-(diethylamino)ethyl]sulfonyl] methyl]benzenepropanamide; 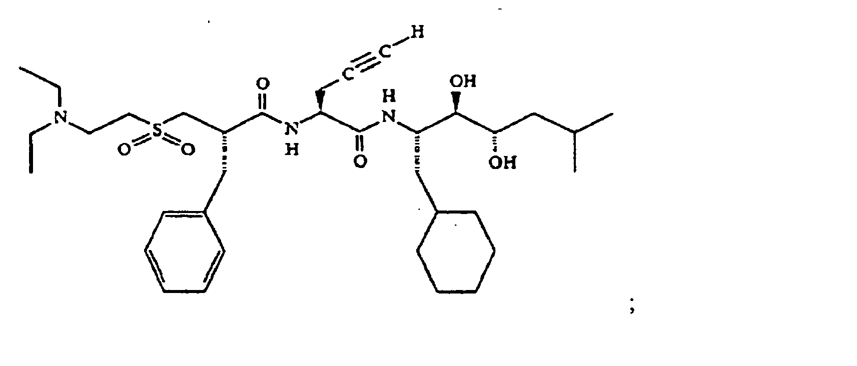 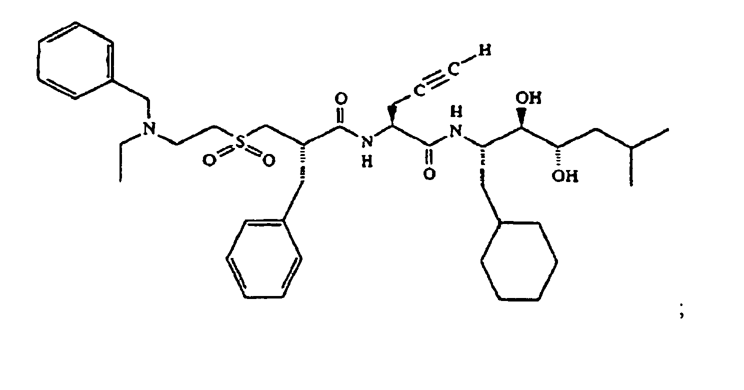 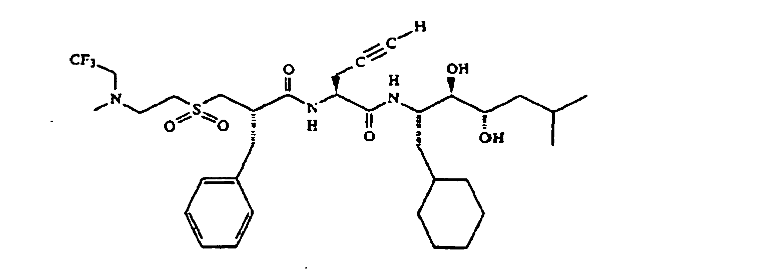 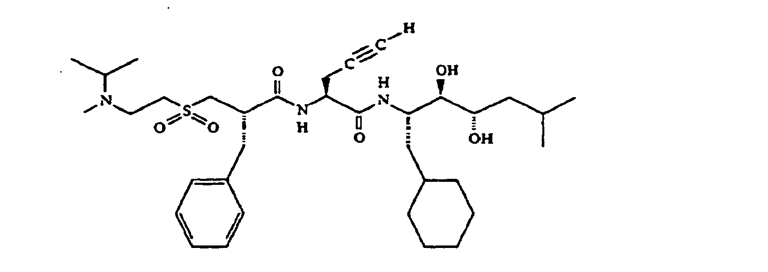 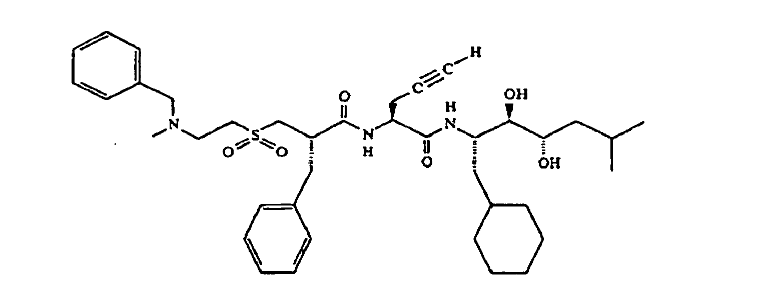 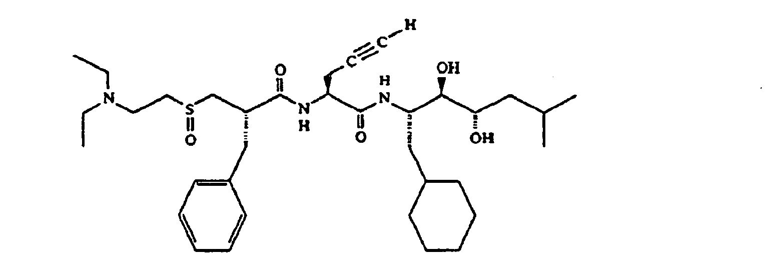 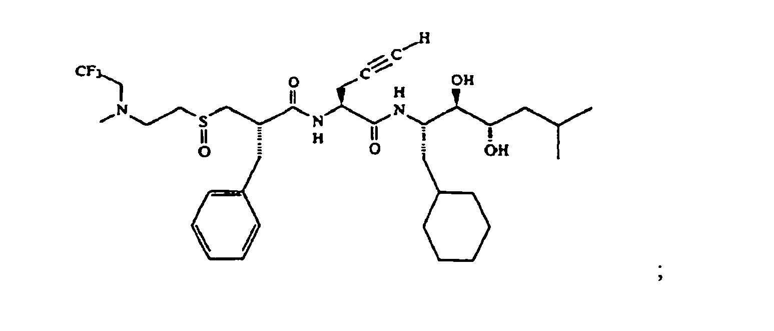 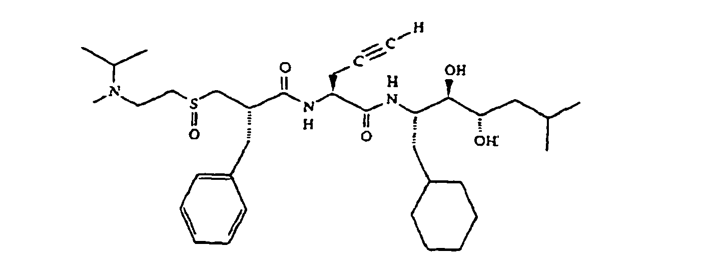 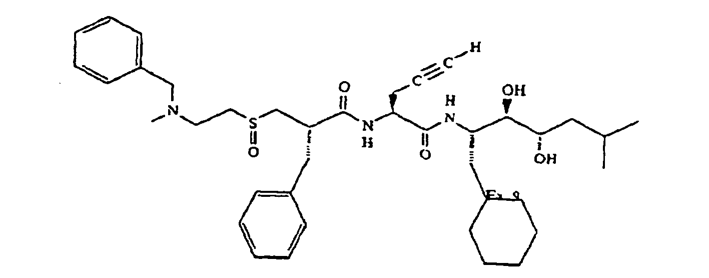 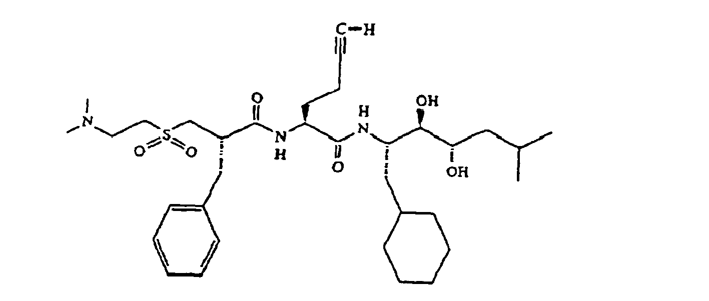 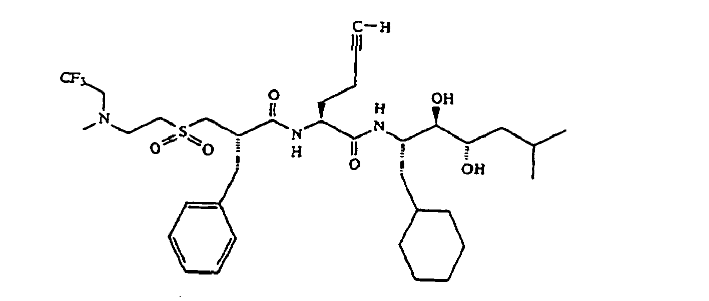 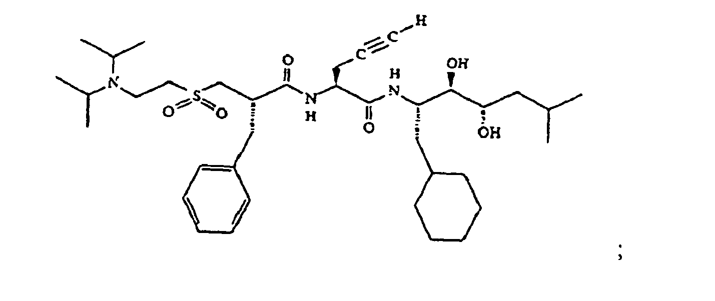 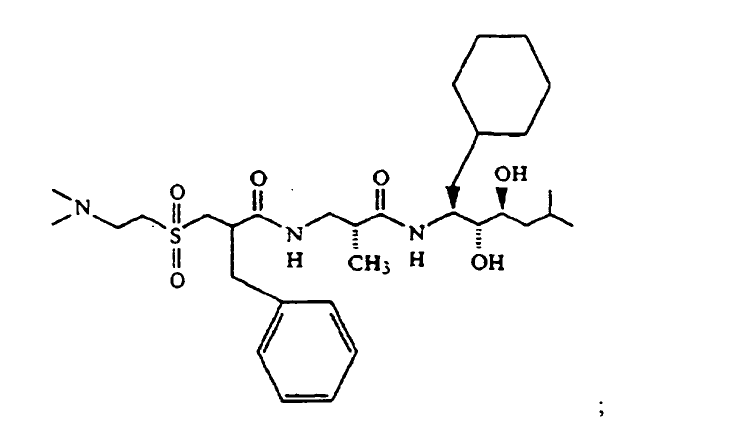 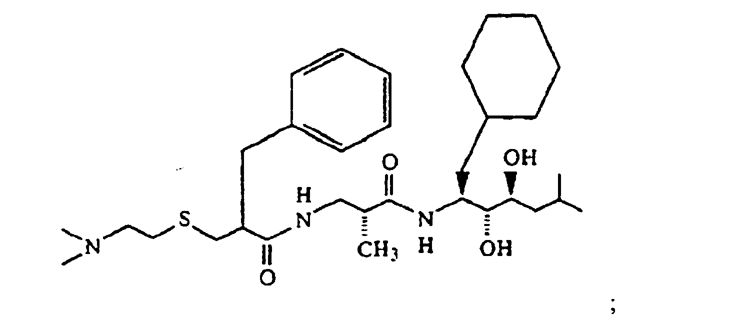 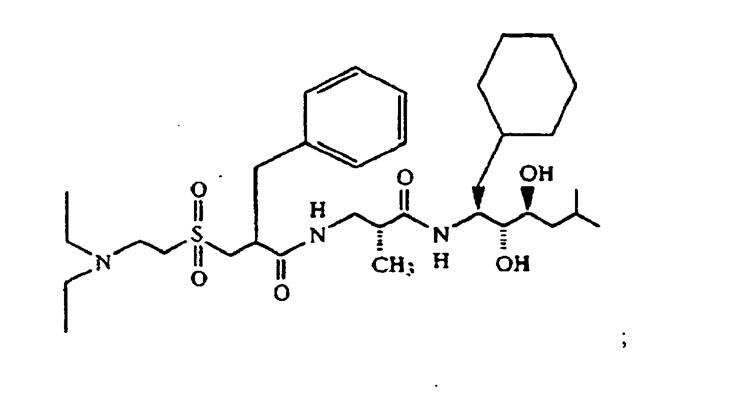 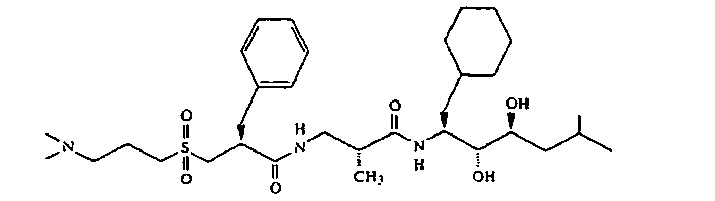 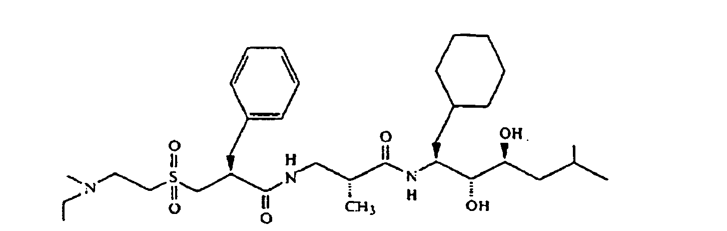 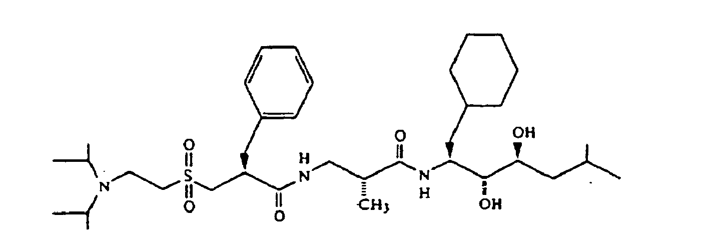 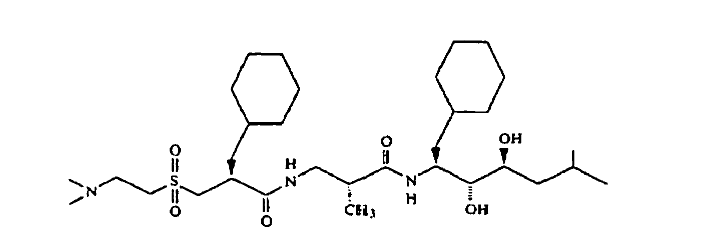 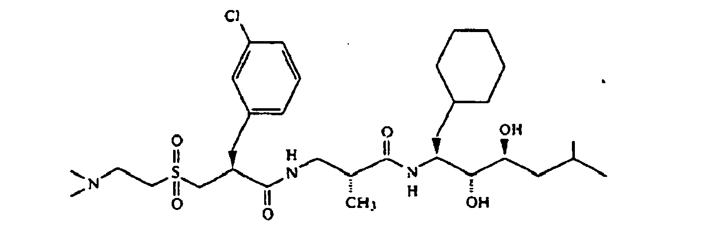 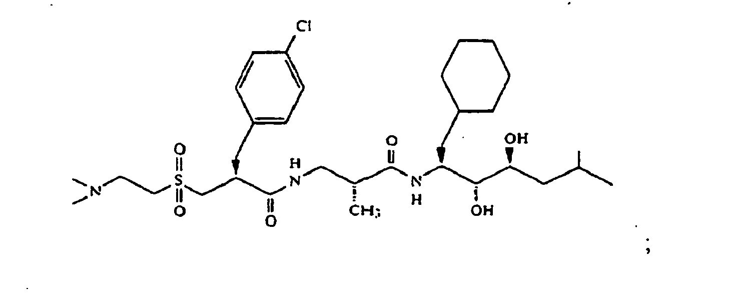 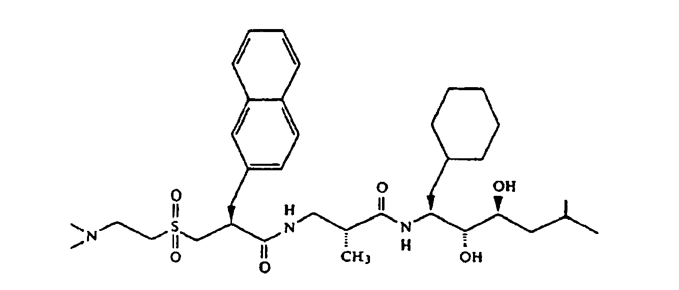 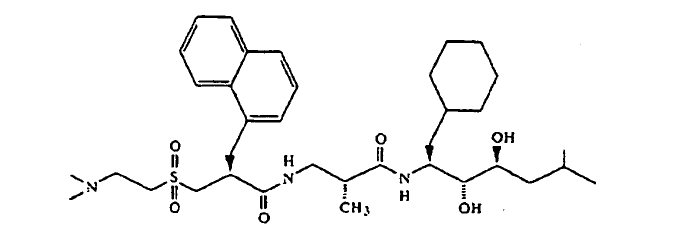 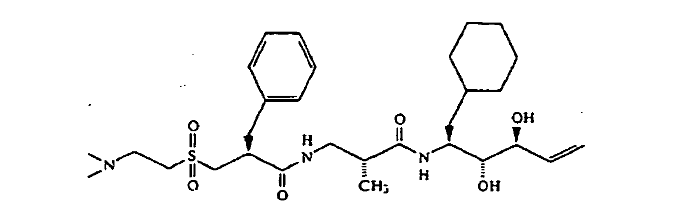 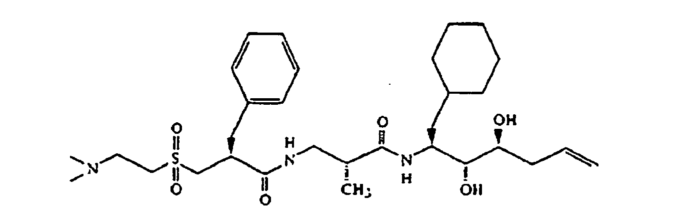 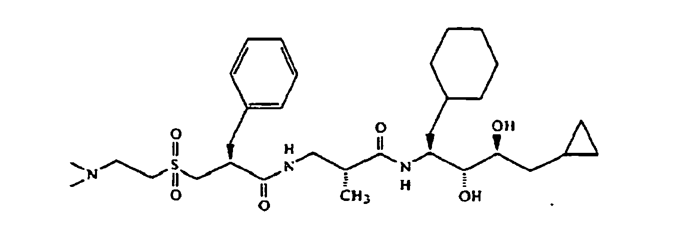 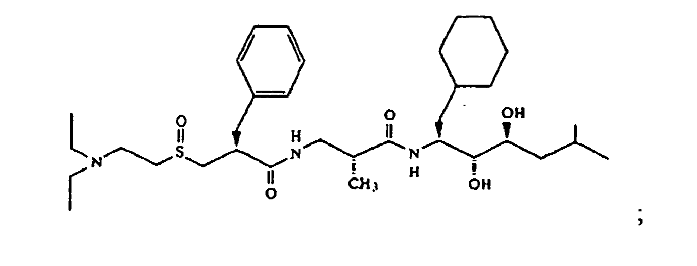 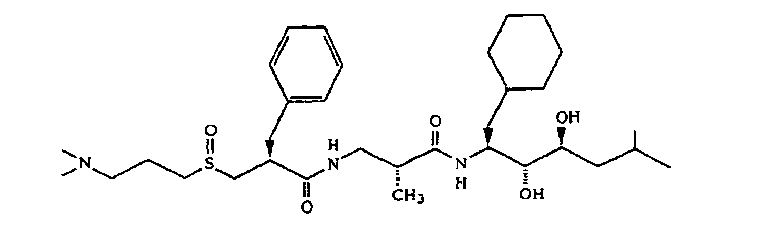 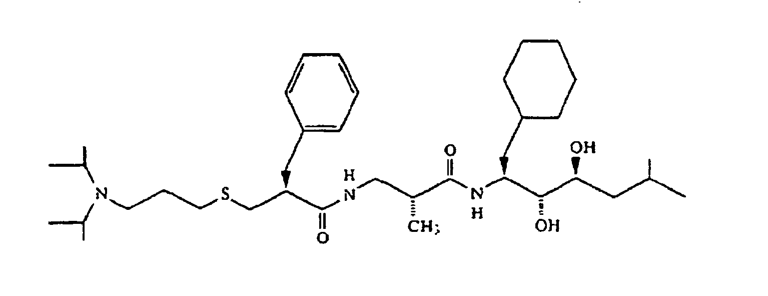 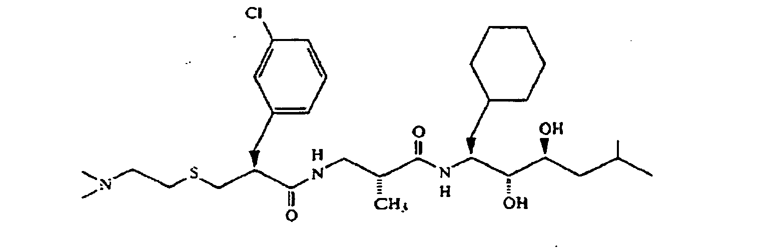 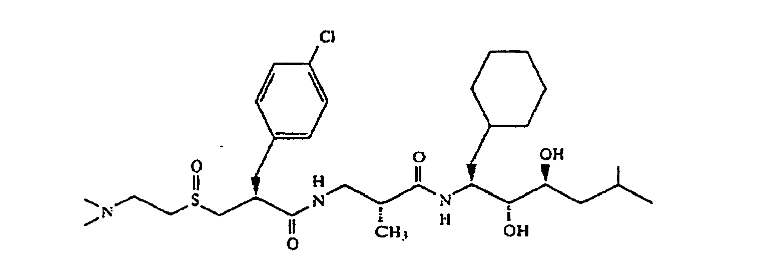 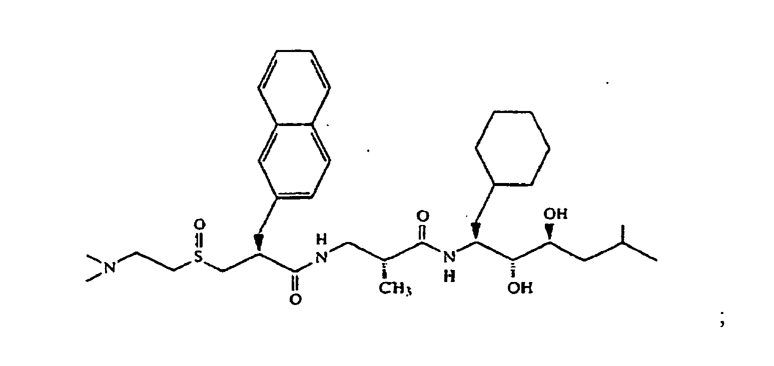 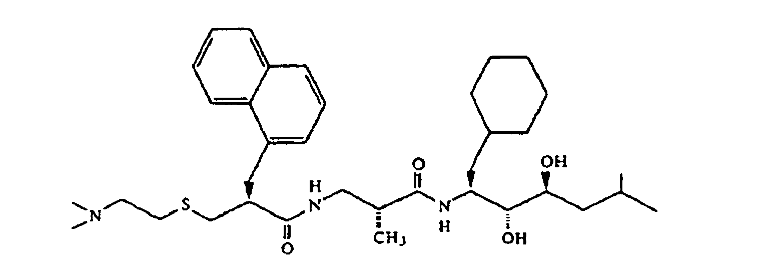 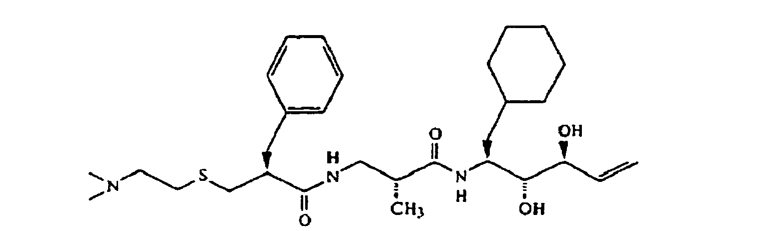 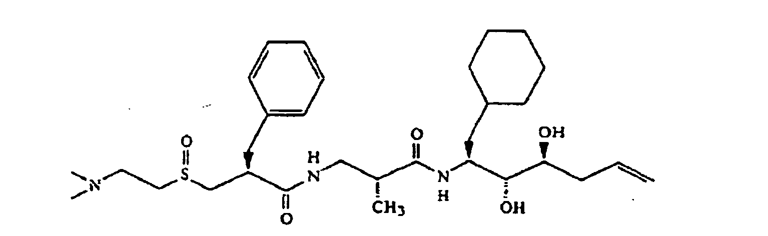 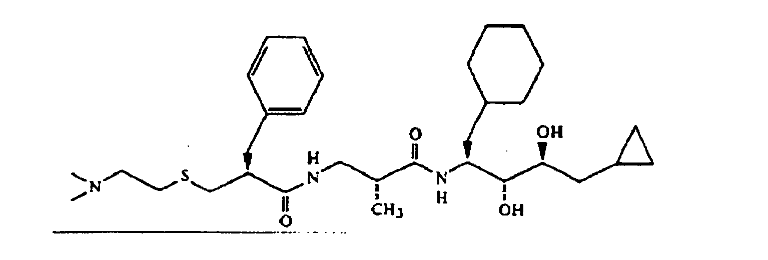 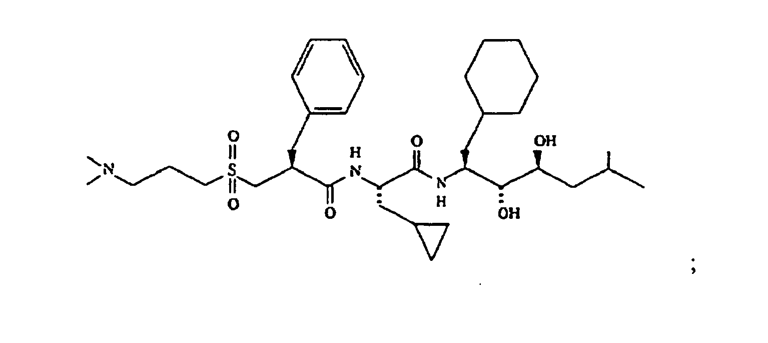 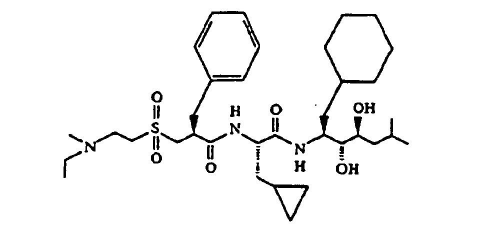 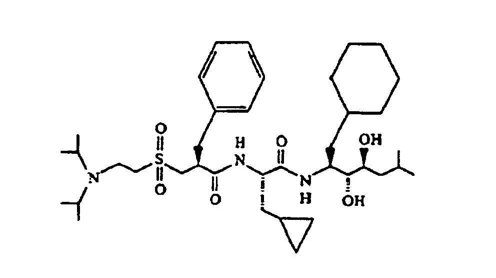 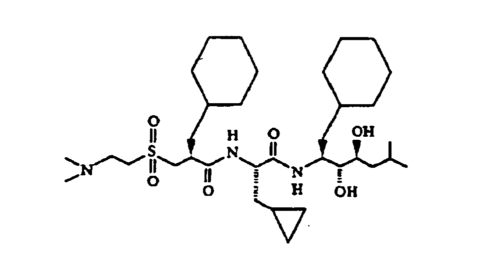 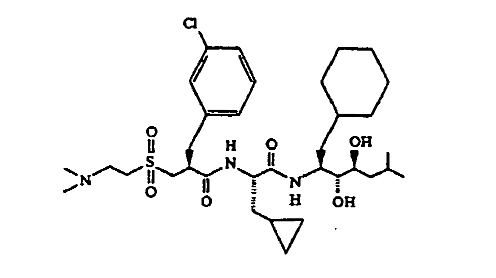 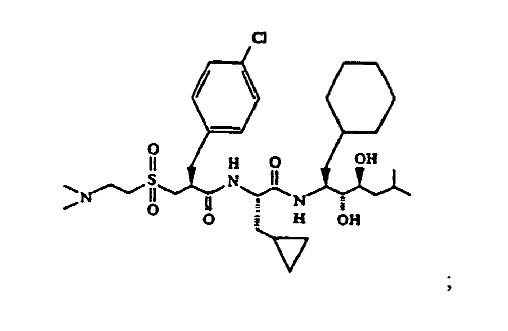 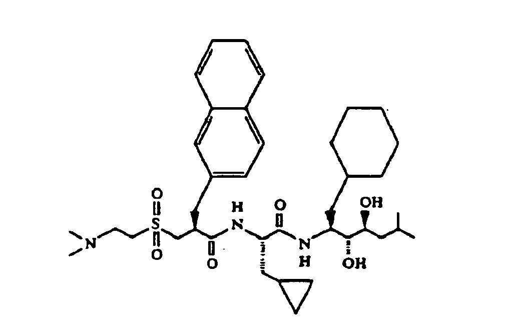 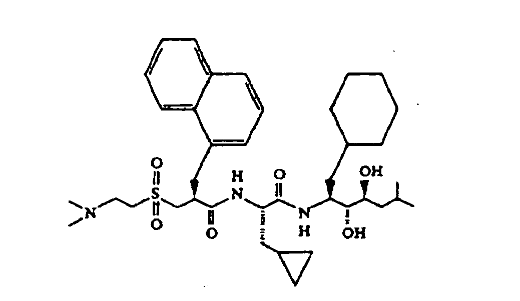 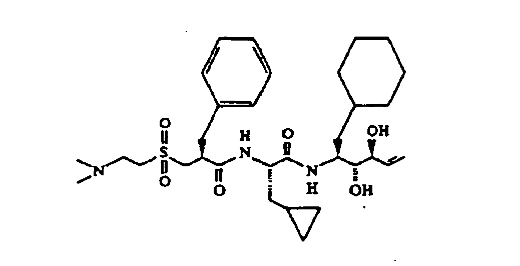 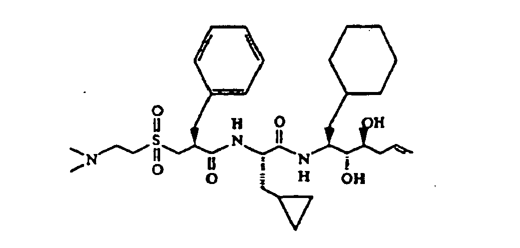 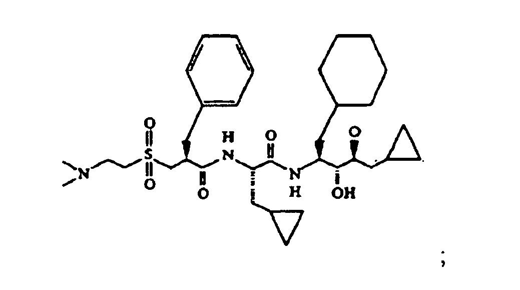 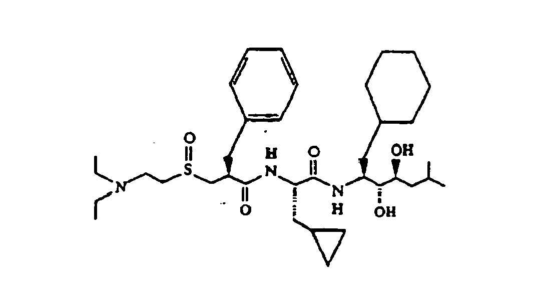 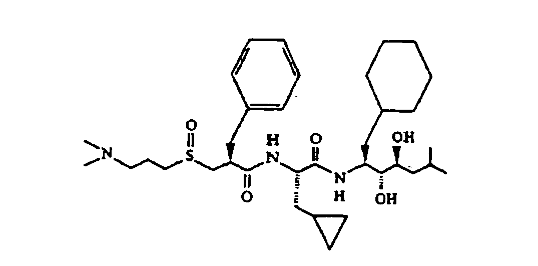 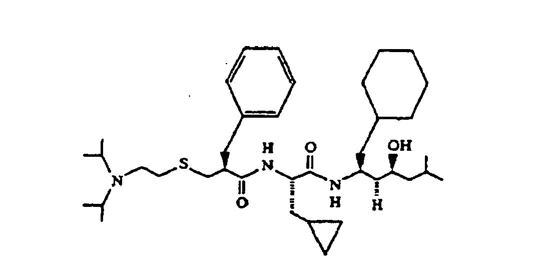 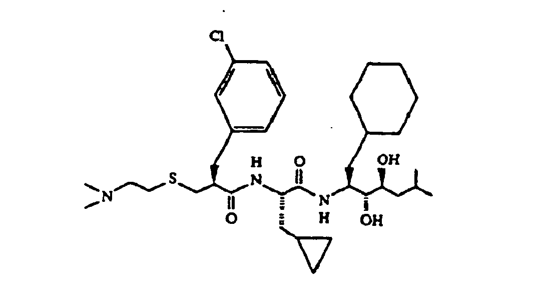 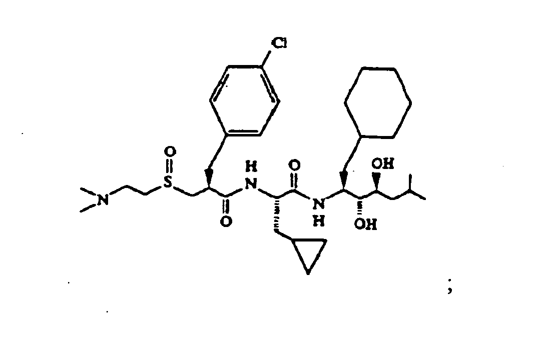 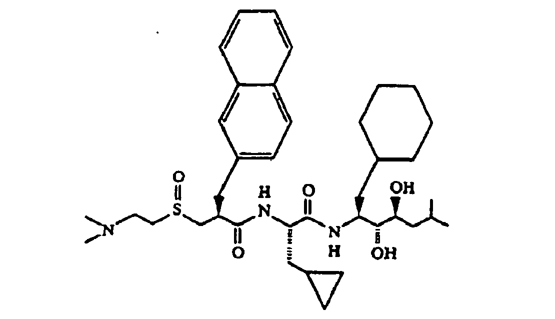 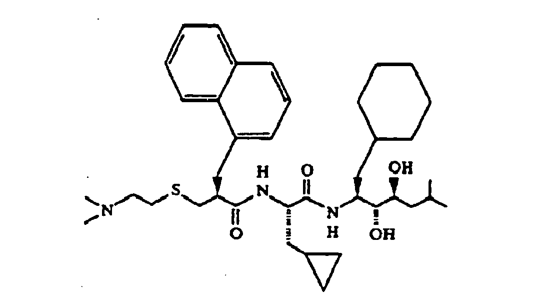 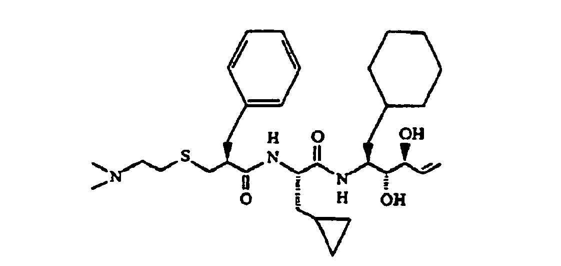 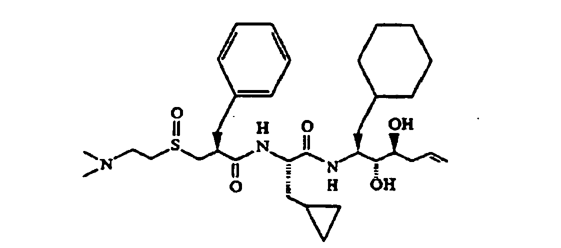 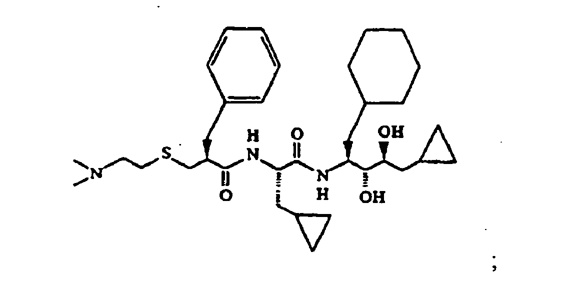 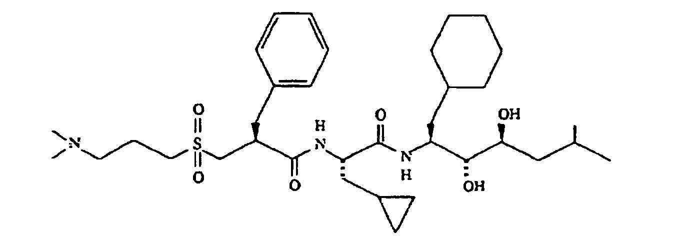 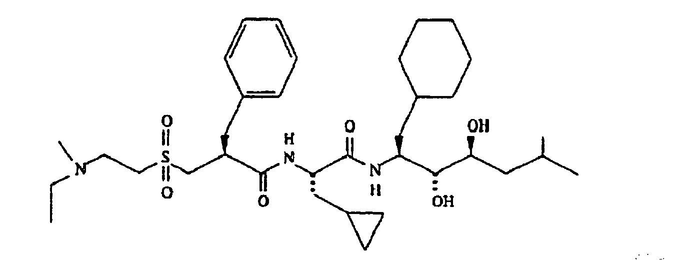 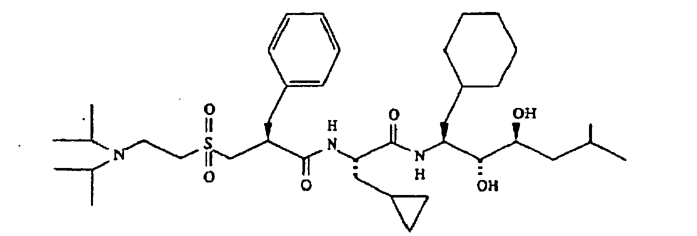 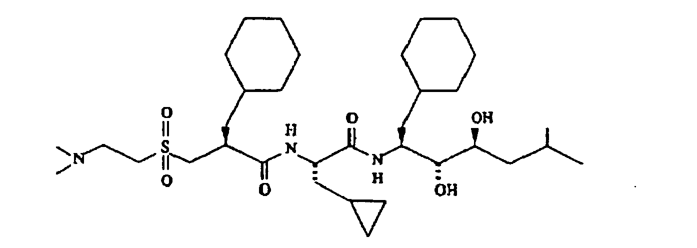 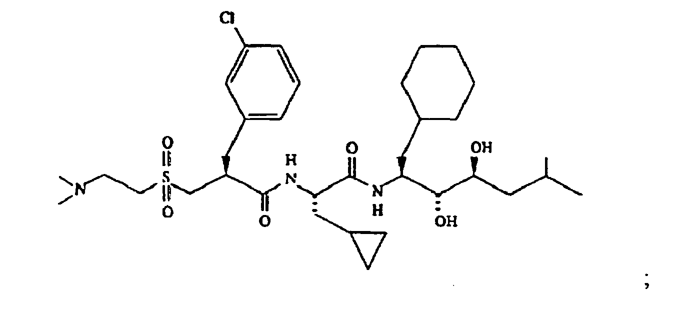 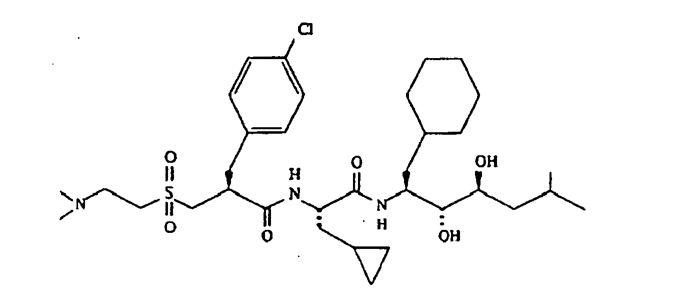 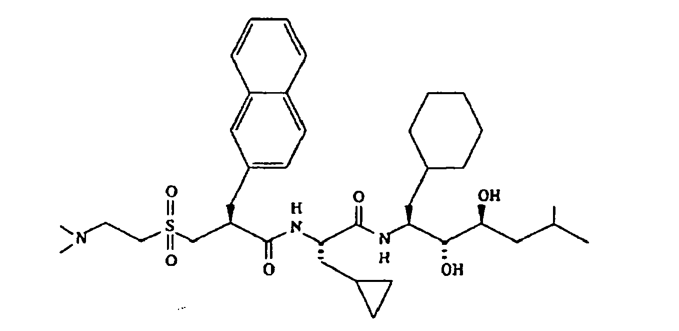 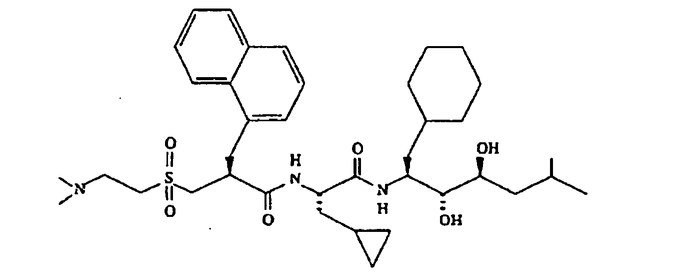 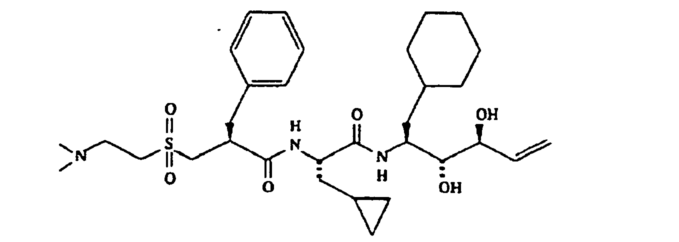 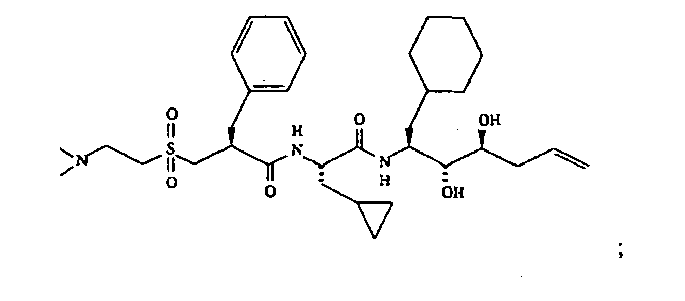 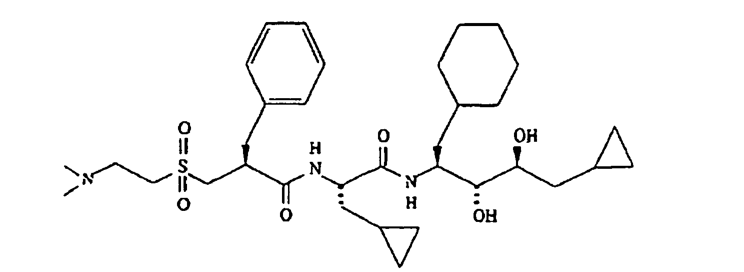 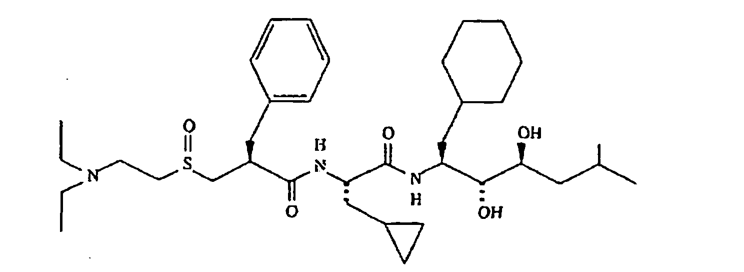 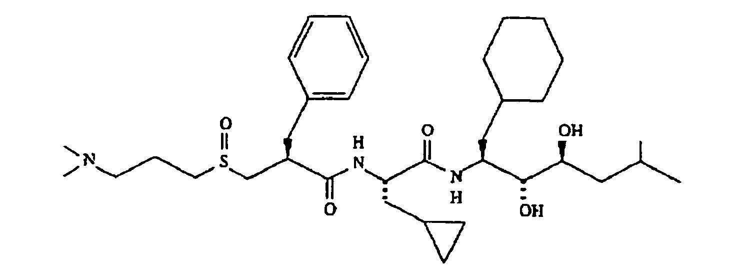 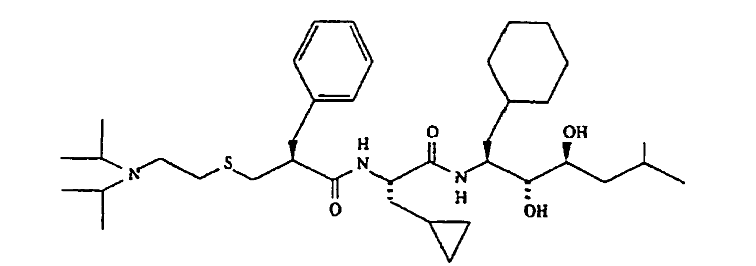 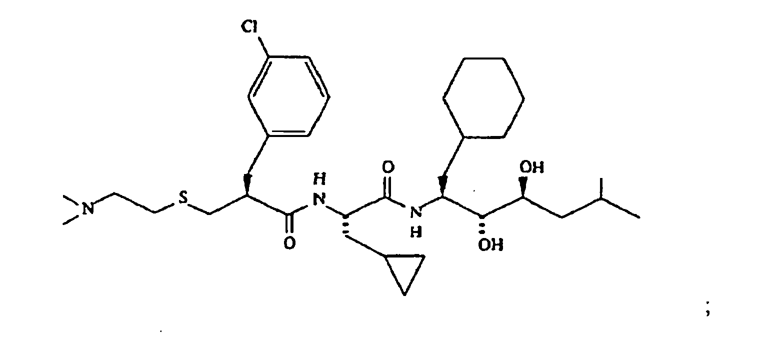 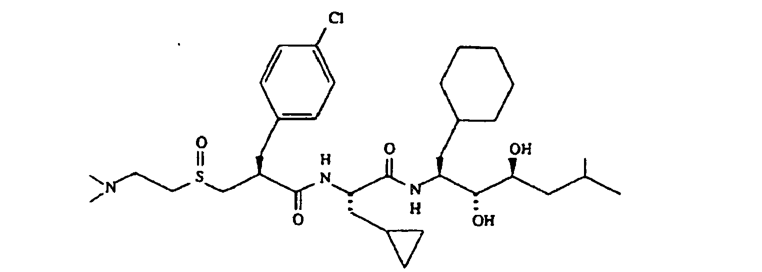 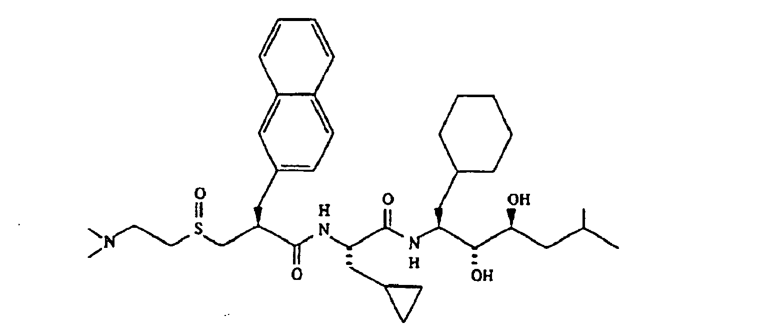 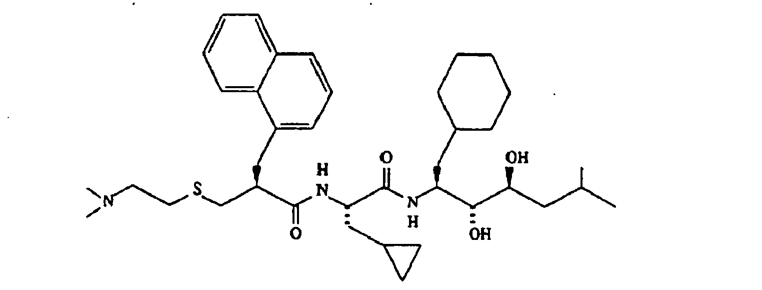 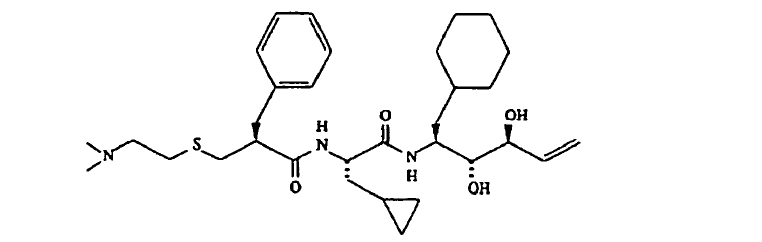 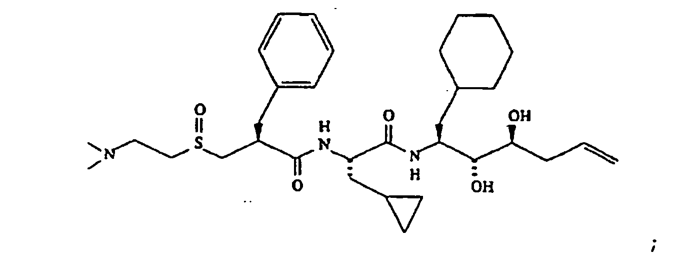 and 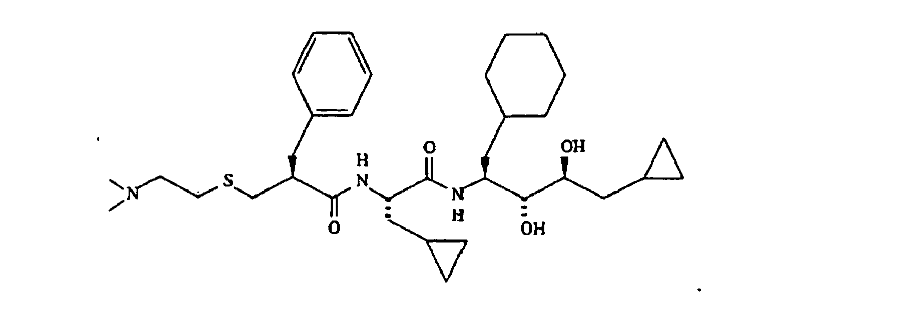
Representative compounds of Formula (V):
   N¹-[1R*-[[[1S,1R*-(cyclohexylmethyl)-2S*,3R*-dihydroxy-5-methylhexyl]amino]carbonyl]-3-butynyl]-N⁴⁻methyl-2S*-(phenylmethyl) -N⁴- [2- (1-piperidinyl)ethyl]butanediamide;
   N¹-[1R*-[[[1S,1R*-(cyclohexylmethyl)-2S*,3R*-dihydroxy-5-methylhexyl] amino] carbonyl] -3-butynyl]-N⁴-[2-(1,3-dihydro-2H-isoindol-2-yl)ethyl]-N⁴-methyl-2S*-(phenylmethyl)butanediamide;
   N¹-[1R*-[[[1S,1R*-(cyclohexylmethyl)-2S*,3R*-dihydroxy-5-methylhexyl]amino]carbonyl]-3-butynyl]-N⁴⁻methyl-2S*-(phenylmethyl)-N⁴-[2-(N-3-azabicyclo[3.2.2]nonanyl)ethyl]butanediamide;
   (2S)-2-Benzyl-3-(1-methylpiperazin-4-ylsulfonyl)propionyl-L-propargylglycyl amide of (2S,3R,4S)-2-amino-1-cyclohexyl-3,4-dihydroxy-6-methylheptane;
   N¹-[1R*-[[[1S,1R*-(cyclohexylmethyl)-2S*,3R*-dihydroxy-5-methylhexyl]amino]carbonyl]-3-butynyl]-N⁴⁻methyl-N4-[2-(4-morpholinyl)ethyl]-2S*-(phenylmethyl)butanediamide;
   N¹-[1R*-[[[lS,lR*-(cyclohexylmethyl)-2S*,3R*-dihydroxy-5-methylhexyl]amino]carbonyl]-3-butynyl]-N⁴-[2-(1H-imidazol-1-yl)ethyl]-N⁴-methyl-2S*-(phenylmethyl)butanediamide;
   N¹-[1R*- [[[1S,1R*-(cyclohexylmethyl)-2S*, 3R*-dihydroxy-5-methylhexyl]amino]carbonyl]-3-butynyl]-N⁴⁻methyl-2S*-(phenylmethyl)-N⁴-[2-(2-pyridinyl) ethyl]butanediamide; 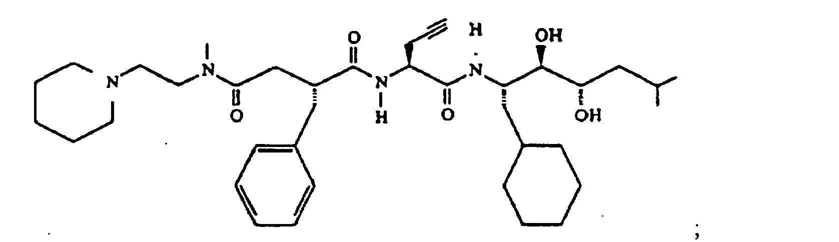 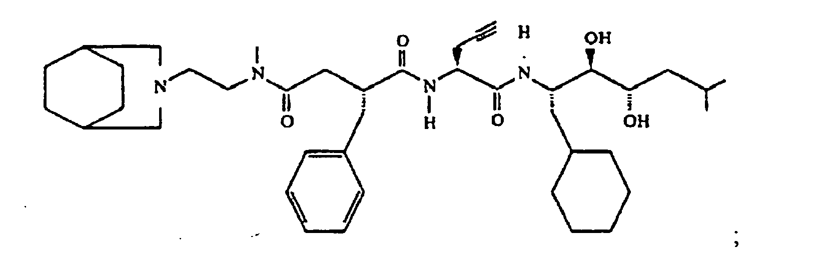 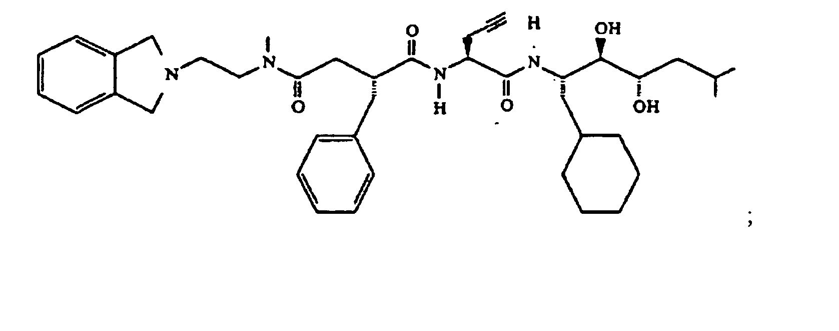 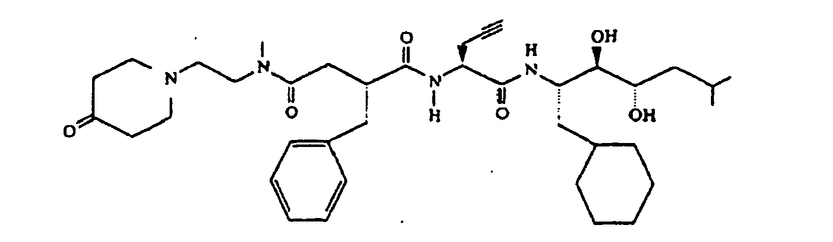 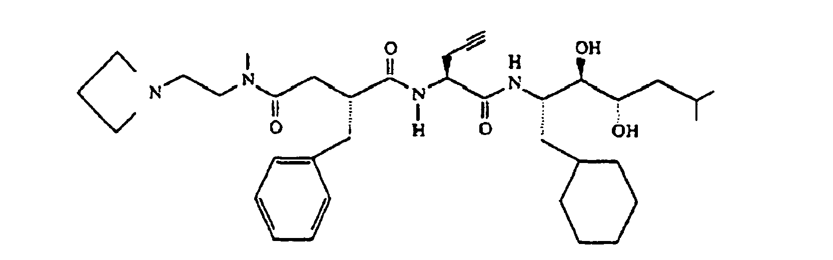 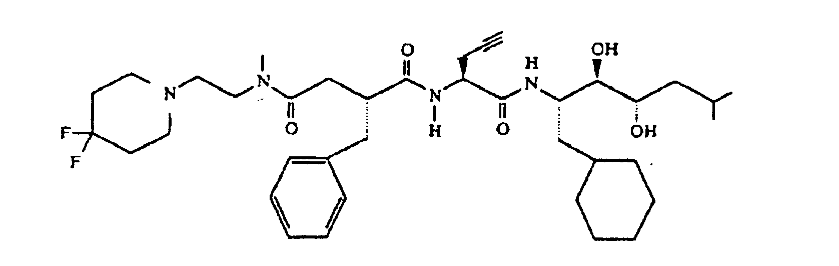 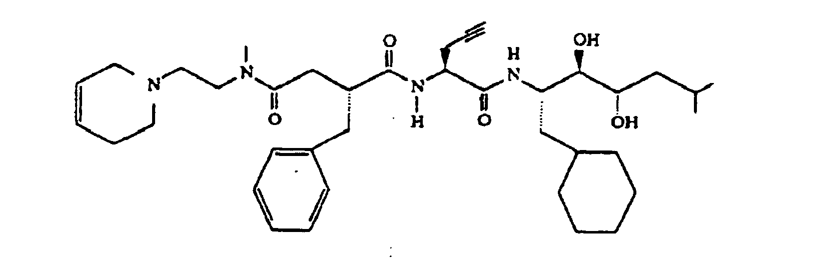 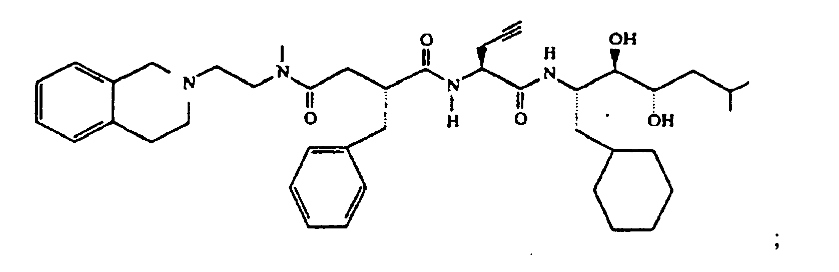 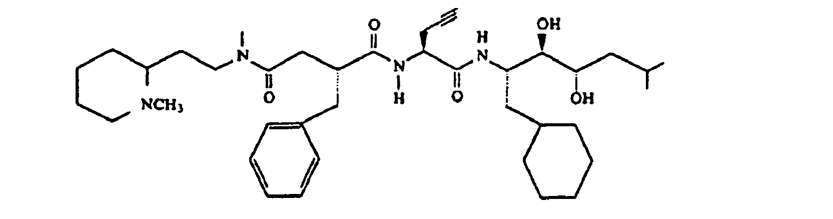 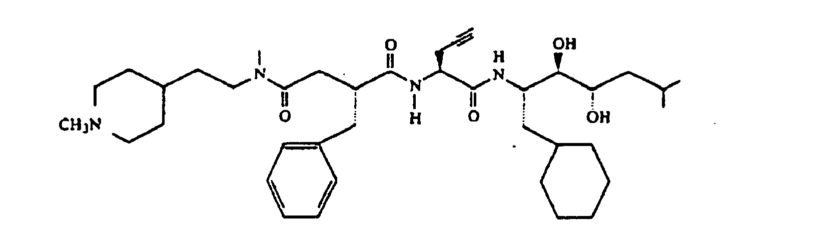 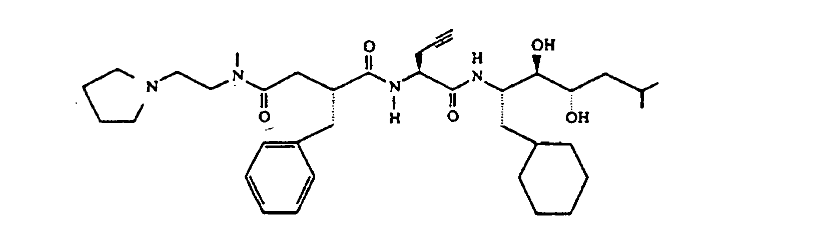 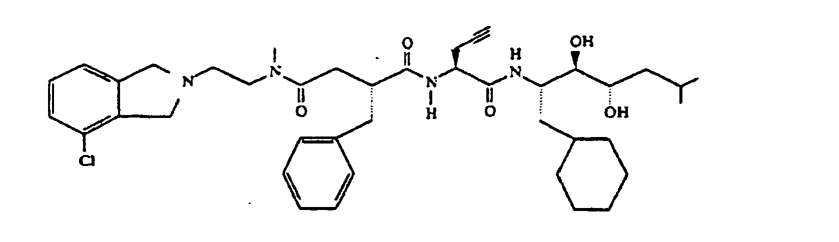 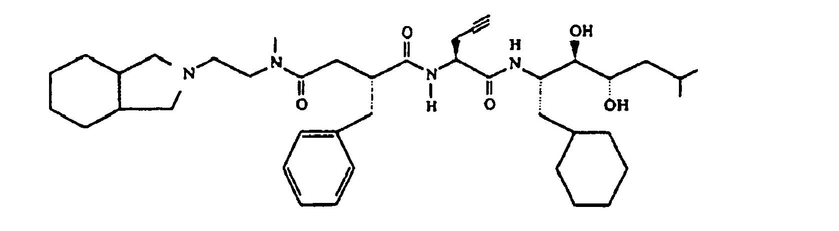 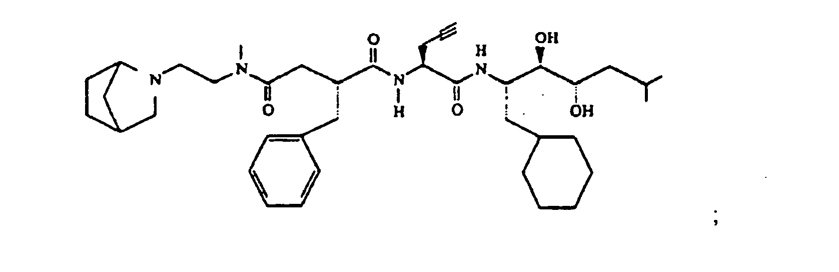 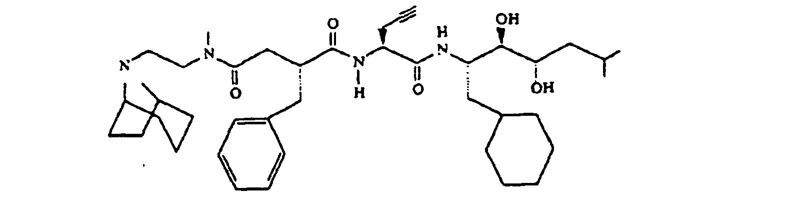 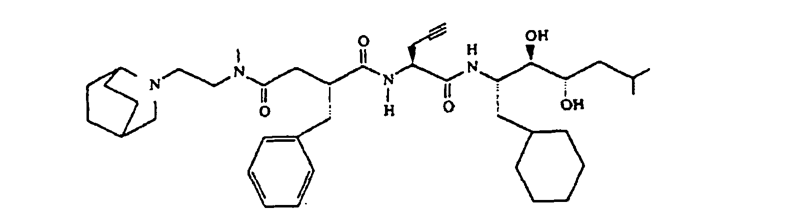 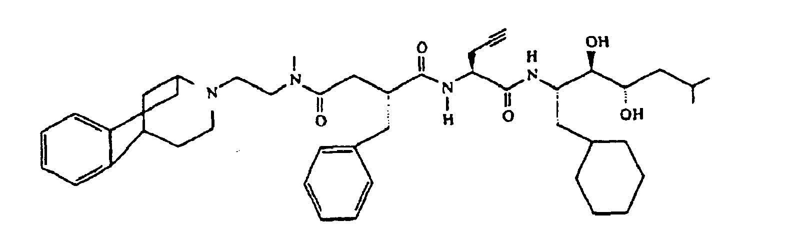 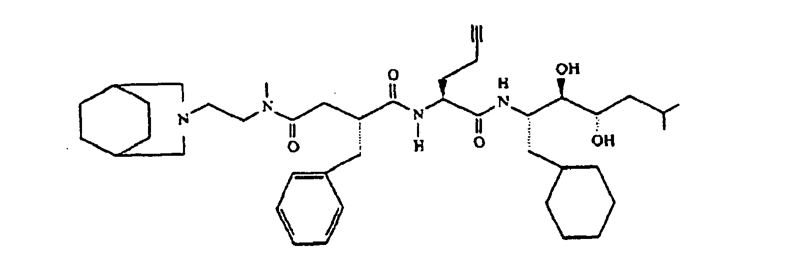 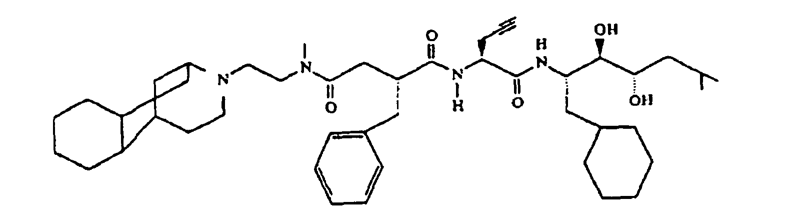 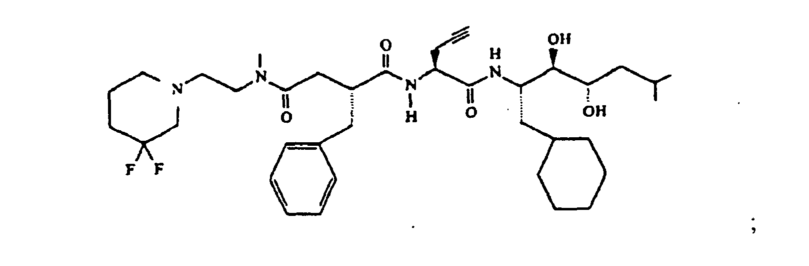 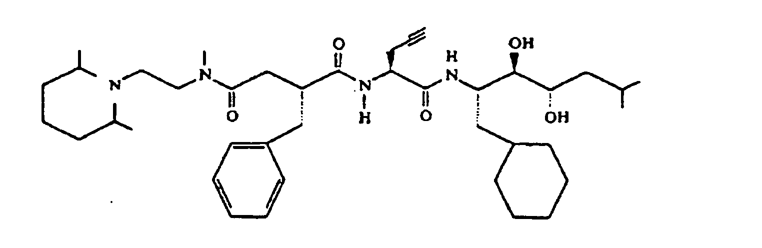 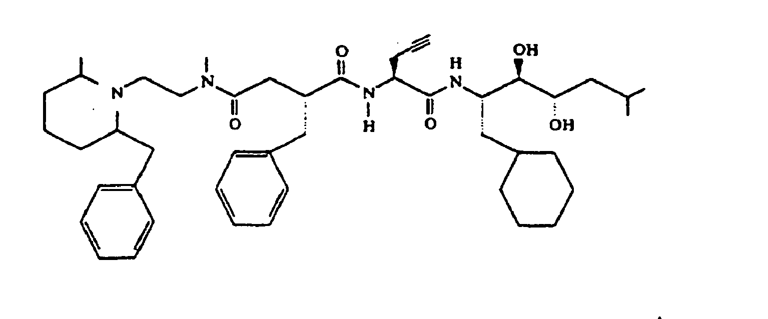 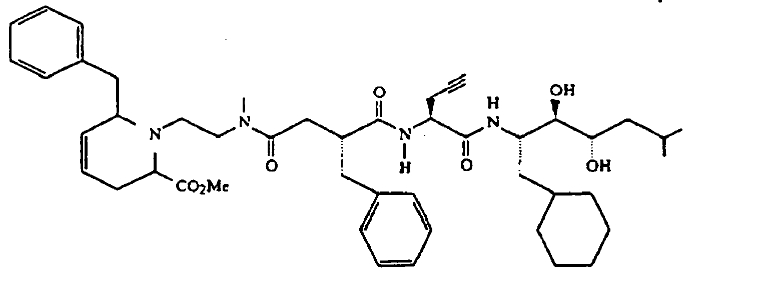 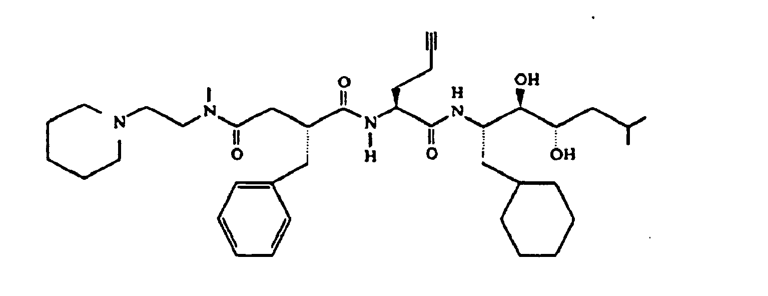 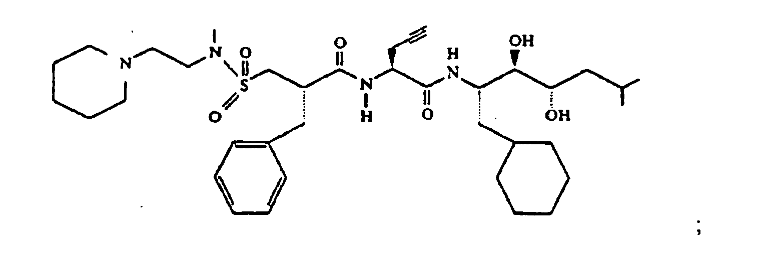 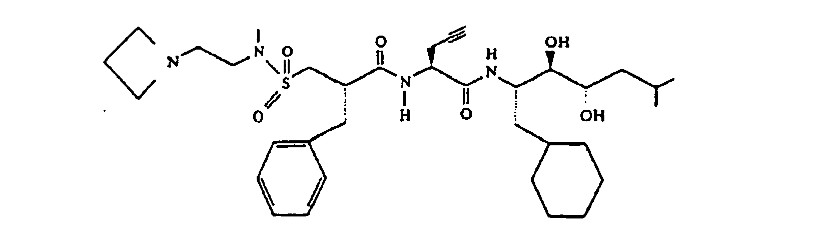 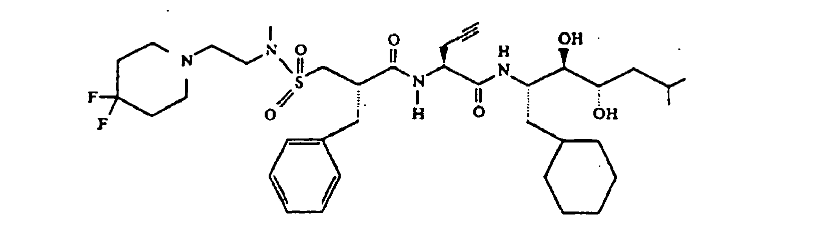 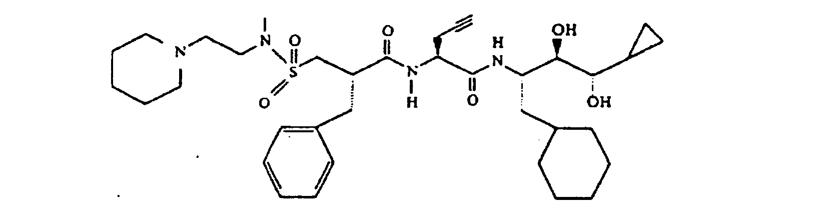 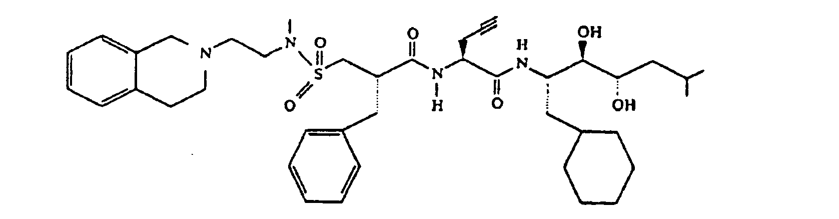 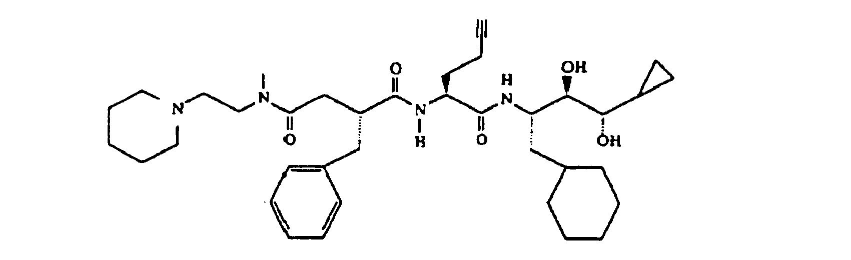 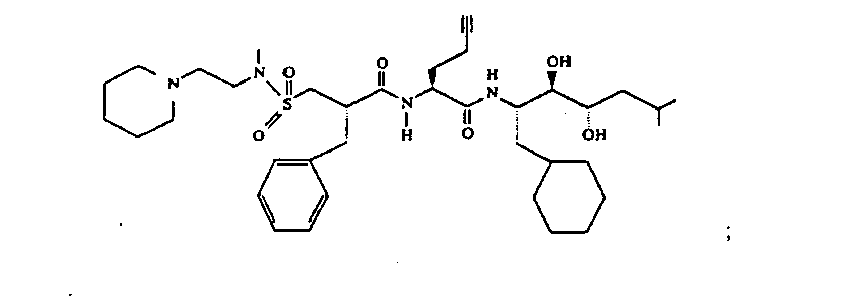 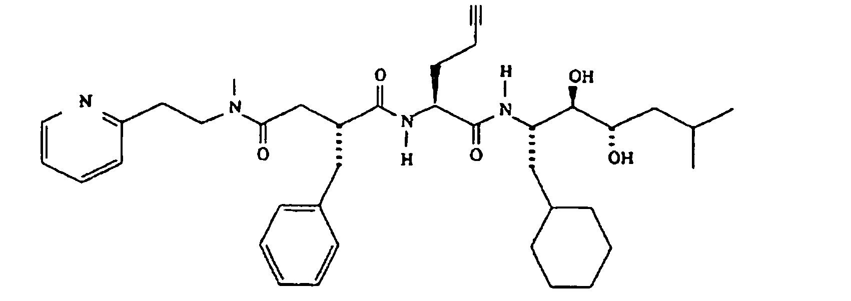 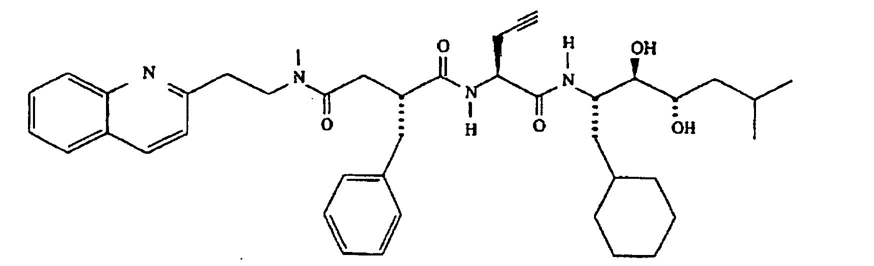 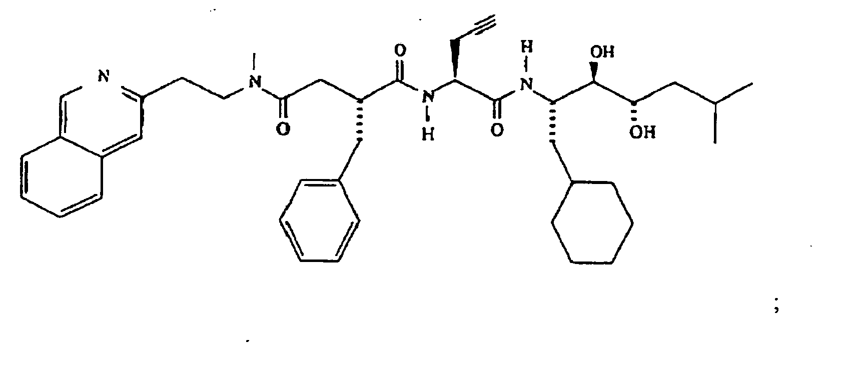 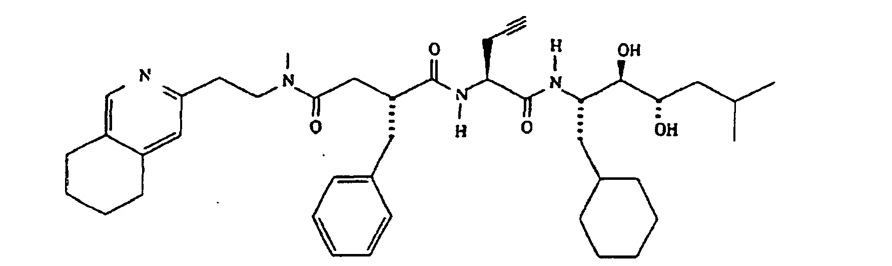 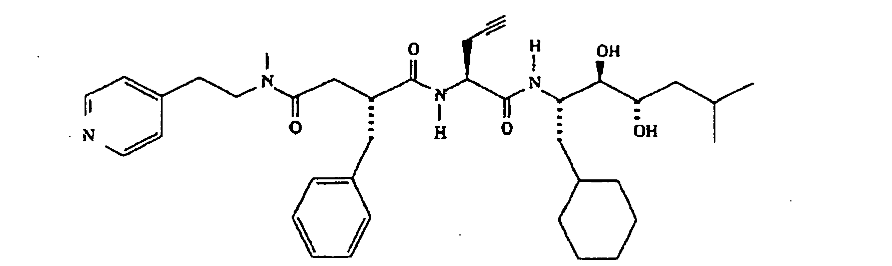 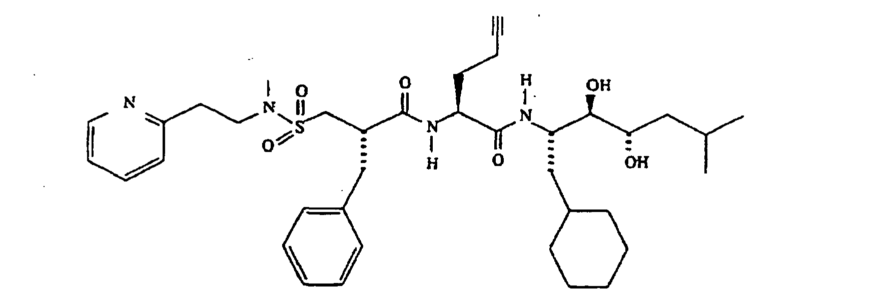 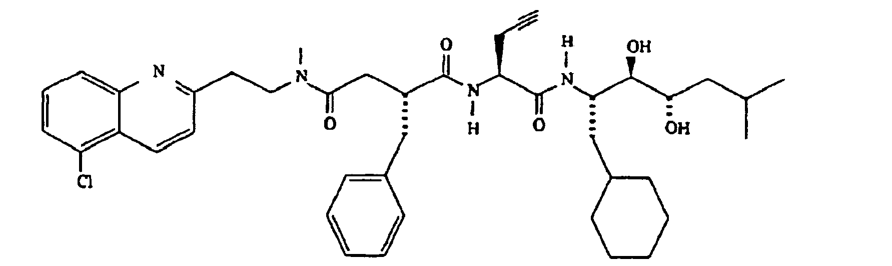 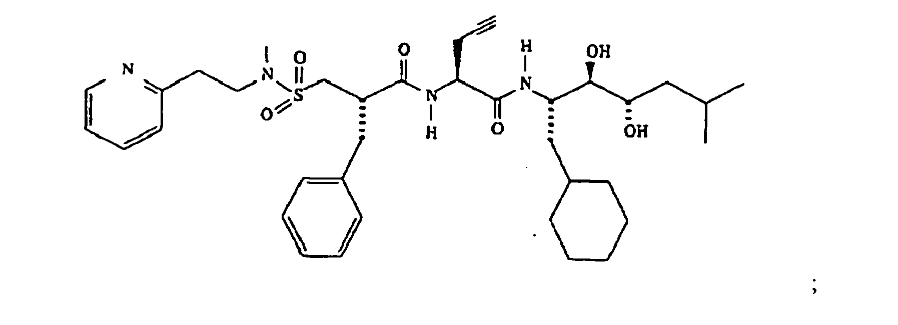 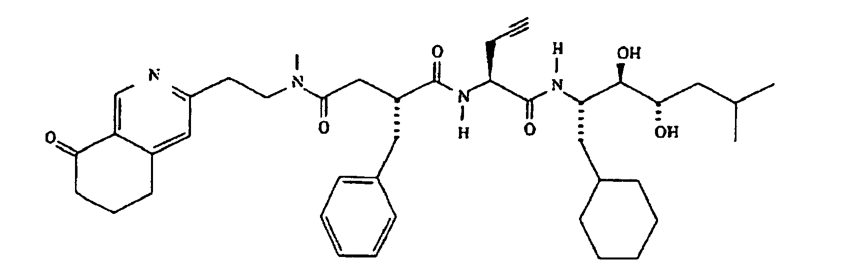 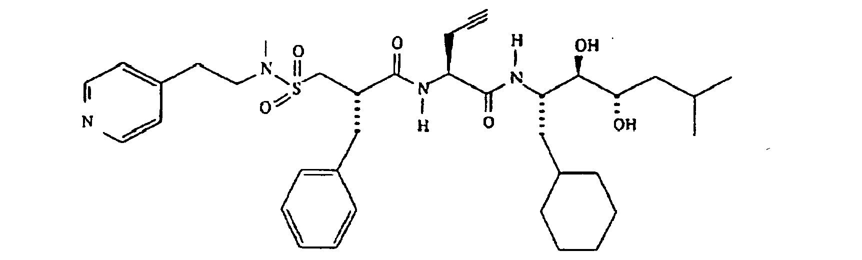 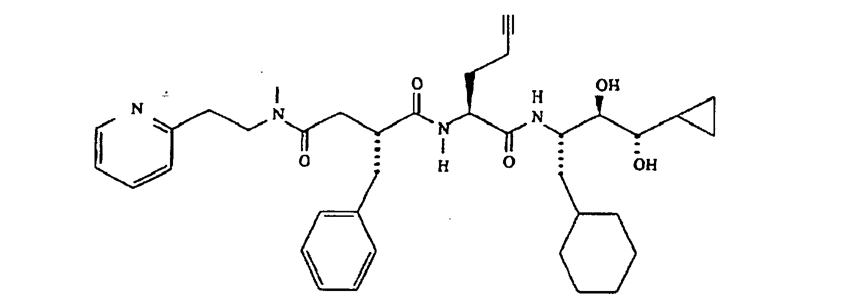 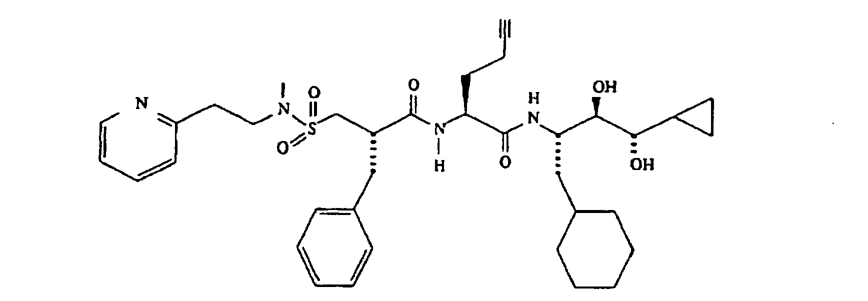 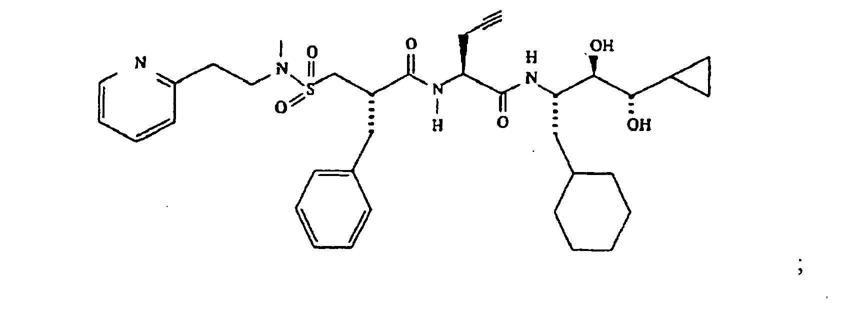 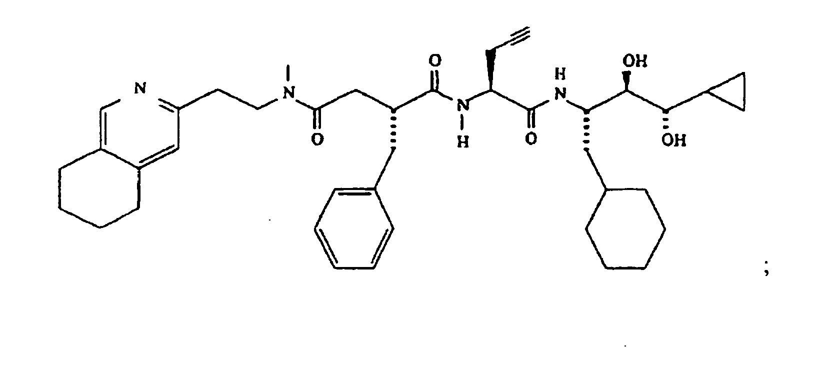 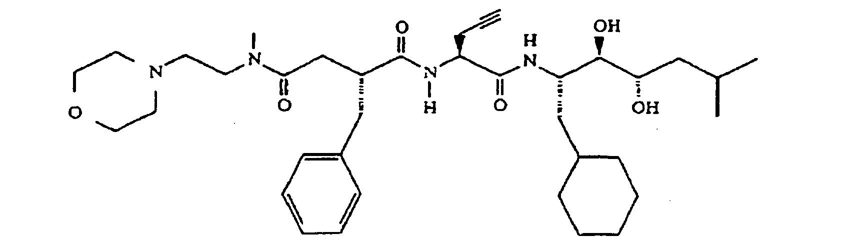 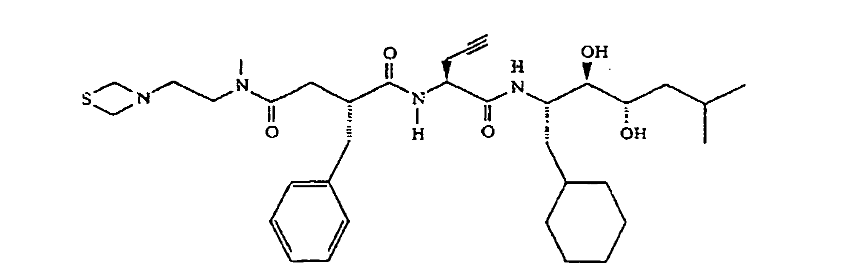 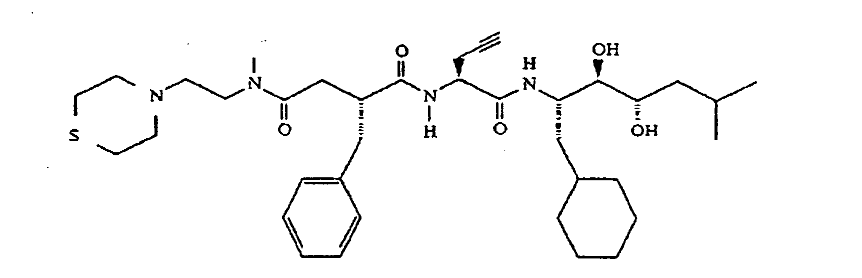 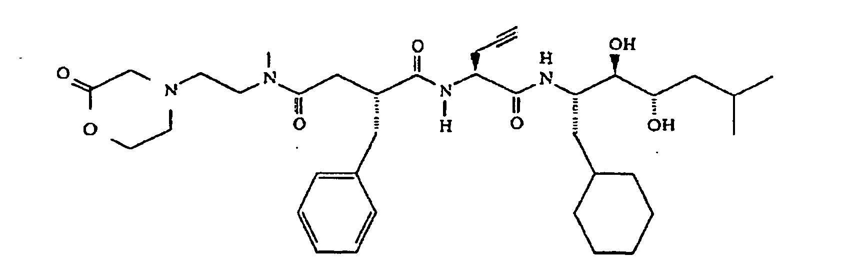 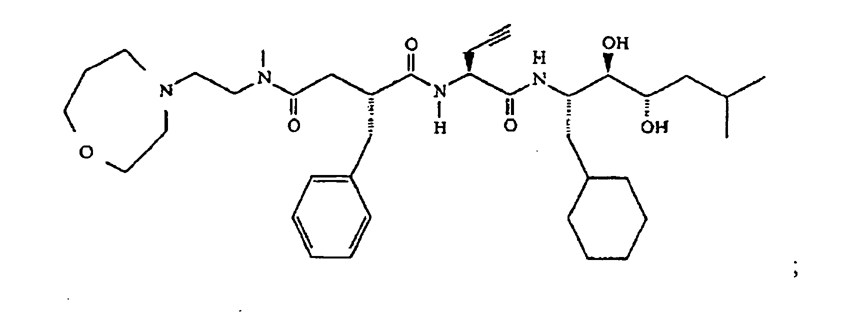 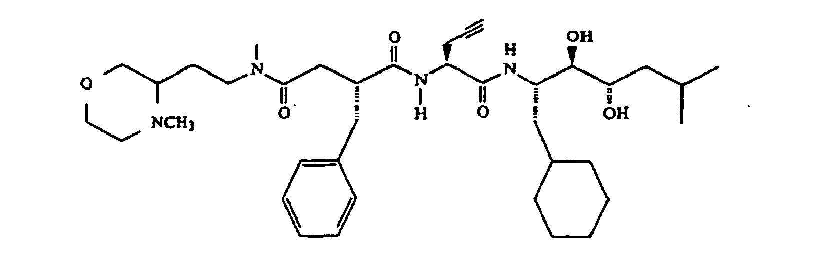 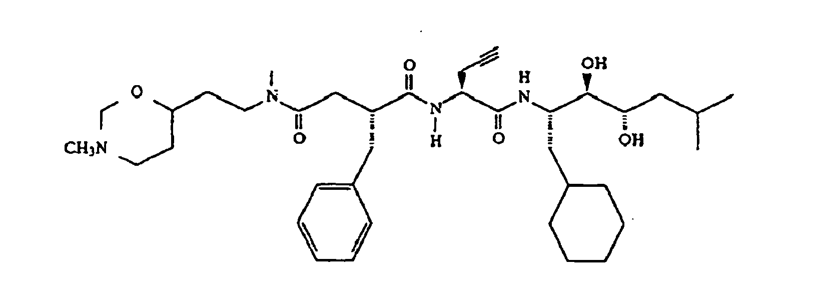 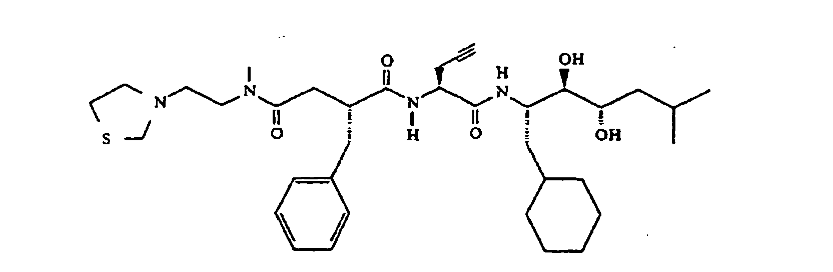 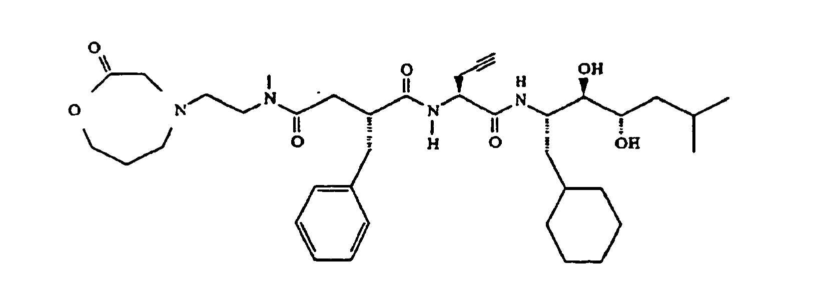 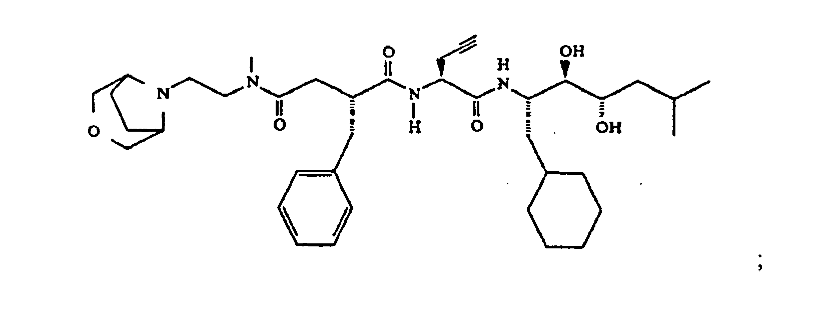 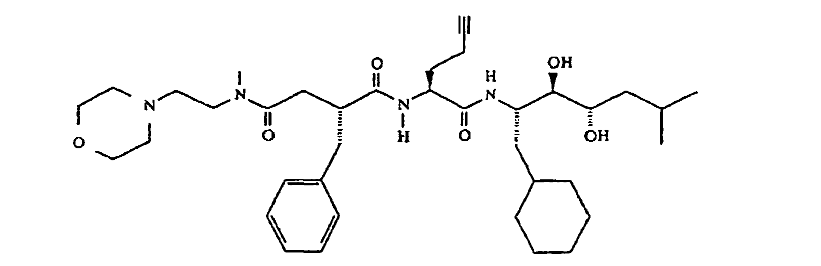 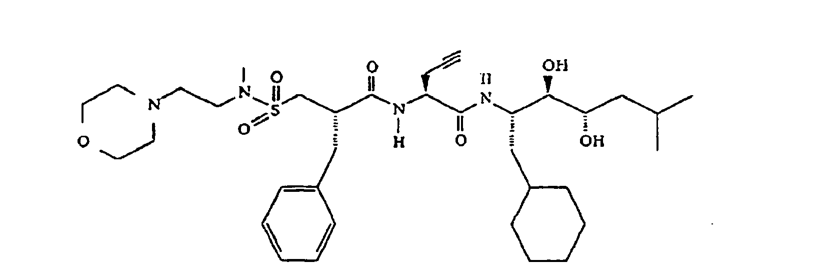 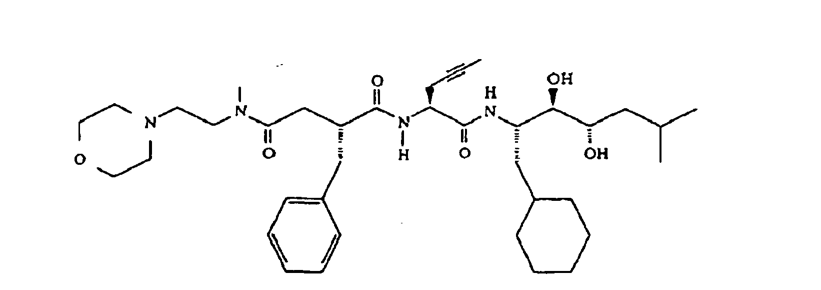 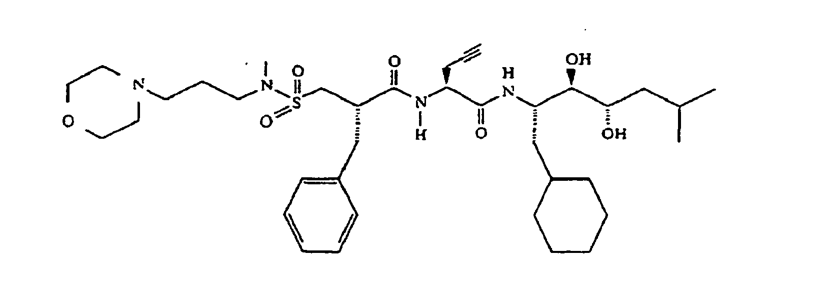 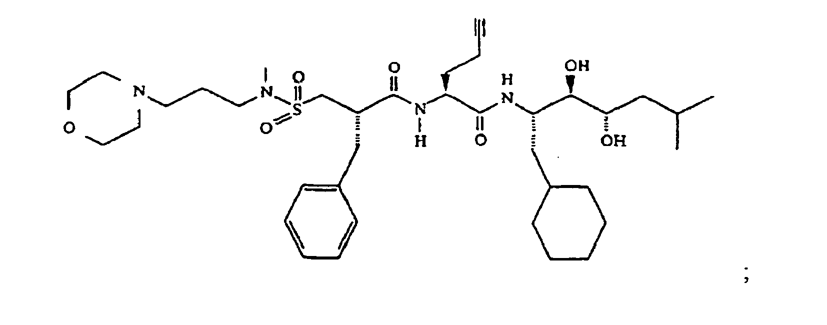 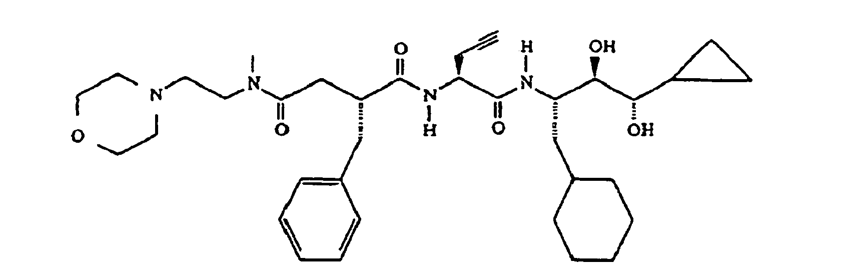 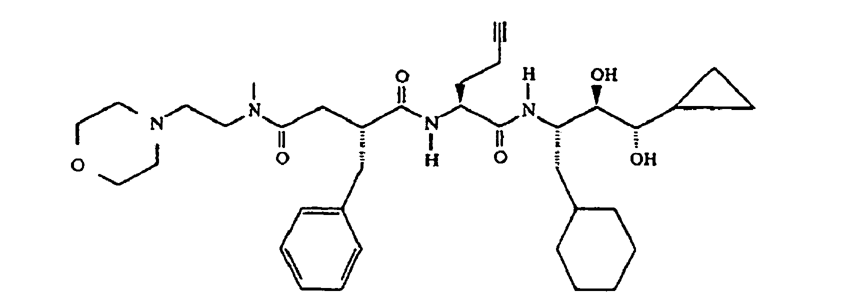 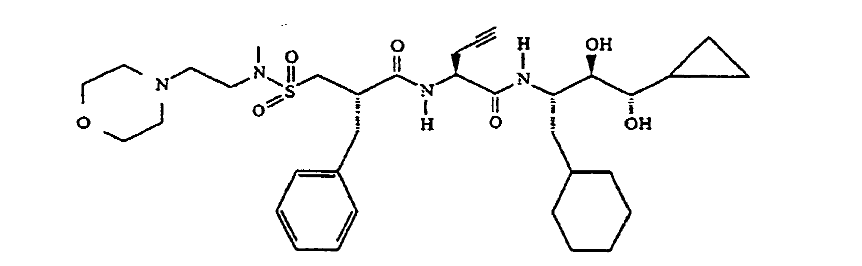 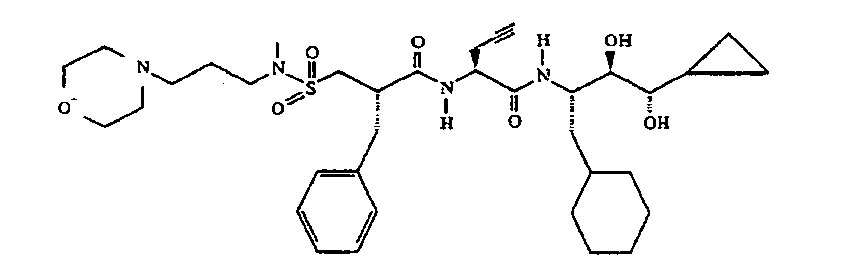 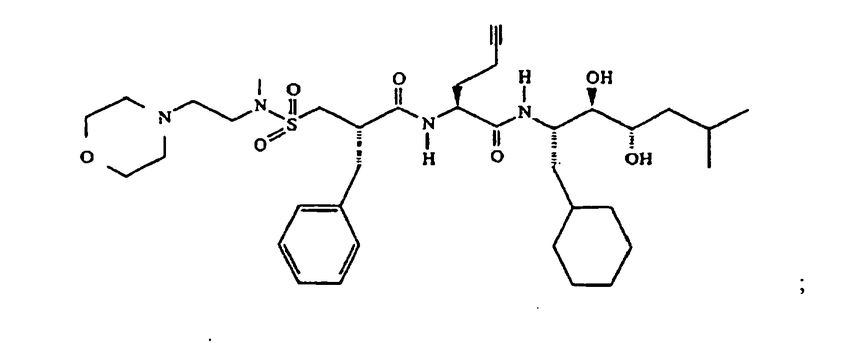 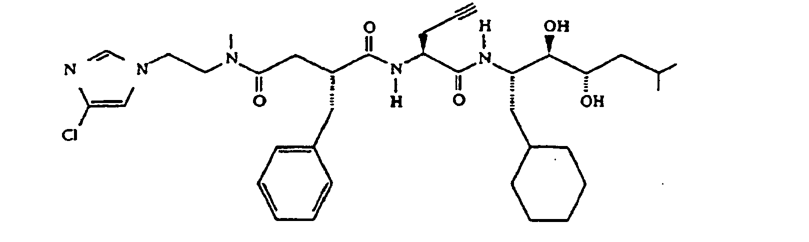 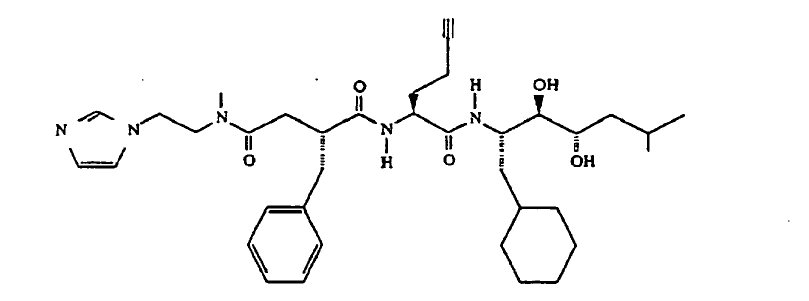 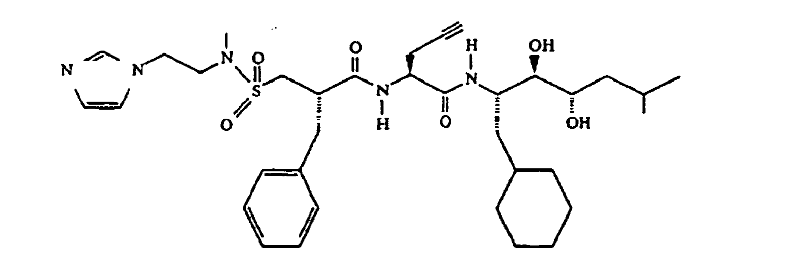 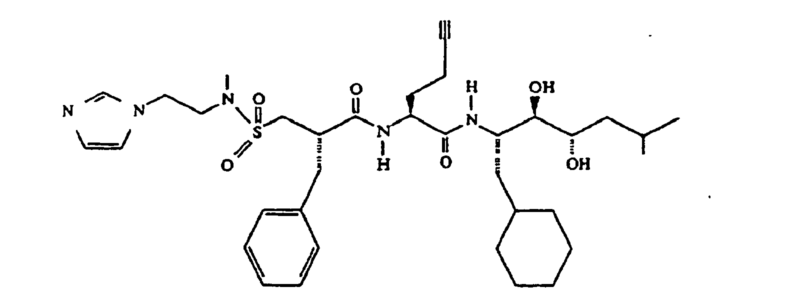 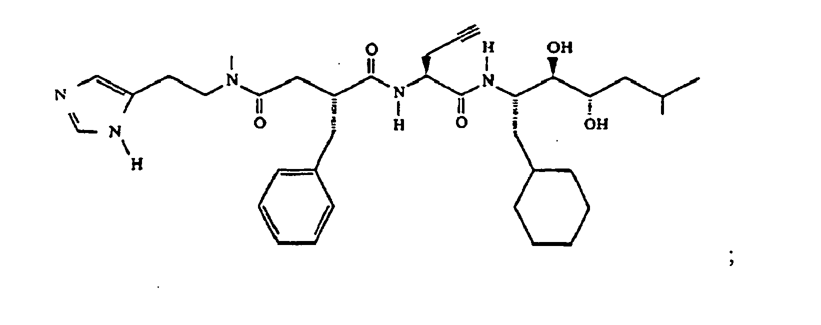 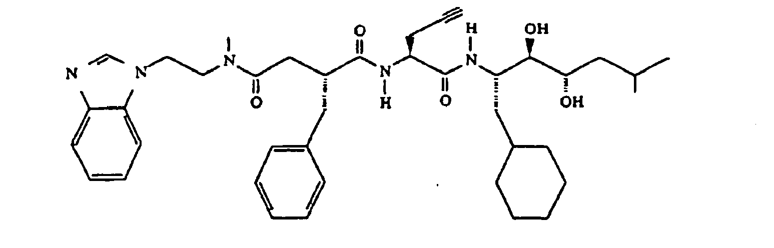 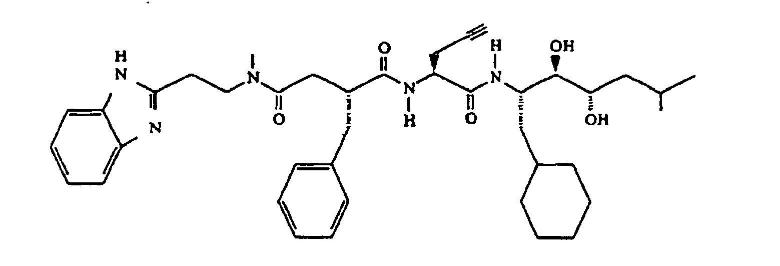 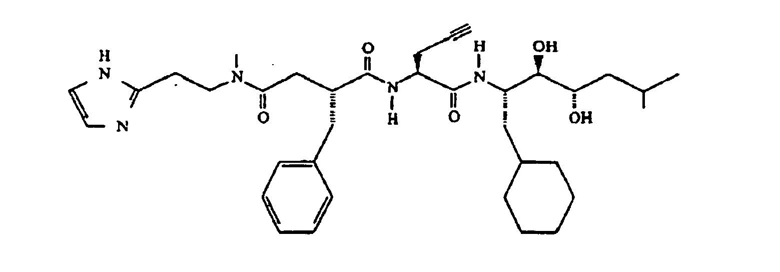 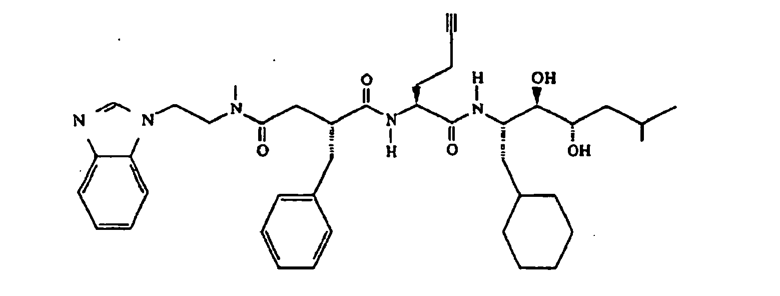 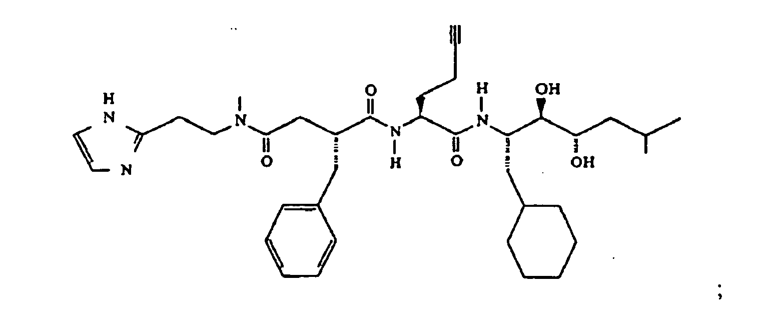 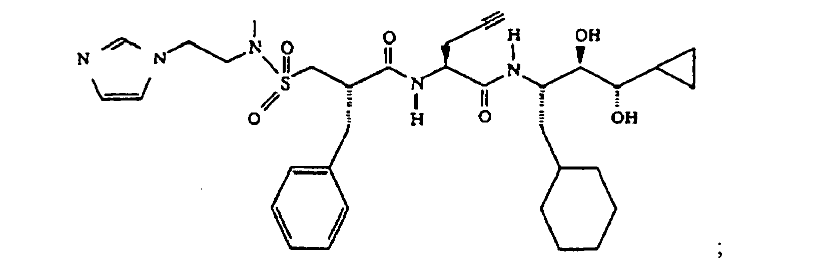 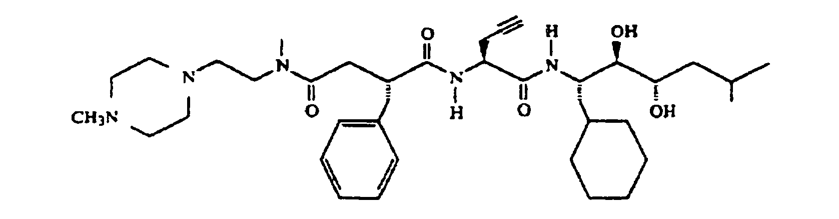 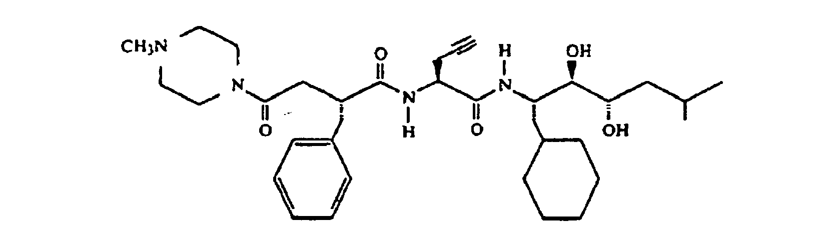 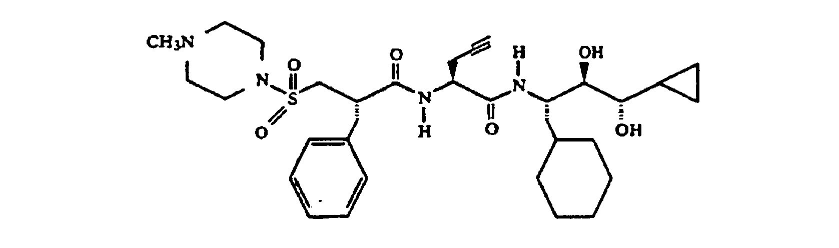 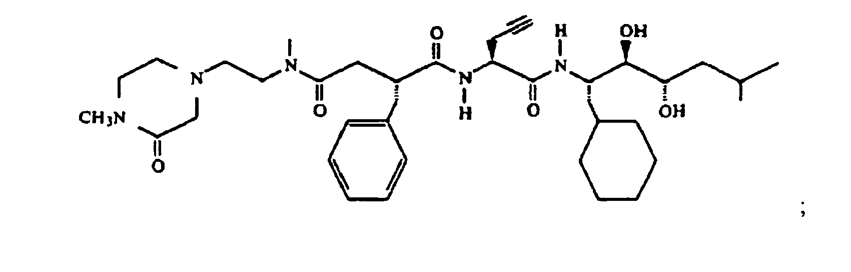 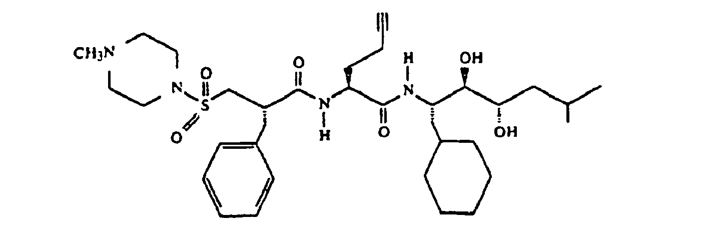 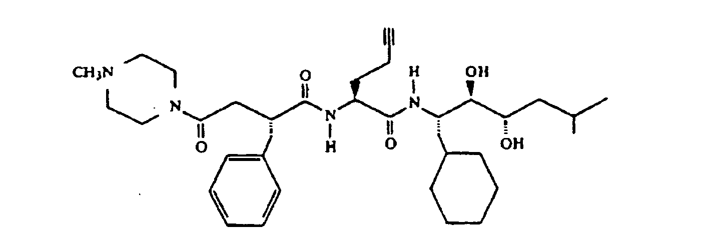 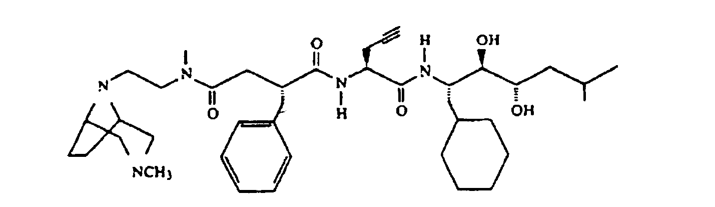 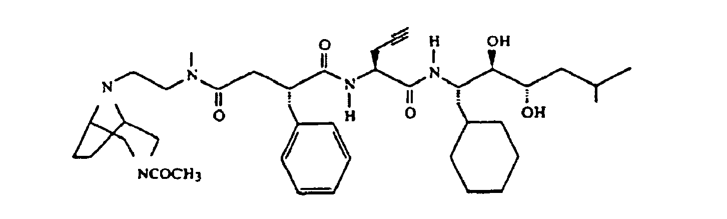 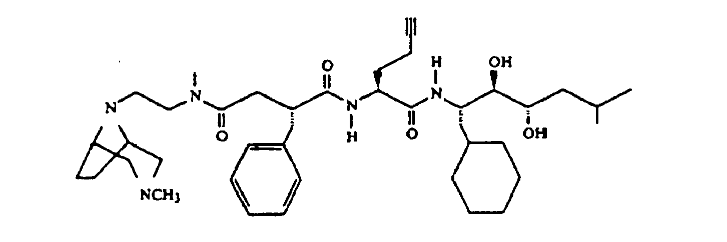 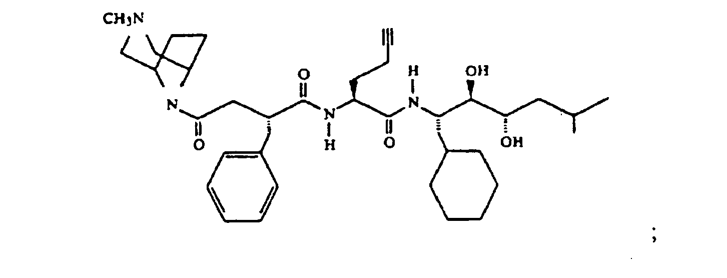 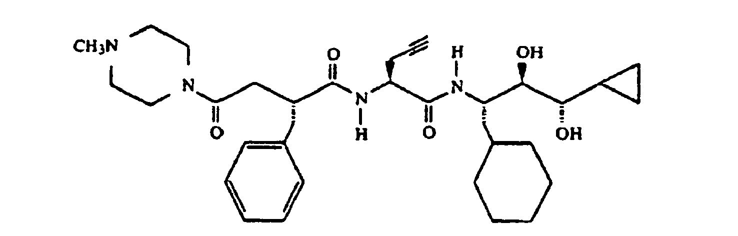 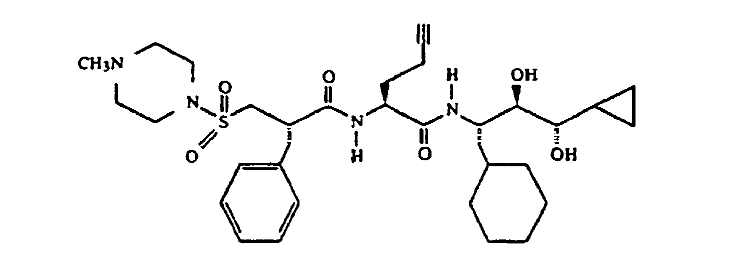 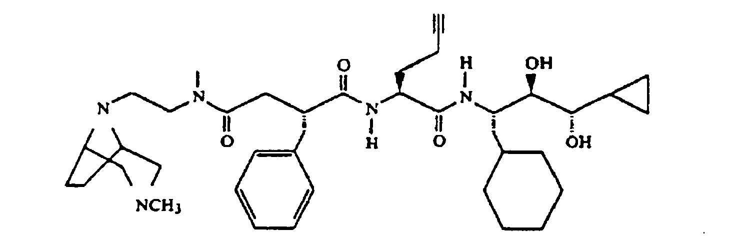 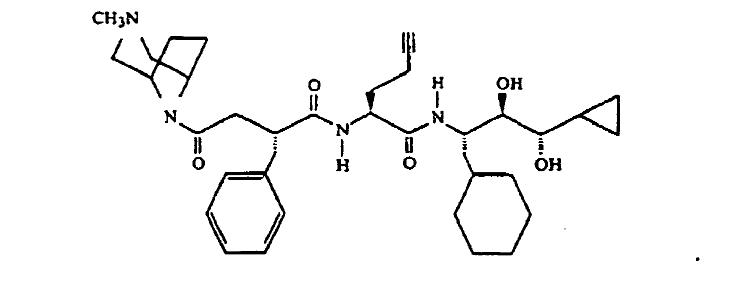
Representative compounds of Formula (VI):
   O-[N-methyl-N-(2-methoxyethyl)aminocarbonyl]-3-L-phenyllactyl-L-histidine amide of (2S, 3R, 4S)-2-amino-1-cyclohexyl-3,4-dihydroxy-6-methylheptane;
   O-[N-methyl-N-(2-ethoxyethyl)aminocarbonyl]-3-L-phenyllactyl-L-histidine amide of (2S, 3R, 4S)-2-amino-1-cyclohexyl-3,4-dihydroxy-6-methylheptane;
   O-[N-methyl-N-(2-methoxyethyl)aminocarbonyl]-3-L-phenyllactyl-L-leucine amide of (2S,3R,4S)-2-amino-1-cyclohexyl-3,4-dihydroxy-6-methylheptane;
   O-[N-methyl-N-(2-ethoxyethyl)aminocarbonyl]-3-L-phenyllactyl-L-leucine amide of (2S,3R,4S)-2-amino-1-cyclohexyl-3,4-dihydroxy-6-methylheptane;
   O-[N-methyl-N-(2-methoxyethyl)aminocarbonyl]-3-L-phenyllactyl-α-(R)-methyl-β-alanine amide of (2S,3R,4S)-2-amino-1-cyclohexyl-3,4-dihydroxy-6-methylheptane;
   O-[N-methyl-N-(2-methoxyethyl)aminocarbonyl]-3-L-homophenyllactyl-α-(R)-methyl-β-alanine amide of (2S,3R,4S)-2-amino-1-cyclohexyl-3,4-dihydroxy-6-methylheptane;
   O-[N-methyl-N-(2-methoxyethyl)aminocarbonyl]-3-L-homophenyllactyl-α-(R)-ethyl-β-alanine amide of (2S,3R,4S)-2-amino-1-cyclohexyl-3,4-dihydroxy-6-methylheptane;
   3-[N-methyl-N-(2-methoxyethyl)aminocarbonyl]-2-(R)-(2-phenylethyl)-propionyl-α-(R)-ethyl-β-alanine amide of (2S,3R,4S)-2-amino-1-cyclohexyl-3,4-dihydroxy-6-methylheptane;
   (3S,4S)-N-[(tert-Butyloxy)carbonyl]-4-amino-3-acetoxy-5-phenylpentene;
   (2R,3S)-N-[(tert-Butyloxy)carbonyl]-3-amino-2-acetoxy-4-phenylbutanal;
   (2S,3R,4S)-N-[(tert-Butyloxy)carbonyl]-2-amino-1-phenyl-3,4-dihydroxy-6-methylheptane;
   (2S,3R,4S)-N-[(tert-Butyloxy)carbonyl]-2-amino-1-cyclohexyl-3,4-dihydroxy-6-methylheptane;
   L-leucine amide of (2S,3R,4S)-2-amino-l-cyclohexyl-3,4-dihydroxy-6-methylheptane;
   Boc-(im-Tosyl)-L-histidine amide of (2S,3R,4S)-2-amino-1-cyclohexyl-3,4-dihydroxy-6-methylheptane;
   (im-Tosyl)-L-histidine amide of (2S,3R,4S)-2-amino-1-cyclohexyl-3,4-dihydroxy-6-methylheptane;
   O-(N-methyl-2-methoxyethylaminocarbonyl)-3-L-phenyllactyl-L-histidine amide of (2S,3R,4S)-2-amino-l-cyclohexyl-3,4-dihydroxy-6-methylheptane;
   O-(N-methyl-2-methoxyethylaminocarbonyl)-3-L-phenyllactyl-L-leucine amide of (2S,3R,4S)-2-amino-1-cyclohexyl-3,4-dihydroxy-6-methylheptane;
   O-(N-methyl-2-methoxyethylaminocarbonyl)-3-L-phenyl lactic acid;
   N-Boc-α-(R)-methyl-β-alanine amide of (2S,3R,4S)-2-amino-1-cyclohexyl-3,4-dihydroxy-6-methylheptane;
   α-(R)-Methyl-β-alanine amide of (2S,3R,4S)-2-amino-1-cyclohexyl-3,4-dihydroxy-6-methylheptane;
   O-(N-methyl-2-methoxyethylaminocarbonyl)-3-L-phenyllactyl-α-(R)-methyl-β-alanine amide of (2S,3R,4S)-2-amino-1-cyclohexyl-3,4-dihydroxy-6-methylheptane;
   3-(N-methyl-2-methoxyethylaminocarbonyl)-2R-phenethyl propionic acid; and
   3-[N-methyl-N-(2-ethoxyethyl)aminocarbonyl]-2-(R)-(2-phenylethyl)-propionyl-α-(R)-ethyl-β-alanine amide of (2S,3R,4S)-2-amino-1-cyclohexyl-3,4-dihydroxy-6-methylheptane.
Representative compounds of Formula (VII):
   (3S,4S)-N-[(tert-Butyloxy)carbonyl]-4-amino-3-acetoxy-5-phenylpentene
   (2R,3S)-N-[(tert-Butyloxy)carbonyl]-3-amino-2-acetoxy-4-phenylbutanal
   (2S,3R,4S)-N-[(tert-Butyloxy)carbonyl]-2-amino-1-phenyl-3,4-dihydroxy-6-methylheptane
   (2S, 3R, 4S)-N-[(tert-Butyloxy)carbonyl]-2-amino-1-cyclohexyl-3, 4-dihydroxy-6-methylheptane
   N-Boc-α-methyl-β-alaninamide of (2S,3R,4S)-2-amino-1-cyclohexyl-3,4-dihydroxy-6-methylheptane (isomer A)
   N-Boc-α-methyl-β-alaninamide of (2S,3R,4S)-2-amino-1-cyclohexyl-3,4-dihydroxy-6-methylheptane (isomer B)
   α-Methyl-β-alaninamide of (2S,3R,4S)-2-amino-1-cyclohexyl-3,4-dihydroxy-6-methylheptane (from isomer A)
   D,L-Monomethyl-2-(1-naphthylmethyl)-succinate
   D, L-Methyl-3-(N-morpholinocarbonyl)-2-(1-naphthylmethyl)-propionate
   D,L-3-(N-Morpholinocarbonyl)-2-(1-naphthylmethyl)-propionic acid
   3-(N-morpholinocarbonyl)-(2R,S)2-(1-naphthylmethyl)-propionyl-α-methyl-β-alaninamide of (2S, 3R, 4S)-2-amino-1-cyclohexyl-3,4-dihydroxy-6-methylheptane (from isomer A)
   α-MeChyl-β-alaninamide of (2S, 3R, 4S)-2-amino-1-cyclohexyl-3,4-dihydroxy-6-methylheptane (from isomer B)
   3-(N-morpholinocarbonyl)-(2R,S)2-(1-naphthylmethyl)-propionyl-α-methyl-β-alaninamide of (2S,3R,4S)-2-amino-1-cyclohexyl-3,4-dihydroxy-6-methylheptane (from isomer B)
   Methyl-D,L-3-aminoisobutyrate
   Boc-L-phenylalaninyl-D,L-α-methyl-β-alanine free acid (2S,3R)-N-Boc-2-amino-1-cyclohexyl-3,4-dihydroxybutane
   Boc-L-phenylalaninyl-D,L-α-methyl-β-alaninamide of (2S,3R)-2-amino-1-cylohexyl-3,4-dihydroxybutane
   Boc-L-phenylalaninyl-D,L-α-methyl-β-alaninamide of (2S,3R,4S)-2-amino-1-cyclohexyl-3,4-dihydroxy-6-methylheptane
   Methyl-L-3-phenyl lactate
   O-(N-morpholinocarbonyl)-3-L-phenyl lactic acid
   O-(N-morpholinocarbonyl)-3-L-phenyllactyl-D,L-α-methyl-β-alanine free acid
   O-(N-morpholinocarbonyl)-3-L-phenyllactyl-D,L-a-methyl-β-alaninamide of (2S,3R,4S)-2-amino-1-cyclohexyl-3,4-dihydroxy-6-methylheptane
   O-(N-morpholinocarbonyl)-3-L-phenyllactyl-a-(R)-methyl-β-alaninamide of (2S,3R,4S)-2-amino-1-cyclohexyl-3,4-dihydroxy-6-methylheptane; and
   O-(N-morpholinocarbonyl)-3-L-phenyllactyl-a-(S)-methyl-β-alaninamide of (2S,3R,4S)-2-amino-1-cyclohexyl-3,4-dihydroxy-6-methylheptane.
Representative compounds of Formula (VIII):
   (3S,4S)-N-[(tert-Butyloxy)carbonyl]-4-amino-3-acetoxy-5-phenylpentene;
   (2R,3S)-N-[(tert-Butyloxy)carbonyl]-3-amino-2-acetoxy-4-phenylbutanal;
   (2S,3R,4S)-N-[(tert-Butyloxy)carbonyl]-2-amino-1-phenyl-3,4-dihydroxy-6-methylheptane;
   (2S,3R,4S)-N-[(tert-Butyloxy)carbonyl]-2-amino-1-cyclohexyl-3,4-dihydroxy-6 -methylheptane;
   L-Leucinamide of (2S,3R,4S)-2-amino-1-cyclohexyl-3,4-dihydroxy-6-methylheptane;
   O-(N-morpholinocarbonyl)-3-L-phenyllactyl-L-leucinamide of (2S,3R,4S)-2-amino-1-cyclohexyl-3,4-dihydroxy-6-methylheptane;
   O-(N-morpholinocarbonyl)-3-L-phenyllactyl-L-leucine ;
   (2S,3R,4S)-N-Boc-2-amino-1-cyclohexyl-3,4-dihydroxypentane;
   O-(N-morpholinocarbonyl)-3-L-phenyllactyl-L-leucinamide of (2S,3R,4S)-2-amino-1-cyclohexyl-3,4-dihydroxypentane;
   (2S,3R)-N-Boc-2-amino-1-cyclohexyl-3,4-dihydroxybutane;
   0-(N-morpholinocarbonyl)-3-L-phenyllactyl-L-leucinamide of (2S, 3R)-2-amino-1-cyclohexyl-3,4-dihydroxybutane;
   Methyl L-3-phenyllactate;
   O-(N-morpholinocarbonyl)-3-L-phenyllactic acid;
   (2S,3R)-Boc-L-Leucinamide of 2-amino-1-cyclohexyl-3,4-dihydroxybutane;
   D,L-Monomethyl-2-(1-naphthylmethyl)succinate ;
   D,L-Methyl 3-(N-morpholinocarbonyl)-2-(1-naphthylmethyl)propionate;
   D,L-3-(N-Morpholinocarbonyl)-2-(1-naphthylmethyl)propionic acid; and
   3-(N-morpholinocarbonyl)-2-(R,S)-(1-naphthylmethyl)propionyl-L-leucinamide of (2S,3R)-2-amino-1-cyclohexyl-3,4-dihydroxybutane.
Representative compounds of Formula (IX):
   O-{N-[2-{N-[2-(N,N-dimethylamino)ethyl]-N-methylamino}-ethyl]-N-methylaminocarbonyl}-3-L-phenyllactyl-L-histidineamide of (2S,3R,4S)-2-amino-1-cyclohexyl-3,4-dihydroxy-6-methylheptane;
   O-{N- [2-{N-[2- (N,N-dimethylamino)ethyl] -N-methylamino}-ethyl]-N-methylaminocarbonyl}-3-L-phenyllactyl-L-leucineamide of (2S,3R,4S)-2-amino-1-cyclohexyl-3,4-dihydroxy-6-methylheptane;
   O-{N-[2-{N-[2-(N,N-dimethylamino)ethyl]-N-methylamino}-ethyl]-N-methylaminocarbonyl}-3-L-phenyllactyl-α-(R)-methyl-β-alanineamide of (2S,3R,4S)-2-amino-1-cyclohexyl-3,4-dihydroxy-6-methylheptane;
   O-{N-[2-{N-[2-(N,N-dimethylamino)ethyl]-N-methylamino}-ethyl]-N-methylaminocarbonyl}-3-L-homophenyllactyl-α-(R)-methyl-β-alanineamide of (2S,3R,4S)-2-amino-1-cyclohexyl-3,4-dihydroxy-6-methylheptane;
   O-{N-[2-{N-[2-N,N-dimethylamino)ethyl]-N-methylamino}-ethyl]-N-methylaminocarbonyl}-3-L-homophenyllactyl-α-(R)-ethyl-β-alanineamide of (2S,3R,4S)-2-amino-1-cyclohexyl-3,4-dihydroxy-6-methylheptane;
   3-{N-[2-(N,N-dimethylamino)ethyl]-N-methylamino}ethyl]-N-methylaminocarbonyl}-2-(R)-(2-phenylethyl)-propionyl-a-(R)-ethyl-p-alanineamide of (2S,3R,4S)-2-amino-1-cyclohexyl-3,4-dihydroxy-6-methylheptane;
   (3S, 4S)-N-[(tert-Butyloxy)carbonyl]-4-amino-3-acetoxy-5-phenylpentene;
   (2R, 3S)-N-[(tert-Butyloxy)carbonyl]-3-amino-2-acetoxy-4-phenylbutanal;
   (2S, 3R, 4S)-N-[(tert-Butyloxy)carbonyl]-2-amino-1-phenyl-3,4-dihydroxy-6-methylheptane;
   (2S, 3R, 4S)-N-[(tert-Butyloxy)carbonyl]-2-amino-1-cyclohexyl-3,4-dihydroxy-6 -methylheptane;
   L-Leucineamide of (2S, 3R, 4S)-2-amino-1-cyclohexyl-3,4-dihydroxy-6-methylheptane;
   Boc-(im-Tosyl)-L-histidineamide of (2S,3R,4S)-2-amino-1-cyclohexyl-3,4-dihydroxy-6-methylheptane;
   (im-Tosyl)-L-histidineamide of (2S,3R,4S)-2-amino-1-cyclohexyl-3,4-dihydroxy-6-methylheptane;
   N-Boc-α-(R)-methyl-β-alanineamide of (2S,3R,4S)-2-amino-1-cyclohexyl-3,4-dihydroxy-6-methylheptane;
   α-(R)-Methyl-β-alanineamide of (2S,3R,4S)-2-amino-1-cyclohexyl-3,4-dihydroxy-6-methylheptane;
   O-{N-[2-{N-[2-(N,N-dimethylamino)ethyl]-N-methylamino}-ethyl]-N-methylaminocarbonyl]-3-L-phenyllactic acid; and
   3{N-[2-{N-[2-(1-imidazole)ethyl]-N-methylamino}ethyl]-N-methylaminocarbonyl]-2-(R)-(2-phenylethyl)propionyl-α-(R)-ethyl-β-alanineamide of (2S,3R,4S)-2-amino-1-cyclohexyl-3,4-dihydroxy-6-methylheptane.
Representative compounds of Formula (X):
   N-[3-[[1S,1R*-(cyclohexylmethyl)-2S*,3R*-5-methylhexyl]amino]-2S*-methyl-3-oxopropyl]-α-[[(1,1-dimethylethyl)sulfonyl]methyl]benzenepropanamide;
   N-[3-[[1S,1R*-(cyclohexylmethyl)-2S*,3R*-dihydroxy-5-methylhexyl]amino]-2S*-methyl-2-3-oxopropyl]-α-[[(1,1-dimethylethyl)thio]methyl]benzenepropanamide;
   N-[[1S,1R*-(cyclohexylmethyl)-2S*,3R*-dihydroxy-5-methylhexyl]-2R*-methyl-3-[[1-oxo-3-(phenylsulfonyl)propyl]amino]propanamide;
   N-[[1R*-[[1S,1R*(cyclohexylmethyl)-2S*,3R*-dihydroxy-5-methylhexyl]amino]carbonyl]-3-butynyl]-α-[[(1,1-dimethylethyl)sulfonyl]methyl]benzenepropanamide;
   N-[[1R*-[[1S,1R*(cyclohexylmethyl)-2S*,3R*-dihydroxy-5-methylhexyl]amino]carbonyl]-3-butynyl]-αR*-[[(1,1-dimethylethyl)sulfonyl]methyl]benzenepropanamide;
   N-[1S,1R*(cyclohexylmethyl)-2S*,3R*-dihydroxy-5-methylhexyl]-2R*-[[1-oxo-3-[phenylsulfonyl]propyl]amino]-4-pentynamide;
   N-[2-[[1S,1R*-(cyclohexymethyl)-2S*,3R*-dihydroxy-5-hexynyl]amino]-1R*-(cyclopropylmethyl)-2-oxoethyl]-αR*-[[(1,1-dimethylethyl)sulfonyl]methyl]benzenepropanamide;
   N-[2-[[1S,1R*-(cyclohexylmethyl)-2S*,3R*-dihydroxy-5-hexynyl]amino]-1R*-(cyclopropylmethyl)-2-oxoethyl]-αR*-[[(2-methylpropyl)sulfonyl]methyl]benzenepropanamide;
   N-[2-[[1S,1R*-(cyclohexylmethyl)-2S*,3R*-dihydroxy-5-hexynyl]amino]-1R*-(cyclopropylmethyl)-2-oxoethyl]-αR*-[phenylsulfonyl)methyl]benzenepropanamide;
   N-[[1R*-[[1S,1R*-(cyclohexylmethyl)-2S*,3R*-dihydroxy-5-hexynyl]amino]carbonyl]-3butynyl]-αR*-[[(1,1-dimethylethyl)sulfonyl]methyl]benzenepropanamide;
   N-[[1R*-[[1S,1R*-(cyclohexylmethyl)-2S*,3R*-dihydroxy-5-hexynyl]amino]carbonyl]-3butynyl]-αR*-[[(2-methylpropyl)sulfonyl]methyl]benzenepropanamide;
   N-[[1R*-[[1S,1R*-(cyclohexylmethyl)-2S*,3R*-dihydroxy-5-hexynyl]amino]carbonyl]-3butynyl]-αR*-[(phenylsulfonyl)methyl]benzenepropanamide; 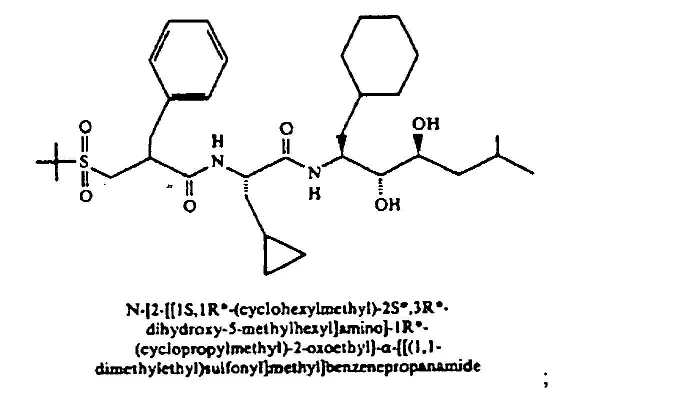 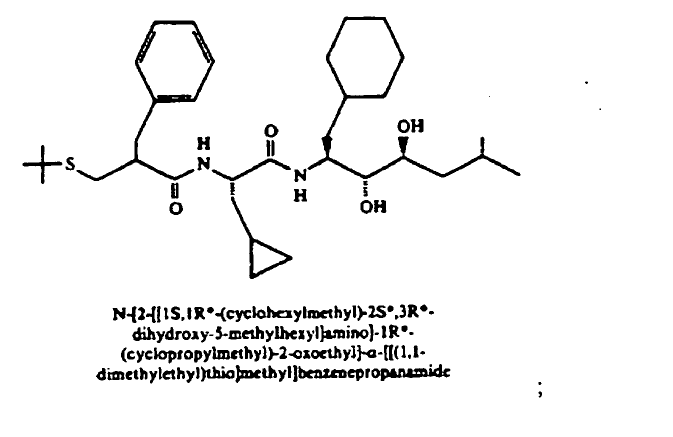 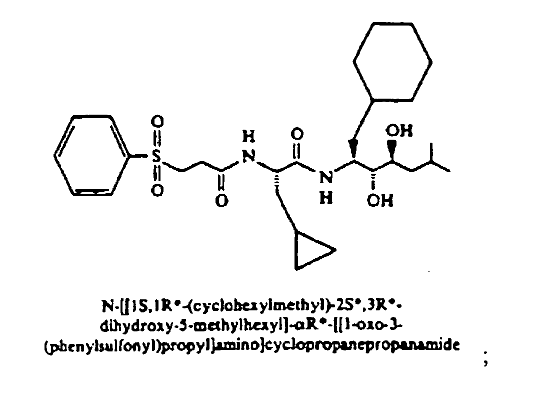 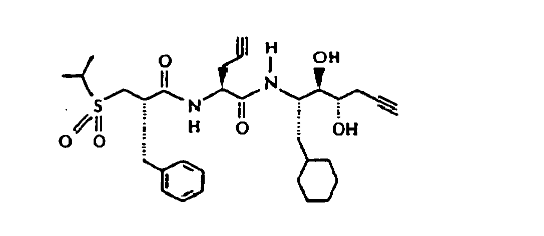 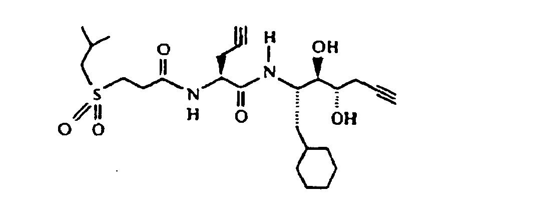 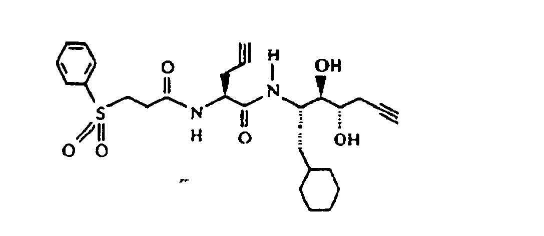 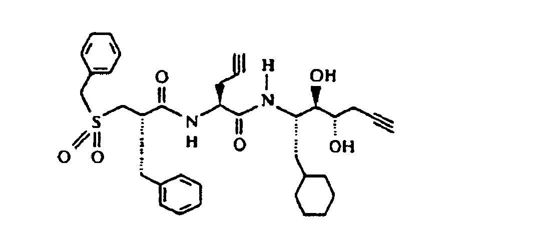 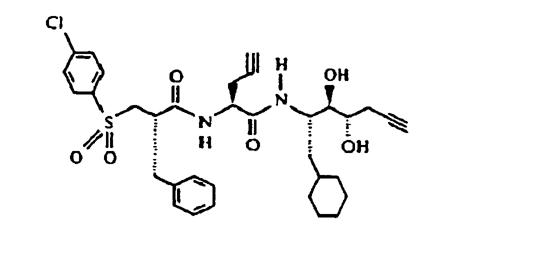 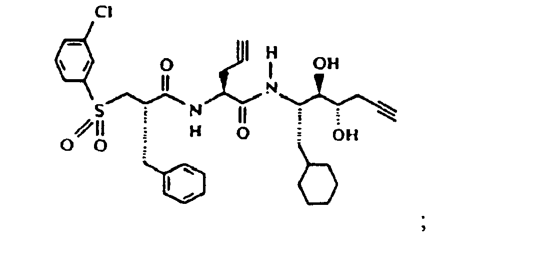 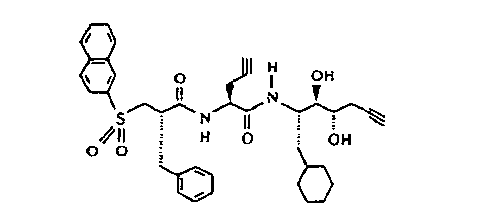 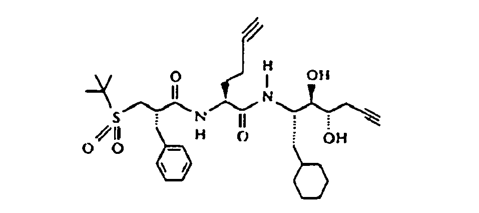 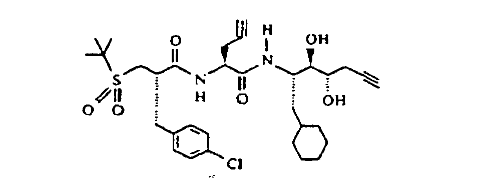 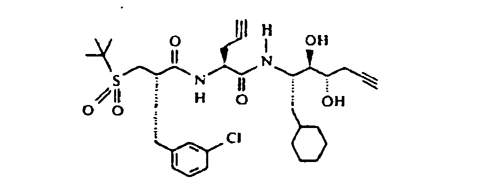 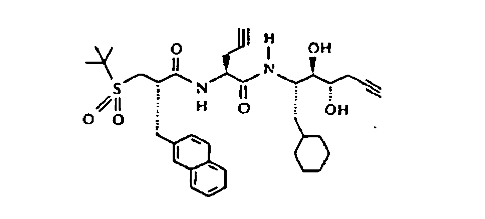 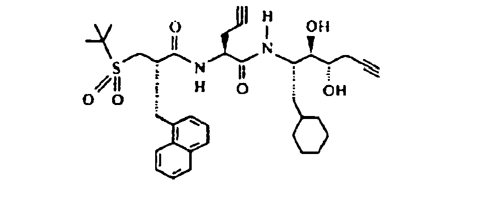 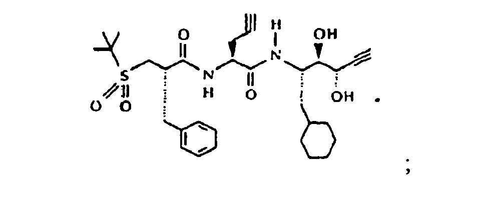 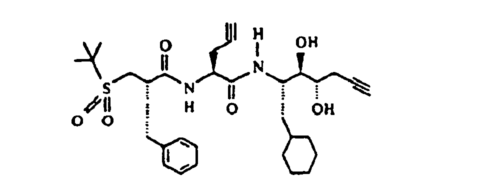 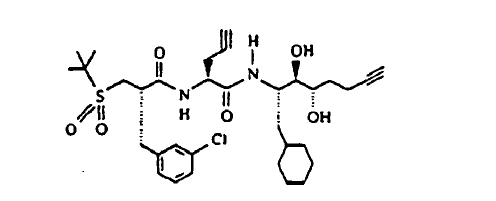 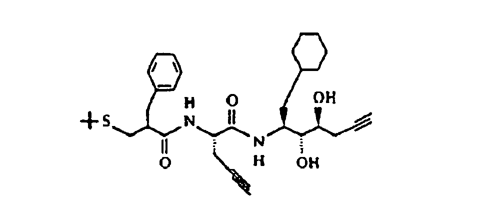 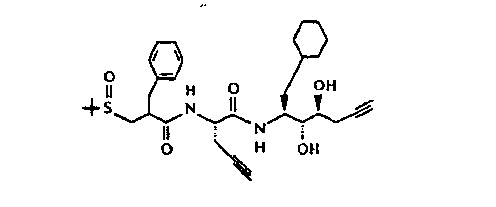 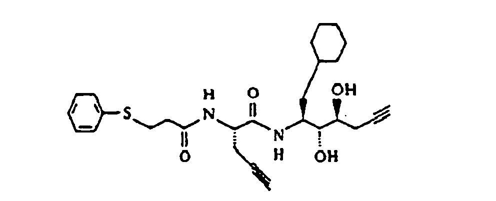 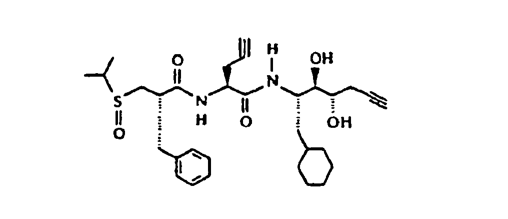 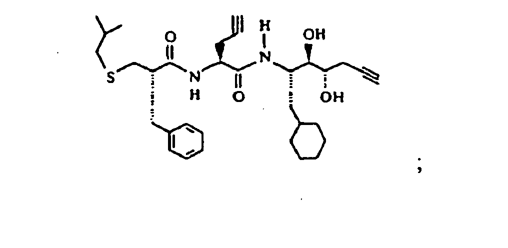 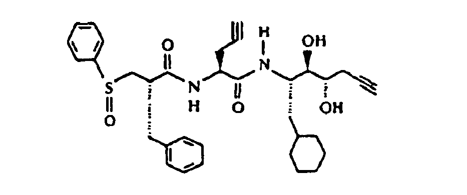 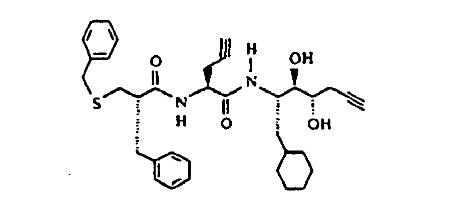 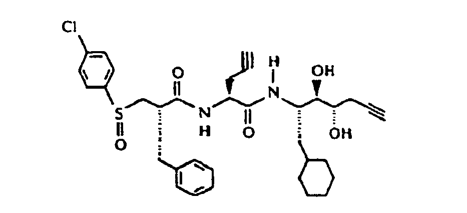 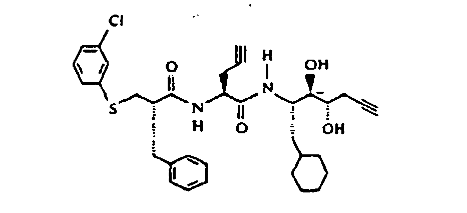 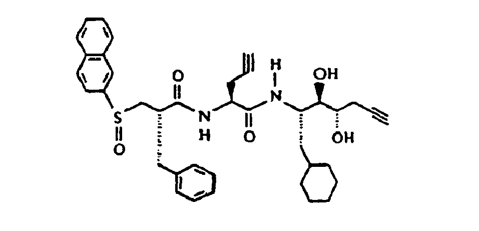 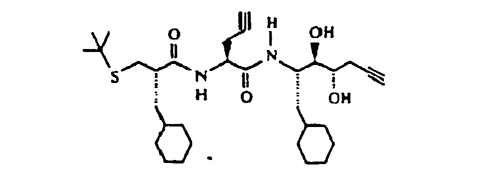 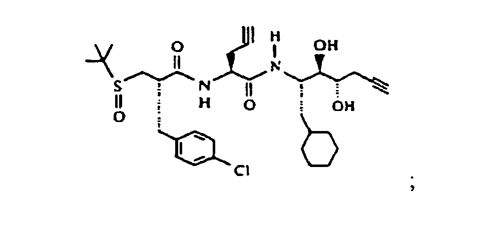 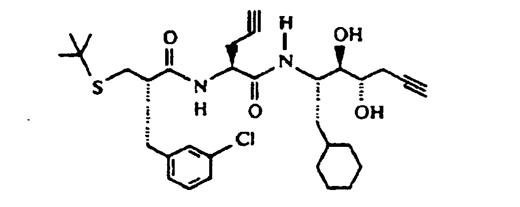 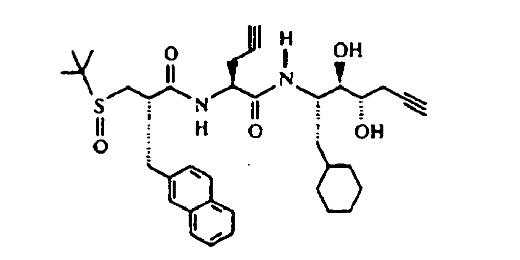 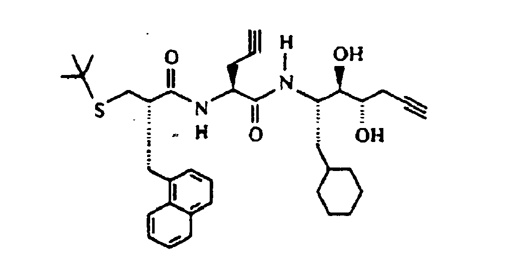 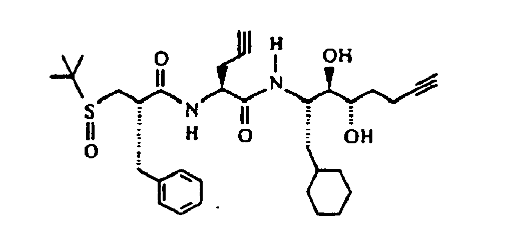 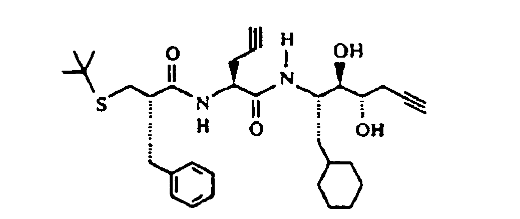 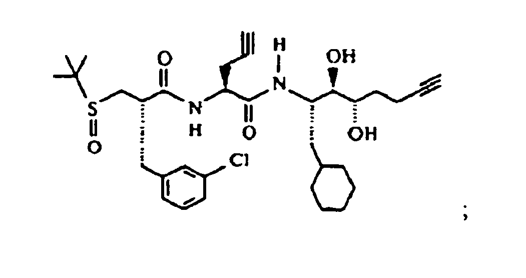 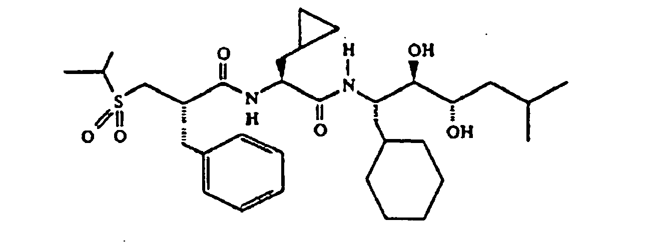 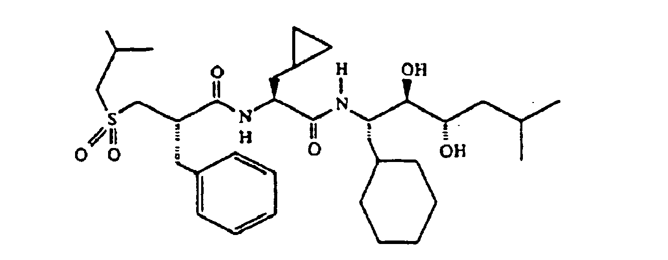 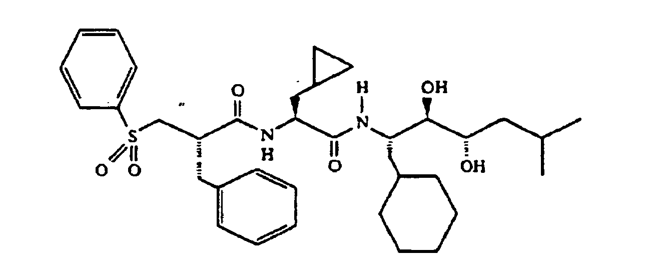 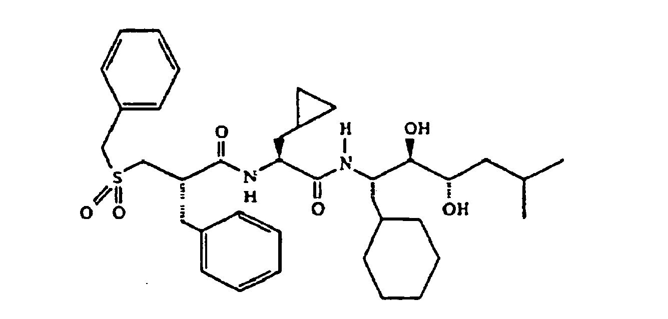 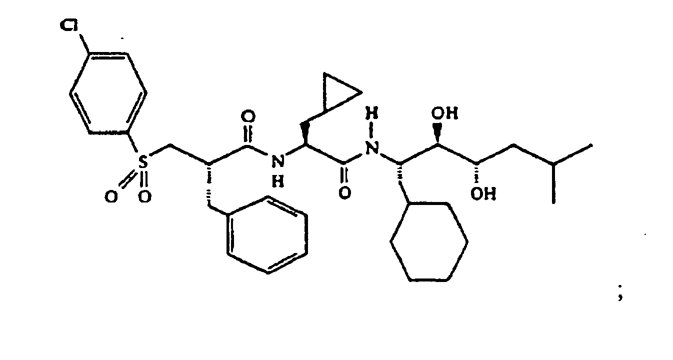 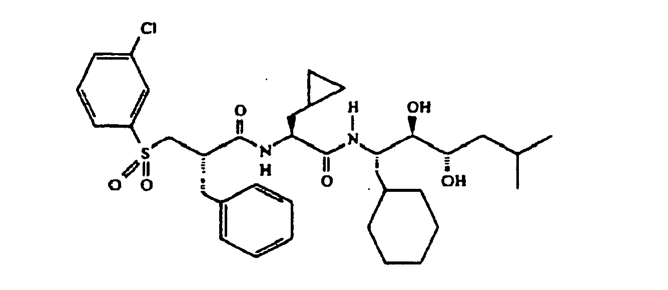 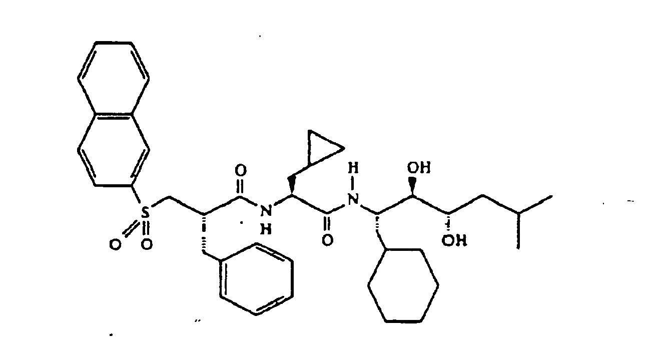 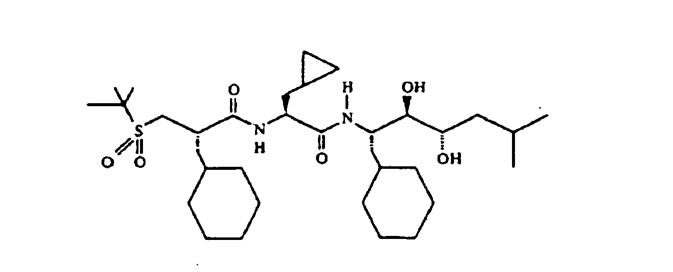 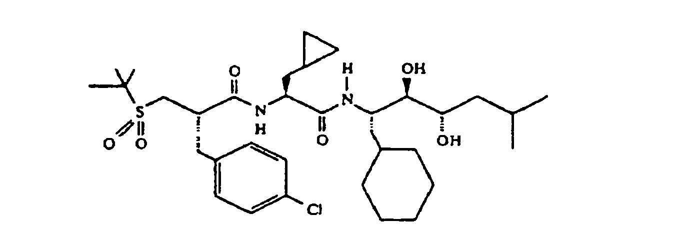 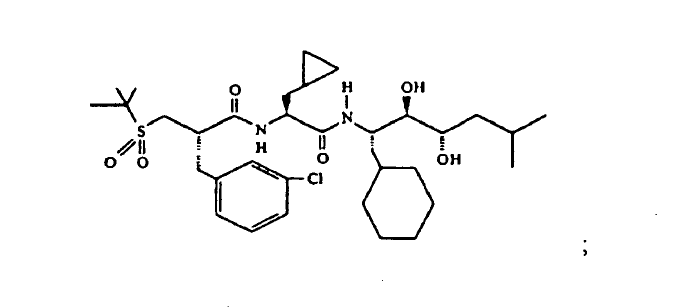 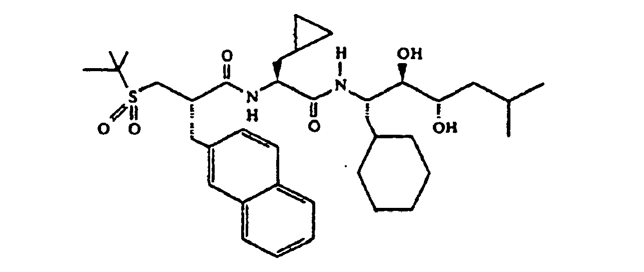 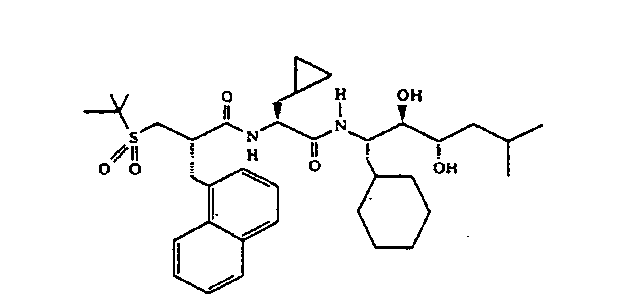 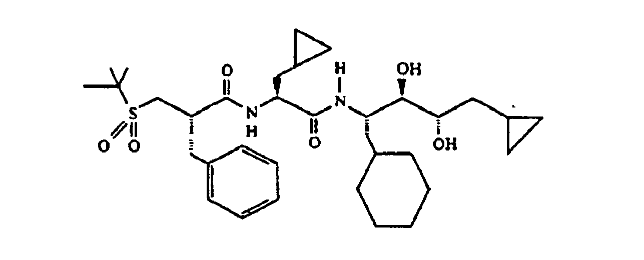 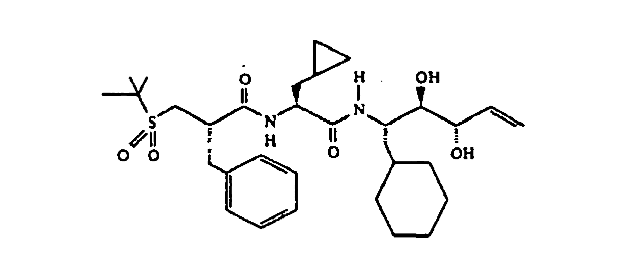 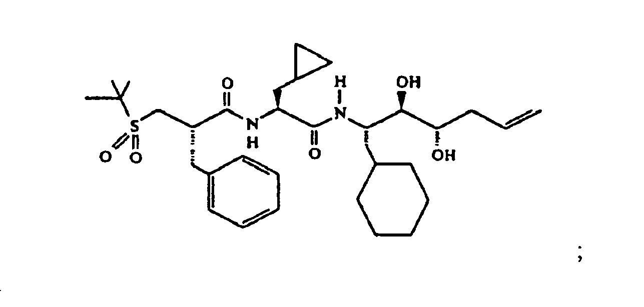 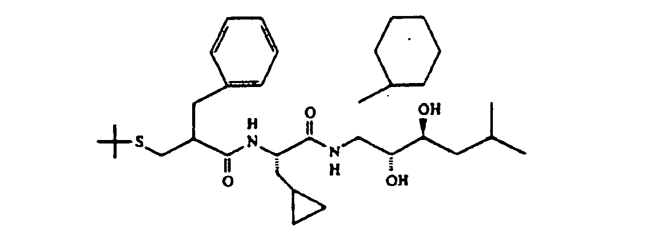 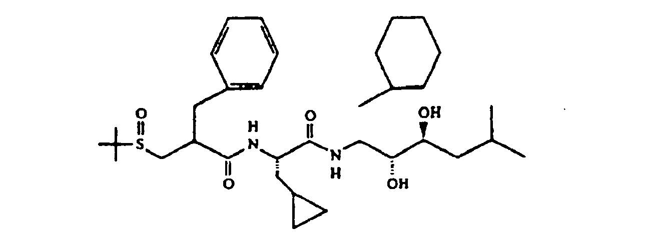 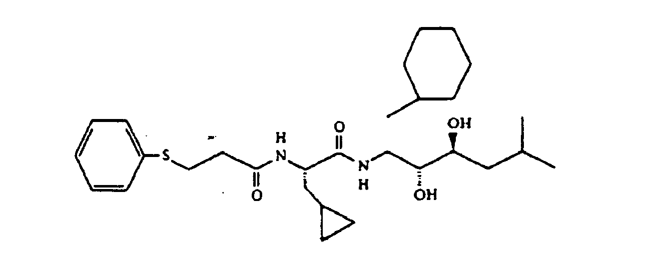 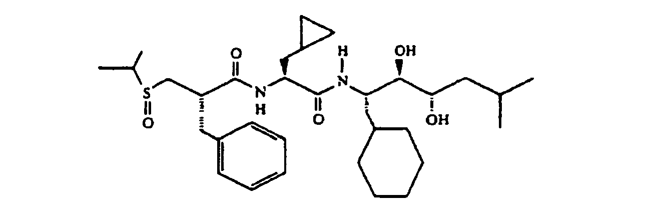 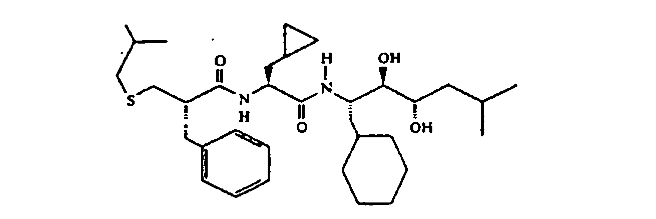 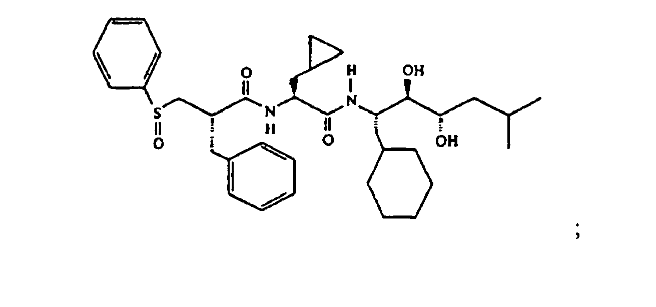 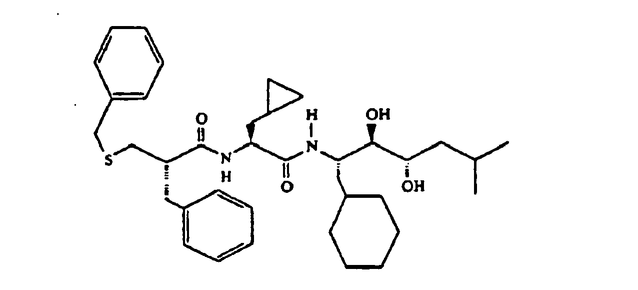 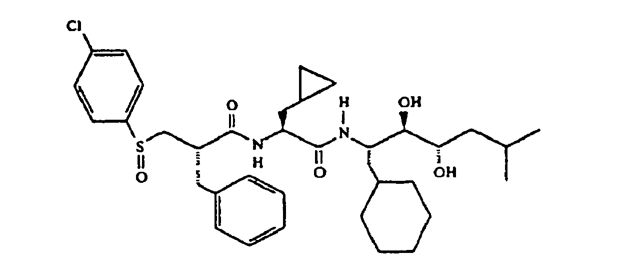 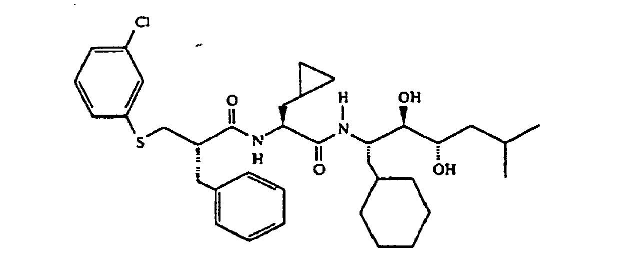 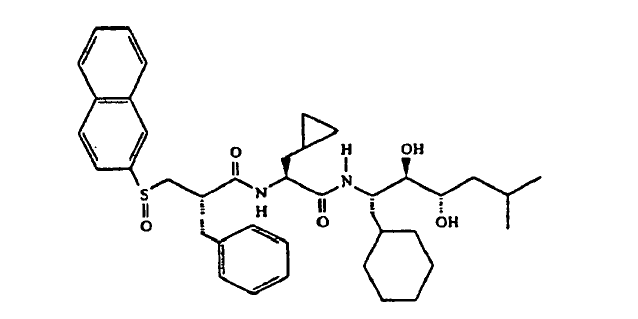 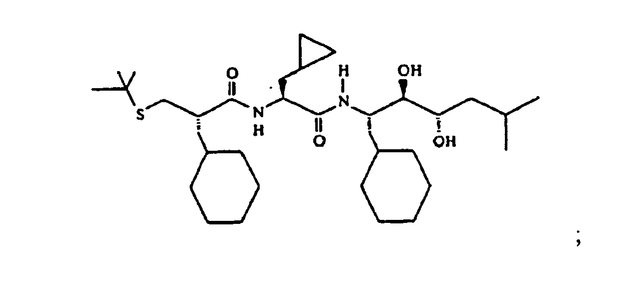 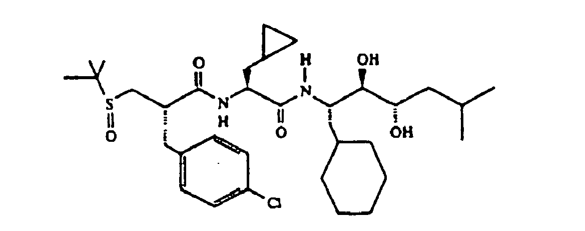 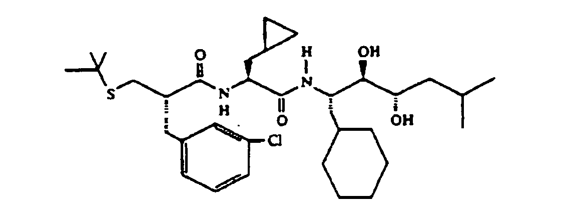 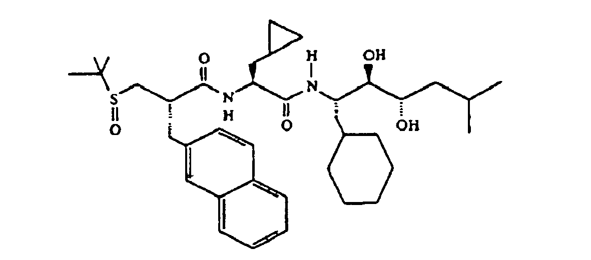 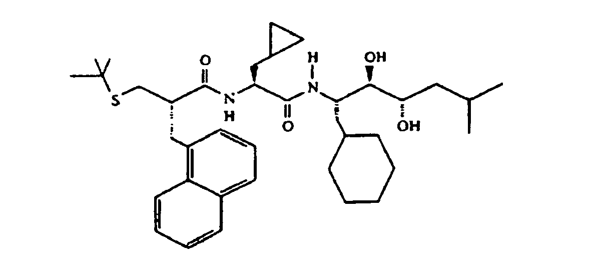 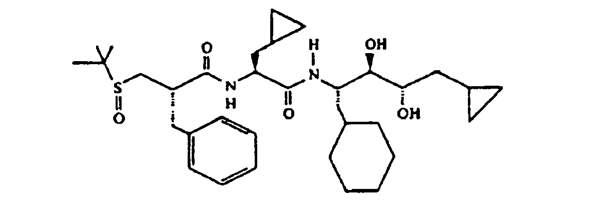 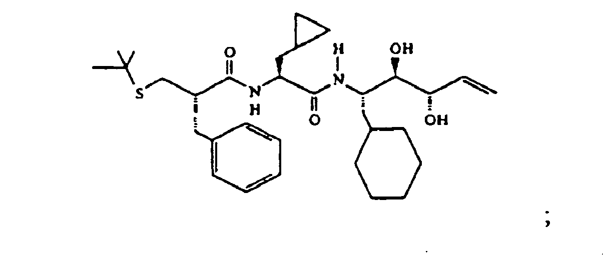 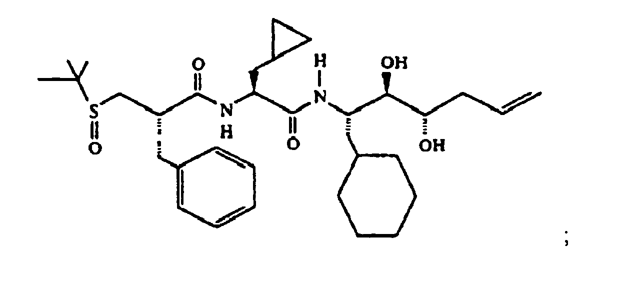 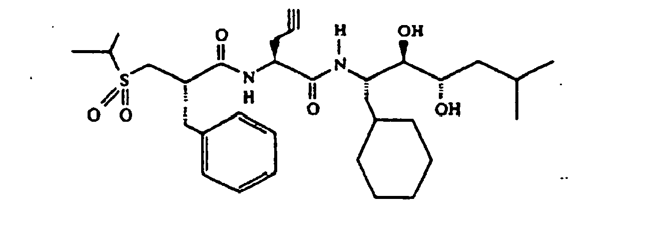 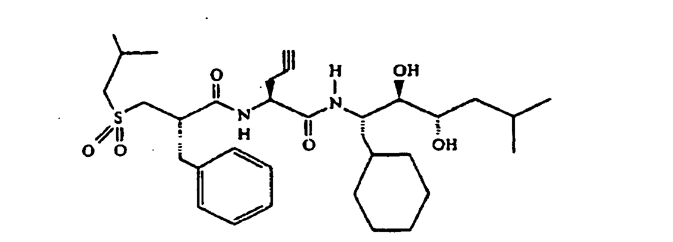 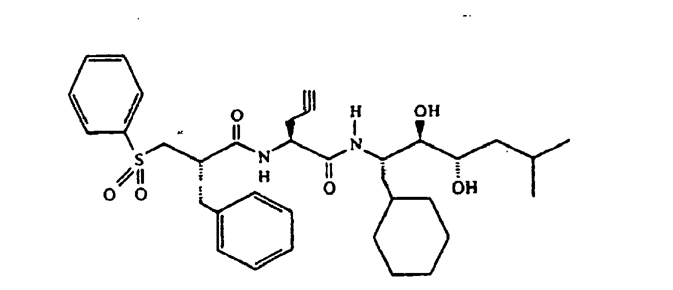 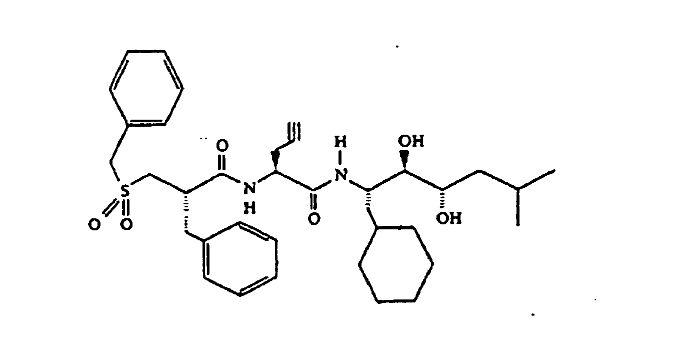 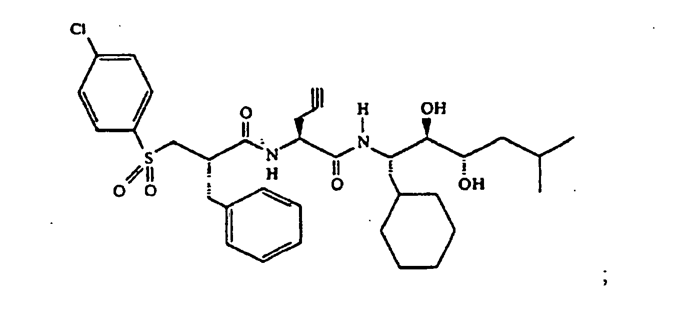 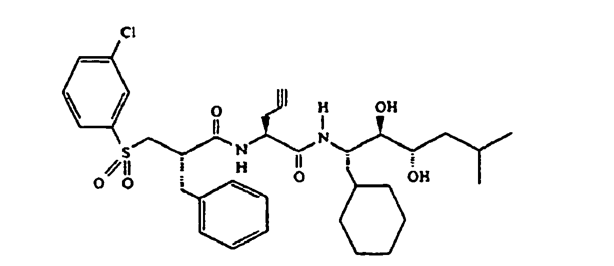 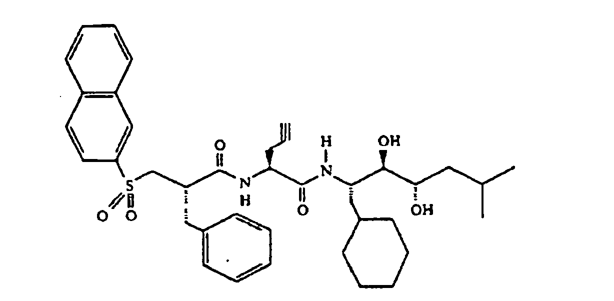 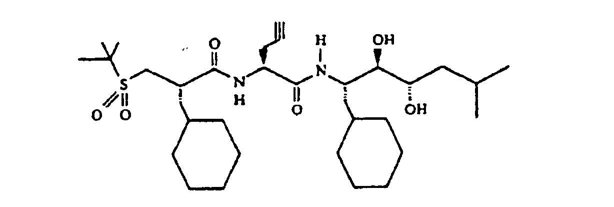 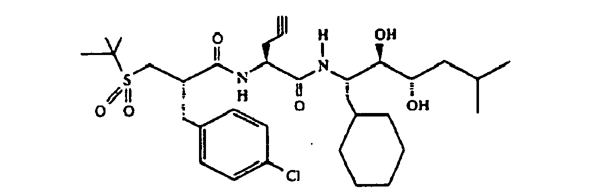 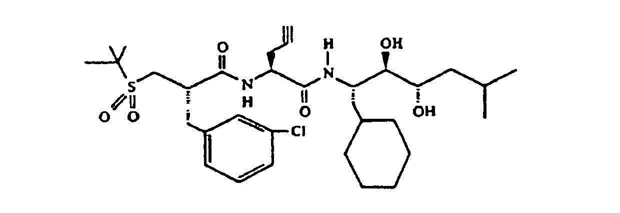 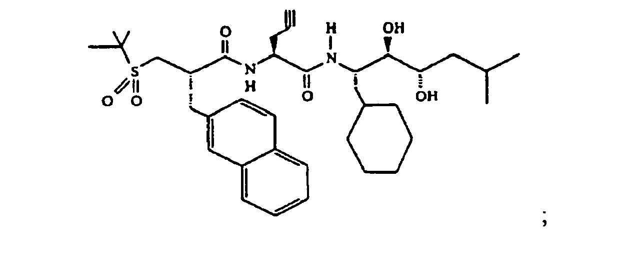 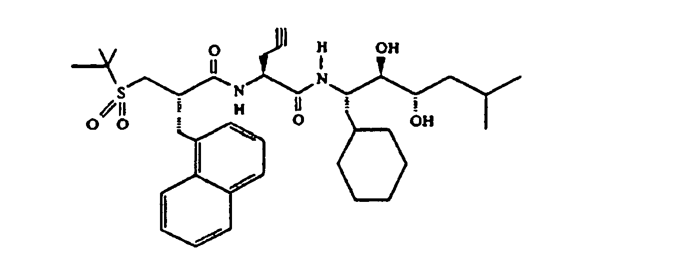 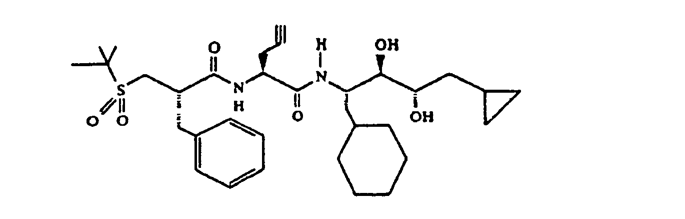 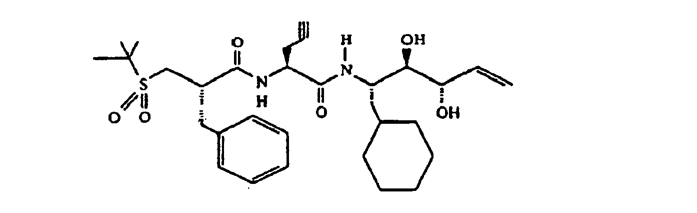 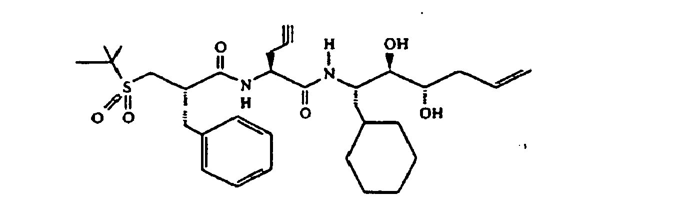 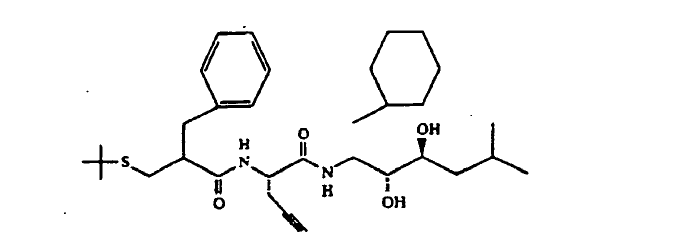 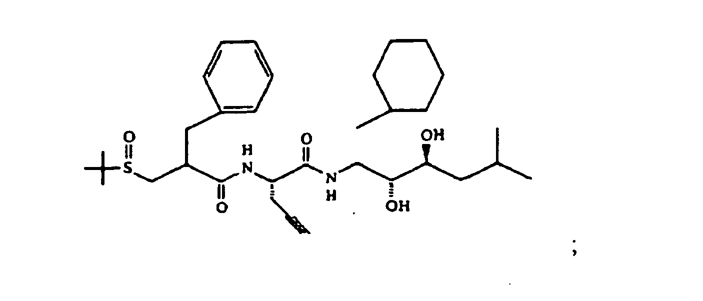 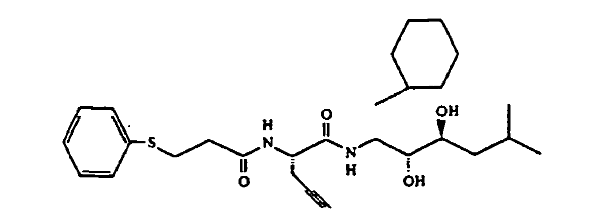 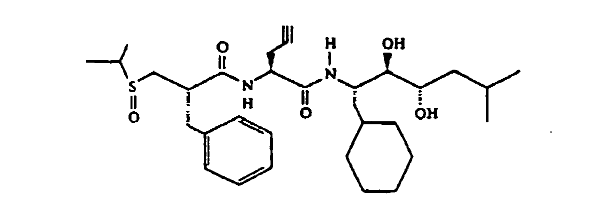 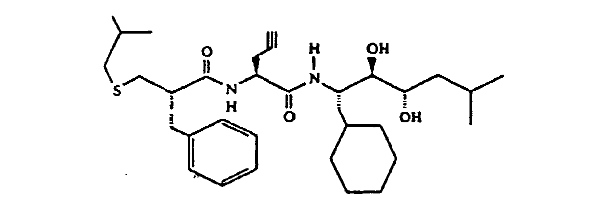 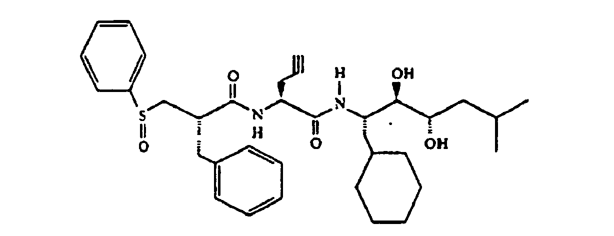 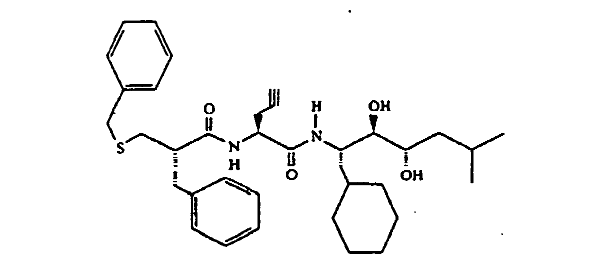 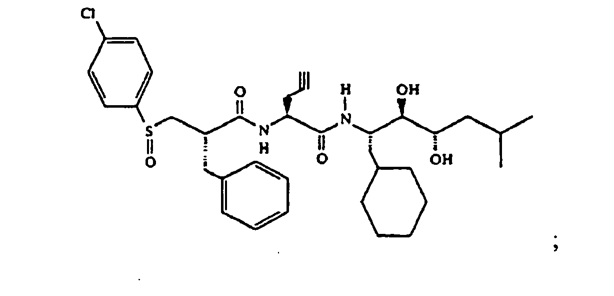 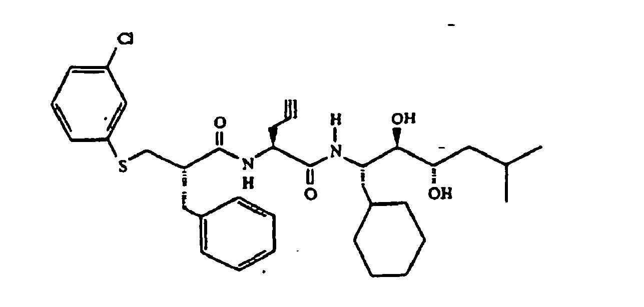 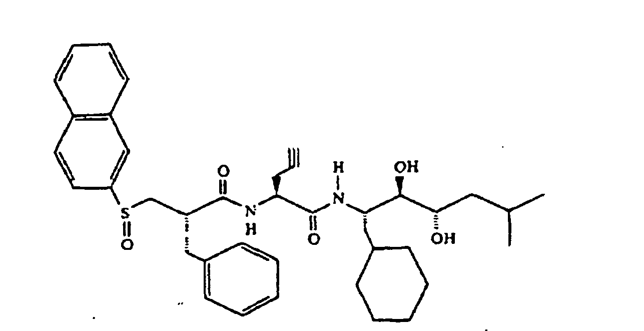 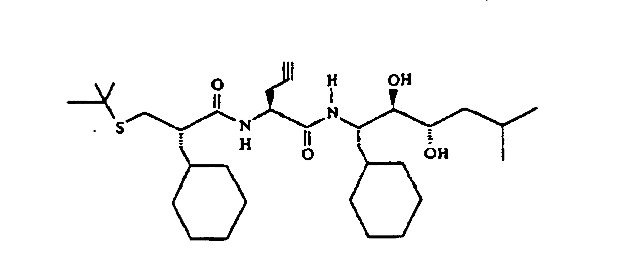 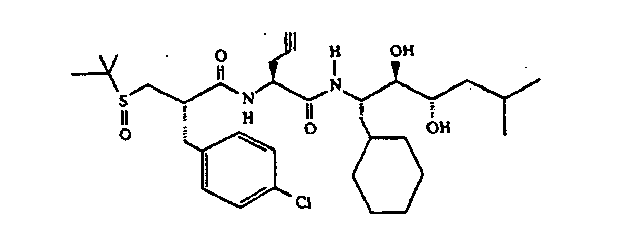 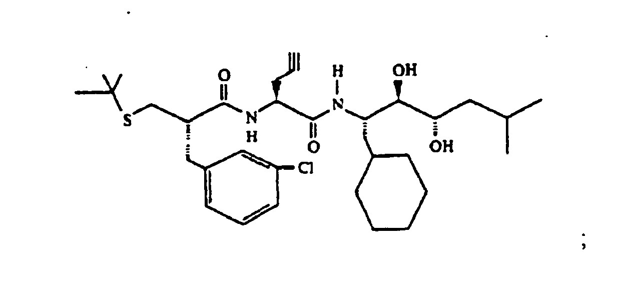 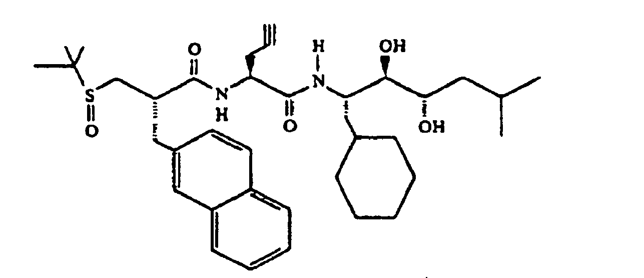 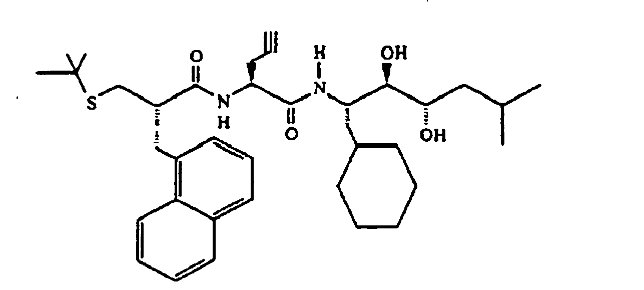 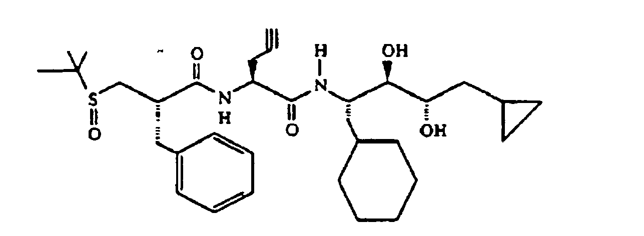 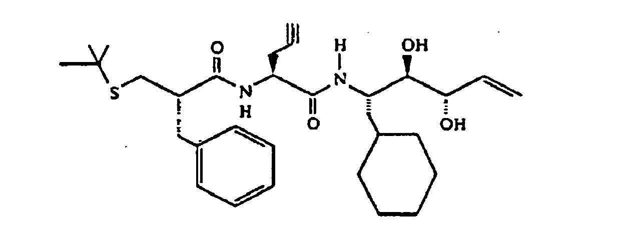 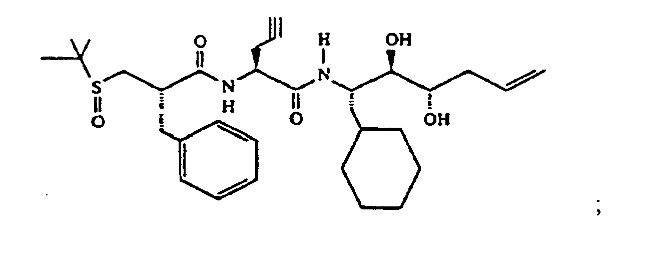 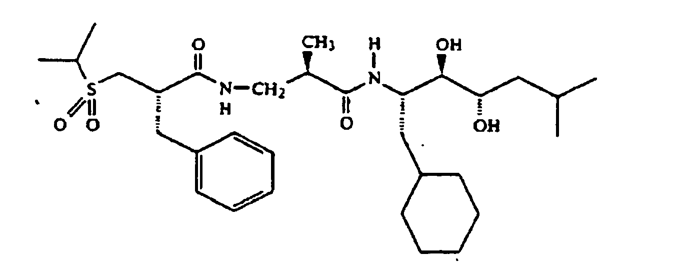 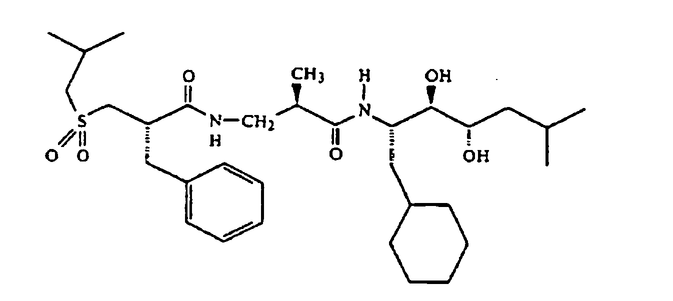 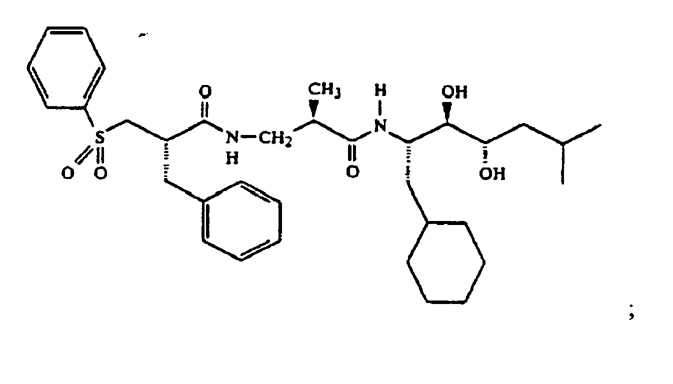 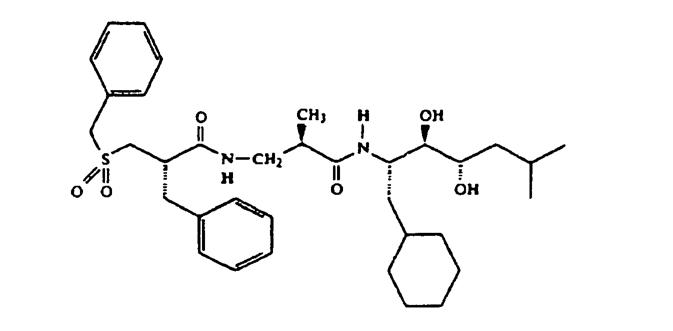 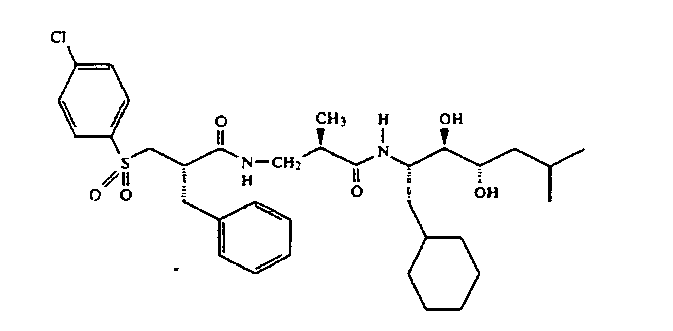 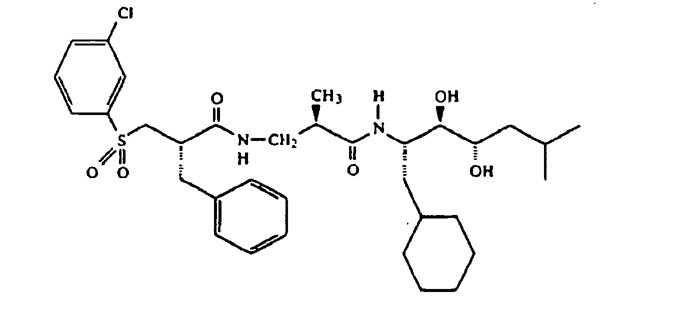 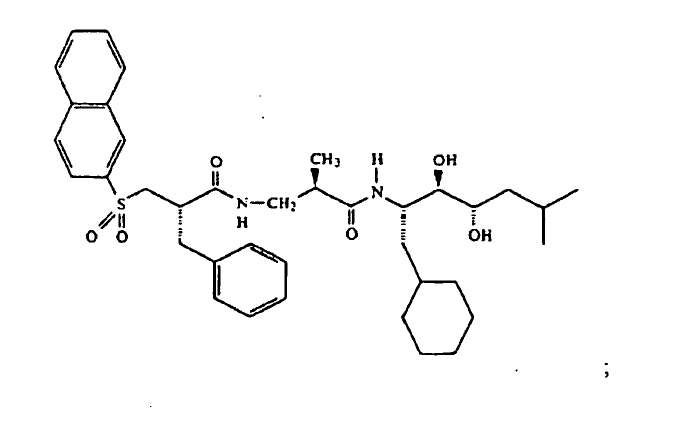 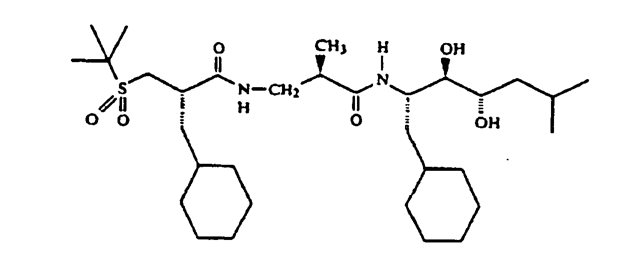 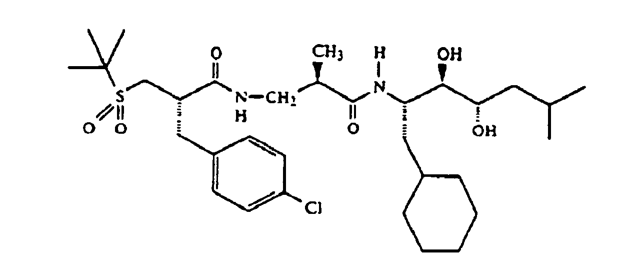 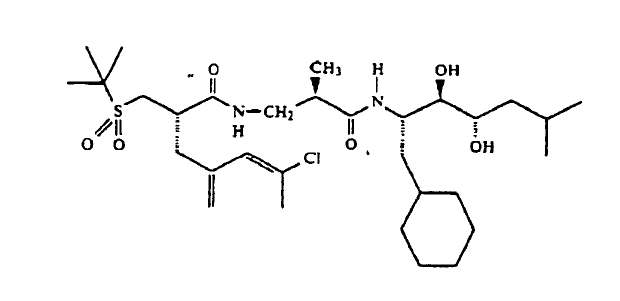 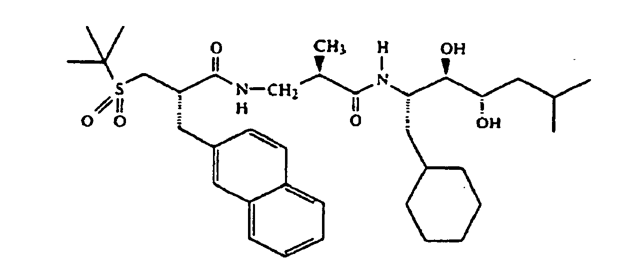 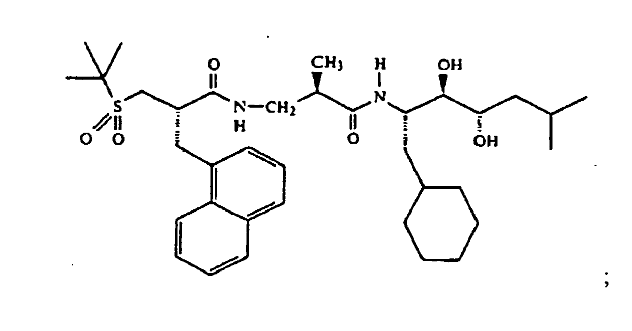 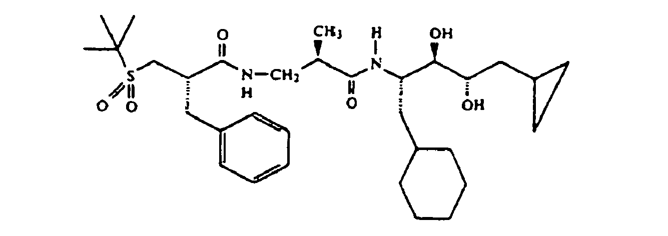 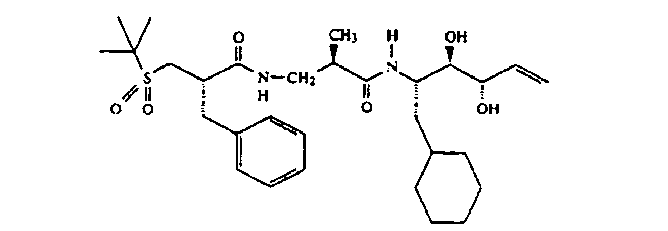 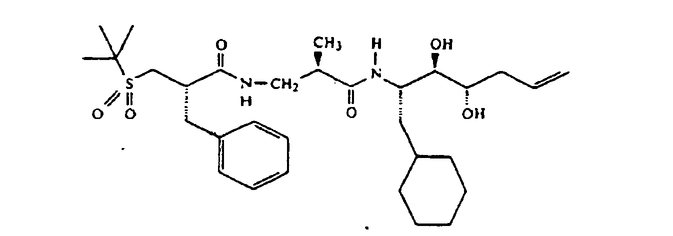 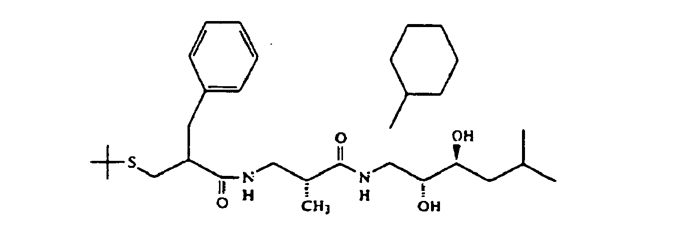 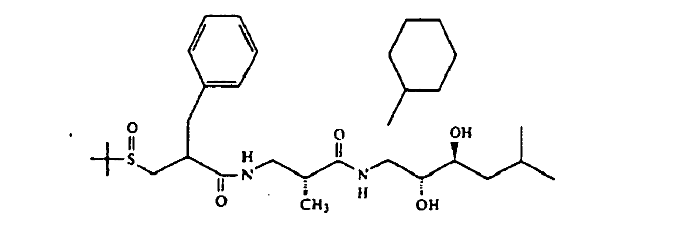 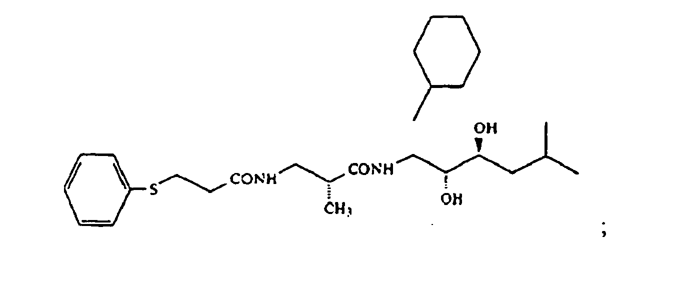 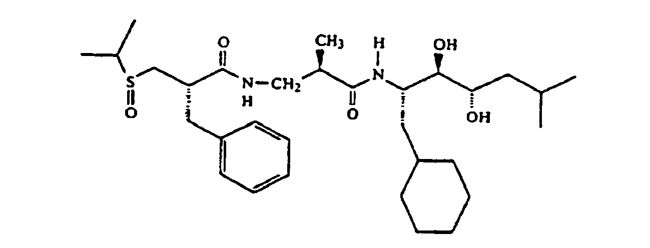 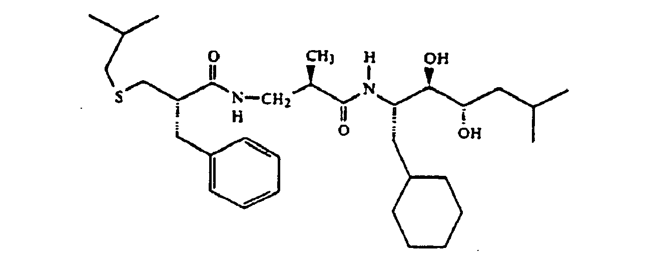 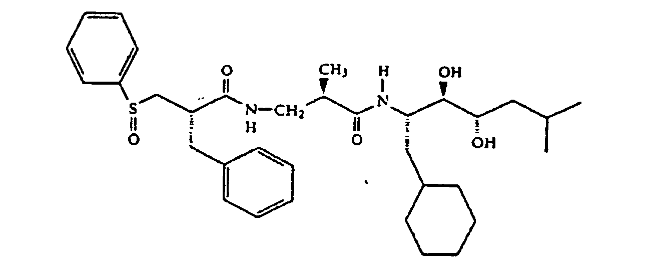 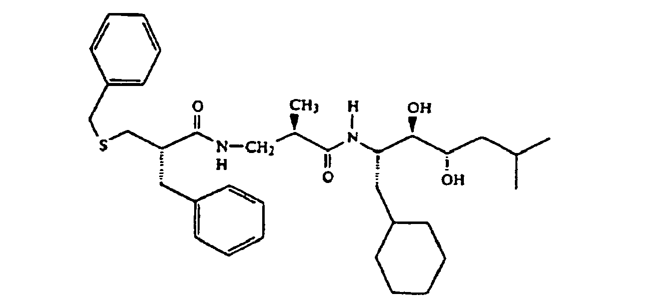 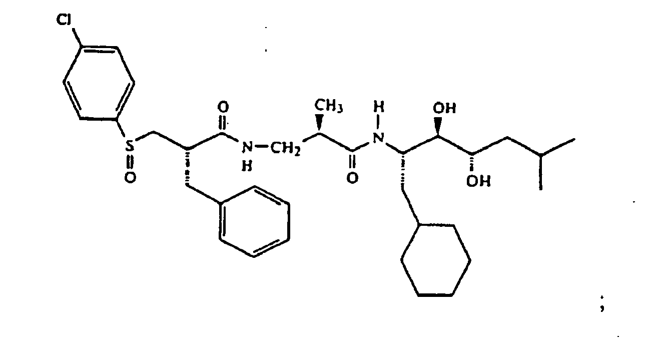 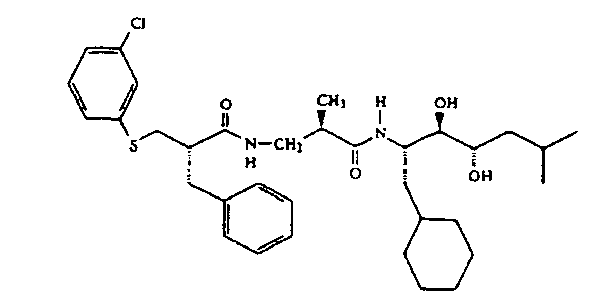 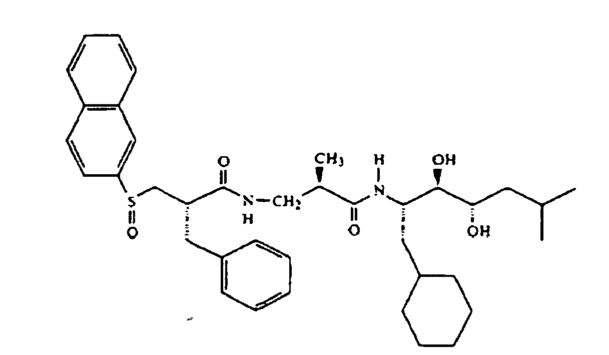 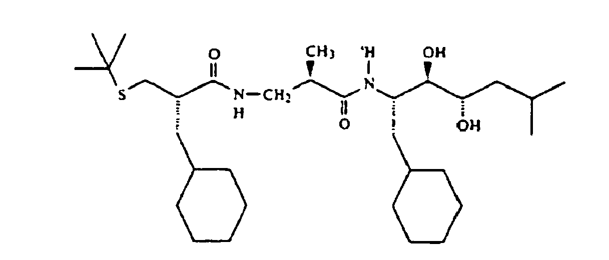 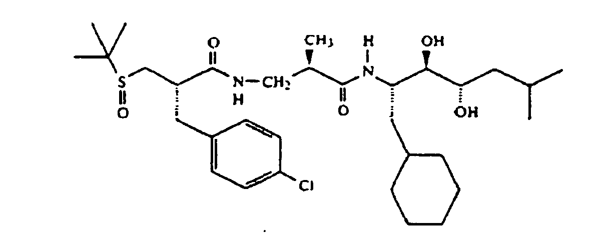 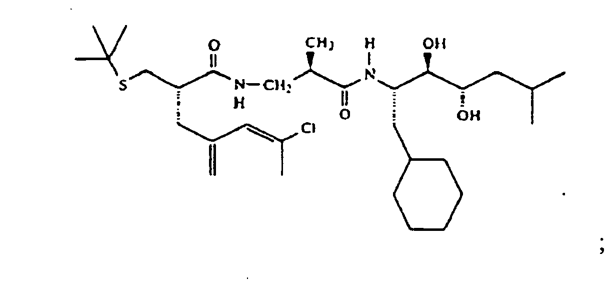 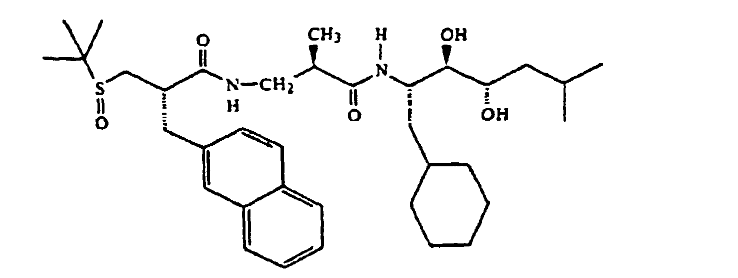 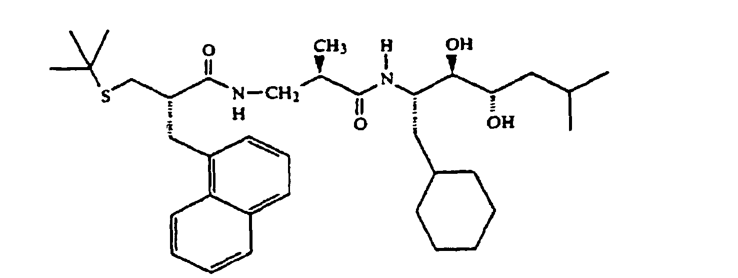 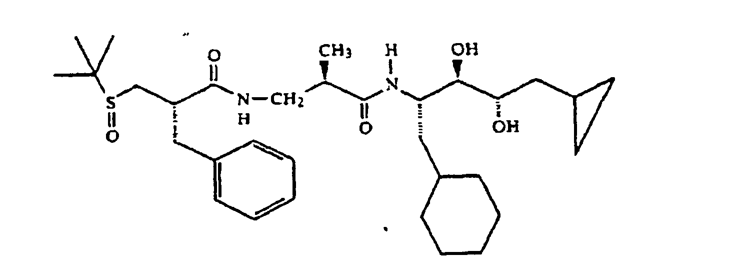 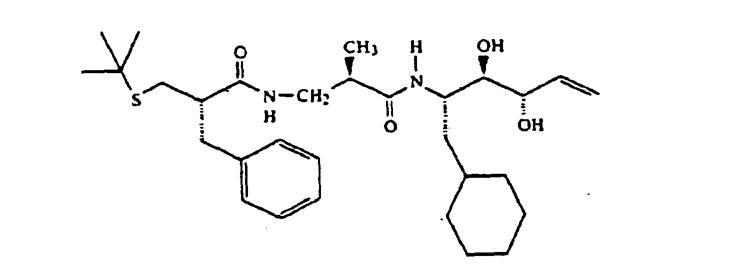 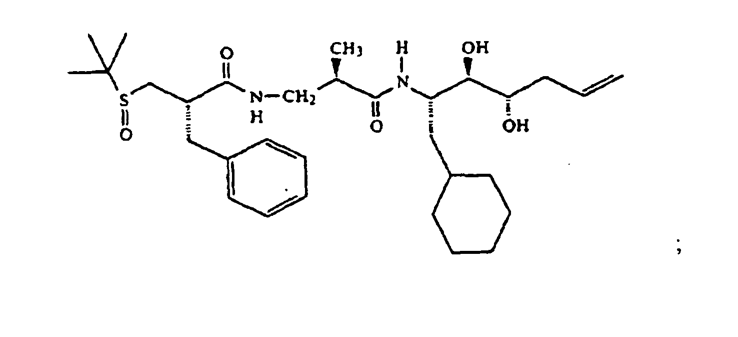 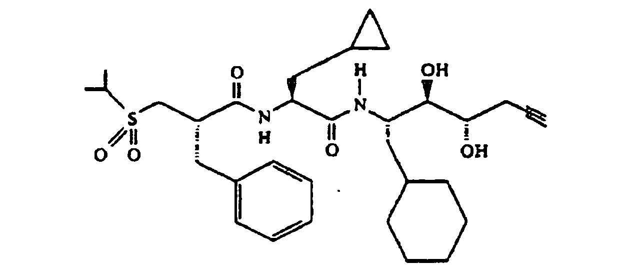 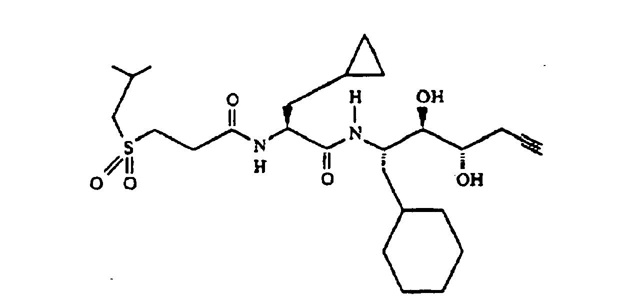 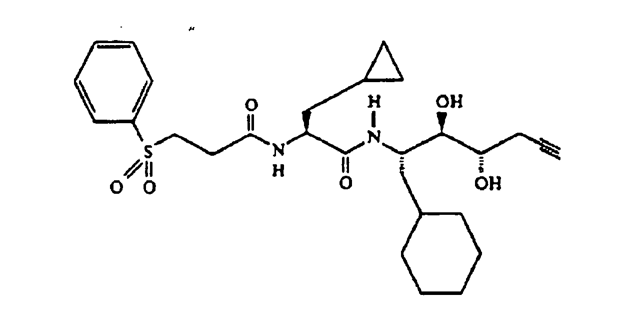 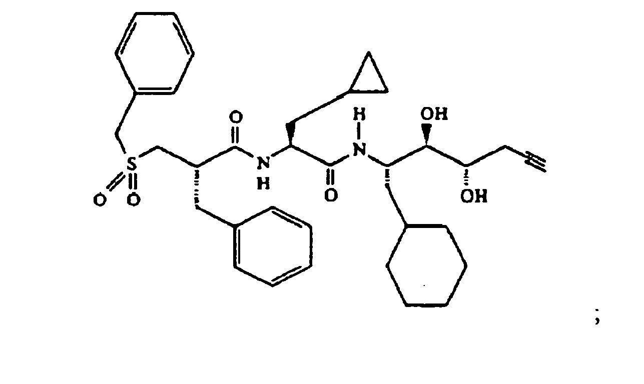 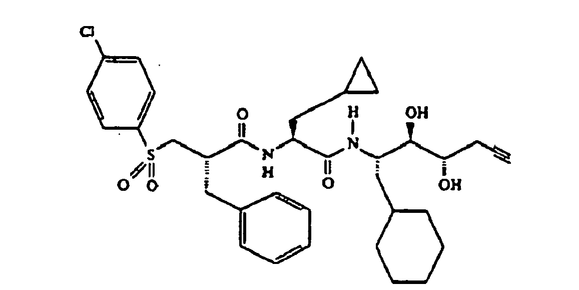 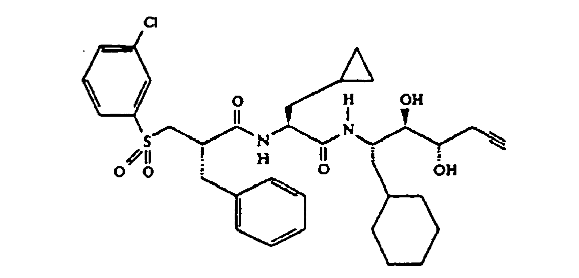 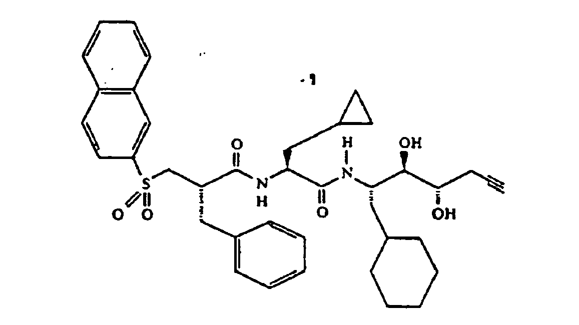 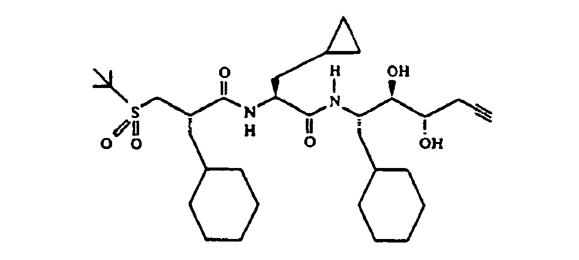 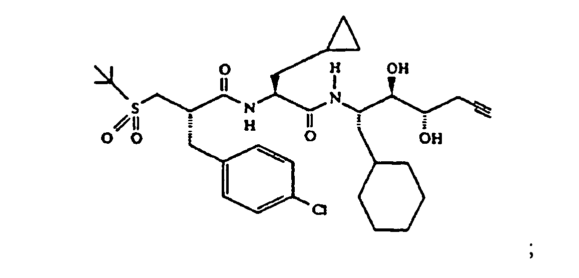 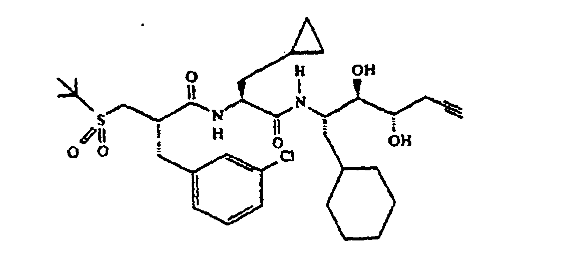 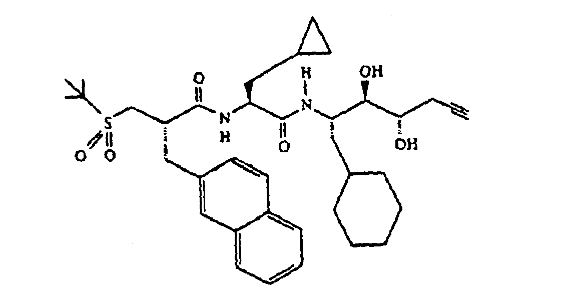 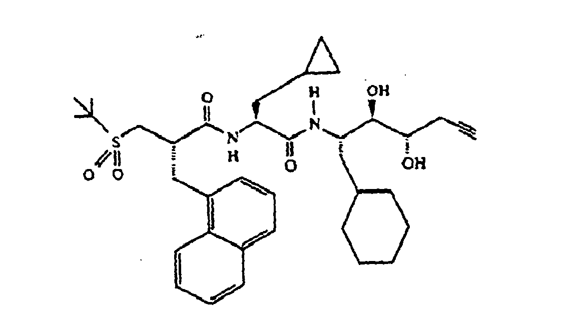 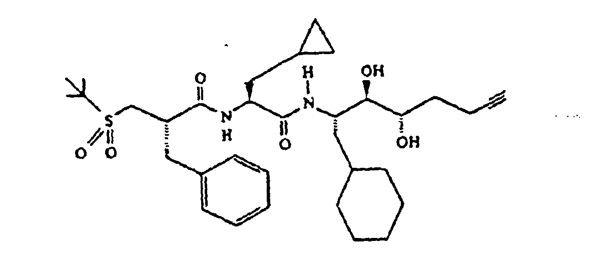 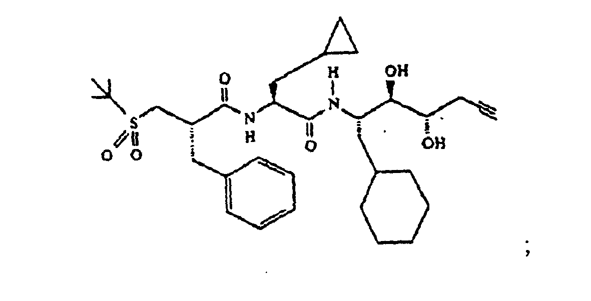 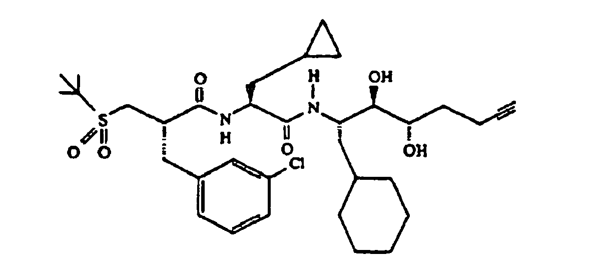 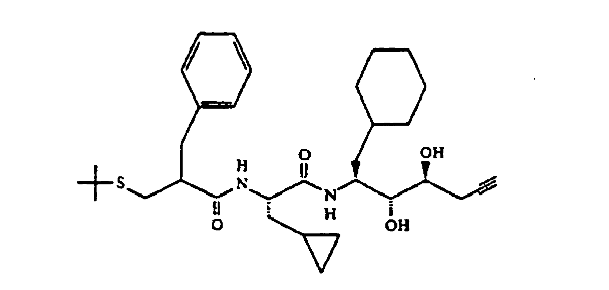 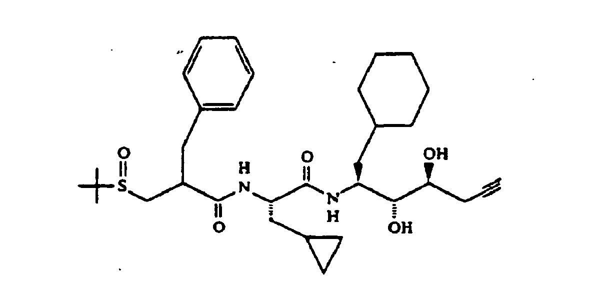 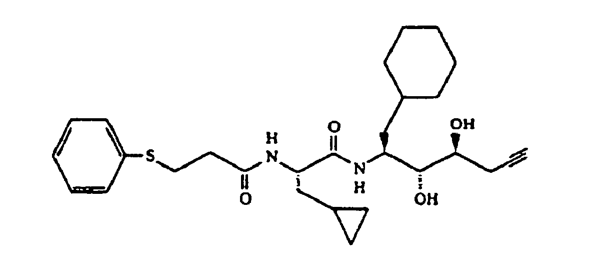 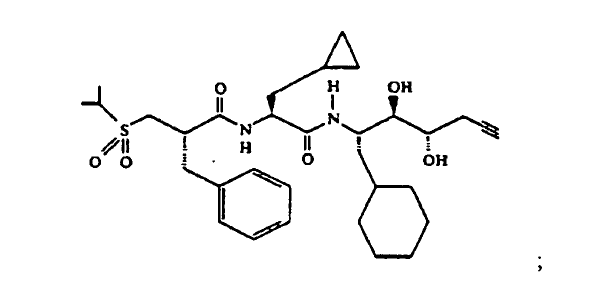 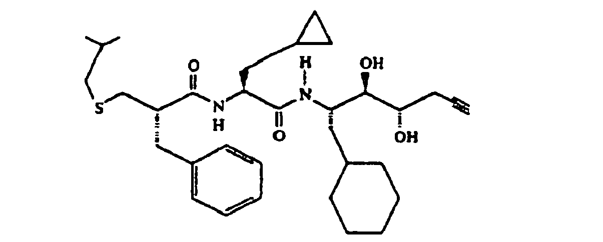 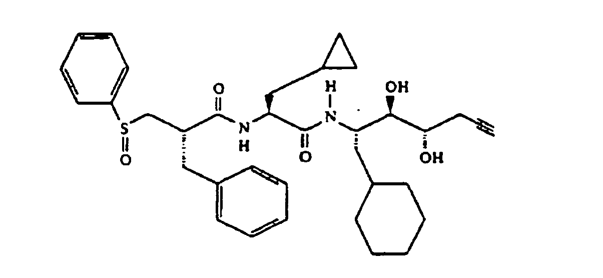 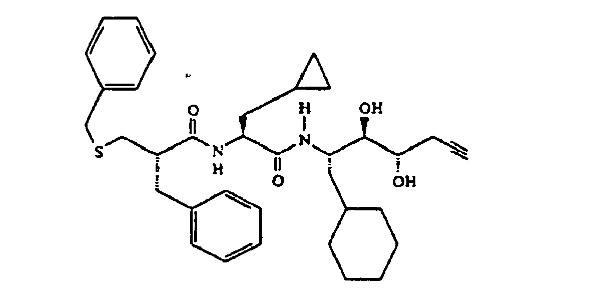 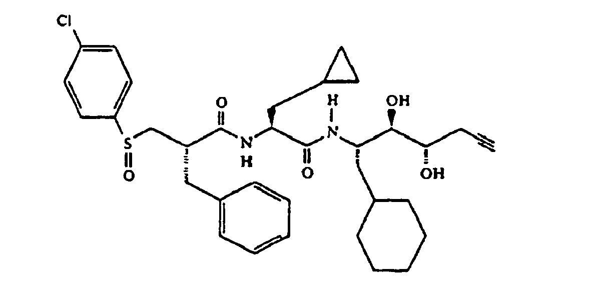 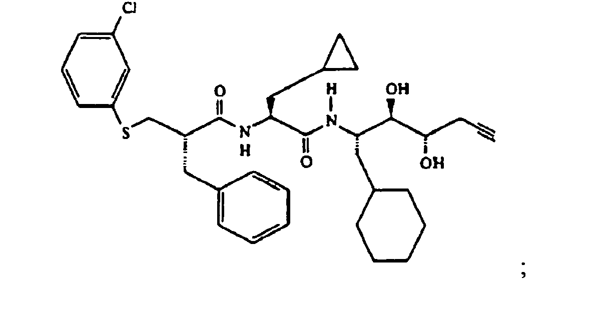 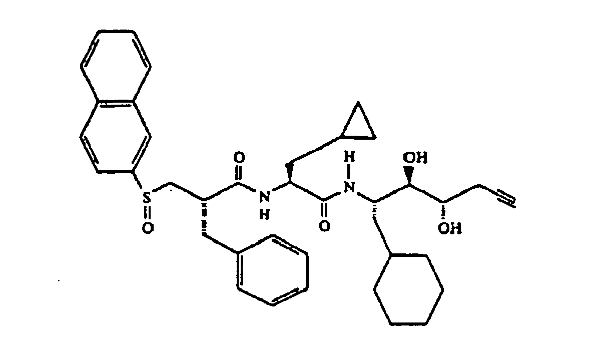 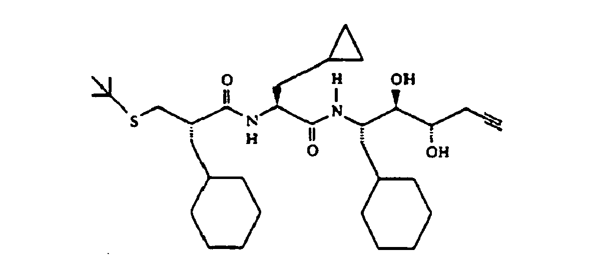 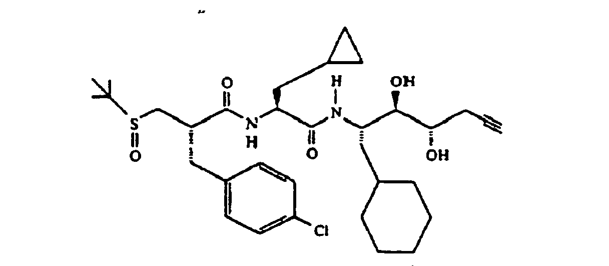 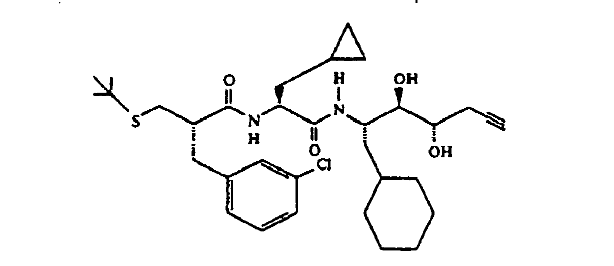 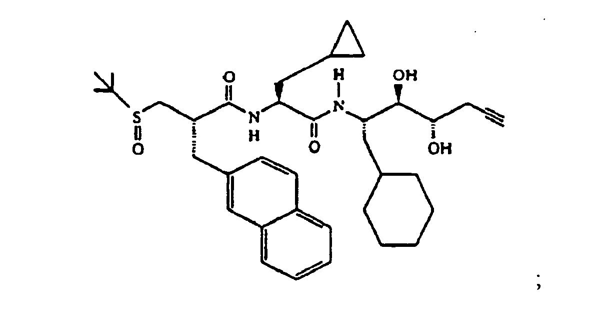 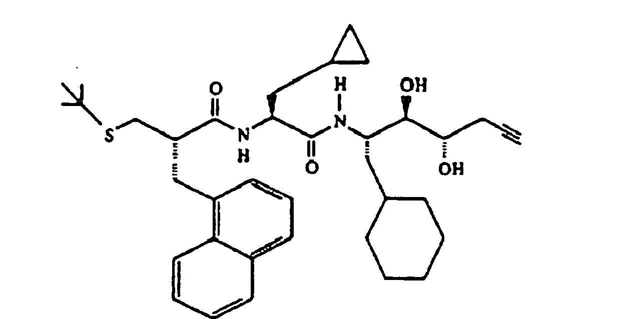 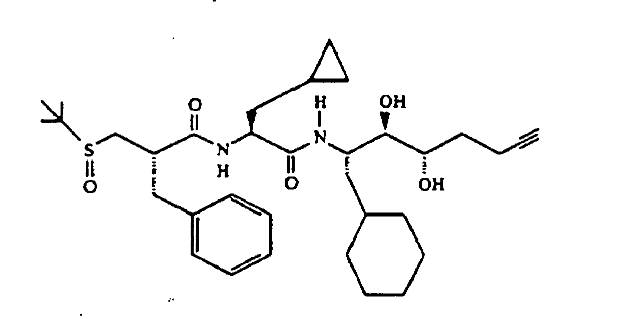 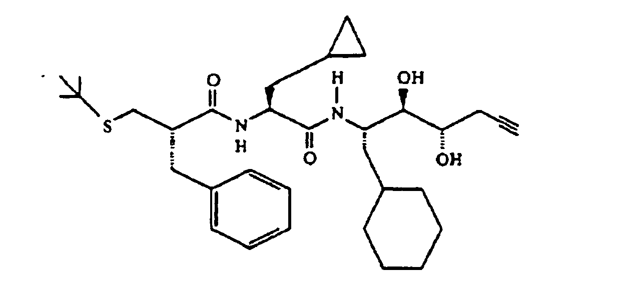 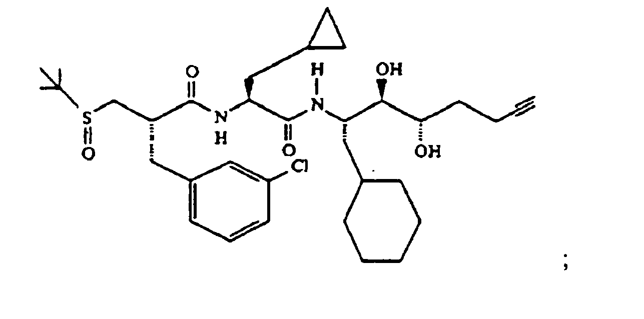 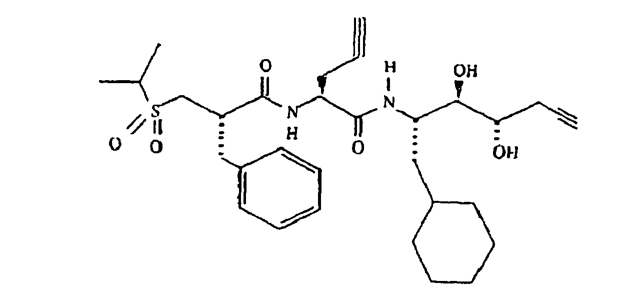 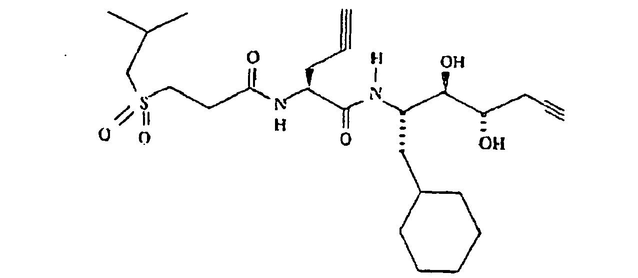 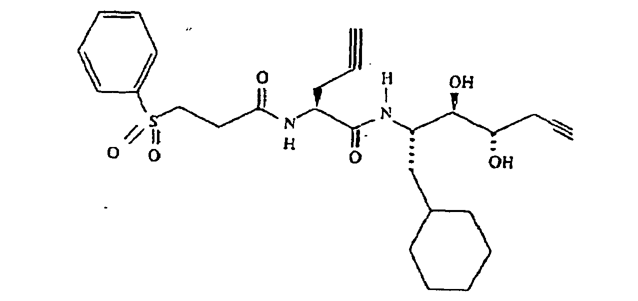 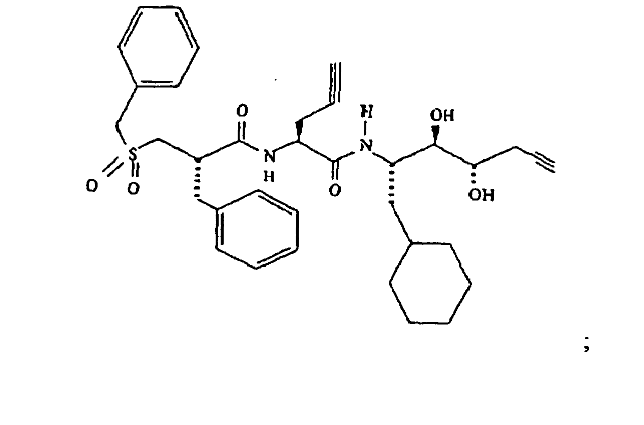 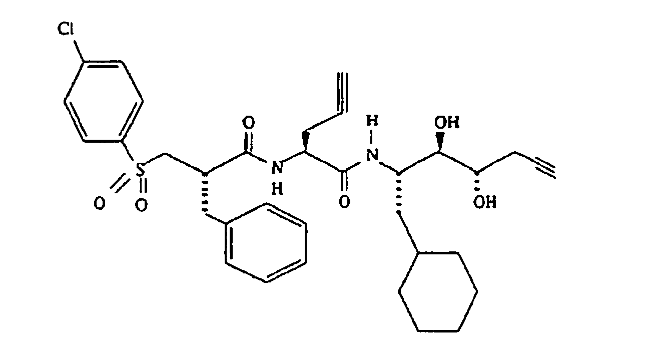 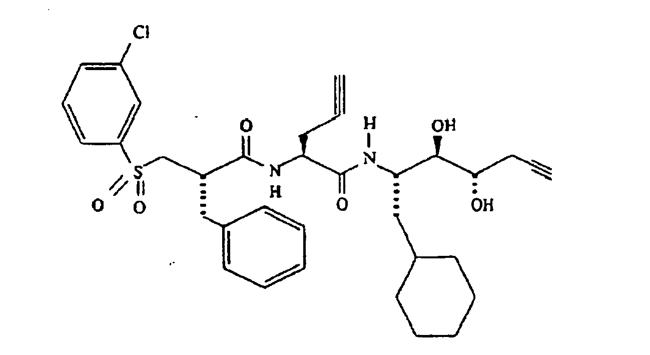 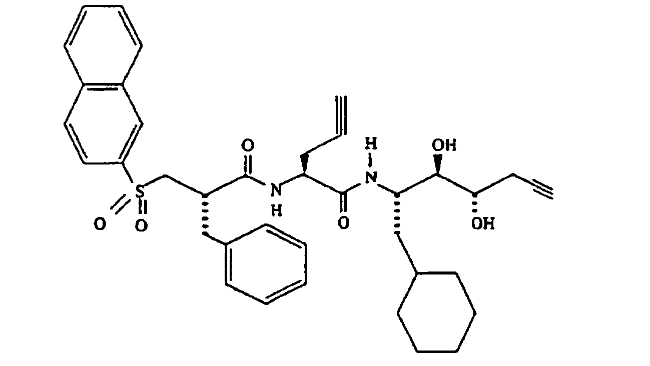 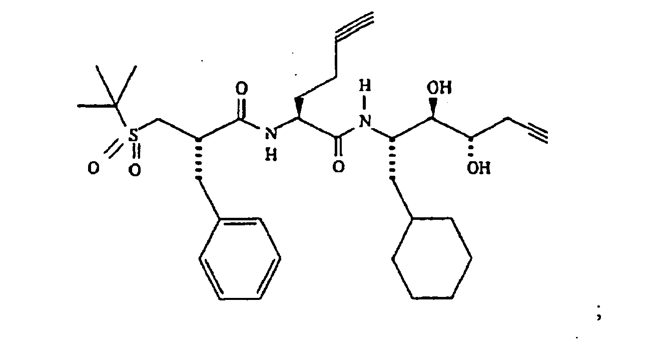 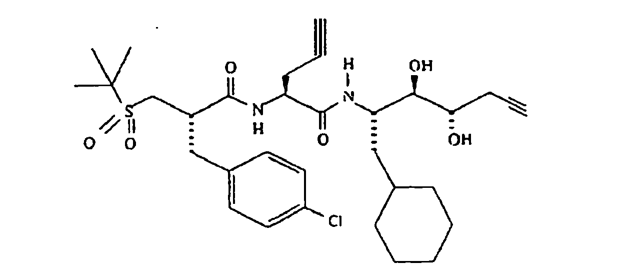 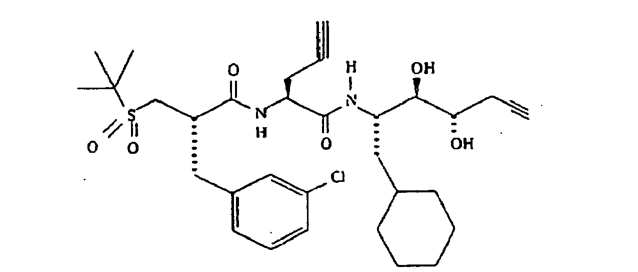 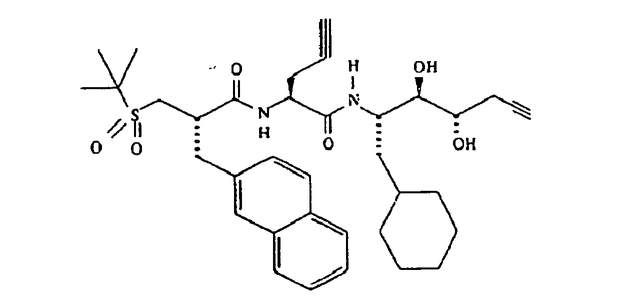 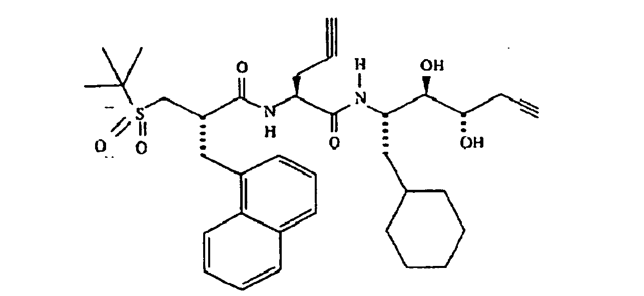 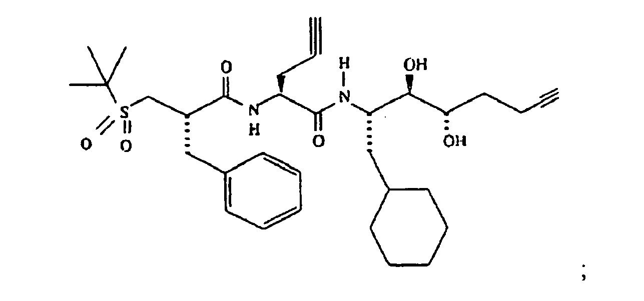 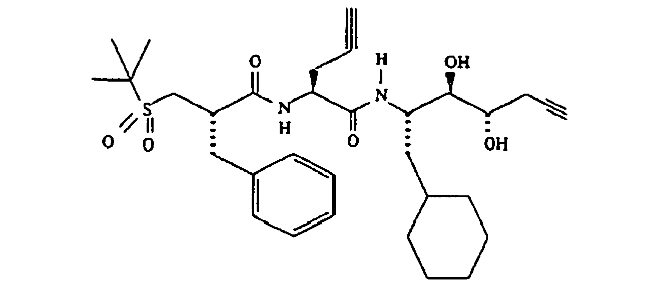 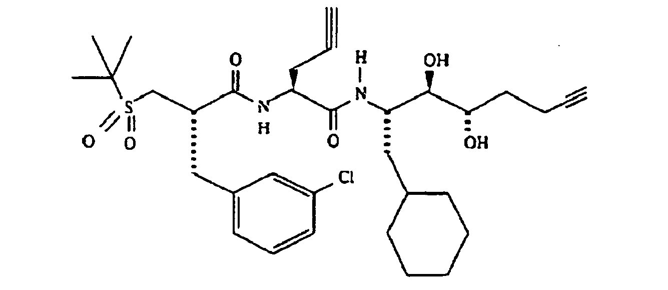 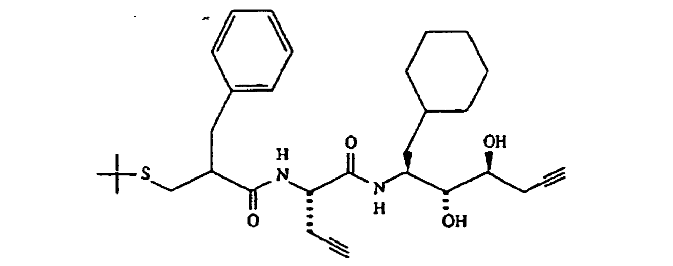 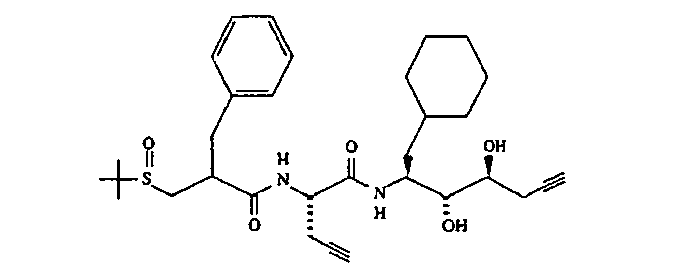 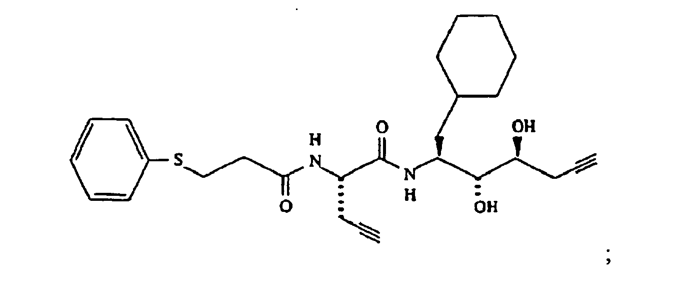 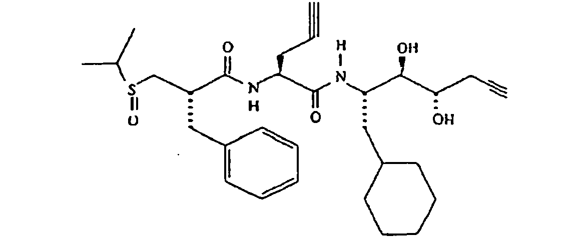 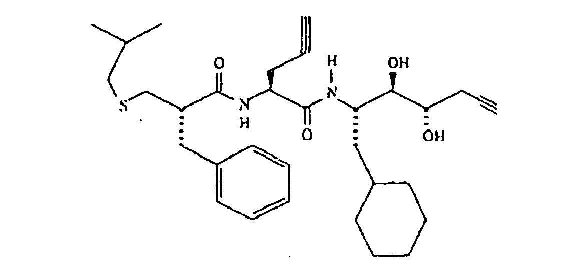 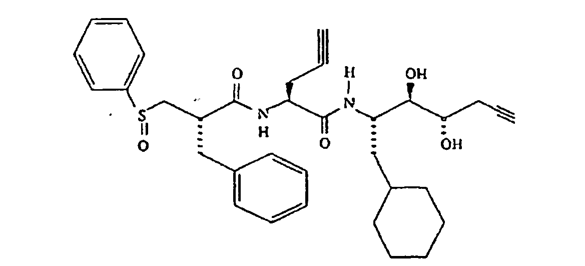 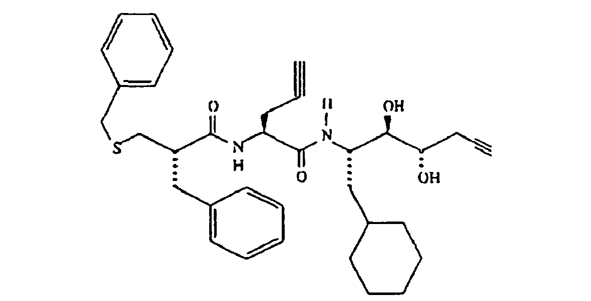 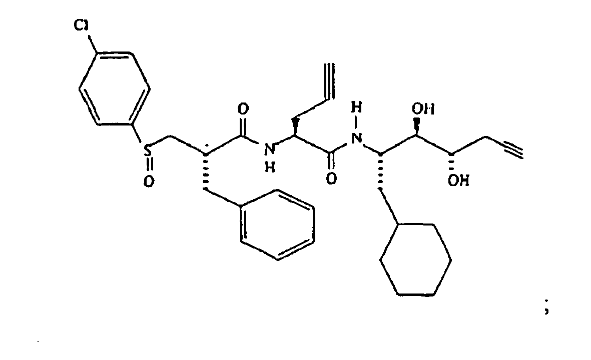 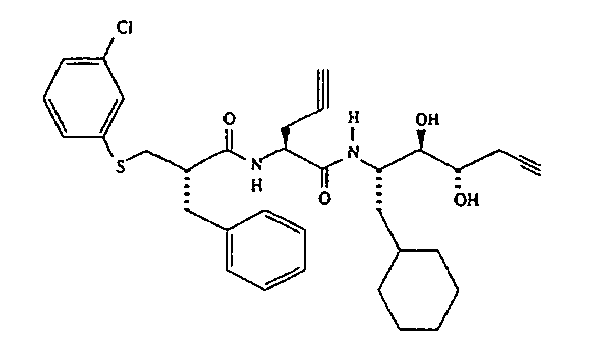 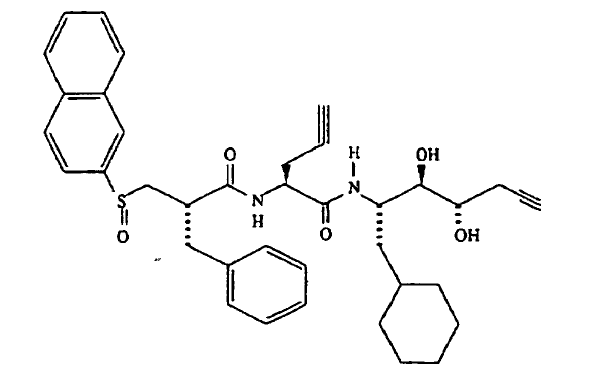 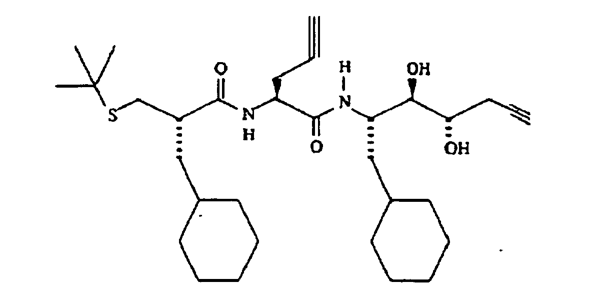 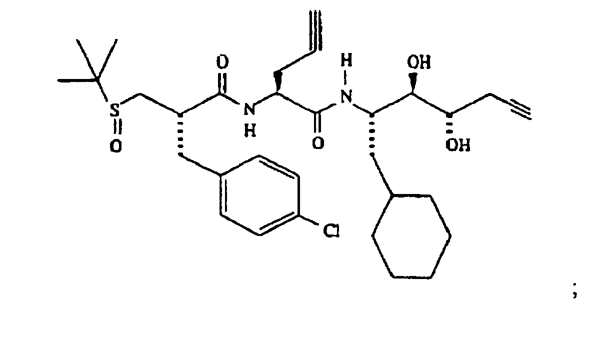 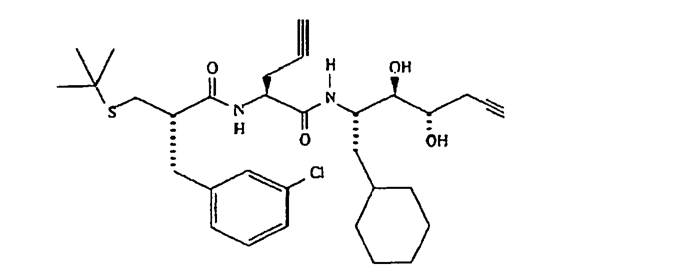 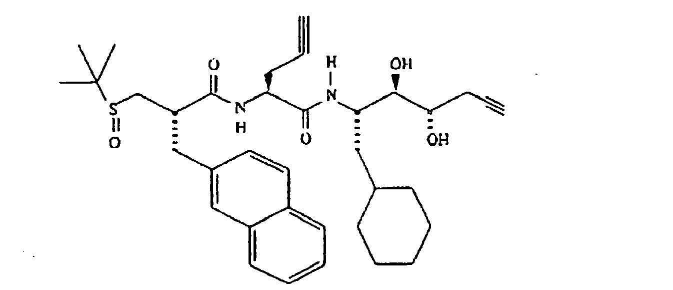 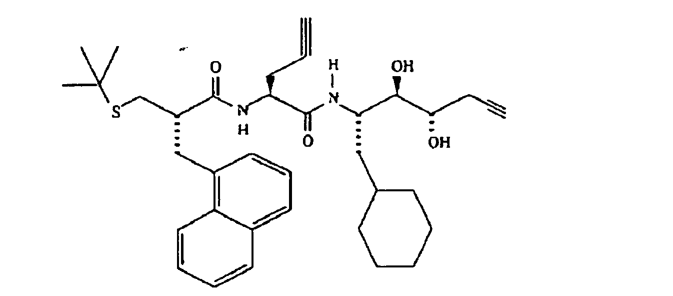 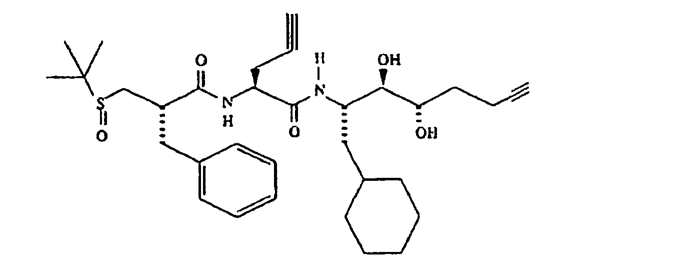 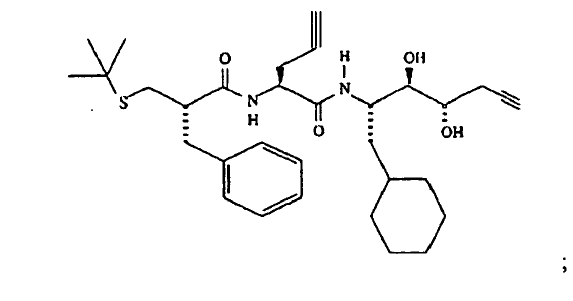 and 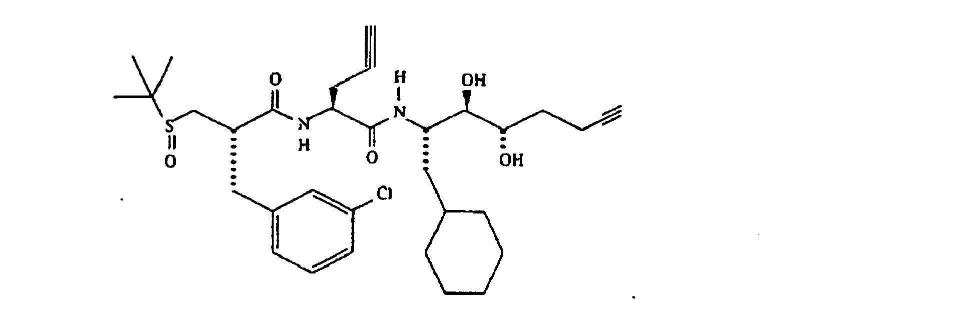
and mixtures thereof.

### Definitions

The compounds employed in the methods of this invention are identified in two ways: by descriptive names (using NamePro software from Advanced Chemistry Development, Inc.) and by reference to structures having various chemical moieties. The following terms may also be used and are defined below.

The term "modulating" refers to the ability of a compound to at least partially block the active site of the beta amyloid converting enzyme, thereby decreasing, or inhibiting the turnover rate of the enzyme.

The term "hydrido" denotes a single hydrogen atom (H). This hydrido group may be attached, for example, to an oxygen atom to form a hydroxyl group; or, as another example, one hydrido group may be attached to a carbon atom to form a >CH- group; or, as another example, two hydrido groups may be attached to a carbon atom to form a -CH₂- group.

The term "halo" refers to atoms including, for example, fluoro, chloro, bromo, or iodo.

Where the term "alkyl" is used, either alone or within other terms such as "haloalkyl" and "hydroxyalkyl", the term "alkyl" embraces linear or branched radicals having one to about twenty carbon atoms or, preferably, one to about twelve carbon atoms. The term "cycloalkyl" embraces cyclic radicals having three to about ten ring carbon atoms, preferably three to about six carbon atoms, such as cyclopropyl, cyclobutyl, cyclopentyl and cyclohexyl.

The term "haloalkyl" embraces radicals wherein any one or more of the alkyl carbon atoms is substituted with one or more halo groups, preferably selected from bromo, chloro and fluoro. Specifically embraced by the term "haloalkyl" are monohaloalkyl, dihaloalkyl and polyhaloalkyl groups. A monohaloalkyl group, for example, may have either a bromo, a chloro, or a fluoro atom within the group. Dihaloalkyl and polyhaloalkyl groups may be substituted with two or more of the same halo groups, or may have a combination of different halo groups. A dihaloalkyl group, for example, may have two fluoro atoms, such as difluoromethyl and difluorobutyl groups, or two chloro atoms, such as a dichloromethyl group, or one fluoro atom and one chloro atom, such as a fluoro-chloromethyl group. Examples of a polyhaloalkyl are trifluoromethyl, 1,1-difluoroethyl, 2,2,2-trifluoroethyl, perfluoroethyl and 2,2,3,3-tetrafluoropropyl groups. The term "difluoroalkyl" embraces alkyl groups having two fluoro atoms substituted on any one or two of the alkyl group carbon atoms.

The terms "alkylol" and "hydroxyalkyl" embrace linear or branched alkyl groups having one to about ten carbon atoms any one of which may be substituted with one or more hydroxyl groups.

The term "alkenyl" embraces linear or branched radicals having two to about twenty carbon atoms, preferably three to about ten carbon atoms, and containing at least one carbon--carbon double bond, which carbon-carbon double bond may have either cis or trans geometry within the alkenyl moiety. The term "alkenylalkyl" denotes a radical having a double-bond unsaturation site between two carbons, and which radical may consist of only two carbons or may be further substituted with alkyl groups which may optionally contain additional double-bond unsaturation.

The term "alkynyl" embraces linear or branched radicals having two to about twenty carbon atoms, preferably two to about ten carbon atoms, and containing at least one carbon--carbon triple bond. The term "cycloalkenyl" embraces cyclic radicals having three to about ten ring carbon atoms including one or more double bonds involving adjacent ring carbons.

The terms "alkoxy" and "alkoxyalkyl" embrace linear or branched oxy-containing radicals each having alkyl portions of one to about ten carbon atoms, such as methoxy group. The term "alkoxyalkyl" also embraces alkyl radicals having two or more alkoxy groups attached to the alkyl radical, that is, to form monoalkoxyalkyl and dialkoxyalkyl groups. The "alkoxy" or "alkoxyalkyl" radicals may be further substituted with one or more halo atoms, such as fluoro, chloro or bromo, to provide haloalkoxy or haloalkoxyalkyl groups.

The term "alkylthio" embraces radicals containing a linear or branched alkyl group, of one to about ten carbon atoms attached to a divalent sulfur atom, such as a methythio group.

The term "aryl" embraces aromatic radicals such as phenyl, naphthyl and biphenyl. The term "aralkyl" embraces aryl-substituted alkyl radicals such as benzyl, diphenylmethyl, triphenylmethyl, phenyl-ethyl, phenylbutyl and diphenylethyl. The terms "benzyl" and "phenylmethyl'' are interchangeable. Preferred aryl groups are those consisting of one, two, or three benzene rings. The terms "aryloxy" and "arylthio" denote radical respectively, aryl groups having an oxygen or sulfur atom through which the radical is attached to a nucleus, examples of which are phenoxy and phenylthio. The term "aralkoxy", alone or within another term, embraces an aryl group attached to an alkoxy group to form, for example, benzyloxy.

The terms "sulfido", "sulfinyl", and "sulfonyl", whether used alone or linked to other terms, denotes respectively divalent radicals S, SO, and SO₂.

The term "acyl" whether used alone, or within a term such as acyloxy, denotes a radical provided by the residue after removal of hydroxyl from an organic acid, examples of such radical being acetyl and benzoyl. "Lower alkanoyl" is an example of a more preferred sub-class of acyl.

The term "amido" denotes a radical consisting of nitrogen atom attached to a carbonyl group, which radical may be further substituted in the manner described herein. The amido radical can be attached to the nucleus of a compound of the invention through the carbonyl moiety or through the nitrogen atom of the amido radical.

The term "heterocyclic", as used alone or within groups such as "heterocyclicalkyl", and "heterocycliccycloalkyl", (hereinafter referred to as "heterocyclic-containing groups") embraces radicals having a saturated, or partially unsaturated, or fully saturated heterocyclic group, wherein the cyclic portion consists of a ring system having one ring or two fused rings, which ring system contains one, two or three hetero atoms as ring members selected from nitrogen, oxygen and sulfur, and which ring system has 4 to about 12 ring members. Non-limting examples of saturated heterocyclic-containing groups are pyrrolidinyl, piperidinyl, pyrrolidinylmethyl, piperidinylmethyl, pyrrolidinylcyclopropyl and piperidinylcyclopropyl. The term "heteroaryl", whether used alone or within the greater terms "heteroarylalkyl" or "heteroarylcycloalkyl", denotes a subset of "heterocyclic-containing groups" having a cyclic portion which is fully-unsaturated, that is, aromatic in character, and which has one or two hetero atoms as ring members, said hetero atoms selected from oxygen, sulfur and nitrogen atoms, and which ring system has five or six ring members. The "heteroaryl" ring may be attached to a linear or branched alkyl radical having one to about ten carbon atoms or may be attached to a cycloalkyl radical having three to about nine carbon atoms. Non-limiting examples of such heteroarylalkyl or heteroarylcycloalkyl groups are pyrazolemethyl, pyrazoleethyl, pyridylmethyl, pyridylethyl, thiazolemethyl, thiazoleethyl, imidazolemethyl, imidazoleethyl, thienylmethyl, thienylethyl, furanylmethyl, furanylethyl, oxazolemethyl, oxazoleethyl, thiazolylcyclopropyl, imidazolecyclopropyl, thienylcyclopropyl, isoxazolemethyl, isoxazoleethyl, pyridazinemethyl, pyridazineethyl, pyrazinemethyl and pyrazineethyl. The "heterocyclic" portion or "heteroaryl" portion of the radical, as well as the alkyl or cycloalkyl portion of groups containing a "heterocyclic" or "heteroaryl" portion, may be substituted at a substitutable position with one or more groups selected from oxo, alkyl, alkoxy, halo, haloalkyl, cyano, aralkyl, aralkoxy, aryl and aryloxy. Such "heterocyclic", "heterocyclic"-containing group, or "heteroaryl" group may be attached as a substituent through a carbon atom of the hetero ring system, or may be attached through a carbon atom of a moiety substituted on a hetero ring-member carbon atom, for example, through the methylene substituent ot an imidazolemethyl moiety. Also, a heterocyclic or heterocyclic-containing group may be attached through a ring nitrogen atom.

The pharmaceutically-acceptable salts of the compounds of Formulas (I)-(X) (in the form of water- or oil-soluble or dispersible products) include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenechyl bromides and others. Other pharmaceutically acceptable salts include the sulfate salt ethanolate and sulfate salts.

Some abbreviations that may appear in this application are as follows:

### ABBREVIATIONS

| Designation | Protecting Group |
|---|---|
| BOC (Boc) | t-butyloxycarbonyl |
| CBZ (Cbz) | benzyloxycarbonyl(carbobenzoxy) |
| TBS (TBDMS) | t-butyl-dimethylsilyl |

### Activating Group

| | |
|---|---|
| HBT(HOBT or HOBt) | 1-hydroxybenzotriazole hydrate |

| Designation | Coupling Reagent |
|---|---|
| BOP reagent | benzotriazol-1-yloxytris-(dimethylamino) phosphonium hexafluorophosphate |
| BOP-C1 | bis(2-oxo-3-oxazolidinyl)phosphinic chloride |
| EDC | 1-ethyl-3-(3-dimethyl-aminopropyl) carbodiimide hydrochloride |

### Other

| | |
|---|---|
| (BOC)₂O (BOC₂O) | di-t-butyl dicarbonate |
| n-Bu₄N⁺F⁻ | tetrabutyl ammonium fluoride |
| nBuLi (n-Buli) | n-butyllithium |
| DMF | dimethylformamide |
| Et₃N | triethylamine |
| EtOAc | ethyl acetate |
| TFA | trifluoroacetic acid |
| DMAP | dimethylaminopyridine |
| DME | dimethoxyethane |
| LDA | lithium diisopropylamide |
| THF | tetrahydrofuran |

### Amino Acid

| | |
|---|---|
| Ile | L-isoleucine |
| Val | L-valine |

In one aspect, this method of treatment can be used where the disease is Alzheimer's disease.

In another aspect, this method of treatment can help prevent or delay the onset of Alzheimer's disease.

In another aspect, this method of treatment can help slow the progression of Alzheimer's disease.

In another aspect, this method of treatment can be used where the disease is mild cognitive impairment.

In another aspect, this method of treatment can be used where the disease is Down's syndrome.

In another aspect, this method of treatment can be used where the disease is Hereditary Cerebral Hemorrhage with Amyloidosis of the Dutch-Type.

In another aspect, this method of treatment can be used where the disease is cerebral amyloid angiopathy.

In another aspect, this method of treatment can be used where the disease is degenerative dementias.

In another aspect, this method of treatment can be used where the disease is diffuse Lewy body type of Alzheimer's disease.

In another aspect, this method of treatment can treat an existing disease, such as those listed above.

In another aspect, this method of treatment can prevent a disease, such as those listed above, from developing or progressing.

The methods of the invention employ therapeutically effective amounts: for oral administration from about 0.1 mg/day to about 1,000 mg/day; for parenteral, sublingual, intranasal, intrathecal administration from about 0.5 to about 100 mg/day; for depo administration and implants from about 0.5 mg/day to about 50 mg/day; for topical administration from about 0.5 mg/day to about 200 mg/day; for rectal administration from about 0.5 mg to about 500 mg.

In a preferred aspect, the therapeutically effective amounts for oral administration is from about 1 mg/day to about 100 mg/day; and for parenteral administration from about 5 to about 50 mg daily.

In a more preferred aspect, the therapeutically effective amounts for oral administration is from about 5 mg/day to about 50 mg/day.

The present invention also includes the use of a compound of formula (I)-(X), or a pharmaceutically acceptable salt thereof for the manufacture of a medicament for use in treating a subject who has, or in preventing a subject from developing, a disease or condition selected from the group consisting of Alzheimer's disease, for helping prevent or delay the onset of Alzheimer's disease, for treating subjects with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, diffuse Lewy body type of Alzheimer's disease and who is in need of such treatment.

In one aspect, this use of a compound of formula (I)-(X) can be employed where the disease is Alzheimer's disease.

In another aspect, this use of a compound of formula (I)-(X) can help prevent or delay the onset of Alzheimer's disease.

In another aspect, this use of a compound of formula (I)-(X) can help slow the progression of Alzheimer's disease.

In another aspect, this use of a compound of formula (I)-(X) can be employed where the disease is mild cognitive impairment.

In another aspect, this use of a compound of formula (I)-(X) can be employed where the disease is Down's syndrome.

In another aspect, this use of a compound of formula (I)-(X) can be employed where the disease is Hereditary Cerebral Hemorrhage with Amyloidosis of the Dutch-Type.

In another aspect, this use of a compound of formula (I)-(X) can be employed where the disease is cerebral amyloid angiopathy.

In another aspect, this use of a compound of formula (I)-(X) can be employed where the disease is degenerative dementias.

In another aspect, this use of a compound of formula (I)-(X) can be employed where the disease is diffuse Lewy body type ot Alzheimer's disease.

In a preferred aspect, this use of a compound of formula (I)-(X) is a pharmaceutically acceptable salt of an acid selected from the group consisting of acids hydrochloric, hydrobromic, hydroiodic, nitric, sulfuric, phosphoric, citric, methanesulfonic, CH₃-(CH₂)ₙ-COOH where n is 0 thru 4, HOOC- (CH₂) ₙ-COOH where n is as defined above, EHOOC-CH=ECH-COOH, and phenyl-COOH.

In another preferred aspect of the invention, the subject or patient is preferably a human subject or patient.

The present invention also includes methods for inhibiting beta-secretase activity, for inhibiting cleavage of amyloid precursor protein (APP), in a reaction mixture, at a site between Met596 and Asp597, numbered for the APP-695 amino acid isotype, or at a corresponding site of an isotype or mutant thereof; for inhibiting production of amyloid beta peptide (A beta) in a cell; for inhibiting the production of beta-amyloid plaque in an animal; and for treating or preventing a disease characterized by beta-amyloid deposits in the brain. These methods each include administration of a therapeutically effective amount of a compound of formula (I), or a pharmaceutically acceptable salt thereof.

The present invention also includes a method for inhibiting beta-secretase activity, including exposing said beta-secretase to an effective inhibitory amount of a compound of formula (I), or a pharmaceutically acceptable salt thereof.

In one aspect, this method includes exposing said beta-secretase to said compound *in vitro.*

In another aspect, this method includes exposing said beta-secretase to said compound in a cell.

In another aspect, this method includes exposing said beta-secretase to said compound in a cell in an animal.

In another aspect, this method includes exposing said beta-secretase to said compound in a human.

The present invention also includes a method for inhibiting cleavage of amyloid precursor protein (APP), in a reaction mixture, at a site between Met596 and Asp597, numbered for the APP-695 amino acid isotype; or at a corresponding site of an isotype or mutant thereof, including exposing said reaction mixture to an effective inhibitory amount of a compound of formula (I)-(X), or a pharmaceutically acceptable salt thereof.

In one aspect, this method employs a cleavage site: between Met652 and Asp653, numbered for the APP-751 isotype; between Met 671 and Asp 672, numbered for the APP-770 isotype; between Leu596 and Asp597 of the APP-695 Swedish Mutation; between Leu652 and Asp653 of the APP-751 Swedish Mutation; or between Leu671 and Asp672 of the APP-770 Swedish Mutation.

In another aspect, this method exposes said reaction mixture *in vitro.*

In another aspect, this method exposes said reaction mixture in a cell.

In another aspect, this method exposes said reaction mixture in an animal cell.

In another aspect, this method exposes said reaction mixture in a human cell.

The present invention also includes a method for inhibiting production of amyloid beta peptide (A beta) in a cell, including administering to said cell an effective inhibitory amount of a compound of formula (I)-(X), or a pharmaceutically acceptable salt thereof.

In an embodiment, this method includes administering to an animal.

In an embodiment, this method includes administering to a human.

The present invention also includes a method for inhibiting the production of beta-amyloid plaque in an animal, including administering to said animal an effective inhibitory amount of a compound of formula (1)-(X), or a pharmaceutically acceptable salt thereof.

In one embodiment of this aspect, this method includes administering to a human.

The present invention also includes a method for treating or preventing a disease characterized by beta-amyloid deposits in the brain including administering to a subject an effective therapeutic amount of a compound of formula (I)-(X), or a pharmaceutically acceptable salt thereof.

In one aspect, this method employs a compound at a therapeutic amount in the range of from about 0.1 to about 1000 mg/day.

In another aspect, this method employs a compound at a therapeutic amount in the range of from about 15 to about 1500 mg/day.

In another aspect, this method employs a compound at a therapeutic amount in the range of from about 1 to about 100 mg/day.

In another aspect, this method employs a compound at a therapeutic amount in the range of from about 5 to about 50 mg/day.

In another aspect, this method can be used where said disease is Alzheimer's disease.

In another aspect, this method can be used where said disease is Mild Cognitive Impairment, Down's Syndrome, of Hereditary Cerebral Hemorrhage with Amyloidosis of the Dutch Type.

The present invention also includes a composition including beta-secretase complexed with a compound of formula (I)-(X), or a pharmaceutically acceptable salt thereof.

The present invention also includes a method for producing a beta-secretase complex including exposing beta-secretase to a compound of formula (I)-(X), or a pharmaceutically acceptable salt thereof, in a reaction mixture under conditions suitable for the production of said complex.

In an embodiment, this method employs exposing in vitro.

In an embodiment, this method employs a reaction mixture that is a cell.

The present invention also includes a component kit including component parts capable of being assembled, in which at least one component part includes a compound of formula (I)-(X) enclosed in a container.

In an embodiment, this component kit includes lyophilized compound, and at least one further component part includes a diluent.

The present invention also includes a container kit including a plurality of containers, each container including one or more unit dose of a compound of formula (I)-(X), or a pharmaceutically acceptable salt thereof.

In an embodiment, this container kit includes each container adapted for oral delivery and includes a tablet, gel, or capsule.

In an embodiment, this container kit includes each container adapted for parenteral delivery and includes a depot product, syringe, ampoule, or vial.

In an embodiment, this container kit includes each container adapted for topical delivery and includes a patch, medipad, ointment, or cream.

The present invention also includes an agent kit including a compound of formula (I)-(X), or a pharmaceutically acceptable salt thereof; and one or more therapeutic agents selected from the group consisting of an antioxidant, an anti-inflammatory, a gamma secretase inhibitor, a neurotrophic agent, an acetyl cholinesterase inhibitor, a statin, an A beta peptide, and an anti-A beta antibody.

The present invention provides compounds, compositions, kits, and methods for inhibiting beta-secretase-mediated cleavage of amyloid precursor protein (APP). More particularly, the compounds, compositions, and methods of the invention are effective to inhibit the production of A beta peptide and to treat or prevent any human or veterinary disease or condition associated with a pathological form of A beta peptide.

The compounds, compositions, and methods of the invention are useful for treating humans who have Alzheimer's Disease (AD), for helping prevent or delay the onset of AD, for treating subjects with mild cognitive impairment (MCI), and preventing or delaying the onset of AD in those subjects who would otherwise be expected to progress from MCI to AD, for treating Down's syndrome, for treating Hereditary Cerebral Hemorrhage with Amyloidosis of the Dutch Type, for treating cerebral beta-amyloid angiopathy and preventing its potential consequences such as single and recurrent lobar hemorrhages, for treating other degenerative dementias, including dementias of mixed vascular and degenerative origin, for treating dementia associated with Parkinson's disease, frontotemporal dementias with parkinsonism (FTDP), dementia associated with progressive supranuclear palsy, dementia associated with cortical basal degeneration, and diffuse Lewy body type AD.

The compounds of the invention possess beta-secretase inhibitory activity. The inhibitory activities of the compounds of the invention are readily demonstrated, for example, using one or more of the assays described herein or known in the art.

The compounds of formula (I)-(X) can form salts when reacted with acids. Pharmaceutically acceptable salts are generally preferred over the corresponding compounds of formula (I)-(X) since they frequently produce compounds which are usually more water soluble, stable and/or more crystalline. Pharmaceutically acceptable salts are any salt which retains the activity of the parent compound and does not impart any deleterious or undesirable effect on the subject to whom it is administered and in the context in which it is administered. Pharmaceutically acceptable salts include acid addition salts of both inorganic and organic acids.

### Methods of the Invention

The compounds of the invention, and pharmaceutically acceptable salts thereof, are useful for treating humans or animals suffering from a condition characterized by a pathological form of beta-amyloid peptide, such as beta-amyloid plaques, and for helping to prevent or delay the onset of such a condition. For example, the compounds are useful for treating Alzheimer's disease, for helping prevent or delay the onset of Alzheimer's disease, for treating subjects with MCI (mild cognitive impairment) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobal hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, and diffuse Lewy body type Alzheimer's disease. The compounds and compositions of the invention are particularly useful for treating, preventing, or slowing the progression of Alzheimer's disease. When treating or preventing these diseases, the compounds of the invention can either be used individually or in combination, as is best for the subject or subject.

With regard to these diseases, the term "treating" means that compounds of the invention can be used in humans with existing disease. The compounds of the invention will not necessarily cure the subject who has the disease but will delay or slow the progression or prevent further progression of the disease thereby giving the individual a more useful life span.

The term "preventing" means that that if the compounds of the invention are administered to those who do not now have the disease but who would normally develop the disease or be at increased risk for the disease, they will not develop the disease. In addition, "preventing" also includes delaying the development of the disease in an individual who will ultimately develop the disease or would be at risk for the disease due to age, familial history, genetic or chromosomal abnormalities, and/or due to the presence of one or more biological markers for the disease, such as a known genetic mutation of APP or APP cleavage products in brain tissues or fluids. By delaying the onset of the disease, compounds of the invention have prevented the individual from getting the disease during the period in which the individual would normally have gotten the disease or reduce the rate of development of the disease or some of its effects but for the administration of compounds of the invention up to the time the individual ultimately gets the disease. Preventing also includes administration of the compounds of the invention to those individuals thought to be predisposed to the disease.

In a preferred aspect, the compounds of the invention are useful for slowing the progression of disease symptoms.

In another preferred aspect, the compounds of the invention are useful for preventing the further progression of disease symptoms.

In treating or preventing the above diseases, the compounds of the invention are administered in a therapeutically effective amount. The therapeutically effective amount will vary depending on the particular compound used and the route of administration, as is known to those skilled in the art.

In treating a subject displaying any of the diagnosed above conditions a physician may administer a compound of the invention immediately and continue administration indefinitely, as needed. In treating subjects who are not diagnosed as having Alzheimer's disease, but who are believed to be at substantial risk for Alzheimer's disease, the physician should preferably start treatment when the subject first experiences early pre-Alzheimer's symptoms such as, memory or cognitive problems associated with aging. In addition, there are some subjects who may be determined to be at risk for developing Alzheimer's through the detection of a genetic marker such as APOE4 or other biological indicators that are predictive for Alzheimer's disease. In these situations, even though the subject does not have symptoms of the disease, administration of the compounds of the invention may be started before symptoms appear, and treatment may be continued indefinitely to prevent or delay the onset of the disease.

### Dosage Forms and Amounts

The compounds of the invention can be administered orally, parenterally, (IV, IM, depo-IM, SQ, and depo SQ), sublingually, intranasally (inhalation), intrathecally, topically, or rectally. Dosage forms known to those of skill in the art are suitable for delivery of the compounds of the invention.

Compositions are provided that contain therapeutically effective amounts of the compounds of the invention. The compounds are preferably formulated into suitable pharmaceutical preparations such as tablets, capsules, or elixirs for oral administration or in sterile solutions or suspensions for parenteral administration. Typically the compounds described above are formulated into pharmaceutical compositions using techniques and procedures well known in the art.

About 1 to 500 mg of a compound or mixture of compounds of the invention or a physiologically acceptable salt or ester is compounded with a physiologically acceptable vehicle, carrier, excipient, binder, preservative, stabilizer, flavor, etc., in a unit dosage form as called for by accepted pharmaceutical practice. The amount of active substance in those compositions or preparations is such that a suitable dosage in the range indicated is obtained. The compositions are preferably formulated in a unit dosage form, each dosage containing from about 2 to about 100 mg, more preferably about 10 to about 30 mg of the active ingredient. The term "unit dosage from" refers to physically discrete units suitable as unitary dosages for human subjects and other mammals, each unit containing a predetermined quantity of active material calculated to produce the desired therapeutic effect, in association with a suitable pharmaceutical excipient.

To prepare compositions, one or more compounds of the invention are mixed with a suitable pharmaceutically acceptable carrier. Upon mixing or addition of the compound(s), the resulting mixture may be a solution, suspension, emulsion, or the like. Liposomal suspensions may also be suitable as pharmaceutically acceptable carriers. These may be prepared according to methods known to those skilled in the art. The form of the resulting mixture depends upon a number of factors, including the intended mode of administration and the solubility of the compound in the selected carrier or vehicle. The effective concentration is sufficient for lessening or ameliorating at least one symptom of the disease, disorder, or condition treated and may be empirically determined.

Pharmaceutical carriers or vehicles suitable for administration of the compounds provided herein include any such carriers known to those skilled in the art to be suitable for the particular mode of administration. In addition, the active materials can also be mixed with other active materials that do not impair the desired action, or with materials that supplement the desired action, or have another action. The compounds may be formulated as the sole pharmaceutically active ingredient in the composition or may be combined with other active ingredients.

Where the compounds exhibit insufficient solubility, methods for solubilizing may be used. Such methods are known and include, but are not limited to, using cosolvents such as dimethylsulfoxide (DMSO), using surfactants such as Tween®, and dissolution in aqueous sodium bicarbonate. Derivatives of the compounds, such as salts or prodrugs may also be used in formulating effective pharmaceutical compositions.

The concentration of the compound is effective for delivery of an amount upon administration that lessens or ameliorates at least one symptom of the disorder for which the compound is administered. Typically, the compositions are formulated for single dosage administration.

The compounds of the invention may be prepared with carriers that protect them against rapid elimination from the body, such as time-release formulations or coatings. Such carriers include controlled release formulations, such as, but not limited to, microencapsulated delivery systems. The active compound is included in the pharmaceutically acceptable carrier in an amount sufficient to exert a therapeutically useful effect in the absence of undesirable side effects on the subject treated. The therapeutically effective concentration may be determined empirically by testing the compounds in known in vitro and *in vivo* model systems for the treated disorder.

The compounds and compositions of the invention can be enclosed in multiple or single dose containers. The enclosed compounds and compositions can be provided in kits, for example, including component parts that can be assembled for use. For example, a compound inhibitor in lyophilized form and a suitable diluent may be provided as separated components for combination prior to use. A kit may include a compound inhibitor and a second therapeutic agent for co-administration. The inhibitor and second therapeutic agent may be provided as separate component parts. A kit may include a plurality of containers, each container holding one or more unit dose of the compound of the invention. The containers are preferably adapted for the desired mode of administration, including, but not limited to tablets, gel capsules, sustained-release capsules, and the like for oral administration; depot products, pre-filled syringes, ampoules, vials, and the like for parenteral administration; and patches, medipads, creams, and the like for topical administration.

The concentration of active compound in the drug composition will depend on absorption, inactivation, and excretion rates of the active compound, the dosage schedule, and amount administered as well as other factors known to those of skill in the art.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or in vitro test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

If oral administration is desired, the compound should be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

Oral compositions will generally include an inert diluent or an edible carrier and may be compressed into tablets or enclosed in gelatin capsules. For the purpose of oral therapeutic administration, the active compound or compounds can be incorporated with excipients and used in the form of tablets, capsules, or troches. Pharmaceutically compatible binding agents and adjuvant materials can be included as part of the composition.

The tablets, pills, capsules, troches, and the like can contain any of the following ingredients or compounds of a similar nature: a binder such as, but not limited to, gum tragacanth, acacia, corn starch, or gelatin; an excipient such as microcrystalline cellulose, starch, or lactose; a disintegrating agent such as, but not limited to, alginic acid and corn starch; a lubricant such as, but not limited to, magnesium stearate; a gildant, such as, but not limited to, colloidal silicon dioxide; a sweetening agent such as sucrose or saccharin; and a flavoring agent such as peppermint, methyl salicylate, or fruit flavoring.

When the dosage unit form is a capsule, it can contain, in addition to material of the above type, a liquid carrier such as a fatty oil. In addition, dosage unit forms can contain various other materials, which modify the physical form of the dosage unit, for example, coatings of sugar and other enteric agents. The compounds can also be administered as a component of an elixir, suspension, syrup, wafer, chewing gum or the like. A syrup may contain, in addition to the active compounds, sucrose as a sweetening agent and certain preservatives, dyes and colorings, and flavors.

The active materials can also be mixed with other active materials that do not impair the desired action, or with materials that supplement the desired action.

Solutions or suspensions used for parenteral, intradermal, subcutaneous, or topical application can include any of the following components: a sterile diluent such as water for injection, saline solution, fixed oil, a naturally occurring vegetable oil such as sesame oil, coconut oil, peanut oil, cottonseed oil, and the like, or a synthetic fatty vehicle such as ethyl oleate, and the like, polyethylene glycol, glycerine, propylene glycol, or other synthetic solvent; antimicrobial agents such as benzyl alcohol and methyl parabens; antioxidants such as ascorbic acid and sodium bisulfite; chelating agents such as ethylenediaminetetraacetic acid (EDTA); buffers such as acetates, citrates, and phosphates; and agents for the adjustment of tonicity such as sodium chloride and dextrose. Parenteral preparations can be enclosed in ampoules, disposable syringes, or multiple dose vials made of glass, plastic, or other suitable material. Buffers, preservatives, antioxidants, and the like can be incorporated as required.

Where administered intravenously, suitable carriers include physiological saline, phosphate buffered saline (PBS), and solutions containing thickening and solubilizing agents such as glucose, polyethylene glycol, polypropyleneglycol, and mixtures thereof. Liposomal suspensions including tissue-targeted liposomes may also be suitable as pharmaceutically acceptable carriers. These may be prepared according to methods known for example, as described in U.S. Patent No. 4,522,811.

The active compounds may be prepared with carriers that protect the compound against rapid elimination from the body, such as time-release formulations or coatings. Such carriers include controlled release formulations, such as, but not limited to, implants and microencapsulated delivery systems, and biodegradable, biocompatible polymers such as collagen, ethylene vinyl acetate, polyanhydrides, polyglycolic acid, polyorthoesters, polylactic acid, and the like. Methods for preparation of such formulations are known to those skilled in the art.

The compounds of the invention can be administered orally, parenterally (IV, IM, depo-IM, SQ, and depo-SQ), sublingually, intranasally (inhalation), intrathecally, topically, or rectally. Dosage forms known to those skilled in the art are suitable for delivery of the compounds of the invention.

Compounds of the invention may be administered enterally or parenterally. When administered orally, compounds of the invention can be administered in usual dosage forms for oral administration as is well known to those skilled in the art. These dosage forms include the usual solid unit dosage forms of tablets and capsules as well as liquid dosage forms such as solutions, suspensions, and elixirs. When the solid dosage forms are used, it is preferred that they be of the sustained release type so that the compounds of the invention need to be administered only once or twice daily.

The oral dosage forms are administered to the subject 1, 2, 3, or 4 times daily. It is preferred that the compounds of the invention be administered either three or fewer times, more preferably once or twice daily. Hence, it is preferred that the compounds of the invention be administered in oral dosage form. It is preferred that whatever oral dosage form is used, that it be designed so as to protect the compounds of the invention from the acidic environment of the stomach. Enteric coated tablets are well known to those skilled in the art. In addition, capsules filled with small spheres each coated to protect from the acidic stomach, are also well known to those skilled in the art.

When administered orally, an administered amount therapeutically effective to inhibit beta-secretase activity, to inhibit A beta production, to inhibit A beta deposition, or to treat or prevent AD is from about 0.1 mg/day to about 1,000 mg/day. It is preferred that the oral dosage is from about 1 mg/day to about 100 mg/day. It is more preferred that the oral dosage is from about 5 mg/day to about 50 mg/day. It is understood that while a subject may be started at one dose, that dose may be varied over time as the subject's condition changes.

Compounds of the invention may also be advantageously delivered in a nano crystal dispersion formulation. Preparation of such formulations is described, for example, in U.S. Patent 5,145,684. Nano crystalline dispersions of HIV protease inhibitors and their method of use are described in U.S. Patent No. 6,045,829. The nano crystalline formulations typically afford greater bioavailability of drug compounds.

The compounds of the invention can be administered parenterally, for example, by IV, IM, depo-IM, SC, or depo-SC. When administered parenterally, a therapeutically effective amount of about 0.5 to about 100 mg/day, preferably from about 5 to about 50 mg daily should be delivered. When a depot formulation is used for injection once a month or once every two weeks, the dose should be about 0.5 mg/day to about 50 mg/day, or a monthly dose of from about 15 mg to about 1,500 mg. In part because of the forgetfulness of the subjects with Alzheimer's disease, it is preferred that the parenteral dosage form be a depo formulation.

The compounds of the invention can be administered sublingually. When given sublingually, the compounds of the invention should be given one to four times daily in the amounts described above for IM administration.

The compounds of the invention can be administered intranasally. When given by this route, the appropriate dosage forms are a nasal spray or dry powder, as is known to those skilled in the art. The dosage of the compounds of the invention for intranasal administration is the amount described above for IM administration.

The compounds of the invention can be administered intrathecally. When given by this route the appropriate dosage form can be a parenteral dosage form as is known to those skilled in the art. The dosage of the compounds of the invention for intrathecal administration is the amount described above for IM administration.

The compounds of the invention can be administered topically. When given by this route, the appropriate dosage form is a cream, ointment, or patch. Because of the amount of the compounds of the invention to be administered, the patch is preferred. When administered topically, the dosage is from about 0.5 mg/day to about 200 mg/day. Because the amount that can be delivered by a patch is limited, two or more patches may be used. The number and size of the patch is not important, what is important is that a therapeutically effective amount of the compounds of the invention be delivered as is known to those skilled in the art. The compounds of the invention can be administered rectally by suppository as is known to those skilled in the art. When administered by suppository, the therapeutically effective amount is from about 0.5 mg to about 500 mg.

The compounds of the invention can be administered by implants as is known to those skilled in the art. When administering a compound of the invention by implant, the therapeutically effective amount is the amount described above for depot administration.

The invention here is the new compounds of the invention and new methods of using the compounds of the invention. Given a particular compound of the invention and a desired dosage form, one skilled in the art would know how to prepare and administer the appropriate dosage form.

The compounds of the invention are used in the same manner, by the same routes of administration, using the same pharmaceutical dosage forms, and at the same dosing schedule as described above, for preventing disease or treating subjects with MCI (mild cognitive impairment) and preventing or delaying the onset of Alzheimer's disease in those who would progress from MCI to AD, for treating or preventing Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, and diffuse Lewy body type of Alzheimer's disease.

The compounds of the invention can be used with each other or with other agents used to treat or prevent the conditions listed above. Such agents include gamma-secretase inhibitors, anti-amyloid vaccines and pharmaceutical agents such as donepezil hydrochloride (ARICEPT Tablets), tacrine hydrochloride (COGNEX Capsules) or other acetylcholine esterase inhibitors and with direct or indirectneurotropic agents of the future.

In addition, the compounds of the invention can also be used with inhibitors of P-glycoproten (P-gp). The use of P-gp inhibitors is known to those skilled in the art. See for example, *Cancer Research,* 53, 4595-4602 (1993), Clin. *Cancer Res.,* 2, 7-12 (1996), *Cancer Research,* 56, 4171-4179 (1996), International Publications WO99/64001 and WO01/10387. The important thing is that the blood level of the P-gp inhibitor be such that it exerts its effect in inhibiting P-gp from decreasing brain blood levels of the compounds of the invention. To that end the P-gp inhibitor and the compounds of the invention can be administered at the same time, by the same or different route of administration, or at different times. The important thing is not the time of administration but having an effective blood level of the P-gp inhibitor.

Suitable P-gp inhibitors include cyclosporin A, verapamil, tamoxifen, quinidine, Vitamin E-TGPS, ritonavir, megestrol acetate, progesterone, rapamycin, 10,11-methanodibenzosuberane, phenothiazines, acridine derivatives such as GF120918, FK506, VX-710, LY335979, PSC-833, GF-102,918 and other steroids. It is to be understood that additional agents will be found that do the same function and are also considered to be useful.

The P-gp inhibitors can be administered orally, parenterally, (IV, IM, IM-depo, SQ, SQ-depo), topically, sublingually, rectally, intranasally, intrathecally and by implant.

The therapeutically effective amount of the P-gp inhibitors is from about 0.1 to about 300 mg/kg/day, preferably about 0.1 to about 150 mg/kg daily. It is understood that while a subject may be started on one dose, that dose may have to be varied over time as the subject's condition changes.

When administered orally, the P-gp inhibitors can be administered in usual dosage forms for oral administration as is known to those skilled in the art. These dosage forms include the usual solid unit dosage forms of tablets and capsules as well as liquid dosage forms such as solutions, suspensions and elixirs. When the solid dosage forms are used, it is preferred that they be of the sustained release type so that the P-gp inhibitors need to be administered only once or twice daily. The oral dosage forms are administered to the subject one through four times daily. It is preferred that the P-gp inhibitors be administered either three or fewer times a day, more preferably once or twice daily. Hence, it is preferred that the P-gp inhibitors be administered in solid dosage form and further it is preferred that the solid dosage form be a sustained release form which permits once or twice daily dosing. It is preferred that what ever dosage form is used, that it be designed so as to protect the P-gp inhibitors from the acidic environment of the stomach. Enteric coated tablets are well known to those skilled in the art. In addition, capsules filled with small spheres each coated to protect from the acidic stomach, are also well known to those skilled in the art.

In addition, the P-gp inhibitors can be administered parenterally. When administered parenterally they can be administered IV, IM, depo-IM, SQ or depo-SQ. The P-gp inhibitors can be given sublingually. When given sublingually, the P-gp inhibitors should be given one thru four times daily in the same amount as for IM administration.

The P-gp inhibitors can be given intranasally. When given by this route of administration, the appropriate dosage forms are a nasal spray or dry powder as is known to those skilled in the art. The dosage of the P-gp inhibitors for intranasal administration is the same as for IM administration.

The P-gp inhibitors can be given intrathecally. When given by this route of administration the appropriate dosage form can be a parenteral dosage form as is known to those skilled in the art.

The P-gp inhibitors can be given topically. When given by this route of administration, the appropriate dosage form is a cream, ointment or patch. Because of the amount of the P-gp inhibitors needed to be administered the path is preferred. However, the amount that can be delivered by a patch is limited. Therefore, two or more patches may be required. The number and size of the patch is not important, what is important is that a therapeutically effective amount of the P-gp inhibitors be delivered as is known to those skilled in the art. The P-gp inhibitors can be administered rectally by suppository as is known to those skilled in the art.

The P-gp inhibitors can be administered by implants as is known to those skilled in the art.

There is nothing novel about the route of administration nor the dosage forms for administering the P-gp inhibitors. Given a particular P-gp inhibitor, and a desired dosage form, one skilled in the art would know how to prepare the appropriate dosage form for the P-gp inhibitor.

The compounds employed in the methods of the invention can be used in combination, with each other or with other therapeutic agents or approaches used to treat or prevent the conditions listed above. Such agents or approaches include: acetylcholine esterase inhibitors such as tacrine (tetrahydroaminoacridine, marketed as COGNEX®), donepezil hydrochloride, (marketed as Aricept® and rivastigmine (marketed as Exelon®)gamma-secretase inhibitors; anti-inflammatory agents such as cyclooxygenase II inhibitors; anti-oxidants such as Vitamin E and ginkolides; immunological approaches, such as, for example, immunization with A beta peptide or administration of anti-A beta peptide antibodies; statins; and direct or indirect neurotropic agents such as Cerebrolysin®, AIT-082 (Emilieu, 2000, *Arch*. *Neurol.* 57:454), and other neurotropic agents of the future.

It should be apparent to one skilled in the art that the exact dosage and frequency of administration will depend on the particular compounds employed in the methods of the invention administered, the particular condition being treated, the severity of the condition being treated, the age, weight, general physical condition of the particular subject, and other medication the individual may be taking as is well known to administering physicians who are skilled in this art.

### Inhibition of APP Cleavage

The compounds of the invention inhibit cleavage of APP between Met595 and Asp596 numbered for the APP695 isoform, or a mutant thereof, or at a corresponding site of a different isoform, such as APP751 or APP770, or a mutant thereof (sometimes referred to as the "beta secretase site"). While not wishing to be bound by a particular theory, inhibition of beta-secretase activity is thought to inhibit production of beta amyloid peptide (A beta). Inhibitory activity is demonstrated in one of a variety of inhibition assays, whereby cleavage of an APP substrate in the presence of a beta-secretase enzyme is analyzed in the presence of the inhibitory compound, under conditions normally sufficient to result in cleavage at the beta-secretase cleavage site. Reduction of APP cleavage at the beta-secretase cleavage site compared with an untreated or inactive control is correlated with inhibitory activity. Assay systems that can be used to demonstrate efficacy of the compound inhibitors of the invention are known. Representative assay systems are described, for example, in U.S. Patents No. 5,942,400, 5,744,346, as well as in the Examples below.

The enzymatic activity of beta-secretase and the production of A beta can be analyzed *in vitro* or *in vivo*, using natural, mutated, and/or synthetic APP substrates, natural, mutated, and/or synthetic enzyme, and the test compound. The analysis may involve primary or secondary cells expressing native, mutant, and/or synthetic APP and enzyme, animal models expressing native APP and enzyme, or may utilize transgenic animal models expressing the substrate and enzyme. Detection of enzymatic activity can be by analysis of one or more of the cleavage products, for example, by immunoassay, fluorometric or chromogenic assay, HPLC, or other means of detection. Inhibitory compounds are determined as those having the ability to decrease the amount of beta-secretase cleavage product produced in comparison to a control, where beta-secretase mediated cleavage in the reaction system is observed and measured in the absence of inhibitory compounds.

### Beta-Secretase

Various forms of beta-secretase enzyme are known, and are available and useful for assay of enzyme activity and inhibition of enzyme activity. These include native, recombinant, and synthetic forms of the enzyme. Human beta-secretase is known as Beta Site APP Cleaving Enzyme (BACE), Asp2, and memapsin 2, and has been characterized, for example, in U.S. Patent No. 5,744,346 and published PCT patent applications WO98/22597, WO00/03819, WO01/23533, and WO00/17369, as well as in literature publications (Hussain et al., 1999, *Mol. Cell. Neurosci.* 14:419-427; Vassar et al., 1999, Science 286:735-741; Yan et al., 1999,_{.} *Nature* 402:533-537; Sinha et al., 1999, Nature 40:537-540; and Lin et al., 2000, *PNAS USA* 97:1456-1460). Synthetic forms of the enzyme have also been described (WO98/22597 and WO00/17369). Beta-secretase can be extracted and purified from human brain tissue and can be produced in cells, for example mammalian cells expressing recombinant enzyme.

Preferred methods employ compounds that are effective to inhibit 50% of beta-secretase enzymatic activity at a concentration of less than about 50 micromolar, preferably at a concentration of less than about 10 micromolar, more preferably less than about 1 micromolar, and most preferably less than about 10 nanomolar.

### APP Substrate

Assays that demonstrate inhibition of beta-secretase-mediated cleavage of APP can utilize any of the known forms of APP, including the 695 amino acid "normal" isotype described by Kang et al., 1987, *Nature* 325:733-6, the 770 amino acid isotype described by Kitaguchi et. al., 1981, Nature 331:530-532, and variants such as the Swedish Mutation (KM670-1NL) (APP-SW), the London Mutation (V7176F), and others. See, for example, U.S. Patent No. 5,766,846 and also Hardy, 1992, *Nature Genet*. 1:233-234, for a review of known variant mutations. Additional useful substrates include the dibasic amino acid modification, APP-KK disclosed, for example, in WO 00/17369, fragments of APP, and synthetic peptides containing the beta-secretase cleavage site, wild type (WT) or mutated form, e.g., SW, as described, for example, in U.S. Patent No 5,942,400 and WO00/03819.

The APP substrate contains the beta-secretase cleavage site of APP (KM-DA or NL-DA) for example, a complete APP peptide or variant, an APP fragment, a recombinant or synthetic APP, or a fusion peptide. Preferably, the fusion peptide includes the beta-secretase cleavage site fused to a peptide having a moiety useful for enzymatic assay, for example, having isolation and/or detection properties. A useful moiety may be an antigenic epitope for antibody binding, a label or other detection moiety, a binding substrate, and the like.

### Antibodies

Products characteristic of APP cleavage can be measured by immunoassay using various antibodies, as described, for example, in Pirttila et al., 1999, *Neuro Lett*. 249:21-4, and in U.S. Patent No. 5,612,486. Useful antibodies to detect A beta include, for example, the monoclonal antibody 6E10 (Senetek, St. Louis, MO) that specifically recognizes an epitope on amino acids 1-16 of the A beta peptide; antibodies 162 and 164 (New York State Institute for Basic Research, Staten Island, NY) that are specific for human A beta 1-40 and 1-42, respectively; and antibodies that recognize the junction region of beta-amyloid peptide, the site between residues 16 and 17, as described in U.S. Patent No. 5,593,846. Antibodies raised against a synthetic peptide of residues 591 to 596 of APP and SW192 antibody raised against 590-596 of the Swedish mutation are also useful in immunoassay of APP and its cleavage products, as described in U.S. Patent Nos. 5,604,102 and 5, 721, 130.

### Assay Systems

Assays for determining APP cleavage at the beta-secretase cleavage site are well known in the art. Exemplary assays, are described, for example, in U.S. Patent Nos. 5,744,346 and 5,942,400, and described in the Examples below.

### Cell Free Assays

Exemplary assays that can be used to demonstrate the inhibitory activity of the compounds of the invention are described, for example, in WO00/17369, WO 00/03819, and U.S. Patents No. 5,942,400 and 5,744,346. Such assays can be performed in cell-free incubations or in cellular incubations using cells expressing a beta-secretase and an APP substrate having a beta-secretase cleavage site.

An APP substrate containing the beta-secretase cleavage site of APP, for example, a complete APP or variant, an APP fragment, or a recombinant or synthetic APP substrate containing the amino acid sequence: KM-DA or NL-DA, is incubated in the presence of beta-secretase enzyme, a fragment thereof, or a synthetic or recombinant polypeptide variant having beta-secretase activity and effective to cleave the beta-secretase cleavage site of APP, under incubation conditions suitable for the cleavage activity of the enzyme. Suitable substrates optionally include derivatives that may be fusion proteins or peptides that contain the substrate peptide and a modification useful to facilitate the purification or detection of the peptide or its beta-secretase cleavage products. Useful modifications include the insertion of a known antigenic epitope for antibody binding; the linking of a label or detectable moiety, the linking of a binding substrate, and the like.

Suitable incubation conditions for a cell-free in vitro assay include, for example: approximately 200 nanomolar to 10 micromolar substrate, approximately 10 to 200 picomolar enzyme, and approximately 0.1 nanomolar to 10 micromolar inhibitor compound, in aqueous solution, at an approximate pH of 4 -7, at approximately 37 degrees C, for a time period of approximately 10 minutes to 3 hours. These incubation conditions are exemplary only, and can be varied as required for the particular assay components and/or desired measurement system. Optimization of the incubation conditions for the particular assay components should account for the specific beta-secretase enzyme used and its pH optimum, any additional enzymes and/or markers that might be used in the assay, and the like. Such optimization is routine and will not require undue experimentation.

One useful assay utilizes a fusion peptide having maltose binding protein (MBP) fused to the C-terminal 125 amino acids of APP-SW. The MBP portion is captured on an assay substrate by anti-MBP capture antibody. Incubation of the captured fusion protein in the presence of beta-secretase results in cleavage of the substrate at the beta-secretase cleavage site. Analysis of the cleavage activity can be, for example, by immunoassay of cleavage products. One such immunoassay detects a unique epitope exposed at the carboxy terminus of the cleaved fusion protein, for example, using the antibody SW192. This assay is described, for example, in U.S. Patent No 5,942,400.

### Cellular Assay

Numerous cell-based assays can be used to analyze beta-secretase activity and/or processing of APP to release A beta. Contact of an APP substrate with a beta-secretase enzyme within the cell and in the presence or absence of a compound inhibitor of the invention can be used to demonstrate beta-secretase inhibitory activity of the compound. Preferably, assay in the presence of a useful inhibitory compound provides at least about 30%, most preferably at least about 50% inhibition of the enzymatic activity, as compared with a non-inhibited control.

In one embodiment, cells that naturally express beta-secretase are used. Alternatively, cells are modified to express a recombinant beta-secretase or synthetic variant enzyme as discussed above. The APP substrate may be added to the culture medium and is preferably expressed in the cells. Cells that naturally express APP, variant or mutant forms of APP, or cells transformed to express an isoform of APP, mutant or variant APP, recombinant or synthetic APP, APP fragment, or synthetic APP peptide or fusion protein containing the beta-secretase APP cleavage site can be used provided that the expressed APP is permitted to contact the enzyme and enzymatic cleavage activity can be analyzed.

Human cell lines that normally process A beta from APP provide a useful means to assay inhibitory activities of the compounds of the invention. Production and release of A beta and/or other cleavage products into the culture medium can be measured, for example by immunoassay, such as Western blot or enzyme-linked immunoassay (EIA) such as by ELISA.

Cells expressing an APP substrate and an active beta-secretase can be incubated in the presence of a compound inhibitor to demonstrate inhibition of enzymatic activity as compared with a control. Activity of beta-secretase can be measured by analysis of one or more cleavage products of the APP substrate. For example, inhibition of beta-secretase activity against the substrate APP would be expected to decrease release of specific beta-secretase induced APP cleavage products such as A beta.

Although both neural and non-neural cells process and release A beta, levels of endogenous beta-secretase activity are low and often difficult to detect by EIA. The use of cell types known to have enhanced beta-secretase activity, enhanced processing of APP to A beta, and/or enhanced production of A beta are therefore preferred. For example, transfection of cells with the Swedish Mutant form of APP (APP-SW); with APP-KK; or with APP-SW-KK provides cells having enhanced beta-secretase activity and producing amounts of A beta that can be readily measured.

In such assays, for example, the cells expressing APP and beta-secretase are incubated in a culture medium under conditions suitable for beta-secretase enzymatic activity at its cleavage site on the APP substrate. On exposure of the cells to the compound inhibitor, the amount of A beta released into the medium and/or the amount of CTF99 fragments of APP in the cell lysates is reduced as compared with the control. The cleavage products of APP can be analyzed, for example, by immune reactions with specific antibodies, as discussed above.

Preferred cells for analysis of beta-secretase activity include primary human neuronal cells, primary transgenic animal neuronal cells where the transgene is APP, and other cells such as those of a stable 293 cell line expressing APP, for example, APP-SW.

### In vivo Assays: Animal Models

Various animal models can be used to analyze beta-secretase activity and /or processing of APP to release A beta, as described above. For example, transgenic animals expressing APP substrate and beta-secretase enzyme can be used to demonstrate inhibitory activity of the compounds of the invention. Certain transgenic animal models have been described, for example, in U.S. Patent Nos.: 5,877,399; 5,612,486; 5,387,742; 5,720,936; 5,850,003; 5,877,015,, and 5,811,633, and in Ganes et al., 1995, Nature 373:523. Preferred are animals that exhibit characteristics associated with the pathophysiology of AD. Administration of the compound inhibitors of the invention to the transgenic mice described herein provides an alternative method for demonstrating the inhibitory activity of the compounds. Administration of the compounds in a pharmaceutically effective carrier and via an administrative route that reaches the target tissue in an appropriate therapeutic amount is also preferred.

Inhibition of beta-secretase mediated cleavage of APP at the beta-secretase cleavage site and of A beta release can be analyzed in these animals by measure of cleavage fragments in the animal's body fluids such as cerebral fluid or tissues. Analysis of brain tissues for A beta deposits or plaques is preferred.

On contacting an APP substrate with a beta-secretase enzyme in the presence of an inhibitory compound of the invention and under conditions sufficient to permit enzymatic mediated cleavage of APP and/or release of A beta from the substrate, the compounds of the invention are effective to reduce beta-secretase-mediated cleavage of APP at the beta-secretase cleavage site and/or effective to reduce released amounts of A beta. Where such contacting is the administration of the inhibitory compounds of the invention to an animal model, for example, as described above, the compounds are effective to reduce A beta deposition in brain tissues of the animal, and to reduce the number and/or size of beta amyloid plaques. Where such administration is to a human subject, the compounds are effective to inhibit or slow the progression of disease characterized by enhanced amounts of A beta, to slow the progression of AD in the, and/or to prevent onset or development of AD in a subject at risk for the disease.

Unless defined otherwise, all scientific and technical terms used herein have the same meaning as commonly understood by one of skill in the art to which this invention belongs. All patents and publications referred to herein are hereby incorporated by reference for all purposes.

APP, amyloid precursor protein, is defined as any APP polypeptide, including APP variants, mutations, and isoforms, for example, as disclosed in U.S. Patent No. 5,766,846.

A beta, amyloid beta peptide, is defined as any peptide resulting from beta-secretase mediated cleavage of APP, including peptides of 39, 40, 41, 42, and 43 amino acids, and extending from the beta-secretase cleavage site to amino acids 39, 40, 41, 42, or 43.

Beta-secretase (BACE1, Asp2, Memapsin 2) is an aspartyl protease that mediates cleavage of APP at the amino-terminal edge of A beta. Human beta-secretase is described, for example, in WOOO/17369.

Pharmaceutically acceptable refers to those properties and/or substances that are acceptable to the subject from a pharmacological/toxicological point of view and to the manufacturing pharmaceutical chemist from a physical/chemical point of view regarding composition, formulation, stability, subject's acceptance and bioavailability.

A therapeutically effective amount is defined as an amount effective to reduce or lessen at least one symptom of the disease being treated or to reduce or delay onset of one or more clinical markers or symptoms of the disease.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

As noted above, depending on whether asymmetric carbon atoms are present, the compounds of the invention can be present as mixtures of isomers, especially as racematco, or in the form of pure icomers, especially optical antipodes.

Salts of compounds having salt-forming groups are especially acid addition salts, salts with bases or, where several salt-forming groups are present, can also be mixed salts or internal salts.

Salts are especially the pharmaceutically acceptable or non-toxic salts of compounds of formula I.

Such salts are formed, for example, by compounds of formula I having an acid group, for example a carboxy group or a sulfo group, and are, for example, salts thereof with suitable bases, such as non-toxic metal salts derived from metals of groups Ia, Ib, IIa and IIb of the Periodic Table of the Elements, for example alkali metal salts, especially lithium, sodium or potassium salts, or alkaline earth metal salts, for example magnesium or calcium salts, also zinc salts or ammonium salts, as well as salts formed with organic amines, such as unsubstituted or hydroxy-substituted mono-, di- or tri-alkylamines, especially mono-, di- or tri-lower alkylamines, or with quaternary ammonium bases, for example with methyl-, ethyl-, diethyl- or triethyl-amine, mono-, bis- or tris-(2-hydroxy-lower alkyl)-amines, such as ethanol-, diethanol- or triethanol-amine, tris(hydroxymethyl)methylamine or 2-hydroxy-tertbutylamine, N,N-di-lower alkyl-N-(hydroxy-lower alkyl)-amines, such as N,N-dimethyl-N-(2-hydroxyethyl)-amine, or N-methyl-D-glucamine, or quaternary ammonium hydroxides, such as tetrabutylammonium hydroxide. The compounds of formula I having a basic group, for example an amino group, can form acid addition salts, for example with suitable inorganic acids, for example hydrohalic acids, such as hydrochloric acid or hydrobromic acid, or sulfuric acid with replacement of one or both protons, phosphoric acid with replacement of one or more protons e.g. orthophosphoric acid or metaphosphoric acid, or pyrophosphoric acid with replacement of one or more protons, or with organic carboxylic, sulfonic, sulfo or phosphonic acids or N-substituted sulfamic acids, for example acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid, as well as with amino acids, such as the .alpha.-amino acids mentioned hereinbefore, and with methanesulfonic acid, ethanesulfonic acid, 2-hydroxyethanesulfonic acid, ethane-1,2-disulfonic acid, benzenesulfonic acid, 4-methylbenzenenesulfonic acid, naphthalene-2-sulfonic acid, 2- or 3-phosphoglycerate, glucose-6-phosphate, or N-cyclohexylsulfamic acid (forming cyclamates) or with other acidic organic compounds, such as ascorbic acid. Compounds of formula I having acid and basic groups can also form internal salts.

For isolation and purification purposes it is also possible to use pharmaceutically unacceptable salts.

The present invention may be better understood with reference to the following examples. These examples are intended to be representative of specific embodiments of the invention, and are not intended as limiting the scope of the invention.

### BIOLOGY EXAMPLES

### Example A

### Enzyme Inhibition Assay

The compounds of the invention are analyzed for inhibitory activity by use of the MBP-C125 assay. This assay determines the relative inhibition of beta-secretase cleavage of a model APP substrate, MBP-C125SW, by the compounds assayed as compared with an untreated control. A detailed description of the assay parameters can be found, for example, in U.S. Patent No. 5,942,400. Briefly, the substrate is a fusion peptide formed of maltose binding protein (MBP) and the carboxy terminal 125 amino acids of APP-SW, the Swedish mutation. The beta-secretase enzyme is derived from human brain tissue as described in Sinha et al, 1999, Nature 40:537-540) or recombinantly produced as the full-length enzyme (amino acids 1-501), and can be prepared, for example, from 293 cells expressing the recombinant cDNA, as described in WO00/47618.

Inhibition of the enzyme is analyzed, for example, by immunoassay of the enzyme's cleavage products. One exemplary ELISA uses an anti-MBP capture antibody that is deposited on precoated and blocked 96-well high binding plates, followed by incubation with diluted enzyme reaction supernatant, incubation with a specific reporter antibody, for example, biotinylated anti-SW192 reporter antibody, and further incubation with streptavidin/alkaline phosphatase. In the assay, cleavage of the intact MBP-C125SW fusion protein results in the generation of a truncated amino-terminal fragment, exposing a new SW-192 antibody-positive epitope at the carboxy terminus. Detection is effected by a fluorescent substrate signal on cleavage by the phosphatase. ELISA only detects cleavage following Leu 596 at the substrate's APP-SW 751 mutation site.

### Specific Assay Procedure:

Compounds are diluted in a 1:1 dilution series to a six-point concentration curve (two wells per concentration) in one 96-plate row per compound tested. Each of the test compounds is prepared in DMSO to make up a 10 millimolar stock solution. The stock solution is serially diluted in DMSO to obtain a final compound concentration of 200 micromolar at the high point of a 6-point dilution curve. Ten (10) microliters of each dilution is added to each of two wells on row C of a corresponding V-bottom plate to which 190 microliters of 52 millimolar NaOAc, 7.9% DMSO, pH 4.5 are pre-added. The NaOAc diluted compound plate is spun down to pellet precipitant and 20 microliters/well is transferred to a corresponding flat-bottom plate to which 30 microliters of ice-cold enzyme-substrate mixture (2.5 microliters MBP-C125SW substrate, 0.03 microliters enzyme and 24.5 microliters ice cold 0.09% TX100 per 30 microliters) is added. The final reaction mixture of 200 micromolar compound at the highest curve point is in 5% DMSO, 20 millimolar NaOAc, 0.06% TX100, at pH 4.5.

Warming the plates to 37 degrees C starts the enzyme reaction. After 90 minutes at 37 degrees C, 200 microliters/well cold specimen diluent is added to stop the reaction and 20 microliters/well was transferred to a corresponding anti-MBP antibody coated ELISA plate for capture, containing 80 microliters/well specimen diluent. This reaction is incubated overnight at 4 degrees C and the ELISA is developed the next day after a 2 hour incubation with anti-192SW antibody, followed by Streptavidin-AP conjugate and fluorescent substrate. The signal is read on a fluorescent plate reader.

Relative compound inhibition potency is determined by calculating the concentration of compound that showed a fifty percent reduction in detected signal (IC₅₀) compared to the enzyme reaction signal in the control wells with no added compound.

### Example B

### Cell Free Inhibition Assay Utilizing a Synthetic APP Substrate

A synthetic APP substrate that can be cleaved by beta-secretase and having N-terminal biotin and made fluorescent by the covalent attachment of Oregon green at the Cys residue is used to assay beta-secretase activity in the presence or absence of the inhibitory compounds of the invention. Useful substrates include the following:

Biotin-SEVNLDAEFRC[Oregon green]KK [SEQ ID NO: 1]

Biotin-SEVKMDAEFRC[Oregon green]KK [SEQ ID NO: 2]

Biotin-GLNIKTEEISEISYEVEFRC[Oregon green]KK [SEQ ID NO: 3]

Biotin-ADRGLTTRPGSGLTNIKTEEISEVNLDAEFC[Oregon green]KK
[SEQ ID NO: 4]

Biotin-FVNQHLCₒₓGSHLVEALY-LVCₒₓGERGFFYTPKAC[Oregon green]KK
[SEQ ID NO: 5]

The enzyme (0.1 nanomolar) and test compounds (0.001 - 100 micromolar) are incubated in pre-blocked, low affinity, black plates (384 well) at 37 degrees for 30 minutes. The reaction is initiated by addition of 150 millimolar substrate to a final volume of 30 microliter per well. The final assay conditions are: 0.001 - 100 micromolar compound inhibitor; 0.1 molar sodium acetate (pH 4.5); 150 nanomolar substrate; 0.1 nanomolar soluble beta-secretase; 0.001% Tween 20, and 2% DMSO. The assay mixture is incubated for 3 hours at 37 degrees C, and the reaction is terminated by the addition of a saturating concentration of immunopure streptavidin. After incubation with streptavidin at room temperature for 15 minutes, fluorescence polarization is measured, for example, using a LJL Acqurest (Ex485 nm/ Em530 nm). The activity of the beta-secretase enzyme is detected by changes in the fluorescence polarization that occur when the substrate is cleaved by the enzyme. Incubation in the presence or absence of compound inhibitor demonstrates specific inhibition of beta-secretase enzymatic cleavage of its synthetic APP substrate.

### Example C

### Beta-Secretase Inhibition: P26-P4'SW Assay

Synthetic substrates containing the beta-secretase cleavage site of APP are used to assay beta-secretase activity, using the methods described, for example, in published PCT application WO00/47618. The P26-P4'SW substrate is a peptide of the sequence: (biotin)CGGADRGLTTRPGSGLTNIKTEEISEVNLDAEF [SEQ ID NO: 6] The P26-P1 standard has the sequence:

(biotin)CGGADRGLTTRPGSGLTNIKTEEISEVNL [SEQ ID NO: 7].

Briefly, the biotin-coupled synthetic substrates are incubated at a concentration of from about 0 to about 200 micromolar in this assay. When testing inhibitory compounds, a substrate concentration of about 1.0 micromolar is preferred. Test compounds diluted in DMSO are added to the reaction mixture, with a final DMSO concentration of 5%. Controls also contain a final DMSO concentration of 5%. The concentration of beta secretase enzyme in the reaction is varied, to give product concentrations with the linear range of the ELISA assay, about 125 to 2000 picomolar, after dilution.

The reaction mixture also includes 20 millimolar sodium acetate, pH 4.5, 0.06% Triton X100, and is incubated at 37 degrees C for about 1 to 3 hours. Samples are then diluted in assay buffer (for example, 145.4 nanomolar sodium chloride, 9.51 millimolar sodium phosphate, 7.7 millimolar sodium azide, 0.05% Triton X405, 6g/liter bovine serum albumin, pH 7.4) to quench the reaction, then diluted further for immunoassay of the cleavage products.

Cleavage products can be assayed by ELISA. Diluted samples and standards are incubated in assay plates coated with capture antibody, for example, SW192, for about 24 hours at 4 degrees C. After washing in TTBS buffer (150 millimolar sodium chloride, 25 millimolar Tris, 0.05% Tween 20, pH 7.5), the samples are incubated with streptavidin-AP according to the manufacturer's instructions. After a one hour incubation at room temperature, the samples are washed in TTBS and incubated with fluorescent substrate solution A (31.2 g/liter 2-amino-2-methyl-1-propanol, 30 mg/liter, pH 9.5). Reaction with streptavidin-alkaline phosphate permits detection by fluorescence. Compounds that are effective inhibitors of beta-secretase activity demonstrate reduced cleavage of the substrate as compared to a control.

### Example D

### Assays using Synthetic Oligopeptide-Substrates

Synthetic oligopeptides are prepared that incorporate the known cleavage site of beta-secretase, and optionally detectable tags, such as fluorescent or chromogenic moieties. Examples of such peptides, as well as their production and detection methods are described in U.S. Patent No: 5,942,400, herein incorporated by reference. Cleavage products can be detected using high performance liquid chromatography, or fluorescent or chromogenic detection methods appropriate to the peptide to be detected, according to methods well known in the art.

By way of example, one such peptide has the sequence (biotin)-SEVNLDAEF [SEQ ID NO: 8], and the cleavage site is between residues 5 an 6. Another preferred substrate has the sequence ADRGLTTRPGSGLTNIKTEEISEVNLDAEF [SEQ ID NO: 9], and the cleavage site is between residues 26 and 27.

These synthetic APP substrates are incubated in the presence of beta-secretase under conditions sufficient to result in beta-secretase mediated cleavage of the substrate. Comparison of the cleavage results in the presence of the compound inhibitor to control results provides a measure of the compound's inhibitory activity.

### Example E

### Inhibition of Beta-Secretase Activity - Cellular Assay

An exemplary assay for the analysis of inhibition of beta-secretase activity utilizes the human embryonic kidney cell line HEKp293 (ATCC Accession No. CRL-1573) transfected with APP751 containing the naturally occurring double mutation Lys651Met52 to Asn651Leu652 (numbered for APP751), commonly called the Swedish mutation and shown to overproduce A beta (Citron et al., 1992, *Nature* 360:672-674), as described in U.S. Patent No. 5,604,102.

The cells are incubated in the presence/absence of the inhibitory compound (diluted in DMSO) at the desired concentration, generally up to 10 micrograms/ml. At the end of the treatment period, conditioned media is analyzed for beta-secretase activity, for example, by analysis of cleavage fragments. A beta can be analyzed by immunoassay, using specific detection antibodies. The enzymatic activity is measured in the presence and absence of the compound inhibitors to demonstrate specific inhibition of beta-secretase mediated cleavage of APP substrate.

### Example F

### Inhibition of Beta-Secretase in Animal Models of AD

Various animal models can be used to screen for inhibition of beta-secretase activity. Examples of animal models useful in the invention include, but are not limited to, mouse, guinea pig, dog, and the like. The animals used can be wild type, transgenic, or knockout models. In addition, mammalian models can express mutations in APP, such as APP695-SW and the like described herein. Examples of transgenic non-human mammalian models are described in U.S. Patent Nos. 5,604,102, 5,912,410 and 5,811,633.

PDAPP mice, prepared as described in Games et al., 1995, Nature 373:523-527 are useful to analyze *in vivo* suppression of A beta release in the presence of putative inhibitory compounds. As described in U.S. Patent No. 6,191,166, 4 month old PDAPP mice are administered compound formulated in vehicle, such as corn oil. The mice are dosed with compound (1-30 mg/ml; preferably 1-10 mg/ml). After time, e.g., 3-10 hours, the animals are sacrificed, and brains removed for analysis.

Transgenic animals are administered an amount of the compound inhibitor formulated in a carrier suitable for the chosen mode of administration. Control animals are untreated, treated with vehicle, or treated with an inactive compound. Administration can be acute, i.e., single dose or multiple doses in one day, or can be chronic, i.e., dosing is repeated daily for a period of days. Beginning at time 0, brain tissue or cerebral fluid is obtained from selected animals and analyzed for the presence of APP cleavage peptides, including A beta, for example, by immunoassay using specific antibodies for A beta detection. At the end of the test period, animals are sacrificed and brain tissue or cerebral fluid is analyzed for the presence of A beta and/or beta-amyloid plaques. The tissue is also analyzed for necrosis.

Animals administered the compound inhibitors of the invention are expected to demonstrate reduced A beta in brain tissues or cerebral fluids and reduced beta amyloid plaques in brain tissue, as compared with non-treated controls.

### Example G

### Inhibition of A Beta Production in Human Subjects

Subjects suffering from Alzheimer's Disease (AD) demonstrate an increased amount of A beta in the brain. AD subjects and patients are administered an amount of the compound inhibitor formulated in a carrier suitable for the chosen mode of administration. Administration is repeated daily for the duration of the test period. Beginning on day 0, cognitive and memory tests are performed, for example, once per month.

Subjects administered the compound inhibitors are expected to demonstrate slowing or stabilization of disease progression as analyzed by changes in one or more of the following disease parameters: A beta present in CSF or plasma; brain or hippocampal volume; A beta deposits in the brain; amyloid plaque in the brain; and scores for cognitive and memory function, as compared with control, non-treated subjects.

### Example H

### Prevention of A Beta Production in Subjects at Risk for AD

Subjects predisposed or at risk for developing AD are identified either by recognition of a familial inheritance pattern, for example, presence of the Swedish Mutation, and/or by monitoring diagnostic parameters. Subjects identified as predisposed or at risk for developing AD are administered an amount of the compound inhibitor formulated in a carrier suitable for the chosen mode of administration. Administration is repeated daily for the duration of the test period. Beginning on day 0, cognitive and memory tests are performed, for example, once per month.

Subjects administered the compound inhibitors are expected to demonstrate slowing or stabilization of disease progression as analyzed by changes in one or more of the following disease parameters: A beta present in CSF or plasma; brain or hippocampal volume; amyloid plaque in the brain; and scores for cognitive and memory function, as compared with control, non-treated subjects.

### Preparation of the Compounds

The compounds of the present invention may be prepared as pharmaceutical compositions comprising any compound of the present invention and a pharmaceutically acceptable carrier.

The compounds employed in the methods of the present invention, may have asymmetric centers and occur as racemates, racemic mixtures and as individual diastereomers, or enantiomers with all isomeric forms being included in the present invention.

The compounds of the invention may be prepared according to any procedure known in the art, as well as the procedures set forth in U.S. Patent Nos. 5,420,127, 5,428,064, 5,416,111, 5,424,307, 5,424,319, 5,418,249, 5,416,104, 5,428,054, 5,416,092, 5,411,957, 5,416,085, 5,411,962, 5,411,958, 5,414,012, 5,416,095, 5,416,083, 4,977,141, 4,931,429, 5,089,471, 4,877,785, 4,900,746, 5,229,420, 4,902,706, 5,229,369, 4,900,745, 5,246,959, 5,175,170, 5,180,744, 5,175,191, 5,171,751, 5, 210, 095 5,283,250, 5,180,725, 5;179,102, 5,212,175, 5,147,888, 5,217,991, 5,223,512, 5,231,111, 5,223,534, 5,223,532, 5,223,514, 5,212,174, 5,215,996, 5,217,989, 5,290,787, 5,217,988, 5,216,013, 5,227,401, 5,484,811, 5,942,548, 6,174,923, 5,223,535, 5,484,812, 5,330,996, 5,508,295, 5,849,773, 5,314,920, 5,317,039, 5,246,969, 5,461,074, 5,268,391, 5,312,838, 5,416,119, 5,488,066, 5,756,549, 5,212,185, 5,304,552, 5,385,898, 5,780,494, 5,422,349, 5,409,922, 5,411,953, 5,411,959, 5,411,975, 5,409,913, 5,409,925, 5,409,921, 5,411,961, 5,413,998, 5,411,954, 5,416,084, 5,409,945, 5,411,950, 5,409,924, 5,373,017, 5,252,591, 5,434,162, 5,563,154, 5,298,505, 5,414,018, 5,488,067, 5,432,201, and 5,968,984. These documents are incorporated herein by reference, in their entirety.

The preparative methods found in the above documents serve merely as exemplary synthetic procedures, and do not limit the scope of the claimed invention. One of skill in the art will be able to modify the reactants and/or reaction conditions for specifically desired compounds. One of skill in the art will also recognize other possible synthetic routes for the compounds described by Formulas (I)-(X).

The invention has been described with reference to various specific and preferred embodiments and techniques. However, it should be understood that many variations and modifications may be made while remaining within the spirit and scope of the invention.

## Claims

1. A method of treating or preventing Alzheimer's disease in a subject in need of such treatment comprising administering a therapeutically effective amount of a composition comprising one or more non-toxic pharmaceutically acceptable carriers and a compound of Formulas (I), (II), (III), (IV), (V), (VI), (VII), (VIII), (IX), and (X), or a pharmaceutically acceptable salt thereof: 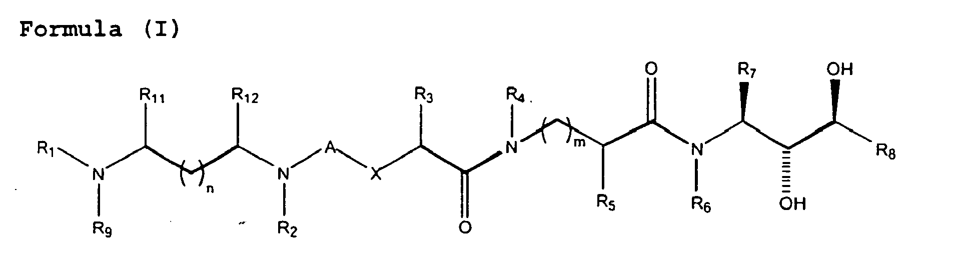 wherein A is selected from methylene, CO, SO, and SO₂;
wherein X is selected from oxygen atom, methylene and 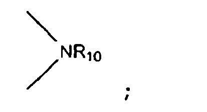 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy and cycloalkyl,
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl;
R₅ is selected from 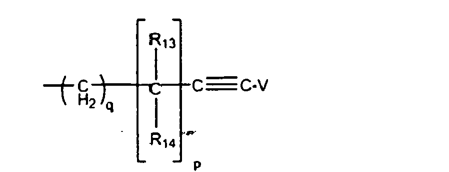 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1 ;
wherein n is an integer selected from 0 through 5;
wherein p is an integer selected from 0 through 5; and
wherein q is an integer selected from 0 through 5; or a pharmaceutically-acceptable salt thereof. 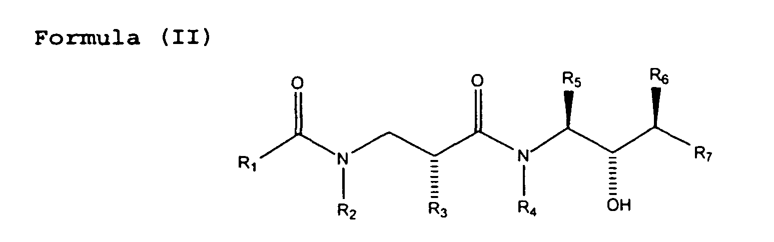 wherein R₁ is selected from aryl, aralkyl, heteroaryl and heteroaralkyl, including the following: 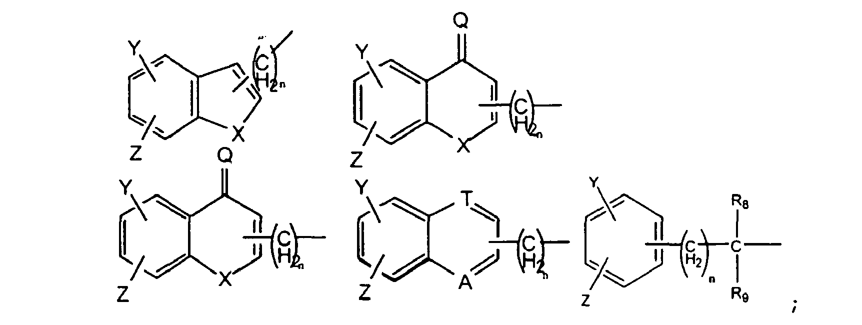 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 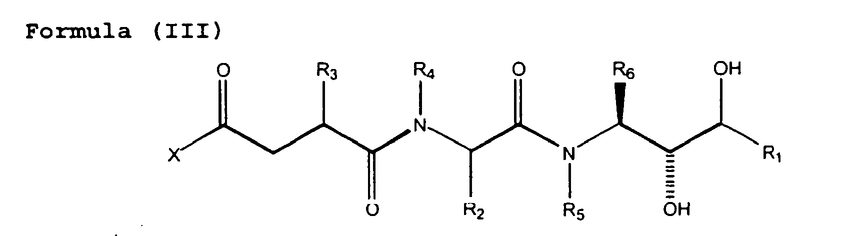 wherein X is selected from 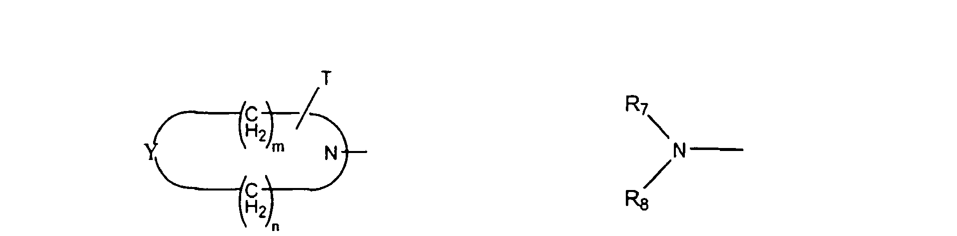 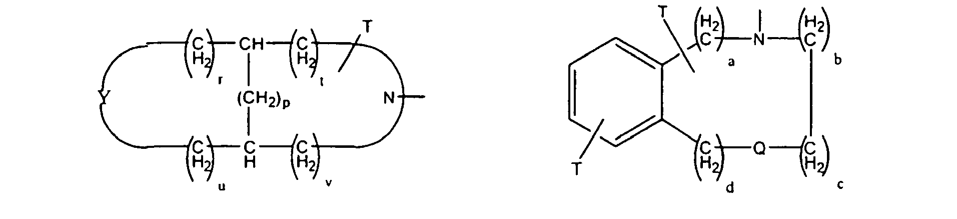 wherein Y and Q are selected from CH₂, CH-O-R₉, O, S, SO, SO₂, and NR₁₀, wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 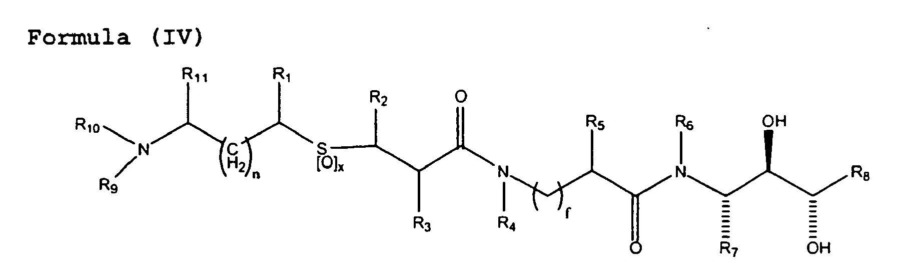 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl and haloaralkyl ;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 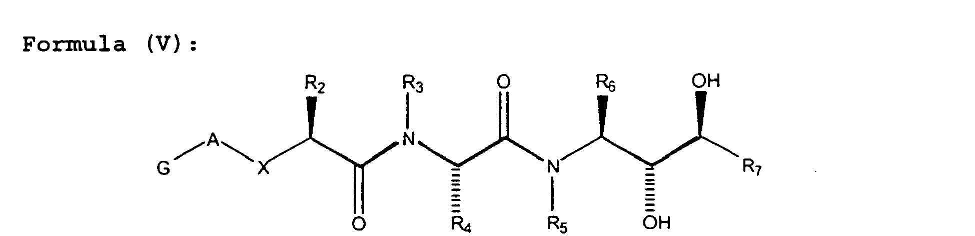 wherein A is selected from CO and SO₂
X is selected form oxygen atom and methylene;
G is selected from B or 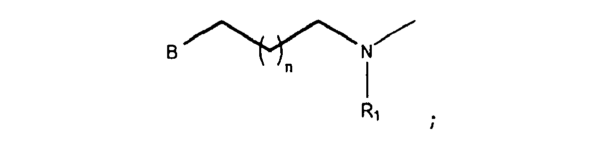 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 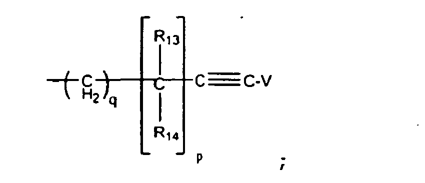 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 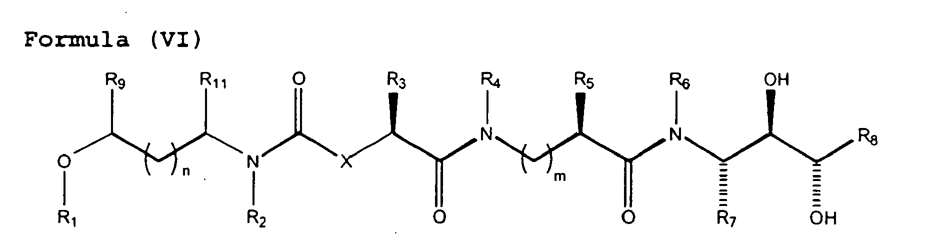 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 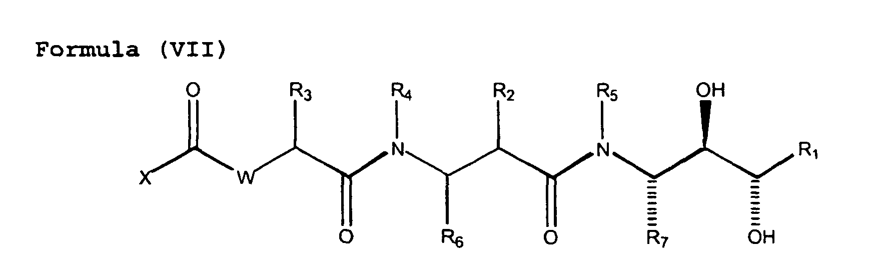 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 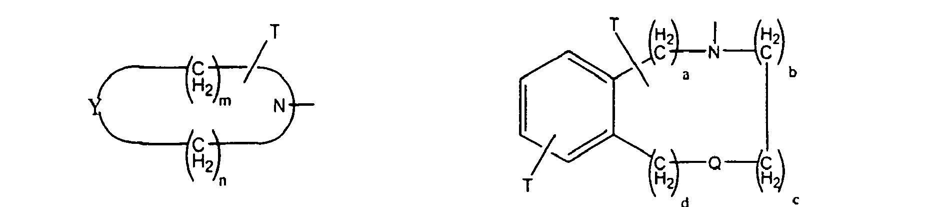 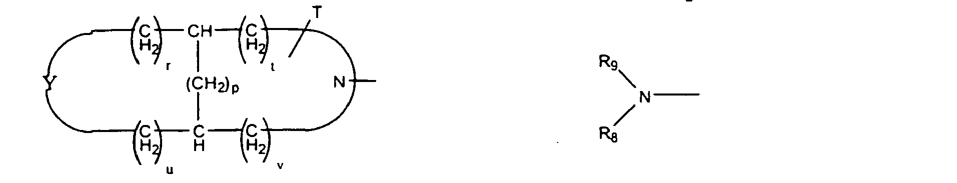 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 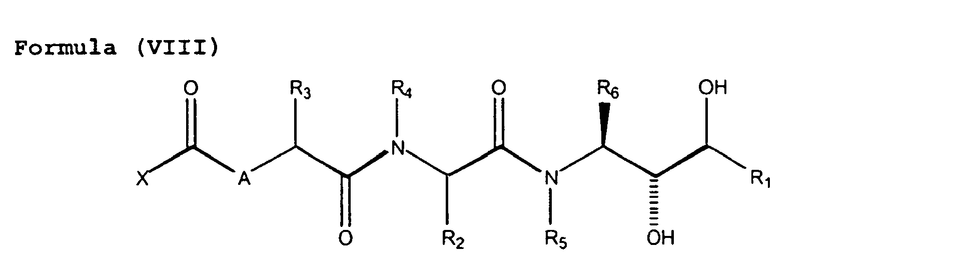 wherein X is selected from 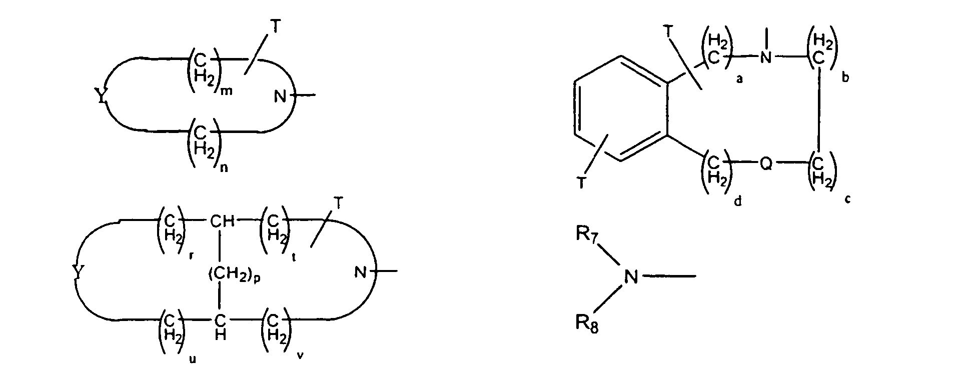 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 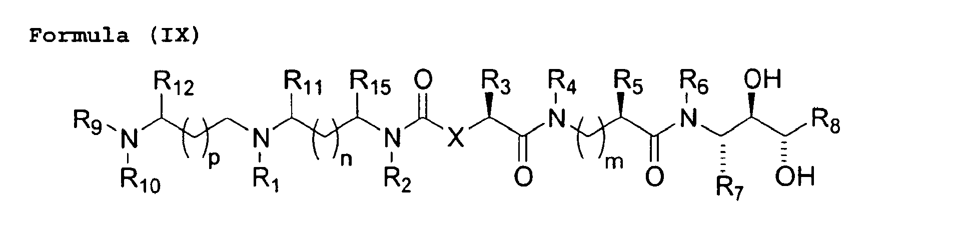 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
Ry is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH (OH) R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅, are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 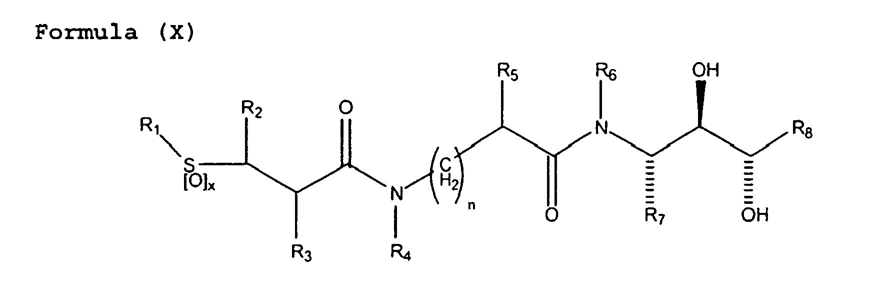 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl.

2. The method according to claim 1 comprising administering a compound, or pharmaceutically acceptable salt thereof, of formula (II).

3. The method according to claim 1 comprising administering a compound, or pharmaceutically acceptable salt thereof, of formula (III).

4. The method according to claim 1 comprising administering a compound, or pharmaceutically acceptable salt thereof, of formula (IV).

5. The method according to claim 1 comprising administering a compound, or pharmaceutically acceptable salt thereof, of formula (V).

6. The method according to claim 1 comprising administering a compound, or pharmaceutically acceptable salt thereof, of formula (VI).

7. The method according to claim 1 comprising administering a compound, or pharmaceutically acceptable salt thereof, of formula (VII).

8. The method according to claim 1 comprising administering a compound, or pharmaceutically acceptable salt thereof, of formula (VIII).

9. The method according to claim 1 comprising administering a compound, or pharmaceutically acceptable salt thereof, of formula (IX).

10. The method according to claim 1 comprising administering a compound, or pharmaceutically acceptable salt thereof, of formula (X).

11. A method of treating Alzheimer's disease in a subject in need of such treatment comprising administering to the subject a compound disclosed in claim 1, or a pharmaceutically acceptable salt thereof.

12. A method of treating Alzheimer's disease by modulating the activity of beta amyloid converting enzyme, comprising administering to a subject in need of such treatment a compound disclosed in claim 1, or a pharmaceutically acceptable salt thereof.

13. The method according to claim 1, further comprising the administration of a P-gp inhibitor, or a pharmaceutically acceptable salt thereof.

14. A method of treating a subject who has, or in preventing a subject from getting, a disease or condition selected from the group consisting of Alzheimer's disease, for helping prevent or delay the onset of Alzheimer's disease, for treating subjects with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease and who is in need of such treatment which includes administration of a therapeutically effective amount of a compound of formula (I)-(X), or a pharmaceutically acceptable salt thereof: 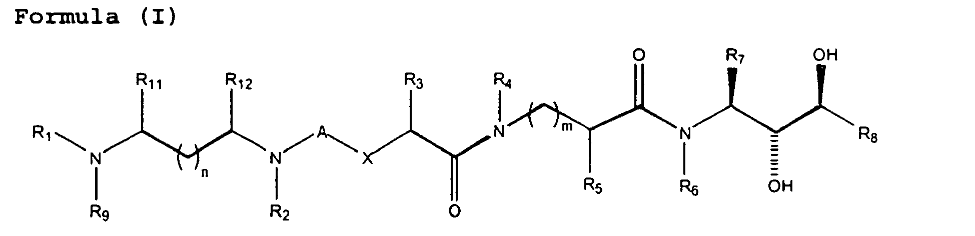 wherein A is selected from methylene, CO, SO, and SO₂;
wherein X is selected from oxygen atom, methylene and 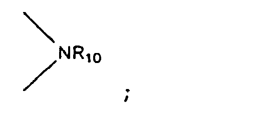 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl ;
R₄ and R₆ are each independently selected from hydrido, alkyl, benzyl and cycloalkyl;
R₅ is selected from 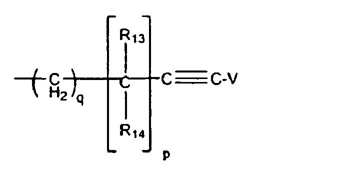 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydride, alkyl, haloalkyl; alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 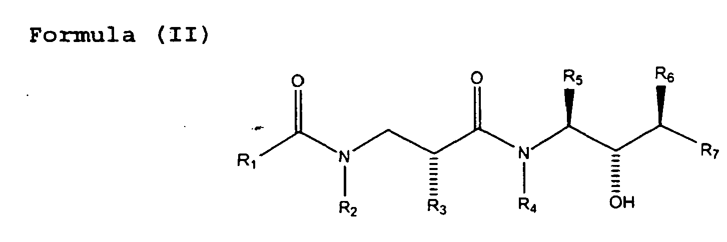 wherein R₁ is selected from aryl, aralkyl, heteroaryl and heteroaralkyl, including the following: 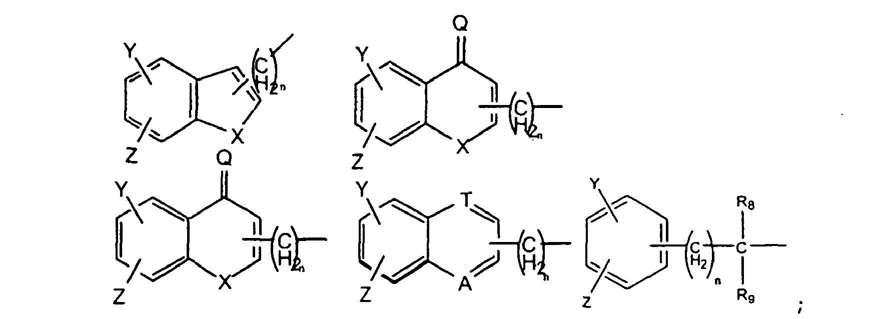 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 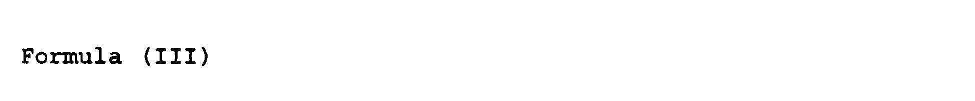 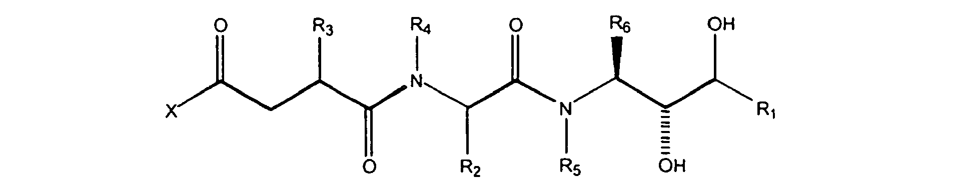 wherein X is selected from 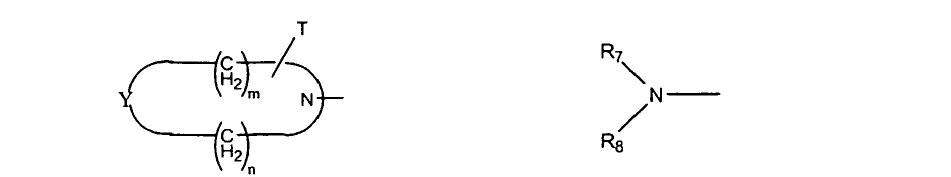 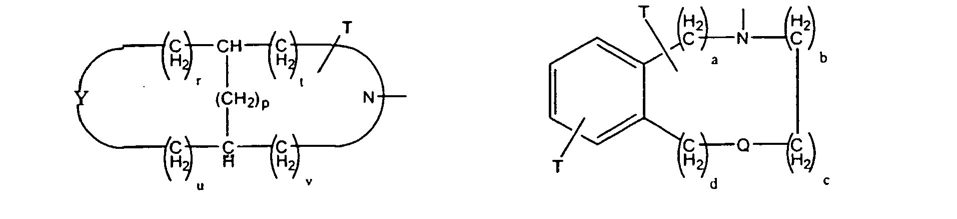 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁ , and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 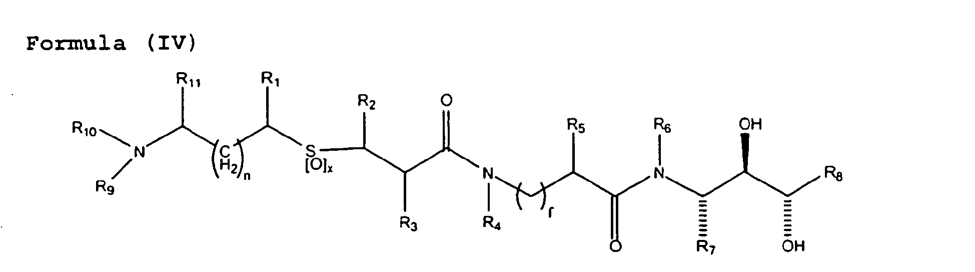 wherein R₁ and R₁₁ are each independently selected from hydrido, alkyl, alkylaminoalkyl, and phenyl;
n is an integer selected from u through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 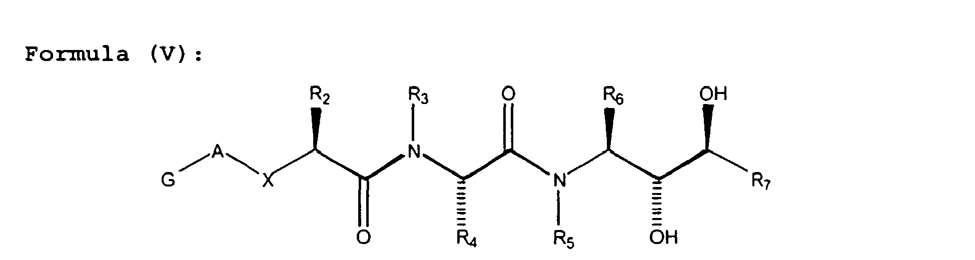 wherein A is selected from CO and SO₂;
X is selected form oxygen atom and methylene;
G is selected from B or 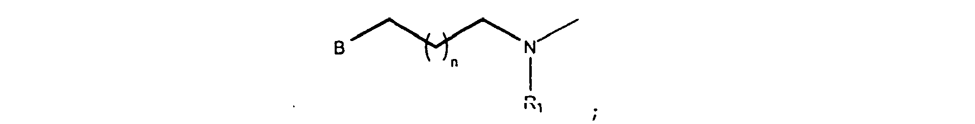 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocycLic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 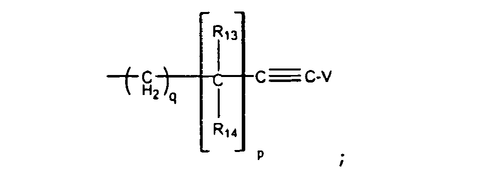 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 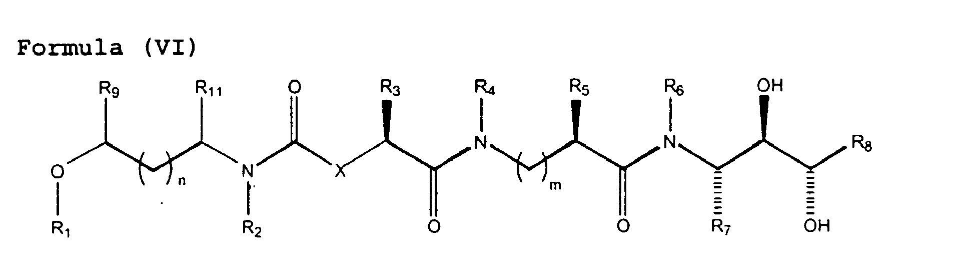 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 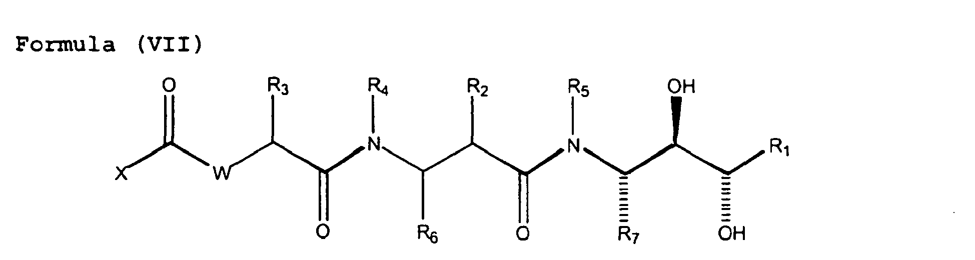 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 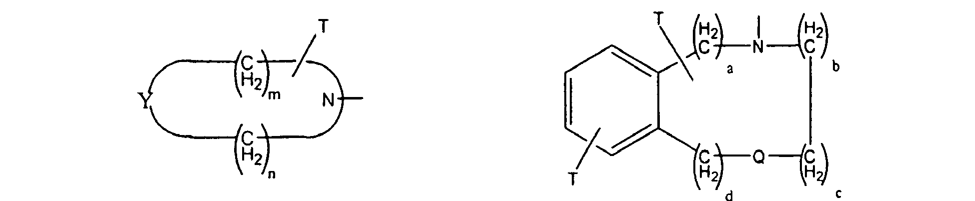 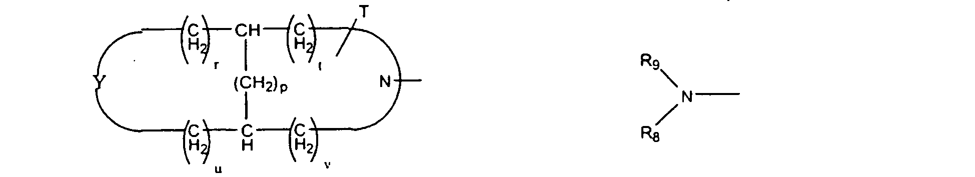 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 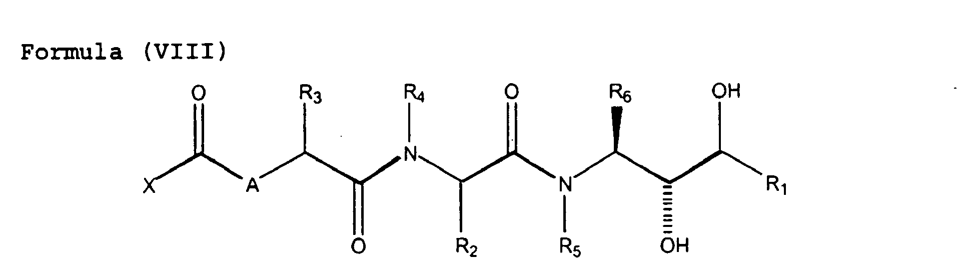 wherein X is selected from 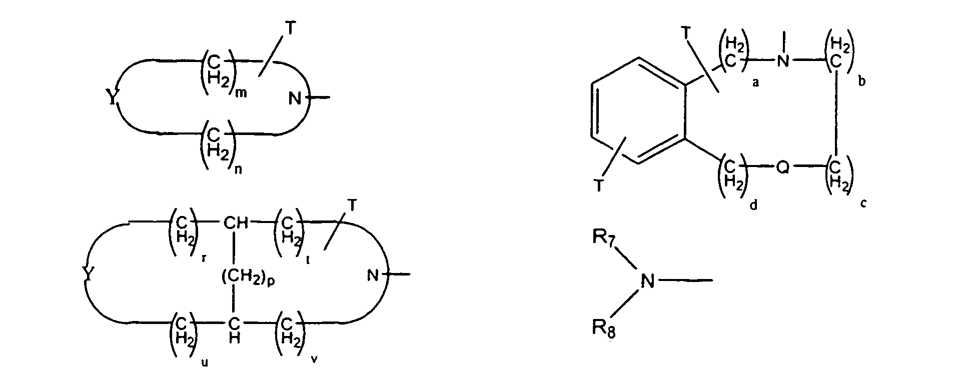 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 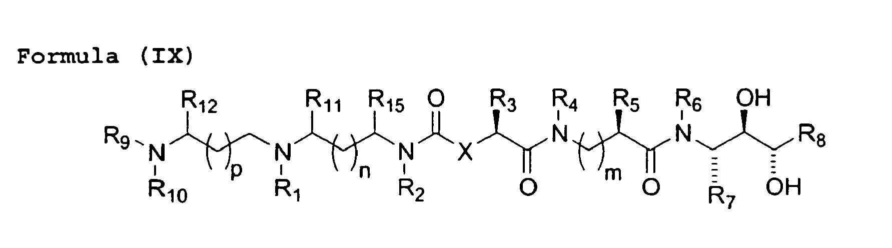 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH (OH) R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 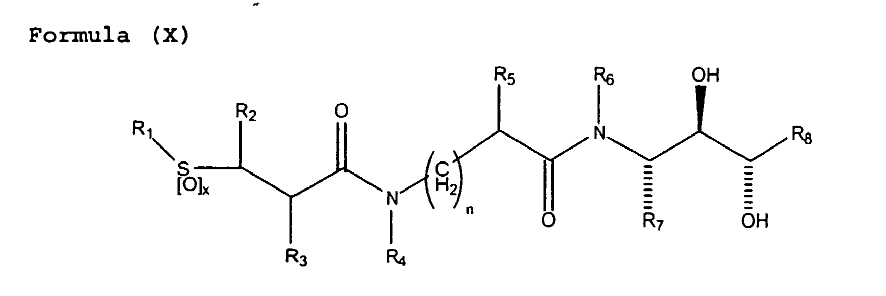 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl

15. The method according to any of claims 1, 11-14, wherein the compound is selected from the group consisting of:
O-{N- [2- (R,N-dimethylamino) ethyl] -N-methylaminocarbonyl}-3-L-phenyllactyl-L-histidine amide of (2S,3R,4S)-2-amino-1-cyclohexyl-3,4-dihydroxy-6-methylheptane;
O-{N-[2-(N-methylamino)ethyl]-N-methylaminocarbonyl}-3-L-phenyllactyl-L-histidine amide of (2S,3R,4S)-2-amino-1-cyclohexyl-3,4-dihydroxy-6-methylheptane;
O-{N-[2-(N-methylamino)ethyl]-N-methylaminocarbonyl}-3-L-phenyllactyl-L-leucine amide of (2S,3R,4S)-2-amino-l-cyclohexyl-3,4-dihydroxy-6-methylheptane;
O-{N-[2-(N,N-dimethylamino)ethyl]-N-methylaminocarbonyl}-3-L-phenyllactyl-L-leucine amide of (2S,3R,4S)-2-amino-1-cyclohexyl-3,4-dihydroxy-6-methylheptane;
O-{N-[2-(N,N-dimethylamino)ethyl]-N-methylaminocarbonyl}-3-L-phenyllactyl-a-(R)-methyl-P-alanine amide of (2S,3R,4S)-2-amino-1-cyclohexyl-3,4-dihydroxy-6-methylheptane;
0-{N-[2-(N,N-dimethylamino)ethyl]-N-methylaminocarbonyl}-3-L-homophenyllactyl-α-(R)-methyl-β-alanine amide of (2S,3R,4S)-2-amino-1-cyclohexyl-3,4-dihydroxy-6-methylheptane;
O-N-[2-(N,N-dimethylamino)ethyl]-N-methylaminocarbonyl}-3-L-homophenyllactyl-(x-(R)-ethyl-β-alanine amide of (2S,3R,4S)-2-amino-1-cyclohexyl-3,4-dihydroxy-6-methylheptane;
3-N- [2- (N,N-dimethylamino) ethyl] -N-methylaminocarbonyl}-2-(R)-(2-phenylethyl)-propionyl-α-(R)-ethyl-(β-alanine amide of (2S,3R,4S)-2-amino-l-cyclohexyl-3,4-dihydroxy-6-methylheptane;
3-{N-[2-(N-piperdino)ethyl]-N-methylaminocarbonyl}-3-L-phenyllactyl-L-histidine amide of (2S,3R,4S)-2-amino-l-cyclohexyl-3,4-dihydroxy-6-methylheptane;
3-N-[2-(N-piperdino)ethyl]-N-methylaminocarbonyl}-2-(R)-(2-phenylethyl)-propionyl-α-(R)-ethyl-β-alanine amide of (2S,3R,4S)-2-amino-1-cyclohexyl-3,4-dihydroxy-6-methylheptane;
O-{N-[2-N,N-dimethylamino)ethyl]-N-methylaminocarbonyl}-3-L-phenyllactyl-L-histindine amide of (2S,3R,4S)-2-amino-1-cyclohexyl-3,4-dihydroxy-6-methylheptane;
O-(N-(N-methyl-N-Boc-aminoethyl)-N-methylaminocarbonyl)-3-L-phenyllactyl-L-(im-tosyl)-histidine amide of (2S,3R,4S)-2-amino-1-cyclohexyl-3,4-dihydroxy-6-methylheptane;
N-Boc-α-(R)-methyl-β-alanine amide of (2S,3R,4S)-2-amino-1-cyclohexyl-3,4-dihydroxy-6-methylheptane;
N1-[1R*-[[[1S,1R*-(cyclohexylmethyl)-2S*,3R*-dihydroxyhexynyl]amino]carbonyl]-3-butynyl]-N4-[2-(dimethylamino)ethyl]-N4-methyl-2S*-(phenylmethyl)butanediamide;
[1R*-[[[1R*-[[[1S,1R*-(cyclohexylmethyl)-2S*,3R*-dihydroxyhexynyl]amino]carbonyl]-3-butynyl]amino]carbonyl]-2-phenylethyl)[2-(dimethylamino)ethyl]methylcarbamate;
N1-[1R*- [[[1S,1R*- (cyclohexylmethyl)-2S*,3R*-dihydroxy-5-methylhexyl]amino]carbonyl]-3-butynyl]-N4-[2-(dimethylamino)ethyl]-N4-[2-(dimethylamino)ethyl]-N4-methyl-2S*-(phenylmethyl)butanediamide;
[1R*- [[[1R*- [[[1S,1R*-(cyclohexylmethyl)-2S*,3R*-dihydroxy-5-methylhexyl]amino]carbonyl]-3-butynyl]amino]carbonyl]-2-phenylethyl)[2-(dimethylamino)ethyl]methylcarbamate;
[1R*-[[[1-[[[1S,1R*-(cyclohexylmethyl)-2S*,3R*-dihydroxy-5-methylhexyl]amino]carbonyl]-3-butynyl]amino]carbonyl]-2-phenylethyl)[2-(dimethylamino).ethyl]methylcarbamate;
[1R*- [[[1-[[[1S,1R*-(cyclohexylmethyl)-2S*,3R*-dihydroxy-5-methylhexyl]amino]carbonyl]-3-butynyl]amino]carbonyl]-2-phenylethyl][2 - (dimethylamino)ethyl]methylcarbamate;
{N-[2-(N,N-dimethylamino)ethyl]-N-methylaminocarbonyl}-3-L-phenyllactyl-α-(R)-methyl-β-alanine amide of (2S,3R,4S)-2-amino-1-cyclohexyl-3,4-dihydroxy-6-methylheptane;
3-{N-[4-(N-methyl-N-boc-amino)butyl-N-methylaminocarbonyl}-2-(R)-phenethylpropionyl-α-(R)-methyl-β-alanine amide of (2S,3R,4S)-2-amino-1-cyclohexyl-3,4-dihydroxy-6-methylheptane;
O-{N-[2-(N,N-dimethylamino)ethyl]-N-methylaminocarbonyl}-3-L-benzyllactyl-α-(R)-methyl-β-alanine amide of (2S,3R,4S)-2-amino-1-cyclohexyl-3,4-dihydroxy-6-methylheptane;
O-{N-[2-(N,N-dimethylamino)ethyl]-N-methylaminocarbonyl}-3-L-benzyllactyl-α-(R)-ethyl-β-alanine amide of (2S,3R,4S)-2-amino-1-cyclohexyl-3,4-dihydroxy-6-methylheptane;
O-{N-[2-(N-piperidino)ethyl]-N-methylaminocarbonyl}-3-L-phenyllactyl-L-histidine amide of (2S,3R,4S)-2-amino-1-cyclohexyl-3,4-dihydroxy-6-methylheptane;
O-{N-[2-(N-piperidino)ethyl]-N-methylaminocarbonyl}-2-(R)-(2-phenylethyl)-propionyl-α-(R)-ethyl-α-alanine amide of (2S, 3R, 4S)-2-amino-1-cyclohexyl-3,4-dihydroxy-6-methylheptane;
3-{N-[2-(N,N-dimethylamino)ethyl-N-methylaminocarbonyl}-2-R-benzyl-propionyl-α-(R)-methyl-β-alanine amide of (2S,3R,4S)-2-amino-1-cyclohexyl-3,4-dihydroxy-6-methylheptane;
O-{N-[2-(N,N-dimethylamino)ethyl]-N-isopropylaminocarbonyl}-3-L-phenyllactyl-L-leucine amide of (2S,3R,4S)-2-amino-1-cyclohexyl-3,4-dihydroxy-6-methylheptane;
3-{N-[4-(N-methylamino)butyl]-N-methylaminocarbonyl}-2-R-phenethyl-propionyl-α-(R)-methyl-β-alanine amide of (2S,3R,4S)-2-amino-1-cyclohexyl-3,4-dihydroxy-6-methylheptane;
O-{N-[2-N-methyl-N-boc-amino)ethyl)butyl]-N-methylaminocarbonyl}-3-L-phenyllactyl-L-histidine amide of (2S,3R,4S)-2-amino-1-cyclohexyl-3,4-dihydroxy-6-methylheptane;
O-{N-[2-(N-methyl-N-boc-amino)ethyl]-N-methylaminocarbonyl}-3-L-phenyllactyl-α-(R)-methyl-β-alanine amide of (2S,3R,4S)-2-amino-1-cyclohexyl-3,4-dihydroxy-6-methylheptane;
O-{N-[2-(N-methylamino)ethyl]-N-methylaminocarbonyl}-3-L-phenyllactyl-α-(R)-methyl-β-alanine amide of (2S,3R,4S)-2-amino-1-cyclohexyl-3,4-dihydroxy-6-methylheptane;
3{N-[2-(N-methylamino)ethyl]-N-methylaminocarbonyl}-2-R-phenethylpropionyl-α-(R)-methyl-β-alanine amide of (2S,3R,4S)-2-amino-1-cyclohexyl-3,4-dihydroxy-6-methylheptane trifluoroacetate salt;
Boc-(im-Tosyl)-L-histidine amide of (2S,3R,4S)-2-amino-1-cyclohexyl-3,4-dihydroxy-6-methylheptane;
(im-Tosyl)-L-histidine amide of (2S,3R,4S)-2-amino-1-cyclohexyl-3,4-dihydroxy-6-methyheptane;
O-{N-[2-(N,N-dimethylamino)ethyl]-N-methylaminocarbonyl}-3-L-phenyllactyl-L-histidine amide of (2S,3R,4S)-2-amino-1-cyclohexyl-3,4-dihydroxy-6-methylheptane;
O-(N-(N-methyl-N-Boc-aminoethyl)-N-methylaminocarbonyl)-3-L-phenyllactic acid;
O-(N-(dimethylaminoethyl)-N-methyl-aminocarbonyl)-3-L-phenyllactic acid;
O-{N-2-(N,N-dimethylamino)ethyl]-N-methylaminocarbonyl}-3-L-phenyllactyl-L-leucine amide of (2S,3R,4S)-2-amino-1-cyclohexyl-3,4-dihydroxy-6-methylheptane;
O-{N-[2-(N,N-dimethylamino)ethyl]-N-methylaminocarbonyl-3-L-phenyllactyl-α-(R)-methyl-β-alanine amide of (2S,3R,4S)-2-amino-1-cyclohexyl-3,4-dihydroxy-6-methylheptane;
O-{N-[2-(N-methyl-N-Boc-amino)ethyl]-N-methylaminocarbonyl-3-L-phenyllactyl-L-histidine amide of (2S,3R,4S)-2-amino-1-cyclohexyl-3,4-dihydroxy-6-methylheptane;
O-{N-[N-[2-(N-methyl-N-Boc-amino)ethyl]-N-methylaminocarbonyl}-3-L-phenyllactyl-α-(R)-methyl-β-alanine amide of (2S,3R,4S)-2-amino-1-cyclohexyl-3,4-dihydroxy-6-methylheptane;
O-{N-2-(N,N-dimethylamino)ethyl]-N-isopropylaminocarbonyl-3-L-phenyl lactic acid;
3-{N-2-(N,N-dimethylamino)ethyl]-N-methylaminocarbonyl}-2-R-benzylpropionic acid hydrochloride salt;
3-{N- [2-(N,N-dimethylamino)ethyl] -N-methylaminocarbonyl}-2-R-benzylpropionyl-histidine amide of (2S,3R,4S)-2-amino-1-cyclohexyl-3,4-dihydroxy-6-methylheptane;
O-{N-[2-(N,N-dimethylamino)-1-(R,S)-methylethyl]-N-methylaminocarbonyl}-3-L-homophenyllactyl-α-(R)-methyl-β-alanine amide of (2S,3R,4S)-2-amino-1-cyclohexyl-3,4-dihydroxy-6-methylheptane;
O-{N- [2- (N,N-dimethylamino) ethyl] -N-methylaminocarbonyl}-3-L-homophenyllactyl-α-(R)-ethyl-β-alanine amide of (2S,3R,4S)-2-amino-1-cyclohexyl-3,4-dihydroxy-6-methylheptane;
3-{N-[2-(N-methyl-N-2-(4-imidazole)ethylamino)ethyl]-N-methylaminocarbonyl}-2-R-benzylpropionyl-histidine amide of (2S,3R,4S)-2-amino-1-cyclohexyl-3,4-dihydroxy-6-methylheptane;
N1-[2-[[1S,1R*-(cyclohexylmethyl-2S*,3R*-dihydroxy-5-hexynyl]amino]-2-oxo-1R*-(4-thiazolylmethyl)ethyl]-N4-(2-(dimethylamino)ethyl]-N4-methyl-2S*-(phenylmethyl)butanediamide;
[[[2-[[1S,1R*-(cyclohexylmethyl)-2S*,3R*-dihydroxy-S-hexynyl]amino]-2-oxo-1R*-(4-thiazolylmethyl)ethyl]amino]-2-oxo-1R*-(phenylmethyl)ethyl](2-(dimethylamino)ethyl]methylcarbamate;
N1-[1R*-[[[1S,1R*-(cyclohexylmethyl)-2S*,3R*-dihydroxy-5-methylhexyl]amino]carbonyl]-3-butynyl]-N4-[2-(dimethyl-amino)ethyl]-N4-methyl-2S*-(phenylmethyl)butanediamide;
[1R*-[[[1R*-[[[1S,1R*-(cyclohexylmethyl)-2S*,3R*-dihydroxy-5-methylhexyl]amino]carbonyl]-3-butynyl] amino] carbonyl] -2-phenylethyl) [2-dimethylamino)ethyl]methylcarbamate; 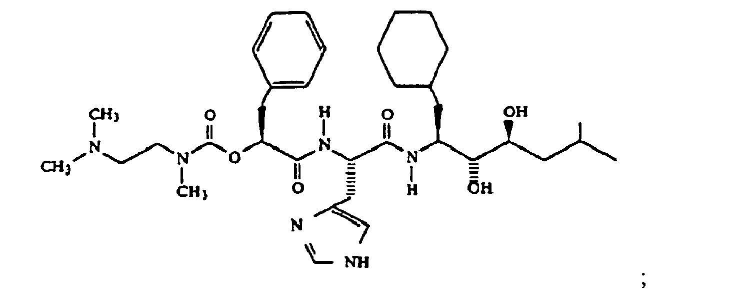 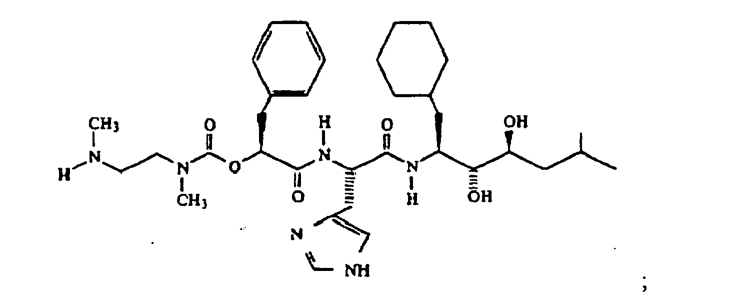 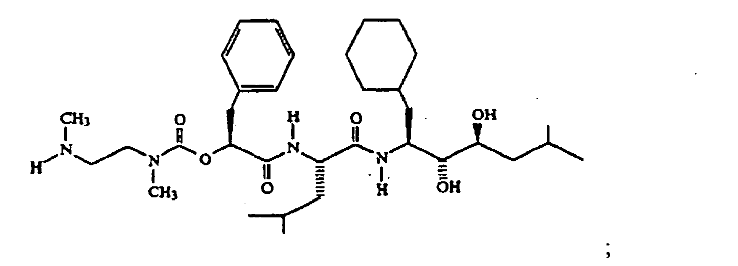 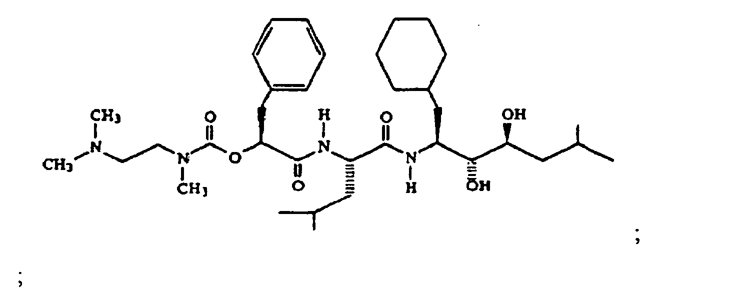 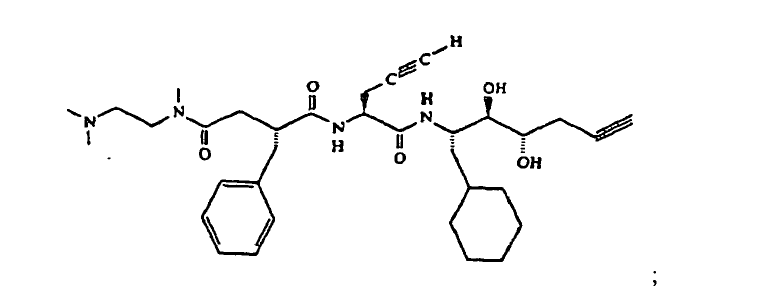 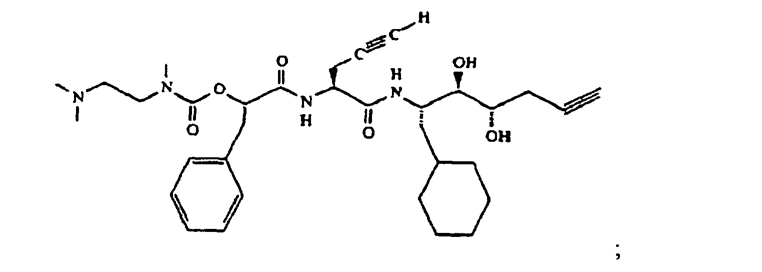 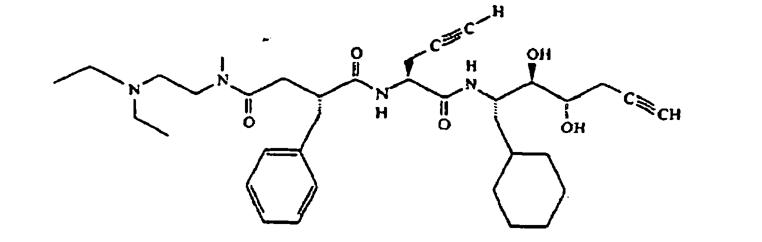 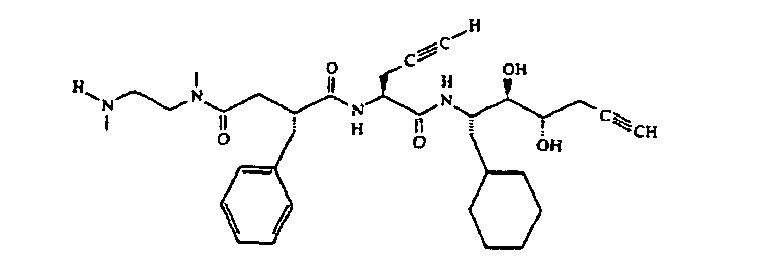 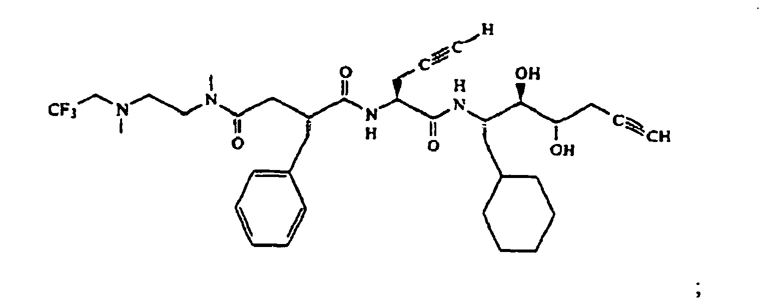 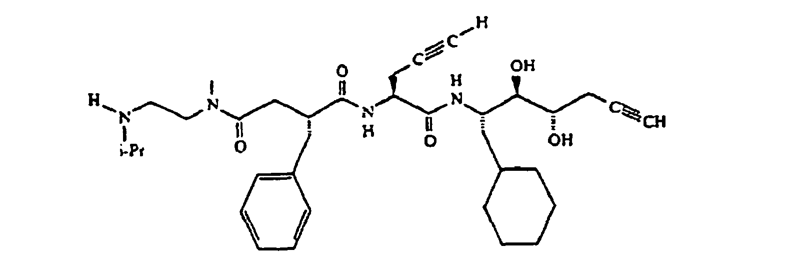 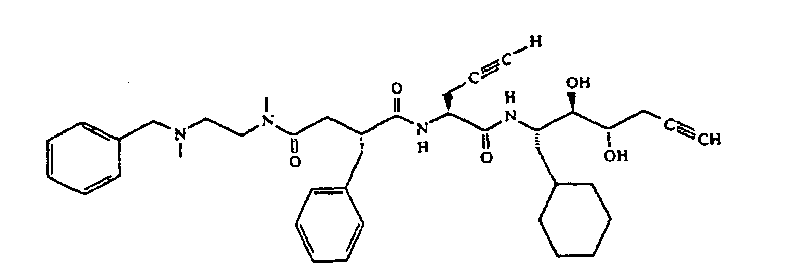 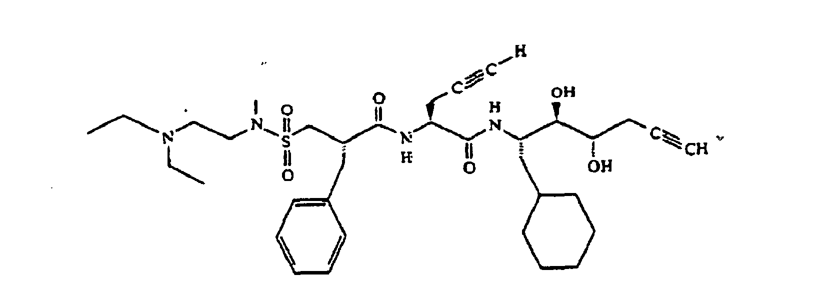 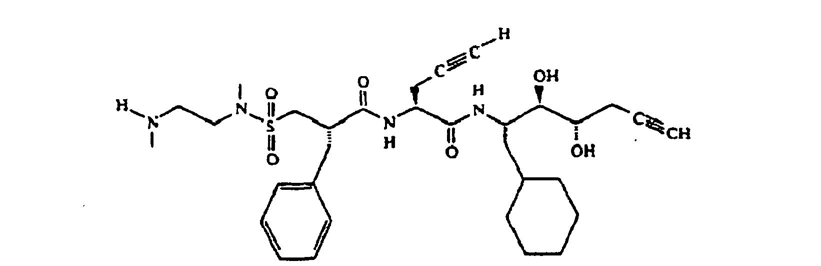 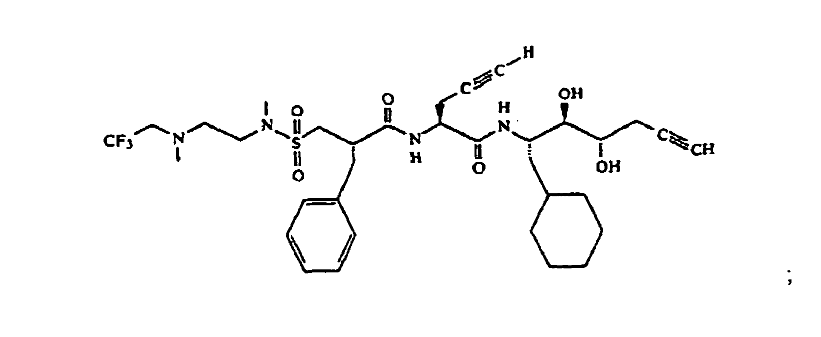 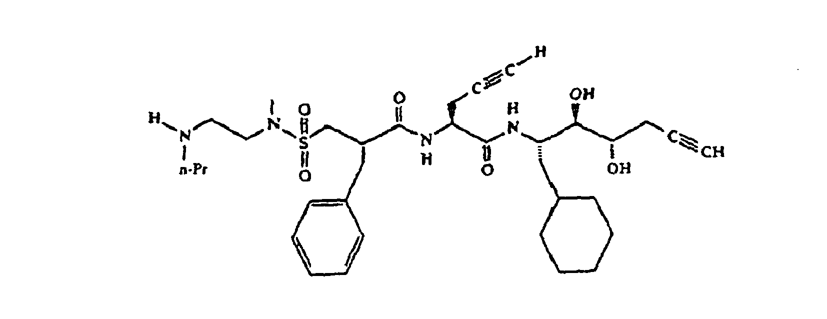 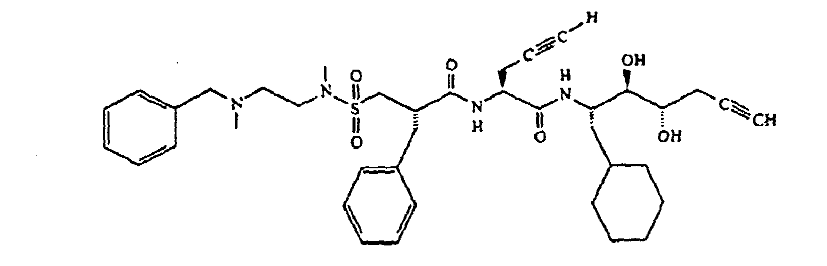 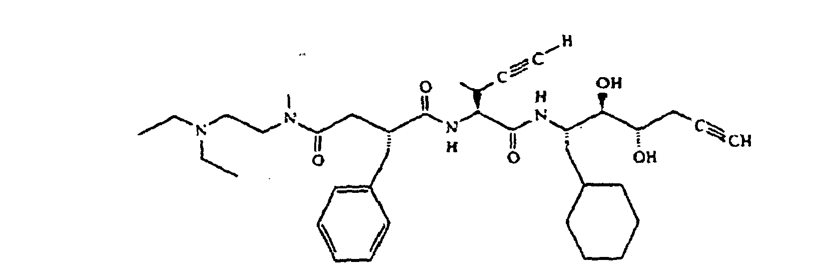 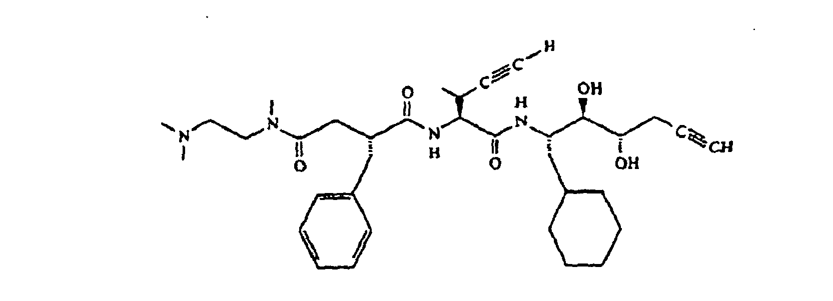 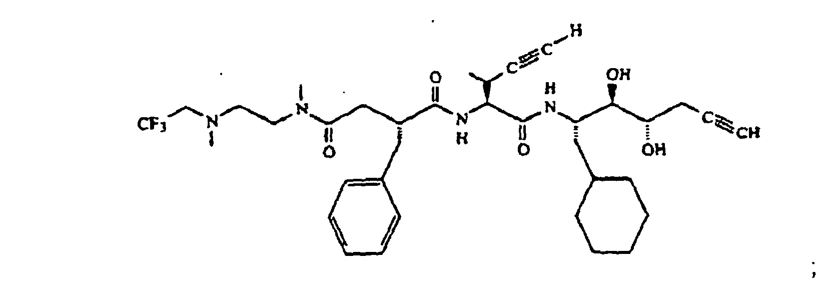 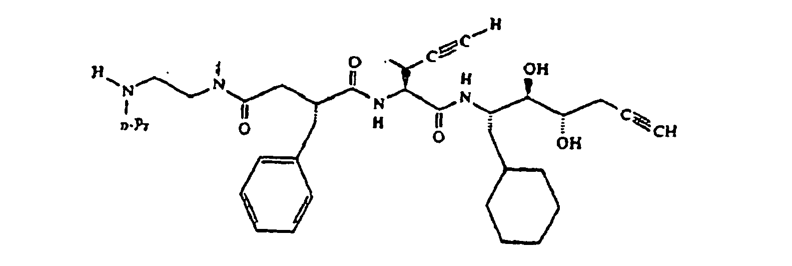 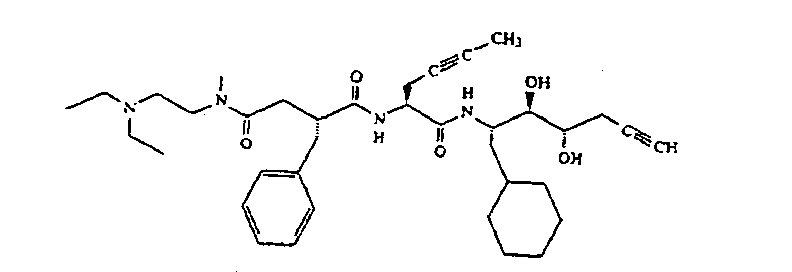 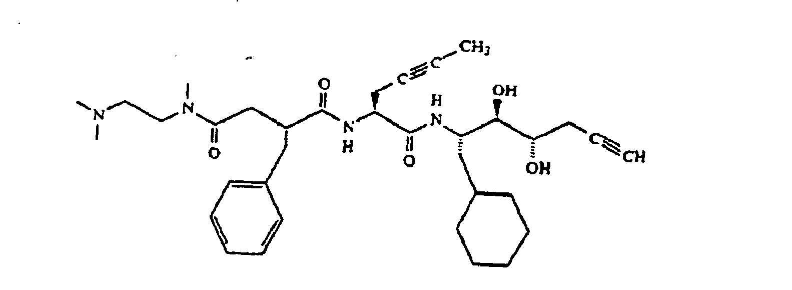 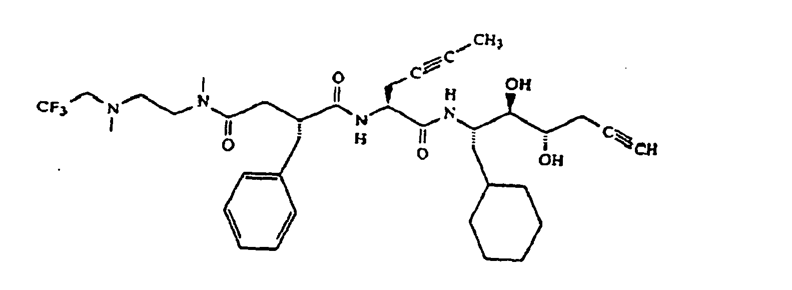 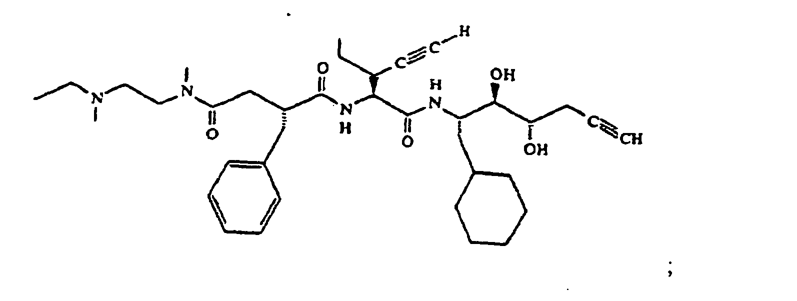 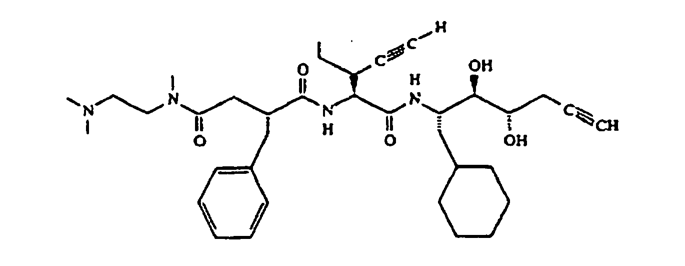 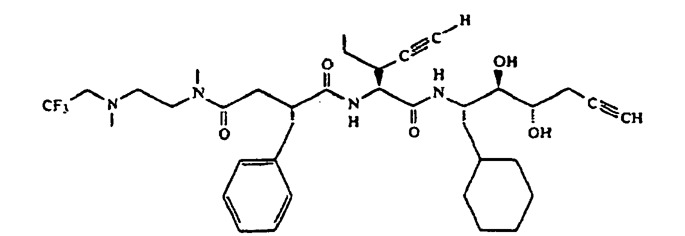 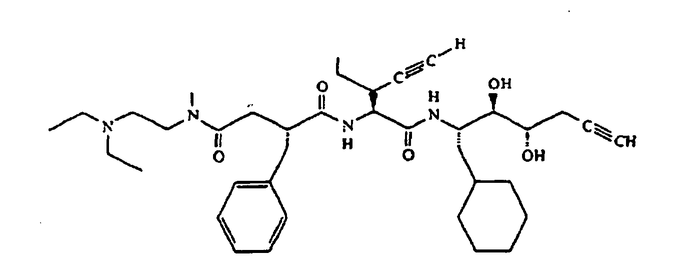 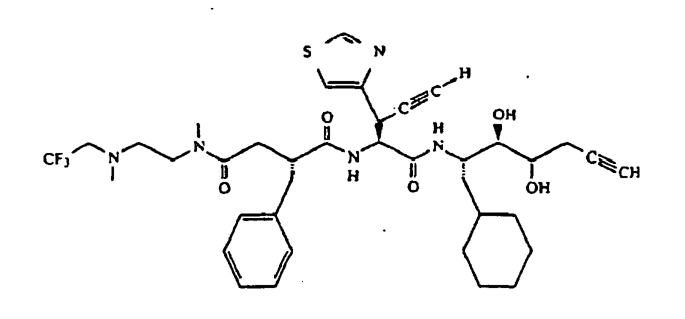 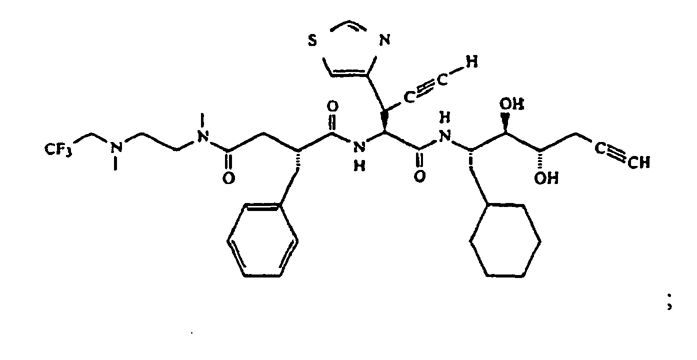 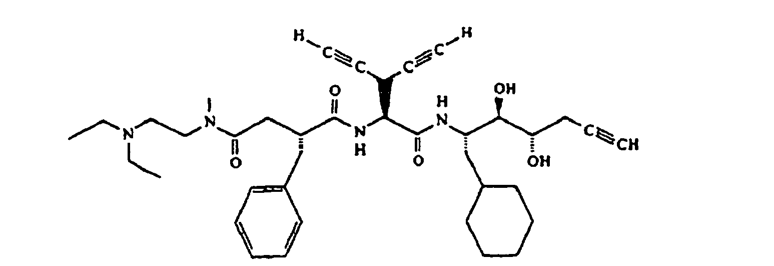 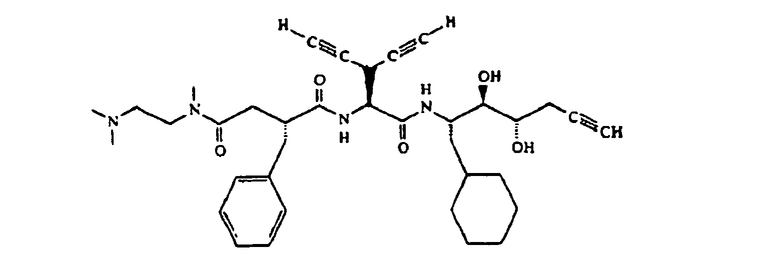 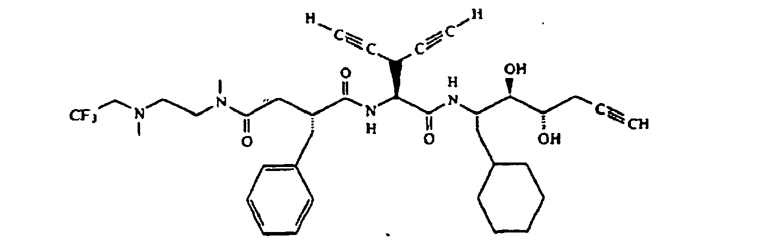 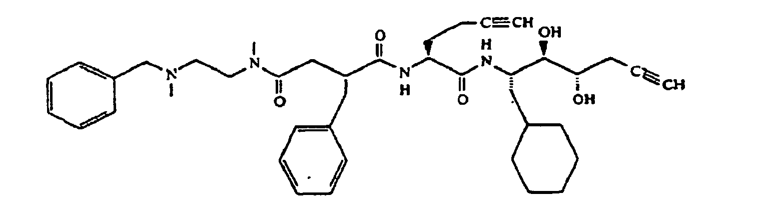 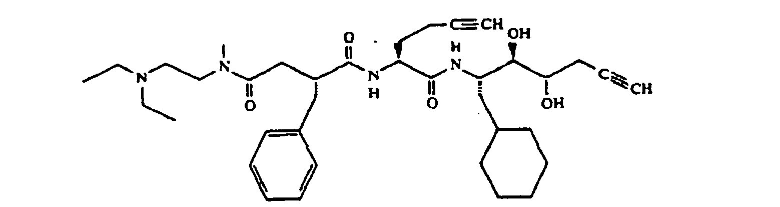 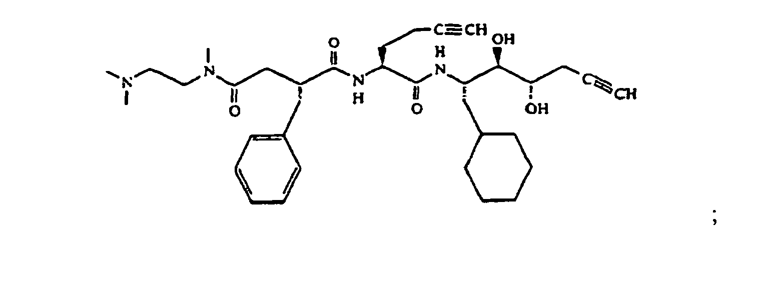 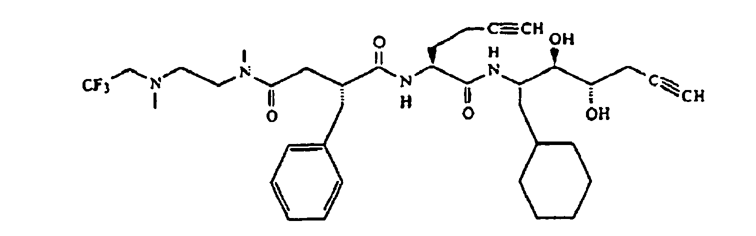 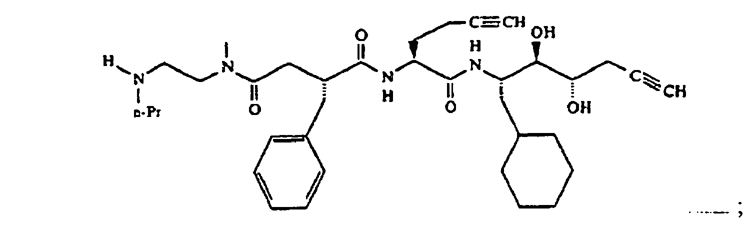 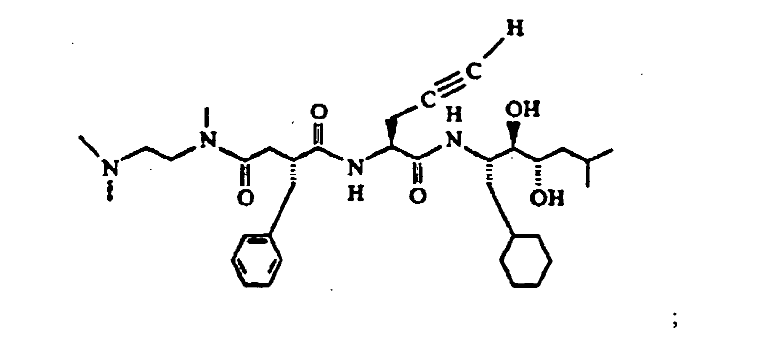 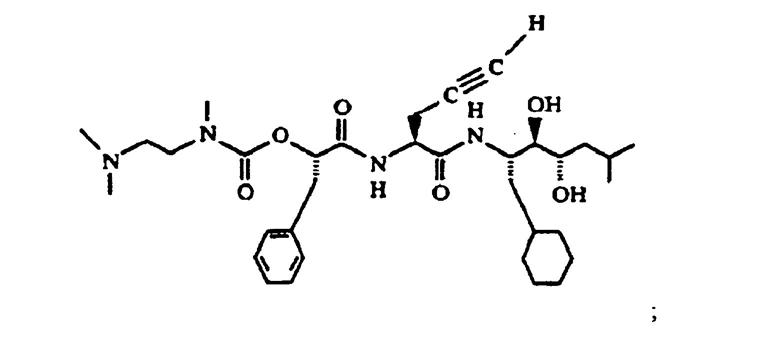 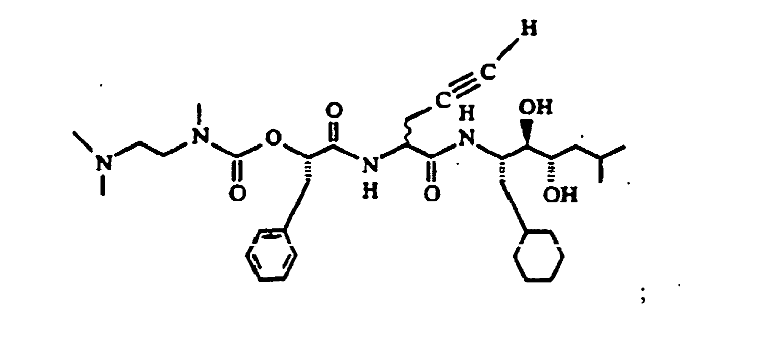 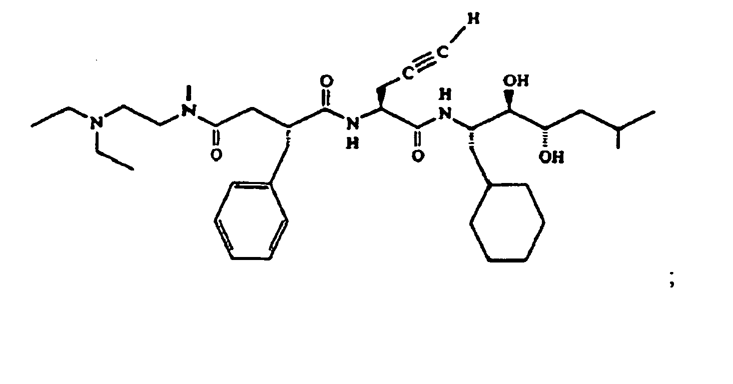 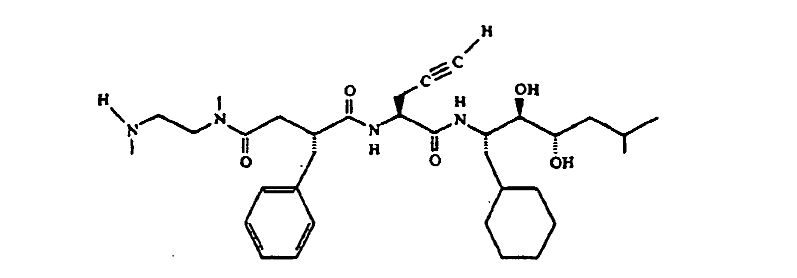 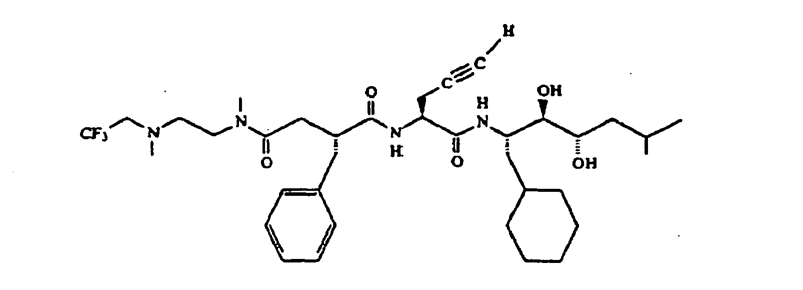 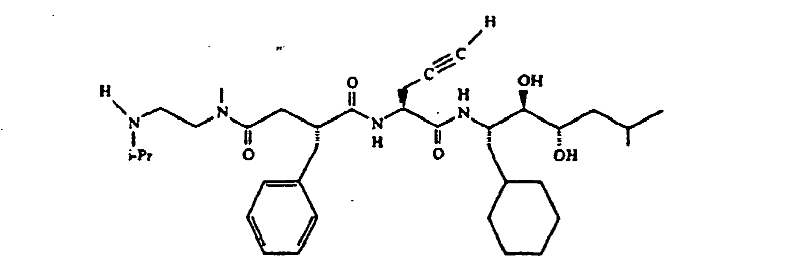 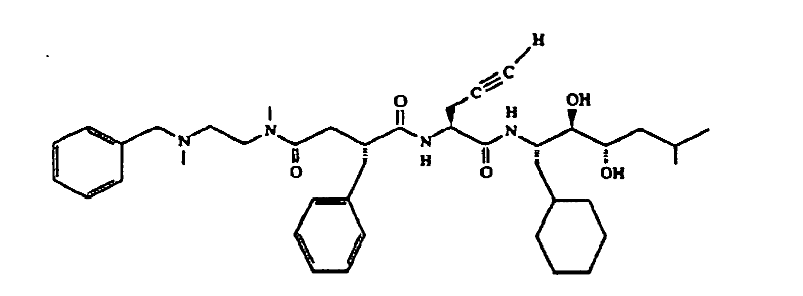 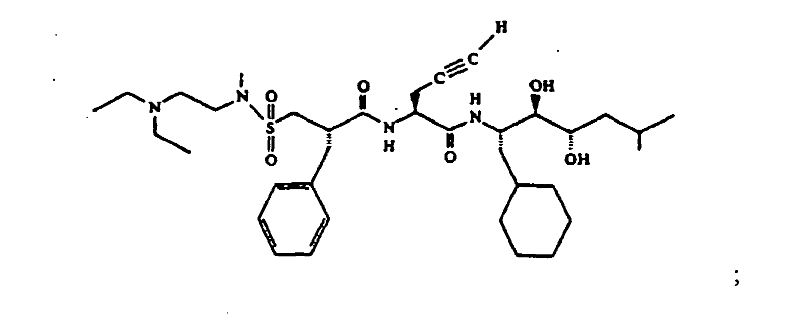 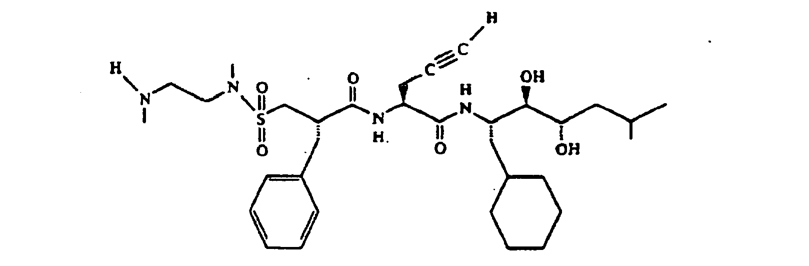 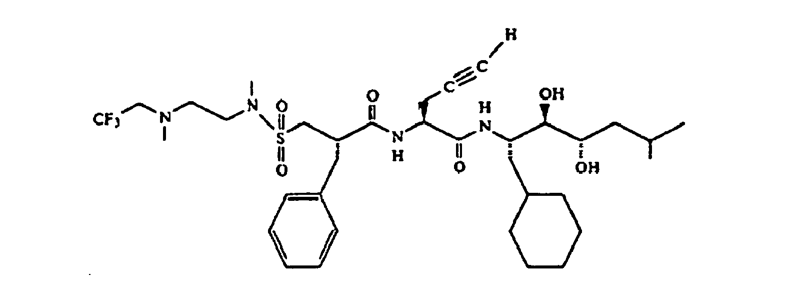 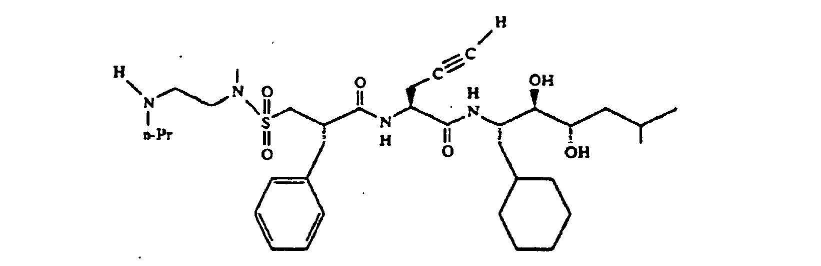 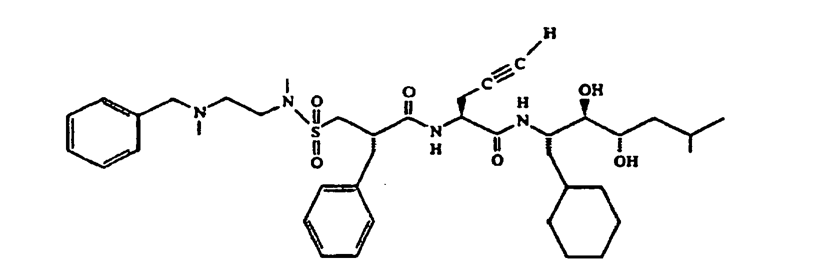 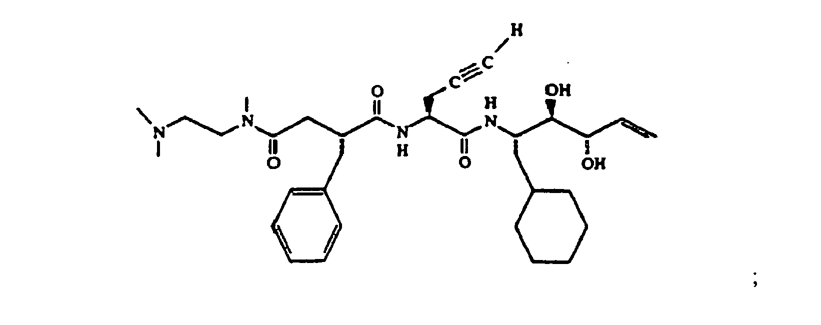 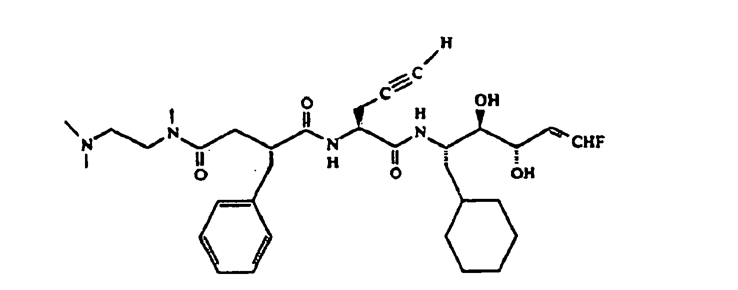 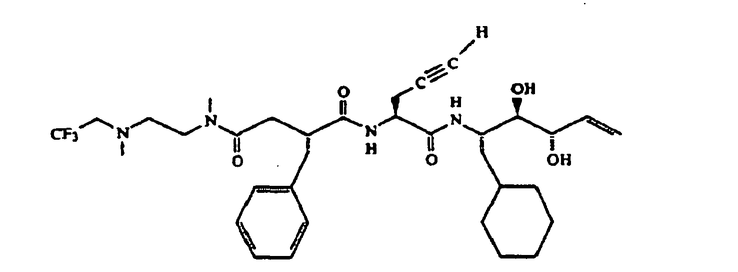 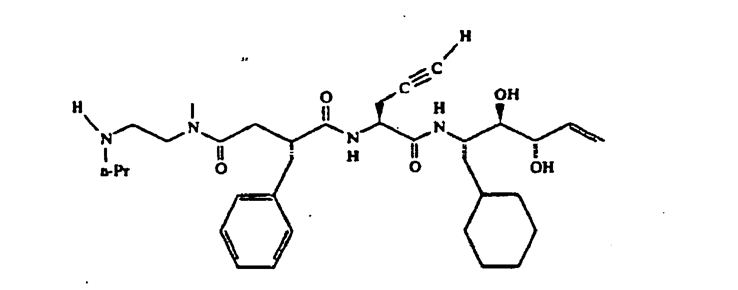 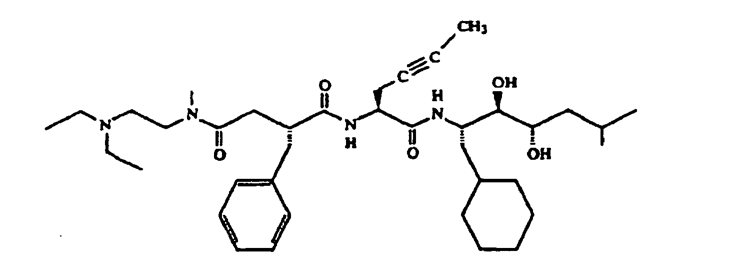 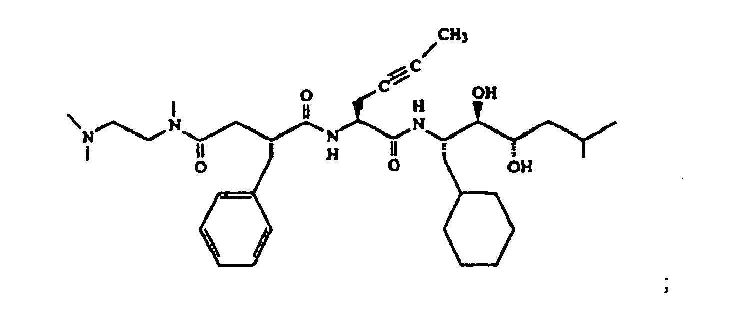 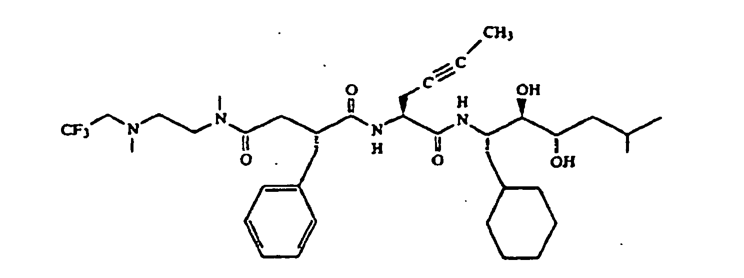 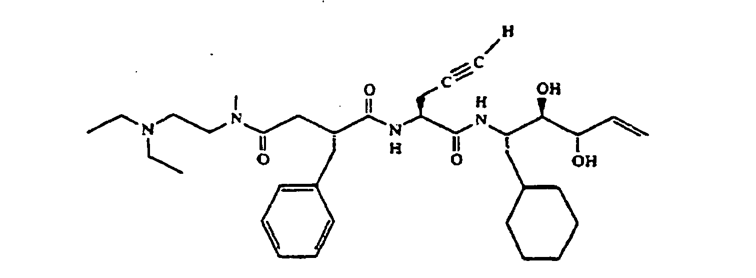 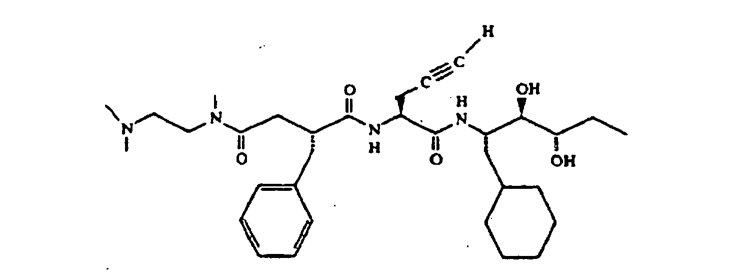 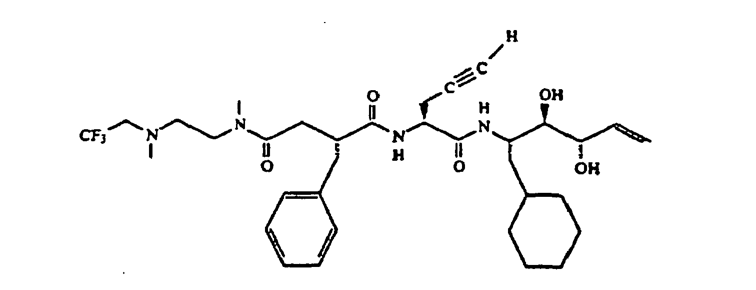 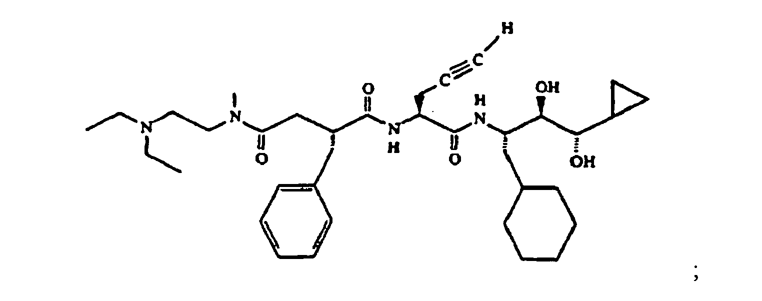 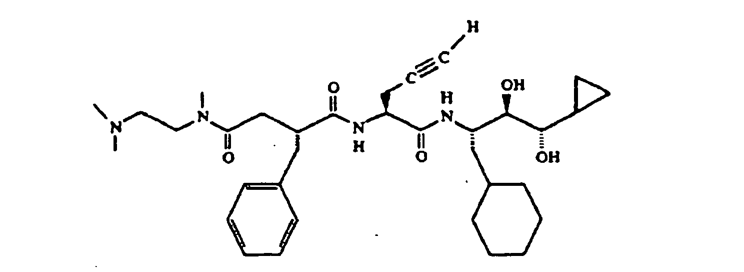 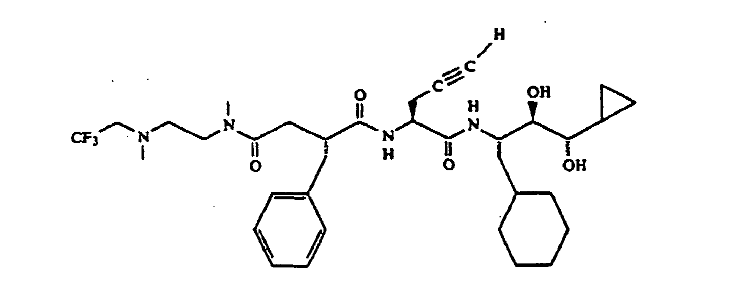 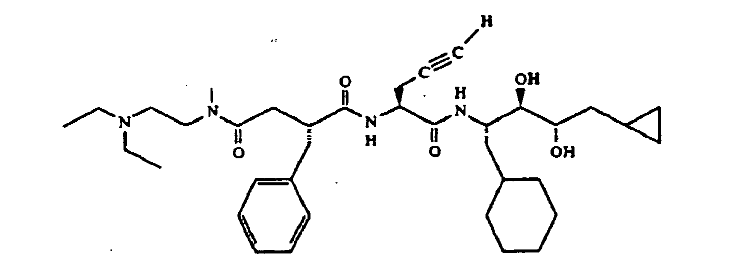 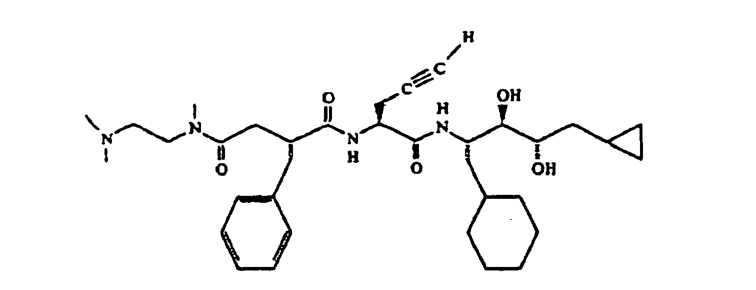 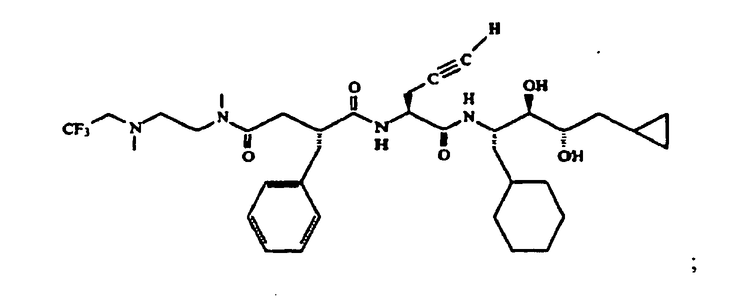 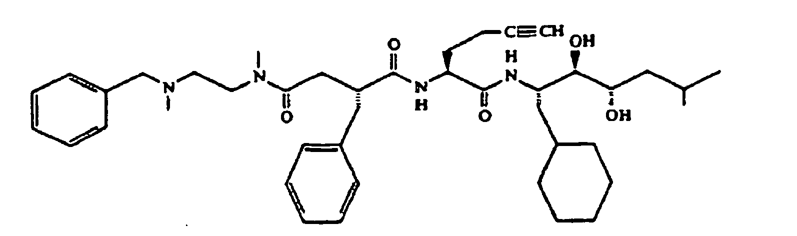 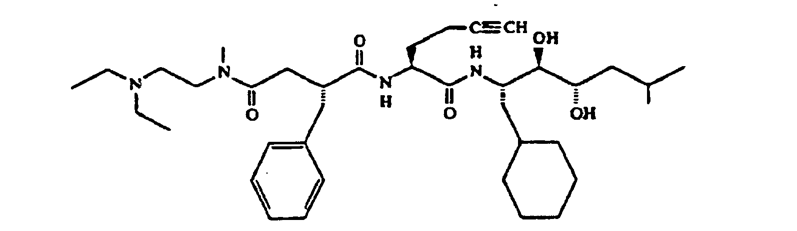 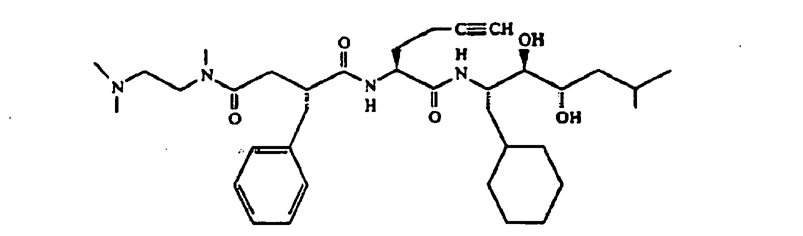 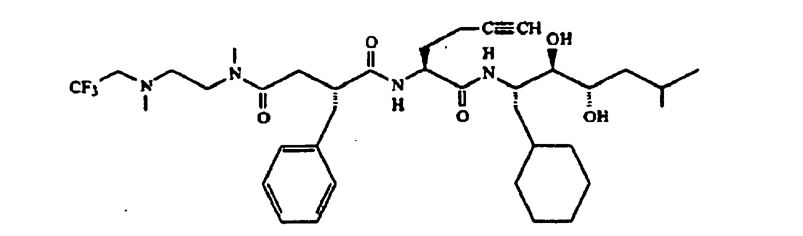 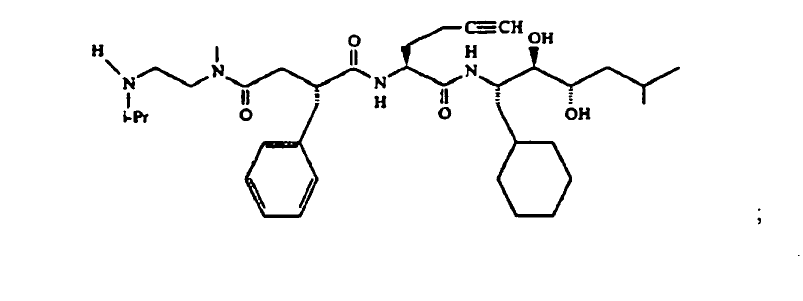 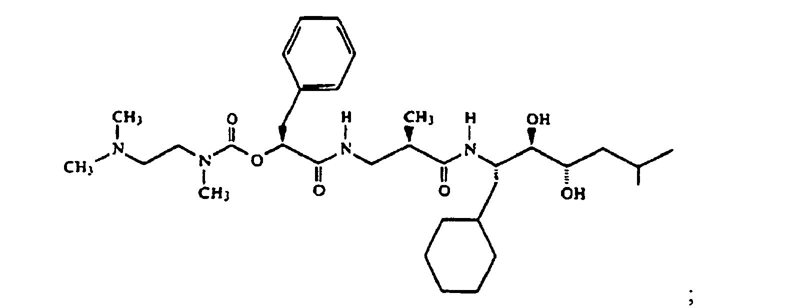 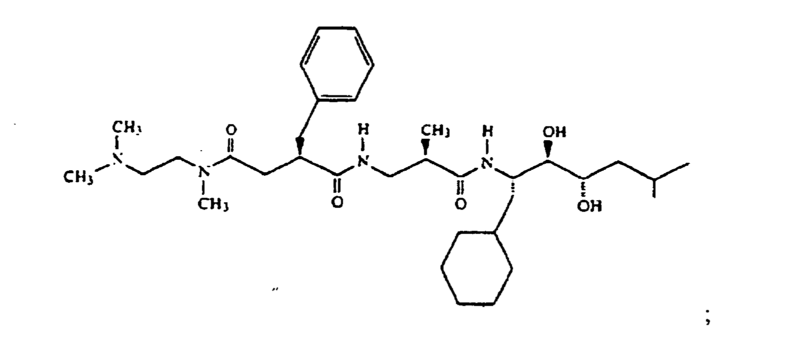 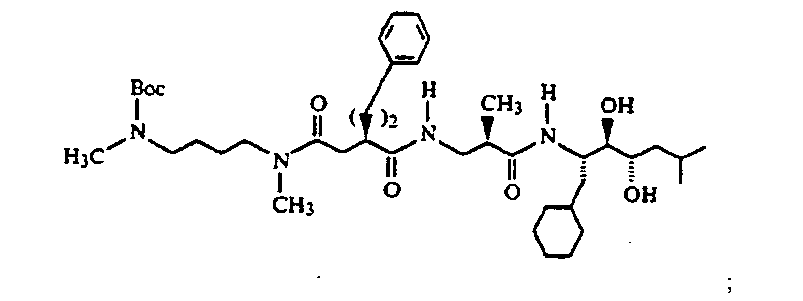 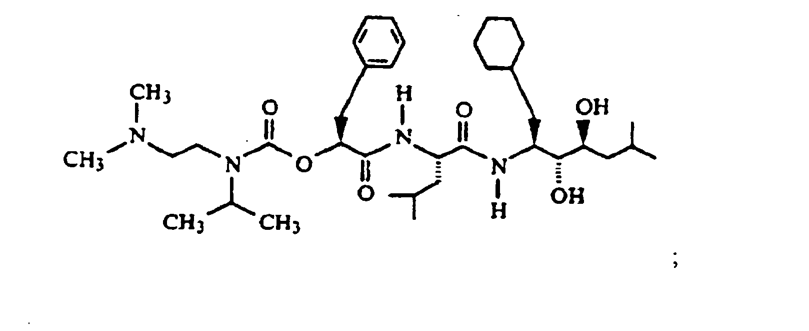 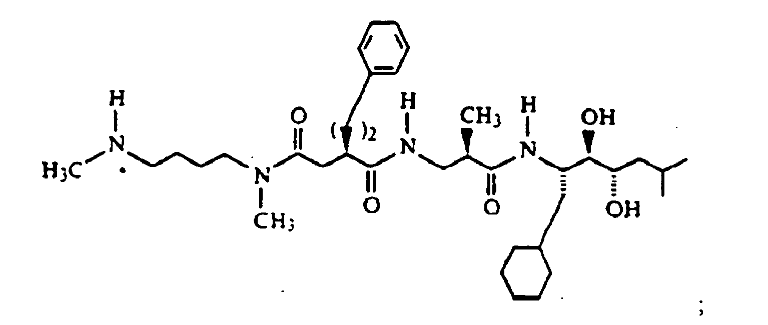 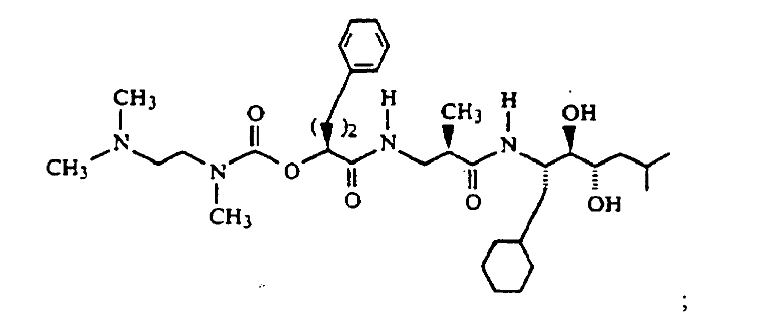 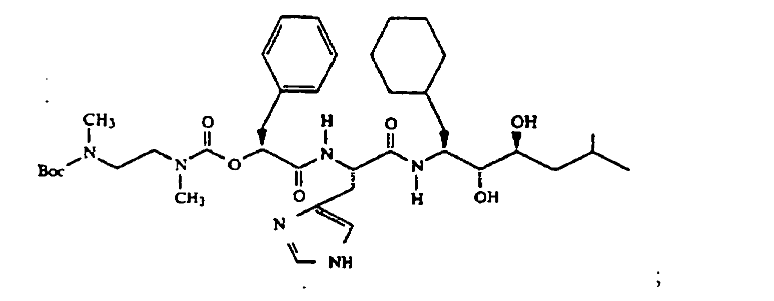 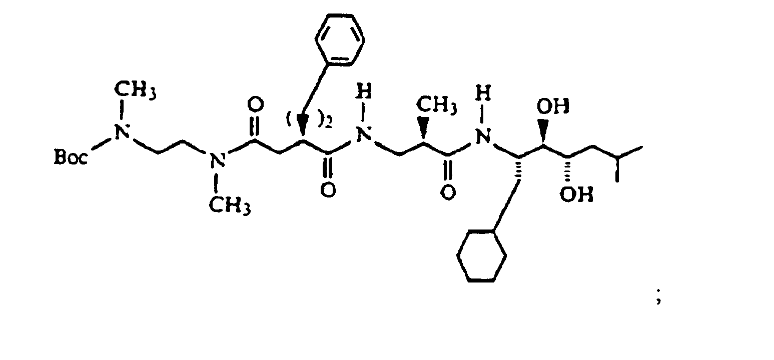 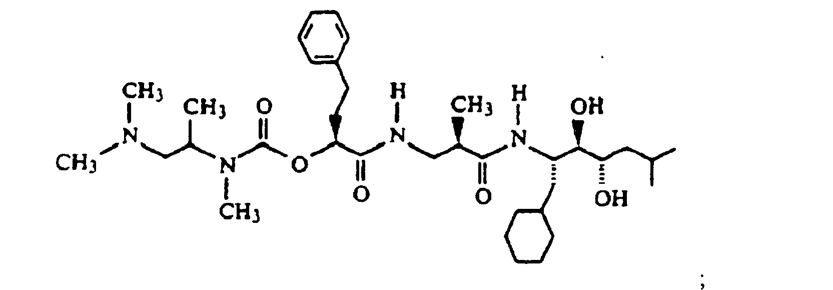 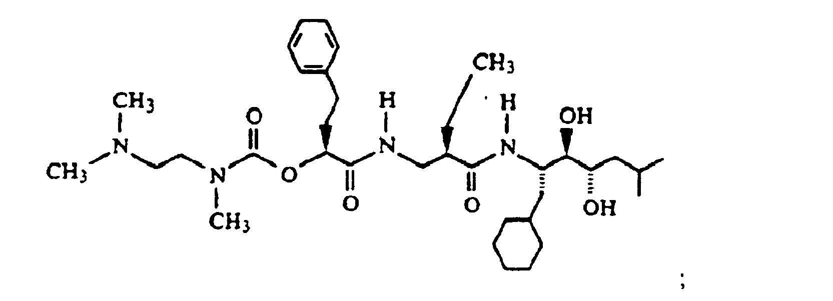 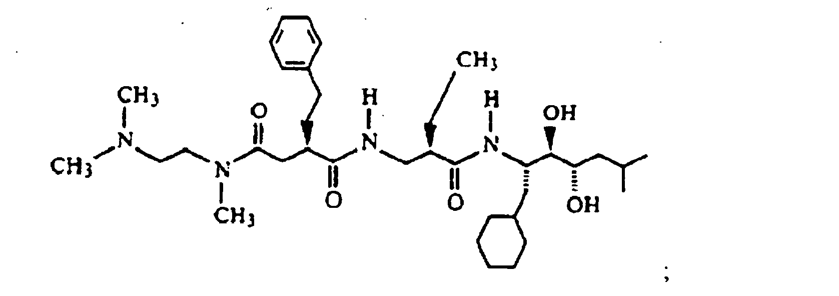 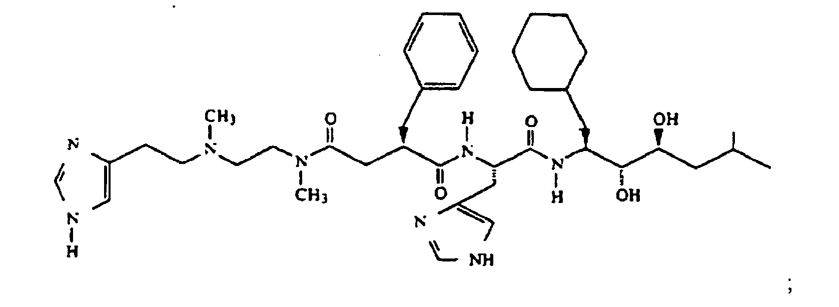 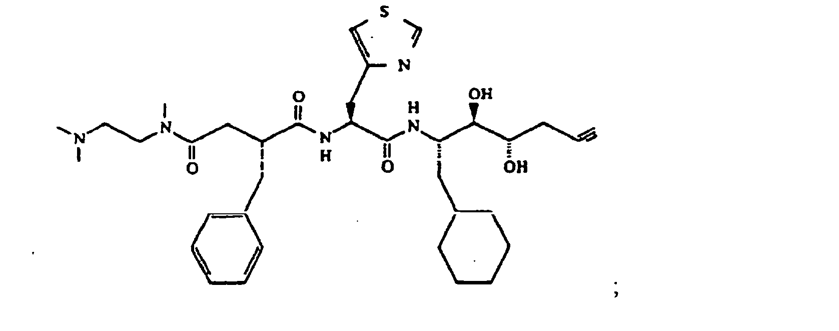 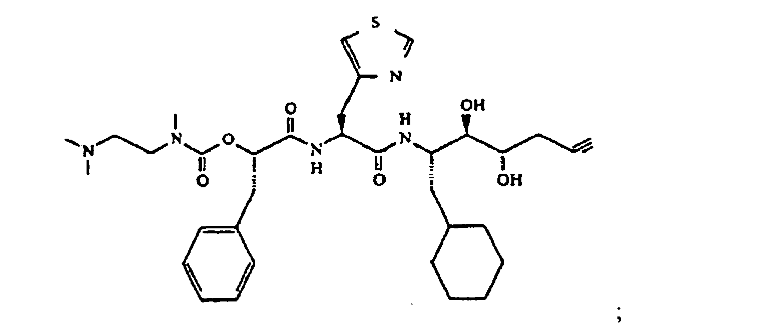 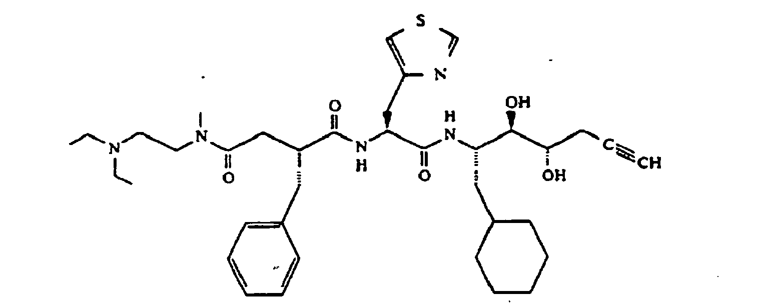 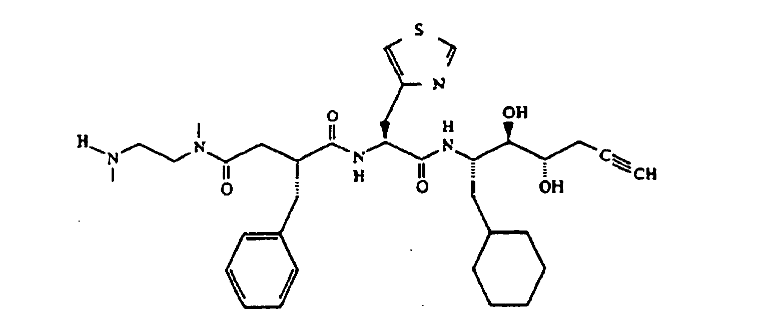 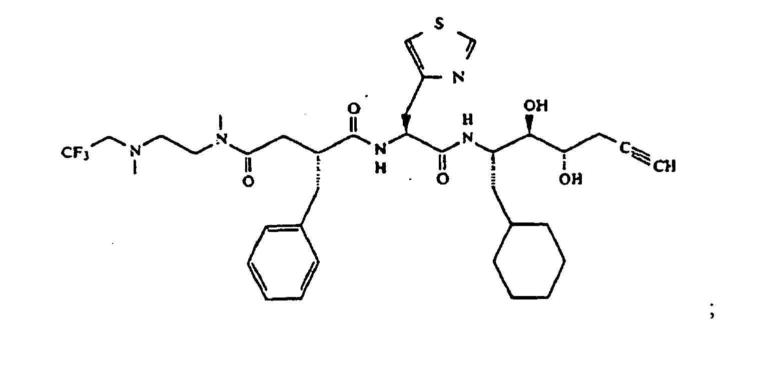 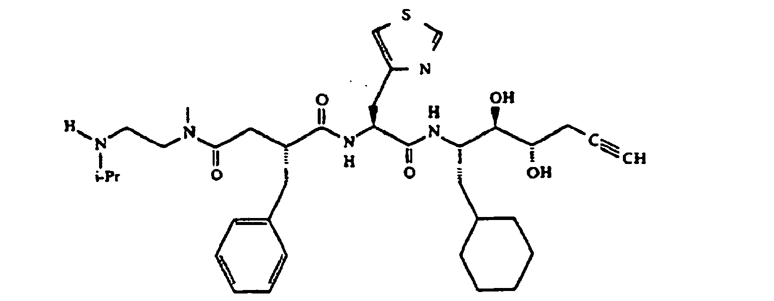 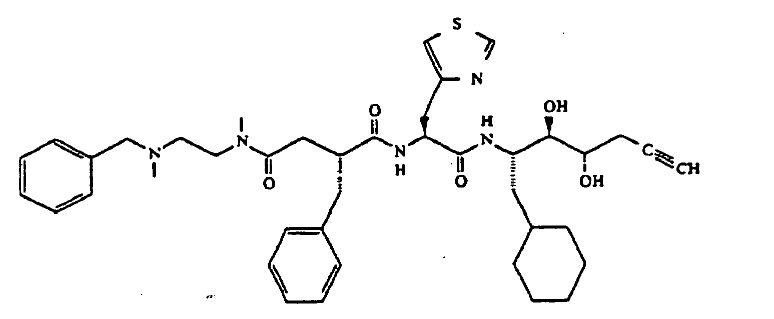 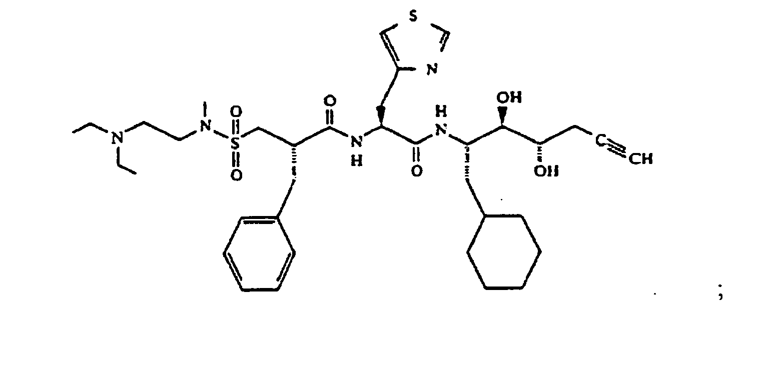 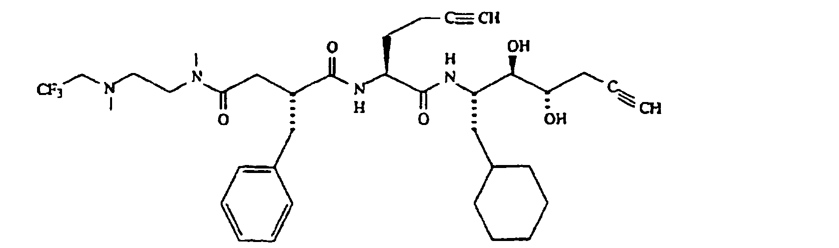 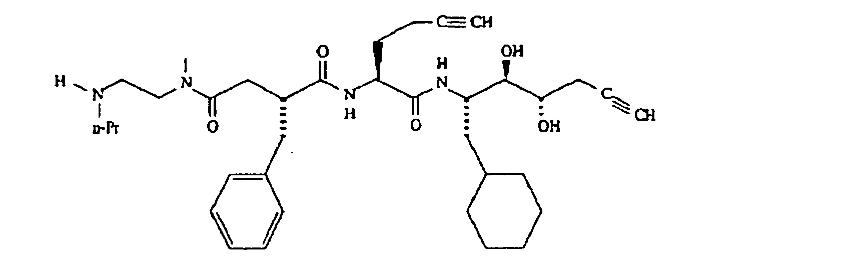 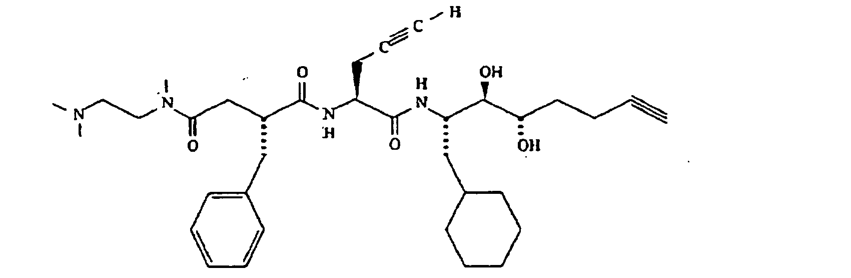 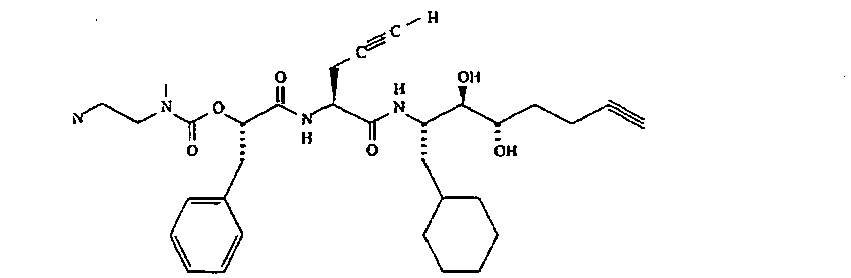 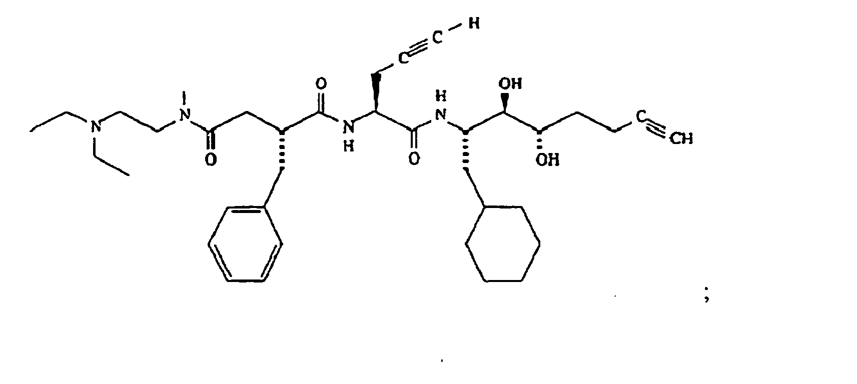 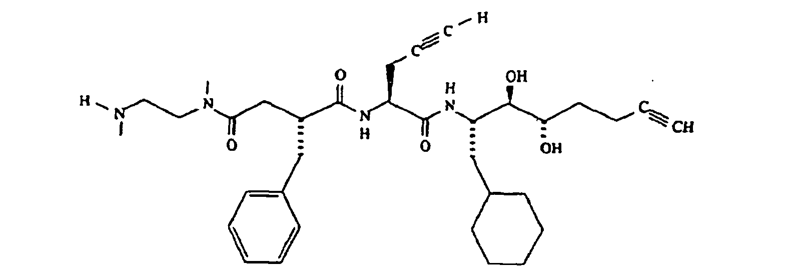 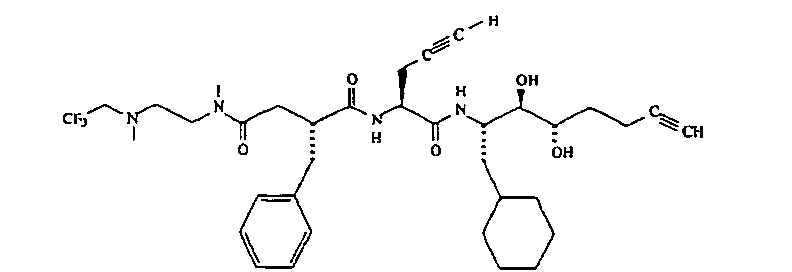 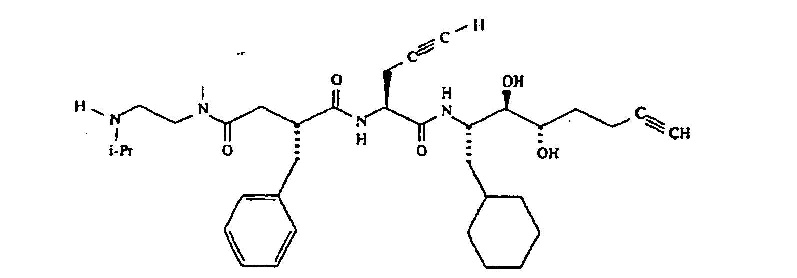 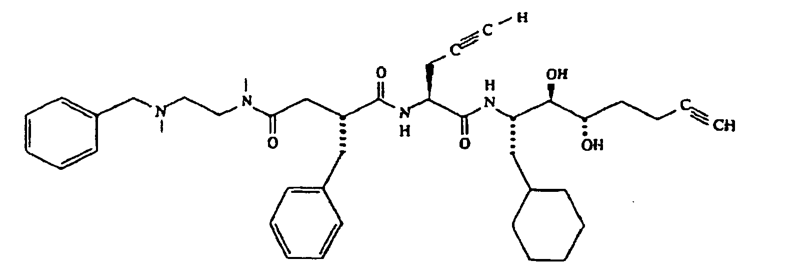 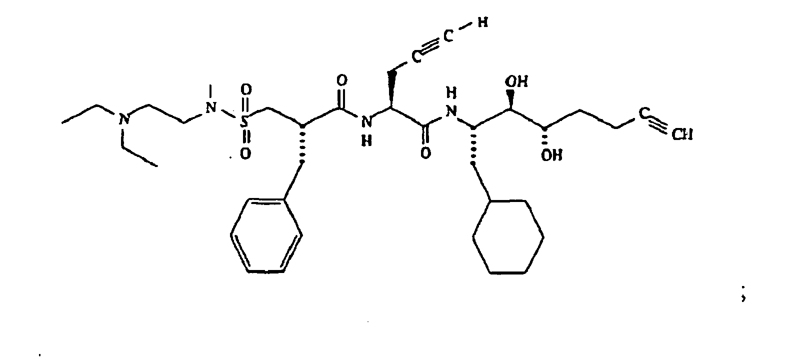 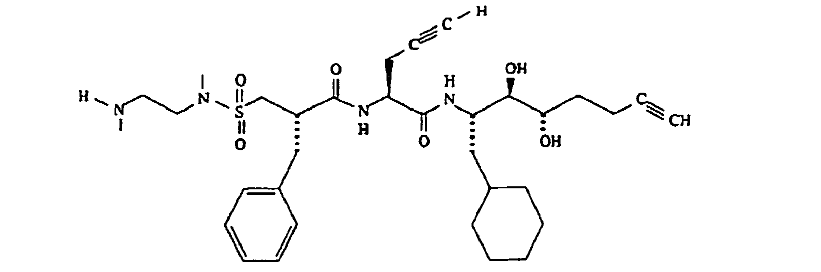 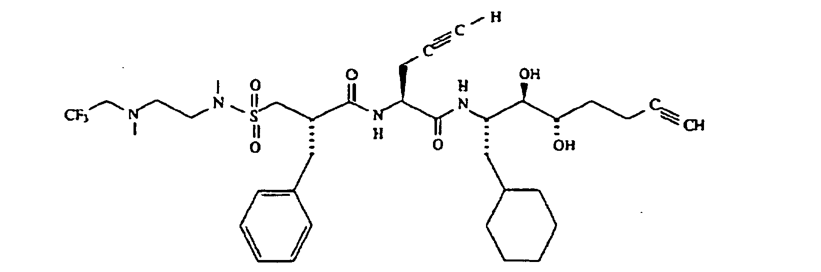 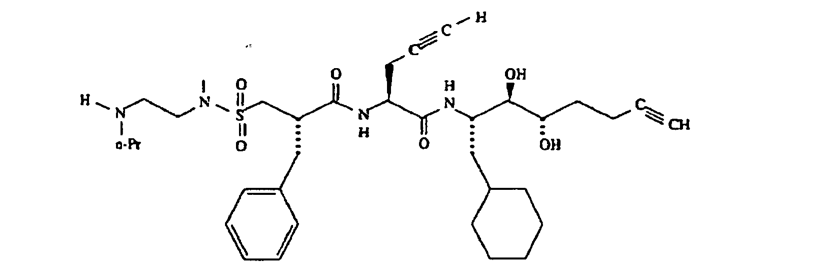 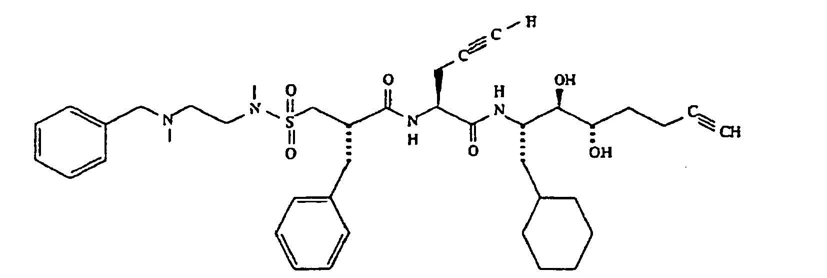 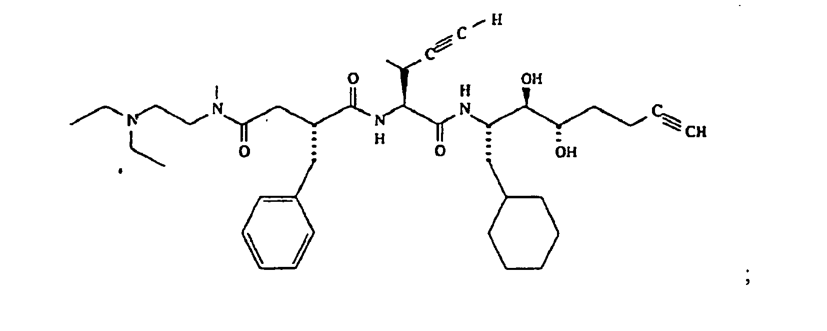 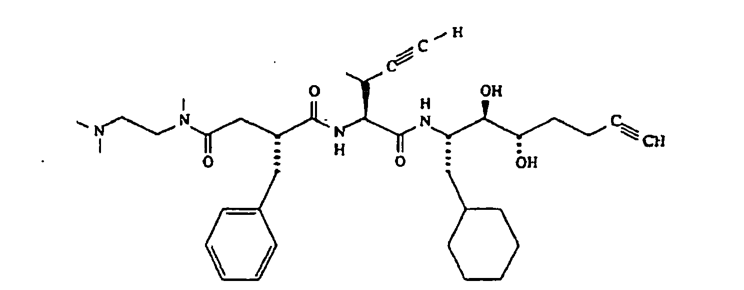 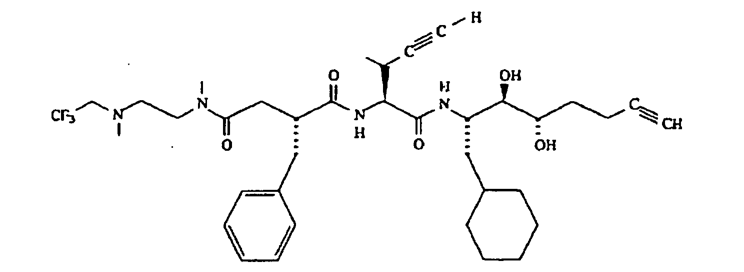 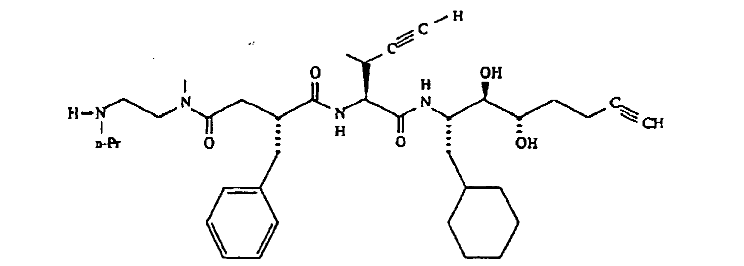 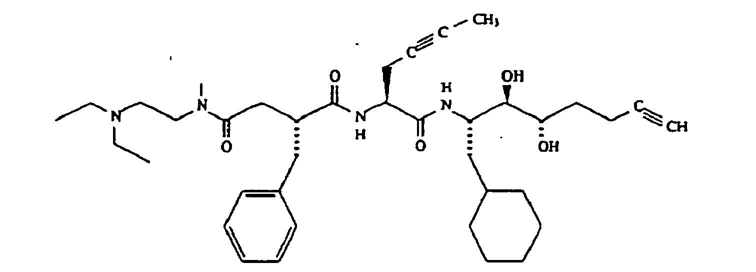 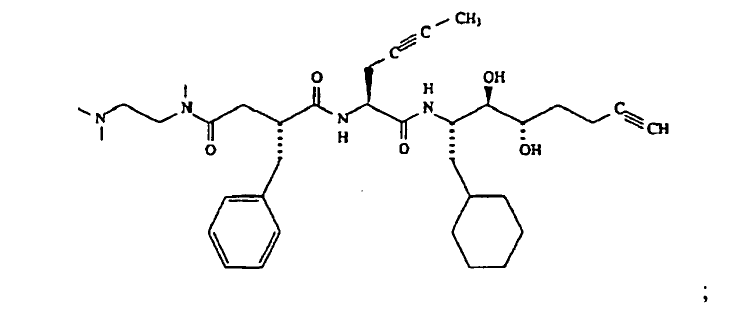 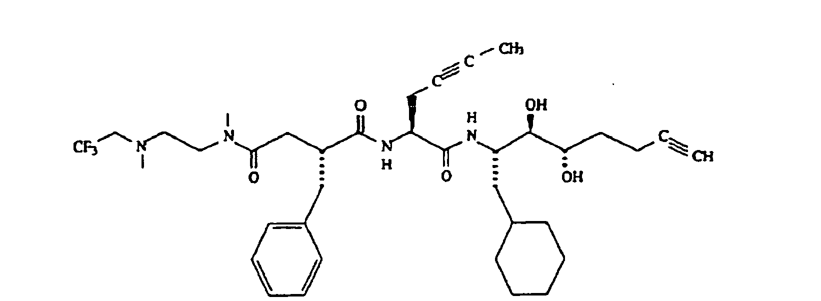 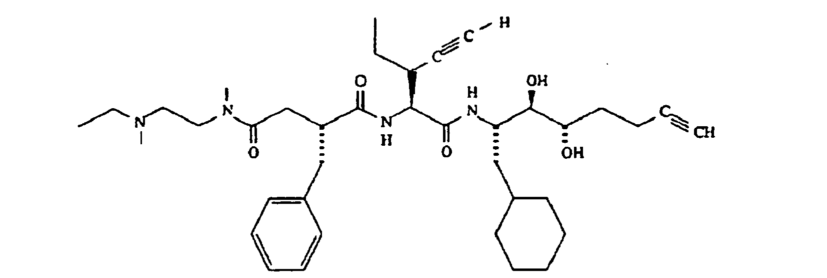 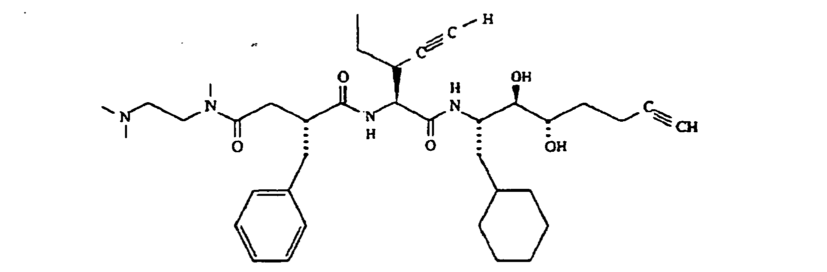 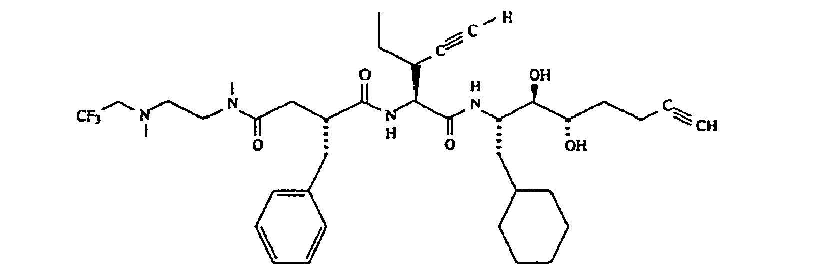 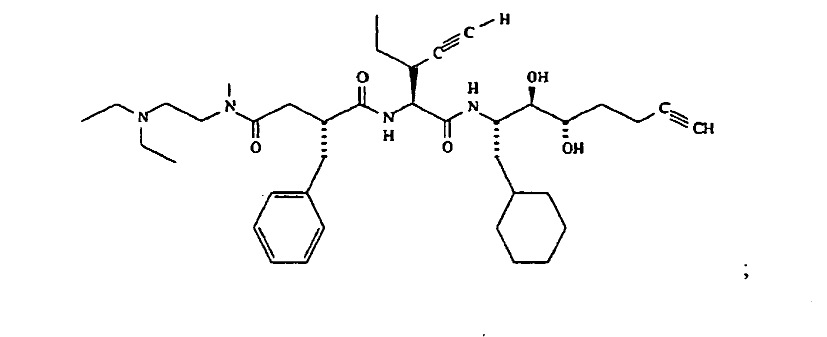 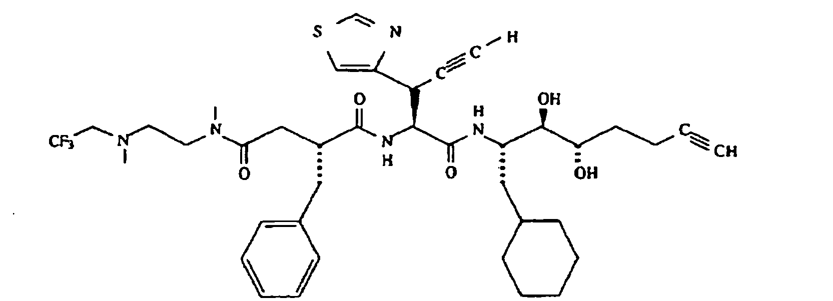 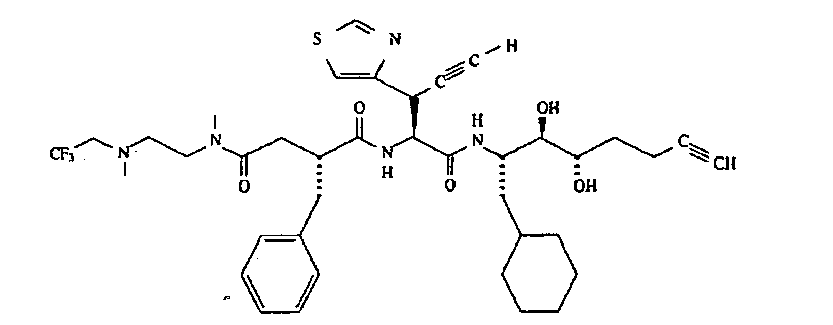 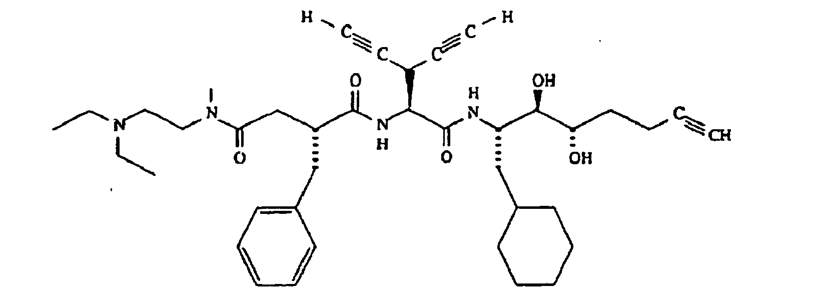 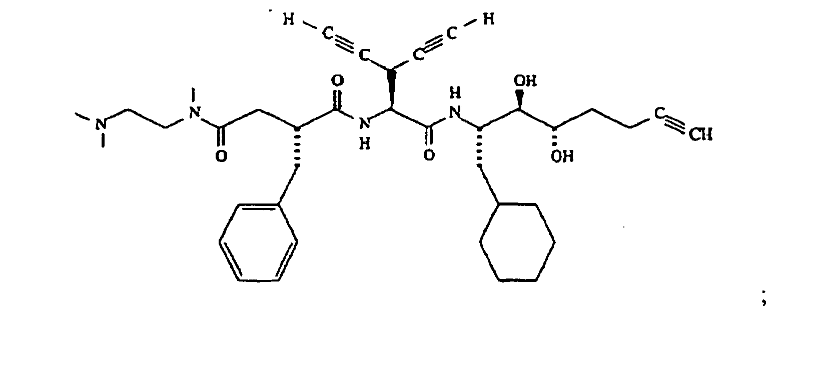 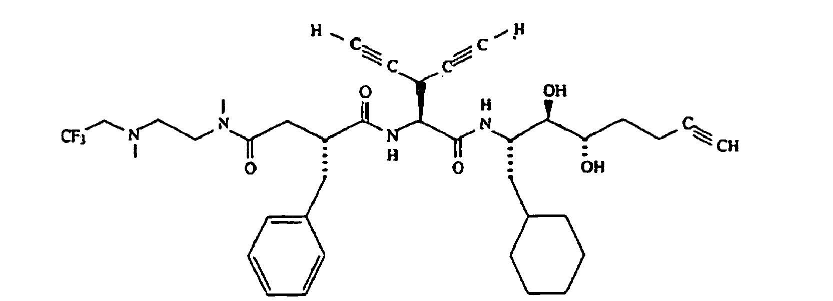 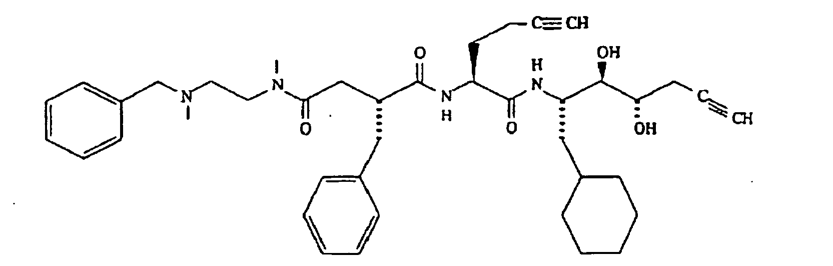 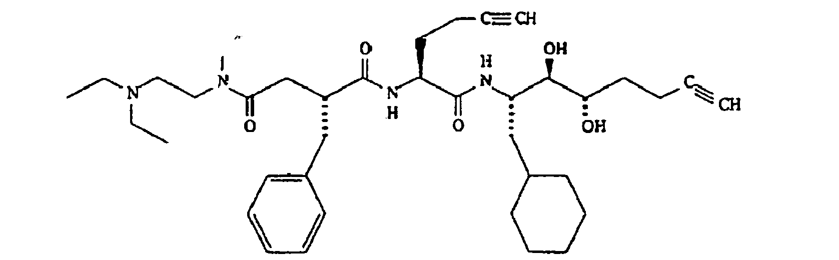 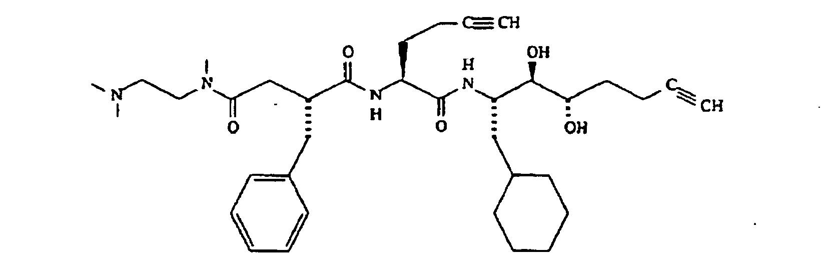 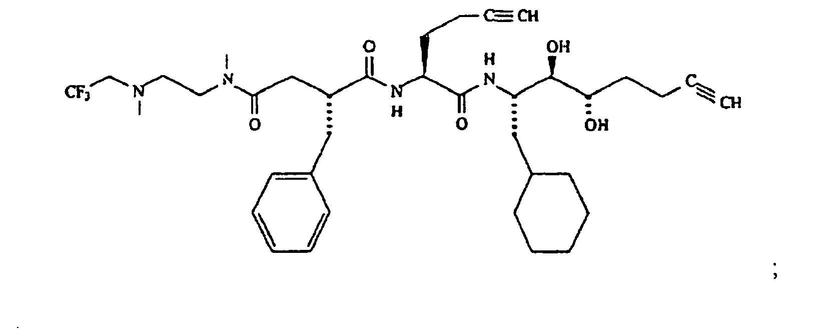 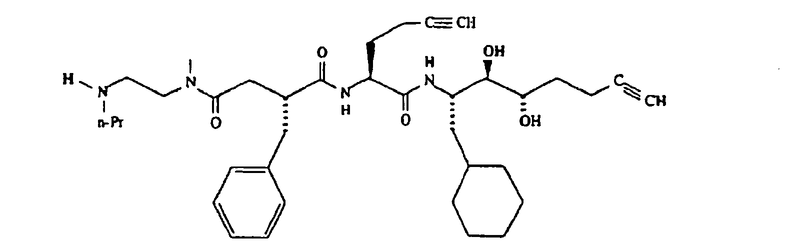 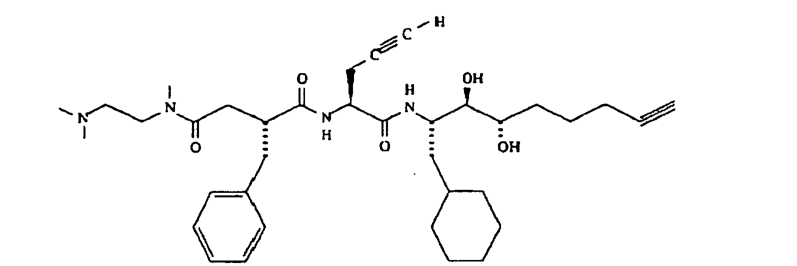 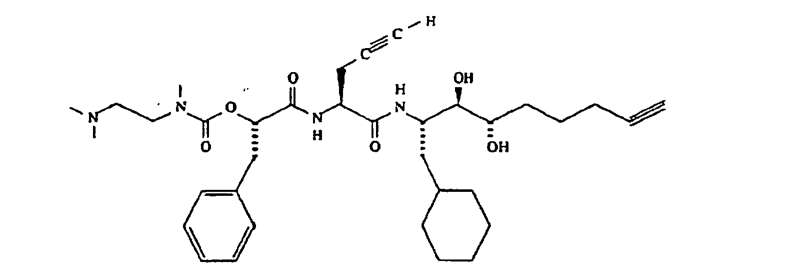 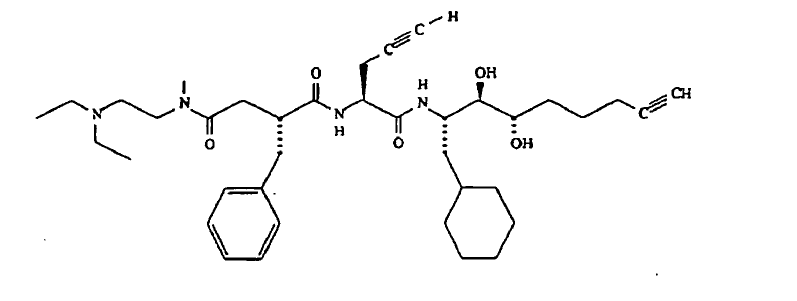 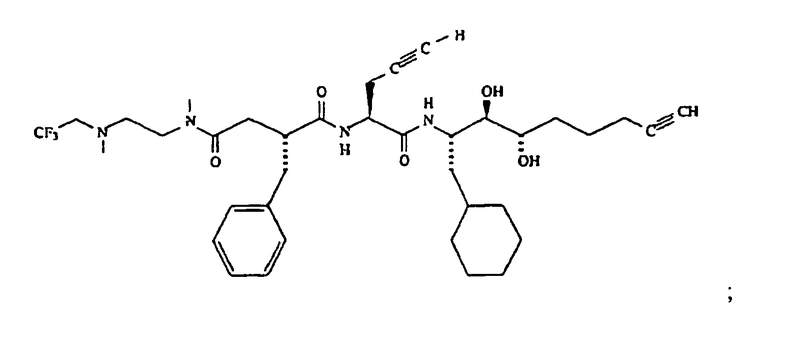 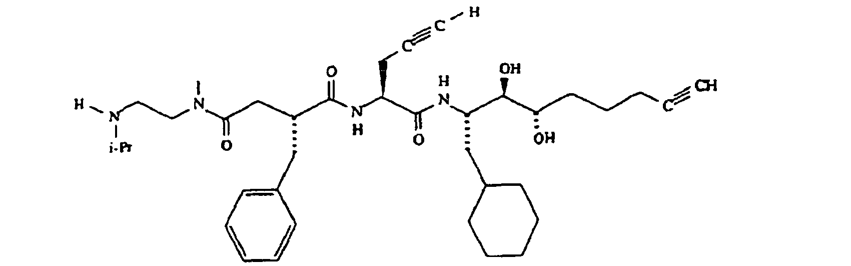 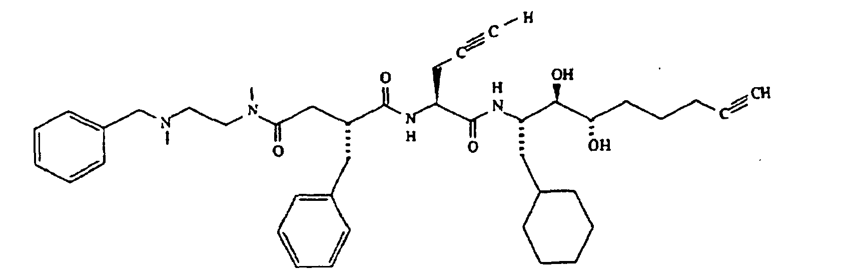 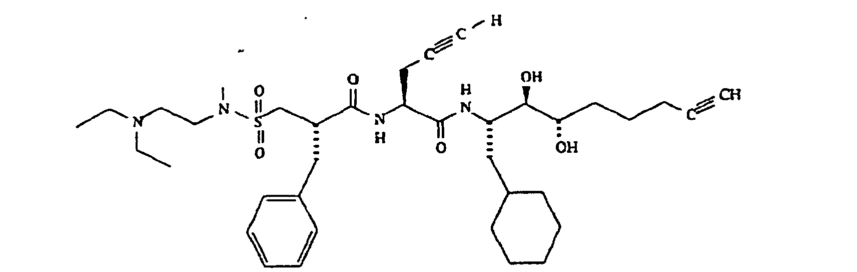 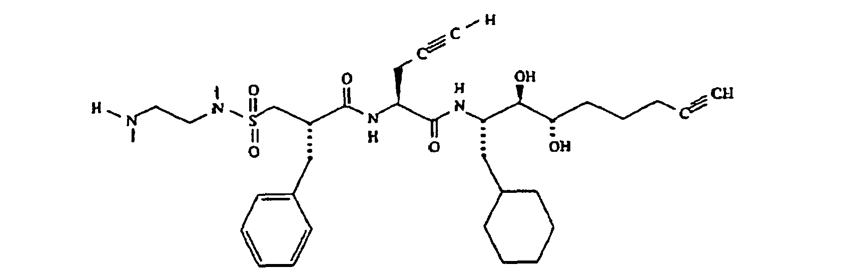 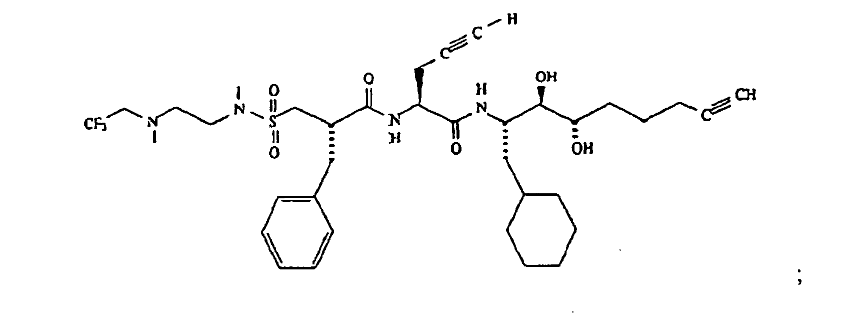 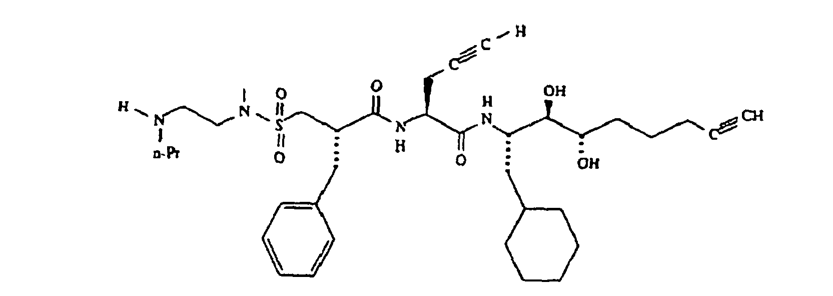 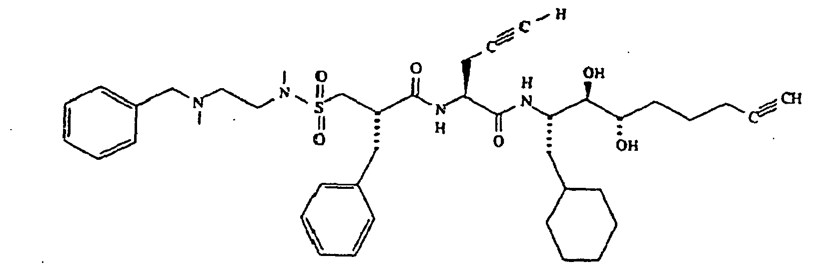 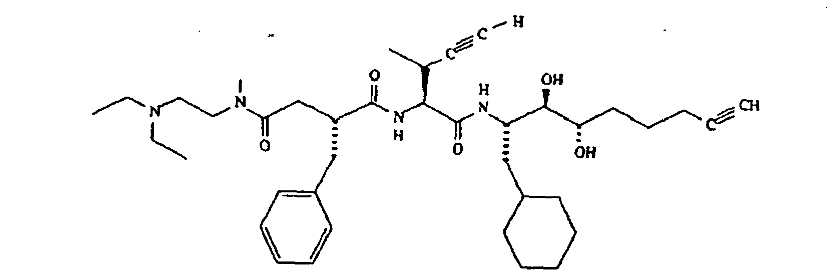 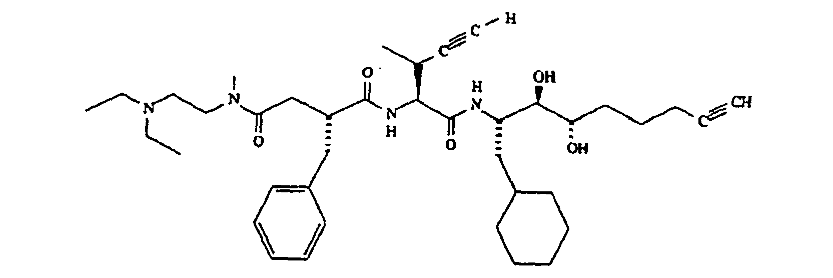 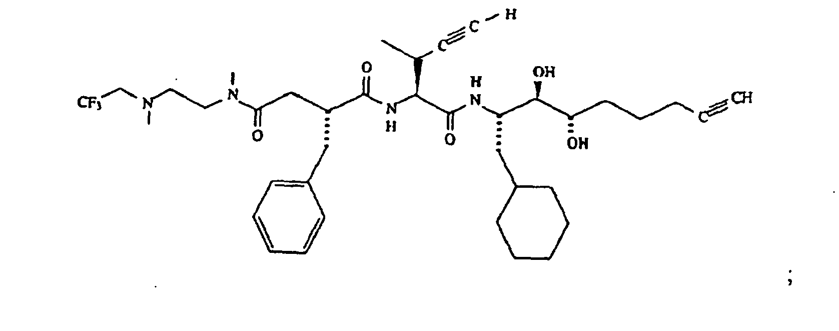 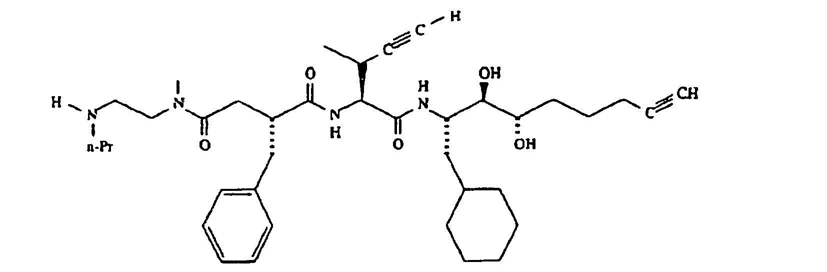 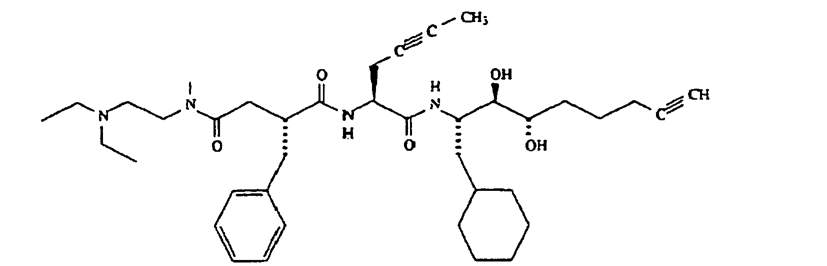 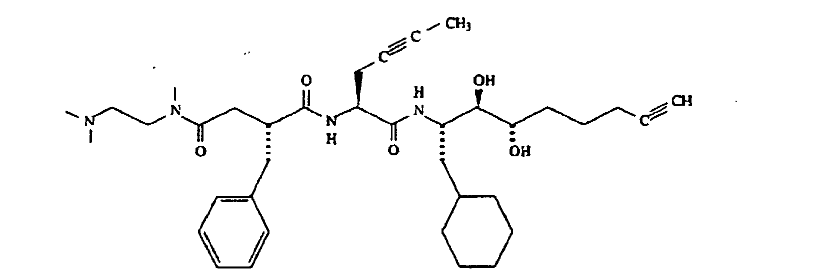 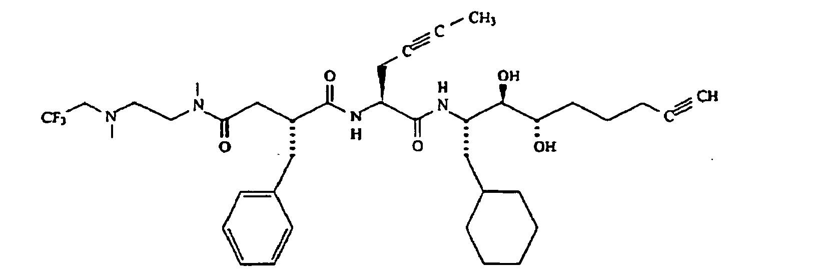 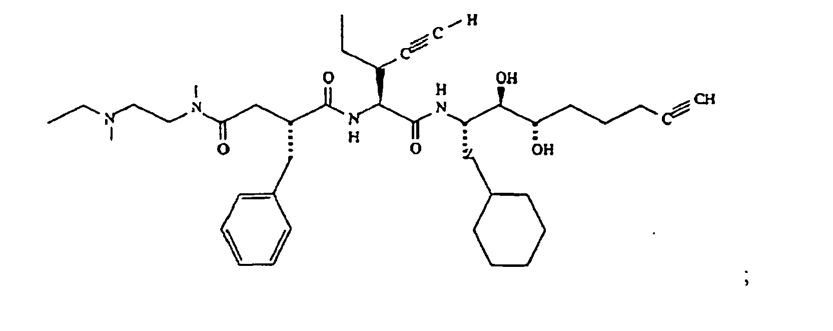 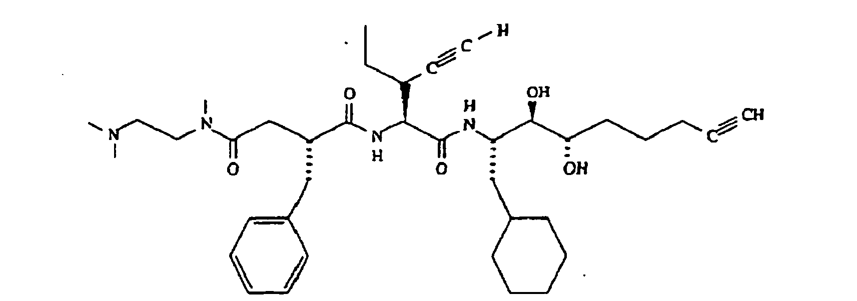 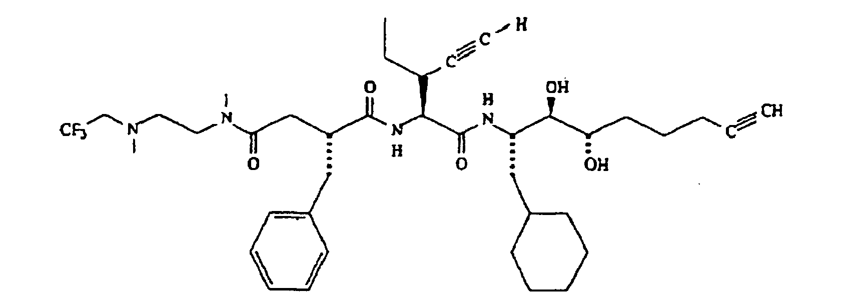 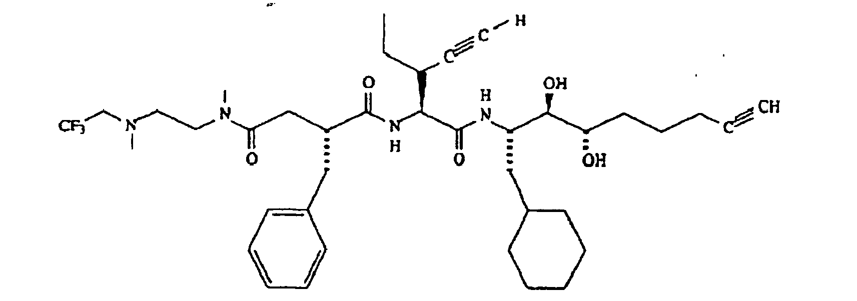 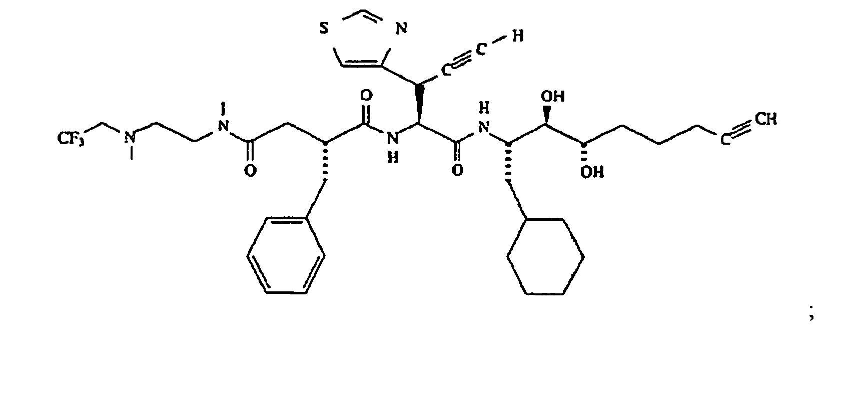 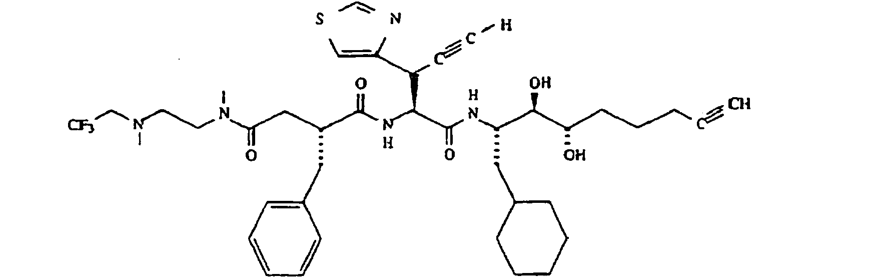 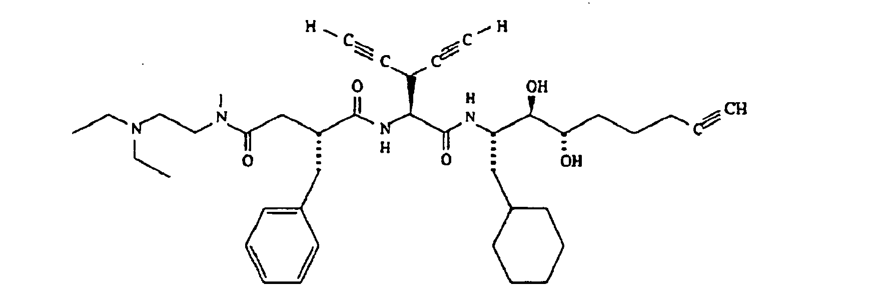 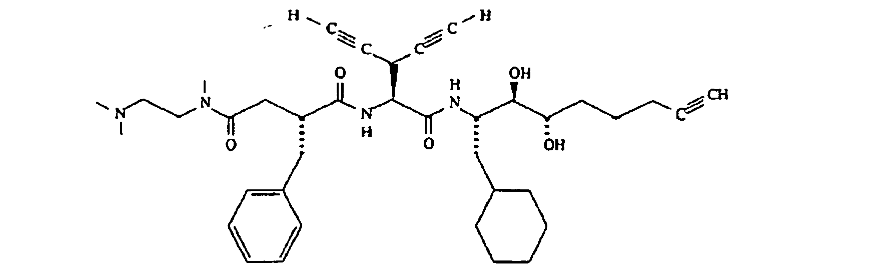 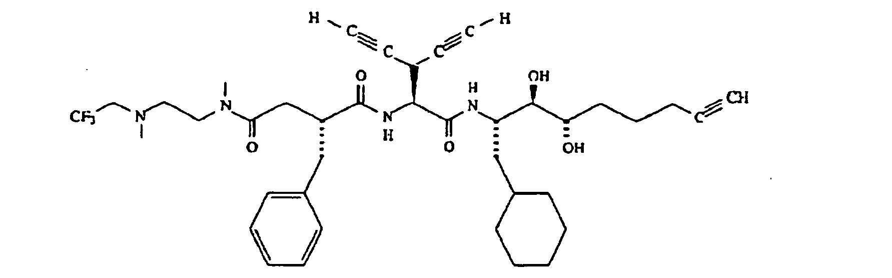 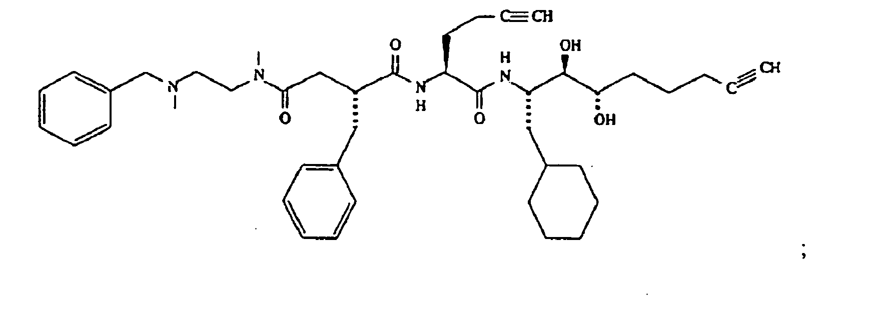 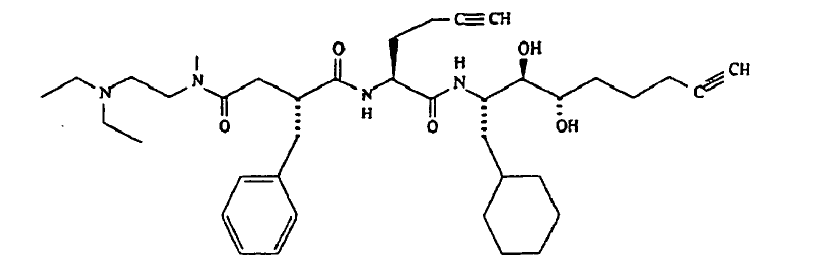 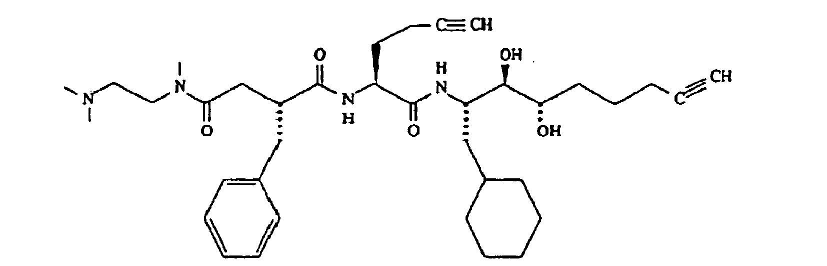 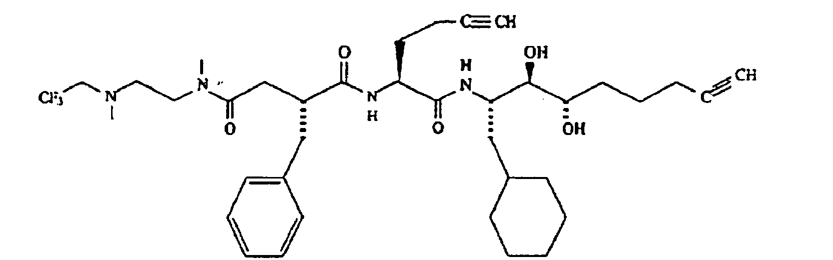 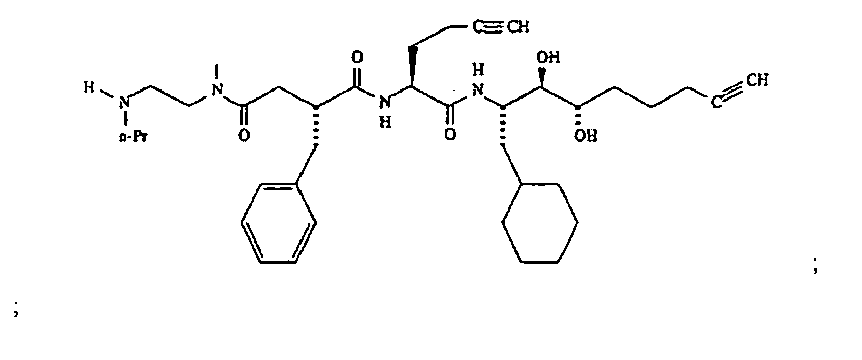 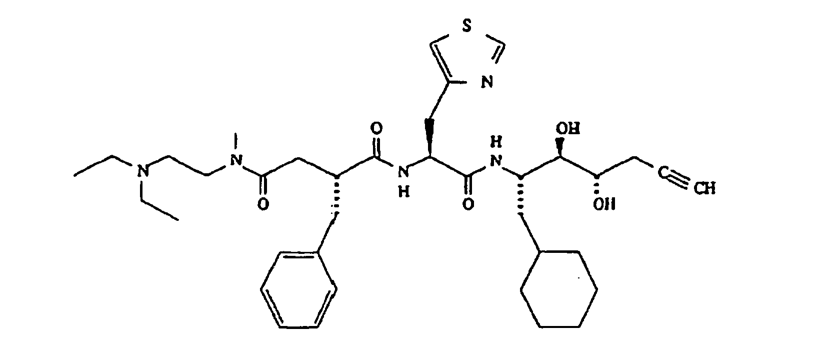 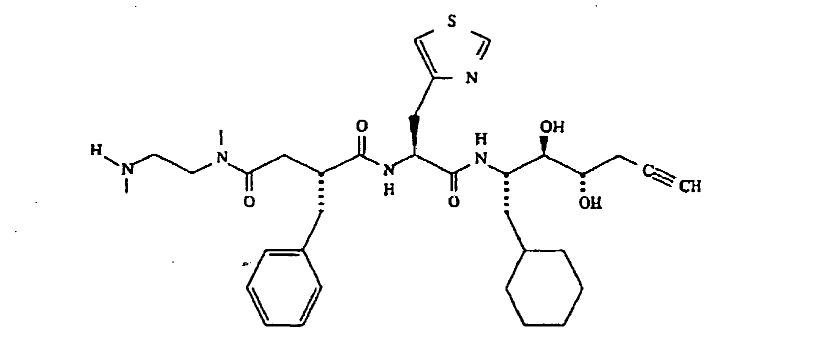 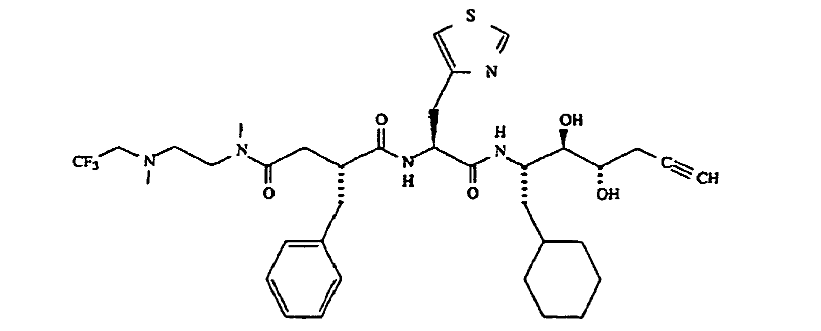 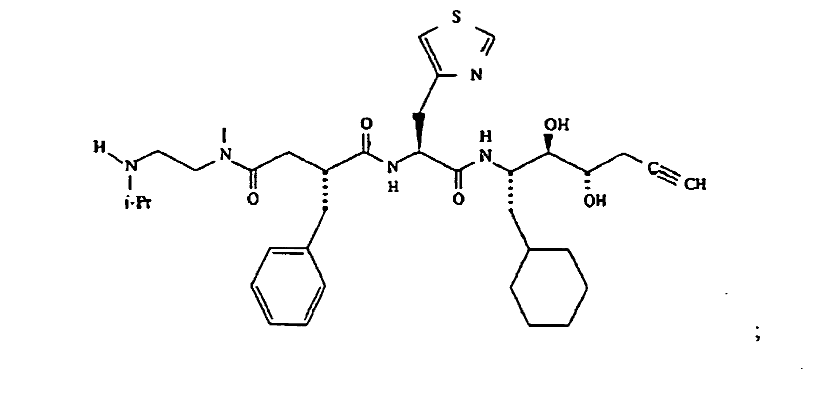 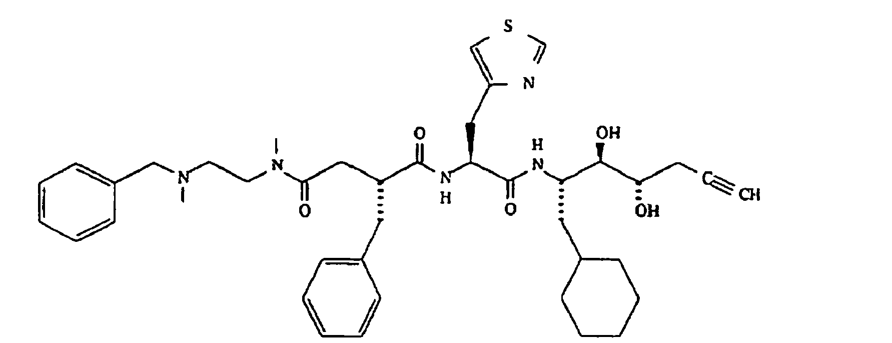 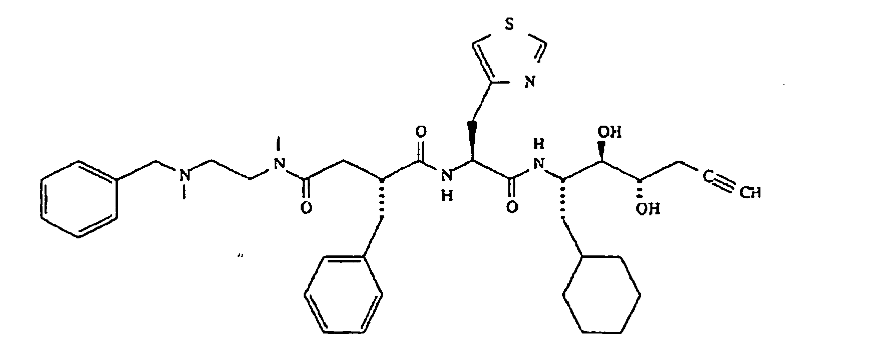 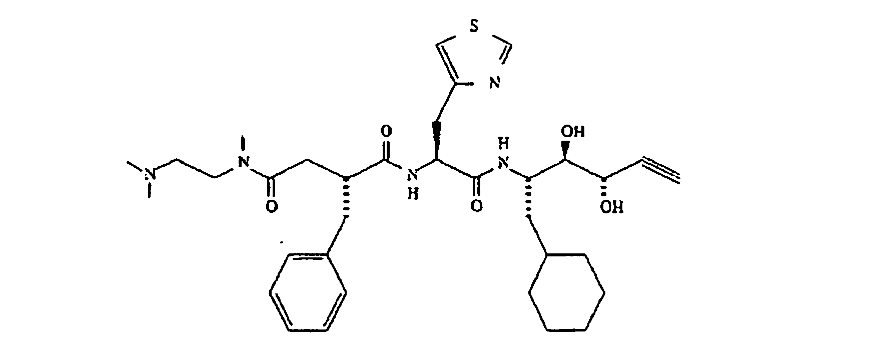 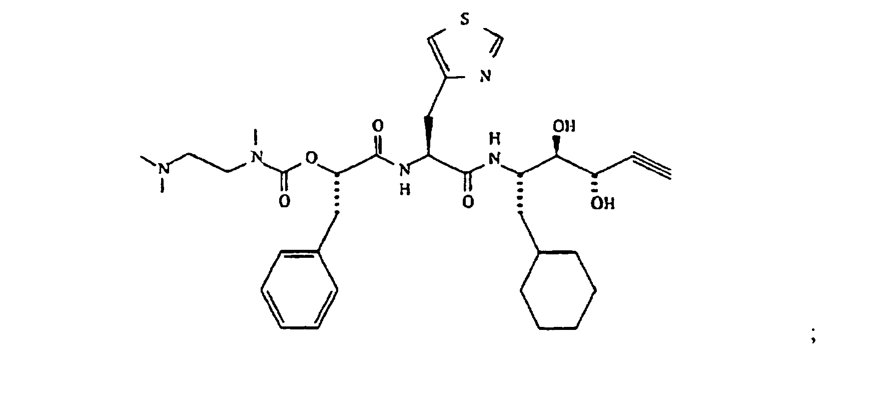 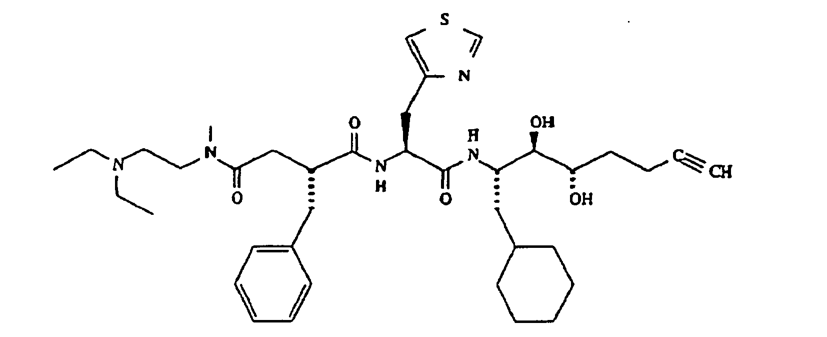 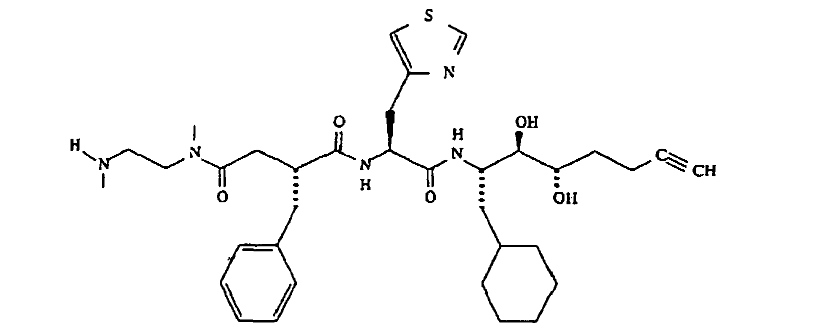 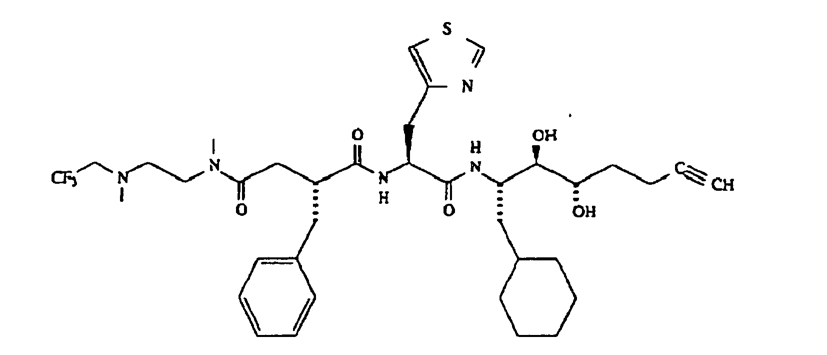 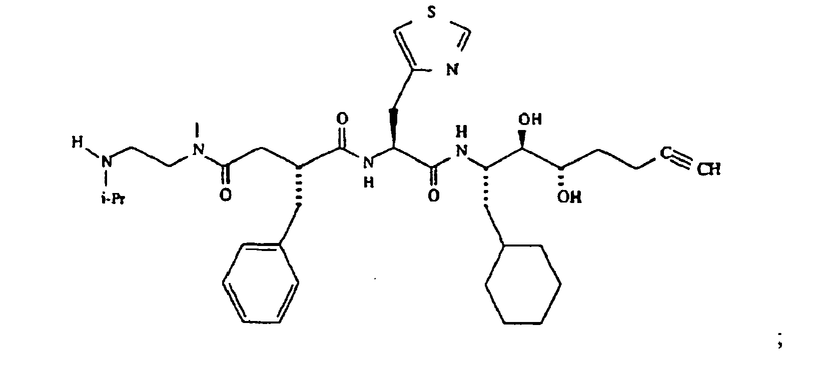 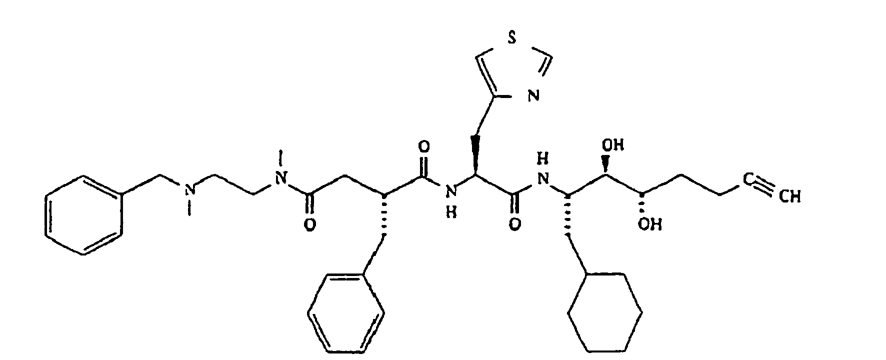 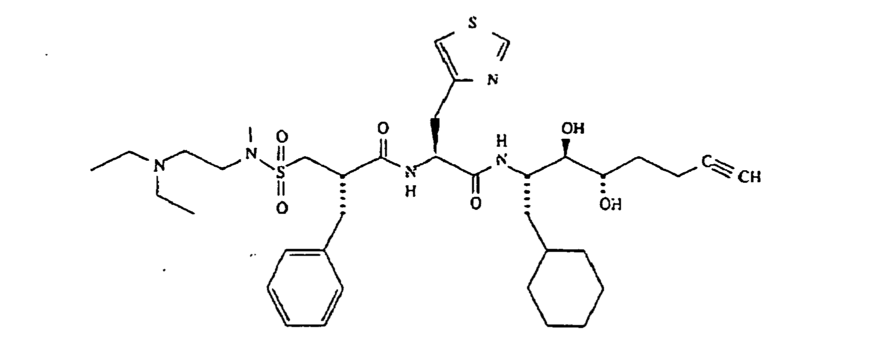 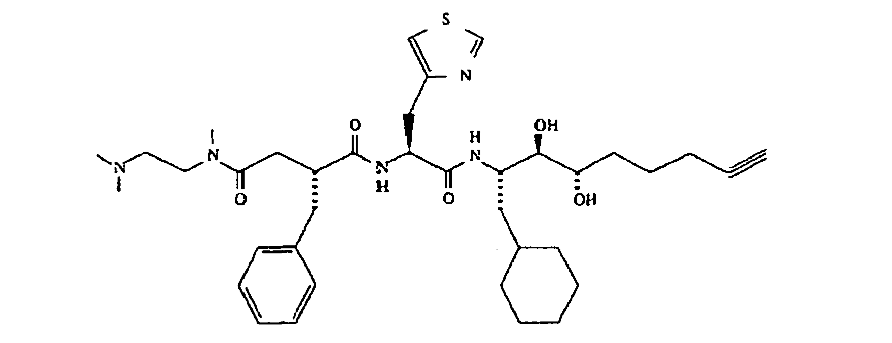 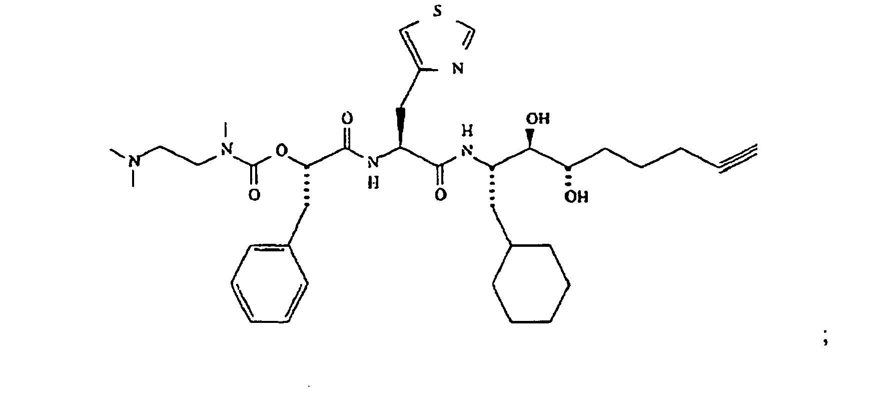 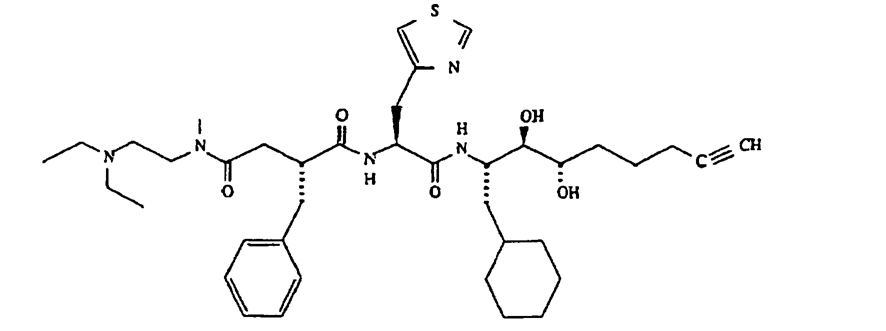 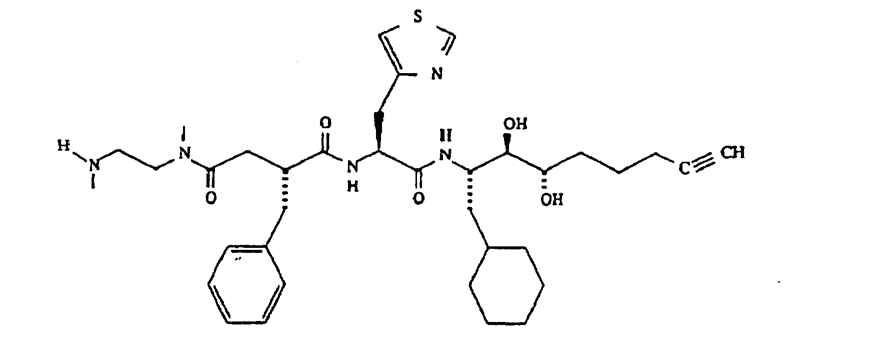 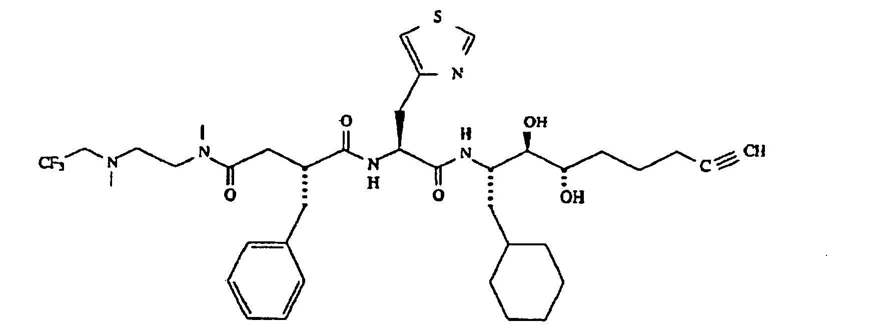 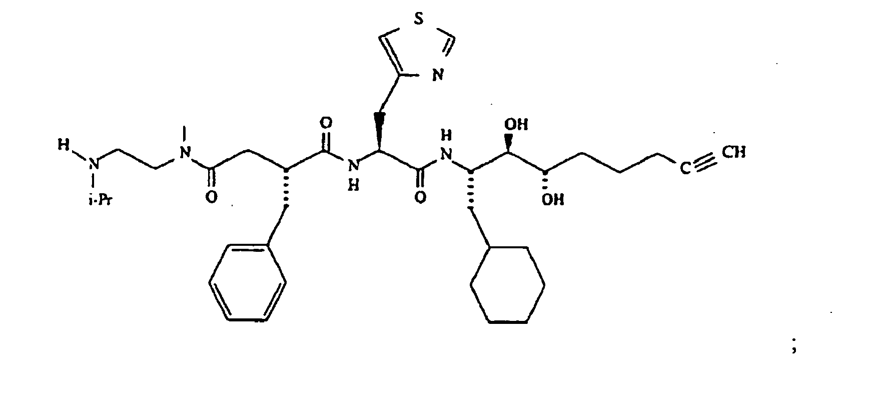 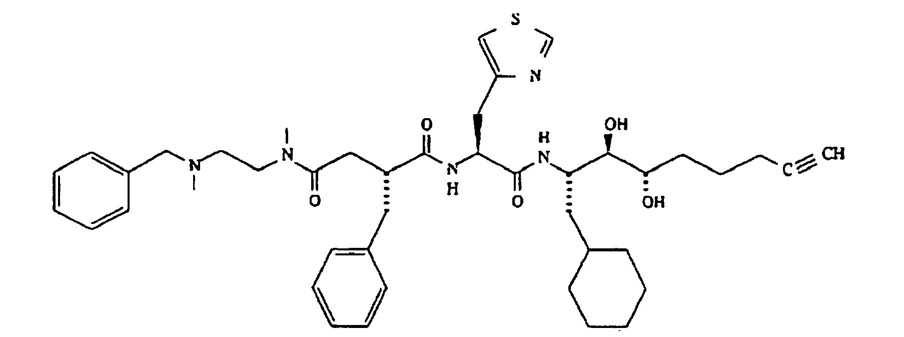 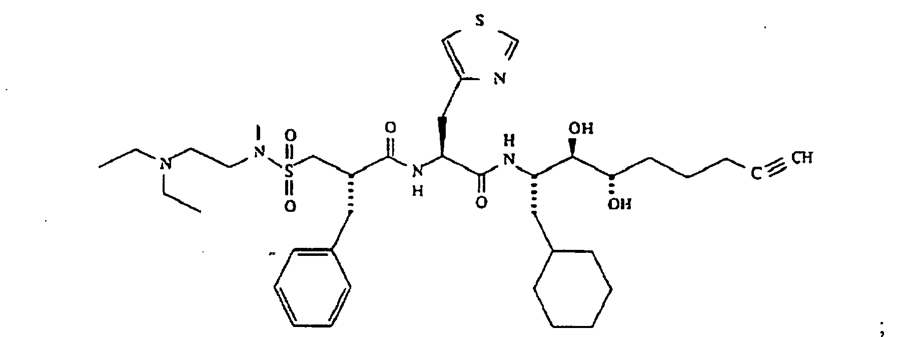 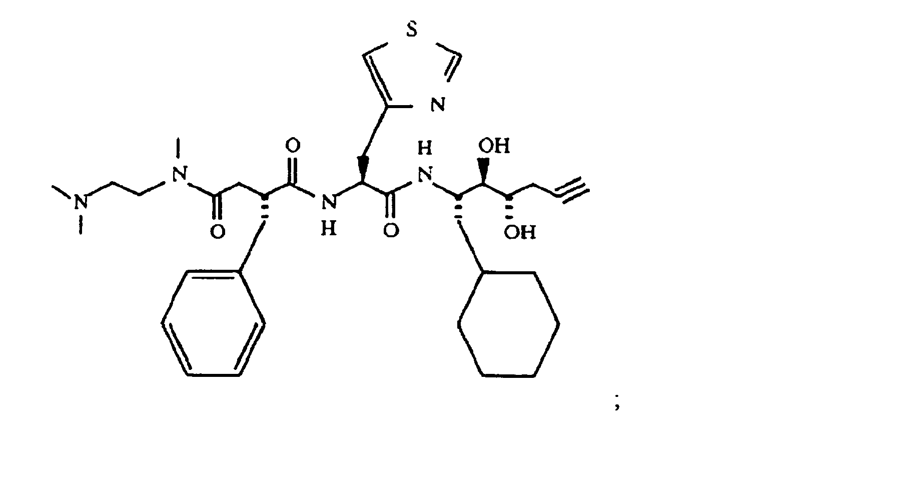 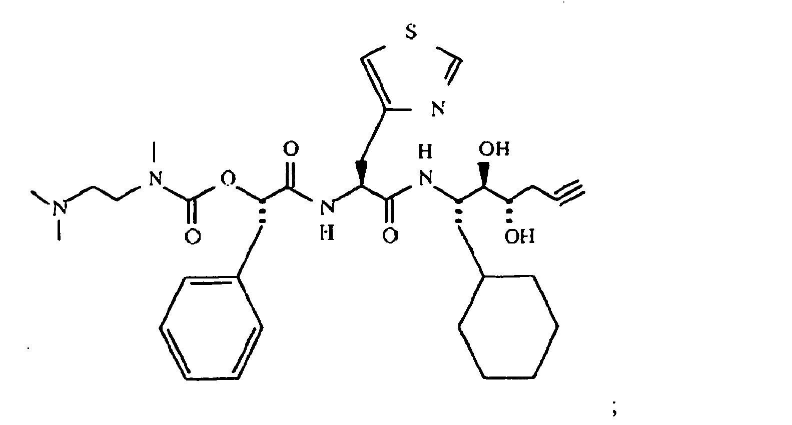 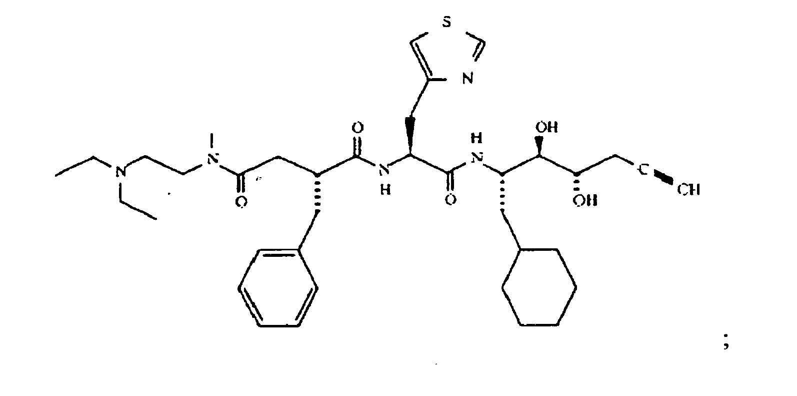 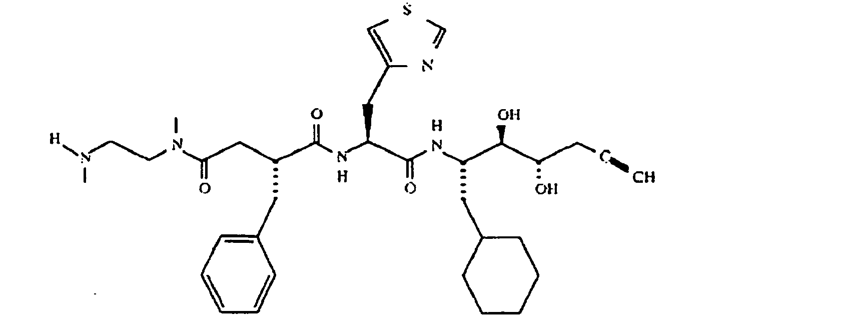 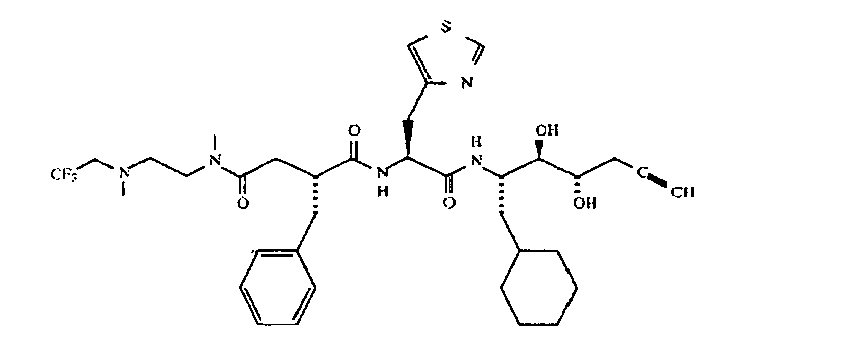 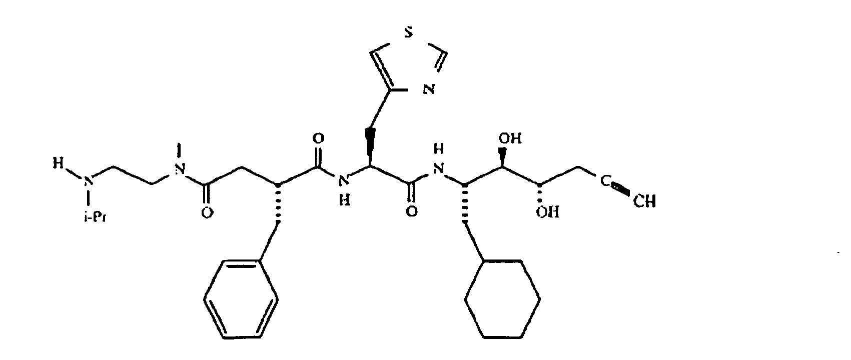 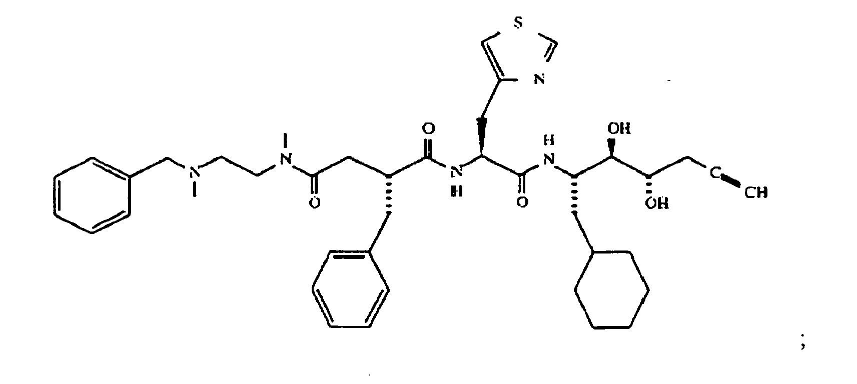 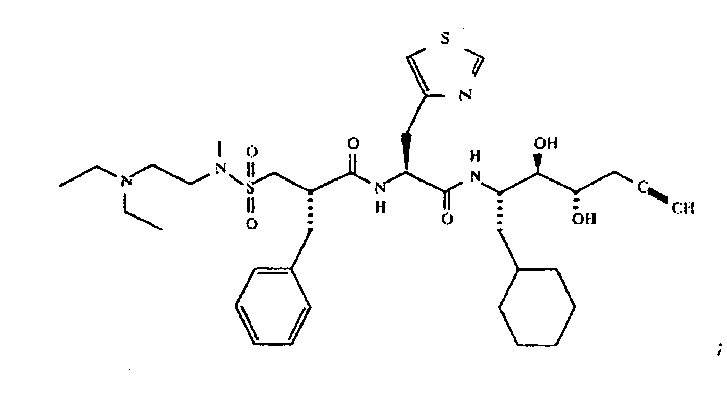 and 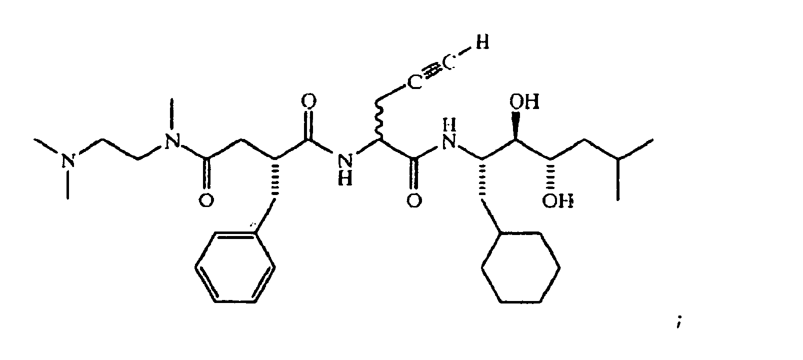
N-[3-[[1S,1R*-(cyclohexylmethyl)-2S*,3R*-dihydroxy-5-methylhexyl]amino]-2S*-methyl-3-oxopropyl]benzenebutanamide;
N-[3-[[1S,1R*-(cyclohexylmethyl)-2S*,3R*-dihydroxy-5-methylhexyl]amino]-2S*-methyl-3-oxopropyl]-1H-indole-2-carboxamide;
N-[3-[[1S,1R*-(cyclohexylmet:hyl)-2S*,3R*-dihydroxy-5-methylhexyl]amino]-2S*-methyl-3-oxopropyl]benzofuran-2-carboxamide;
N-3-[[1S,1R*-(cyclohexylmethyl)-2S*,3R*-dihydroxy-5-methylhexyl]amino]-2S*-methyl-3-oxopropyl]1H-indole-2-acetamide;
N-[3-[[1S,1R*-(cyclohexylmethyl)-2S*,3R*-dihydroxy-5-methylhexyl]amino-]-2S*-methyl-3-oxopropyl]-N-methyl-benzenebutanamide;
N-[3-[[1S,1R*-(cyclohexylmethyl)-2S*,3R*-dihydroxy-5-methylhexyl]amino]-2S*-methyl-3-oxopropyl]cyclohexanebutanamide;
N-[3-[[1S,1R*-(cyclohexylmethyl)-2S*,3R*-dihydroxy-S-methylhexyl]amino]-2S*-methyl-3-oxopropyl]-N-methyl-cyclohexanebutanamide;
N-[3-[[1S,1R*-(cyclohexylmethyl)-2S*,3R*-dihydroxy-5-methylhexyl]amino]-2S*-methyl-3-oxopropyl]-4-methoxybenzenepropanamide;
N-3-[[1S,1R*-(cyclohexylmethyl)-2S*,3R*-dihydroxy-5-methylhexyl]amino]-2S*-methyl-3-oxopropyl]-2-quinaldylamide;
N-[3-[[1S,1R*-(cyclohexylmethyl)-2S*,3R*-dihydroxy-5-methylhexyl]amino]-2S*-methyl-3-oxopropyl]-2-quinoxalinecarboxamide;
N-[3-[[1S,1R*-(cyclohexylmethyl)-2S*,3R*-dihydroxy-5-methylhexyl]amino] -2S*-methyl-3-oxopropyl]-nalidixylamide;
N-[3-[(1S,1R*-(cyclohexylmethyl)-2S*,3R*-dihydroxy-5-methylhexyl]amino] -2S*-methyl-3-oxopropyl]-N-nalidixylamide;
N-[3-[[1S,1R*-(cyclohexylmethyl)-2S*,3R*-dihydroxy-5-methylhexyl]amino]-2S*-methyl-3-oxopropyl]-(R,S)-α-methyl-hydrocinnamide;
N-[3-[[1S,1R*-(cyclohexylmethyl)-2S*,3R*-dihydroxy-5-methylhexyl]amino]-2S*-methyl-3-oxopropyl]N-methyl-1H-indole-2-carboxamide;
N-[3-[[1S,1R*-(cyclohexylmethyl)-2S*,3R*-dihydroxy-5-methylhexyl]amino]-2S*-methyl-3-oxopropyl]-5-fluoro-1H-indole-3-carboxamide;
N- [3- [[1S,1R*-(cyclohexylmethyl)-2S*,3R*-dihydroxy-5-methylhexyl]aminol-2S*-methyl-3-oxopropyl]-5-fluoro-1H-indole-2-carboxamide;
N-[3-[[1S,1R*-(cyclohexylmethyl)-2S*,3R*-dihydroxy-5-methylhexyl]amino]-2S*-methyl-3-oxopropyl]-1H-indole-3-carboxamide;
N- [3- [[1S,1R*-(cyclohexylmethyl)-2S*,3R*-dihydroxy-5-methylhexyl]amino]-2S*-methyl-3-oxopropyl]-1H-indole-3-acetamide;
N-[3-[[1S,1R*-(cyclohexylmethyl)-2S*,3R*-dihydroxy-5-methylhexyl]amino]-2S*-methyl-3-oxopropyl]-1H-indole-3-propanamide;
N-[3-[[1S,1R*-(cyclohexylmethyl)-2S*,3R*-dihydroxy-5-methylhexyl]amino]-2S*-methyl-3-oxopropyl]-7-methoxy-benzofuran-2-carboxamide;
N-[3-[[1S,1R*-(cyclohexylmethyl)-2S*,3R*-dihydroxy-5-methyhexyl]amino]-2S*-methyl-3-oxopropyl]-4-oxo-4H-1-benzopyran-3-carboxamide;
N-[3-[[1S,1R*-(cyclohexylmethyl)-2S*,3R*-dihydroxy-5-methylhexyl]amino]-2S*-methyl-3-oxopropyl]-4-oxo-4H-1-benzopyran-2-carboxamide; and
N-[3-[[1S,1R*-(cyclohexylmethyl)-2S*,3R*-dihydroxy-5-methylhexyl]amino]-2S*-methyl-3-oxopropyl]-7-chloro-benzofuran-2-carboxamide;
(3S,4S)-N-[(tert-Butyloxy)carbonyl]-4-amino-3-acetoxy-5-phenylpentene;
(2R,3S)-N-[(tert-Butyloxy)carbonyl]-3-amino-2-acetoxy-4-phenylbutanal;
(2S,3R,4S)-N-[(tert-Butyloxy)carbonyl]-2-amino-1-phenyl-3,4-dihydroxy-6-methylheptane;
(2S,3R,4S)-N-[(tert-Butyloxy)carbonyl]-2-amino-1-cyclohexyl-3,4-dihydroxy-6-methylheptane;
(2S.3R)-Boc-L-Leucinamide of 2-amino-l-cyclohexyl-3,4-dihydroxybutane; .
D,L-Monomethyl-2-(1-naphthylmethyl)succinate;
D,L-Methyl-3-(N-morpholinocarbonyl)-2-(1-naphthylmethyl) propionate;
D,L-3-(N-Morpholinocarbonyl)-2-(1-naphthylmethyl)propionic acid; and
3-(N-morpholinocarbonyl)-2-(R,S)-(1-naphthylmethyl)propionyl-L-leucinamide of (2S,3R)-2-amino-1-cyclohexyl-3,4-dihydroxybutane;
N-[1R*-[[[1S,1R*-(cyclohexylmethyl)-2S*,3R*-dihydroxy-5-methylhexyl]amino]carbonyl]-3-butynyl]-αR*-[[[2-(dimethylamino)ethyl]sulfonyl]methyl]benzenepropanamide;
N-[2-[[1S,1R^{*}-(cyclohexylmethyl)-2S^{*},3R^{*} -dihydroxy-5-methylhexyl]amino]-1R^{*}-(cyclopropylmethyl)-2-oxoethyl]-α-[[[2-(dimethylamino)ethyl]sulfonyl]methyl] benzenepropanamide;
N-[2-[[1S,1R*-(cyclohexylmethyl)-2S*,3R*-dihydroxy-5-methylhexyl]amino]-1R * -(cycloproylmethyl)-2-oxoethyl]-α-[[[2-(dimethylamino)ethyl]thio]methyl]benzenepropanamide;
N-[2-[[1S,1R*-(cyclohexylmethyl)-2S*,3R*-dihydroxy-5-methylhexyl]amino]-1R * -(cyclopropylmethyl)-2-oxoethyl]-a-[[[2-(diethylamino)ethyl]sulfonyl] methyl]benzenepropanamide; 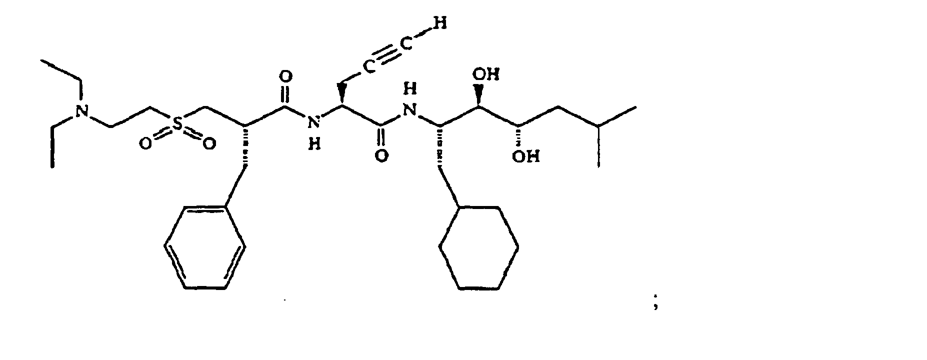 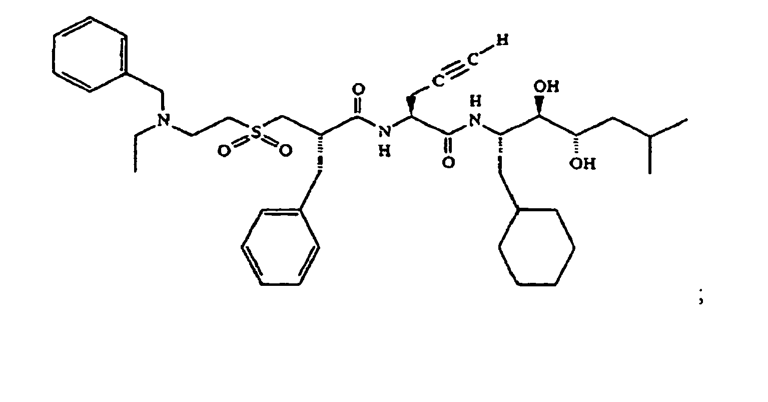 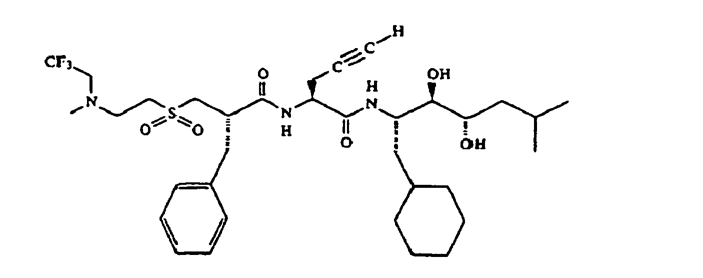 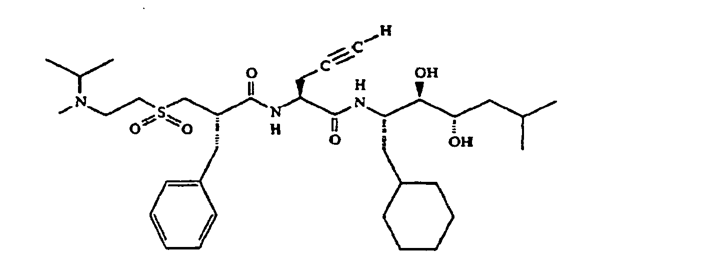 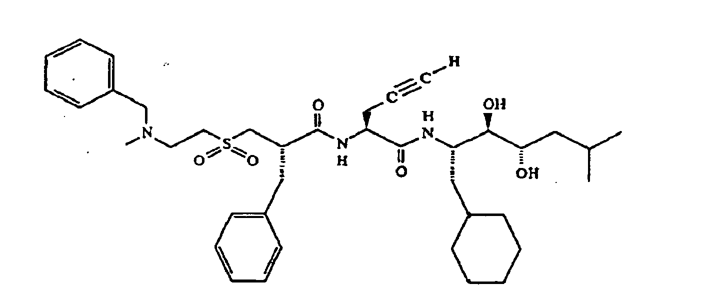 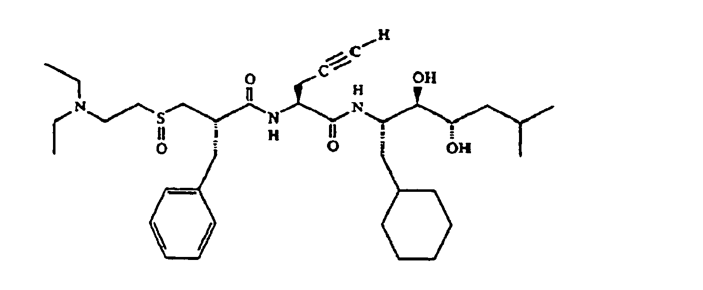 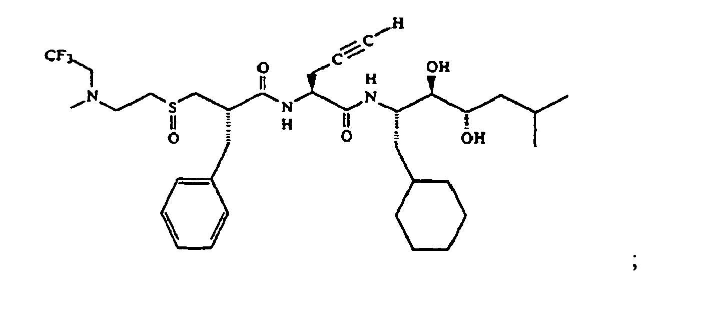 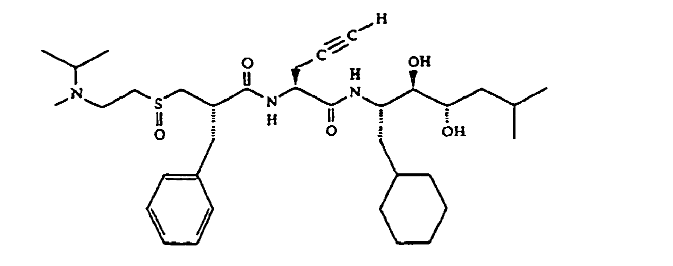 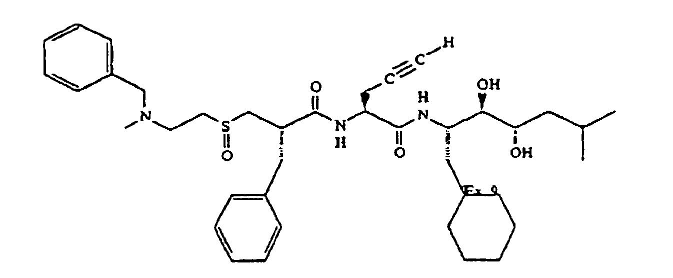 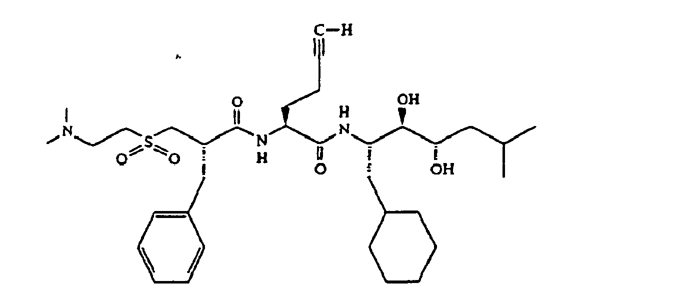 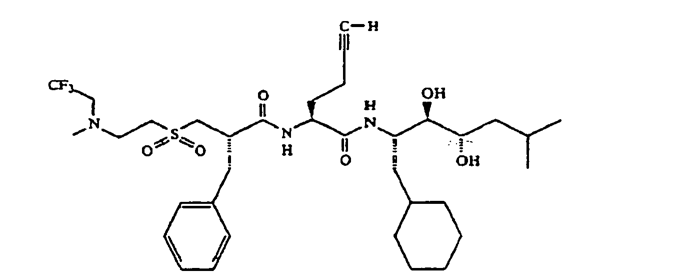 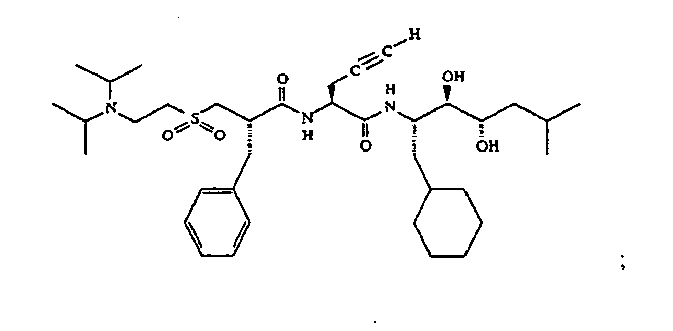 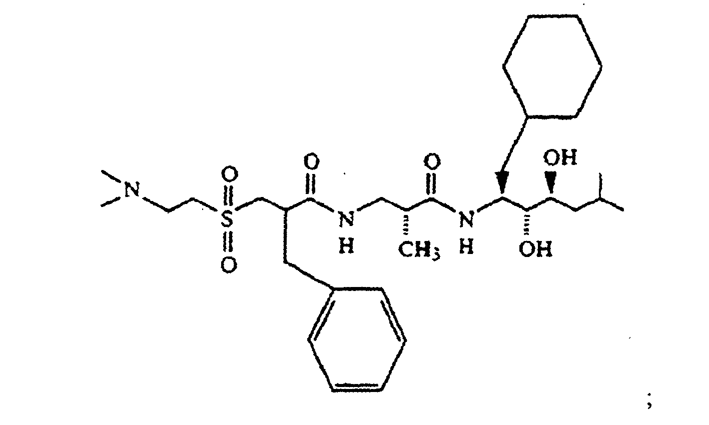 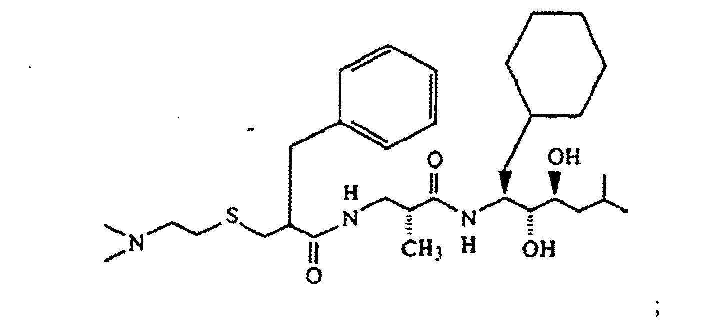 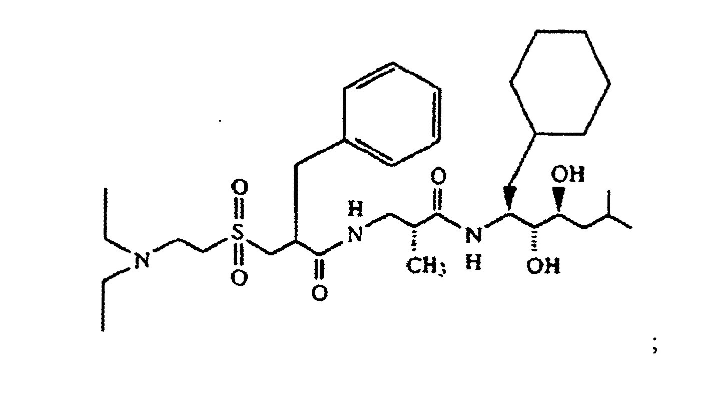 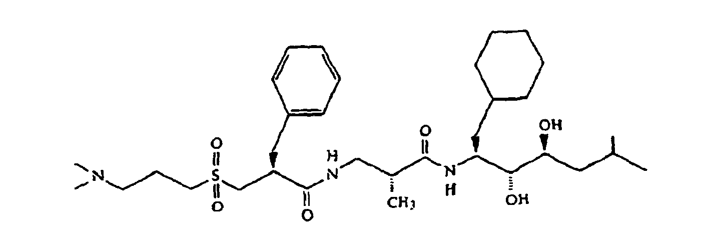 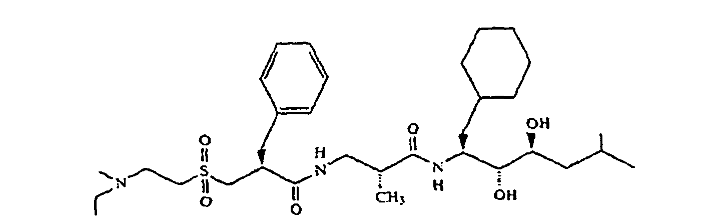 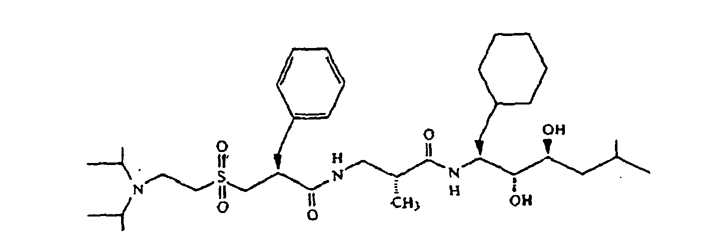 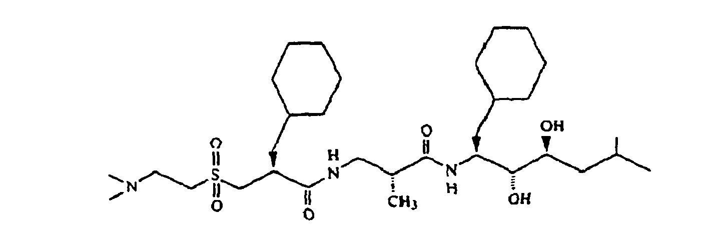 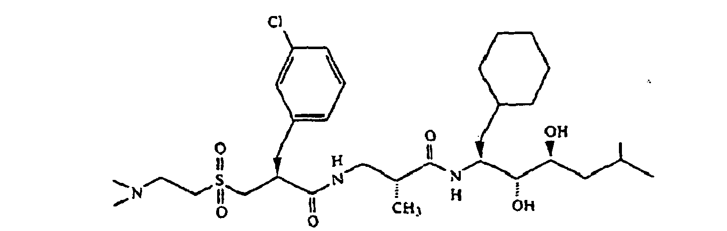 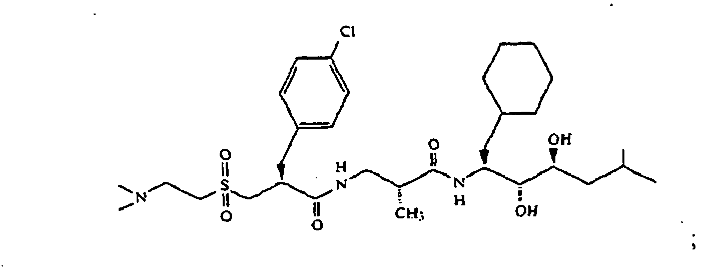 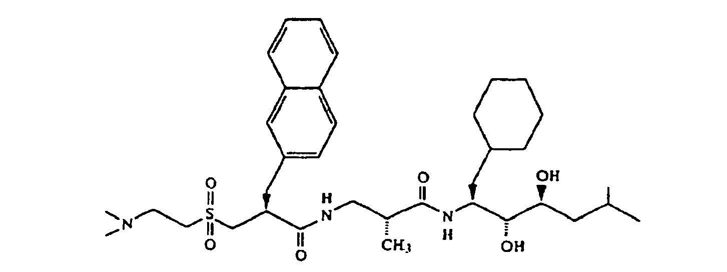 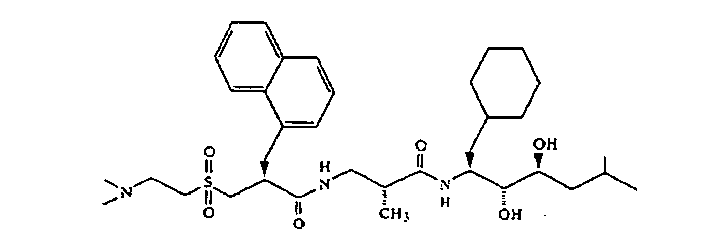 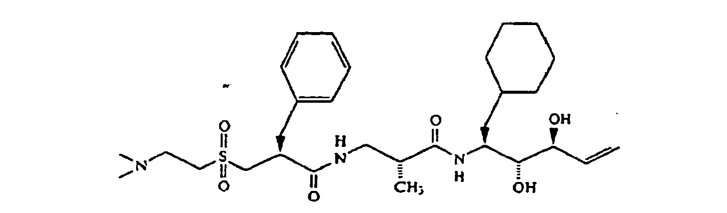 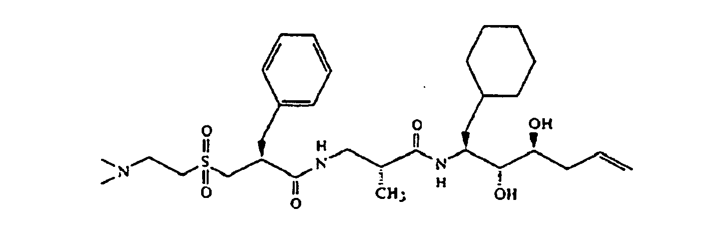 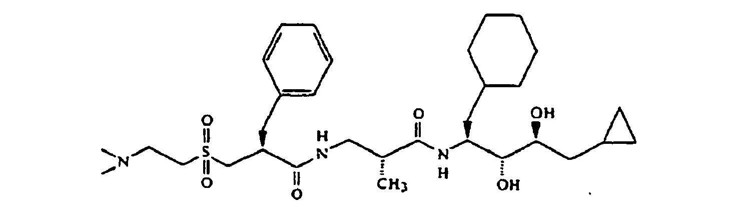 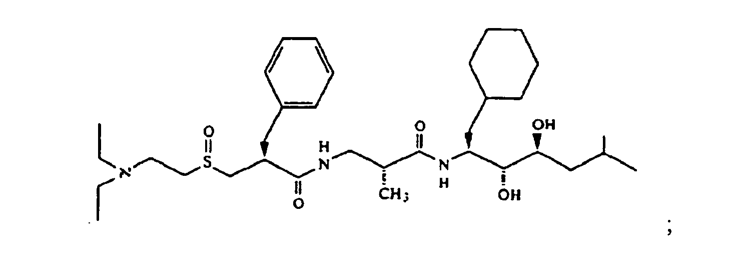 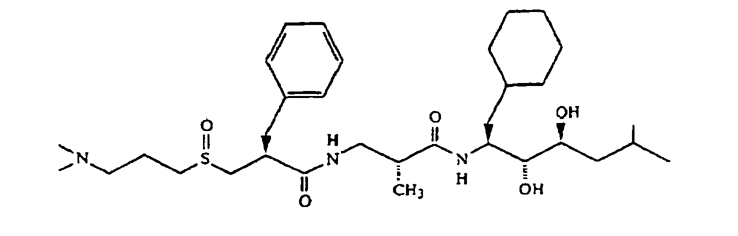 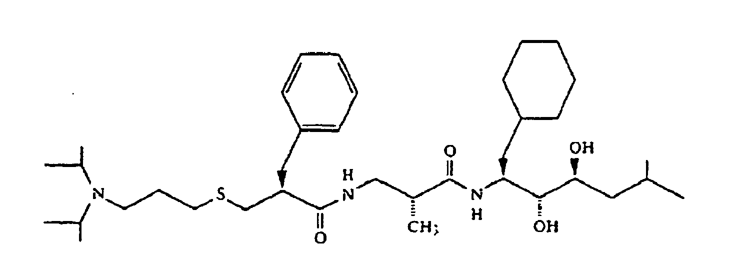 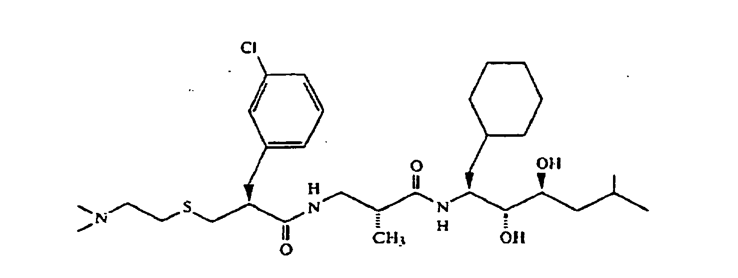 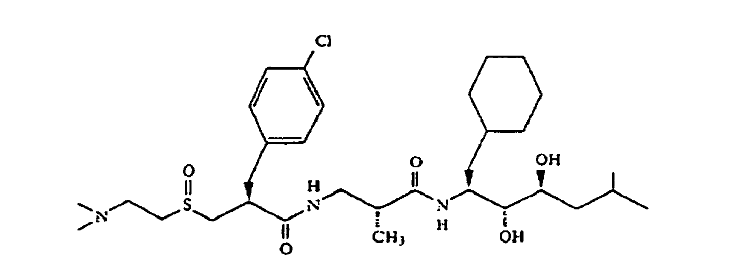 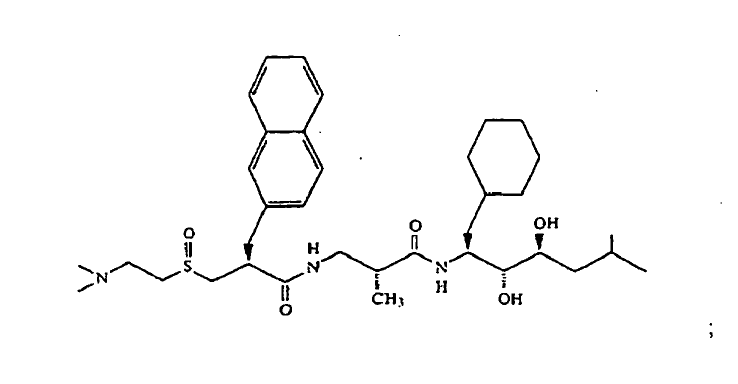 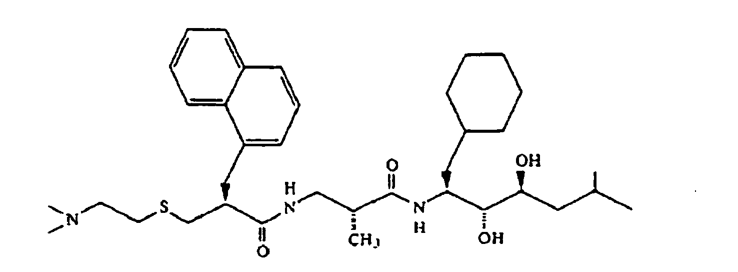 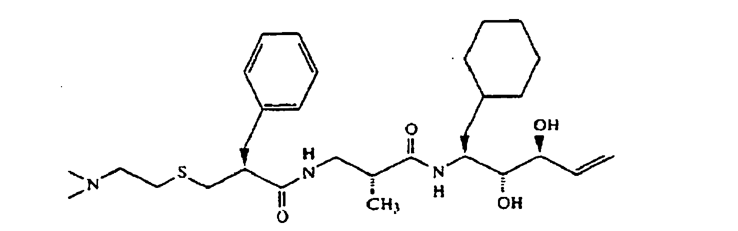 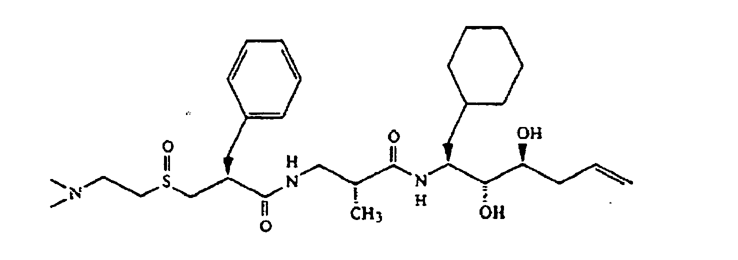 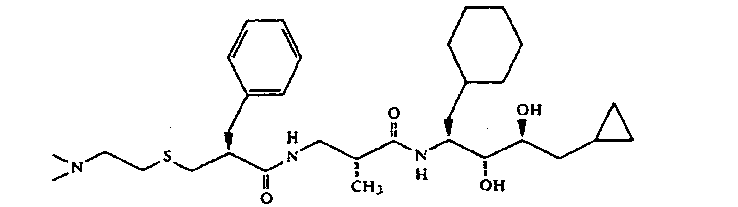 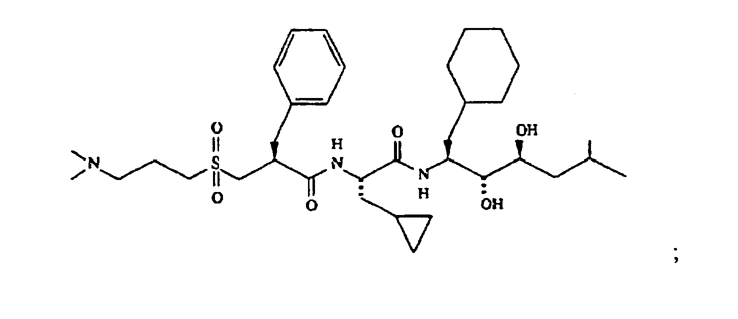 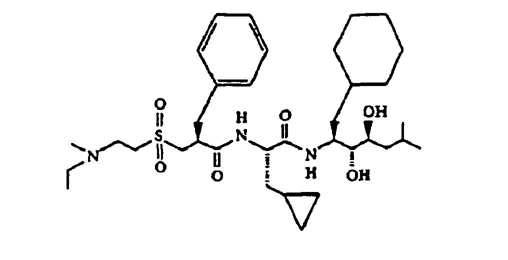 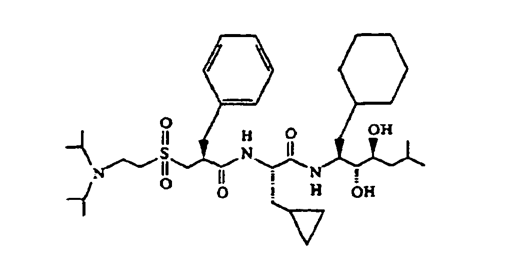 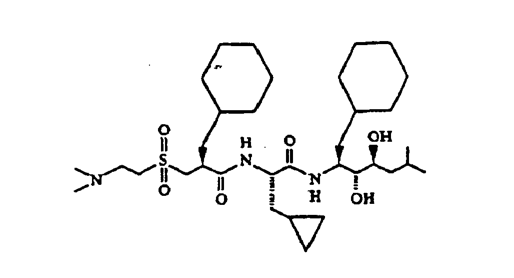 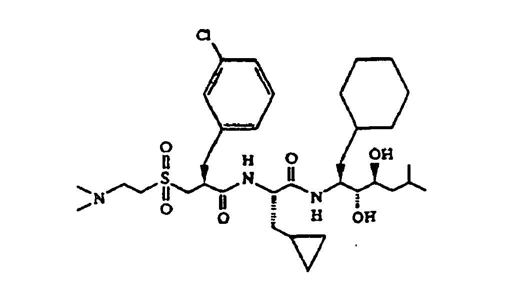 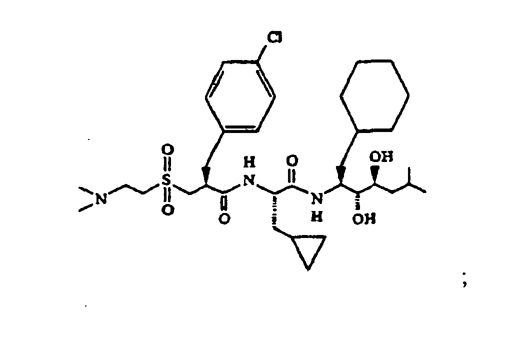 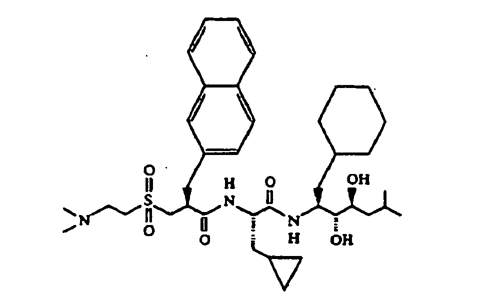 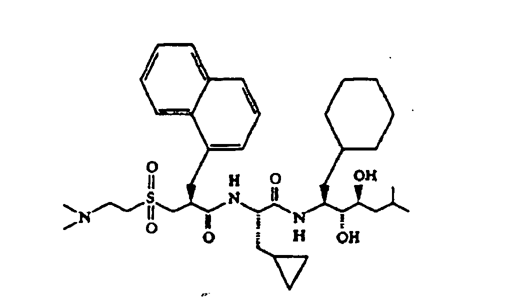 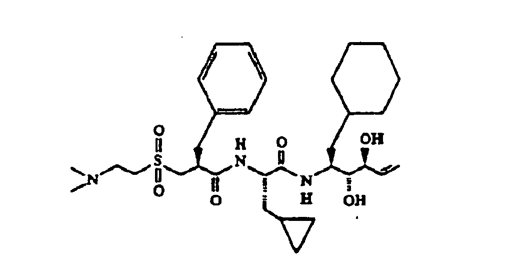 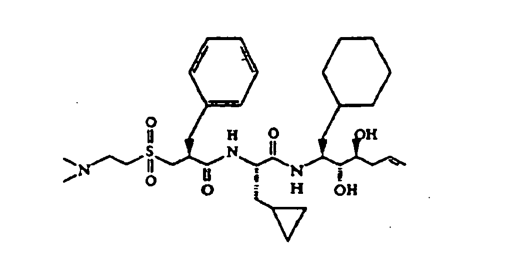 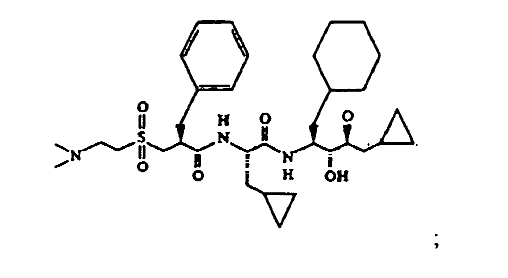 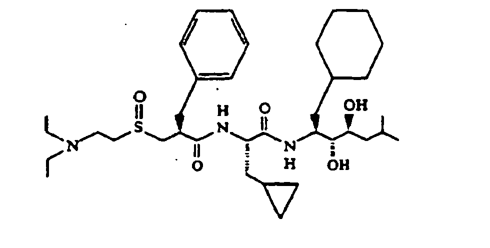 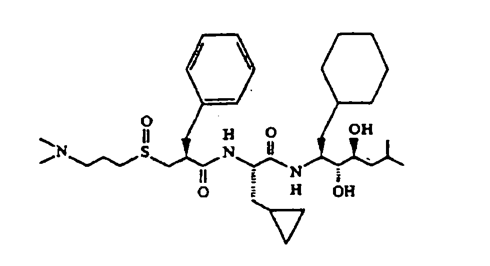 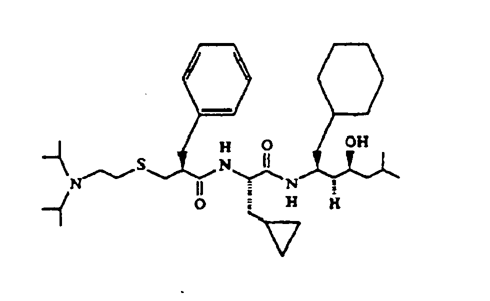 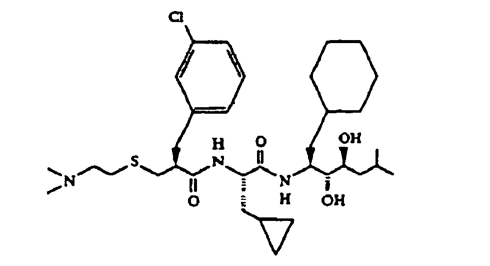 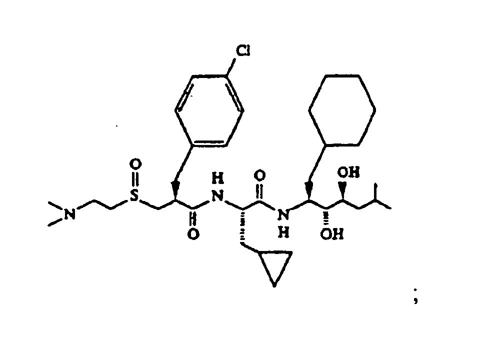 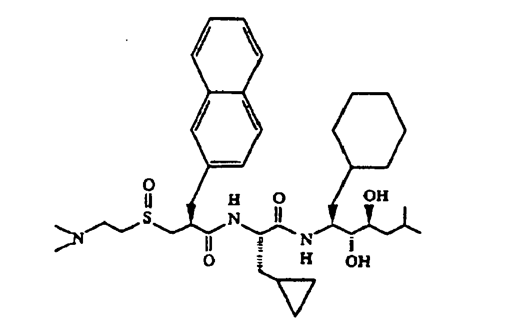 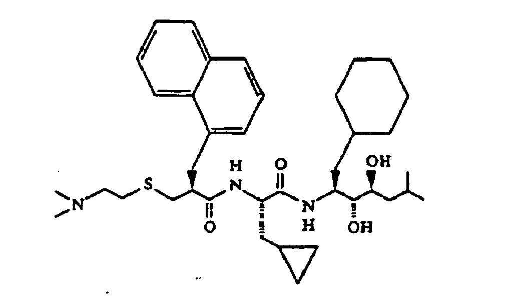 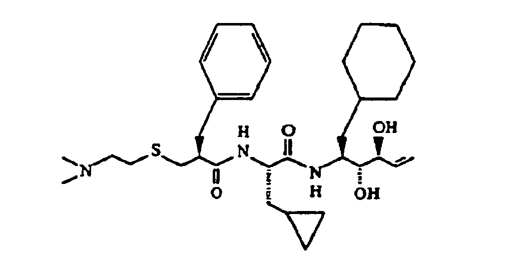 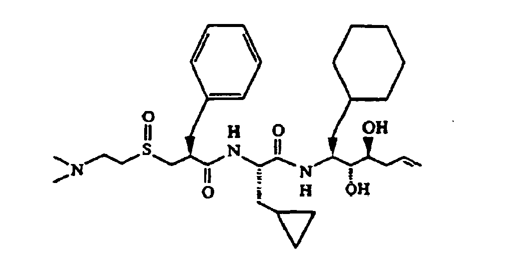 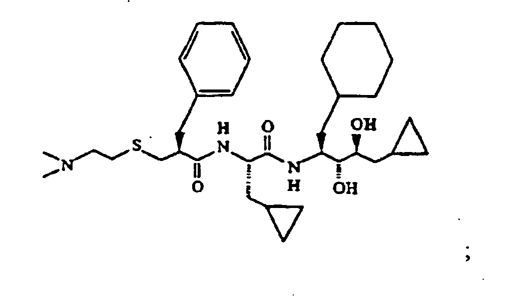 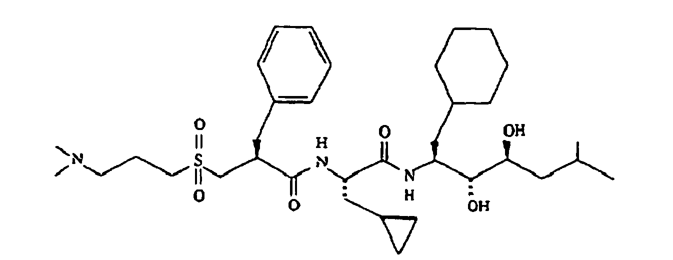 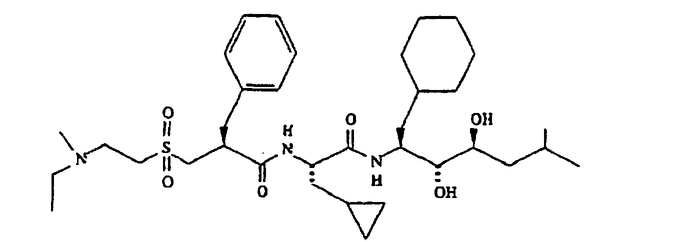 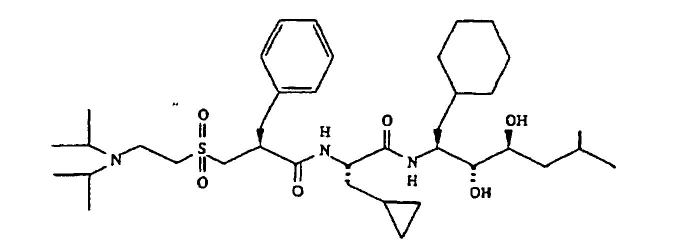 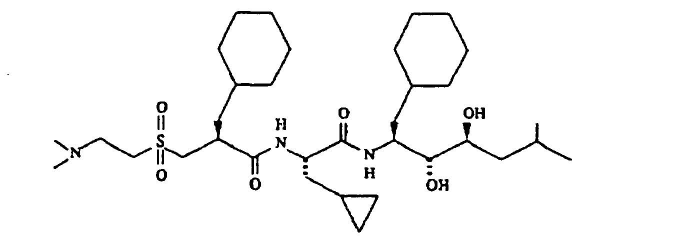 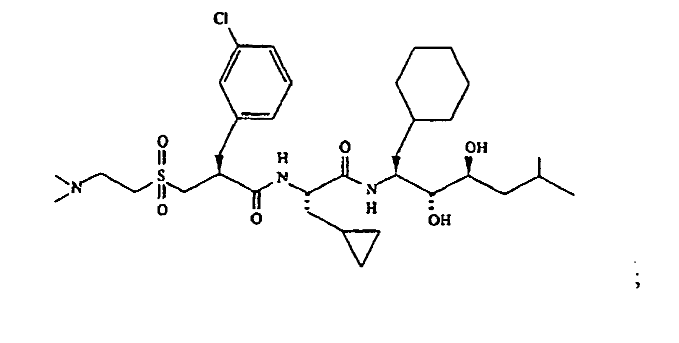 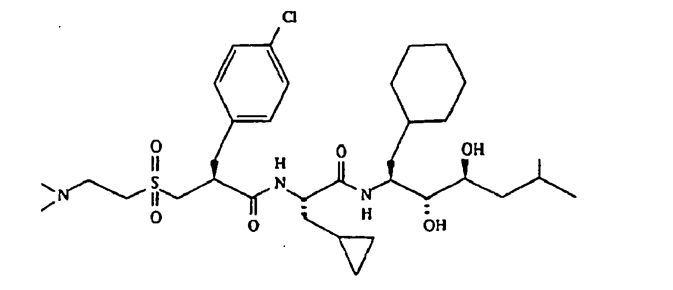 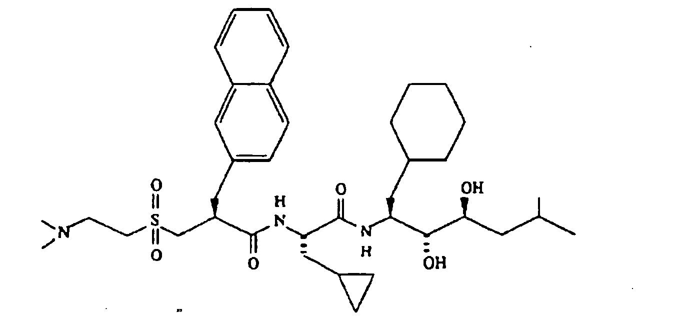 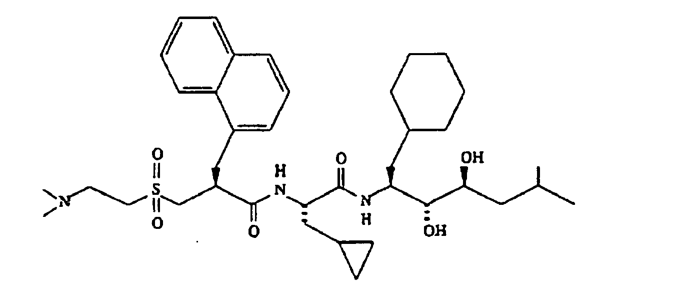 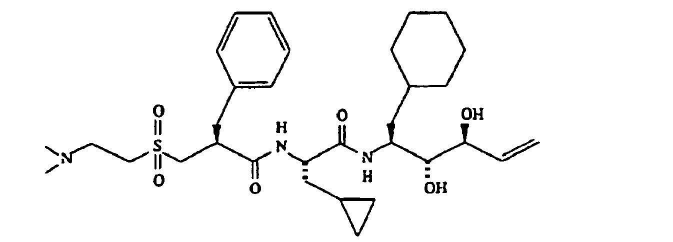 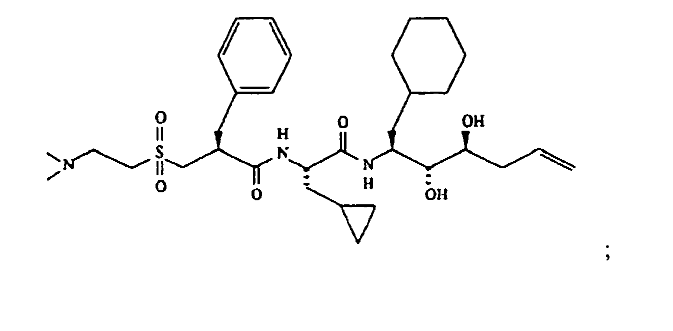 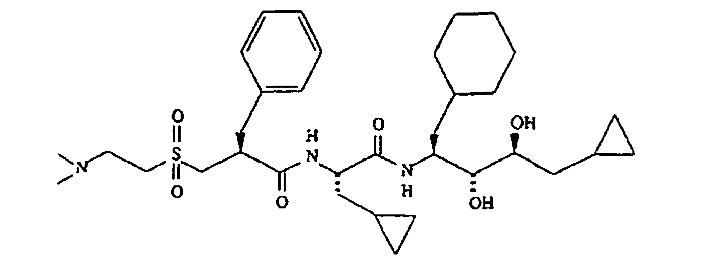 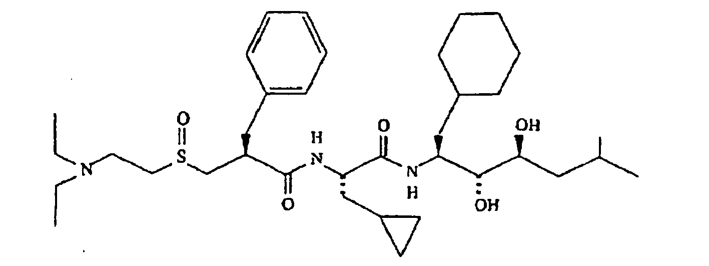 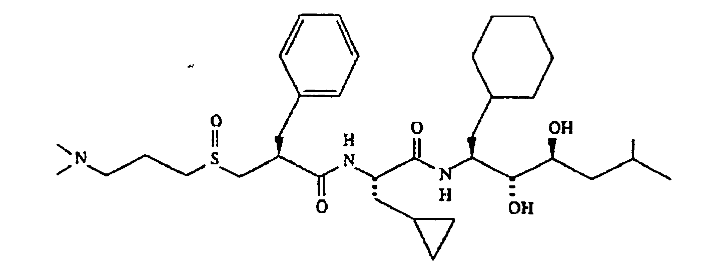 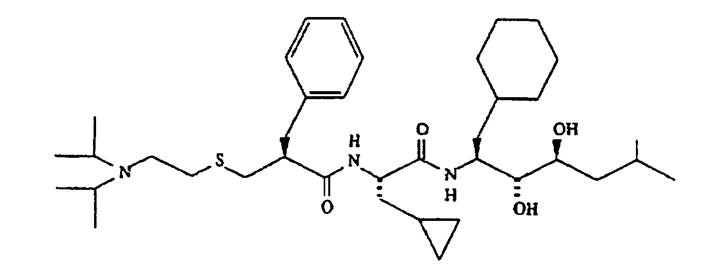 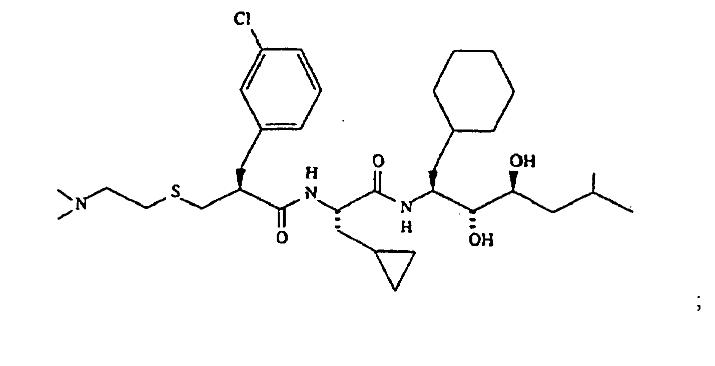 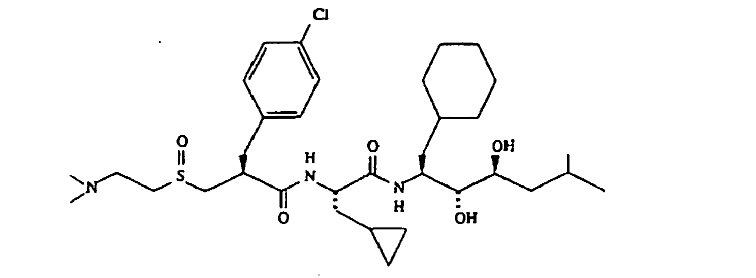 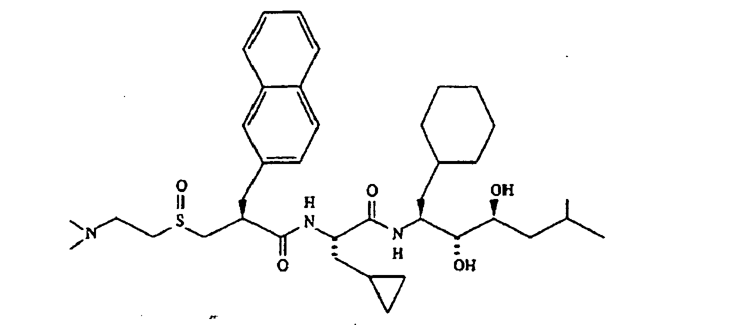 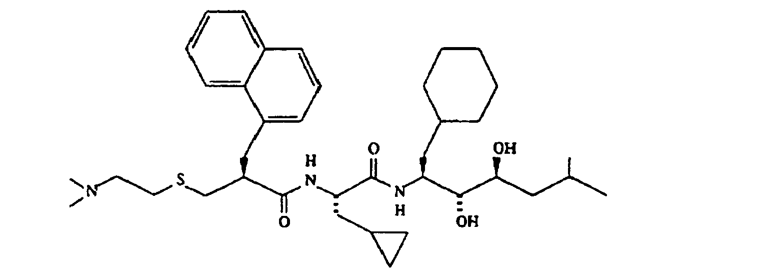 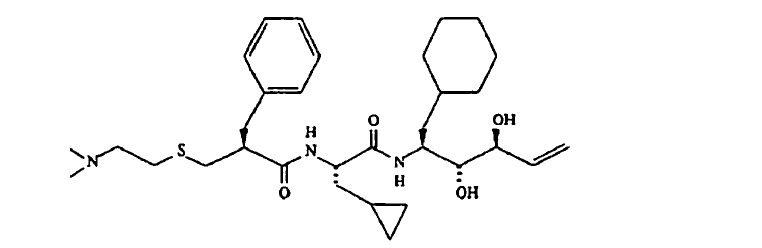 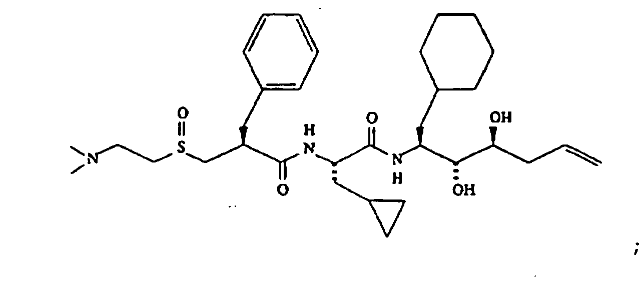 and 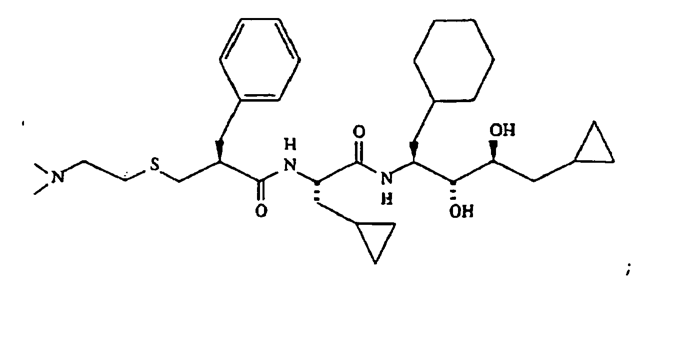
N¹-[1R*-[[[1S,1R*-(cyclohexylmethyl)-2S*,3R*-dihydroxy-5-methylhexyl]amino]carbonyl]-3-butynyl]-N⁴⁻methyl-2S*- (phenylmethyl) -N⁴- [2- (1-piperidinyl)ethyl]butanediamide;
N¹-[1R*-[[[1S,1R*-(cyclohexylmethyl)-2S*,3R*-dihydroxy-5-methylhexyl] amino] carbonyl] -3-butynyl] -N9- [2-(1,3-dihydro-2H-isoindol-2-yl)ethyl]-N⁴-methyl-2S*-(phenylmethyl)butanediamide;
N¹-[1R*-[[[1S,1R*-(cyclohexylmethyl)-2S*,3R*-dihydroxy-5-methylhexyl]amino]carbonyl]-3-butynyl]-N⁴⁻methyl-2S*-(phenylmethyl)-N⁴-[2-(N-3-azabicyclo[3.2.2]nonanyl)ethyl]butanediamide;
(2S)-2-Benzyl-3-(1-methylpiperazin-4-ylsulfonyl)propionyl-L-propargylglycyl amide of (2S,3R,4S)-2-amino-1-cyclohexyl-3,4-dihydroxy-6-methylheptane;
N¹-[1R*-[[[1S,1R*-(cyclohexylmethyl)-2S*,3R*-dihydroxy-5-methylhexyl]amino]carbonyl]-3-butynyl]-N⁴⁻methyl-N⁴- [2-(4-morpholinyl)ethyl]-2S*-(phenylmethyl)butanediamide;
N¹- [1R*-[[[1S, 1R*-(cyclohexylmethyl)-2S*, 3R*-dihydroxy-5-methylhexyl]amino]carbonyl]-3-butynyl]-N⁴-[2-(1H-imidazol-1-yl)ethyl]-N⁴-methyl-2S*-(phenylmethyl)butanediamide;
N¹-[1R*-[[[1S,1R*-(cyclohexylmethyl)-2S*, 3R*-dihydroxy-5-methylhexyl]amino]carbonyl]-3-butynyl]-N⁴⁻methyl-2S*-(phenylmethyl)-N⁴-[2-(2-pyridinyl) ethyl]butanediamide; 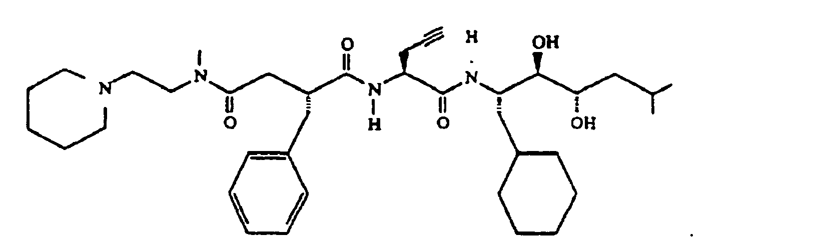 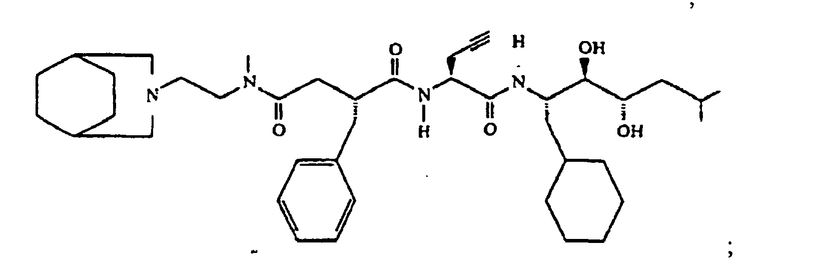 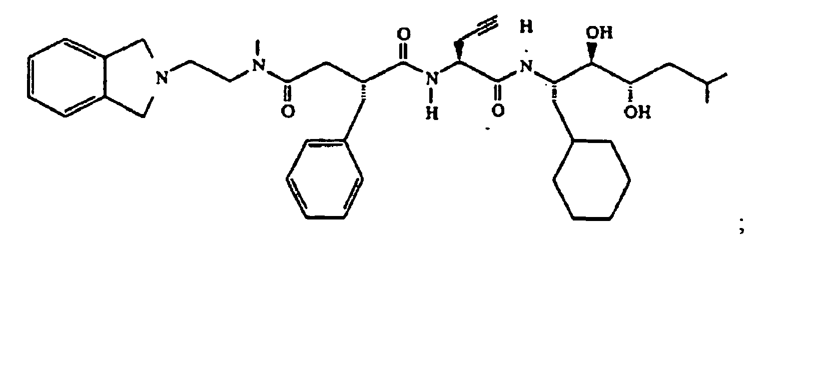 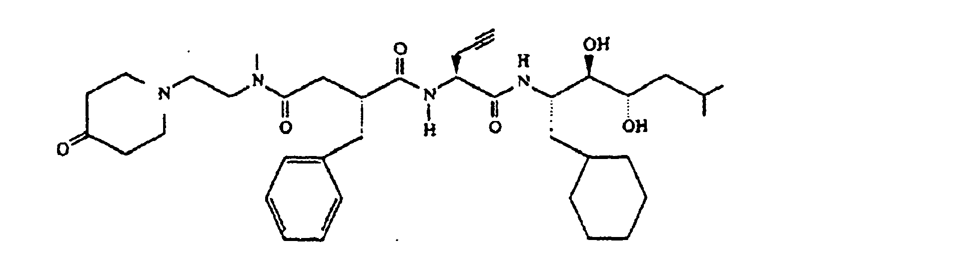 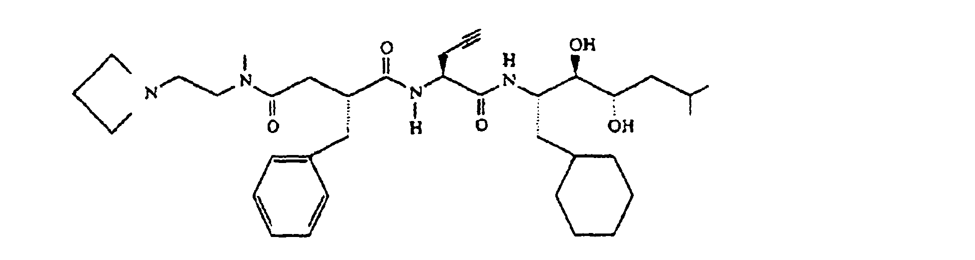 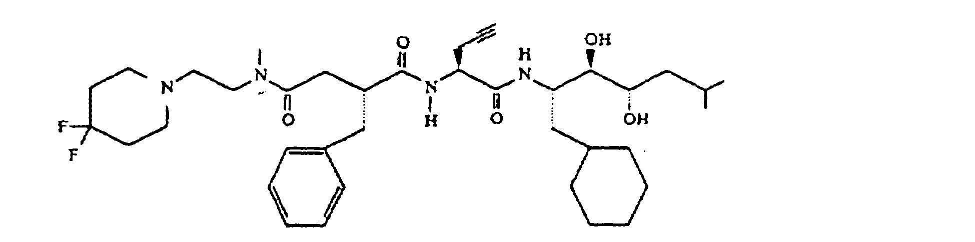 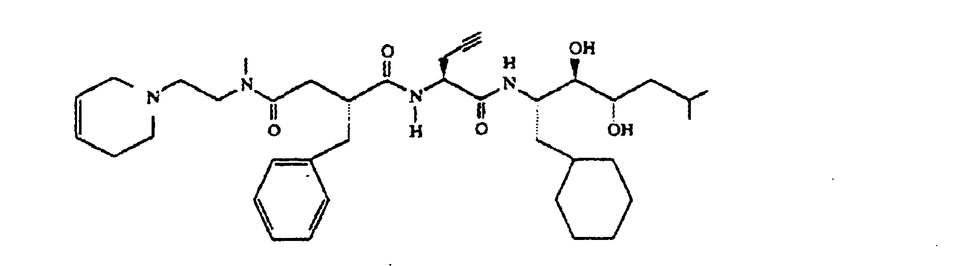 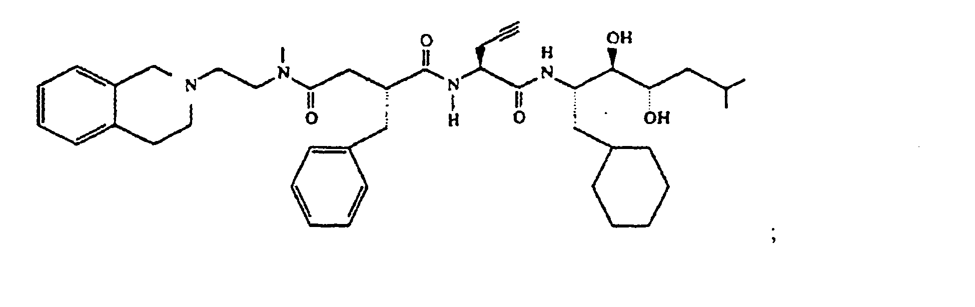 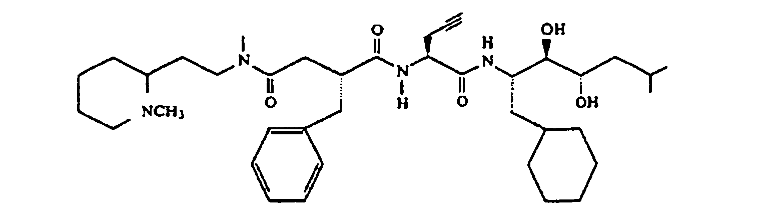 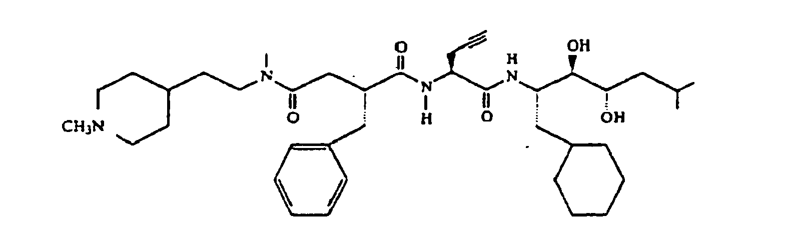 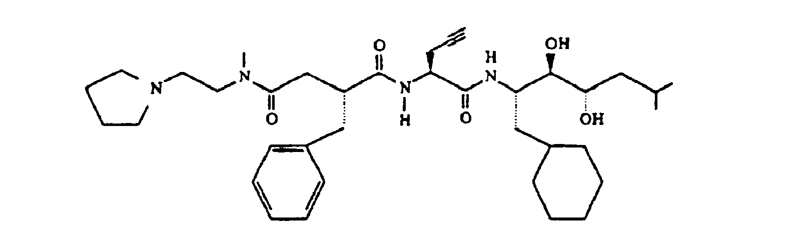 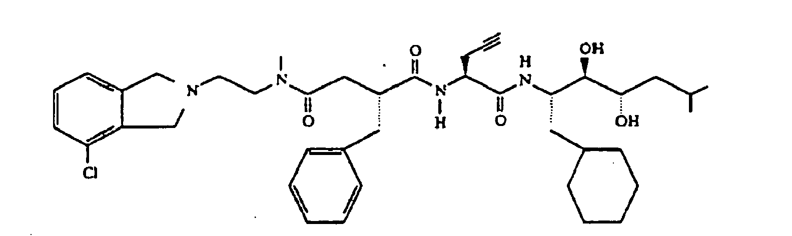 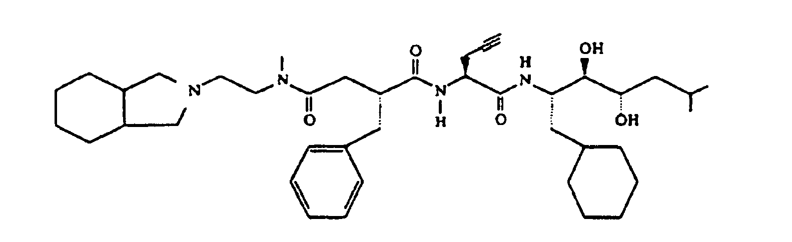 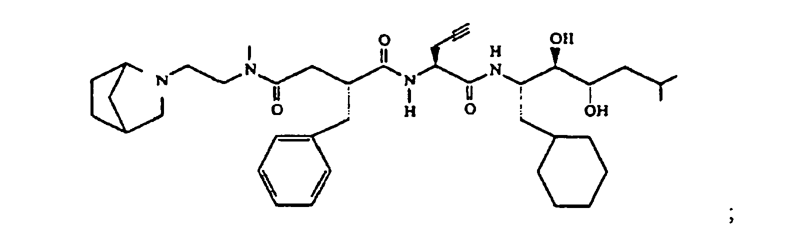 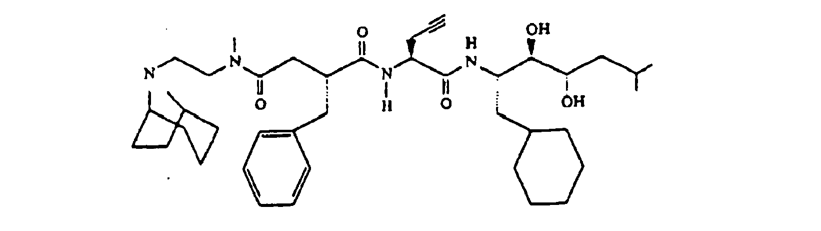 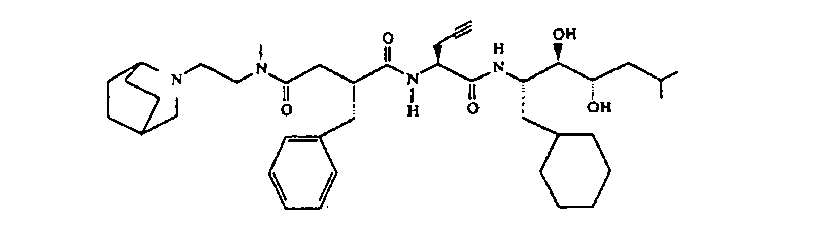 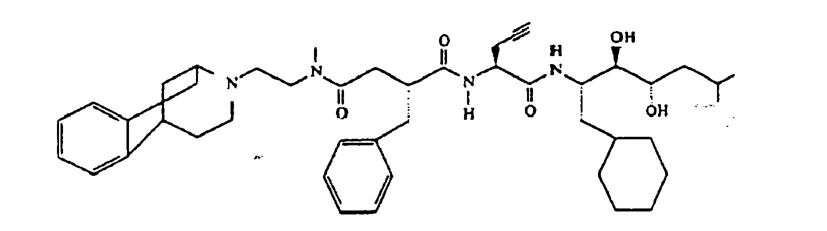 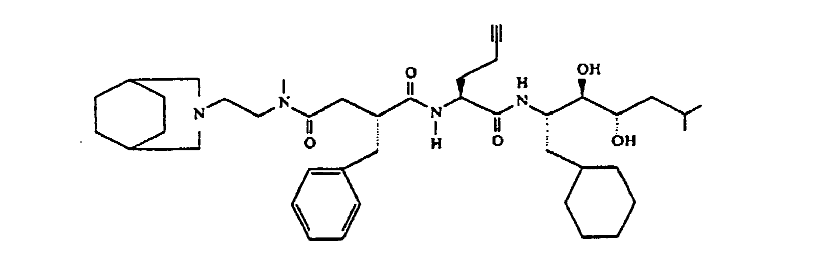 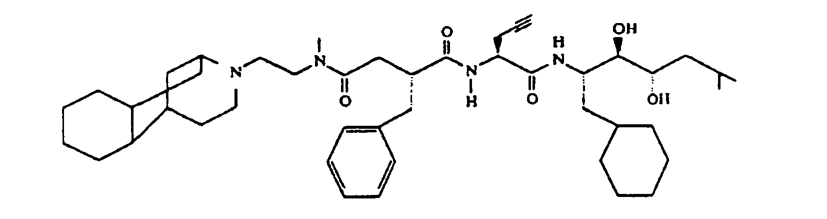 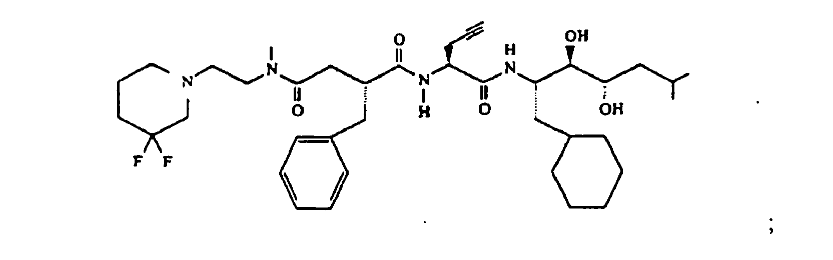 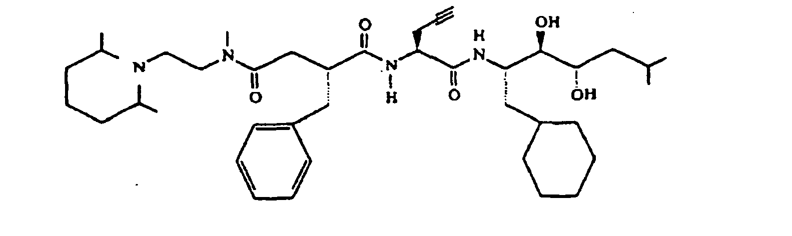 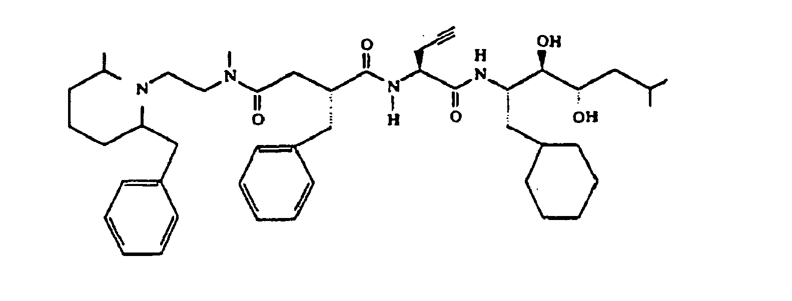 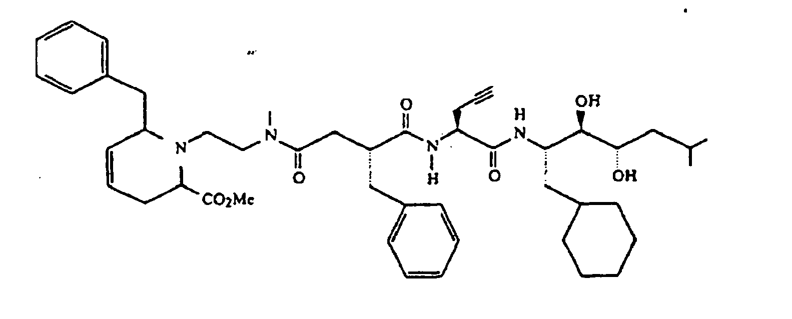 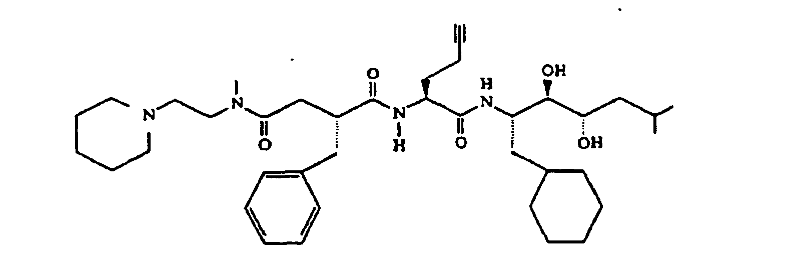 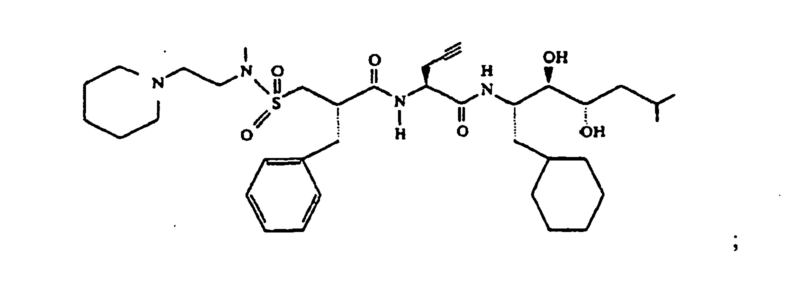 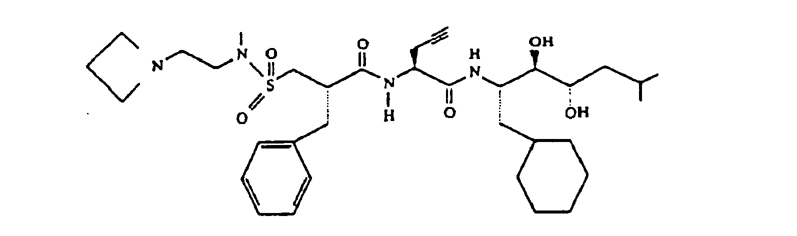 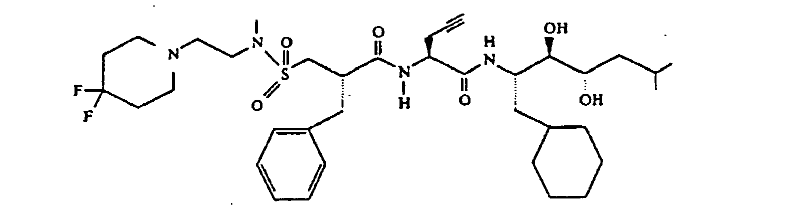 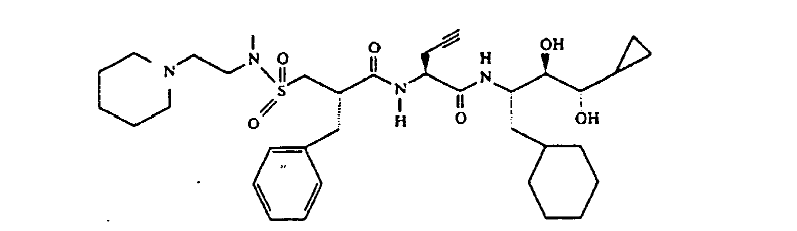 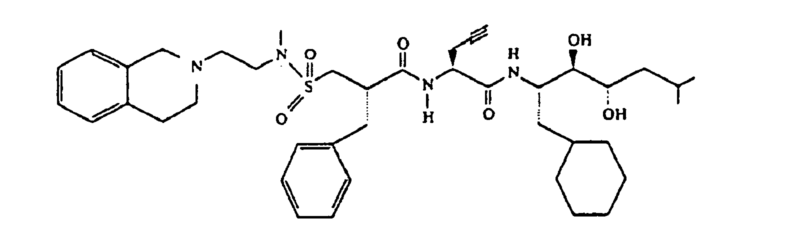 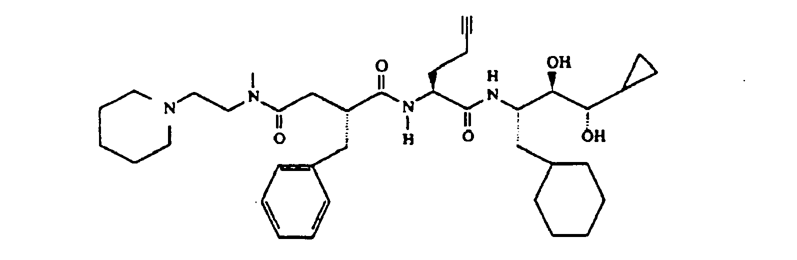 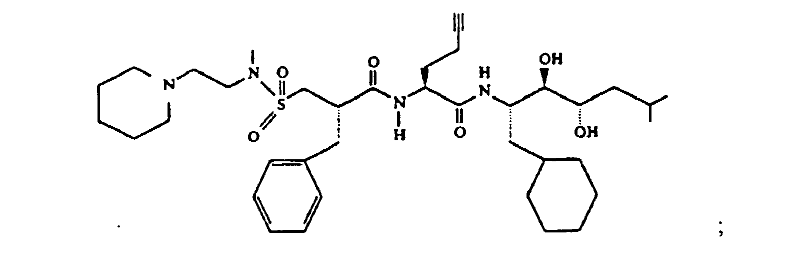 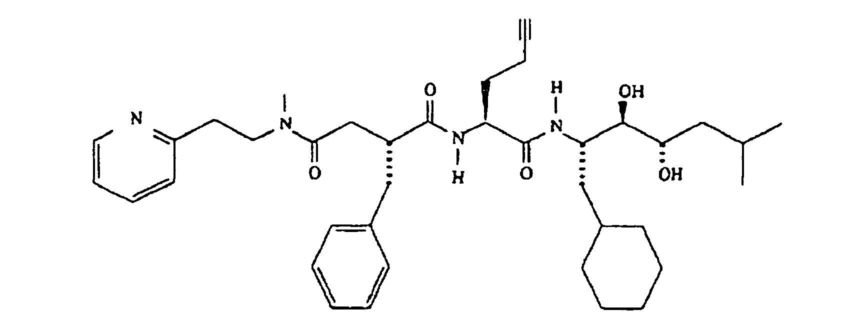 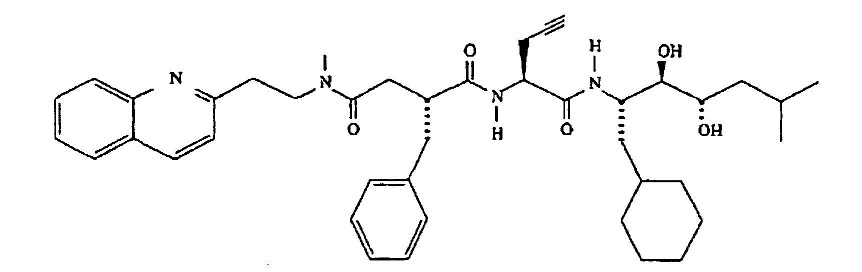 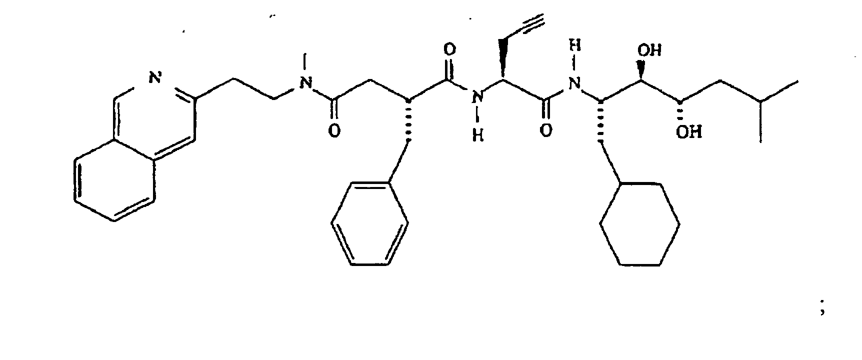 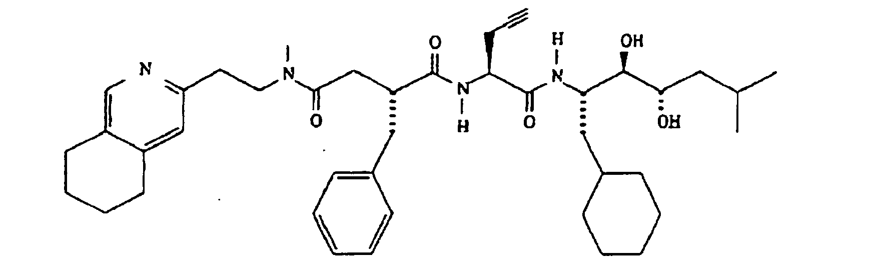 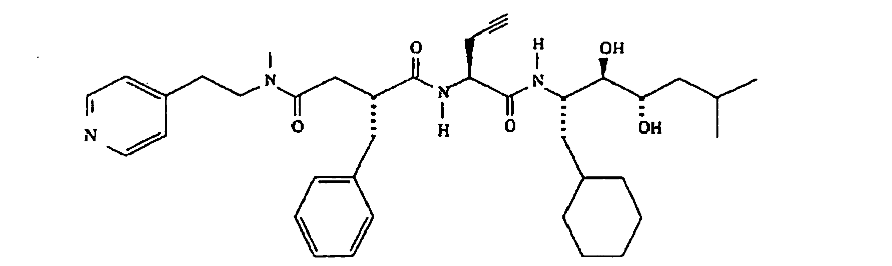 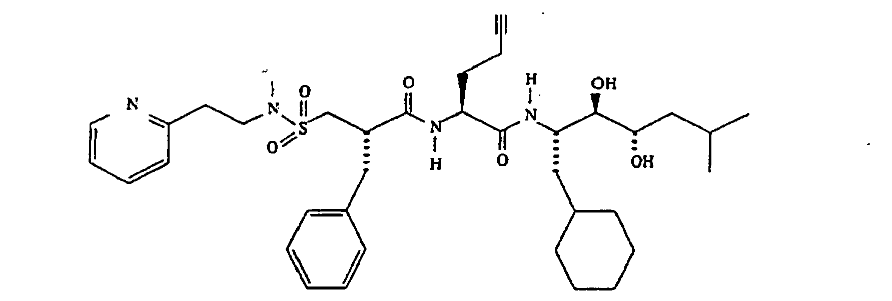 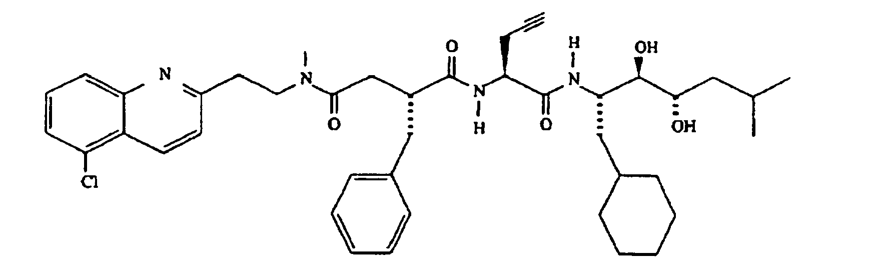 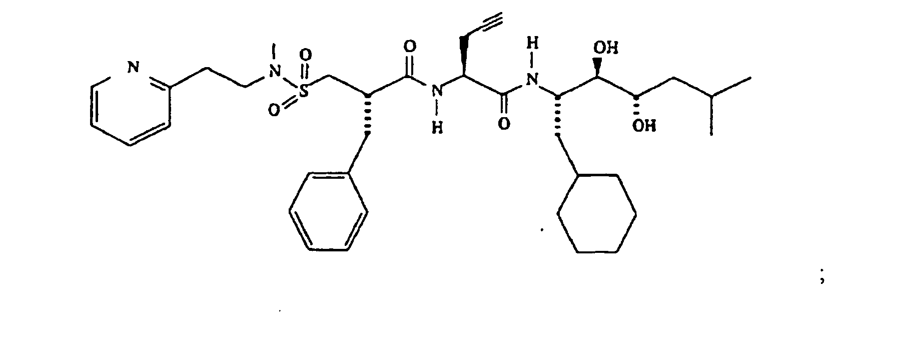 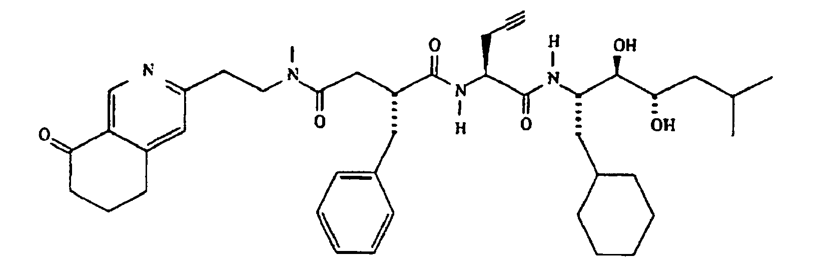 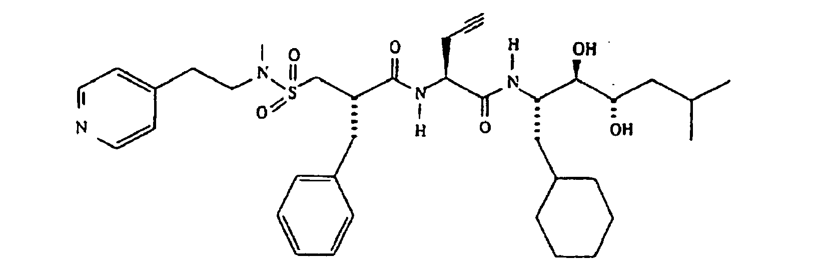 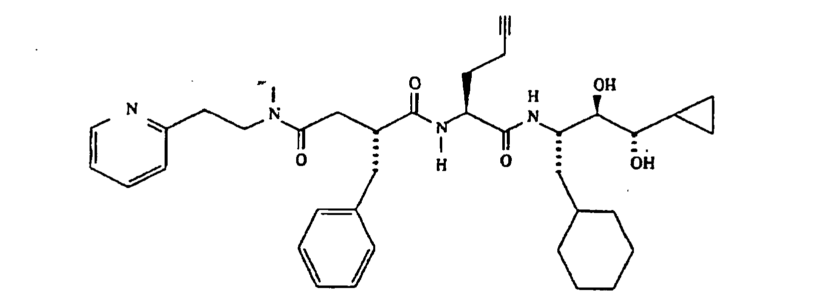 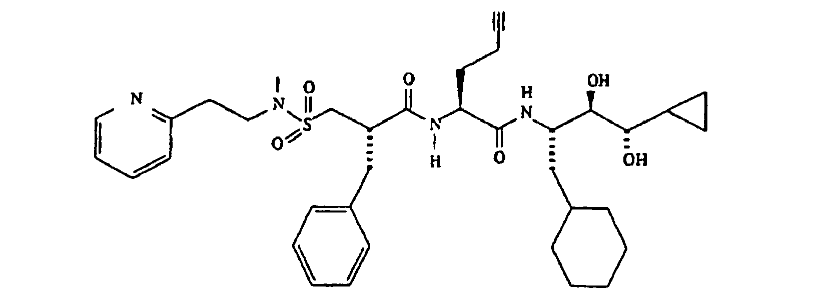 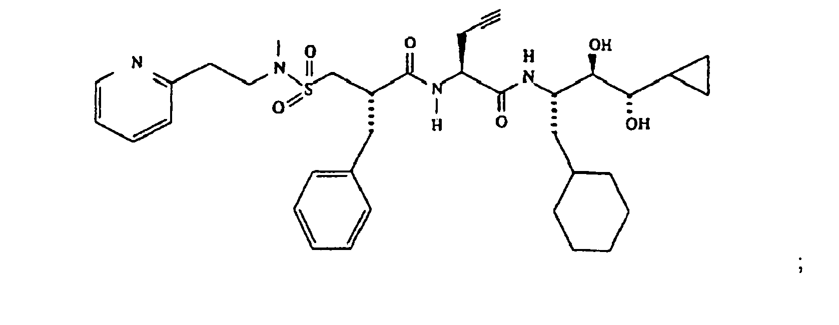 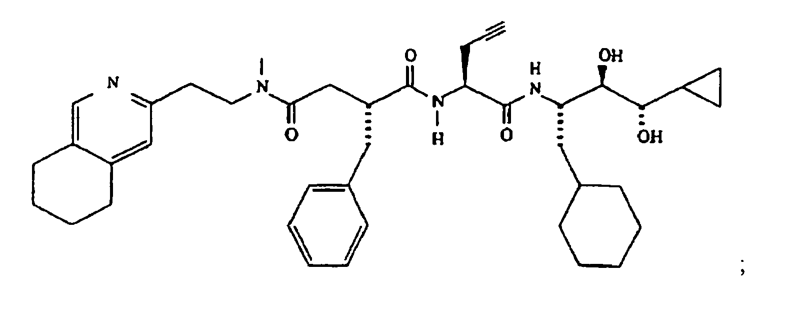 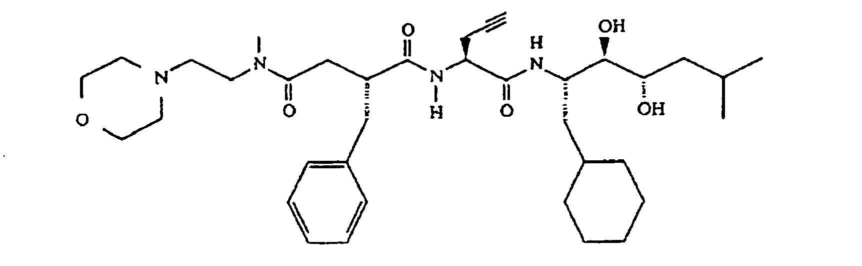 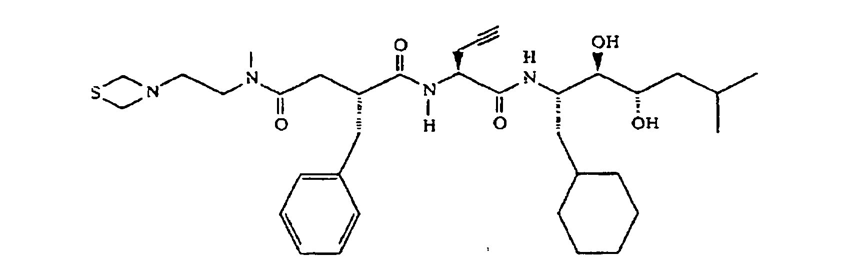 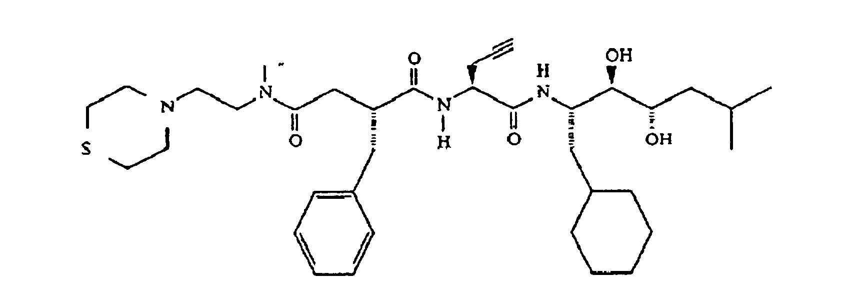 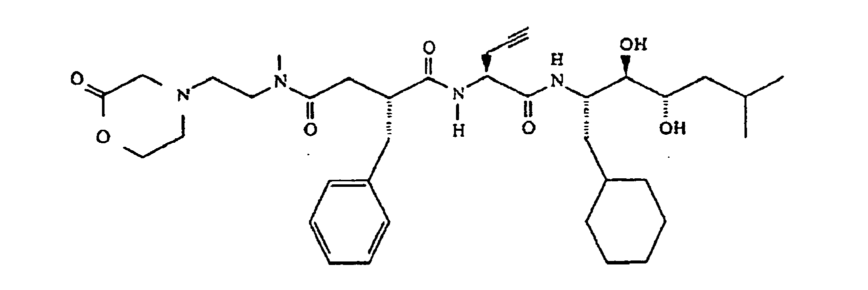 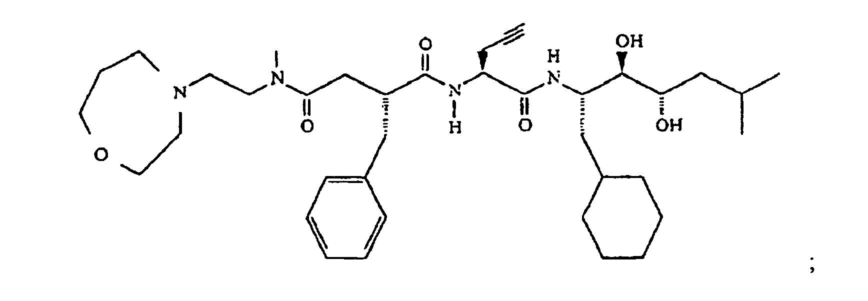 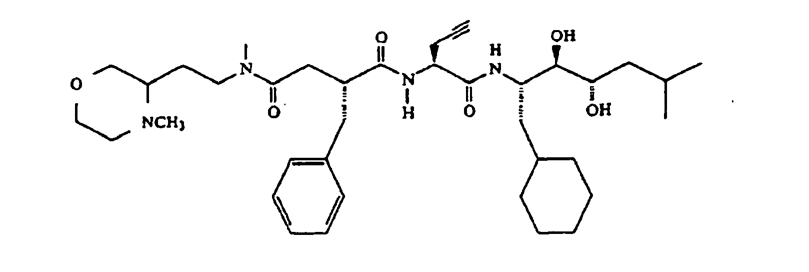 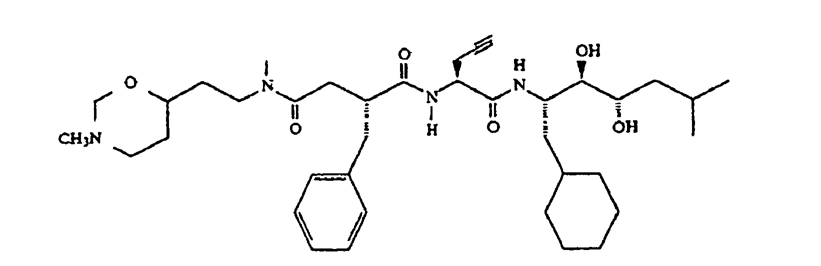 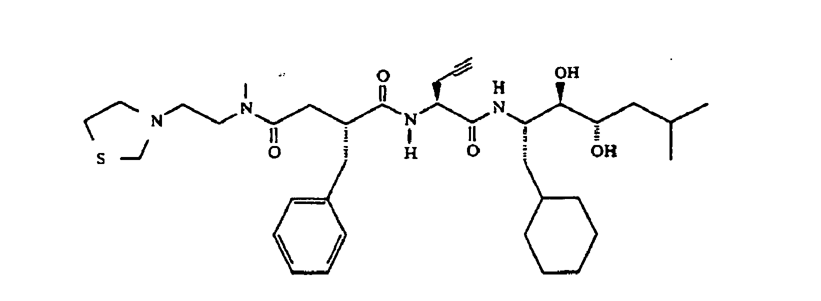 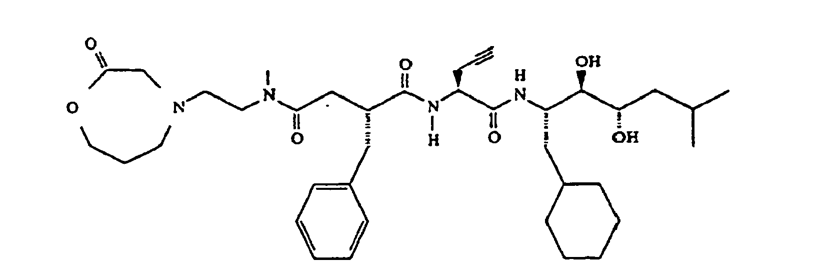 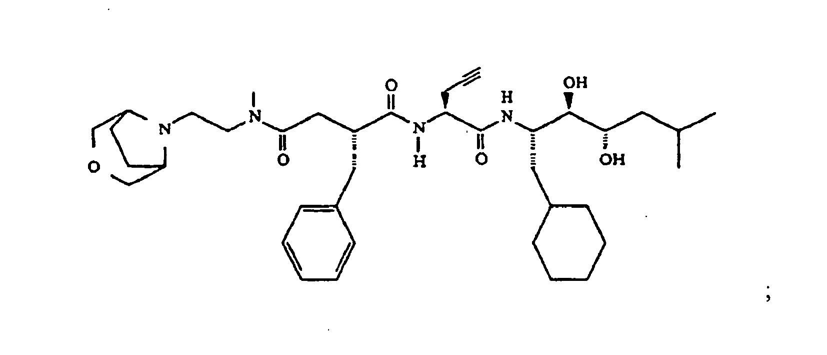 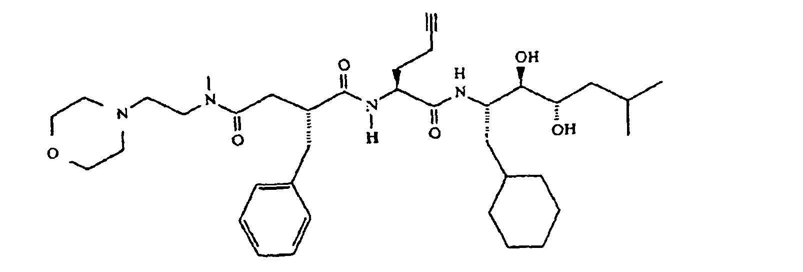 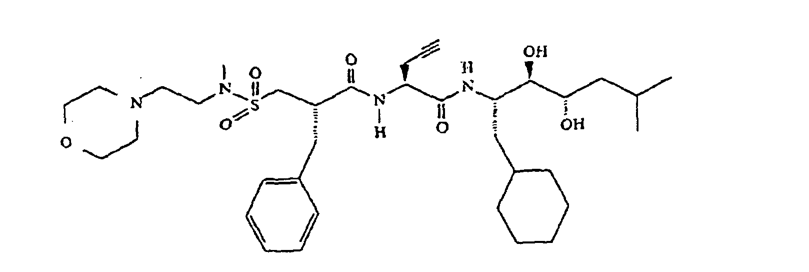 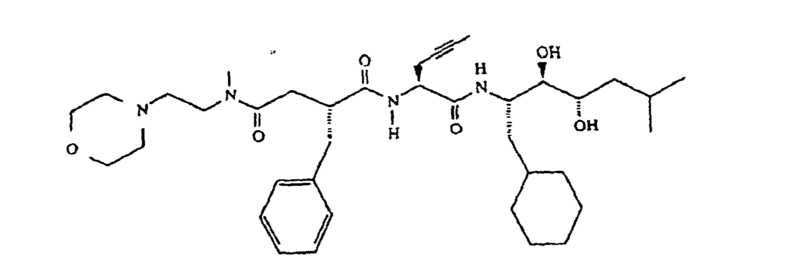 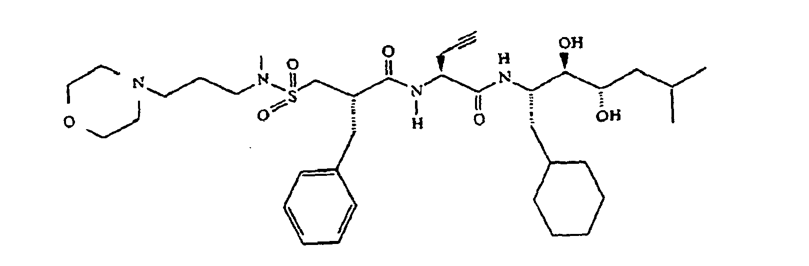 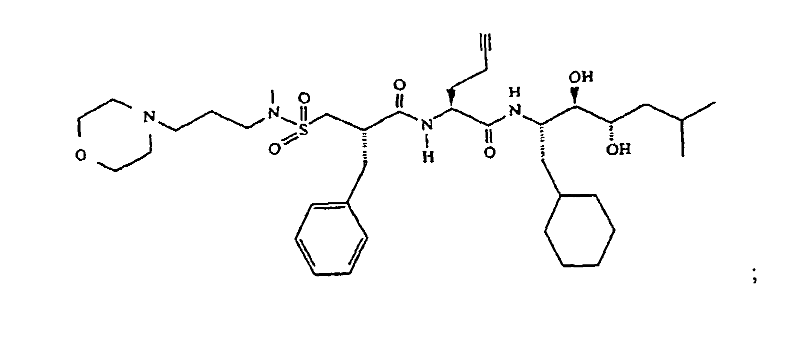 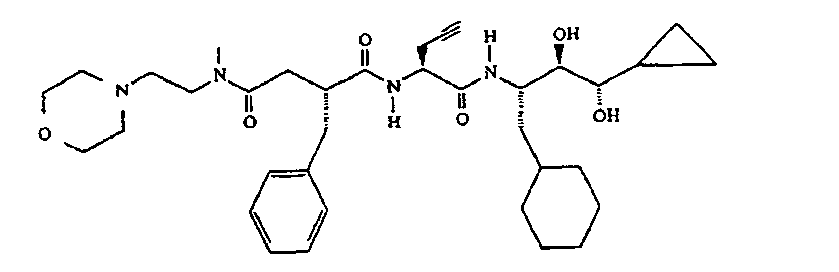 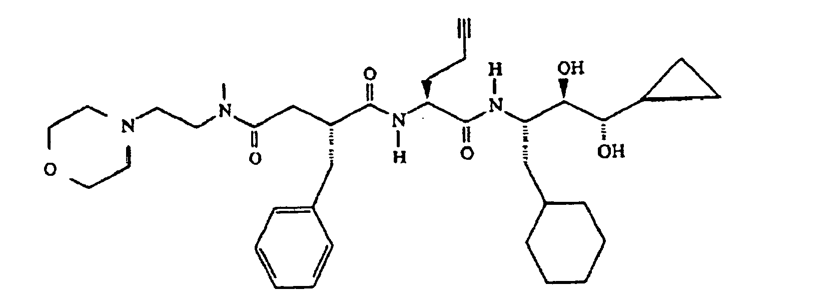 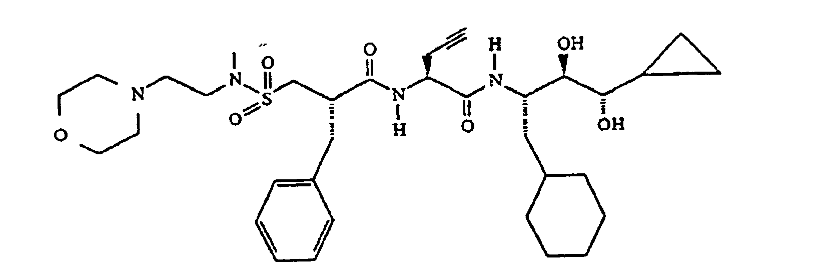 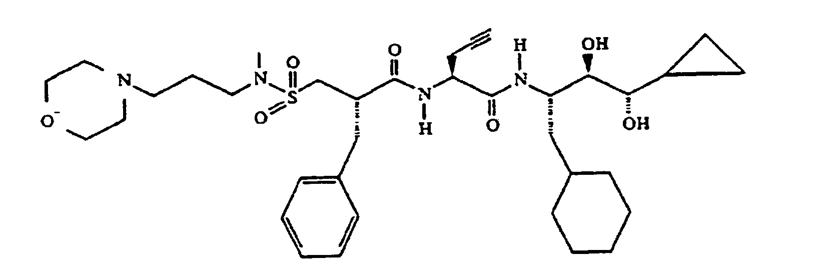 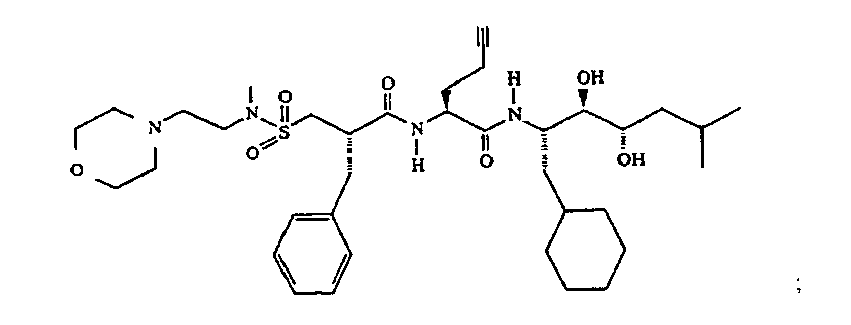 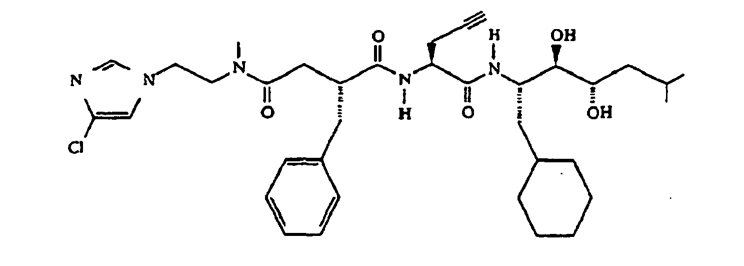 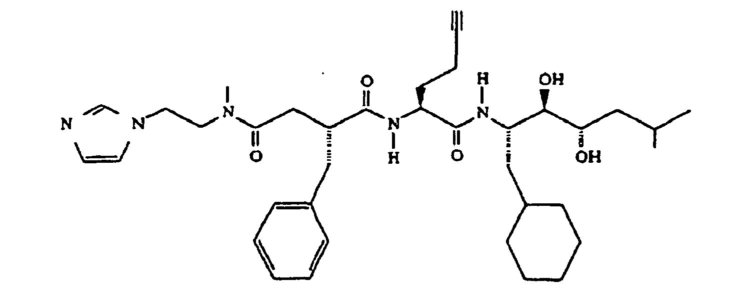 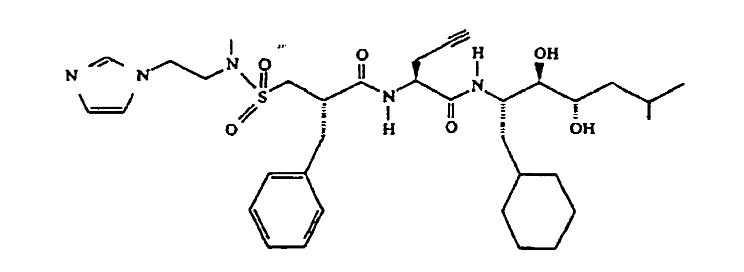 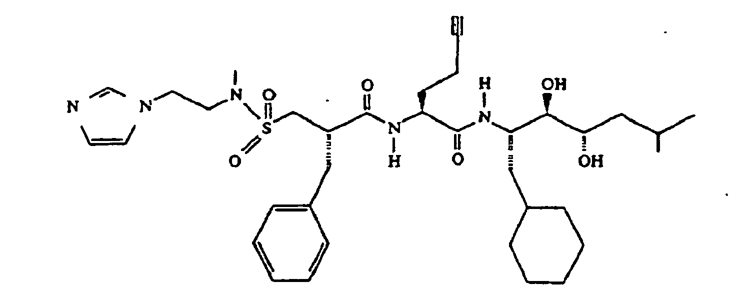 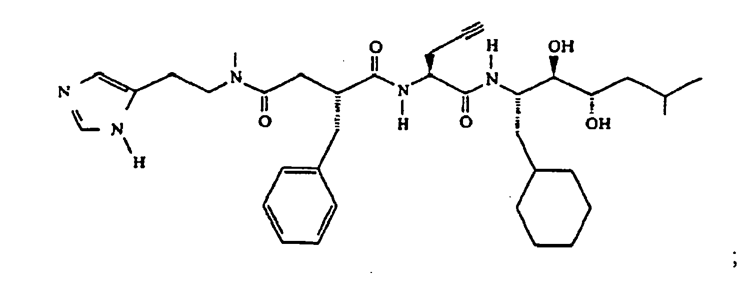 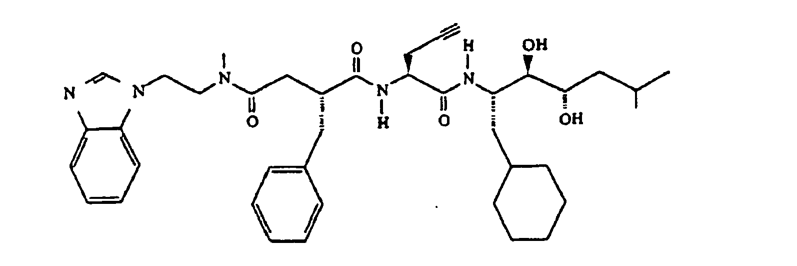 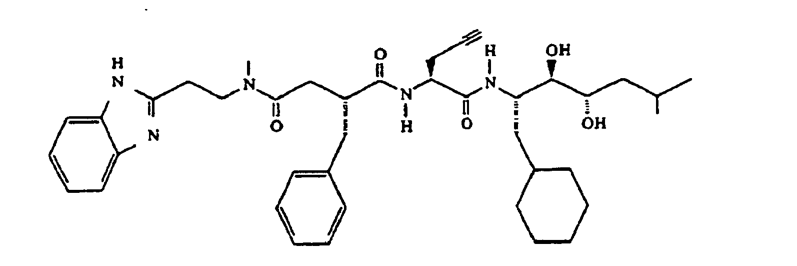 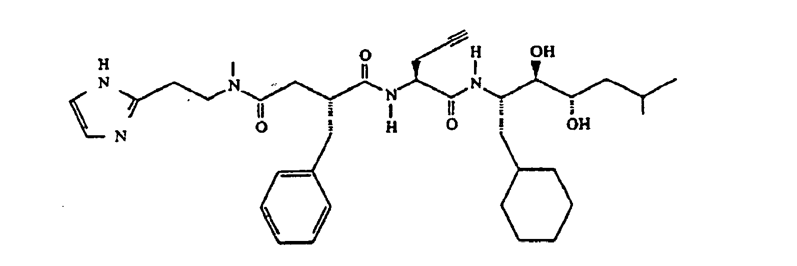 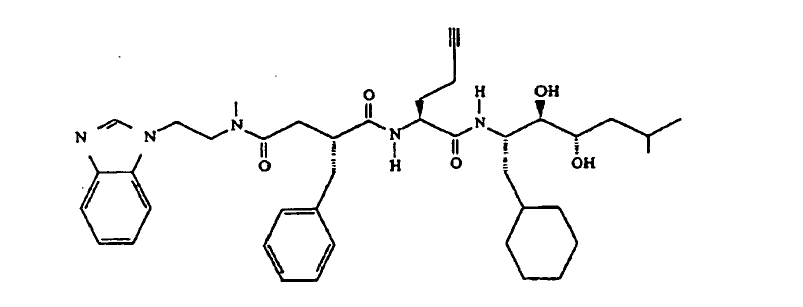 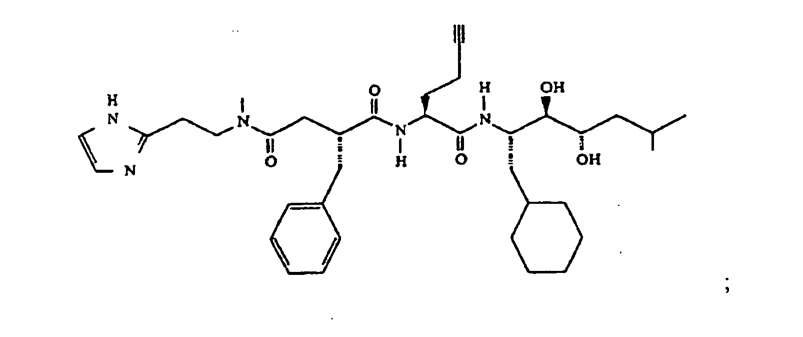 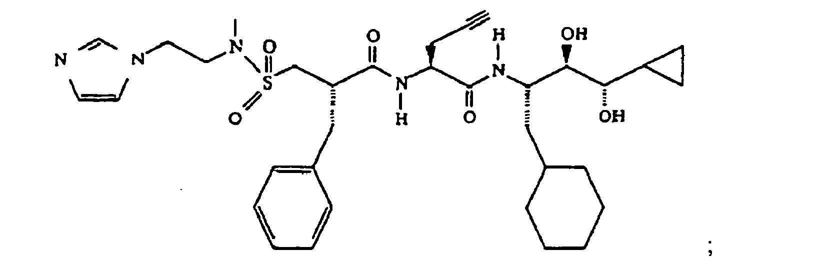 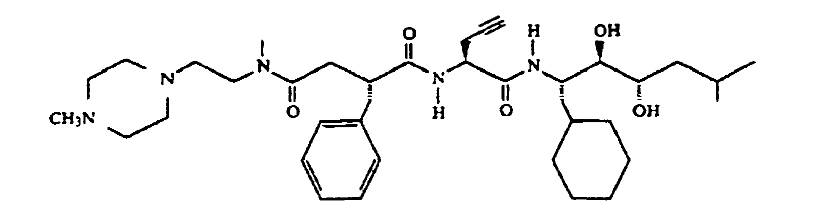 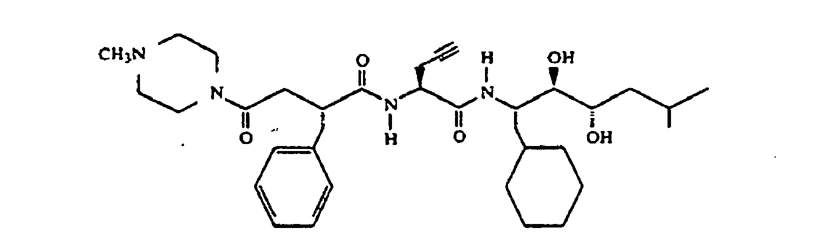 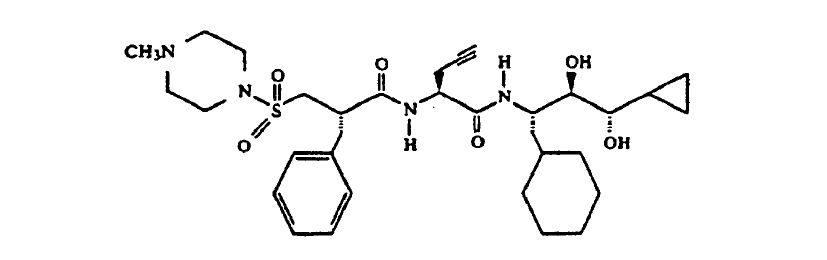 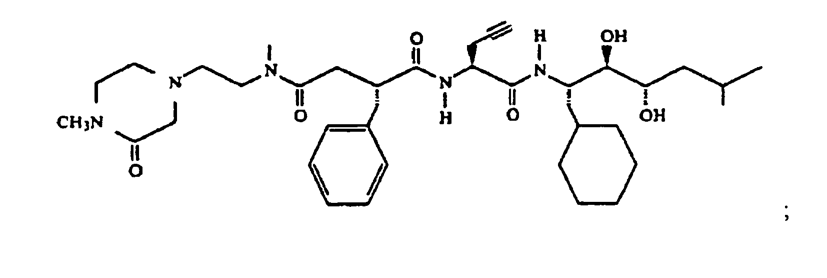 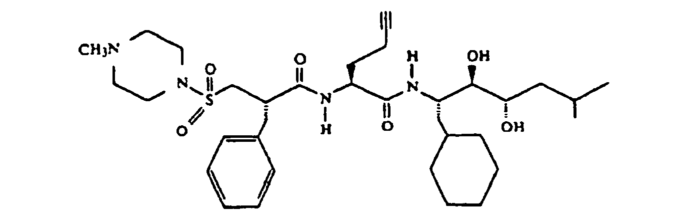 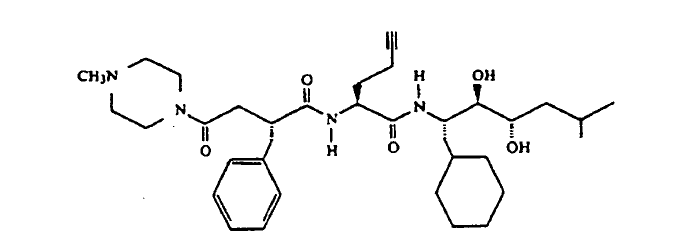 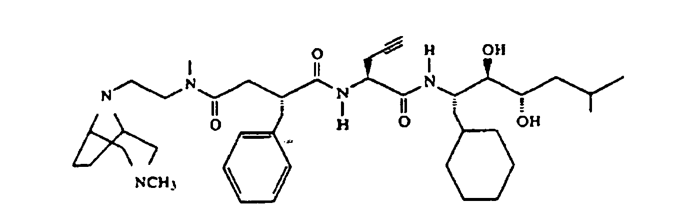 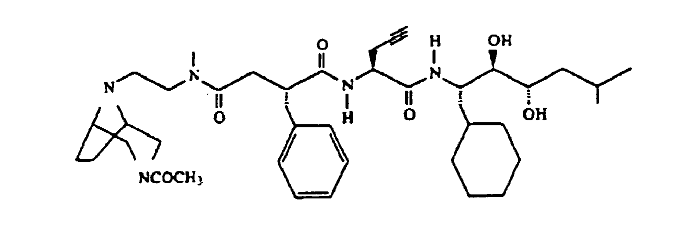 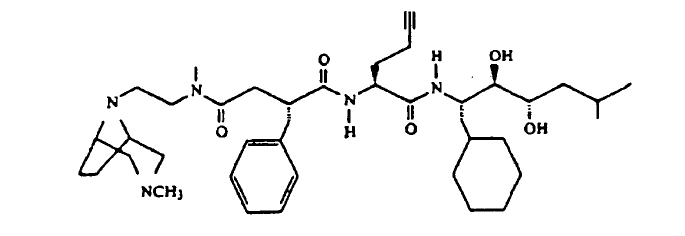 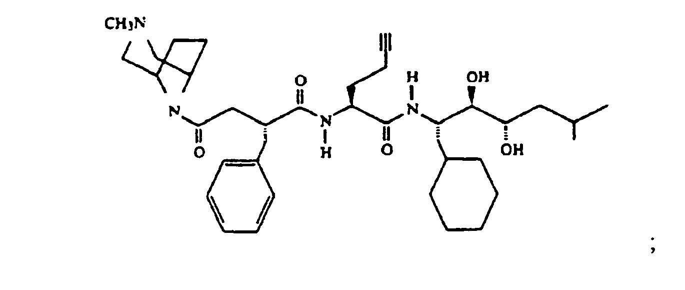 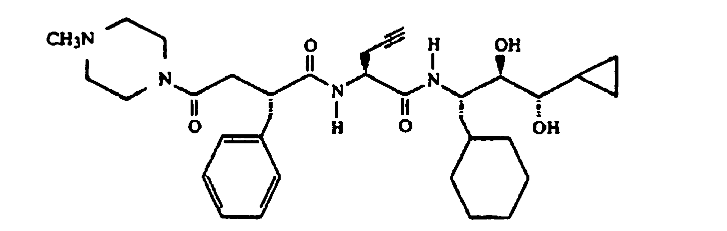 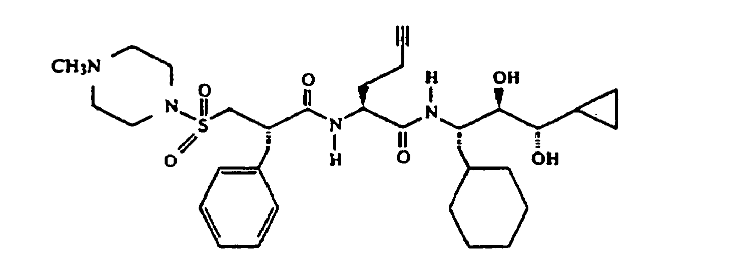 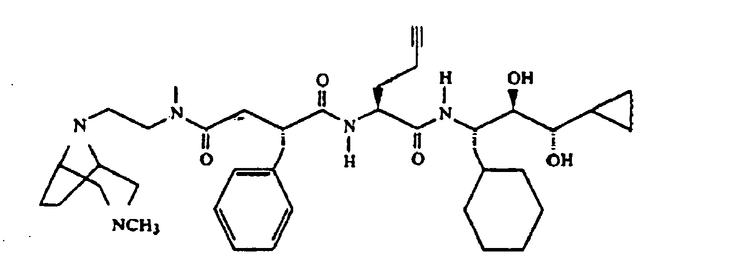 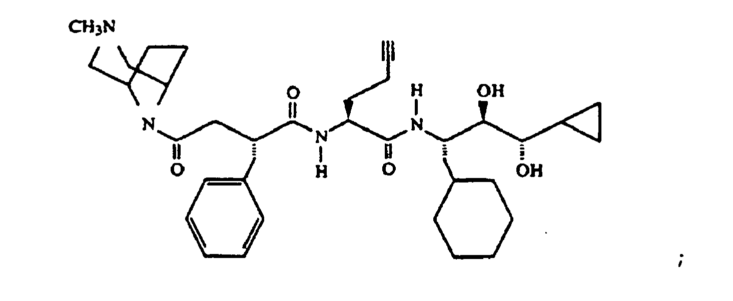
O-[N-methyl-N-(2-methoxyethyl)aminocarbonyl]-3-L-phenyllactyl-L-histidine amide of (2S,3R,4S)-2-amino-1-cyclohexyl-3,4-dihydroxy-6-methylheptane;
O-[N-methyl-N-(2-ethoxyethyl)aminocarbonyl]-3-L-phenyllactyl-L-histidine amide of (2S,3R,4S)-2-amino-1-cyclohexyl-3,4-dihydroxy-6-methylheptane;
O-[N-methyl-N-(2-methoxyethyl)aminocarbonyl]-3-L-phenyllactyl-L-leucine amide of (2S,3R,4S)-2-amino-1-cyclohexyl-3,4-dihydroxy-6-methylheptane;
O-[N-methyl-N-(2-ethoxyethyl)aminocarbonyl]-3-L-phenyllactyl-L-leucine amide of (2S,3R,4S)-2-amino-1-cyclohexyl-3,4-dihydroxy-6-methylheptane;
O-[N-methyl-N-(2-methoxyethyl)aminocarbonyl]-3-L-phenyllactyl-α-(R)-methyl-β-alanine amide of (2S,3R,4S)-2-amino-1-cyclohexyl-3,4-dihydroxy-6-methylheptane;
O-[N-methyl-N-(2-methoxyethyl)aminocarbonyl]-3-L-homophenyllactyl-α-(R)-methyl-β-alanine- amide of (2S,3R,4S)-2-amino-1-cyclohexyl-3,4-dihydroxy-6-methylheptane;
O-[N-methyl-N-(2-methoxyethyl)aminocarbonyl]-3-L-homophenyllactyl-α-(R)-ethyl-P-alanine amide of (2S,3R,4S)-2-amino-1-cyclohexyl-3,4-dihydroxy-6-methylheptane;
3-[N-methyl-N-(2-methoxyethyl)aminocarbonyl]-2-(R)-(2-phenylethyl)-propionyl-α-(R)-ethyl-β-alanine amide of (2S,3R,4S)-2-amino-1-cyclohexyl-3,4-dihydroxy-6-methylheptane;
(3S,4S)-N-[(tert-Butyloxy)carbonyl]-4-amino-3-acetoxy-5-phenylpentene;
(2R,3S)-N-[(tert-Butyloxy)carbonyl]-3-amino-2-acetoxy-4-phenylbutanal;
(2S,3R,4S)-N-[(tert-Butyloxy)carbonyl]-2-amino-1-phenyl-3,4-dihydroxy-6-methylheptane;
(2S,3R,4S)-N-[(tert-Butyloxy)carbonyl]-2-amino-1-cyclohexyl-3,4-dihydroxy-6-methylheptane;
L-leucine amide of (2S,3R,4S)-2-amino-1-cyclohexyl-3,4-dihydroxy-6-methylheptane;
Boc-(im-Tosyl)-L-histidine amide of (2S,3R,4S)-2-amino-1-cyclohexyl-3,4-dihydroxy-6-methylheptane;
(im-Tosyl)-L-histidine amide of (2S,3R,4S)-2-amino-1-cyclohexyl-3,4-dihydroxy-6-methylheptane;
O-(N-methyl-2-methoxyethylaminocarbonyl)-3-L-phenyllactyl-L-histidine amide of (2S,3R,4S)-2-amino-1-cyclohexyl-3,4-dihydroxy-6-methylheptane;
O-(N-methyl-2-methoxyethylaminocarbonyl)-3-L-phenyllactyl-L-leucine amide of (2S,3R,4S)-2-amino-1-cyclohexyl-3,4-dihydroxy-6-methylheptane;
O-(N-methyl-2-methoxyethylaminocarbonyl)-3-L-phenyl lactic acid;
N-Boc-α-(R)-methyl-β-alanine amide of (2S,3R,4S)-2-amino-1-cyclohexyl-3,4-dihydroxy-6-methylheptane;
α-(R)-Methyl-β-alanine amide of (2S, 3R, 4S)-2-amino-1-cyclohexyl-3,4-dihydroxy-6-methylheptane;
O-(N-methyl-2-methoxyethylaminocarbonyl)-3-L-phenyllactyl-α-(R)-methyl-β-alanine amide of (2S,3R,4S)-2-amino-1-cyclohexyl-3,4-dihydroxy-6-methylheptane;
3-(N-methyl-2-methoxyethylaminocarbonyl)-2R-phenethyl propionic acid; and
3-[N-methyl-N-(2-ethoxyethyl)aminocarbonyl]-2-(R)-(2-phenylethyl)-propionyl-α-(R)-ethyl-β-alanine amide of (2S,3R,4S)-2-amino-1-cyclohexyl-3,4-dihydroxy-6-methylheptane;
(3S,4S)-N-[(tert-Butyloxy)carbonyl]-4-amino-3-acetoxy-5-phenylpentene
(2R,3S)-N-[(tert-Butyloxy)carbonyl]-3-amino-2-acetoxy-4-phenylbutanal
(2S,3R,4S)-N-[(tert-Butyloxy)carbonyl]-2-amino-1-phenyl-3,4-dihydroxy-6-methylheptane
(2S,3R,4S)-N-[(tert-Butyloxy)carbonyl]-2-amino-1-cyclohexyl-3,4-dihydroxy-6-methylheptane
N-Boc-α-methyl-β-alaninamide of (2S,3R,4S)-2-amino-1-cyclohexyl-3,4-dihydroxy-6-methylheptane (isomer A)
N-Boc-α-methyl-β-alaninamide of (2S,3R,4S)-2-amino-1-cyclohexyl-3,4-dihydroxy-6-methylheptane (isomer B)
α-Methyl-β-alaninamide of (2S,3R,4S)-2-amino-1-cyclohexyl-3,'4-dihydroxy-6-methylheptane (from isomer A)
D,L-Monomethyl-2-(1-naphthylmethyl)-succinate
D,L-Methyl-3-(N-morpholinocarbonyl)-2-(1-naphthylmethyl)-propionate
D,L-3-(N-Morpholinocarbonyl)-2-(1-naphthylmethyl)-propionic acid
3-(N-morpholinocarbonyl)-(2R,S)2-(1-naphthylmethyl)-propionyl-α-methyl-β-alaninamide of (2S,3R,4S)-2-amino-1-cyclohexyl-3,4-dihydroxy-6-methylheptane (from isomer A)
α-methyl-β-alaninamide of (2S,3R,4S)-2-amino-1-cyclohexyl-3,4-dihydroxy-6-methylheptane (from isomer B)
3-(N-morpholinocarbonyl)-(2R,S)2-(1-naphthylmethyl)-propionyl-α-methyl-β-alaninamide of (2S,3R,4S)-2-amino-1-cyclohexyl-3,4-dihydroxy-6-methylheptane (from isomer B)
Methyl-D,L-3-aminoisobutyrate
Boc-L-phenylalaninyl-D,L-α-methyl-β-alanine free acid
(2S,3R)-N-Boc-2-amino-1-cyclohexyl-3,4-dihydroxybutane
Boc-L-phenylalaninyl-D,L-α-methyl-β-alaninamide of (2S,3R)-2-amino-1-cylohexyl-3,4-dihydroxybutane
Boc-L-phenylalaninyl-D,L-α-methyl-β-alaninamide of (2S,3R,4S)-2-amino-1-cyclohexyl-3,4-dihydroxy-6-methylheptane
Methyl-L-3-phenyl lactate
O-(N-morpholinocarbonyl)-3-L-phenyl lactic acid
O-(N-morpholinocarbonyl)-3-L-phenyllactyl-D,L-a-methyl-p-alanine free acid
O-(N-morpholinocarbonyl)-3-L-phenyllactyl-D,L-a-methyl-β-alaninamide of (2S,3R,4S)-2-amino-1-cyclohexyl-3,4-dihydroxy-6-methylheptane
O-(N-morpholinocarbonyl)-3-L-phenyllactyl-α-(R)-methyl-β-alaninamide of (2S,3R,4S)-2-amino-1-cyclohexyl-3,4-dihydroxy-6-methylheptane; and
O-(N-morpholinocarbonyl)-3-L-phenyllactyl-α-(S)-methyl-β-alaninamide of (2S,3R,4S)-2-amino-1-cyclohexyl-3,4-dihydroxy-6-methylheptane;
(3S,4S)-N-[(tert-Butyloxy)carbonyl]-4-amino-3-acetoxy-5-phenylpentene;
(2R,3S)-N-[(tert-Butyloxy)carbonyl]-3-amino-2-acetoxy-4-phenylbutanal;
(2S,3R,4S)-N-[(tert-Butyloxy)carbonyl]-2-amino-1-phenyl-3,4-dihydroxy-6-methylheptane;
(2S,3R,4S)-N-[(tert-Butyloxy)carbonyl]-2-amino-1-cyclohexyl-3,4-dihydroxy-6 -methylheptane;
L-Leucinamide of (2S,3R,4S)-2-amino-1-cyclohexyl-3,4-dihydroxy-6-methylheptane;
O-(N-morpholinocarbonyl)-3-L-phenyllactyl-L-leucinamide of (2S,3R,4S)-2-amino-1-cyclohexyl-3,4-dihydroxy-6-methylheptane;
O-(N-morpholinocarbonyl)-3-L-phenyllactyl-L-leucine ;
(2S, 3R, 4S)-N-Boc-2-amino-1-cyclohexyl-3,4-dihydroxypentane;
O-(N-morpholinocarbonyl)-3-L-phenyllactyl-L-leucinamide of (2S, 3R, 4S)-2-amino-1-cyclohexyl-3,4-dihydroxypentane;
(2S, 3R)-N-Boc-2-amino-1-cyclohexyl-3,4-dihydroxybutane;
O-(N-morpholinocarbonyl)-3-L-phenyllactyl-L-leucinamide of (2S, 3R)-2-amino-1-cyclohexyl-3,4-dihydroxybutane;
Methyl L-3-phenyllactate;
O-(N-morpholinocarbonyl)-3-L-phenyllactic acid;
(2S,3R)-Boc-L-Leucinamide of 2-amino-1-cyclohexyl-3,4-dihydroxybutane;
D,L-Monomethyl-2-(1-naphthylmethyl)succinate ;
D,L-Methyl 3-(N-morpholinocarbonyl)-2-(1-naphthylmethyl)propionate;
D,L-3-(N-Morpholinocarbonyl)-2-(1-naphthylmethyl)propionic acid; and
3-(N-morpholinocarbonyl)-2-(R,S)-(1-naphthylmethyl)propionyl-L-leucinamide of (2S,3R)-2-amino-1-cyclohexyl-3,4-dihydroxybutane;
O- {N- [2- {N- [2- (N, N-dimethylamino) ethyl] -N-methylamino}-ethyl]-N-methylaminocarbonyl}-3-L-phenyllactyl-L-histidineamide of (2S,3R,4S)-2-amino-1-cyclohexyl-3,4-dihydroxy-6-methylheptane;
O-{N- [2-{N- [2- (N,N-dimethylamino) ethyl] -N-methylamino}-ethyl]-N-methylaminocarbonyl}-3-L-phenyllactyl-L-leucineamide of (2S,3R,4S)-2-amino-1-cyclohexyl-3,4-dihydroxy-6-methylheptane;
O-{N- [2-{N- [2- (N,N-dimethylamino) ethyl] -N-methylamino}-ethyl]-N-methylaminocarbonyl}-3-L-phenyllactyl-α-(R)-methyl-β-alanineamide of (2S, 3R, 4S)-2-amino-1-cyclohexyl-3,4-dihydroxy-6-methylheptane;
O-{N-[2-{N-[2-(N,N-dimethylamino)ethyl]-N-methylamino}-ethyl]-N-methylaminocarbonyl}-3-L-homophenyllactyl-α-(R)-methyl-β-alanineamide of (2S, 3R, 4S)-2-amino-1-cyclohexyl-3,4-dihydroxy-6-methylheptane;
O-{N-[2-{N-[2-N,N-dimethylamino)ethyl]-N-methylamino}-ethyl]-N-methylaminocarbonyl}-3-L-homophenyllactyl-α-(R)-ethyl-β-alanineamide of (2S,3R,4S)-2-amino-1-cyclohexyl-3,4-dihydroxy-6-methylheptane;
3-{N-[2-(N,N-dimethylamino)ethyl]-N-methylamino}ethyl]-N-methylaminocarbonyl}-2-(R)-(2-phenylethyl)-propionyl-α-(R)-ethyl-β-alanineamide of (2S,3R,4S)-2-amino-1-cyclohexyl-3,4-dihydroxy-6-methylheptane;
(3S,4S)-N-[(tert-Butyloxy)carbonyl]-4-amino-3-acetoxy-5-phenylpentene;
(2R,3S)-N-[(tert-Butyloxy)carbonyl]-3-amino-2-acetoxy-4-phenylbutanal;
(2S,3R,4S)-N-[(tert-Butyloxy)carbonyl]-2-amino-1-phenyl-3,4-dihydroxy-6-methylheptane;
(2S,3R,4S)-N-[(tert-Butyloxy)carbonyl]-2-amino-1-cyclohexyl-3,4-dihydroxy-6 -methylheptane;
L-Leucineamide of (2S,3R,4S)-2-amino-1-cyclohexyl-3,4-dihydroxy-6-methylheptane;
Boc-(im-Tosyl)-L-histidineamide of (2S,3R,4S)-2-amino-1-cyclohexyl-3,4-dihydroxy-6-methylheptane;
(im-Tosyl)-L-histidineamide of (2S,3R,4S)-2-amino-1-cyclohexyl-3,4-dihydroxy-6-methylheptane;
N-Boc-α-(R)-methyl-β-alanineamide of (2S,3R,4S)-2-amino-1-cyclohexyl-3,4-dihydroxy-6-methylheptane;
α-(R)-Methyl-β-alanineamide of (2S,3R,4S)-2-amino-1-cyclohexyl-3,4-dihydroxy-6-methylheptane;
O-{N-[2-{N-[2-(N,N-dimethylamino)ethyl]-N-methylamino}-ethyl]-N-methylaminocarbonyl]-3-L-phenyllactic acid; and
3{N- [2-(N- [2- (1-imidazole) ethyl] -N-methylamino}ethyl]-N-methylaminocarbonyl]-2-(R)-(2-phenylethyl)propionyl-α-(R)-ethyl-β-alanineamide of (2S,3R,4S)-2-amino-1-cyclohexyl-3,4-dihydroxy-6-methylheptane;
N-[3-[[1S,1R*-(cyclohexylmethyl)-2S*,3R*-5-methylhexyl]amino]-2S*-methyl-3-oxopropyl]-α-[[(1,1-dimethylethyl)sulfonyl]methyl]benzenepropanamide;
N-[3-[[1S,1R*-(cyclohexylmethyl)-2S*,3R*-dihydroxy-5-methylhexyl]amino]-2S*-methyl-2-3-oxopropyl]-α-[[(1,1-dimethylethyl)thio]methyl]benzenepropanamide;
N-[[1S,1R*-(cyclohexylmethyl)-2S*,3R*-dihydroxy-5-methylhexyl]-2R*-methyl-3-[[1-oxo-3-(phenylsulfonyl)propyl]amino]propanamide;
N-[[1R*-[[1S,1R*(cyclohexylmethyl)-2S*,3R*-dihydroxy-5-methylhexyl]amino]carbonyl]-3-butynyl]-α-[[(1,1-dimethylethyl)sulfonyl]methyl]benzenepropanamide;
N-[[1R*-[[1S,1R*(cyclohexylmethyl)-2S*,3R*-dihydroxy-5-methylhexyl]amino]carbonyl]-3-butynyl]-αR*-[[(1,1-dimethylethyl)sulfonyl]methyl]benzenepropanamide;
N-[1S,1R*(cyclohexylmethyl)-2S*,3R*-dihydroxy-5-methylhexyl]-2R*-[[1-oxo-3-[phenylsulfonyl]propyl]amino]-4-pentynamide;
N-[2-[[1S,1R*-(cyclohexymethyl)-2S*,3R*-dihydroxy-5-hexynyl]amino]-1R*-(cyclopropylmethyl)-2-oxoethyl]-aR*-[[(1,1-dimethylethyl)sulfonyl]methyl]benzenepropanamide;
N-[2-[[1S,1R*-(cyclohexylmethyl)-2S*,3R*-dihydroxy-5-hexynyl]amino]-1R*-(cyclopropylmethyl)-2-oxoethyl]-aR*-[[(2-methylpropyl)sulfonyl]methyl]benzenepropanamide;
N-[2-[[1S,1R*-(cyclohexylmethyl)-2S*,3R*-dihydroxy-5-hexynyl]amino]-1R*-(cyclopropylmethyl)-2-oxoethyl]-αR*-[phenylsulfonyl)methyl]benzenepropanamide;
N-[[1R*-[(1S,1R*-(cyclohexylmethyl)-2S*,3R*-dihydroxy-5-hexynyl]amino]carbonyl]-3butynyl]-αR*-[[(1,1-dimethylethyl)sulfonyl]methyl]benzenepropanamide;
N-[[1R*-[[1S,1R*-(cyclohexylmethyl)-2S*,3R*-dihydroxy-5-hexynyl]amino]carbonyl]-3butynyl]-αR*-[[(2-methylpropyl)sulfonyl]methyl]benzenepropanamide;
N-[[1R*-[[1S,1R*-(cyclohexylmethyl)-2S*,3R*-dihydroxy-5-hexynyl]amino]carbonyl]-3butynyl]-aR*-[(phenylsulfonyl)methyl]benzenepropanamide; 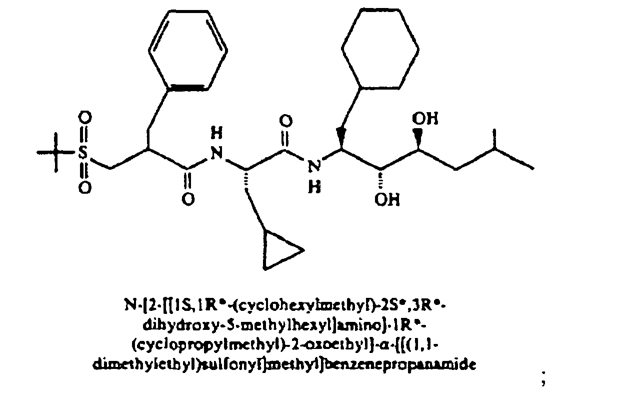 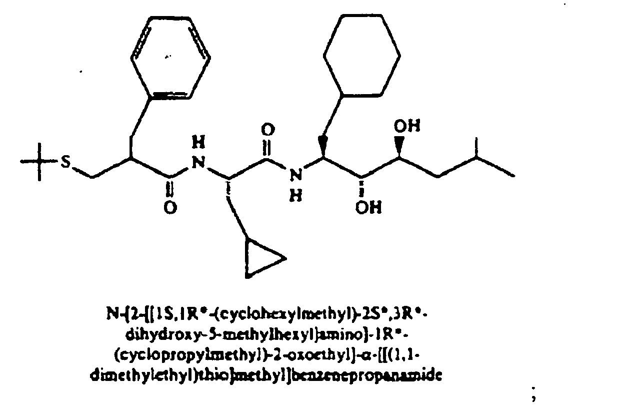 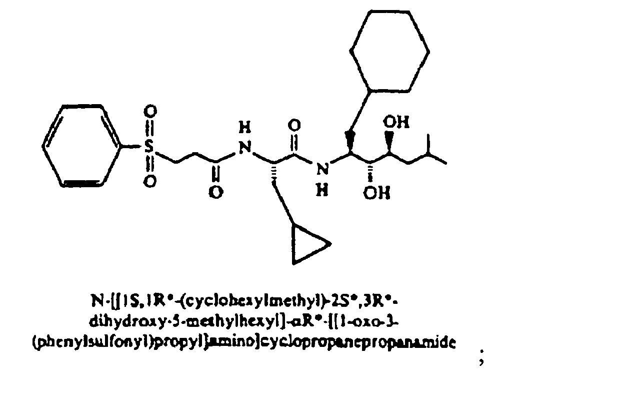 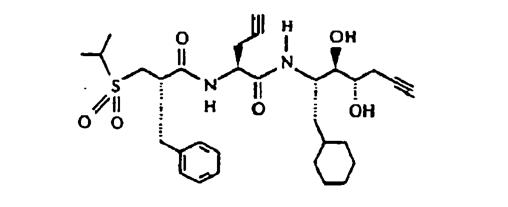 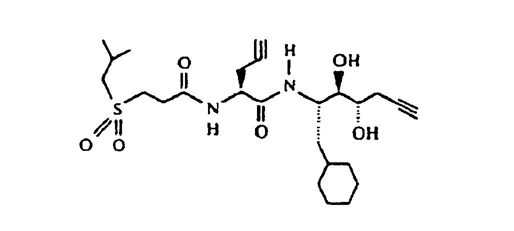 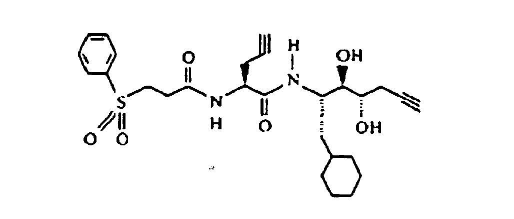 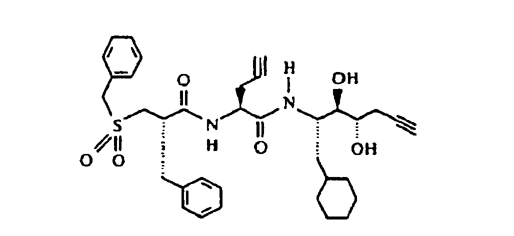 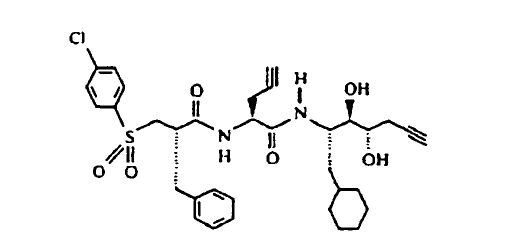 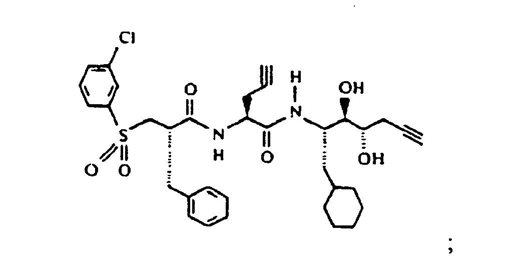 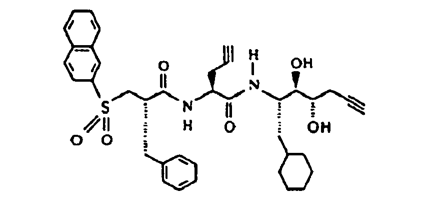 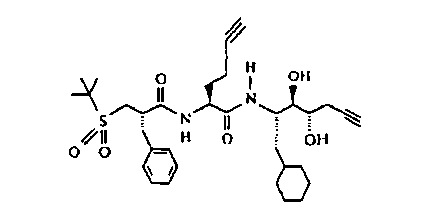 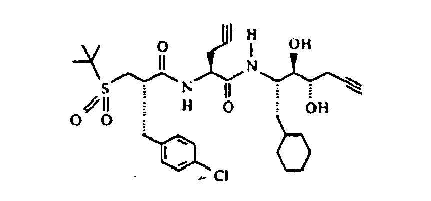 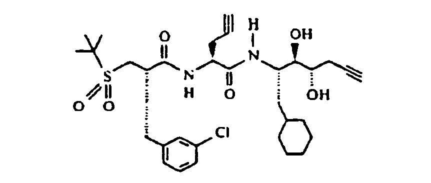 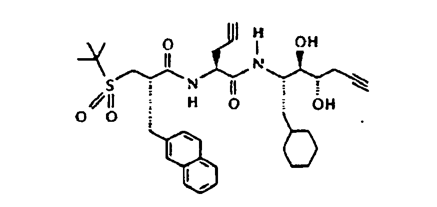 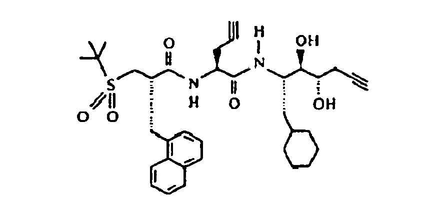 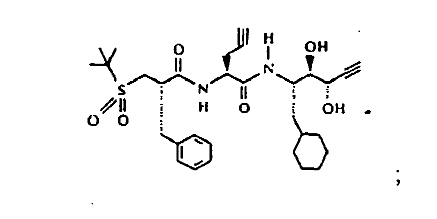 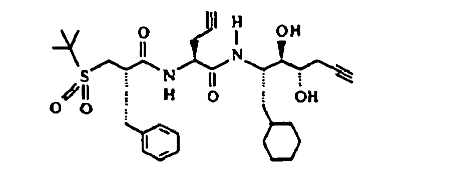 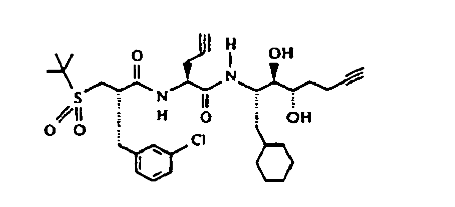 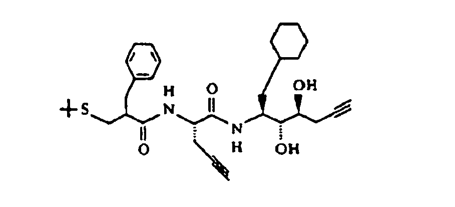 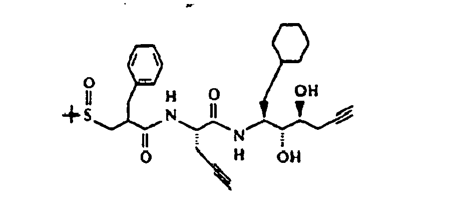 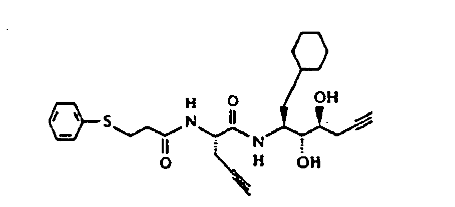 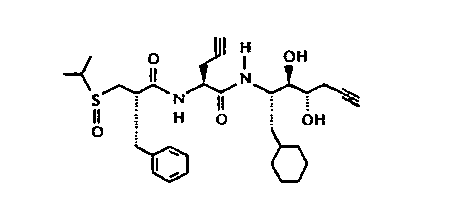 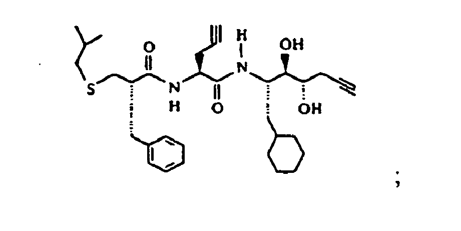 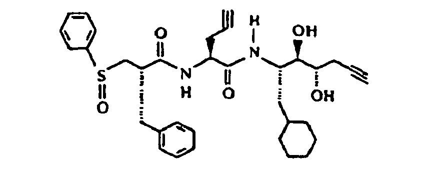 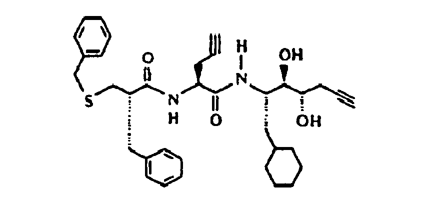 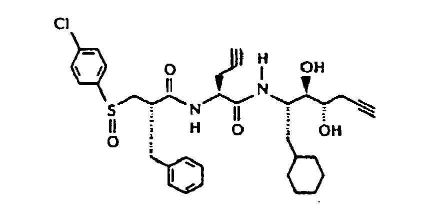 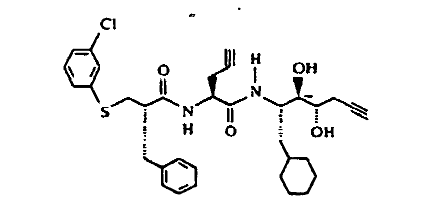 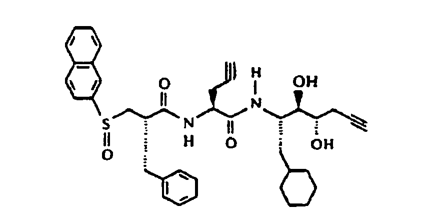 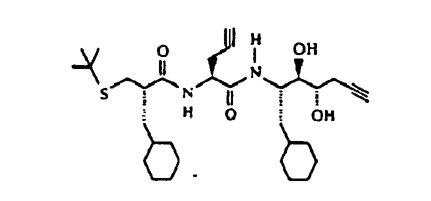 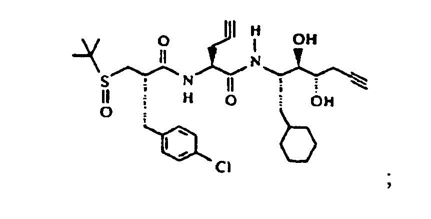 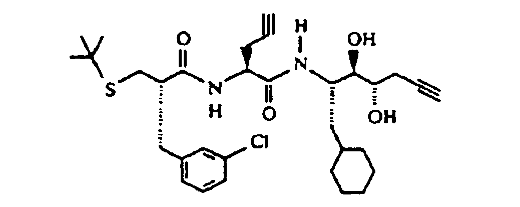 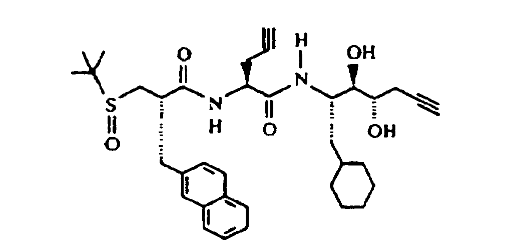 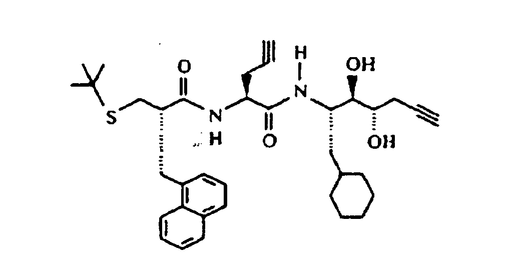 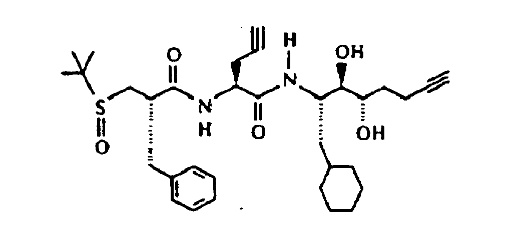 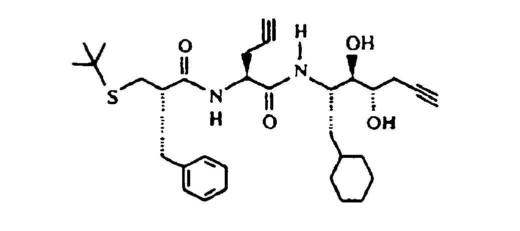 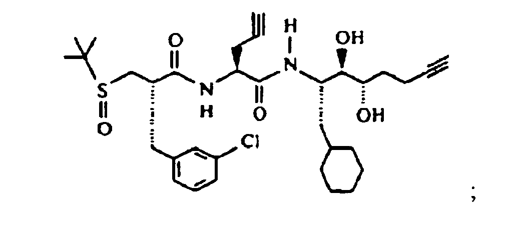 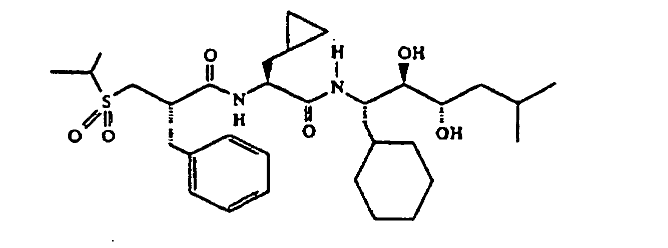 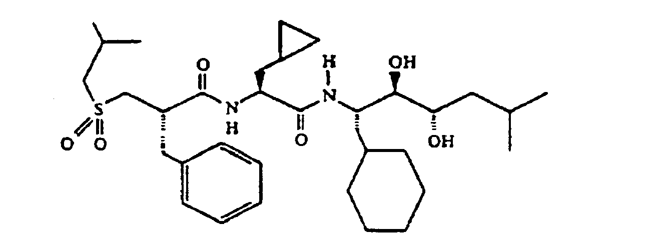 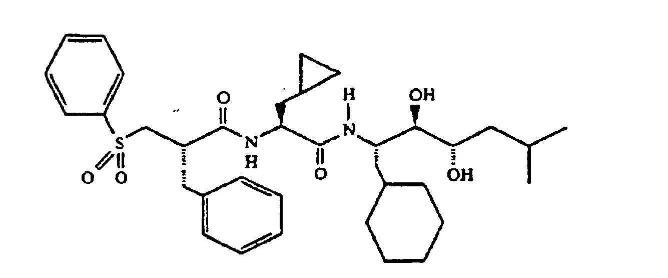 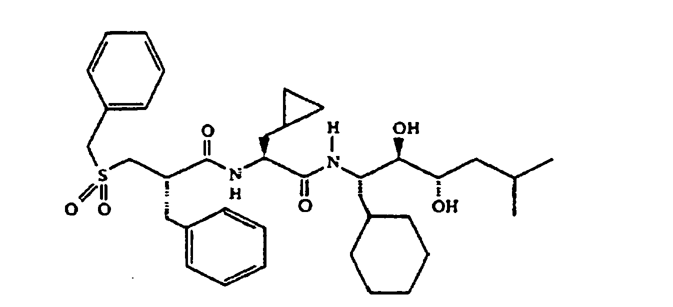 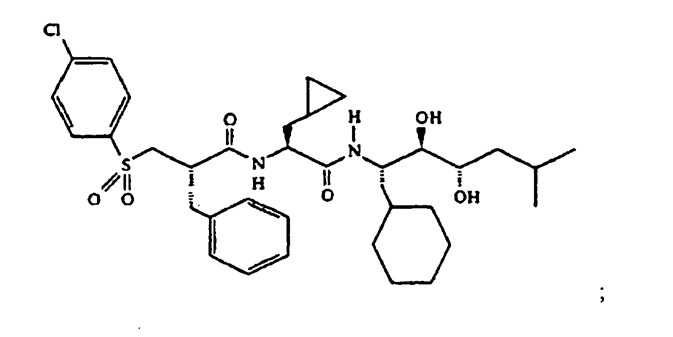 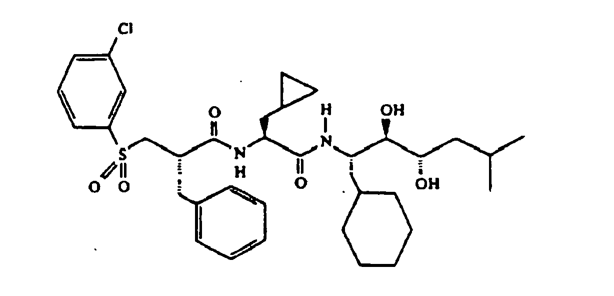 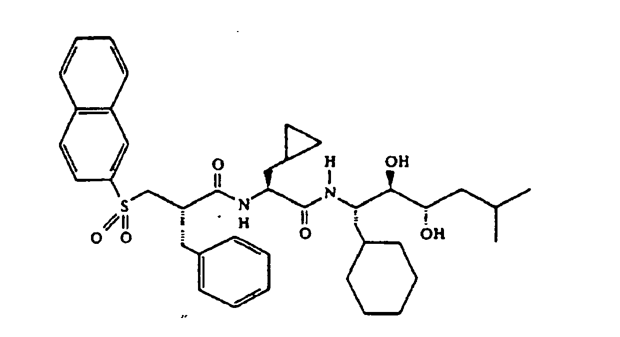 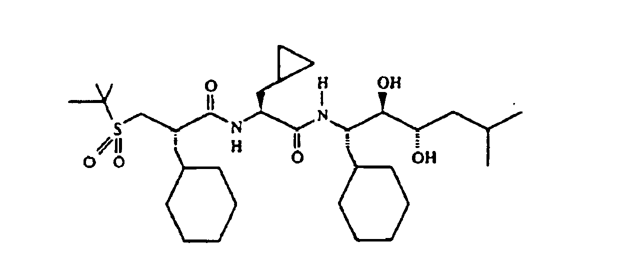 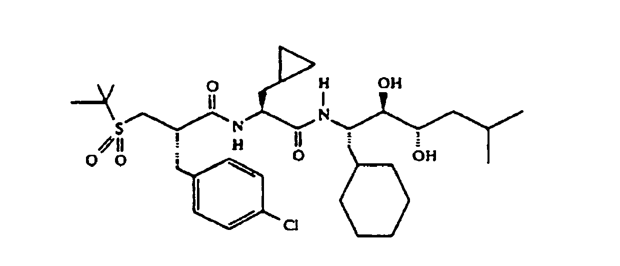 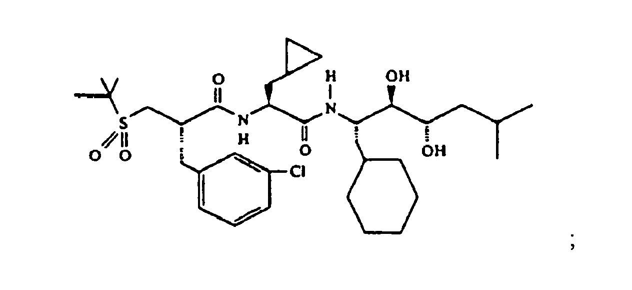 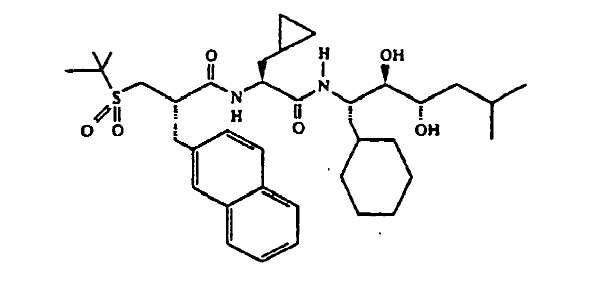 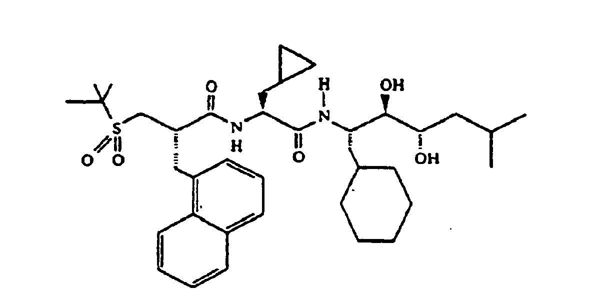 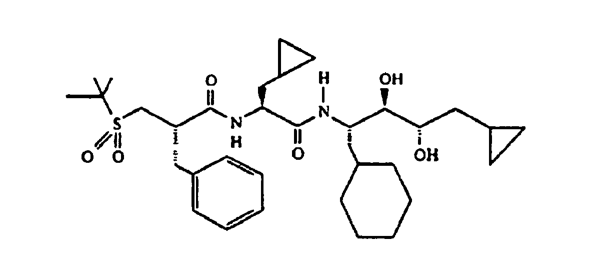 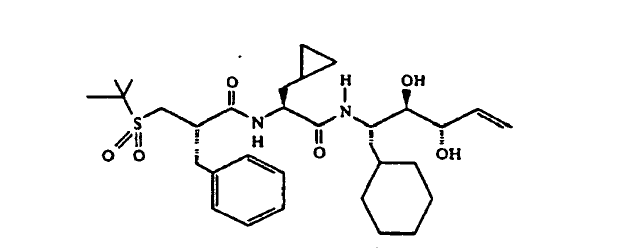 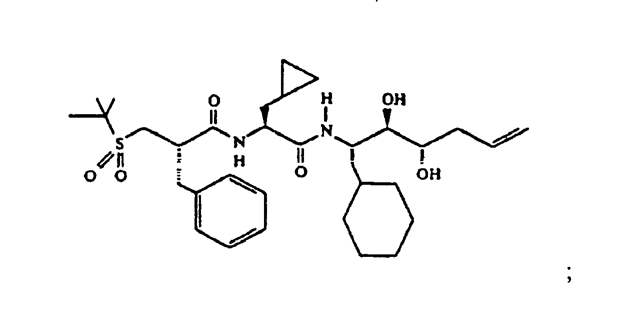 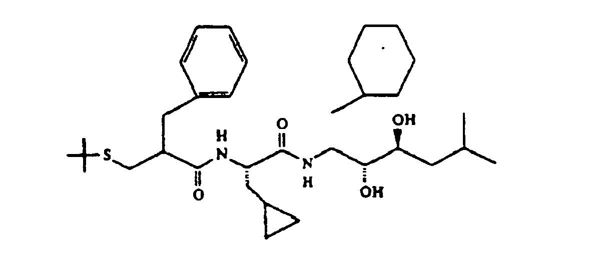 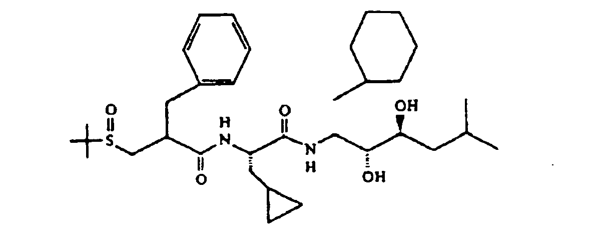 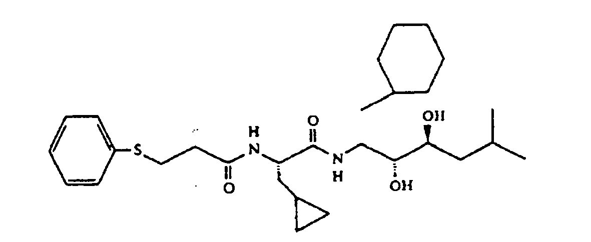 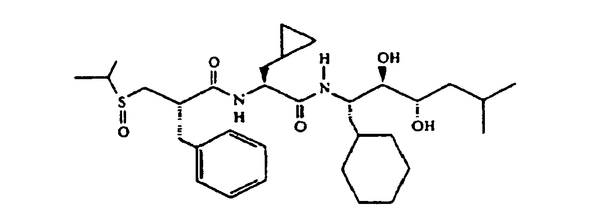 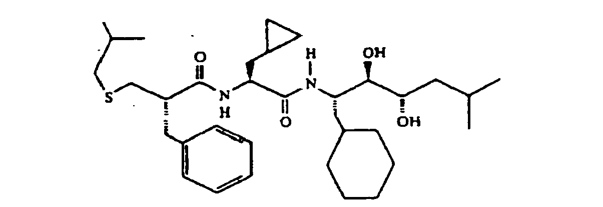 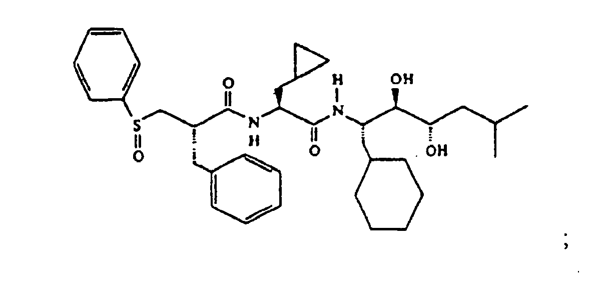 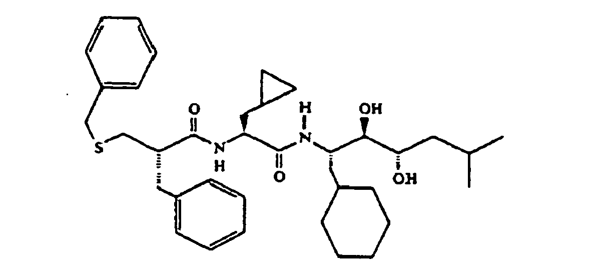 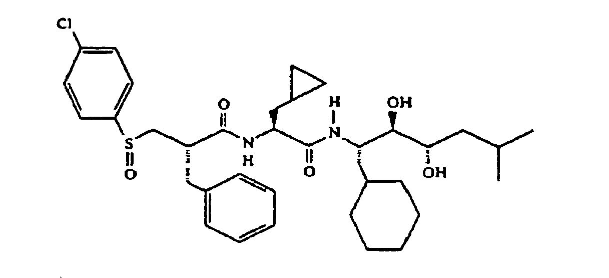 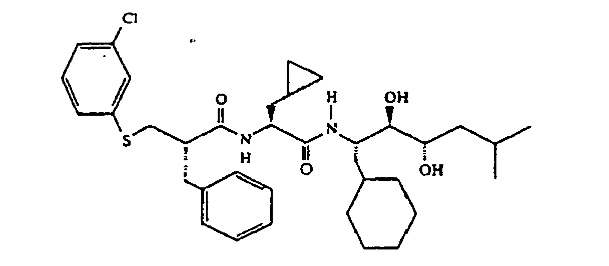 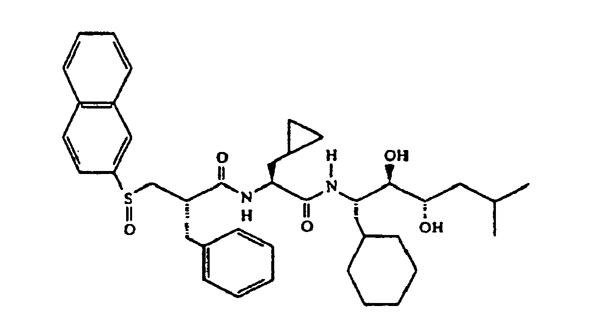 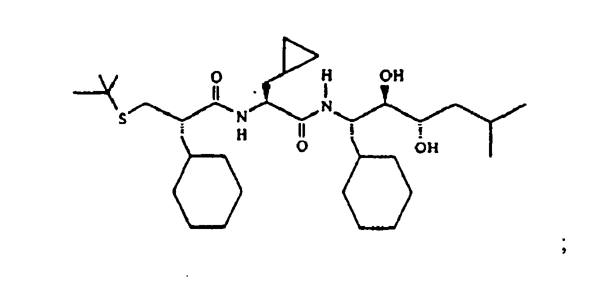 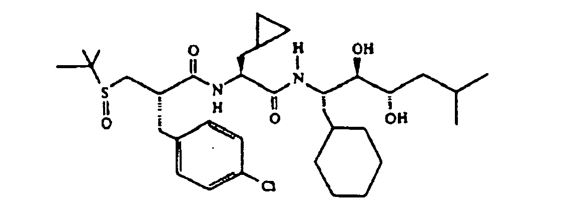 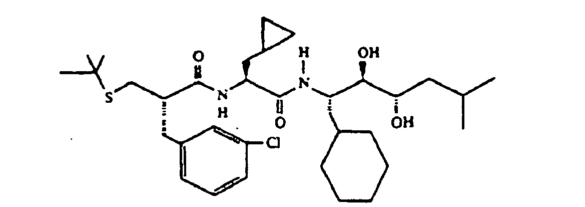 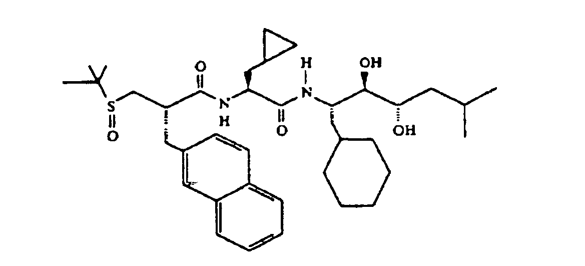 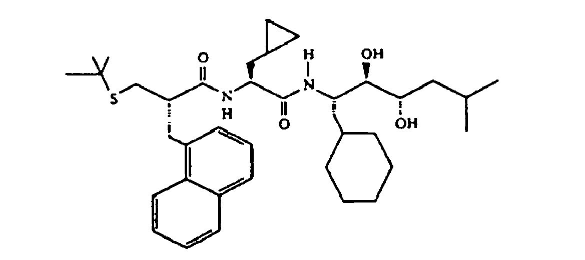 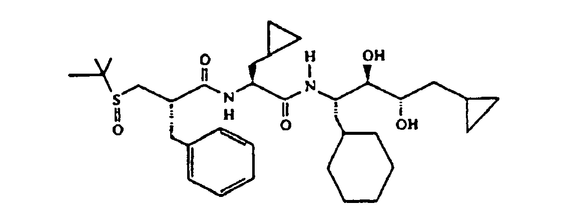 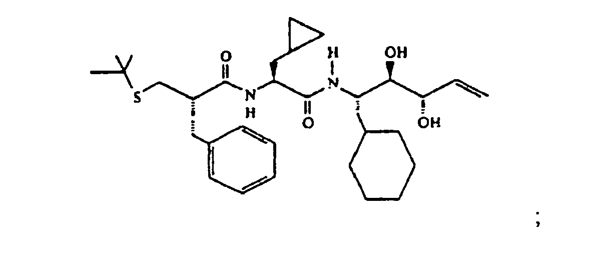 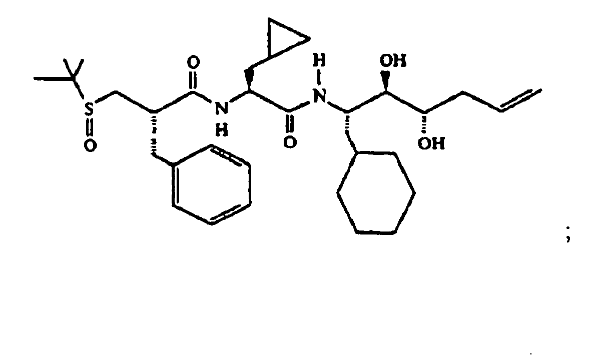 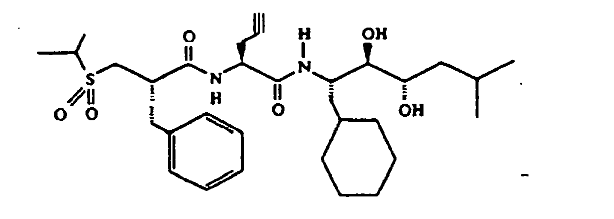 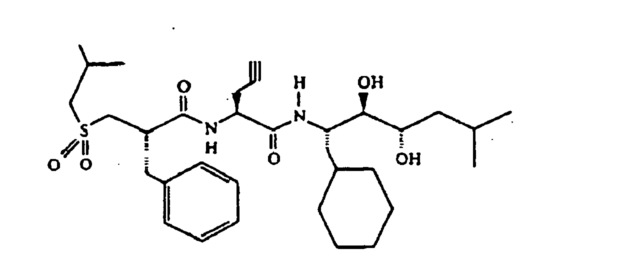 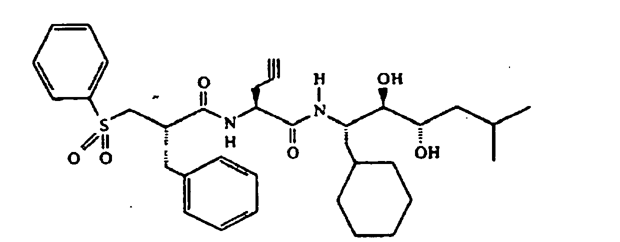 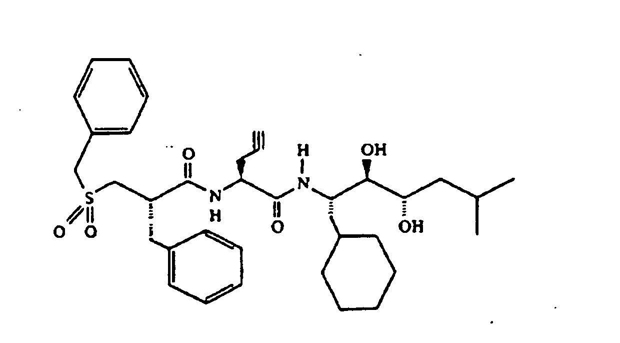 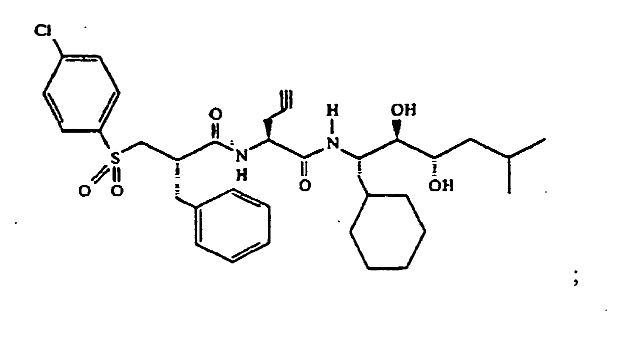 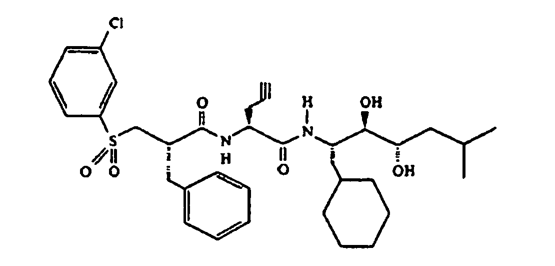 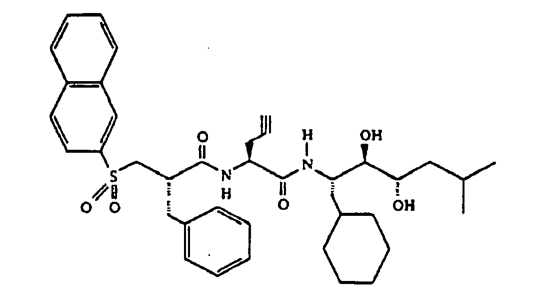 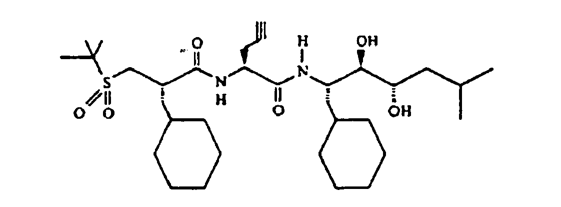 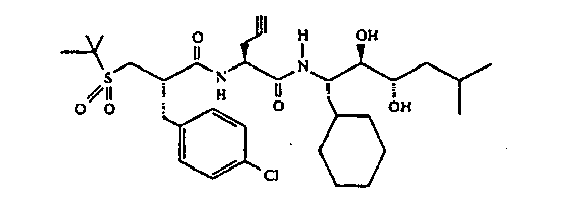 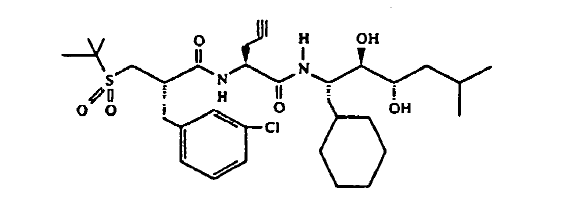 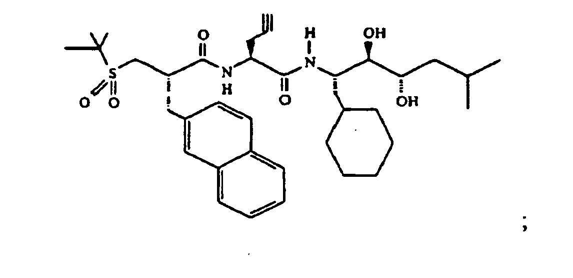 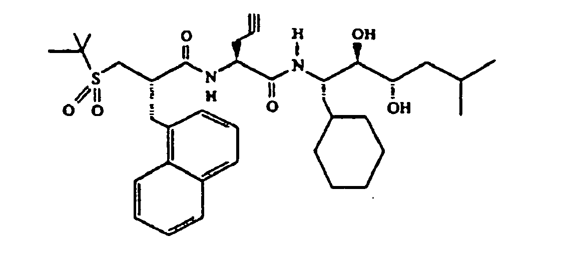 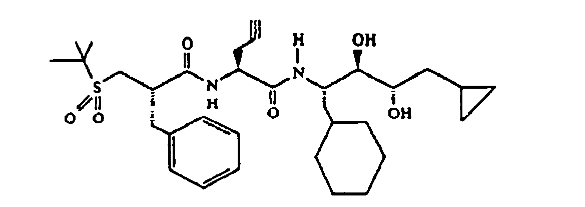 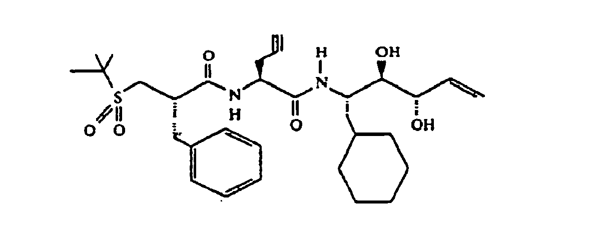 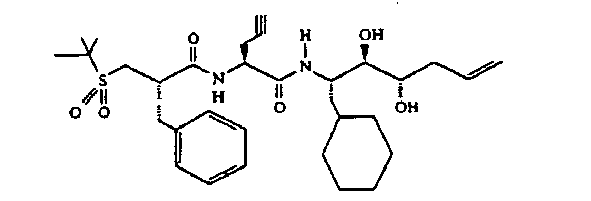 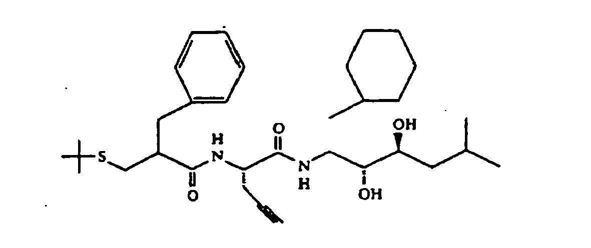 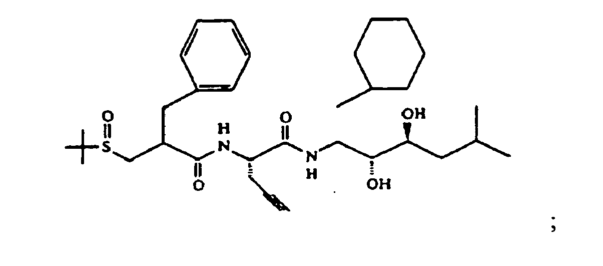 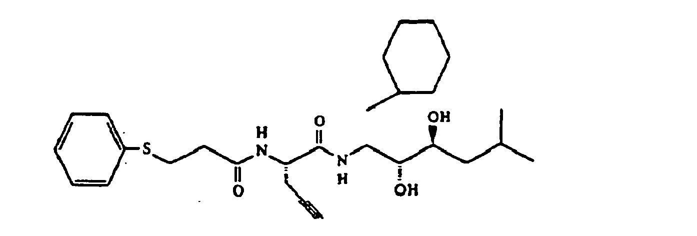 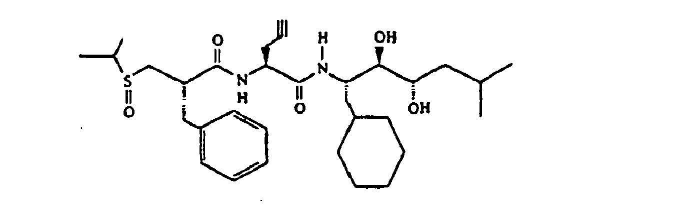 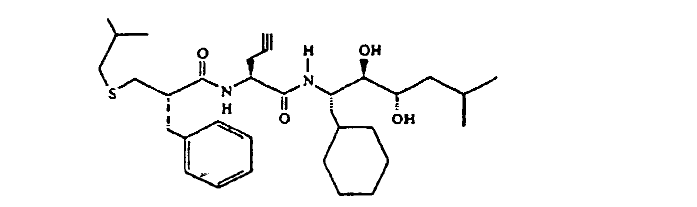 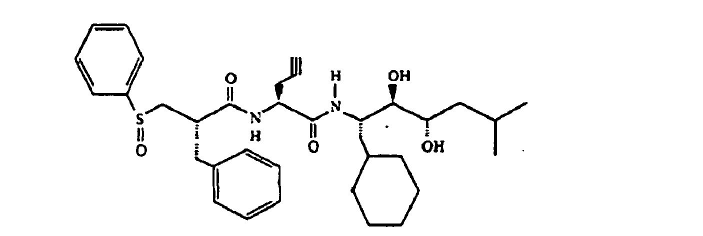 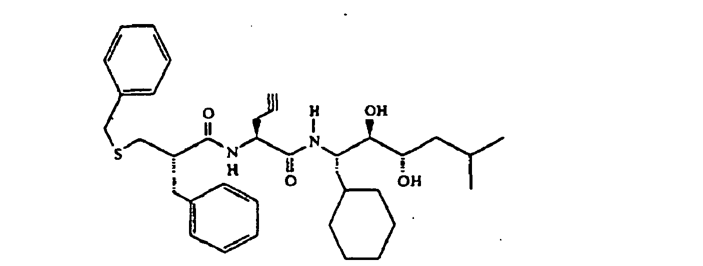 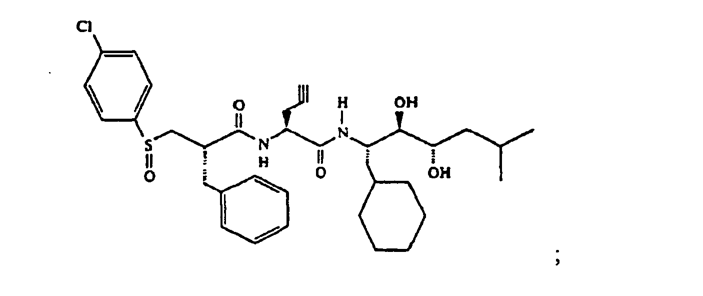 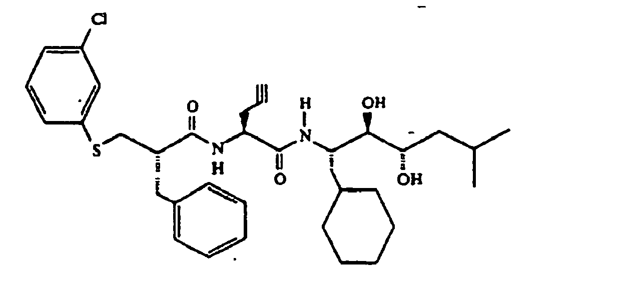 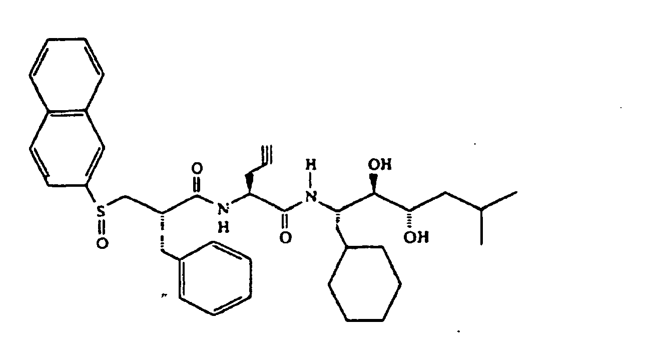 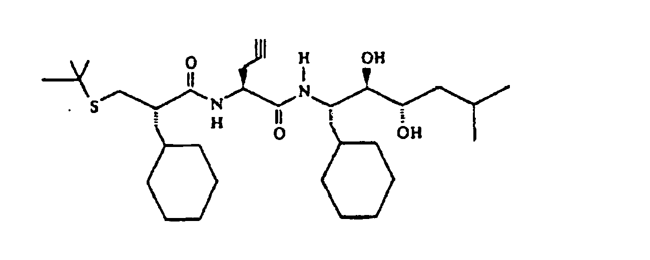 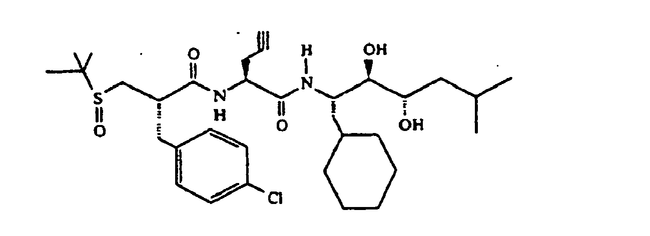 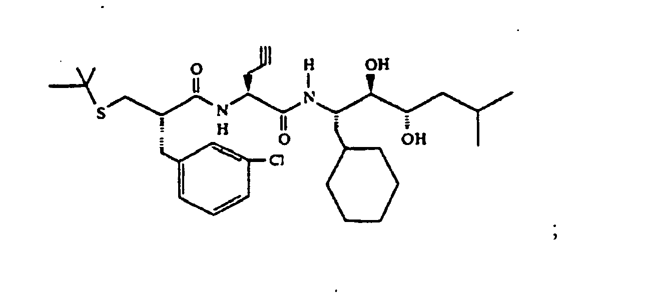 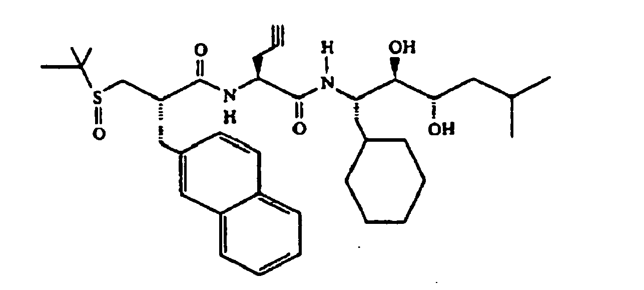 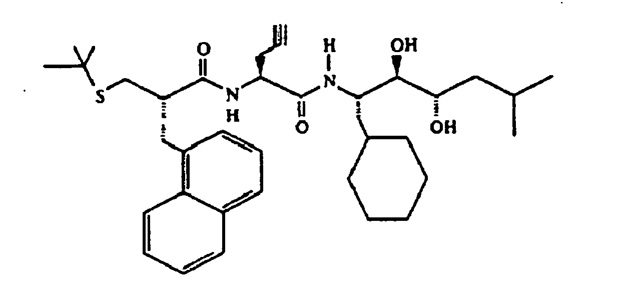 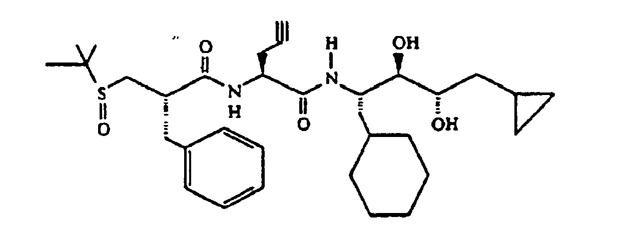 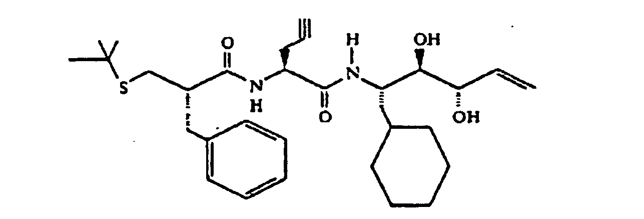 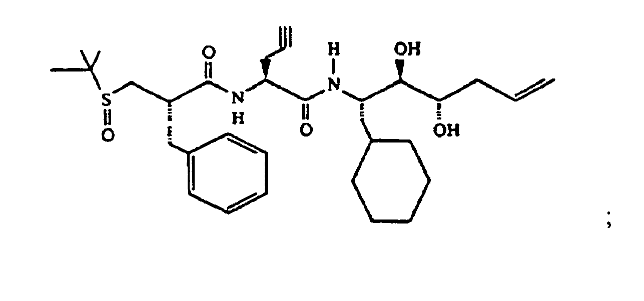 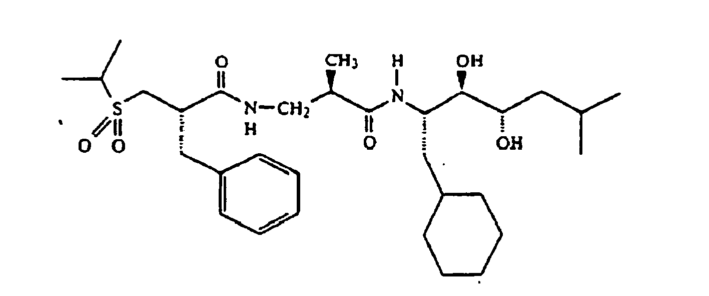 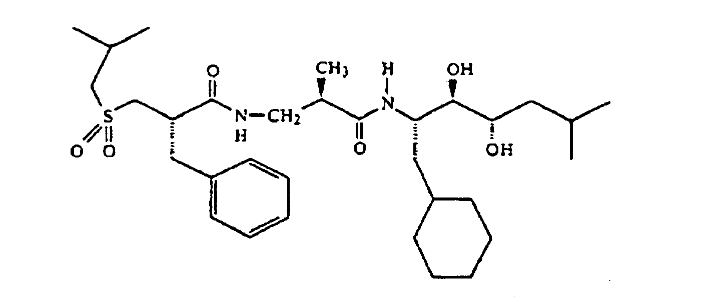 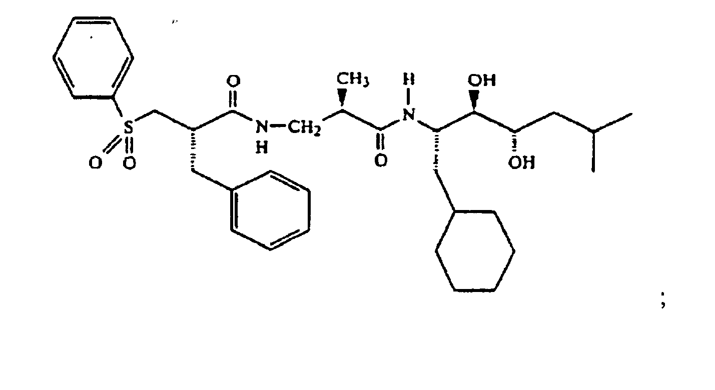 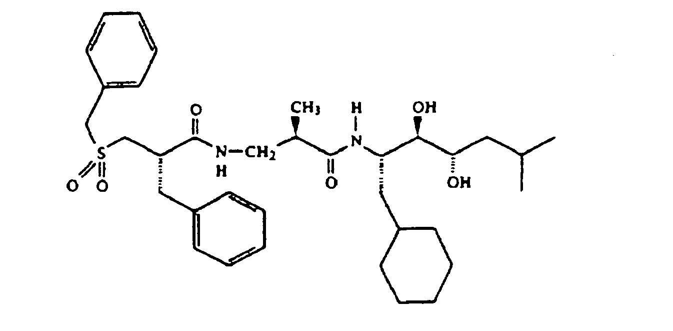 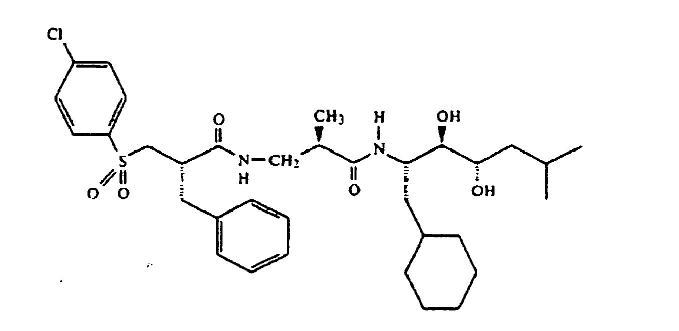 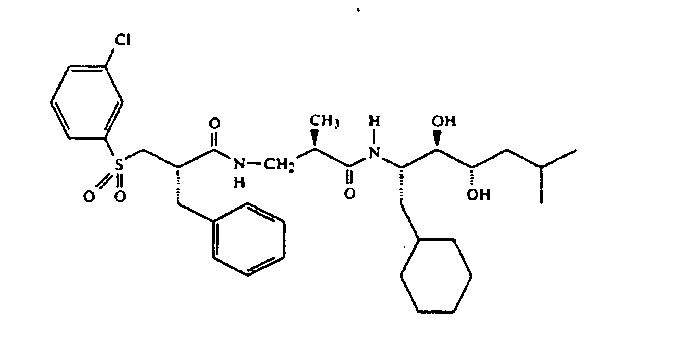 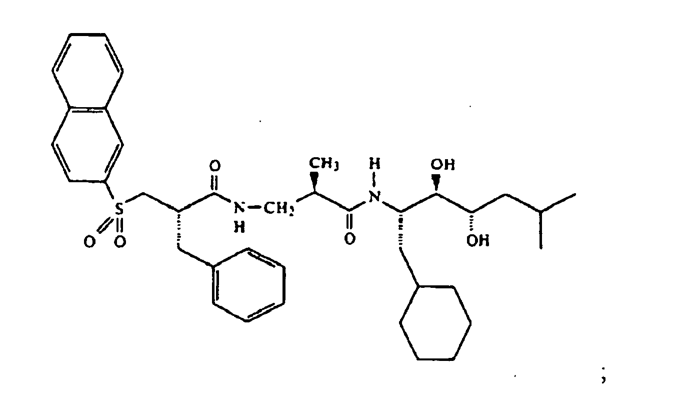 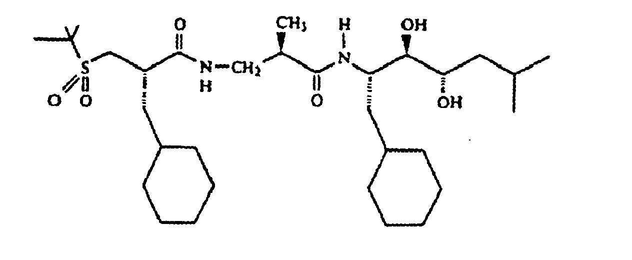 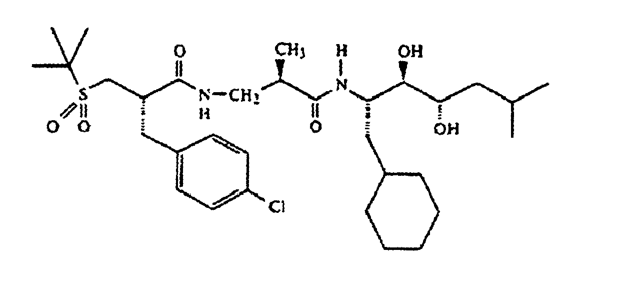 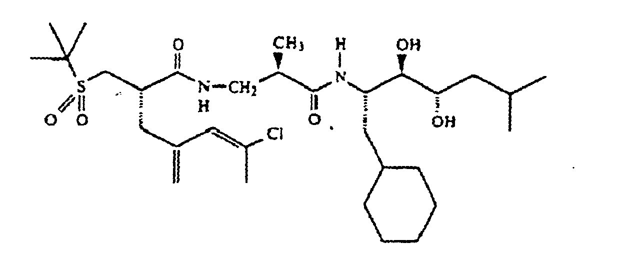 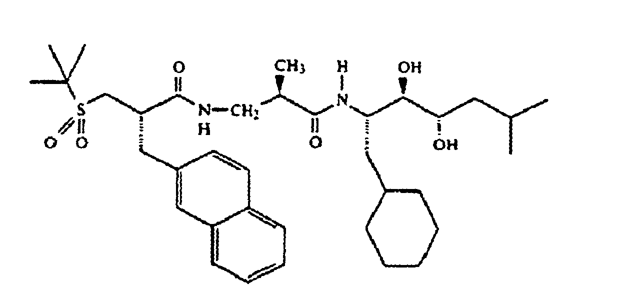 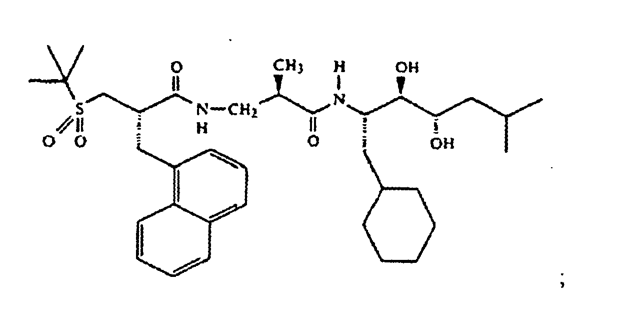 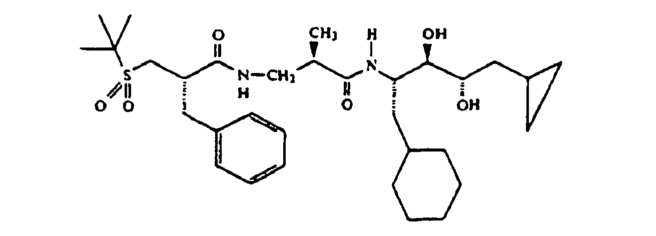 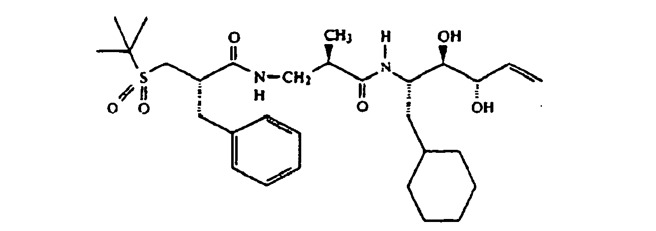 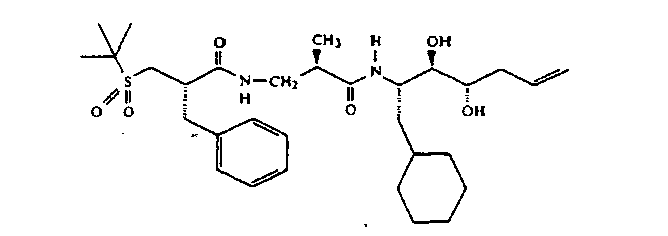 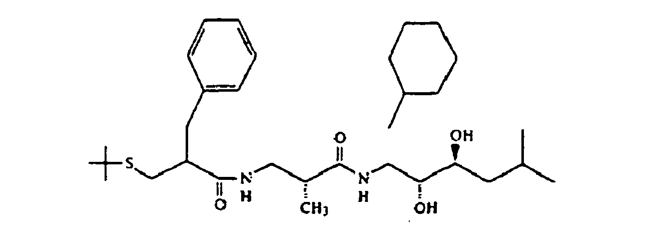 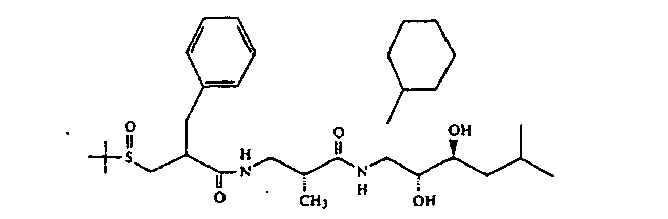 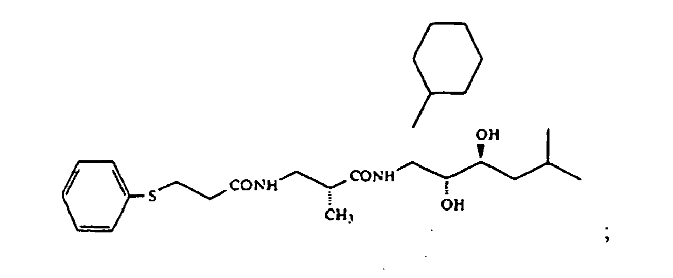 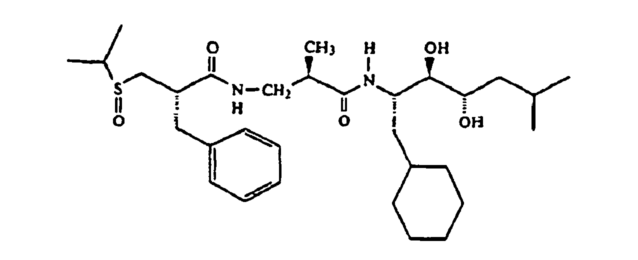 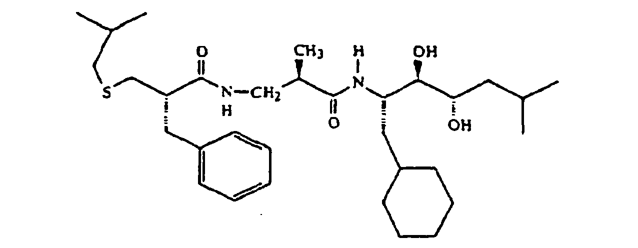 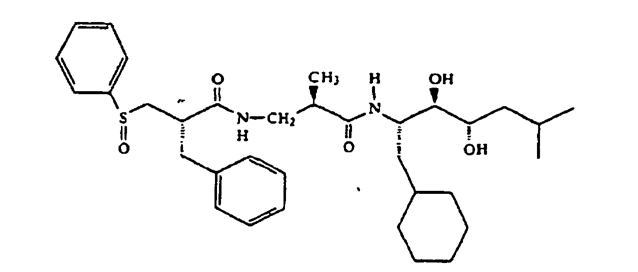 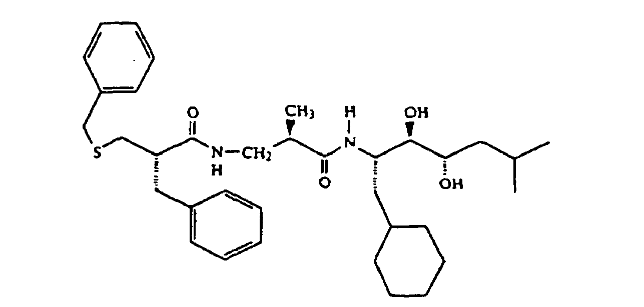 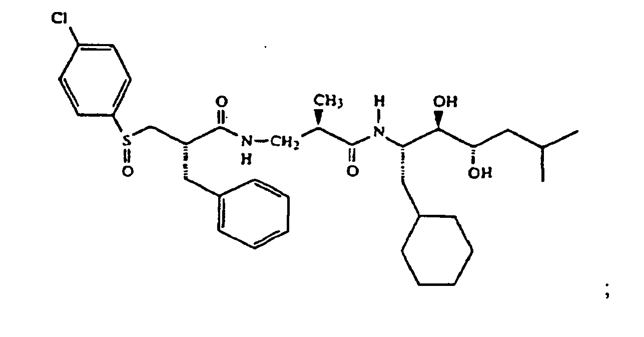 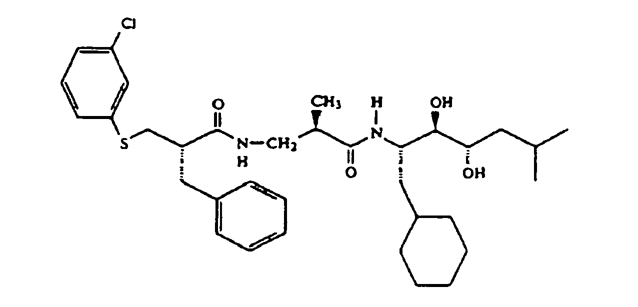 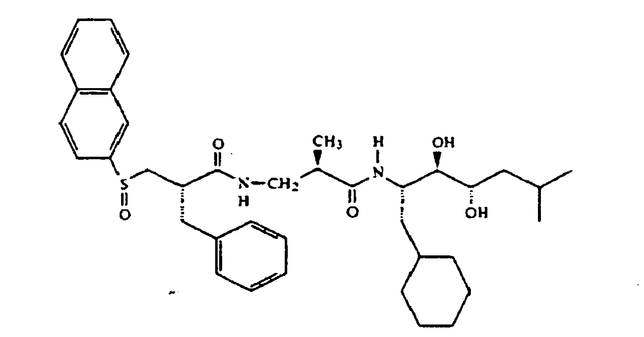 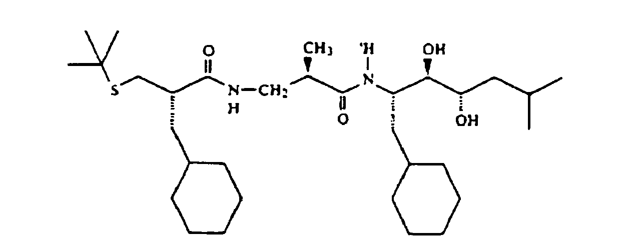 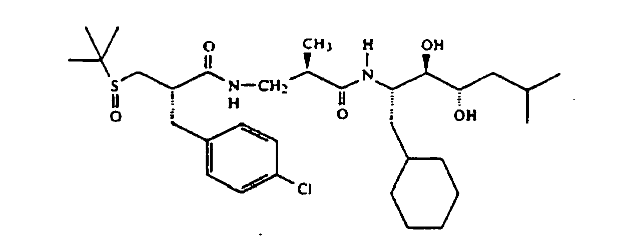 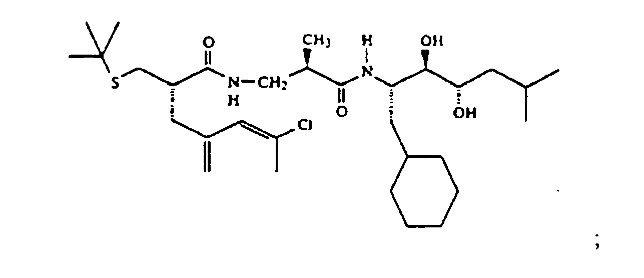 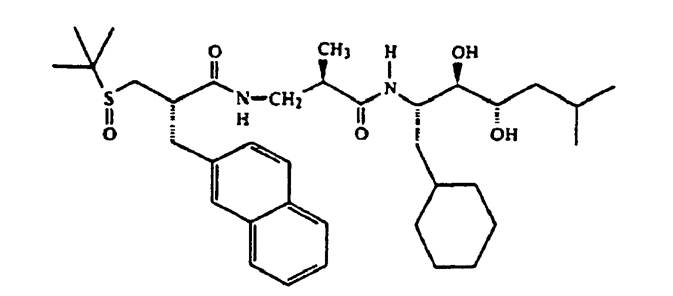 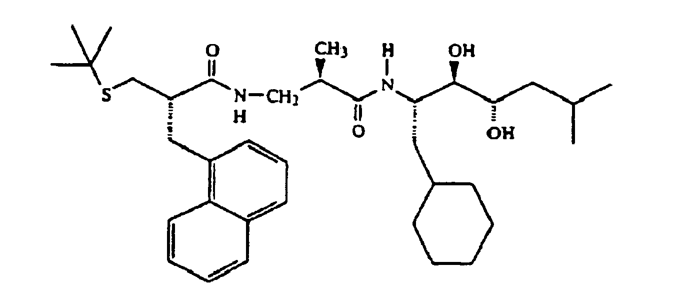 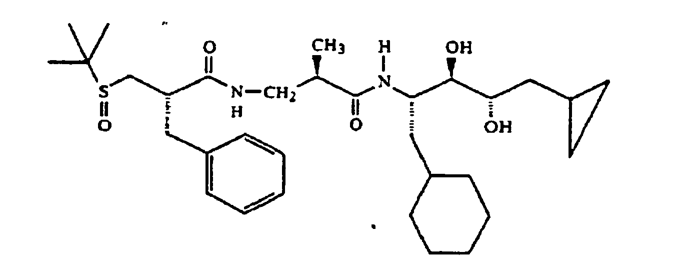 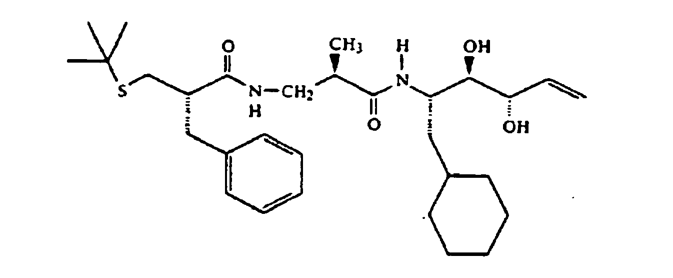 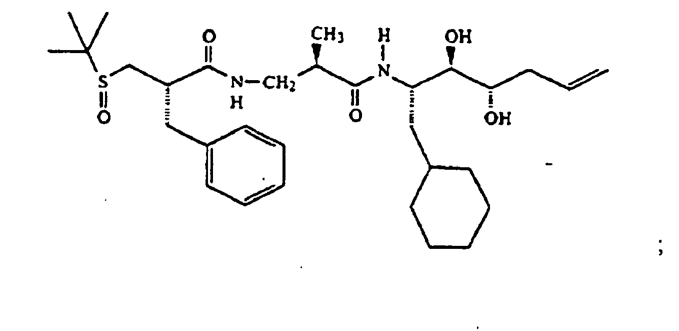 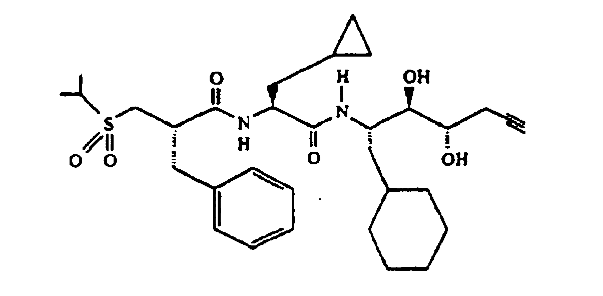 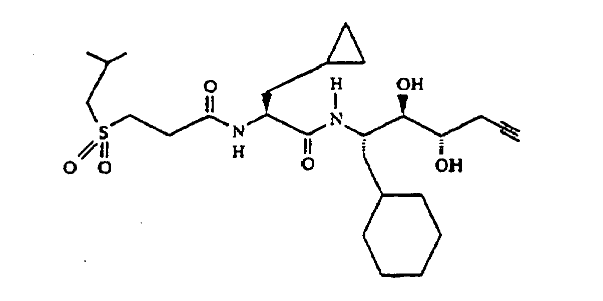 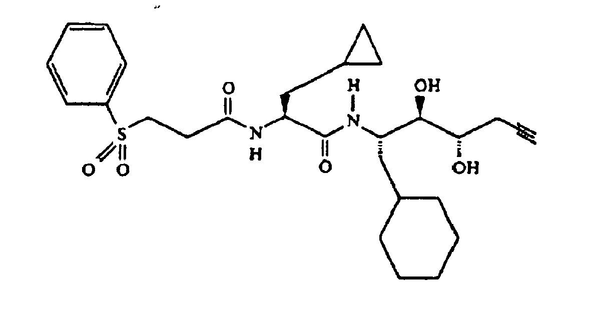 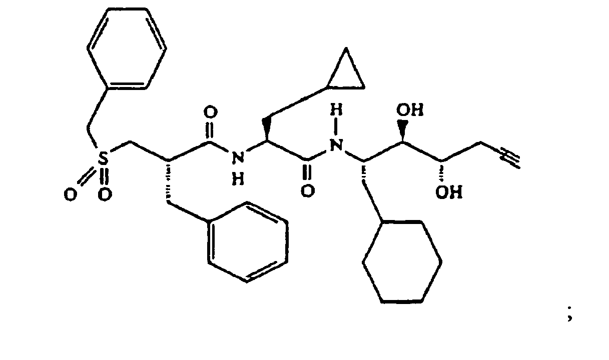 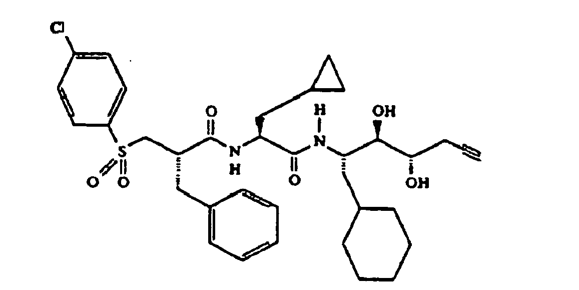 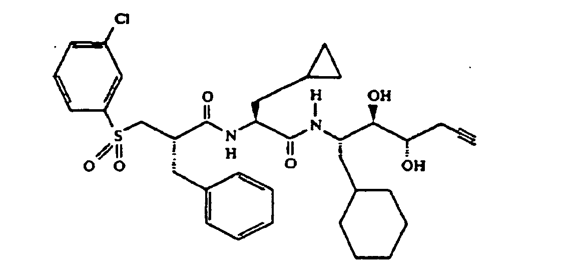 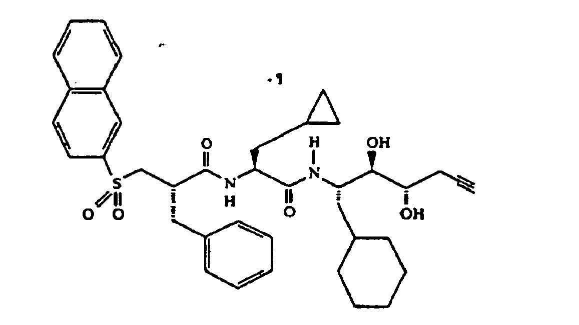 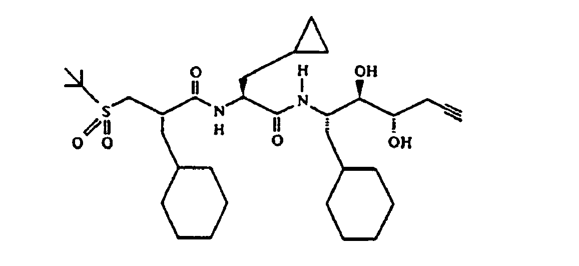 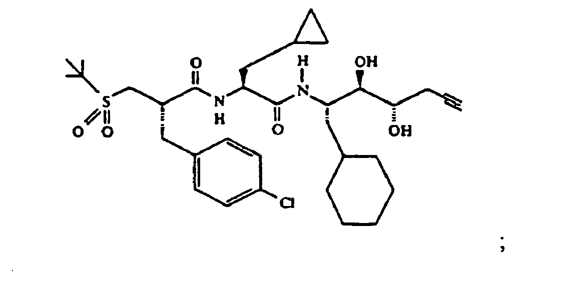 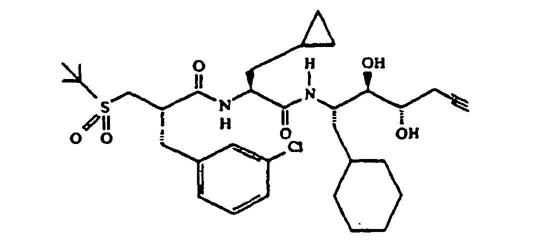 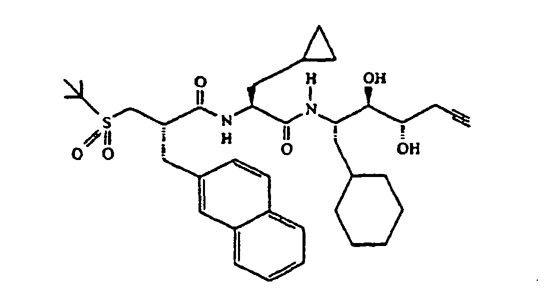 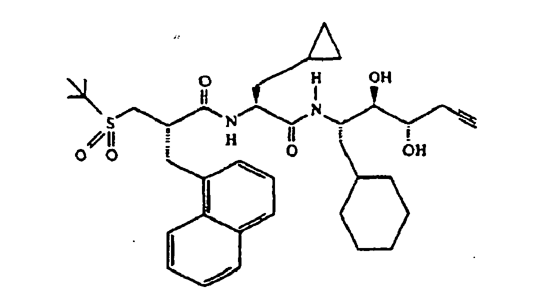 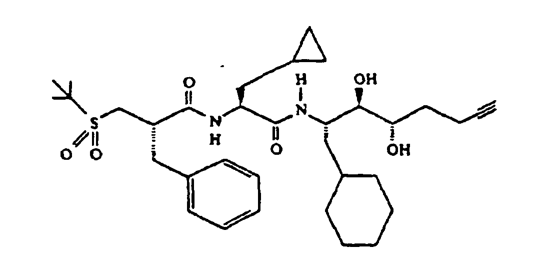 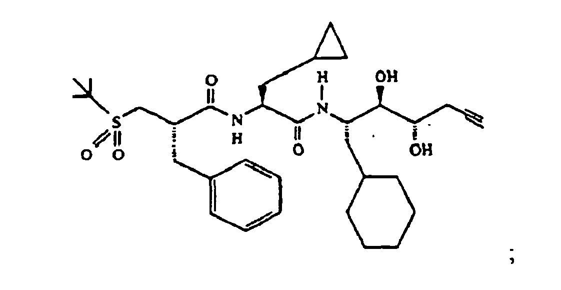 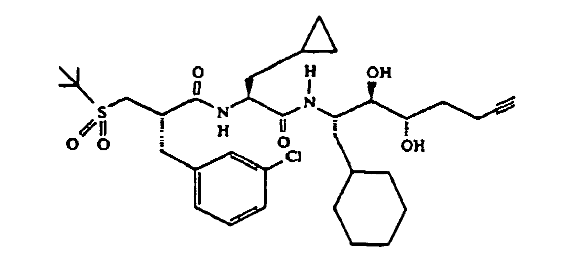 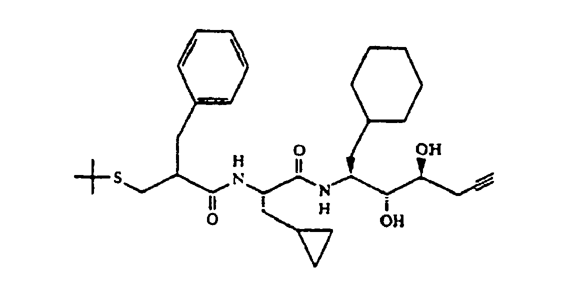 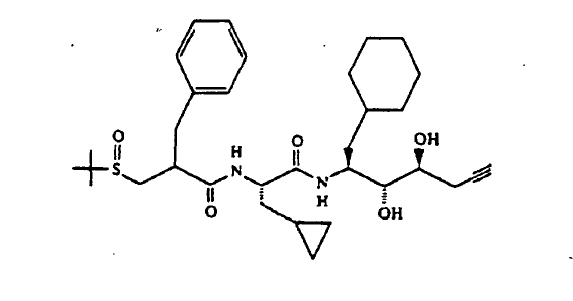 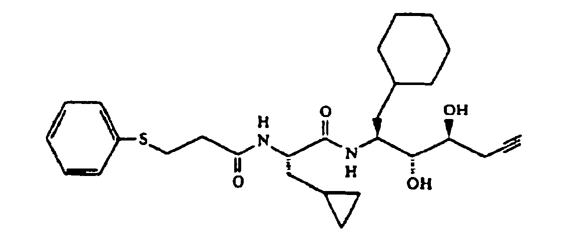 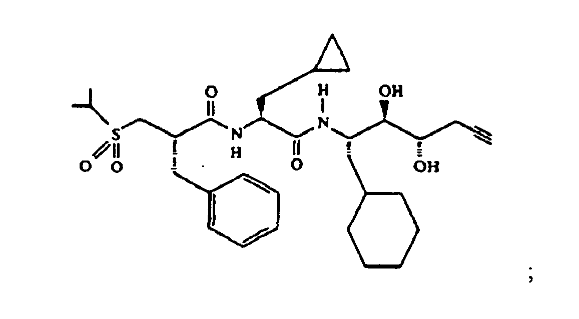 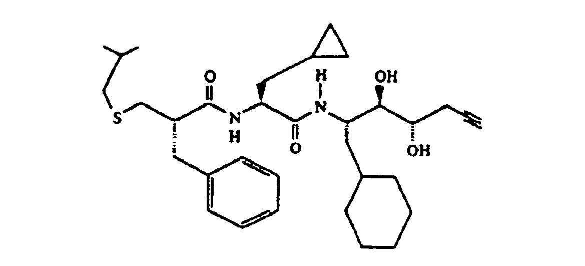 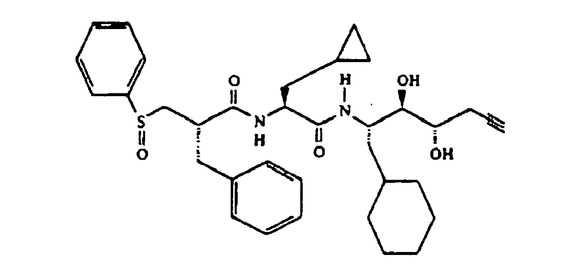 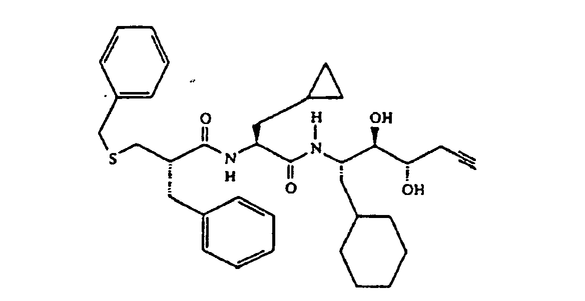 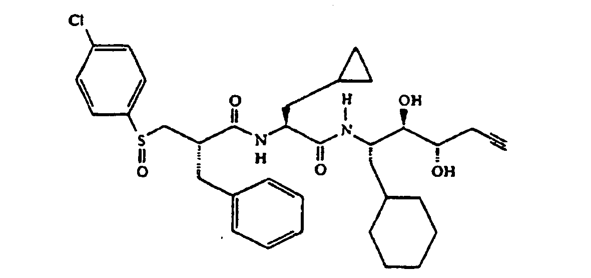 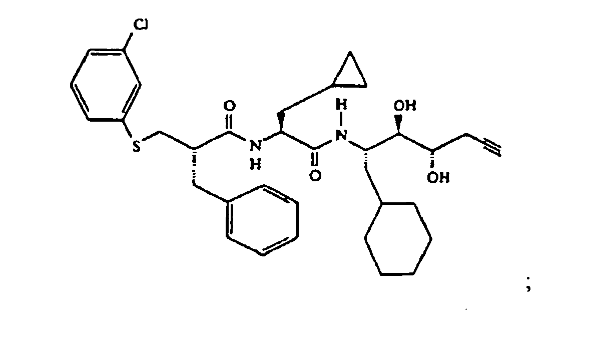 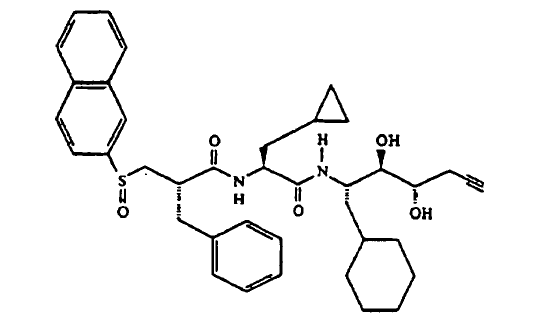 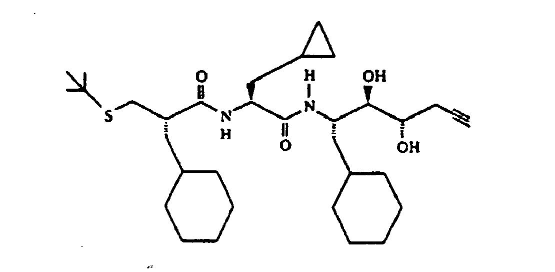 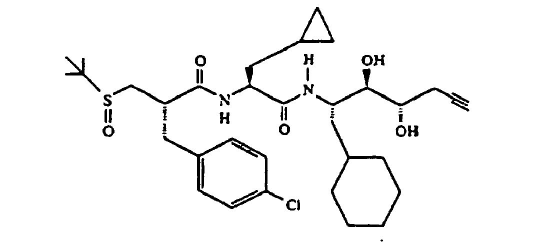 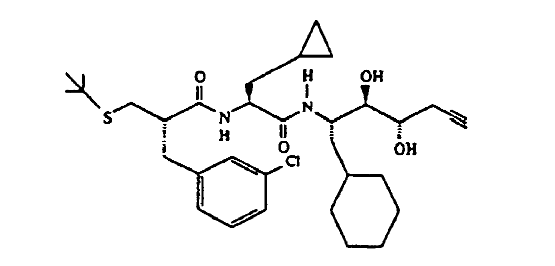 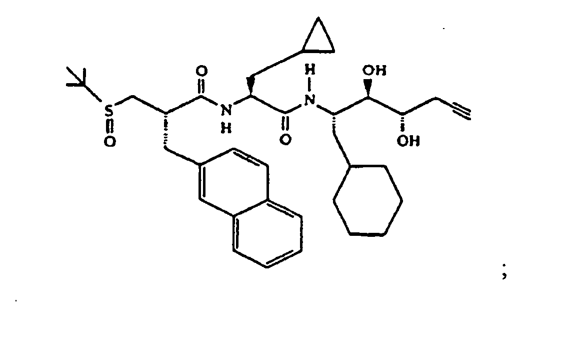 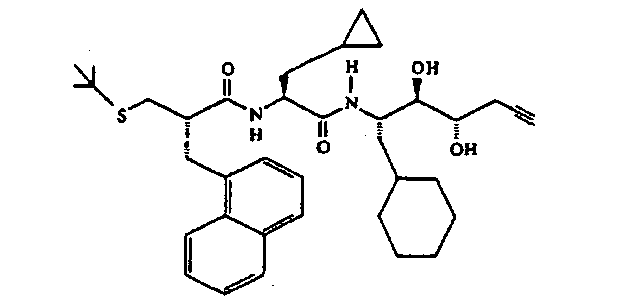 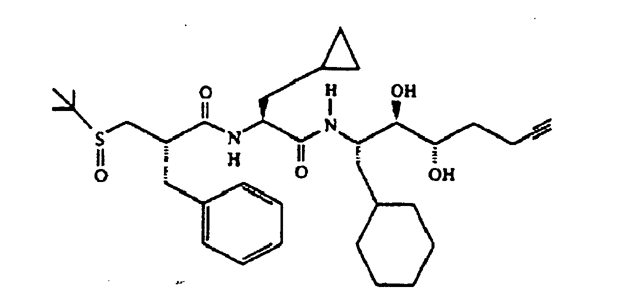 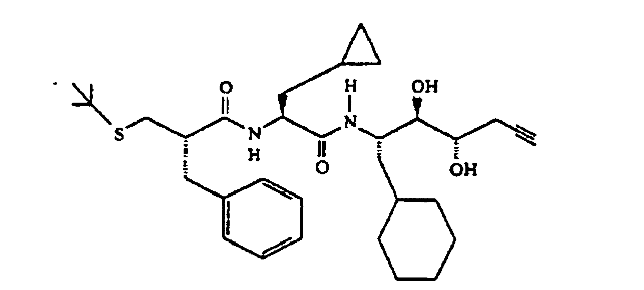 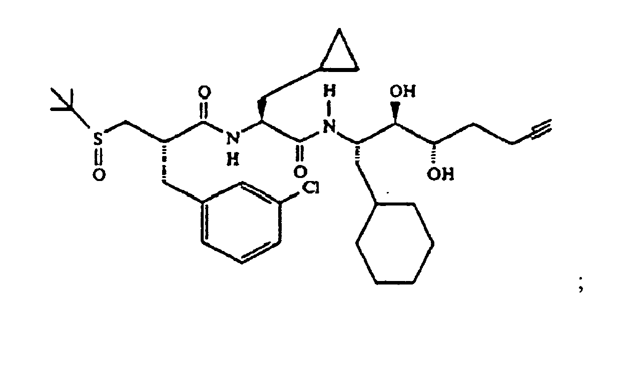 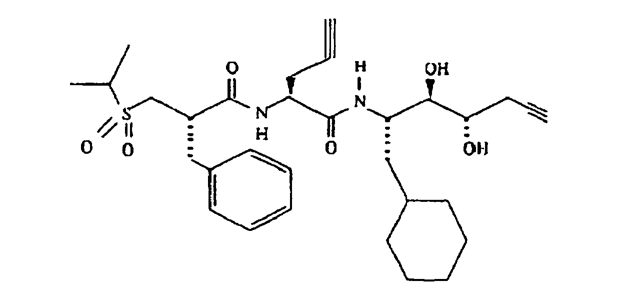 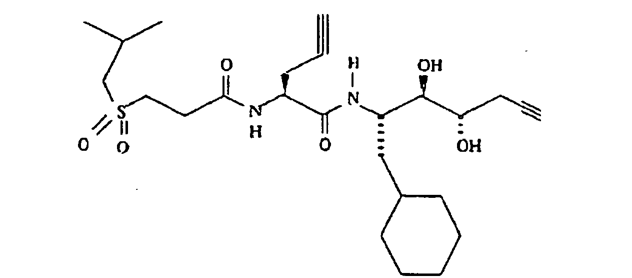 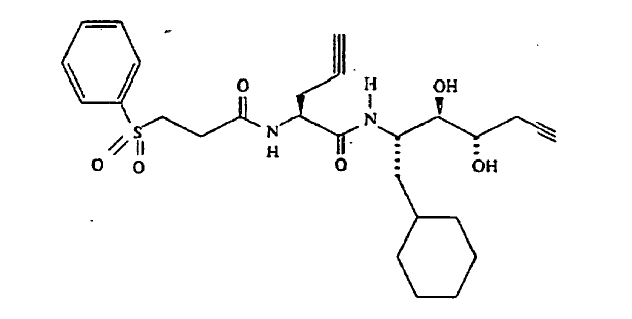 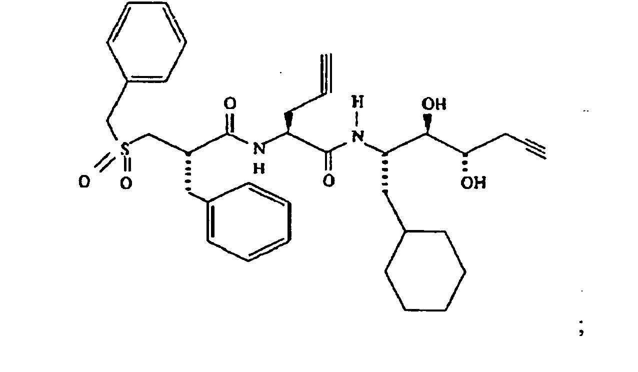 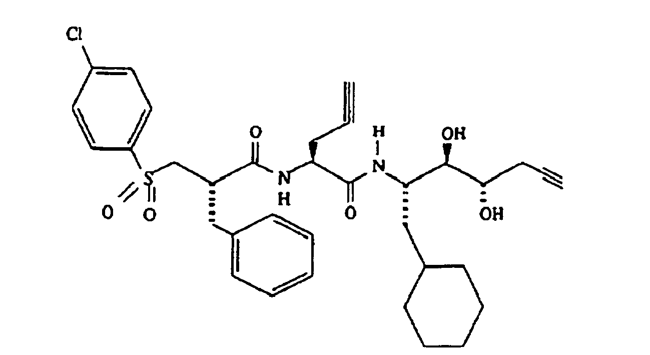 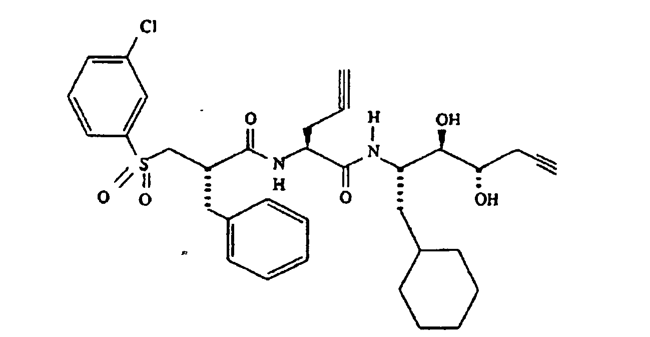 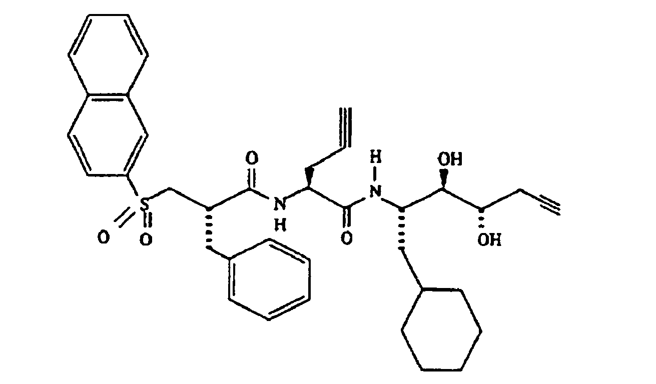 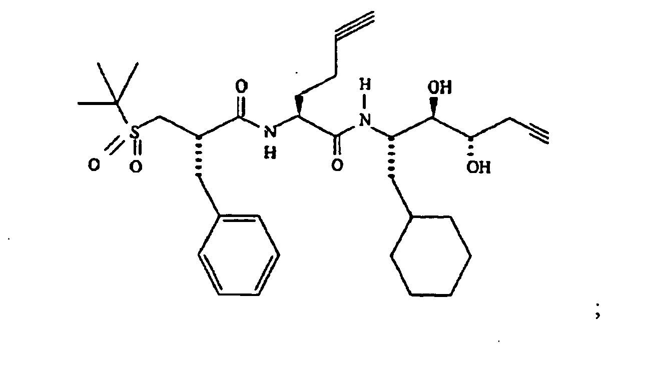 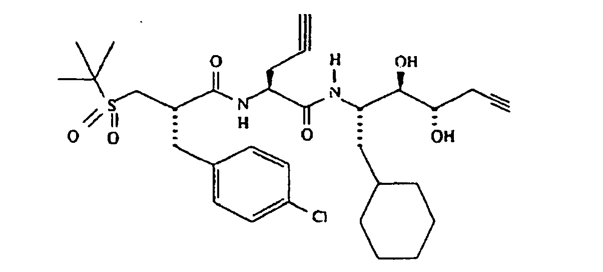 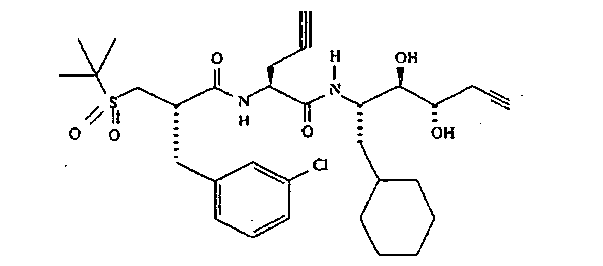 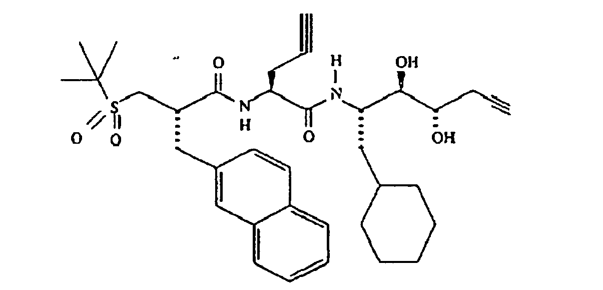 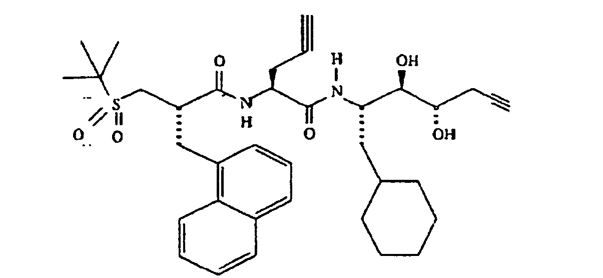 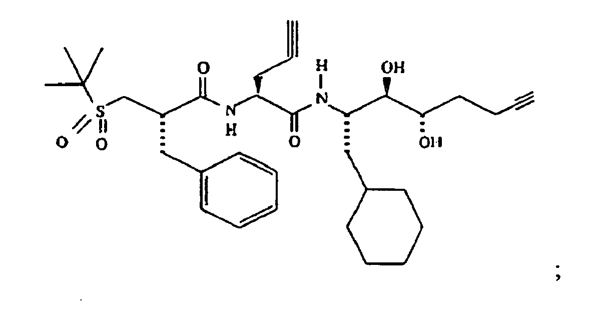 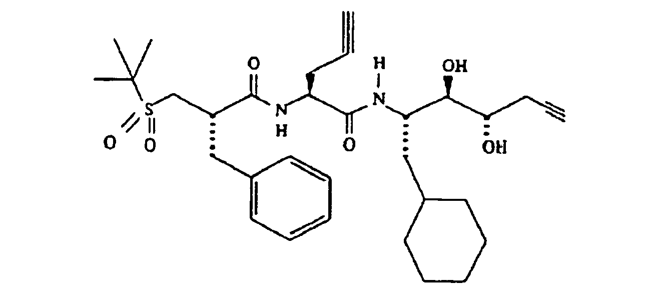 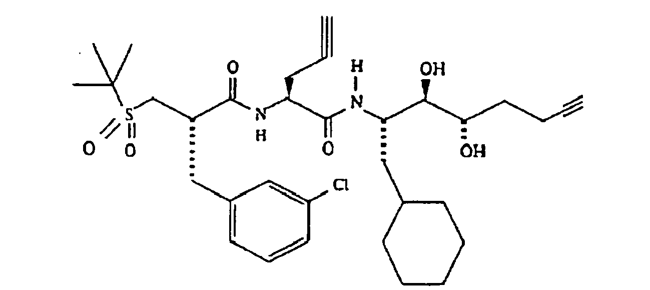 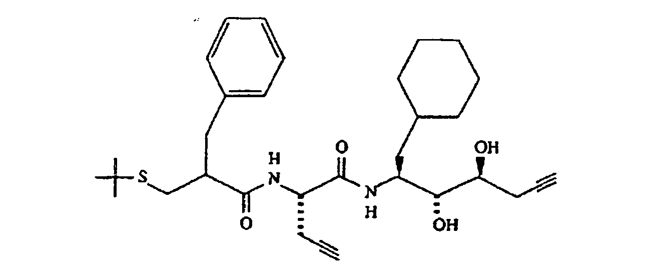 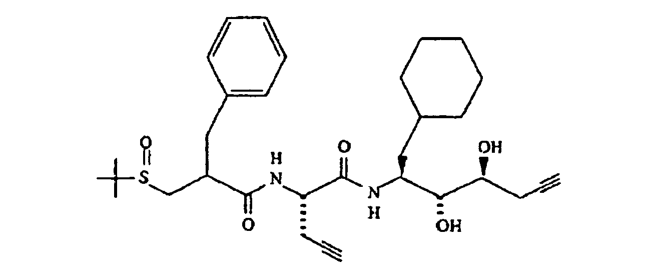 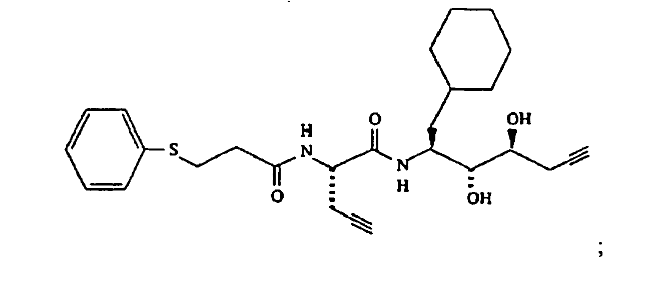 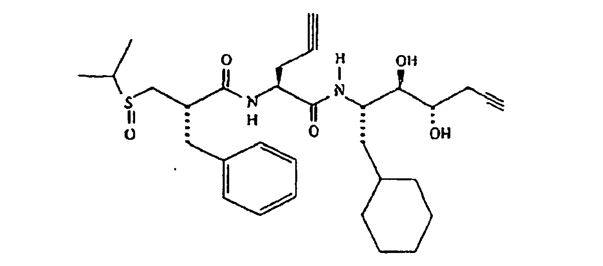 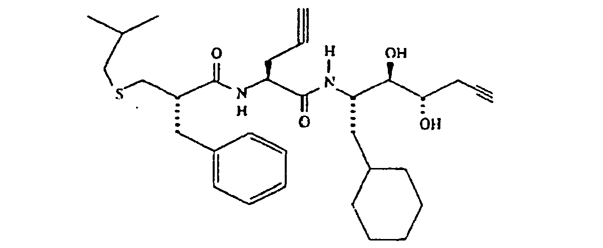 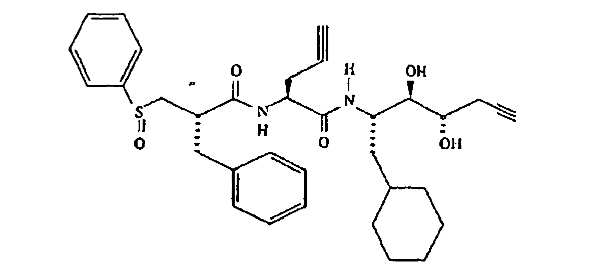 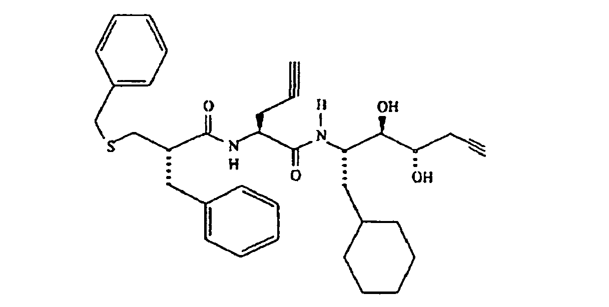 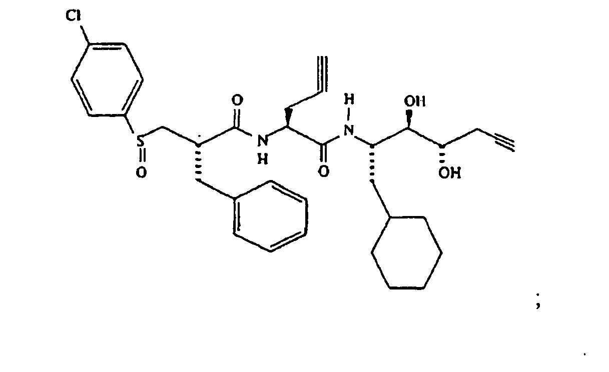 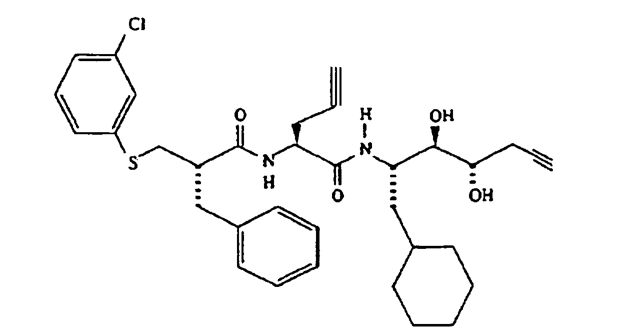 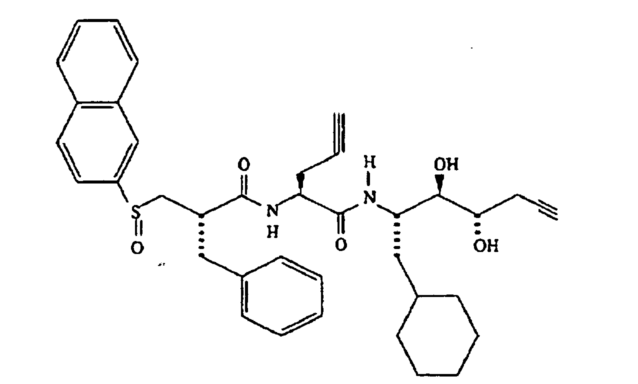 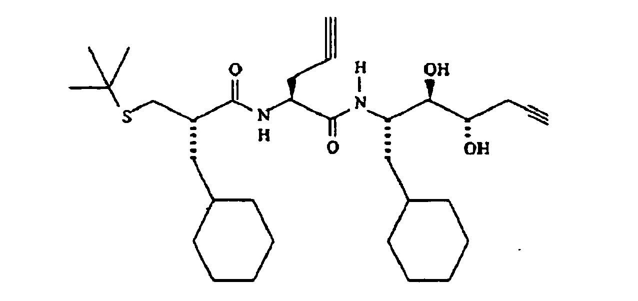 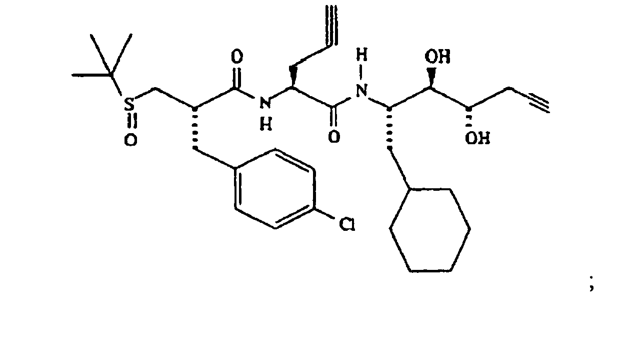 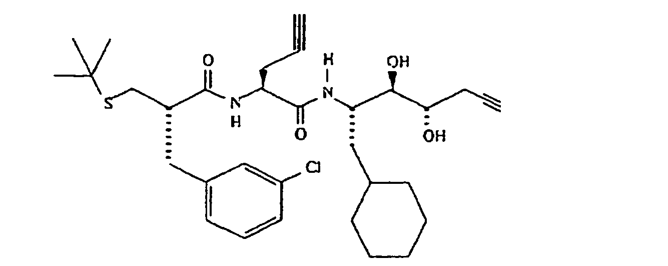 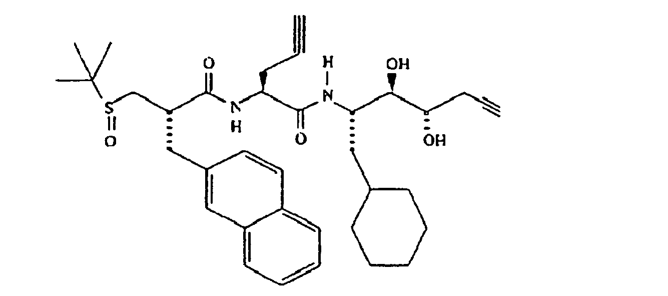 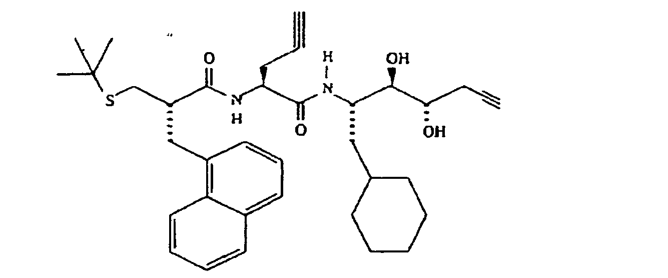 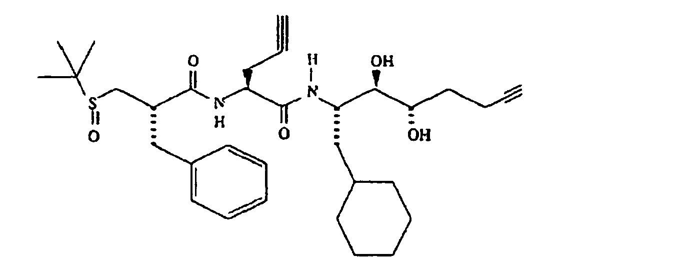 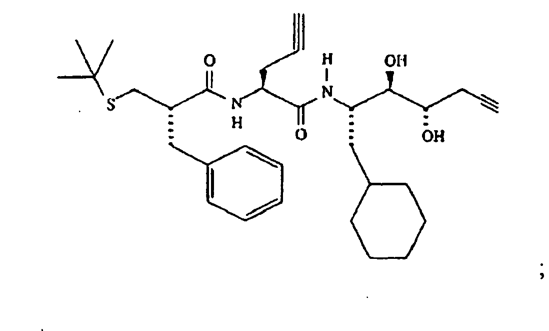 and 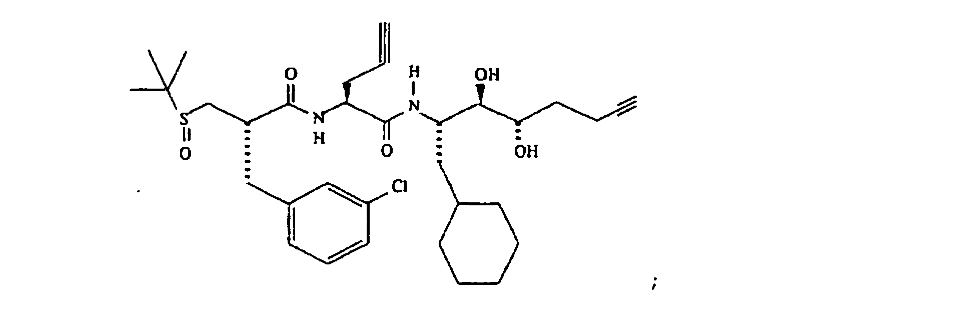
or a pharmaceutically acceptable salts thereof.

16. A method of treating or preventing Alzheimer's disease in a subject in need of such treatment comprising administering a therapeutically effective amount of a composition comprising one or more pharmaceutically acceptable carriers and a compound of Formula (I), (II), (III), (IV), (V), (VI), (VII), (VIII), (IX), or (X) or a pharmaceutically acceptable salt thereof: 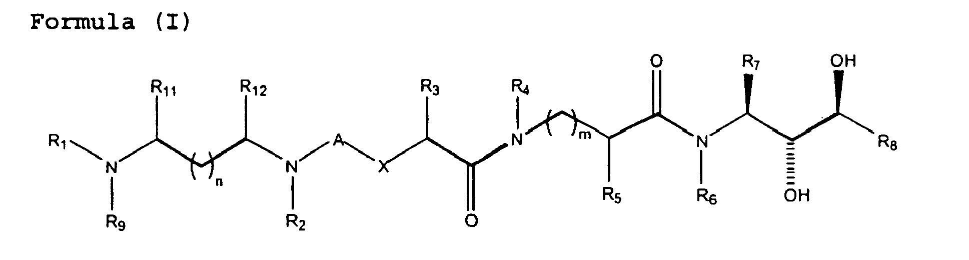 wherein A is selected from methylene, CO, SO, and SO₂;
wherein X is selected from oxygen atom, methylene and 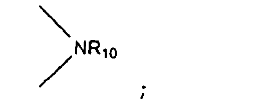 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl;
R₅ is selected from 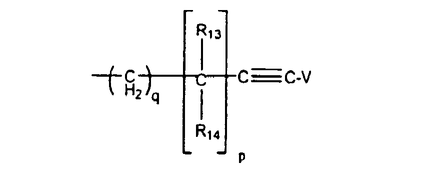 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 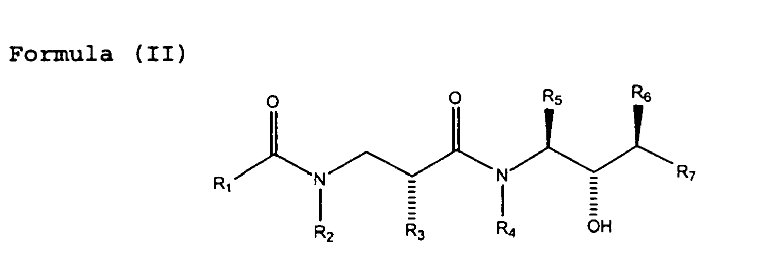 wherein R₁ is selected from aryl, aralkyl, heteroaryl and heteroaralkyl, including the following: 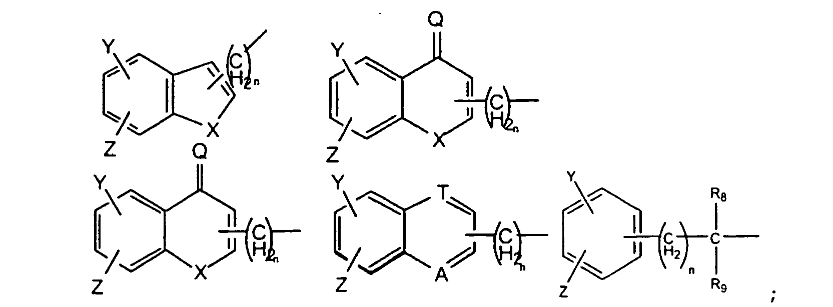 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 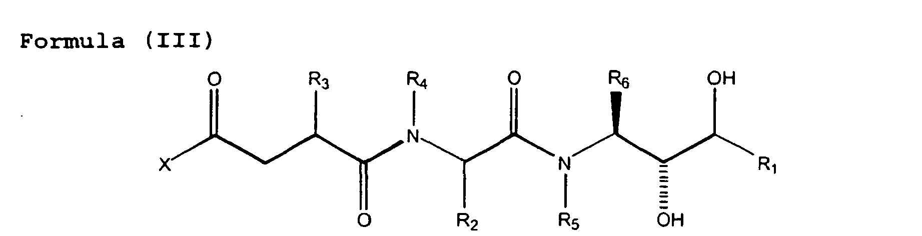 wherein X is selected from 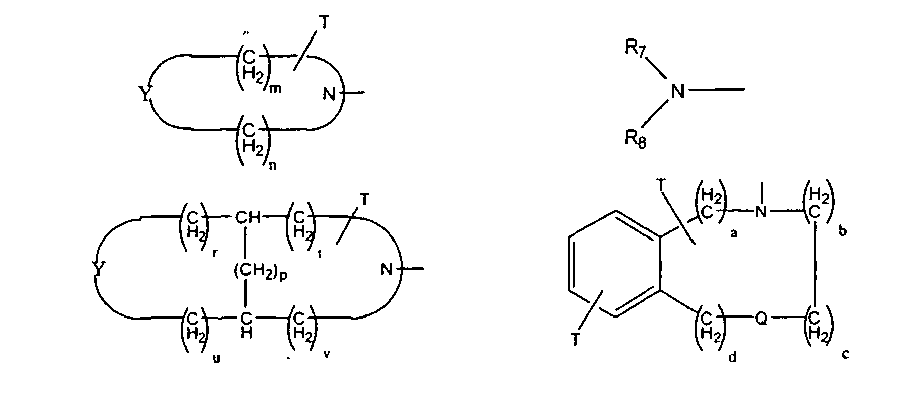 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 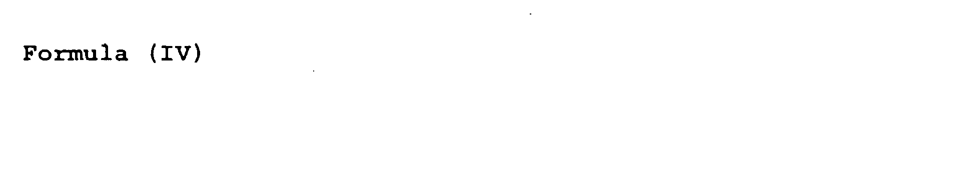 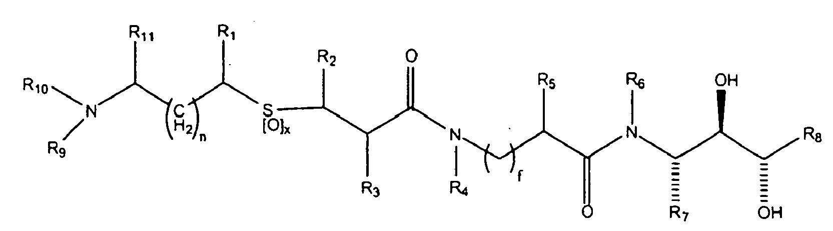 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 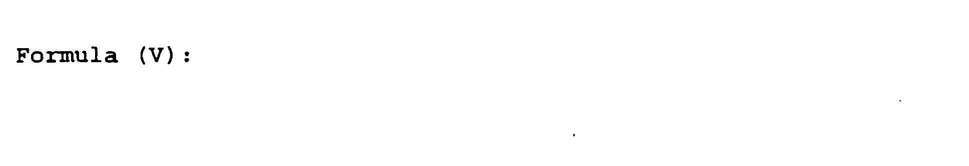 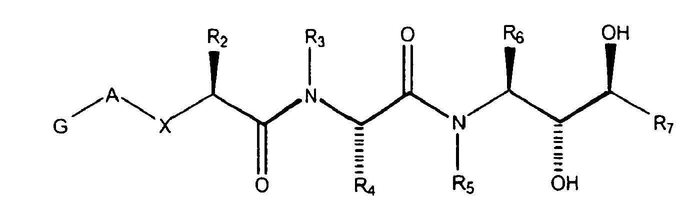 wherein A is selected from CO and SO₂;
X is selected form oxygen atom and methylene;
G is selected from B or 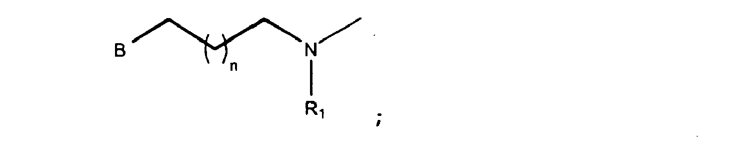 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 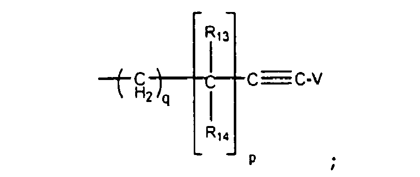 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 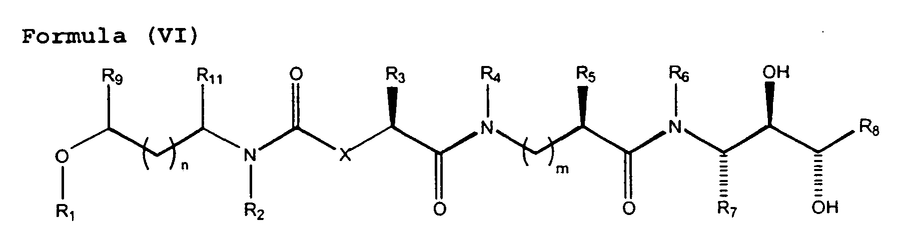 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals
selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 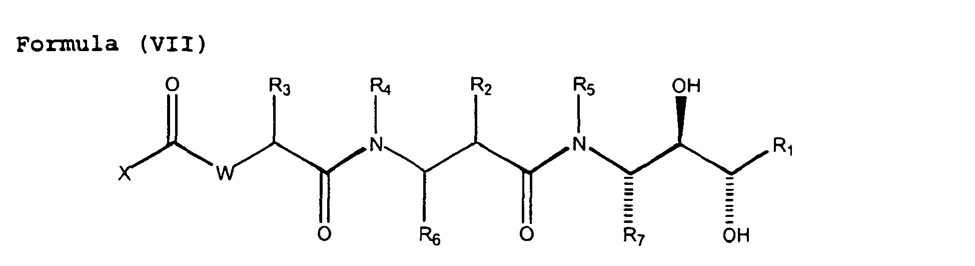 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 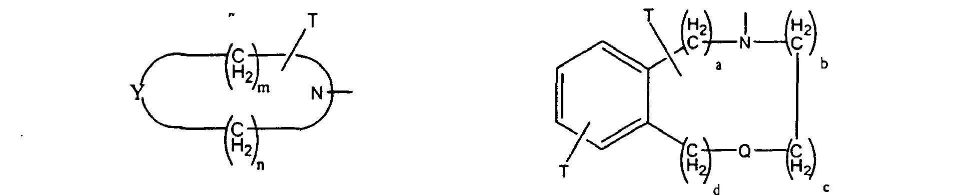 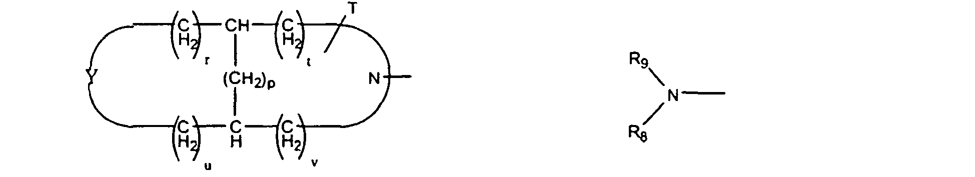 wherein Y and Q are selected from CH₂, CH-O-R₁₀, O, S, SO, SO₂ and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 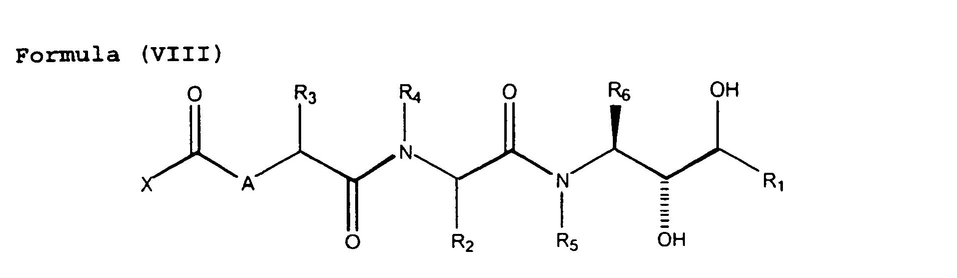 wherein X is selected from 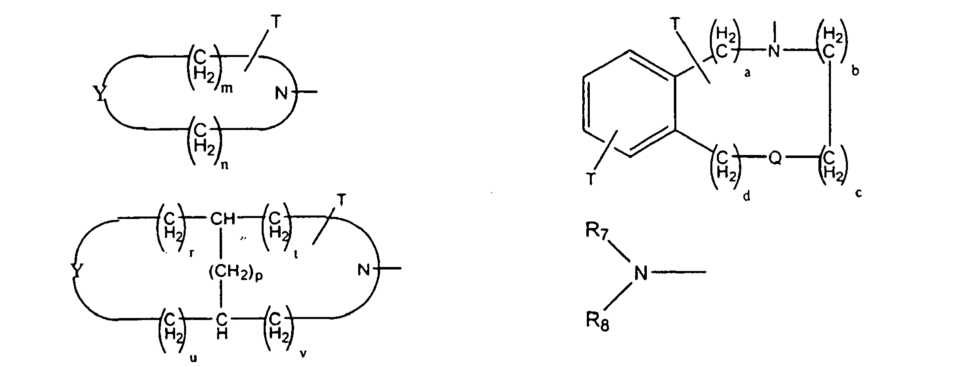 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 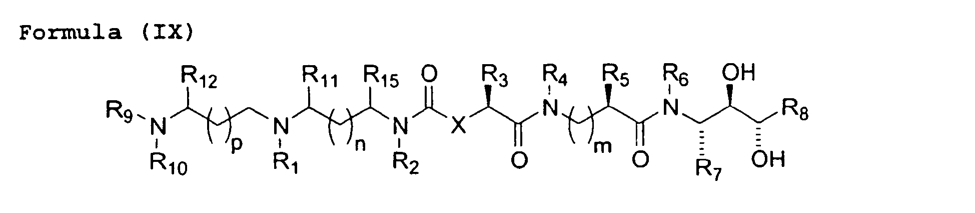 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH(OH)R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5 ;or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 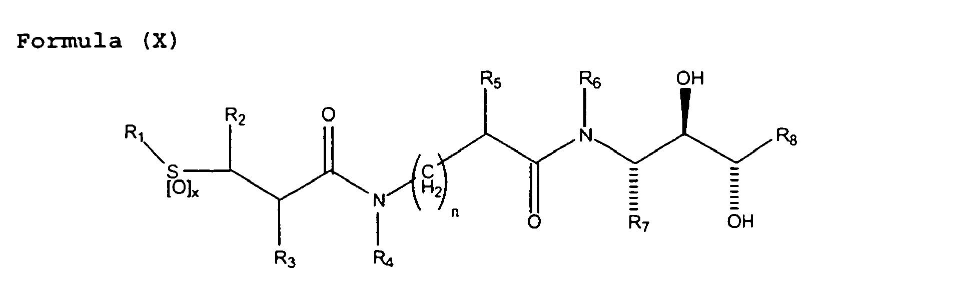 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl, or pharmaceutically-acceptable salts thereof.

17. Use of a compound of Formula (I), (II), (III), (IV), (V), (VI), (VII), (VIII), (IX), or (X) in the manufacture of a medicament for the treatment or prevention of conditions selected from the group consisting of Alzheimer's disease, mild cognitive impairment (MCI) Down's syndrome, Hereditary Cerebral Hemorrhage with Amyloidosis of the Dutch-Type, cerebral amyloid angiopathy, degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease: 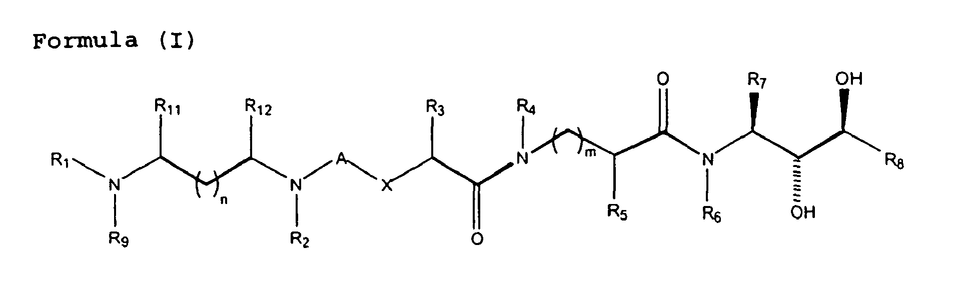 wherein A is selected from methylene, CO, SO, and SO₂;
wherein X is selected from oxygen atom, methylene and 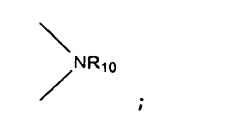 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl;
R₅ is selected from 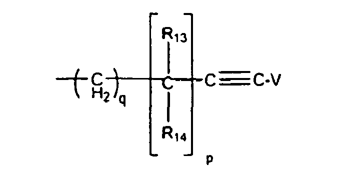 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 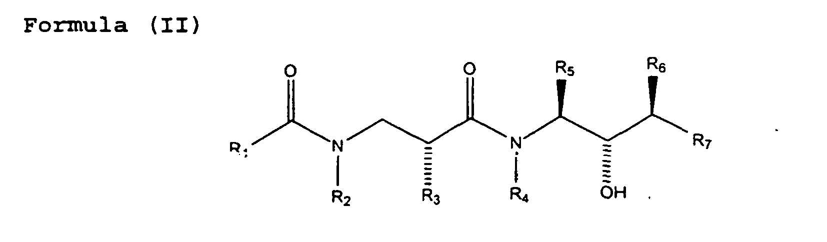 wherein R₁ is selected from aryl, aralkyl, heteroaryl and heteroaralkyl, including the following: 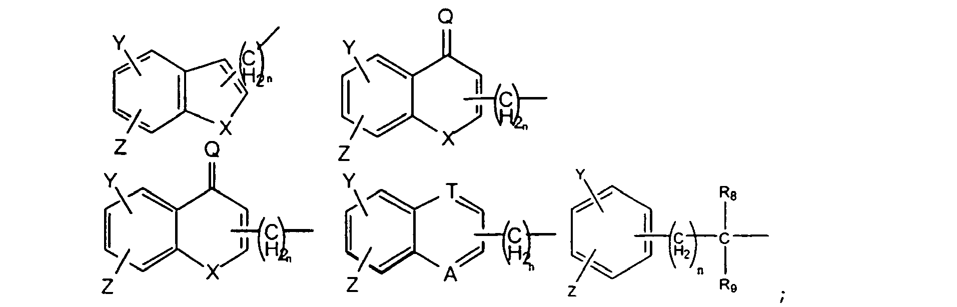 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 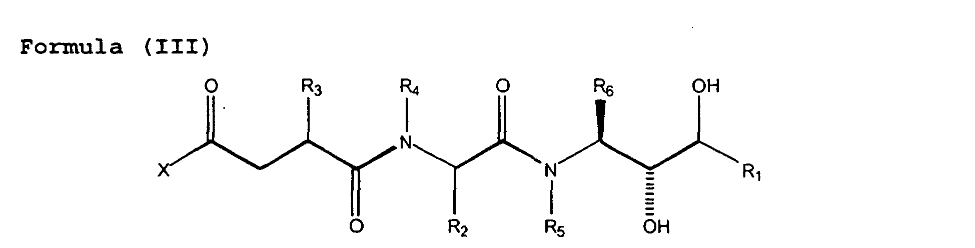 wherein X is selected from 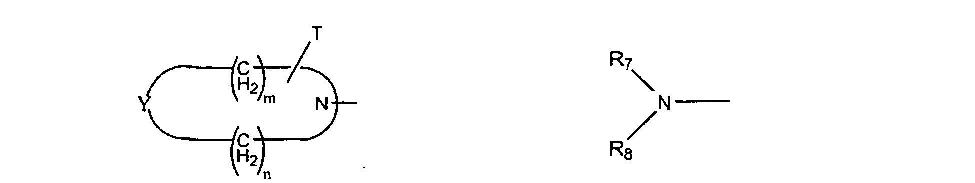 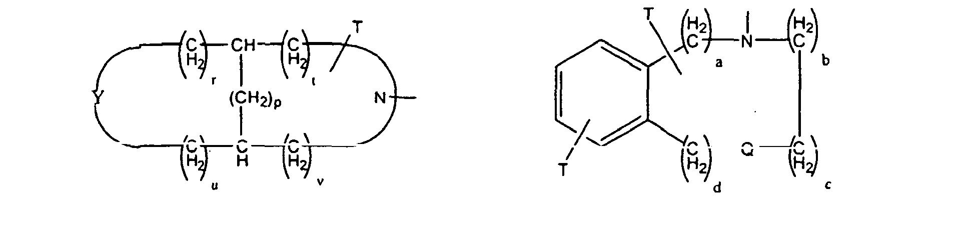 wherein Y and Q are selected from CH₂, CH-O-R₉, O, S, SO, SO₂ and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 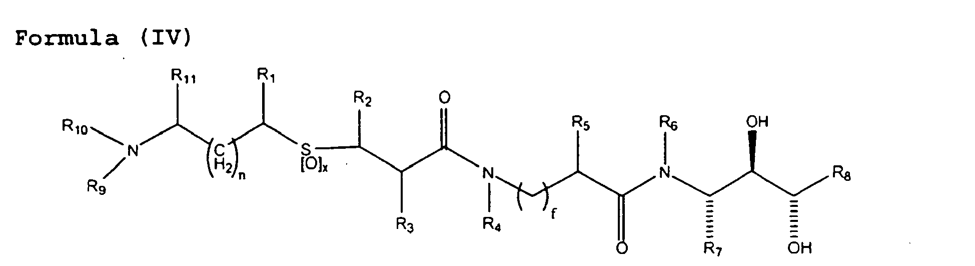 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 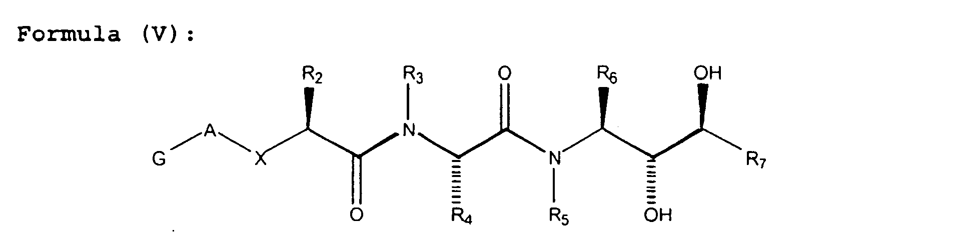 wherein A is selected from CO and SO₂;
X is selected form oxygen atom and methylene;
G is selected from B or 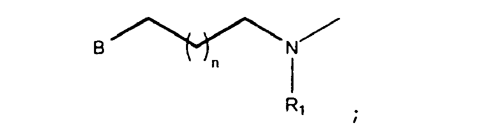 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen co form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 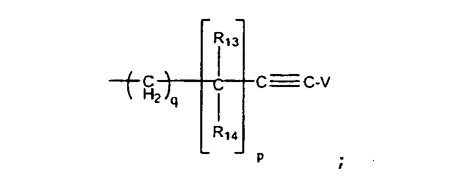 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 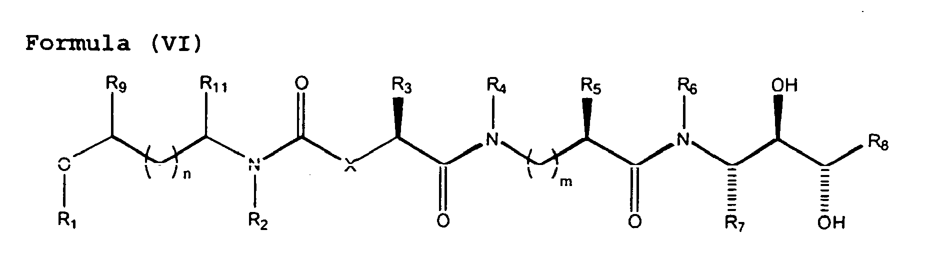 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 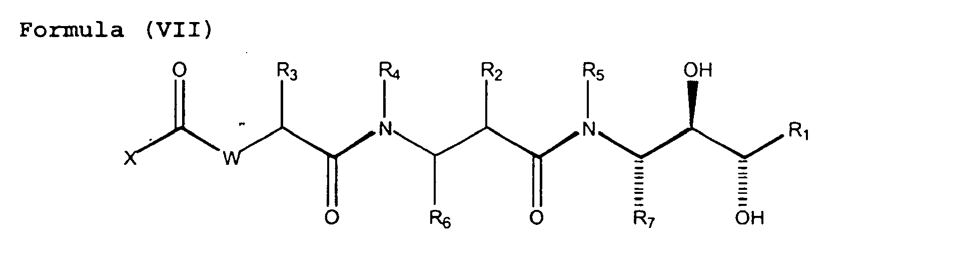 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 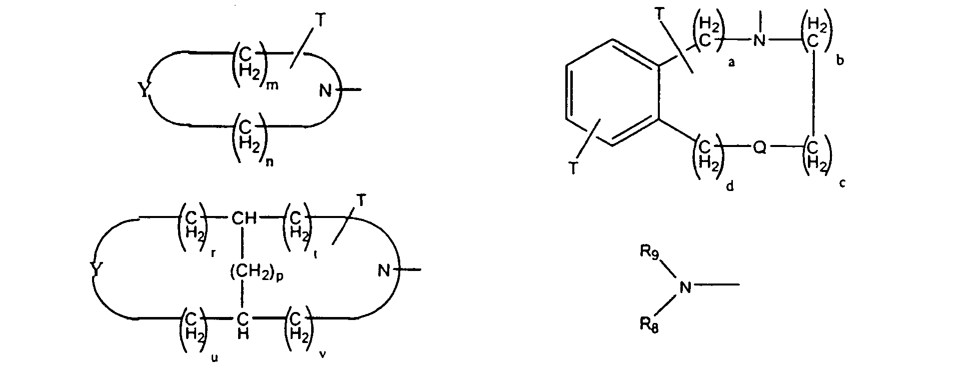 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂ ;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 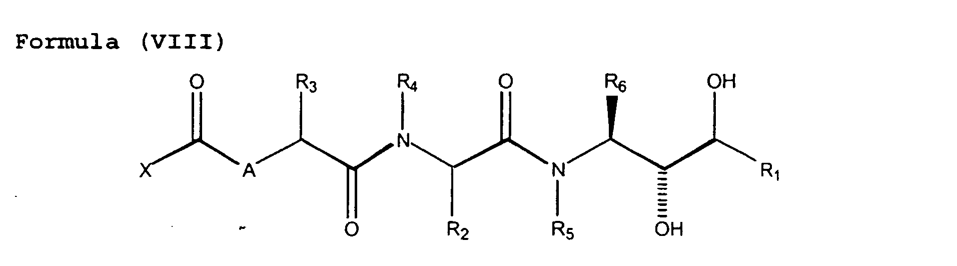 wherein X is selected from 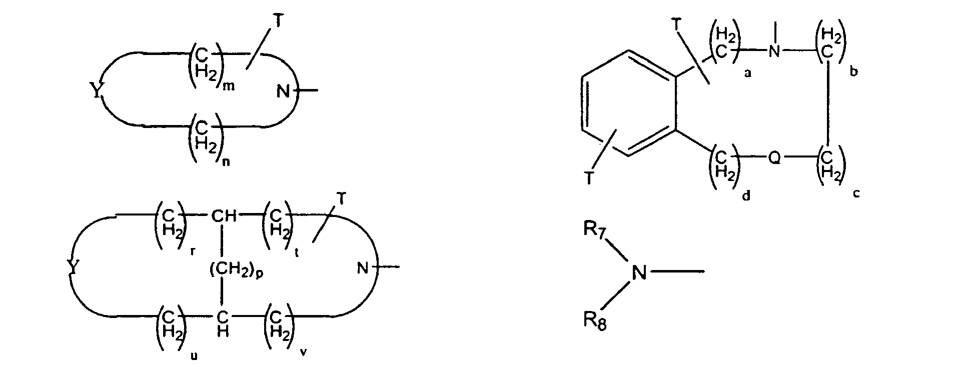 wherein Y and Q are selected from CH₂, CH-O-R₉, O, S, SO, SO₂ and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 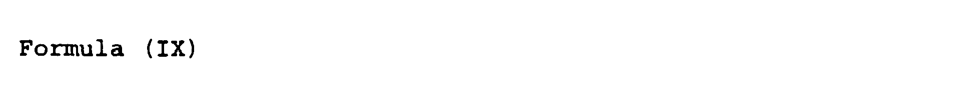 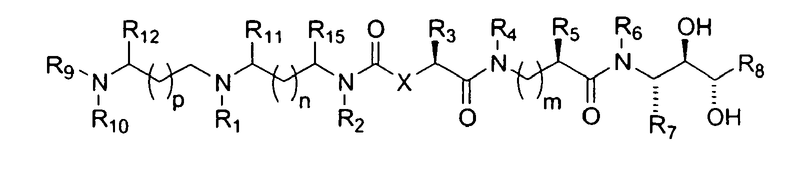 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH (OH) R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁ R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 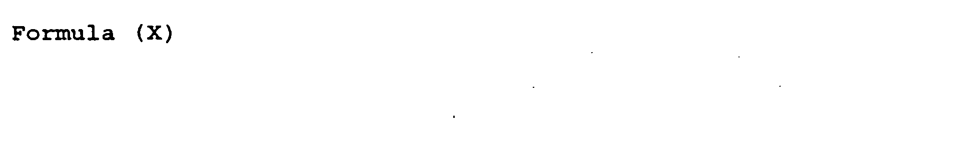 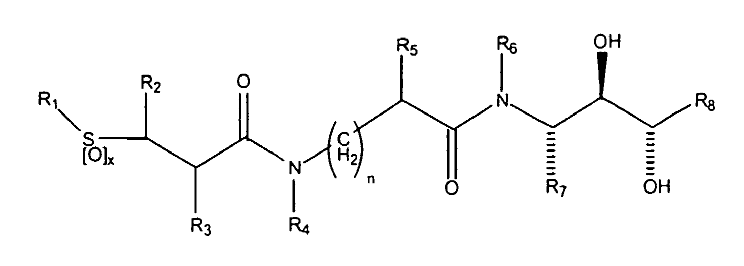 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl, or pharmaceutically-acceptable salts thereof.

18. A method for inhibiting beta-secretase activity, comprising contacting an effective amount for inhibition of a compound of formula (I), (II), (III), (IV), (V), (VI), (VII), (VIII), (IX), or (X): 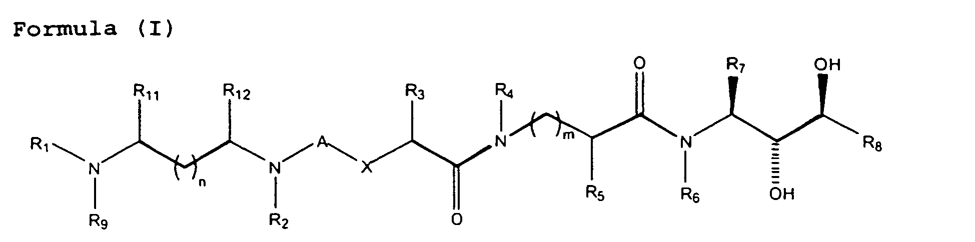 wherein A is selected from methylene, CO, SO, and SO₂;
wherein X is selected from oxygen atom, methylene and 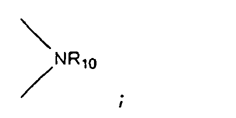 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl;
R₅ is selected from 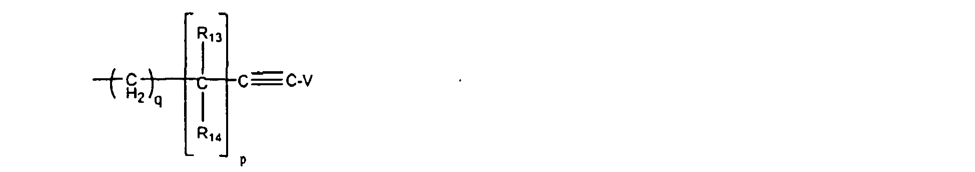 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 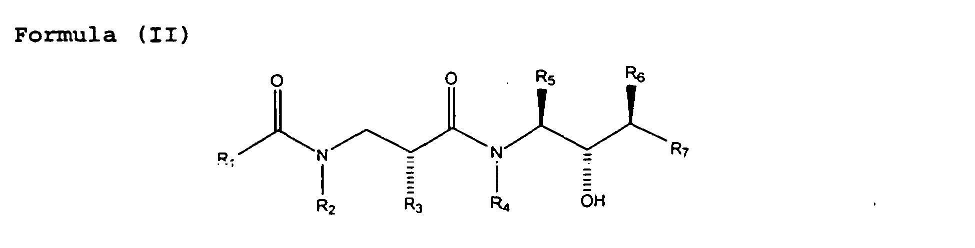 wherein R₁ is selected from aryl, aralkyl, heteroaryl and heteroaralkyl, including the following: 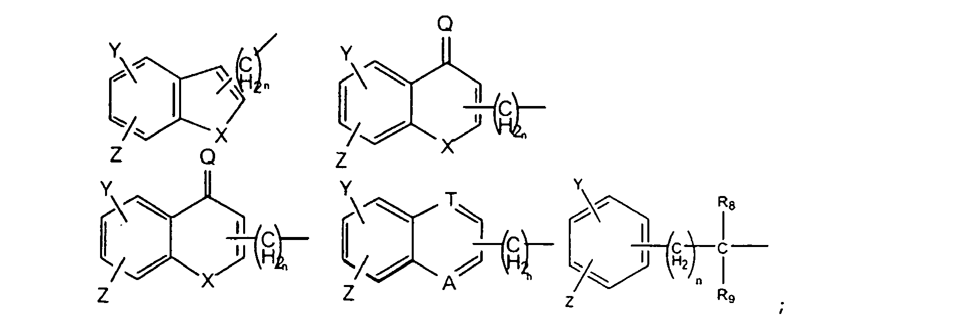 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 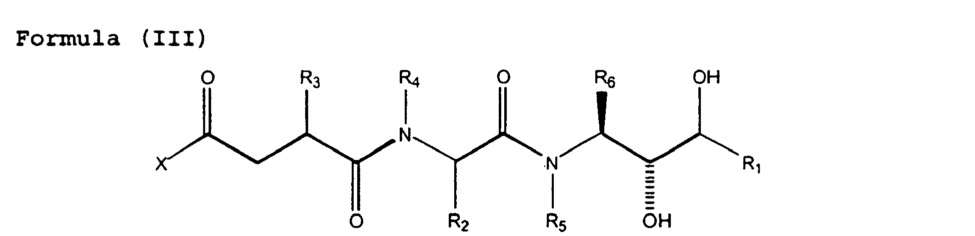 wherein X is selected from 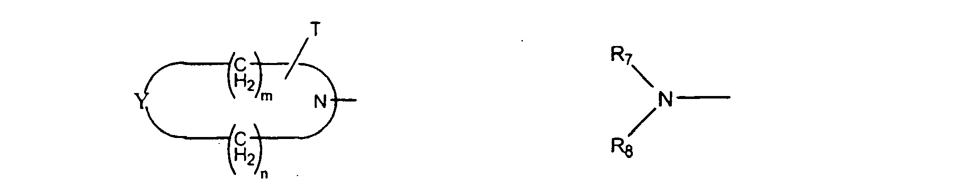 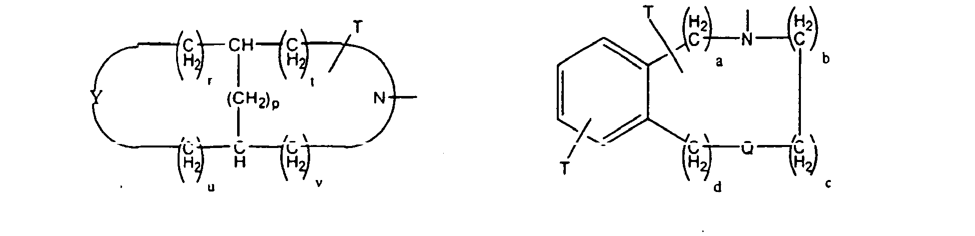 wherein Y and Q are selected from CH₂, CH-O-R₉, O*,* S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 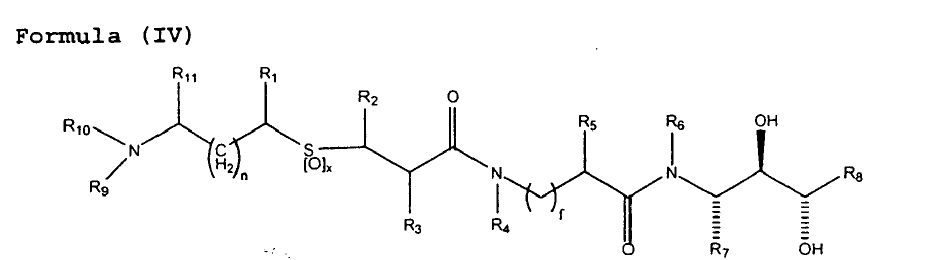 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl, and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 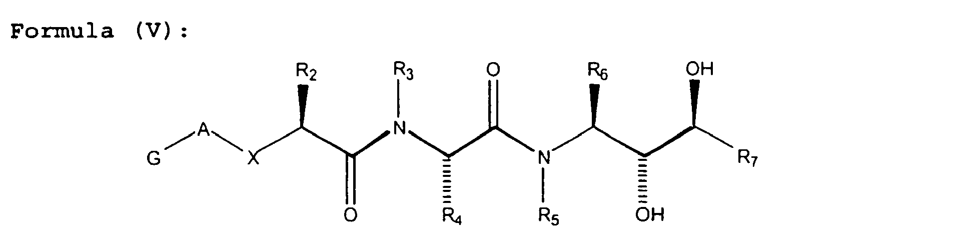 wherein A is selected from CO and SO₂;
X is selected form oxygen atom and methylene;
G is selected from B or 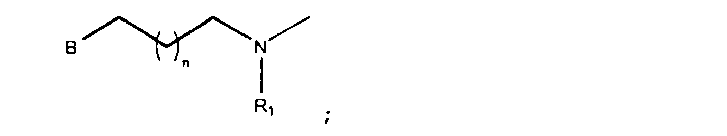 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 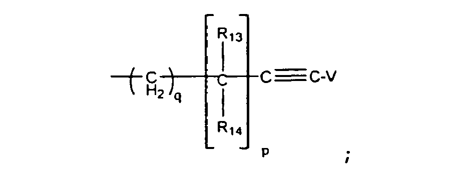 wherein V is selected from hydrido, alkyl, benzyl, and phenyl;
R₁₃ and R₁₄ are each independently a radical selected from alkyl, cycloalkylalkyl and phenylalkyl, any
one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 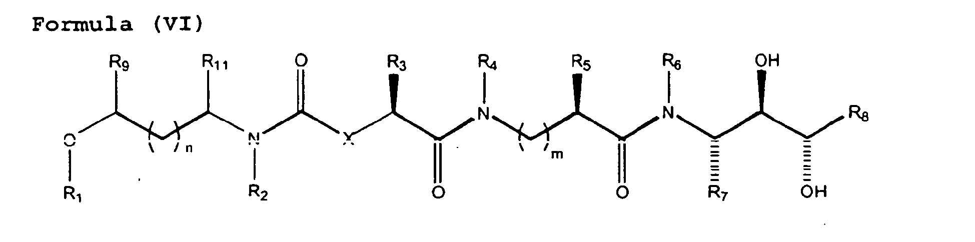 wherein X is selected from oxygen atom, methylene, and NR₁₀.
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 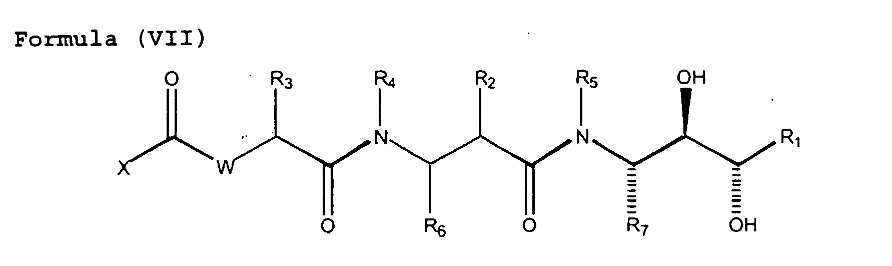 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 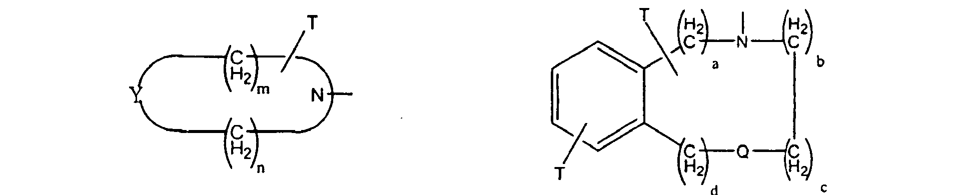 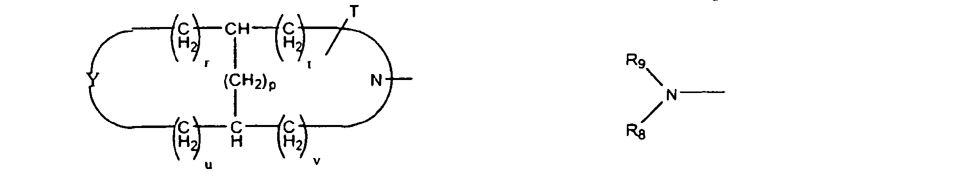 wherein Y and Q are selected from CH₂, CH-O-R₁₀, O , S, SO, SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano, and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 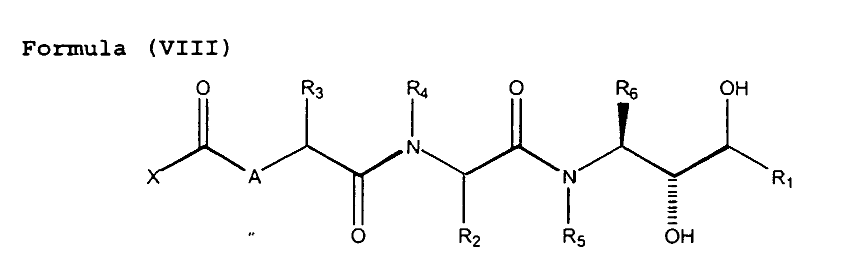 wherein X is selected from 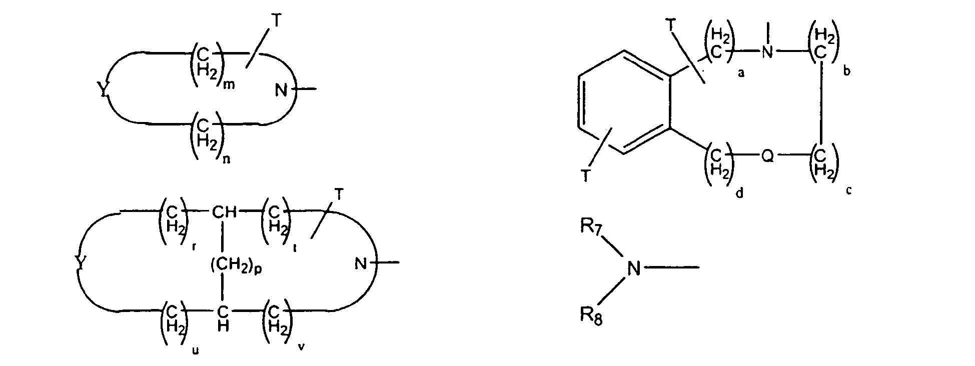 wherein Y and Q are selected from CH₂, CH-O-R₉, O, S, SO, SO₂, and NR_{10,}
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 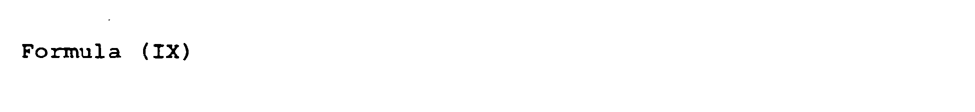 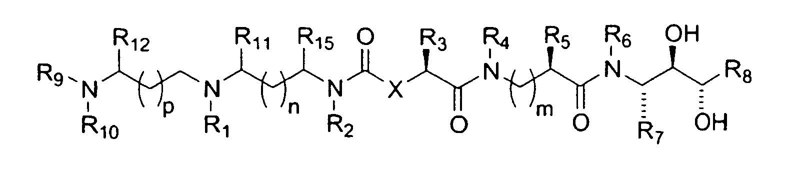 wherein X is selected from oxygen atom, methylene and >NR₁₃ .
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH (OH) R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 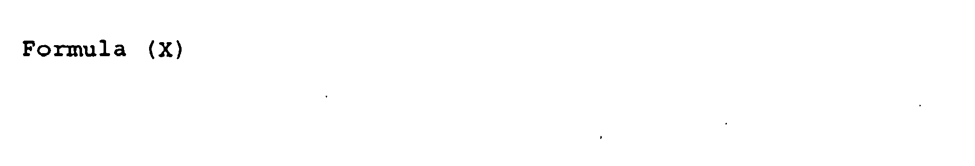 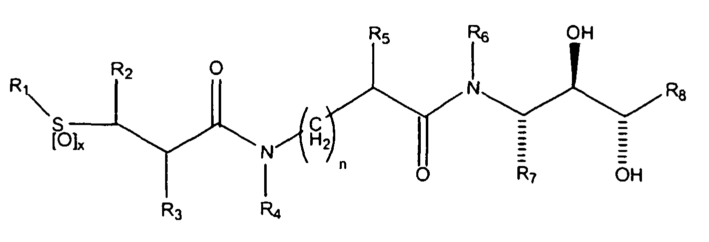 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl, or pharmaceutically-acceptable salts thereof.

19. A method for inhibiting cleavage of an amyloid precursor protein (APP) isotype at a site in the APP isotype that is susceptible to cleavage, comprising contacting said APP isotype with an effective cleavage inhibitory amount of a compound of formula (I), (II), (III), (IV), (V), (VI), (VII), (VIII), (IX), or (X) : 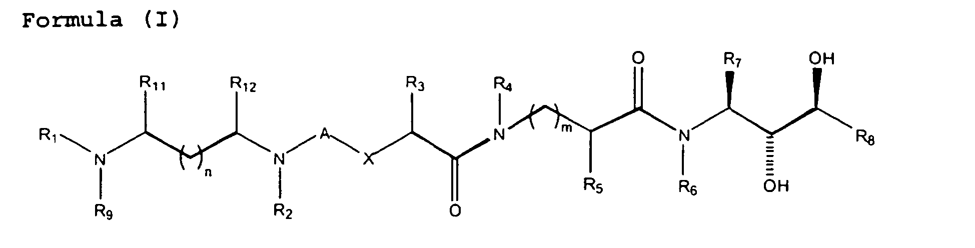 wherein A is selected from methylene, CO, SO, and SO₂;
wherein X is selected from oxygen atom, methylene and 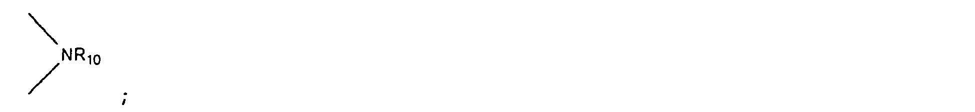 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy and alkyl;
R₄ and R₆ are each independently selected from hydrido, alkyl, benzyl and cycloalkyl;
R₅ is selected from 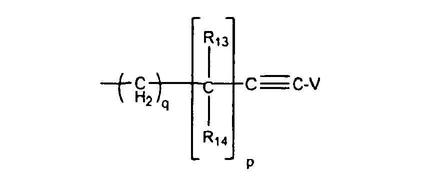 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 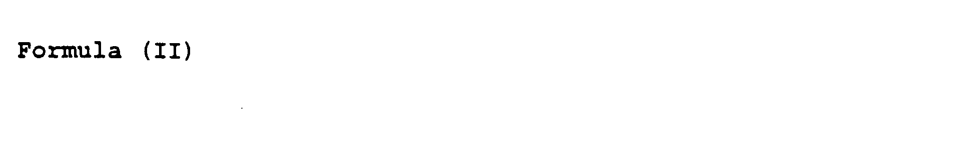 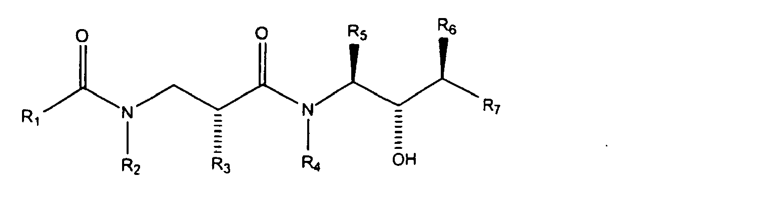 wherein R₁ is selected from aryl, aralkyl, heteroaryl and heteroaralkyl, including the following: 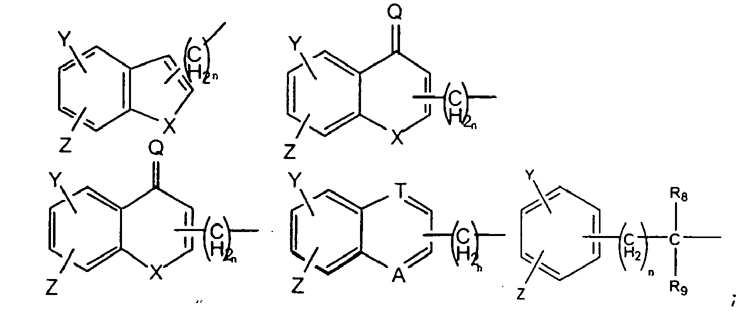 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 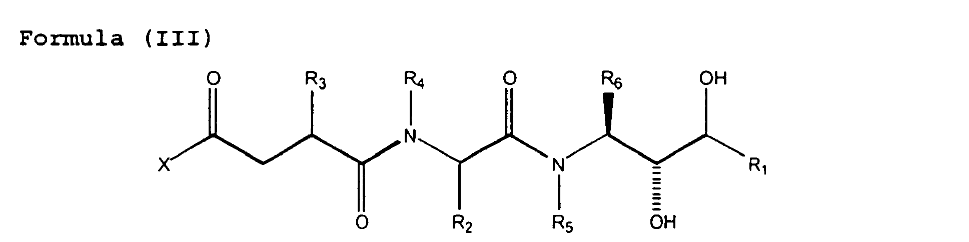 wherein X is selected from 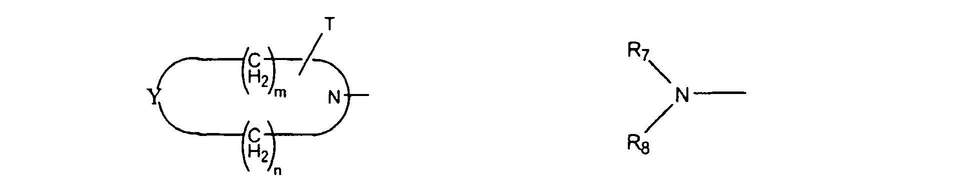 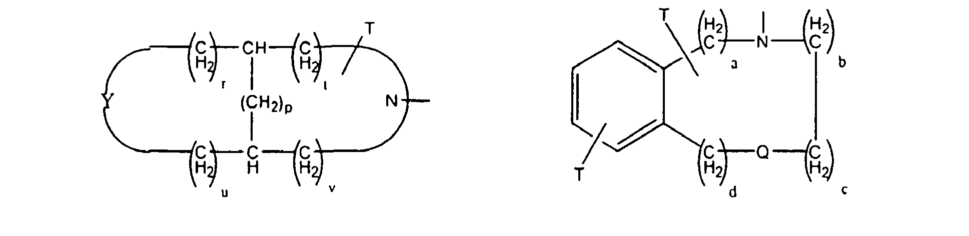 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 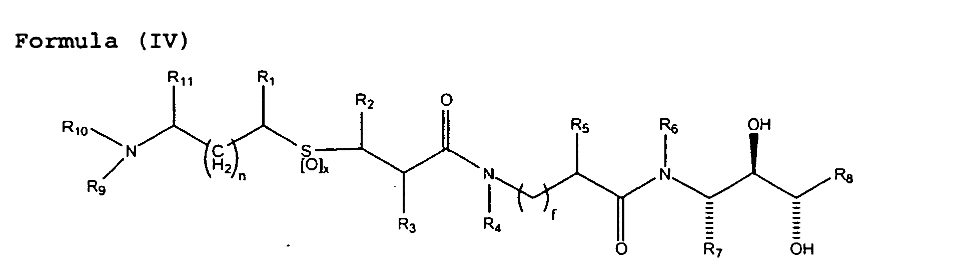 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 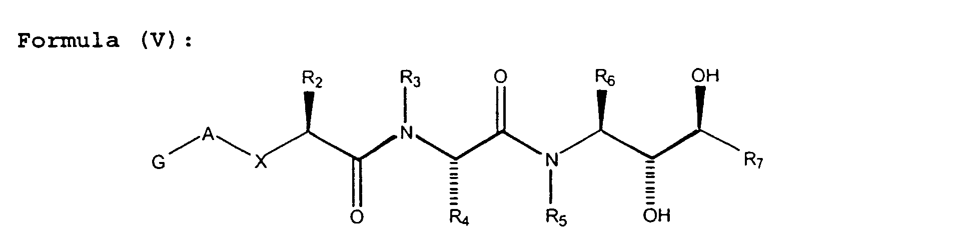 wherein A is selected from CO and SO₂;
X is selected form oxygen atom and methylene;
G is selected from B or 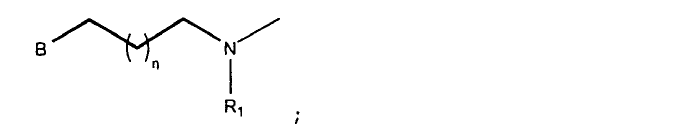 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 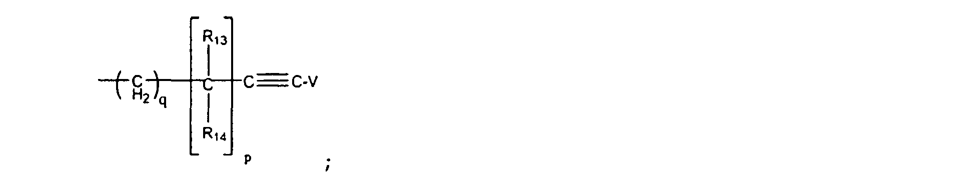 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 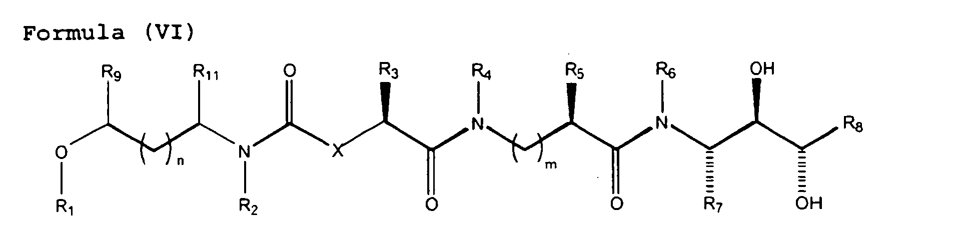 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 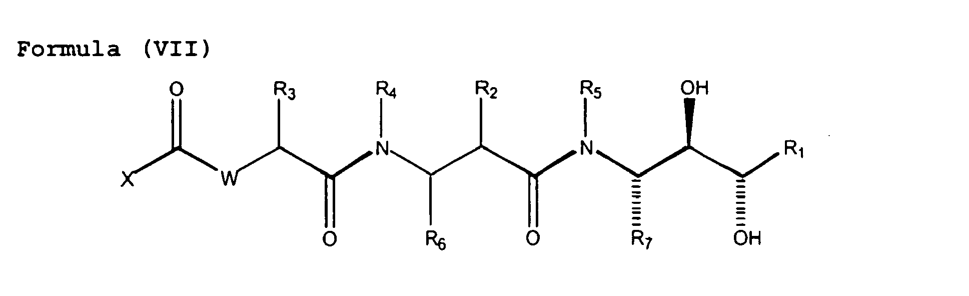 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 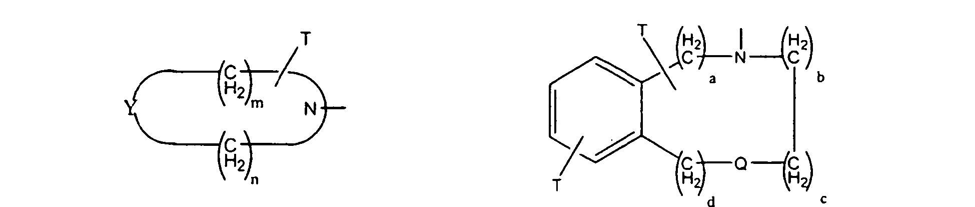 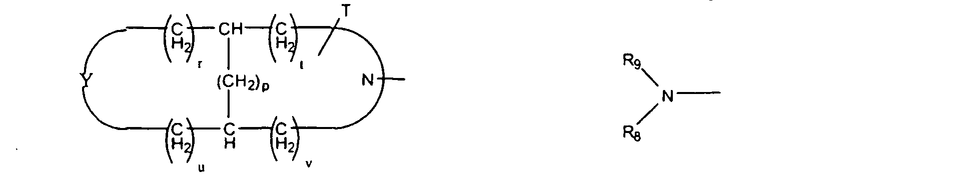 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 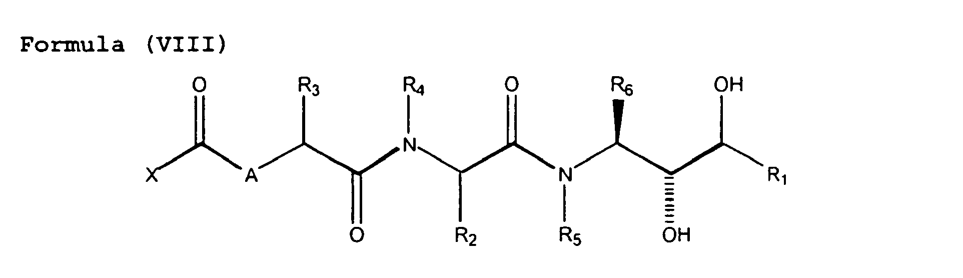 wherein X is selected from 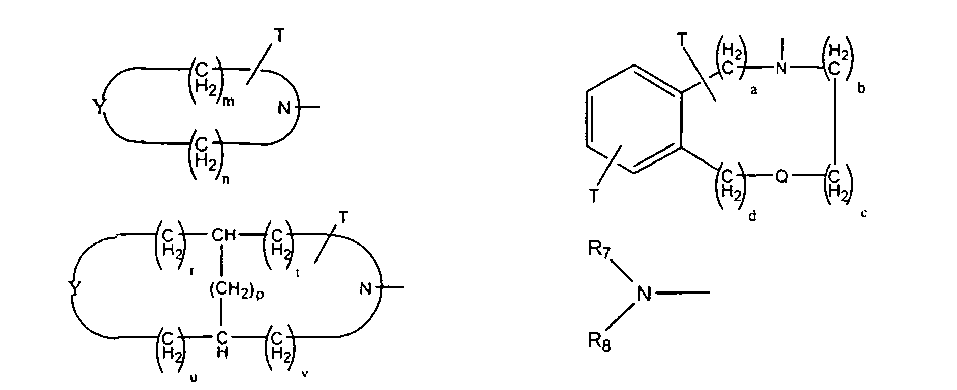 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 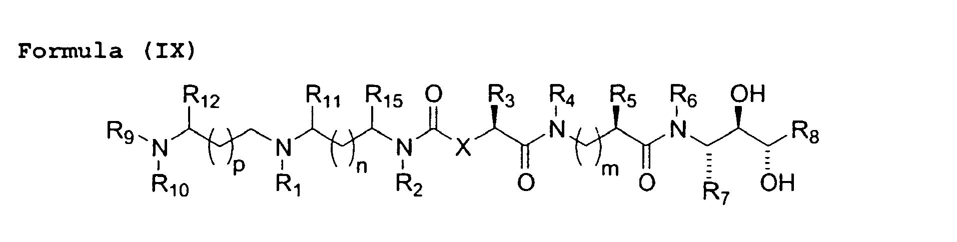 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH (OH) R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 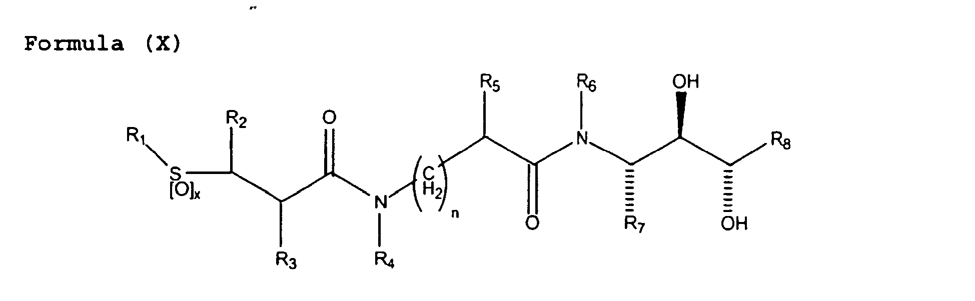 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl, or pharmaceutically-acceptable salts thereof.

20. A method for inhibiting production of amyloid beta peptide (A beta) in a cell, comprising administering to said cell an effective inhibitory amount of a compound of formula (I), (II), (III), (IV), (V), (VI), (VII), (VIII), (IX), or (X) : 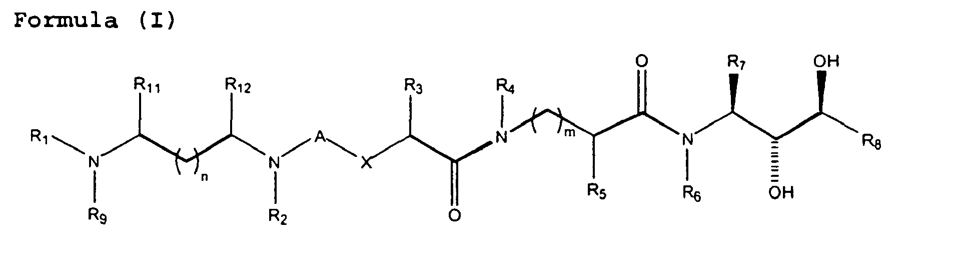 wherein A is selected from methylene, CO, SO, and SO₂;
wherein X is selected from oxygen atom, methylene and 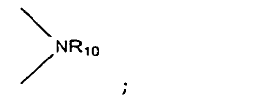 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl;
R₅ is selected from 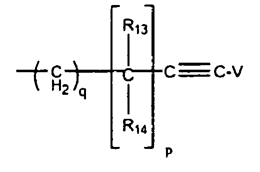 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 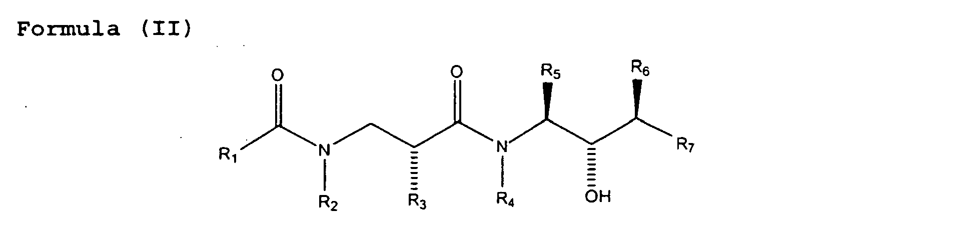 wherein R₁ is selected from aryl, aralkyl, heteroaryl and heteroaralkyl, including the following: 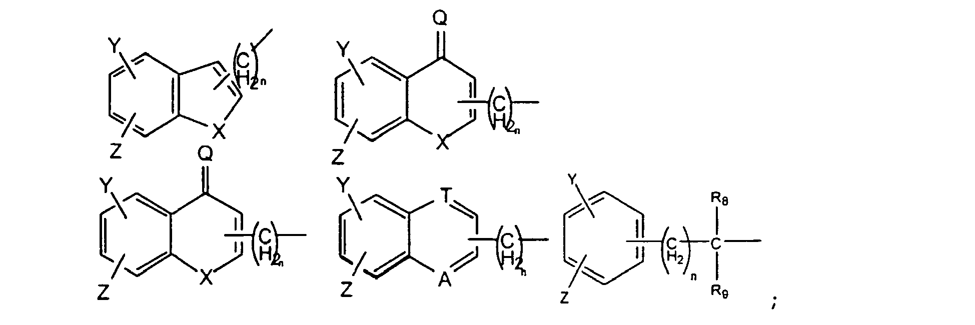 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy; alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 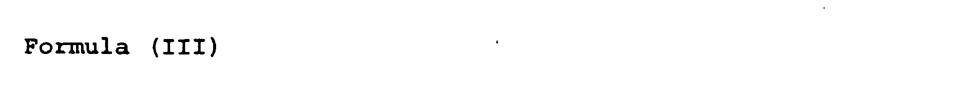 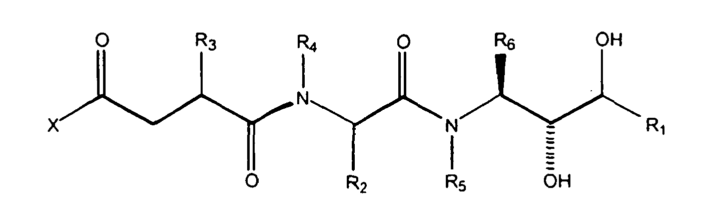 wherein X is selected from 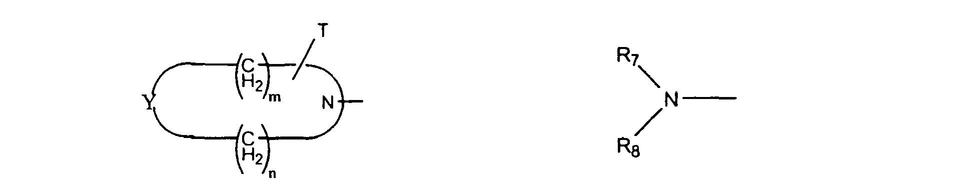 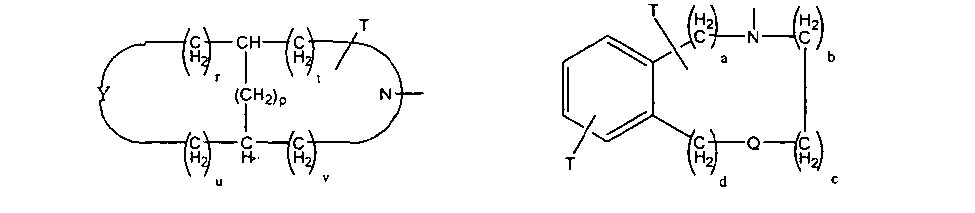 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 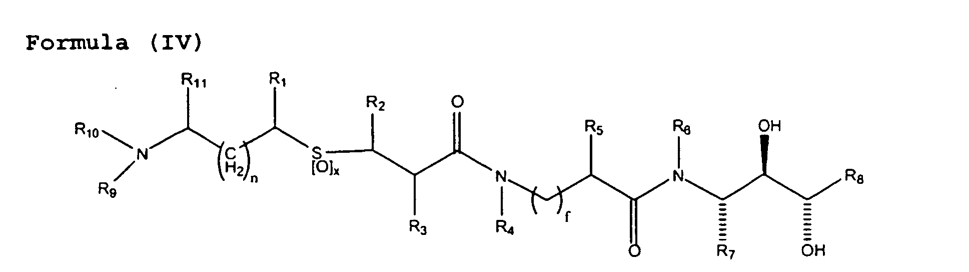 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 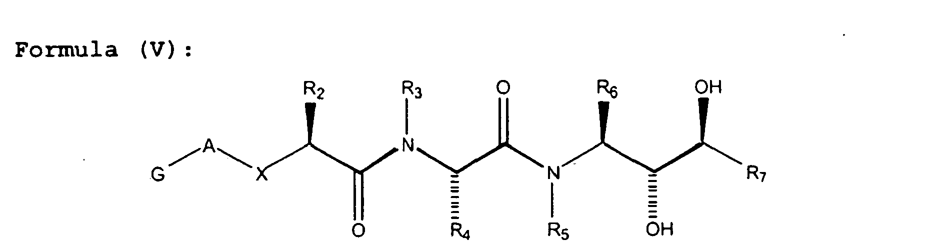 wherein A is selected from CO and SO₂
X is selected form oxygen atom and methylene;
G is selected from B or 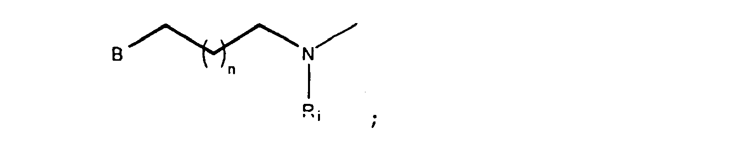 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 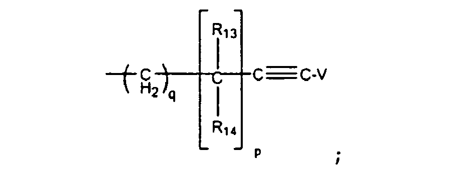 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 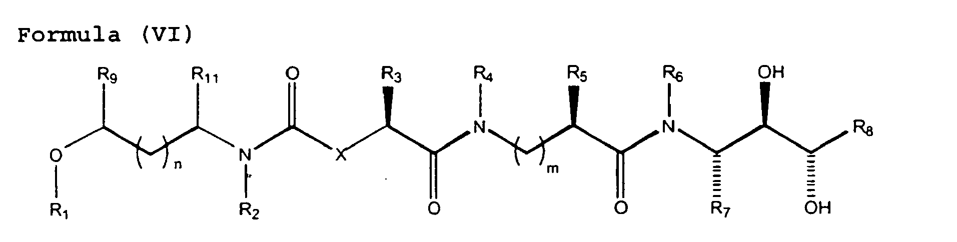 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphtylmethyl, aryl and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 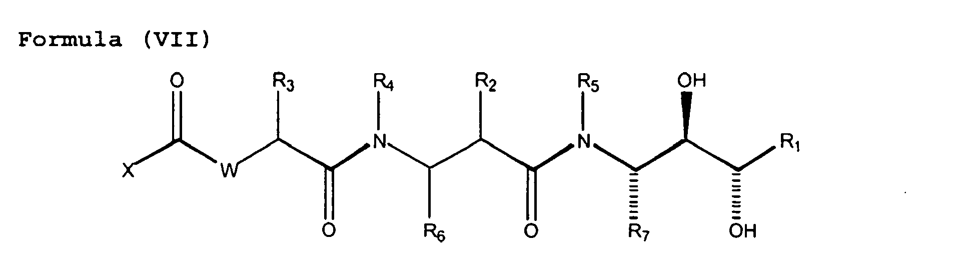 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 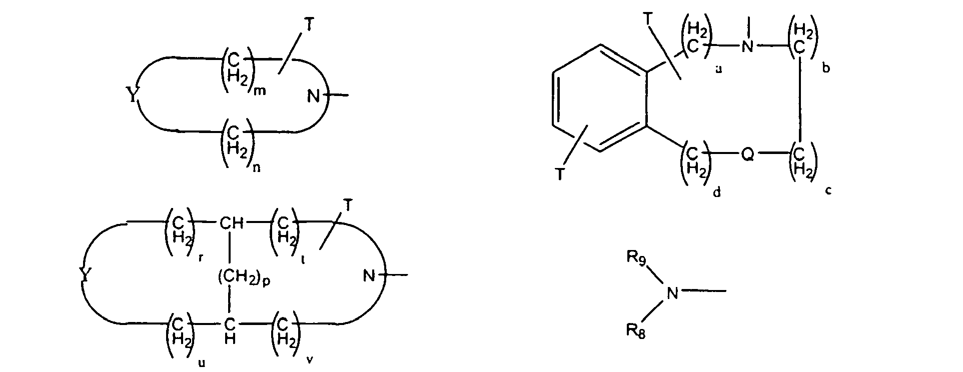 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 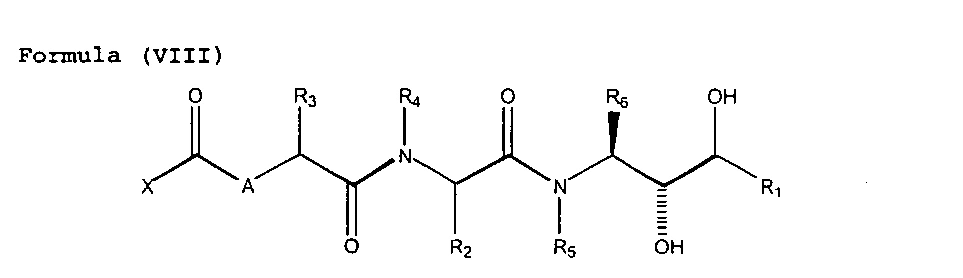 wherein X is selected from 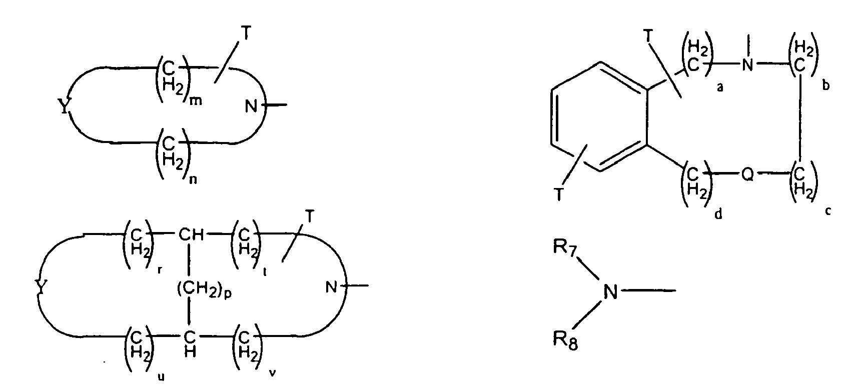 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 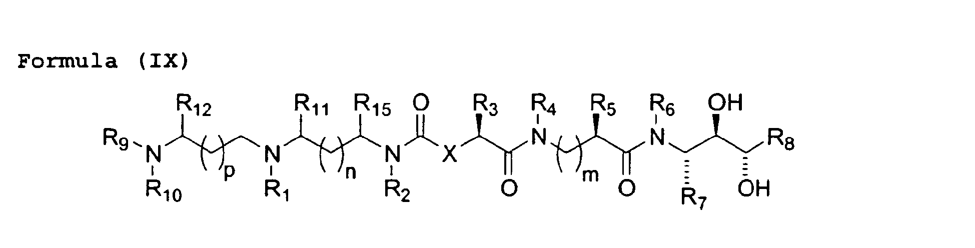 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH(OH)R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 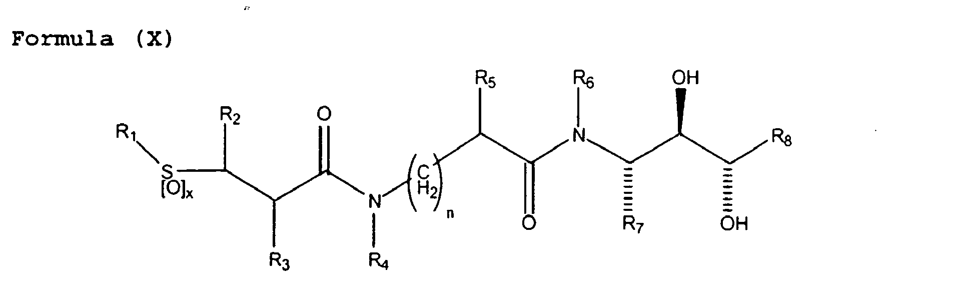 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl, or pharmaceutically-acceptable salts thereof.

21. The method of claim 20, wherein the cell is an animal cell.

22. The method of claim 21, wherein the animal cell is a mammalian cell.

23. The method of claim 22, wherein the mammalian cell is human.

24. A composition comprising beta-secretase complexed with a compound of formula (I), (II), (III), (IV), (V), (VI), (VII), (VIII), (IX), or (X) : 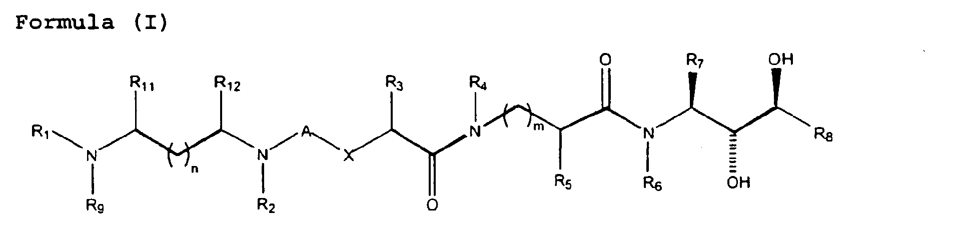 wherein A is selected from methylene, CO, SO, and SO₂
wherein X is selected from oxygen atom, methylene and 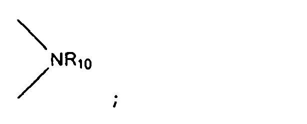 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl;
R₅ is selected from 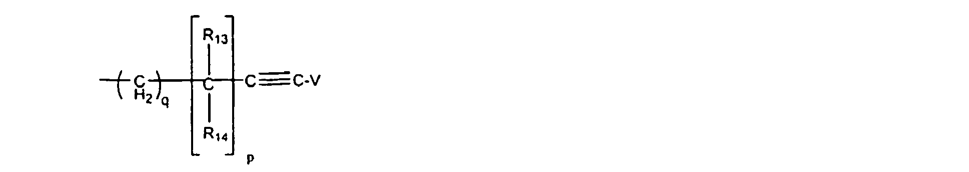 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 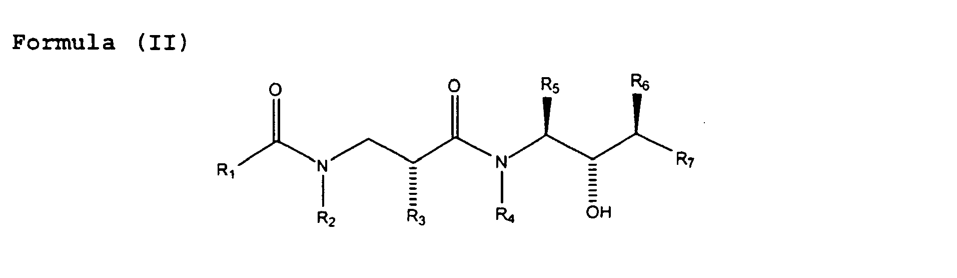 wherein R₁ is selected from aryl, aralkyl, heteroaryl and heteroaralkyl, including the following: 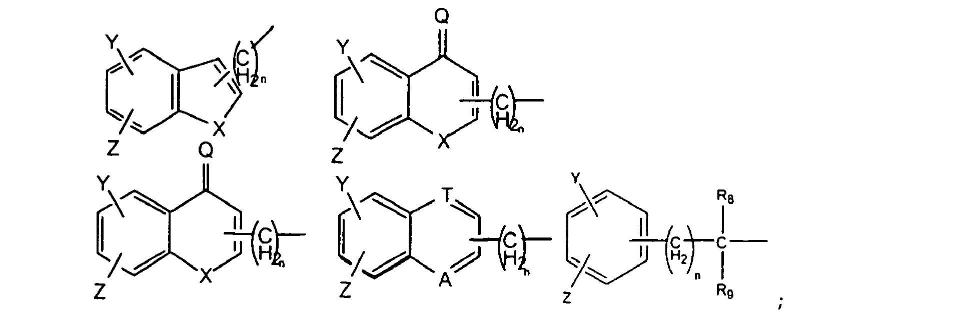 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and.A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 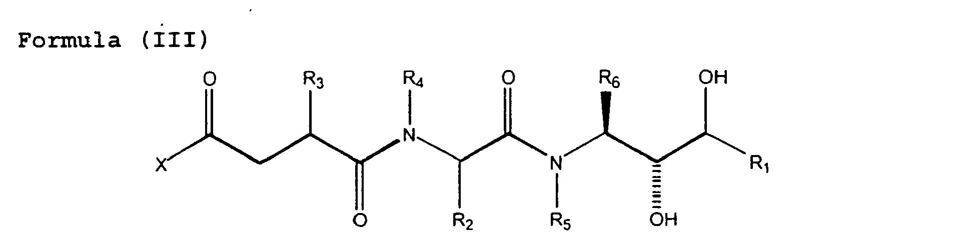 wherein X is selected from 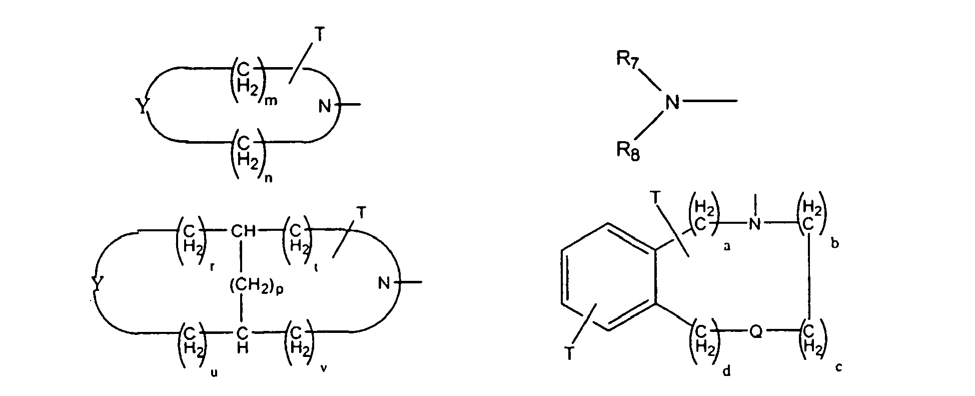 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 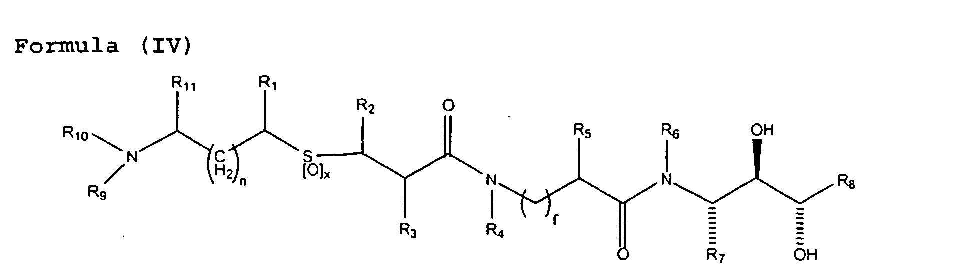 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 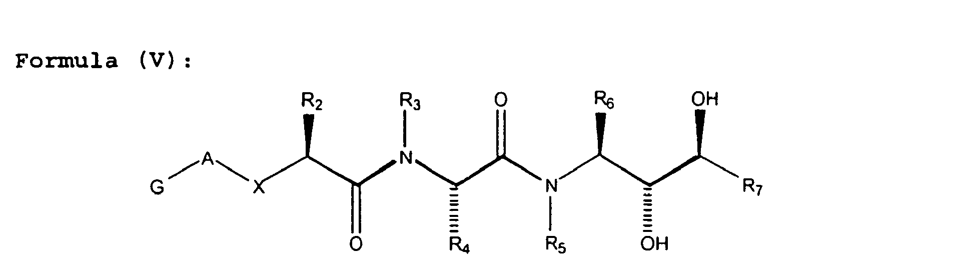 wherein A is selected from CO and SO₂;
X is selected form oxygen atom and methylene;
G is selected from B or 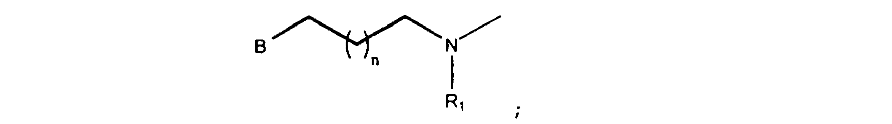 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo, k hydroxy, alkoxy, and alkyl;
R₃ and R₅ are each independently selected from hydrido and alkyl;
R₄ is 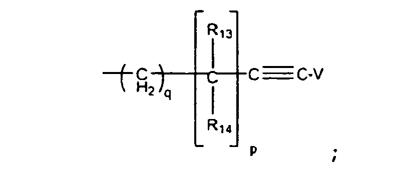 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 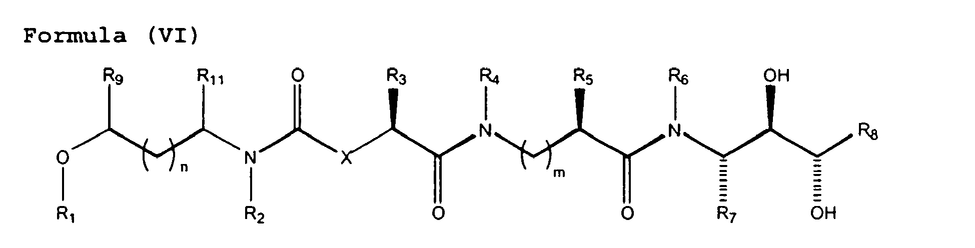 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl, and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 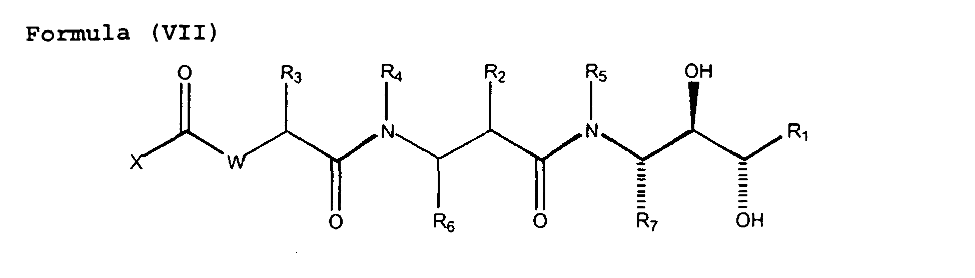 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 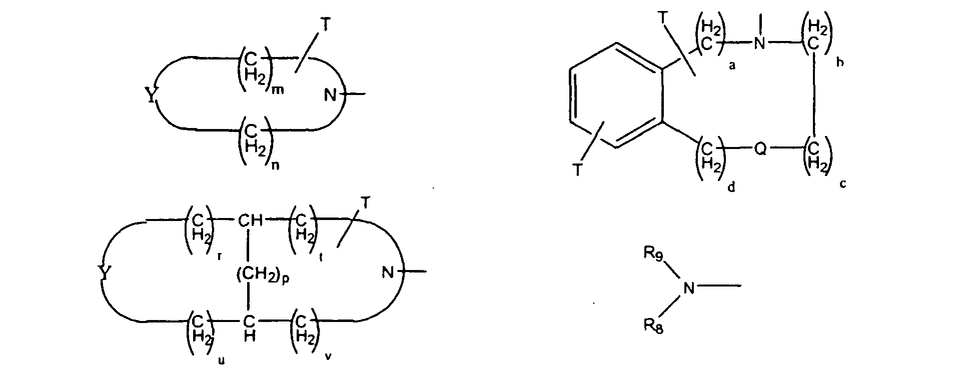 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower cilkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl;
with the proviso that at least one or R₈ and R₉ is an aryl group. 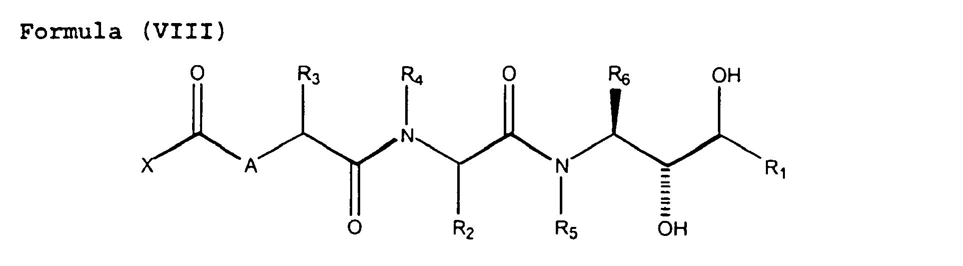 wherein X is selected from 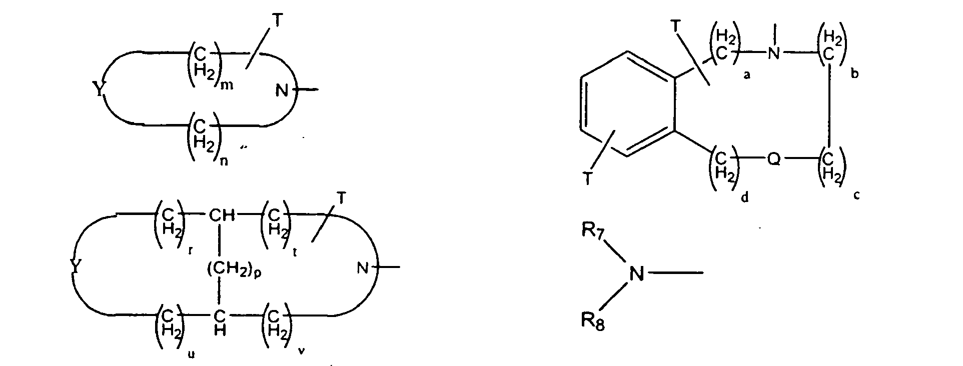 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 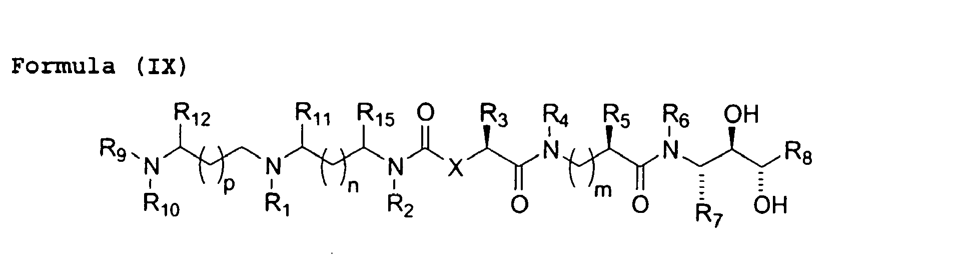 wherein X is selected from oxygen atom, methylene and >NR₁₃ ,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH(OH)R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 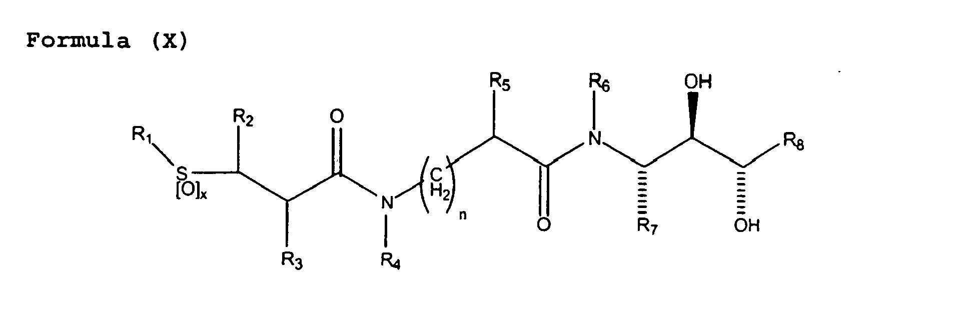 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl, or pharmaceutically-acceptable salts thereof.

25. A method for producing a beta-secretase complex comprising the composition of claim 24.

26. A method for inhibiting the production of beta-amyloid plaque in an animal, comprising administering to said animal an effective inhibiting amount of a compound of formula (I), (II), (III), (IV), (V), (VI), (VII), (VIII), (IX), or (X) : 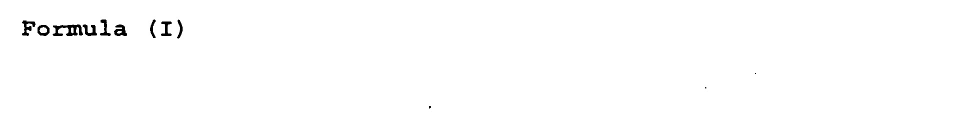 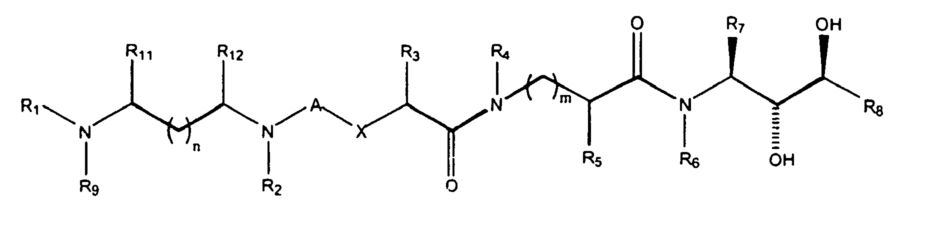 wherein A is selected from methylene, CO, SO, and SO₂;
wherein X is selected from oxygen atom, methylene and 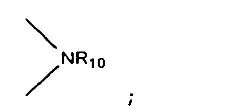 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl, benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl;
R₅ is selected from 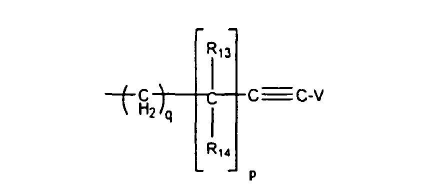 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 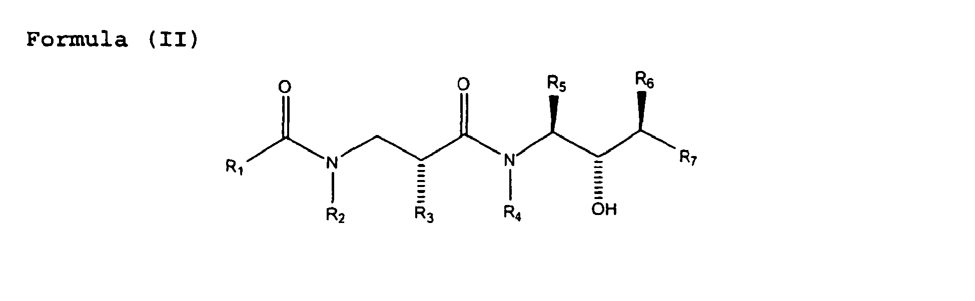 wherein R₁ is selected from aryl, aralkyl, heteroaryl and heteroaralkyl, including the following: 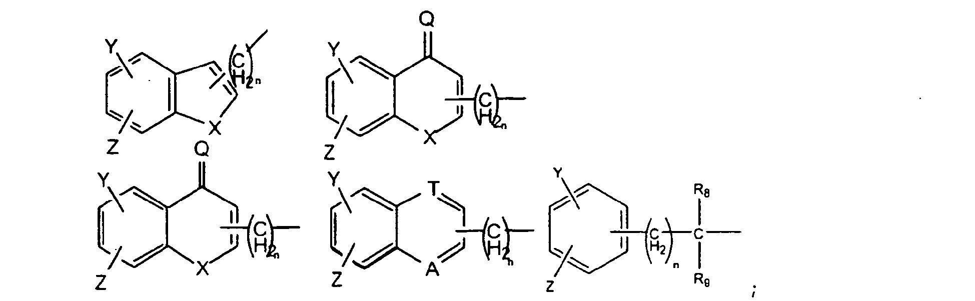 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 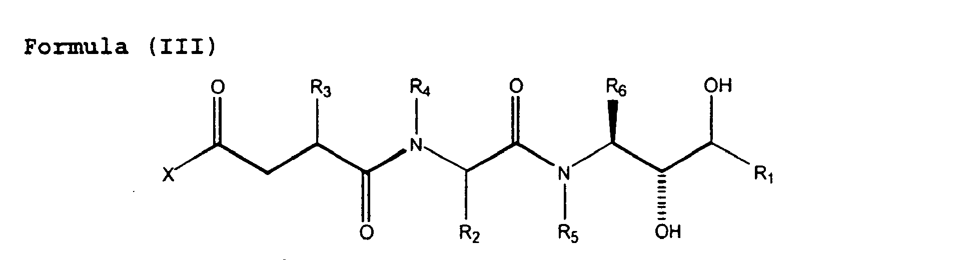 wherein X is selected from 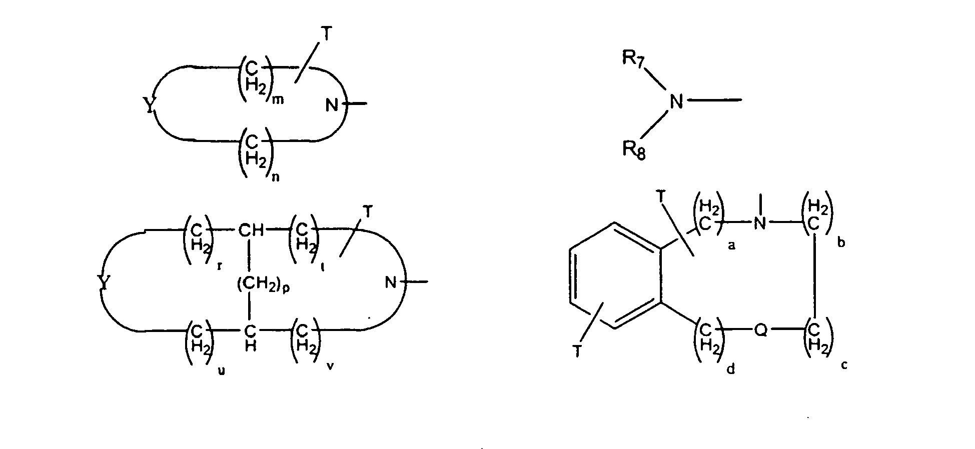 wherein Y and Q are selected from CH₂, CH-0-Rg, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydride, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 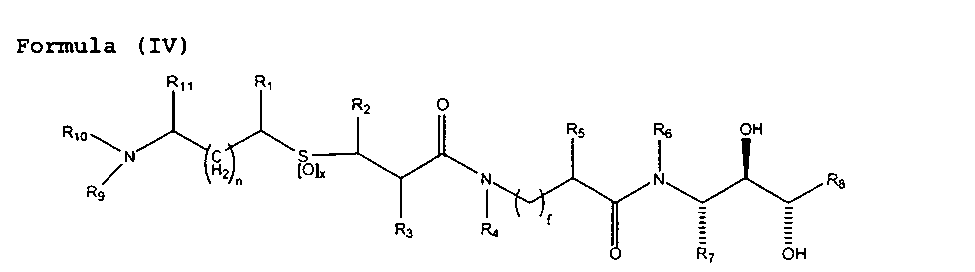 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 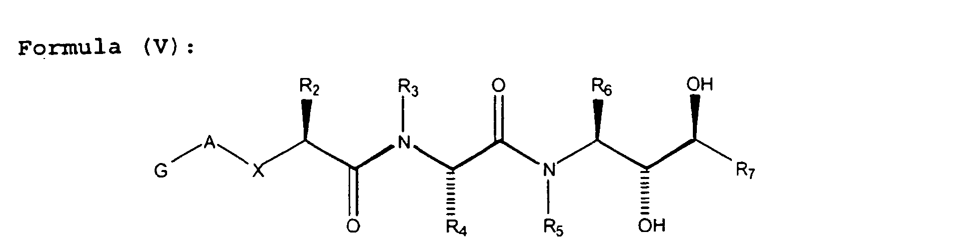 wherein A is selected from CO and SO₂
X is selected form oxygen atom and methylene;
G is selected from B or 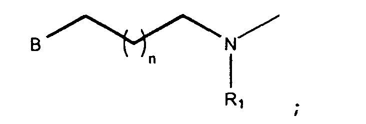 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 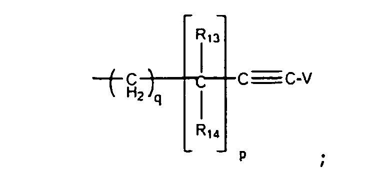 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 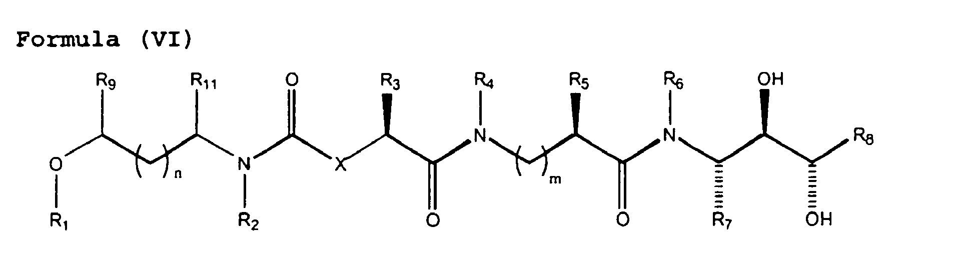 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and.naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 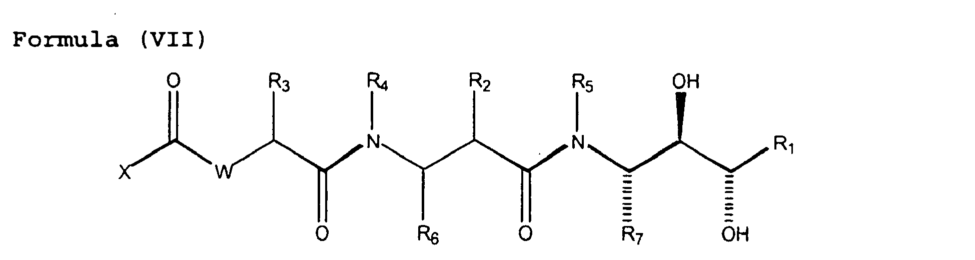 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 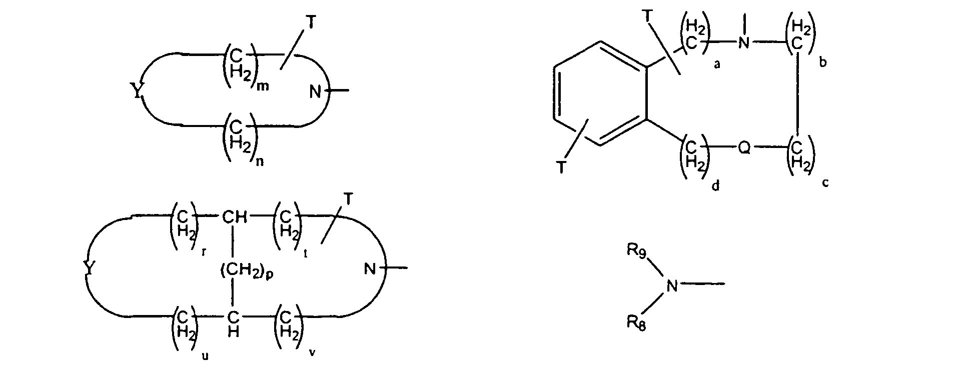 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, Linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 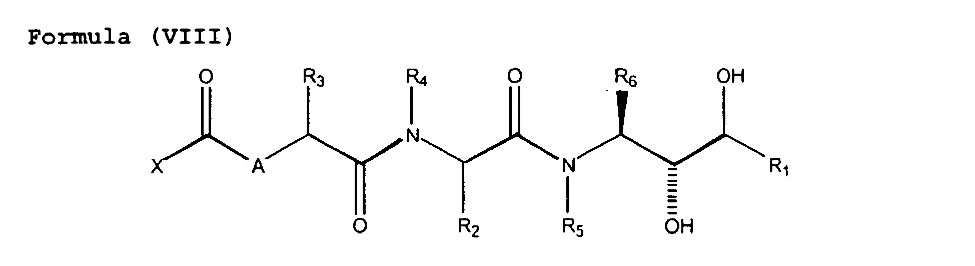 wherein X is selected from 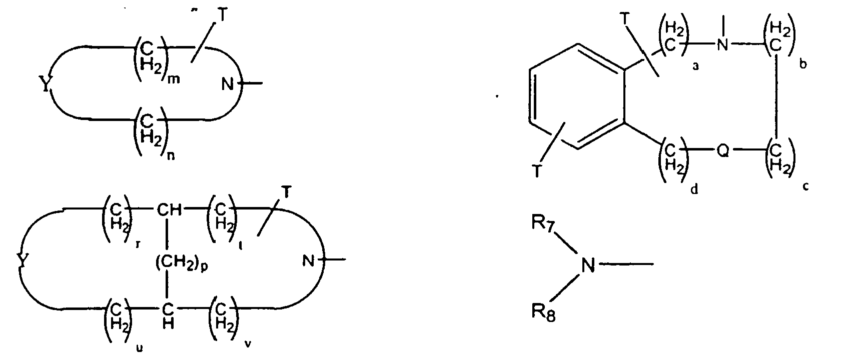 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 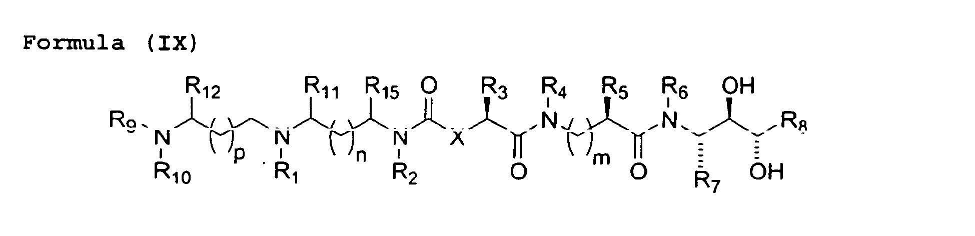 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R7 is selected form subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH(OH)R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 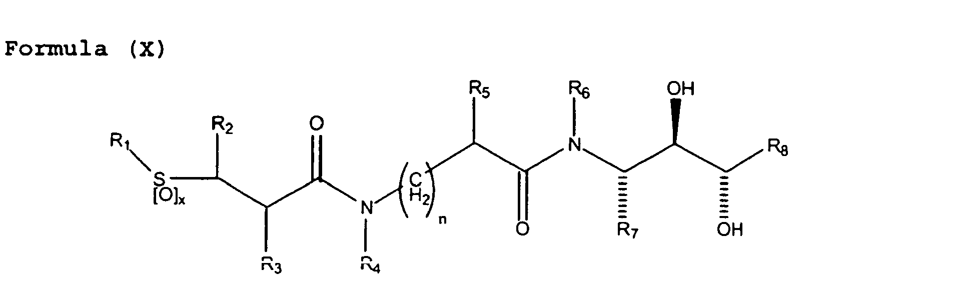 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl, or pharmaceutically-acceptable salts thereof.

27. The method of claim 26, wherein said animal is a human.

28. A method for treating or preventing a disease **characterized by** beta-amyloid deposits on or in the brain, comprising administering to a subject in need of such treatment or prevention an effective therapeutic amount of a compound of formula (I), (II), (III), (IV), (V), (VI), (VII), (VIII), (IX), or (X): 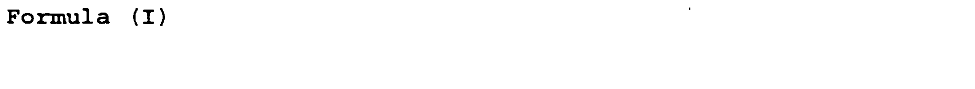 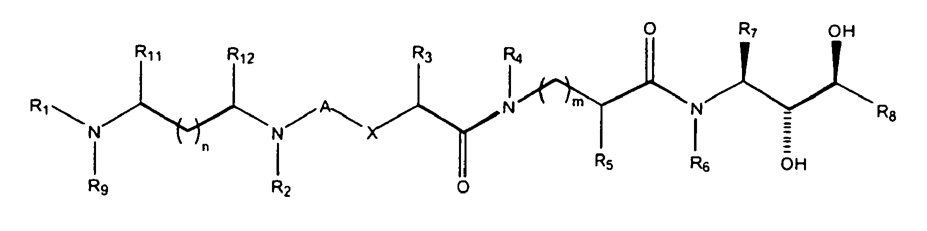 wherein A is selected from methylene, CO, SO, and SO₂
wherein X is selected from oxygen atom, methylene and 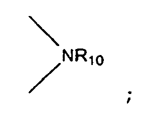 with R₁₀ selected from hydrido, alkyl, and benzyl;
wherein R₁ and R₉ are each independently selected from hydrido, alkyl, cycloalkyl, alkoxyacy, haloalkyl, alkoxycarbonyl", benzyloxycarbonyl, loweralkanoyl haloalkyacyl, phenyl, benzyl, naphthyl, and naphthylmethyl, any one of which groups having a substitutable position may be optionally substituted with one or more radicals selected from alkyl, alkoxy, alkenyl, alkynyl, halo, haloaklyl, cyano, and phenyl, and wherein the nitrogen atom to which R₁ and R₉ are attached may be combined with oxygen to form an N-oxide;
R₂ is selected from hydrido, alkyl, dialkylaminoalkyl, alkylacylaminoalkyl, benzy, and cycloalkyl;
R₃ is selected from alkyl, cycloalkylalkyl, acylaminoalkyl, phenylalkyl, naphthylmethyl, aryl, heterocyclicalkyl, and heterocyclic-cycloalkyl, wherein the cyclic portion of any of said phenylalkyl, naphthylmethyl, aryl, heterocyclicalkyl and heterocycliccycloalkyl groups may be substituted by one or more radicals selected from halo, hydroxy, alkoxy, and alkyl;
R₄ and R₆ are each independently selected from hydrido, alkyl, benzyl and cycloalkyl,
R₅ is selected from 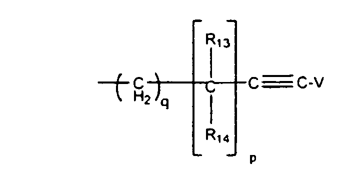 wherein V is selected from hydrido, alkyl, cycloalkyl, haloalkyl, benzyl, and phenyl;
R₁₃ and R₁₄ are each a radical independently selected from hydrido, alkyl, alkenyl, alkynyl, cycloalkyl, phenyl, heterocyclic, heterocyclicalkyl, and heterocycliccycloalkyl;
R₇ is selected from substituted or unsubstituted alkyl, cycloalkyl, phenyl, cycloalkylalkyl, and phenylalkyl, and one of which may be substituted with one or more groups selected from alkyl, hydroxy, alkoxy, halo, haloalkyl, alkenyl, alkynyl, and cyano;
R₈ is selected from hydrido, alkyl, haloalkyl, alkylcycloalkyl, cycloalkyl, cycloalkylalkyl, hydroxyalkyl, alkenyl, alkylcycloalkenyl, and alkoxycarbonyl;
R₁₁ and R₁₂ are each independently selected from hydrido, alkyl, haloalkyol, dialkylamino, and phenyl;
wherein m is 0 or 1;
wherein n is an integer selected from 0 through 5;
wherein p is an integer selected from 0 through 5; and
wherein q is an integer selected from 0 through 5; or a pharmaceutically-acceptable salt thereof. 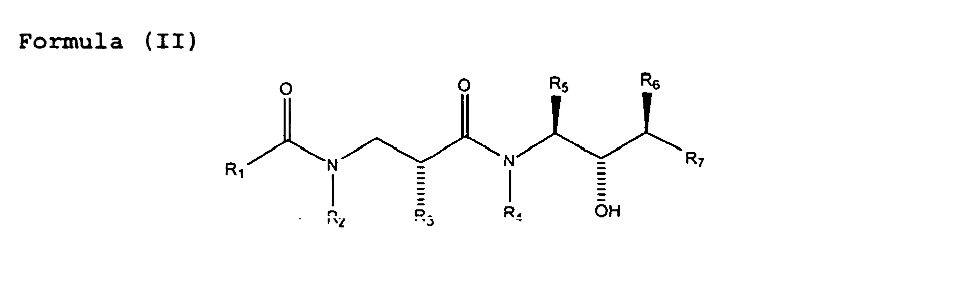 wherein R₁ is selected from aryl, aralkyl, heteroaryl and heteroaralkyl, including the following: 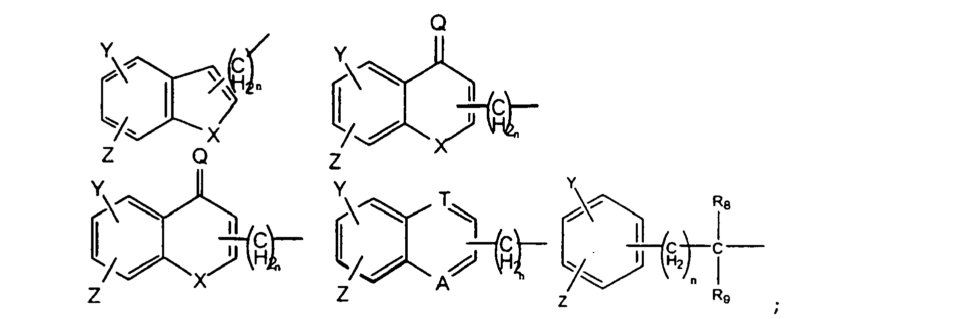 wherein X is selected from O, S, alkylamino, and NH;
Y and Z are each independently selected from lower alkyl, hydroxy, halo, alkoxy, carboxy, amino, alkylamino, dialkylamino, aryl, sufhydryl, and thioalkyl;
Q is selected from O and S
T and A are each independently selected from N and CH;
n is an integer selected from 0 through 5, inclusive;
R₈ and R₉ are each independently selected from hydrido, alkyl, phenylalkyl, cycloalkyl, heterocyclicalkyl, and phenyl;
wherein R₈ and R₉ may be taken together to form a cycloalkyl, cycloalkyalky, cycloalkenyl, or heterocyclic ring consisting of from three to about eight ring members, which heterocyclic ring contains a hetero ring atom selected from oxygen atom, sulfur atom, and >NH;
R₂ and R₄ are each independently selected from hydrido and lower alkyl;
R₃ is selected from hydrido, alkyl, benzyl, cycloalkyl, cycloalkylalkyl, alkoxyalkyl, alkylthioalkyl and imidazolemethyl;
R₅ is selected from cycloalkyl, phenyl, lower alkyl, cycloalkylalkyl and phenylalkyl;
R₆ is selected from hydrido, hydroxy, alkoxy, amino, alkylamino, dialkylamino, lower alkyl and cycloalkyl;
R₇ is selected from hydrido, alkyl, haloalkyl, cycloalkylalkyl, alkylcycloalkyl, alkylcycloalkenyl and alkoxycarbonyl;
wherein R₆ and R₇ may be taken together to form a carbocyclic or heterocyclic ring consisting of from 3 to about 8 ring members, which heterocyclic ring contains a hetero ring atom selected from oxygen atom, sulfur atom and NH; and
wherein any of the foregoing R₁ through R₉ substituents having a substitutable position may be substituted with one or more groups selected from alkyl, alkoxy, halo, haloalkyl, alkenyl, alkynyl and cyano; or a pharmaceutically-acceptable salt thereof. 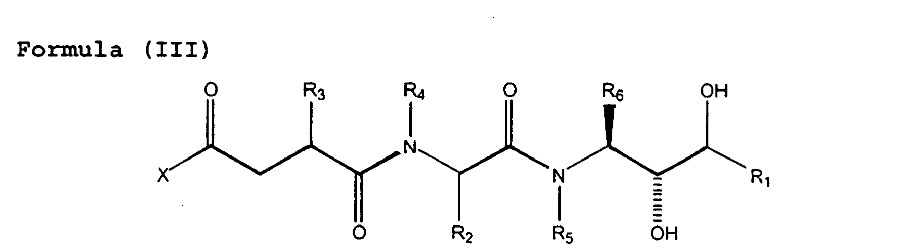 wherein X is selected from 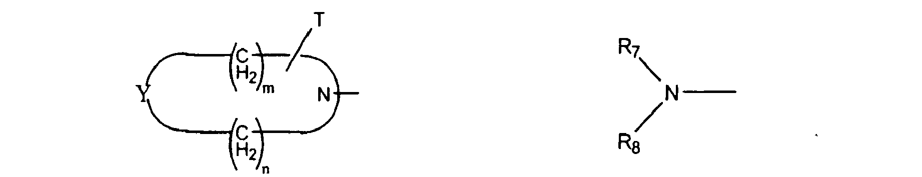 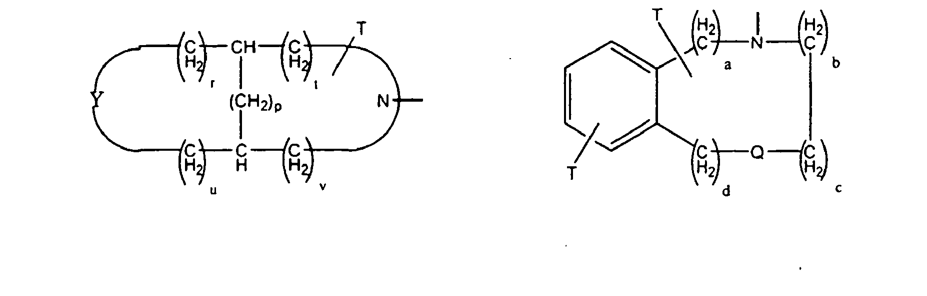 wherein Y and Q are selected from CH₂, CH-O-R₉, O, S, SO, SO₂, and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at thephenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₁ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group. 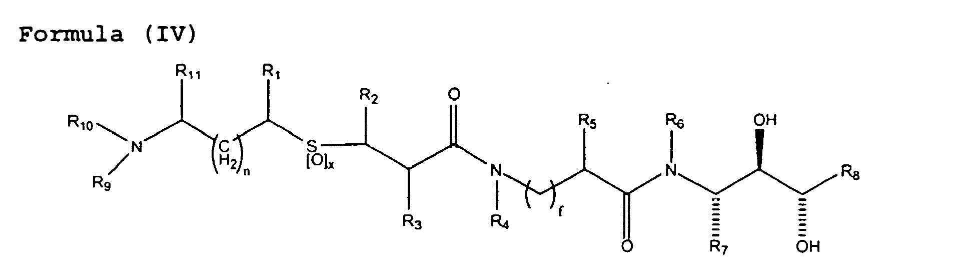 wherein R₁ and R₁₁ are each independently selected from hydrido, alkyl, alkylaminoalkyl, and phenyl;
n is an integer selected from 0 through 5;
x is an integer selected from 0 through 2;
f is an integer selected from 0 or 1;
R₂ is selected from hydrido and alkyl;
R₃ is a group selected from hydrido, cycloalkylalkyl, aralkyl, and haloaralkyl;
R₄ and R₆ each are independently selected from hydrido and methyl;
R₅ is selected from cycloalkylalkyl groups containing from 3 to about 12 carbon atoms
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, and haloalkenyl;
R₉ and R₁₀ each are independently selected from hydrido, alkyl, cycloalkyl, cycloalkylalkyl, alkyacyl, aryl, aralkyl, haloaryl, and haloaralkyl; and
wherein any one of said groups R₁ through R₁₁ having a substitutable position may be substituted with one or more groups selected from alkyl, hydroxy, hydroxyalkyl, halo, alkoxy, alkoxyalkyl, and alkenyl; or a pharmaceutically-acceptable salt thereof 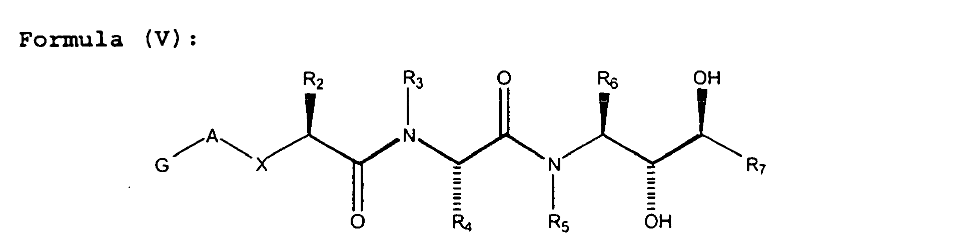 wherein A is selected from CO and SO₂;
X is selected form oxygen atom and methylene;
G is selected from B or 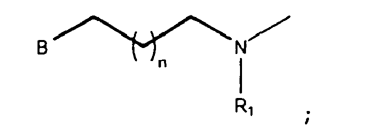 wherein R₁ is selected from hydrido and alkyl;
B is a saturated heterocyclic ring system of five to ten ring members with two ring system members being nitrogen atoms, wherein said ring system may be monocyclic or bicyclic and may be fused to a benzene or cyclohexane ring,
wherein the point of attachment of B to the backbone of the structure of Formula V, or the structure described for G above, may be through a bond to any substitutable position on said heterocyclic ring system of B and wherein any substitutable position of B may be optionally substituted with one or more radicals selected from alkyl, alkoxy, alkenyl, acetyl, alkynyl, halo, trifluoromethyl, oxo, cyano, and phenyl, and wherein the said heterocyclic ring nitrogen atom may be combined with oxygen to form an N-oxide;
R₂ is selected from alkyl, cycloalkylalkyl, acylaminoalkyl, phenyalkyl, and naphthylalkyl, and wherein the cyclic portion of any of said phenylalkyl, cycloalkyalkyl, and naphthylalkyl groups may be substituted by one or more radicals selected fromhalo,k hydroxy, alkoxy, and alkyl;
R₃ and R₅ are each independently selected from hydrido and alkyl;
R₄ is 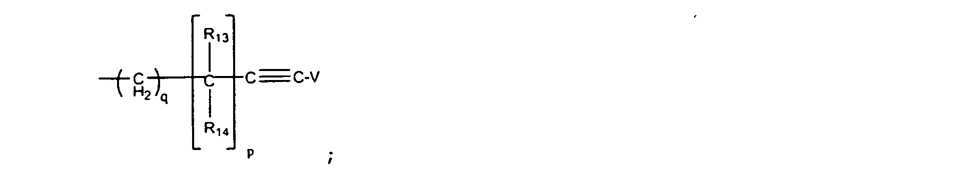 wherein V is selected from hydrido, alkyl, benzyl, and phenyl;
R₁₃ and R₁₄ are each independently a radical selected from alkyl, cycloalkylalkyl and phenylalkyl, any one of which may be substituted with one or more groups selected from alkyl, hydroxy, and alkoxy;
p is an integer selected from zero through five, inclusive;
q is an integer selected from zero through five, inclusive;
n is an integer selected from zero through five, inclusive; and
R₇ is selected from hydrido, alkyl, cycloalkyl, cycloslkylalkyl, hydroxyalkyl, and alkenyl; or a pharmaceutically-acceptable salt thereof. 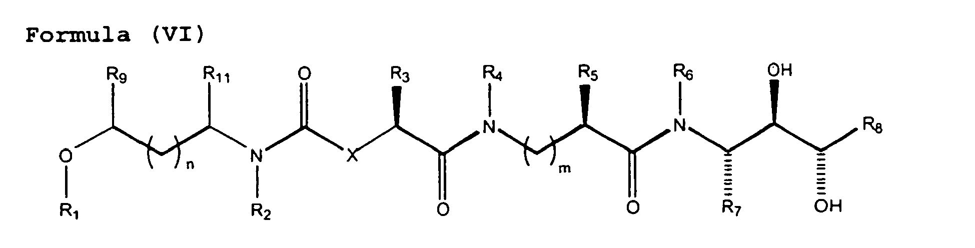 wherein X is selected from oxygen atom, methylene, and NR₁₀,
wherein R₁₀ is selected from hydrido, alkyl, and benzyl;
R₁ is selected from alkyl, cycloalkyl, alkylacyl, haloalkylacyl, phenyl, heterocyclicalkyll, dialkylaminoalkyl, benzyl, naphthyl, and naphthylmethyl, any one of which groups having a substitutable position may be optionally substituted with one or more radicals selected from alkyl, alkoxy, alkenyl, alkynyl, halo, haloalkyl, cyano, and phenyl;
R₂ is selected from hydrido, alkyl, alkylaminoalkyl, alkylacylaminoalkyl, benzyl, and cycloalkyl;
R₃ is selected from alkyl, acylaminoalkyl, phenylalkyl, naphthylmethyl, aryl, and heterocyclicalkyl,
wherein the aromatic portion of any of said phenylalkyl, naphthylmethyl, aryl, and heterocyclicalkyl may be substituted by one or more halo or alkyl or by both;
R₄ and R₆ are each independently selected from hydrido, alkyl, benzyl, and cycloalkyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, alkenyl, alkynyl, and cyano;
R₈ is selected from hydrido, alkyl, haloalkyl, alkylcycloalkyl, alkylcycloalkenyl, and alkoxycarbonyl;
R₉ and R₁₁ are each independently selected from hydrido, alkyl, alkylaminoalkyl, and phenyl;
m is an integer from 0 to 1; and
n is an integer selected from 1 to 5,
with the proviso that where m is 0, then R₅ is selected from hydrido, alkyl, benzyl, cycloalkyl, cycloalkylalkyl, hydroxyalkyl, alkoxyalkyl, alkylthioalkyl, heterocyclicalkyl, sulfonylheterocyclicalkyl, and acylheterocyclicalkyl; and
with the further proviso that when m is 1, then R₅ is selected from hydrido, alkyl, benzyl, cycloalkyl, cycloalkylalkyl, alkoxyalkyl, alkylthioalkyl, and imidazolemethyl; or pharmaceutically-acceptable salts thereof. 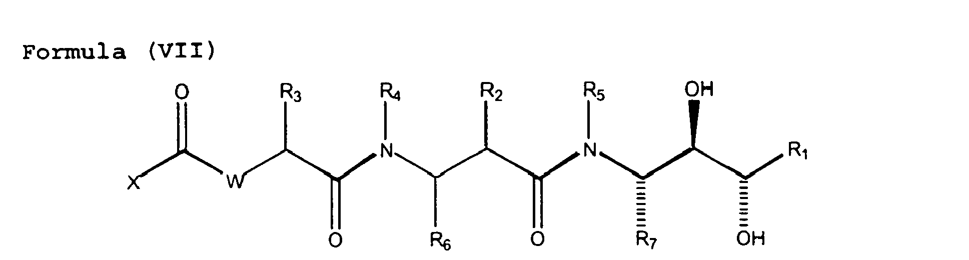 wherein X is selected from hydrido, lower alkyl, alkoxy, alkylamino, benzyloxycarbonyl, phenyl, phenyl substituted with one or more of halo, methoxy, hydroxy, alkyl, amino, aminoalkyl, trifluoromethyl, and 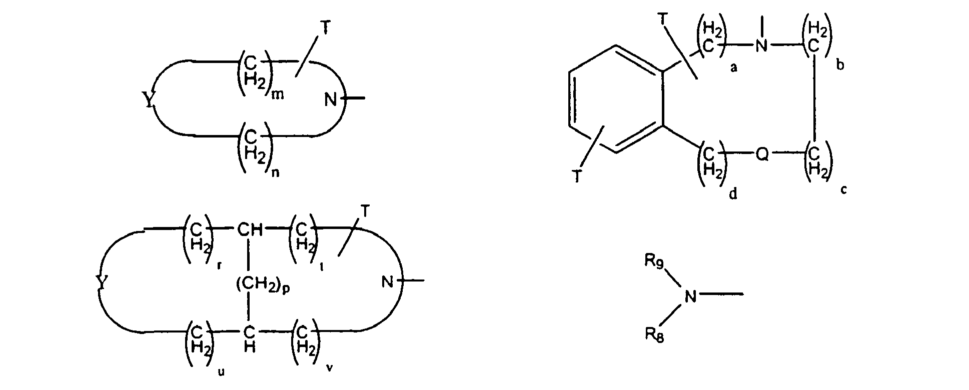 wherein Y and Q are selected from CH₂, CH-O-R₁₀, O, S, SO, SO₂, and NR₁₁,
wherein R₁₀ is selected from hydrido or lower alkyl,
R₁₁ is selected from hydrido, phenyl, and O=CR₁₂ ;
wherein R₁₂ is selected from hydrido or lower alkyl;
w is selected from NR₁₃ and CH₂;
wherein R₁₃ is selected from hydrido and lower alkyl;
m and n are each independently an integer from 1 through 4;
r, t, u, and v are each independently an integer from 0 through 2;
p is an integer from 1 through 3;
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
R₁ is selected from hydrido, linear or branched lower alkyl, haloalkyl, alkylcycloalkyl, alkylcycloalkenyl, and alkoxycarbonyl;
R₂ is selected from linear or branched lower alkyl, imidazolemethyl, and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from hydrido or phenyl;
R₇ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₈ and R₉ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₈ and R₉ is an aryl group. 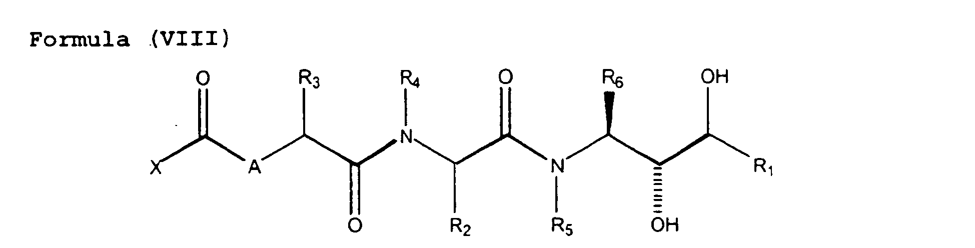 wherein X is selected from 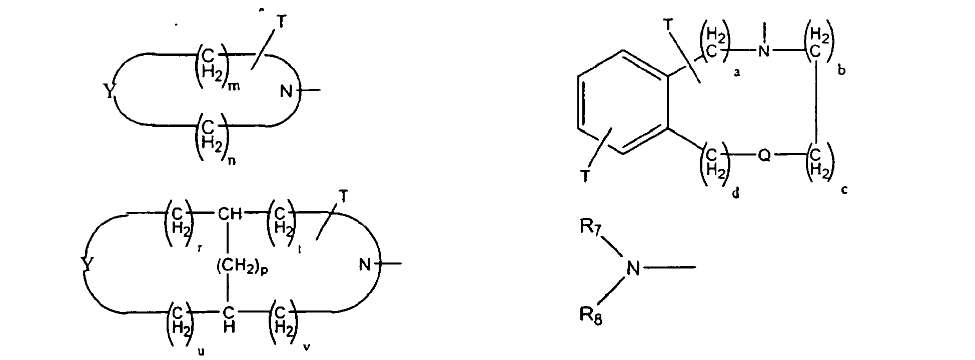 wherein Y and Q are selected from CH₂, CH-O-R₉, O, S, SO, SO₂ and NR₁₀,
wherein R₉ is hydrido or lower alkyl,
R₁₀ is selected from hydrido, phenyl, and O=CR₁₁, and
wherein R₁₁ is hydrido or lower alkyl;
m and n are each independently an integer from 1 through 4;
r, t, u, and v are each independently an integer from 0 through 2;
p is an integer from 1 though 3,
a, b, c, and d are each independently an integer from 0 through 3;
T is selected from one or more groups selected from hydrido, linear or branched lower alkyl, alkoxy, oxo, halo, haloalkyl, lower alkenyl, lower alkynyl, and cyano;
A is selected from O and S;
R₁ is selected from hydrido, linear or branched lower alkyl, haloalkyl, alkylcycloalkyl, alkylcycloalkenyl, and alkoxycarbonyl;
R₂ is selected from linear or branched lower alkyl and benzyl;
R₃ is selected from lower alkyl, acylaminoalkyl, benzyl, naphthylmethyl, aryl, and benzyl substituted at the phenyl portion by halo or lower alkyl or by both;
R₄ and R₅ are each independently selected from hydrido or lower alkyl;
R₆ is selected from substituted or unsubstituted cycloalkyl, phenyl, cycloalkylalkyl, and phenylalkyl, any one of which may be substituted with one or more groups selected from alkyl, alkoxy, halo, haloalkyl, lower alkenyl, lower alkynyl, and cyano; and
R₇ and R₈ are each independently selected from the groups hydrido, lower alkyl, cycloalkyl, phenyl, benzyl, naphthyl, and naphthylmethyl, any one of which groups having a substitutable position may be optionally substituted with one or more of lower alkyl, alkoxy, alkenyl, alkynyl, halo, haloalky, cyano, and phenyl,
with the proviso that at least one or R₇ and R₈ is an aryl group; or pharmaceutically-acceptable salts thereof. 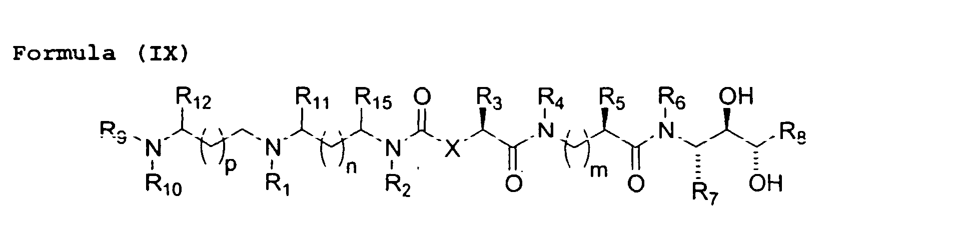 wherein X is selected from oxygen atom, methylene and >NR₁₃,
wherein NR₁₃ is selected from hydrido, alkyl, and benzyl;
R₉ and R₁₀ are each independently selected from hydrido, alkyl, cycloalkyl, alkoxycarbonyl, benzyloxycarbonyl, lower alkanoyl, alkyaminoalkyl, dialkylaminoalkyl, aminoalkyl, alkylaminoalkylaminoalkyl, haloalkylacyl, phenyl, benzyl, heterocyclicalkyl, naphthyl, and naphthylmethyl, any one of which groups having a substitutable position can be optionally substituted with one or more radicals selected from alkyl, alkoxy, alkenyl, alkynyl, halo, haloalkyl, cyano, and phenyl;
wherein R₉ and R₁₀ can be taken together to form a heterocyclic ring having one or two hetero atoms as ring atoms selected from nitrogen, oxygen, and sulfur, which heterocyclic ring has 4 to 10 ring members and contains as a ring member the nitrogen atom of Formula IX to which R₉ and R₁₀ are attached;
R₁ and R₂ are each independently selected from hydrido, alkyl, alkylaminoalkyl, dialkylaminoalkyl, alkyacylaminoalkyl, benzyl, and cycloalkyl
R₃ is selected from alkyl, acylaminoalkyl, phenylalkyl, naphthylmethyl, cycloalkylalkyl, aryl, and heterocyclicalkyl, wherein the aromatic portion of any of said phenyalkyl, naphthylmethyl, aryl and heterocyclicalkyl can be substituted by one or more halo or alkyl or by both;
R₄ and R₆ are each independently selected from hydrido, alkyl, benzyl, and cycloalkyl
R₇ is selected from subsituted or unsubsituted cycloalkyl, phenyl, cycloalkyalkyl, and phenylalkyl, any one of which may be subsitited with one or more groups selected from alkyl, alkoxy, halo, haloalkyl, alkenyl, alkynyl, and cyano;
R₈ is selected from hydrido, alkyl, haloalkyl, alkycycloalkyl, alkycycloalkenyl, alkoxycarbonyl, -COOR₁₆, and -CH (OH) R₁₆,
wherein the carbon bearing the hydroxyl radical is in the S configuration;
wherein R₁₆ is selected from hydrido, alkyl, haloalkyl, alkycycloalkyl, alkylcycloalkenyl, and alkoxycarbonyl;
R₁₁, R₁₂, R₁₄, R₁₅ are each independently selected from hydrido, alkyl, alkylaminoalkyl, and phenyl;
m is 0 or 1;
n and p are each independently an integer selected from 0 through 5; or a pharmaceutically-acceptable salt thereof;
with the proviso that where m is 0, then R₅ is selected from hydrido, alkyl, benzyl, cycloalkyl, cycloalkylalkyl, hydroxyalkyl, alkoxyalkyl, alkythioalkyl, heterocyclicalkyl, sulfonylheterocyclicalkyl, and acylheterocyclicalkyl; and
with the further proviso that when m is 1, the R₅ is selected from hydrido, alkyl, benzyl, cycloalkyl, cycloalkylalkyl, alkoxyalkyl, alkylthioalkyl, and imidazolemethyl. 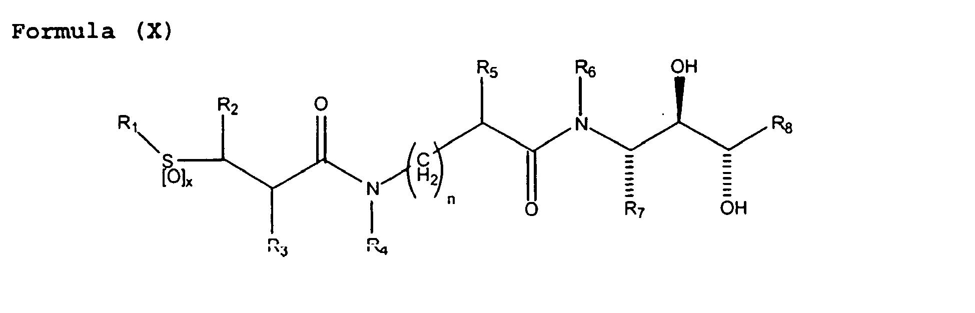 wherein R₁ is a group selected from alkyl, trifluoromethyl, cycloalkyl, cycloalkylalkyl, aryl, haloaryl, aralkyl, and haloaralkyl;
x is a number selected from 0, 1, and 2;
n is an number selected from 0 and 1;
R₂ is selected from hydrido and alkyl;
R₃ is a group selected from hydrido, cycloalkylalkyl, aralkyl and haloaralkyl;
R₄ and R₆ are each independently selected from hydrido and methyl;
R₅ is selected from linear and branched alkyl groups containing from one to about four carbon atoms;
R₇ is a group selected from alkyl, cycloalkylalkyl, and aralkyl;
R₈ is a group selected from hydrido, alkyl, hydroxyalkyl, cycloalkyl, cycloalkylalkyl, alkenyl, alkenylalkyl, and haloalkenyl; and
wherein any one of said R₁ through R₈ groups having a substitutable position may be substituted with one or more groups selected from alkyl, haloalkyl, hydroxy, hydroxyalkyl, alkoxy, alkoxyalkyl, and alkenyl, or pharmaceutically-acceptable salts thereof.

29. A method of treatment according to any of claims 1, 11, 12, 13, or 14, further comprising administration of one or more therapeutic agents selected from the group consisting of an antioxidant, an anti-inflammatory, a gamma secretase inhibitor, a neurotrophic agent, an acetyl cholinesterase inhibitor, a statin, P-gp inhibitors, an A beta peptide, and an anti-A beta peptide.

30. Use of a compound according to claim 15, or mixtures thereof, for the manufacture of a medicament for the treatment or prevention of conditions selected from the group consisting of: Alzheimer's disease, mild cognitive impairment (MCI) Down's syndrome, Hereditary Cerebral Hemorrhage with Amyloidosis of the Dutch-Type, cerebral amyloid angiopathy, degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease.
